(19) Europäisches Patentamt
European Patent Office
Office européen des brevets

(11) EP 1 632 488 A1

(12) **EUROPEAN PATENT APPLICATION**
published in accordance with Art. 158(3) EPC

(43) Date of publication:
**08.03.2006 Bulletin 2006/10**

(21) Application number: **04746020.9**

(22) Date of filing: **10.06.2004**

(51) Int Cl.:
***C07D 401/14*** *(1990.01)* ***C07D 409/14*** *(1990.01)*
***C07D 471/04*** *(1985.01)* ***A61K 31/444*** *(2000.01)*
***A61K 31/437*** *(2000.01)* ***A61P 1/04*** *(2000.01)*
***A61P 1/16*** *(2000.01)* ***A61P 3/10*** *(2000.01)*
***A61P 9/10*** *(2000.01)* ***A61P 11/06*** *(2000.01)*
***A61P 13/12*** *(2000.01)* ***A61P 17/06*** *(2000.01)*
***A61P 19/00*** *(2000.01)* ***A61P 19/02*** *(2000.01)*
***A61P 19/10*** *(2000.01)* ***A61P 25/28*** *(2000.01)*
***A61P 29/00*** *(2000.01)* ***A61P 31/04*** *(2000.01)*
***A61P 31/12*** *(2000.01)* ***A61P 35/00*** *(2000.01)*
***A61P 37/02*** *(2000.01)*

(86) International application number:
**PCT/JP2004/008492**

(87) International publication number:
**WO 2004/111037 (23.12.2004 Gazette 2004/52)**

(84) Designated Contracting States:
**AT BE BG CH CY CZ DE DK EE ES FI FR GB GR HU IE IT LI LU MC NL PL PT RO SE SI SK TR**

(30) Priority: **11.06.2003 JP 2003165991**

(71) Applicant: **Sankyo Company, Limited**
**Tokyo 103-8426 (JP)**

(72) Inventors:
- **KIMURA, Tomio,**
  **c/o Sankyo Company, Limited**
  **Shinagawa-ku, Tokyo 1408710 (JP)**
- **OHKAWA, Nobuyuki,**
  **c/o Sankyo Company, Limited**
  **Shinagawa-ku, Tokyo 1408710 (JP)**
- **NAKAO, Akira,**
  **c/o Sankyo Company, Limited**
  **Shinagawa-ku, Tokyo 1408710 (JP)**
- **NAGASAKI, Takayoshi,**
  **c/o Sankyo Company, Limited**
  **Shinagawa-ku, Tokyo 1408710 (JP)**
- **SHIMOZATO, Takaichi,**
  **c/o Sankyo Company, Limited**
  **Shinagawa-ku, Tokyo 1408710 (JP)**

(74) Representative: **Gibson, Christian John Robert**
**Marks & Clerk**
**90 Long Acre**
**London WC2E 9RA (GB)**

# (54) CYCLIC TERTIARY AMINE COMPOUND

(57) The present invention provides a cyclic tertiary amine compound which is capable of inhibiting the production of inflammatory cytokines. It is either a compound having a structure represented by the following general formula (I):

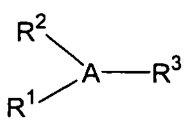

(I)

(wherein A represents an optionally substituted trivalent group derived from pyrimidine, pyrrole, or the like; $R^1$ represents an aryl or a heteroaryl group which may optionally be substituted; $R^2$ represents a heteroaryl group which may optionally be substituted; and $R^3$ represents a cyclic tertiary amino group) or a pharmacologically acceptable salt of the compound.

EP 1 632 488 A1

## Description

[Technical field]

The present invention relates to cyclic tertiary amine compounds. The present invention relates to cyclic tertiary amine compounds that have inhibitory action against production of inflammatory cytokines such as interleukin (IL)-1, IL-6, IL-8, tumor necrosis factor (TNF), and the like, and that are useful as therapeutic or prophylactic agents for autoimmune diseases such as inflammation with fever and pain as well as rheumatoid arthritis, osteoarthritis, diabetes mellitus (particularly type I diabetes mellitus), osteogenic disorders such as osteoporosis, and other diseases that are mediated by cytokines listed above.

[Background art]

Non-steroidal anti-inflammatory drugs (NSAIDs) have been widely used for the treatment and prophylaxis of various inflammatory diseases and in pain relief because they have, as their main pharmacological activity, anti-pyretic, analgesic, and anti-inflammatory activity which is based on their ability to inhibit the biosynthesis of prostaglandin (PG) through the inhibition of cyclooxygenase activity. For the treatment of rheumatoid arthritis, NSAIDs are used nosotropically and immunomodulators (DMARDs: the disease-modifying anti-rheumatic drugs) are used etiotropically.
Current NSAIDs can induce alimentary canal disorders such as gastric ulcer and so forth due to their ability to inhibit the biosynthesis of PGs. Thus NSAIDs present difficulties for continuous long-term administration. DMARDs have not yet been clearly shown to exhibit a stable, long-lasting effect. Recently, active substances that are secreted from immunocytes have been found, which are generally called cytokines. Among them, interleukin (IL)-1, IL-6, IL-8, tumor necrosis factor (TNF), and so forth are known as the inflammatory cytokines and their various functions as inflammatory mediators include activation of arachidonic acid metabolism to produce PGs, migration of leukocytes, production of acute phase protein, and activation of osteoclasts.
Thus, substances that inhibit the production of these inflammatory cytokines are expected to act as novel agents against autoimmune diseases such as inflammation with fever and pain as well as rheumatoid arthritis, osteogenic disorders such as osteoporosis, and other diseases mediated by cytokines listed above.
Although there exist several heteroaryl compounds that inhibit production of inflammatory cytokines (see for example, WO96/21452, WO97/5877, WO97/23479, WO98/52937, WO00/31063, WO02/57255, WO02/57264, WO02/57265, EP1070711, J. Med. Chem., vol. 39, 3929 - 3937 (1996)), a need remains for the development of superior compounds in terms of actions, pharmacokinetics, and safety.

[Disclosure of the invention]

As a result of seeking to develop compounds having inhibitory activity against production of the inflammatory cytokines listed above, and conducting diligent research over an extended period of time on the synthesis of various compounds and their pharmacological activity, the inventors of the present invention have discovered cyclic tertiary amine compounds having a marked inhibitory effect on inflammatory cytokine production and superior safety, and have completed the present invention.
The present invention relates to the compounds having the general formula (I) shown below and their pharmacologically acceptable salts:

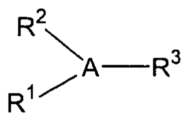
(I)

wherein, A represents a trivalent group selected from the group consisting of benzene, pyridine, pyridazine, pyrimidine, pyrrole, furan, thiophene, pyrazole, imidazole, isoxazole and isothiazole which may optionally be substituted with group (s) selected from Substituent group δ;
$R^1$ represents an aryl group which may optionally be substituted with group(s) selected from the group consisting of Substituent group α and Substituent group β; or a heteroaryl group which may optionally be substituted with group(s) selected from the group consisting of Substituent group α and Substituent group β;
$R^2$ represents a heteroaryl group which contains at least one nitrogen atom and further may optionally be substituted with group(s) selected from the group consisting of Substituent group α and Substituent group β;
$R^3$ represents a group having general formula (IIa) or (IIb) shown below::

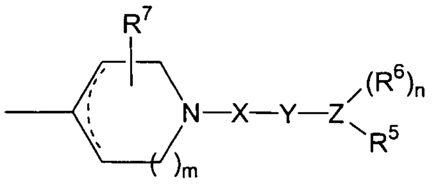

(IIa)

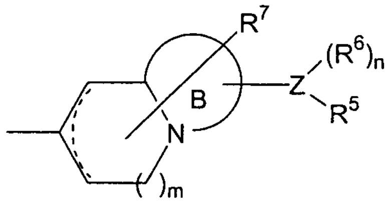

(IIb)

[wherein, the bond including a dotted line moiety represents a single bond or a double bond;
Ring B represents a 4- to 7-membered heterocyclyl ring (said ring is saturated or unsaturated; and may optionally be fused with another group such as an aryl group, a heteroaryl group, a cycloalkyl group or a heterocyclyl group);
X represents a straight or branched alkylene group having from 1 to 5 carbon atoms;
Y represents a single bond or a group having formula: $C(R^8a)(R^8b)$ ($R^8a$ and $R^8b$ are the same or different and each represents independently a hydrogen atom, a hydroxyl group, a halogen atom, a lower alkyl group or a lower alkoxy group, or $R^8a$ and $R^8b$ together form an oxo group or a methylene group, or $R^8a$ and $R^8b$ together with the carbon atom to which they are bonded form a 3- to 6-membered cycloalkyl group);
Z represents an arylene group or a heteroarylene group;
m represents an integer of from 0 to 2;
$R^5$ represents a carboxyl group, a lower alkoxycarbonyl group, an aralkyloxycarbonyl group, an aryloxycarbonyl group, a group having formula: $CONR^aR^b$, a group having formula: $COR^c$, a group having formula: $SO_2NR^aR^b$, a group having formula: $SO_2R^c$ or a group having formula: $SOR^c$;
$R^a$ and $R^b$ are the same or different and each represents independently a hydrogen atom; a hydroxyl group; a lower alkyl group which may optionally be substituted with group(s)selected from Substituent group α; a lower alkenyl group which may optionally be substituted with group(s) selected from Substituent group α; a lower alkynyl group which may optionally be substituted with group(s) selected from Substituent group α; a lower alkoxy group; a lower alkenyloxy group; a lower alkynyloxy group; an aralkyloxy group; a cycloalkyl group; a lower alkyl group substituted with a cycloalkyl group; an aryl group; an aralkyl group; a heteroaryl group; a lower alkyl group substituted with a heteroaryl group; an amino group; or a mono- or di-lower alkylamino group;
$R^c$ represents a hydrogen atom, a lower alkyl group, a halogeno lower alkyl group, a lower alkoxy lower alkyl group or a hydroxy lower alkyl group;
$R^6$ represents 1 to 2 groups selected from the group consisting of a hydrogen atom, a hydroxyl group, an amino group, a nitro group, a cyano group, a halogen atom, a lower alkyl group, a lower alkoxy group, a lower alkylthio group, a halogeno lower alkyl group, a halogeno lower alkoxy group and a halogeno lower alkylthio group;
n represents an integer of from 1 to 2 (when n is 2, each $R^6$ may be the same or different);
$R^7$ represents 1 to 3 groups selected from the group consisting of a hydrogen atom, a hydroxyl group, a halogen atom, a lower alkyl group, a lower alkoxy group, a lower alkylthio group, a halogeno lower alkyl group, a halogeno lower alkoxy group and a halogeno lower alkylthio group];

Substituent group α is the group consisting of a hydroxyl group, a nitro group, a cyano group, a halogen atom, a lower alkoxy group, a halogeno lower alkoxy group, a carboxyl group, a lower alkoxycarbonyl group, a carbamoyl group, a lower acyloxy group, a lower alkylthio group, a halogeno lower alkylthio group and a group having formula: $-NR^dR^e$ (wherein, $R^d$ and $R^e$ are the same or different and each represents independently a hydrogen atom, a lower alkyl group, a lower alkenyl group, a lower alkynyl group, an aralkyl group, a lower alkylsulfonyl group or a lower alkylcarbonyl group, or $R^d$ and $R^e$ together with the nitrogen atom to which they are bonded form a heterocyclyl group);

Substituent group β is the group consisting of a lower alkyl group which may optionally be substituted with group(s) selected from Substituent group α; a lower alkenyl group which may optionally be substituted with group(s) selected from Substituent group α; a lower alkynyl group which may optionally be substituted with group(s) selected from Substituent group α; arylalkyl group and a cycloalkyl group;

Substituent group γ is the group consisting of an oxo group, a hydroxyimino group, a lower alkoxyimino group, a lower alkylene group, a lower alkylenedioxy group, a lower alkylsulfinyl group and a lower alkylsulfonyl group; and

Substituent group δ is the group consisting of: a group selected from Substituent group β; a cycloalkyl group substituted with group(s) selected from the group consisting of Substituent group α, Substituent group β and Substituent group γ; an aryl group which may optionally be substituted with group(s) selected from the group consisting of Substituent group α, Substituent group β and Substituent group γ; a heteroaryl group which may optionally be substituted with group (s) selected from the group consisting of Substituent group α, Substituent group β and Substituent group γ; and a heterocyclyl group which may optionally be substituted with group(s) selected from the group consisting of Substituent

group $\alpha$, Substituent group $\beta$ and Substituent group $\gamma$,
PROVIDED THAT each of the atoms of the Ring A to which $R^1$ and $R^3$ are bonded is adjacent to the atom of the Ring A to which $R^2$ is bonded.

Of the compounds above, examples of the preferable compounds are as follows:

(2) compounds according to (1) wherein A is a trivalent group selected from the group consisting of a pyrrole group which may optionally be substituted with two groups selected from Substituent group $\delta$ and a pyrazole group which may optionally be substituted with one group selected from Substituent group $\delta$, or pharmacologically acceptable salts thereof;
(3) compounds according to (1) wherein A is a pyrrole group which may optionally be substituted with two groups selected from Substituent group $\delta$, or pharmacologically acceptable salts thereof;
(4) compounds according to (1) wherein A is a pyrrole group, or pharmacologically acceptable salts thereof;
(5) compounds according to any one of (1) to (4) wherein $R^1$ is an aryl group which may optionally be substituted with group(s) selected from the group consisting of Substituent group $\alpha$ and Substituent group $\beta$, or pharmacologically acceptable salts thereof;
(6) compounds according to any one of (1) to (4) wherein $R^1$ is a phenyl group or a naphthyl group which may optionally be substituted with group(s) selected from the group consisting of Substituent group $\alpha$ and Substituent group $\beta$, or pharmacologically acceptable salts thereof;
(7) compounds according to any one of (1) to (4) wherein $R^1$ is a phenyl group which may optionally be substituted with group(s) selected from the group consisting of Substituent group $\alpha^1$ and Substituent group $\beta^1$;
Substituent group $\alpha^1$ is the group consisting of a hydroxyl group, a cyano group, a halogen atom, a lower alkoxy group and a halogeno lower alkoxy group; and
Substituent group $\beta^1$ is the group consisting of a lower alkyl group, a halogeno lower alkyl group and a hydroxy lower alkyl group, or pharmacologically acceptable salts thereof;
(8) compounds according to any one of (1) to (4) wherein $R^1$ is a phenyl group or a phenyl group which is substituted with group(s) selected from the group consisting of a hydroxyl group, a cyano group, a halogen atom, a lower alkoxy group, a halogeno lower alkyl group and a halogeno lower alkoxy group, or pharmacologically acceptable salts thereof;
(9) compounds according to any one of (1) to (4) wherein $R^1$ is a phenyl, 3-cyanophenyl, 4-fluorophenyl, 3-fluorophenyl, 4-chlorophenyl, 3-chlorophenyl, 2,4-difluorophenyl, 3,4-difluorophenyl, 3,4-di chlorophenyl, 3,4,5-trifluorophenyl, 3-chloro-4-fluorophenyl, 3-methoxyphenyl, 3-difluoromethoxyphenyl, 3-trifluoromethoxyphenyl, 3-trifluoromethylphenyl or 4-fluoro-3-methoxyphenyl group, or pharmacologically acceptable salts thereof;
(10) compounds according to any one of (1) to (4) wherein $R^1$ is a phenyl, 3-cyanophenyl, 4-fluorophenyl, 3-fluorophenyl, 3-chlorophenyl, 3,4-difluorophenyl, 3,4,5-trifluorophenyl, 3-chloro-4-fluorophenyl, 3-methoxyphenyl, 3-difluoromethoxyphenyl, 3-trifluoromethylphenyl or 4-fluoro-3-methoxyphenyl, or pharmacologically acceptable salts thereof;
(11) compounds according to any one of (1) to (4) wherein $R^1$ is a phenyl, 4-fluorophenyl, 3-fluorophenyl, 3-chlorophenyl, 3,4-difluorophenyl, 3-chloro-4-fluorophenyl or 3-trifluoromethylphenyl group, or pharmacologically acceptable salts thereof;
(12) compounds according to any one of (1) to (11) wherein $R^2$ is a 5- to 6-membered heteroaryl group which contains one or two nitrogen atoms and further may optionally be substituted with group(s) selected from the group consisting of Substituent group $\alpha$ and Substituent group $\beta$, or pharmacologically acceptable salts thereof;
(13) compounds according to any one of (1) to (11) wherein $R^2$ is a pyridyl group or a pyrimidinyl group which may optionally be substituted with group(s) selected from the group consisting of Substituent group $\alpha$ and Substituent group $\beta$, or pharmacologically acceptable salts thereof;
(14) compounds according to any one of (1) to (11) wherein $R^2$ is a 4-pyridyl group or a 4-pyrimidinyl group which may optionally be substituted with group(s) selected from the group consisting of Substituent group $\alpha$ and Substituent group $\beta$, or pharmacologically acceptable salts thereof;
(15) compounds according to any one of (1) to (11) wherein $R^2$ is a 4-pyridyl group or a 4-pyrimidinyl group, of which the 2-position may optionally be substituted with one group selected from the group consisting of Substituent group $\alpha$ and Substituent group $\beta$, or pharmacologically acceptable salts thereof;
(16) compounds according to any one of (1) to (11) wherein $R^2$ is a 4-pyridyl group or a 4-pyrimidinyl group, of which the 2-position may optionally be substituted with one group selected from the group consisting of a methoxy, amino, methylamino, benzylamino and $\alpha$-methylbenzylamino group, or pharmacologically acceptable salts thereof;
(17) compounds according to any one of (1) to (16) wherein m is 1, or pharmacologically acceptable salts thereof;
(18) compounds according to any one of (1) to (17) wherein $R^3$ is a group having formula: (IIa), X is an alkylene group having from 1 to 4 carbon atoms, Y is a group having formula: $C(R^8a)(R^8b)$ ($R^8a$ and $R^8b$ are the same or different and each represents independently a hydrogen atom, a hydroxyl group, a halogen atom, an alkyl group

having from 1 to 4 carbon atoms or an alkoxy group having from 1 to 4 carbon atoms, or $R^8$a and $R^8$b together form an oxo group or methylene group, or $R^8$a and $R^8$b together with the carbon atom to which they are bonded form a 3-to 6-membered cycloalkyl group), or pharmacologically acceptable salts thereof;

(19) compounds according to any one of (1) to (17) wherein $R^3$ is a group having formula: (IIa) ; X is a methylene group; and Y is a group having formula: C($R^8$a)($R^8$b) ($R^8$a and $R^3$b are the same or different and each represents independently a hydrogen atom, a hydroxyl group, a fluorine atom, a methyl group, an ethyl group, a methoxy group or an ethoxy group, or $R^8$a and $R^8$b together form an oxo group or a methylene group, or $R^8$a and $R^8$b together with the carbon atom to which they are bonded form a cyclopropyl group), or pharmacologically acceptable salts thereof;

(20) compounds according to any one of (1) to (17) wherein $R^3$ is a group having formula: (IIa) ; X is a methylene group; and Y is a group having formula: C($R^8$a)($R^8$b) ($R^8$a and $R^8$b are the same or different and each represents independently a hydrogen atom, a fluorine atom, a methyl group, a hydroxyl group or an oxo group), or pharmacologically acceptable salts thereof;

(21) compounds according to any one of (1) to (17) wherein $R^3$ is a group having formula: (IIa) ; X is a methylene group; and Y is a group having formula: $CH_2$, or pharmacologically acceptable salts thereof;

(22) compounds according to any one of (1) to (17) wherein $R^3$ is a group having formula: (IIb), and Ring B is a 5- or 6-membered heterocyclyl ring which contains one nitrogen atom and further may optionally contain one atom or group selected from the group consisting of a nitrogen atom, an oxygen atom, a sulfur atom, a group having formula: =SO and a group having formula: $=SO_2$ (said ring is saturated or unsaturated, and may optionally be fused with an aryl group, a heteroaryl group, a cycloalkyl group or a heterocyclyl group), or pharmacologically acceptable salts thereof;

(23) compounds according to any one of (1) to (17) wherein $R^3$ is a group having formula: (IIb), and Ring B is a 5- or 6-membered heterocyclyl ring which contains one nitrogen atom (said ring is saturated or unsaturated, and may optionally be fused with an aryl group, a heteroaryl group, a cycloalkyl group or a heterocyclyl group), or pharmacologically acceptable salts thereof;

(24) compounds according to any one of (1) to (17) wherein $R^3$ is a group having formula: (IIb), and Ring B is a pyrrolidine or pyrroline group, or pharmacologically acceptable salts thereof;

(25) compounds according to any one of (1) to (24) wherein Z is a phenylene group, a thiophenediyl group, a furandiyl group, a pyrrolediyl group, an oxazolediyl group, a thiazolediyl group, a thiadiazolediyl group or a pyridinediyl group, or pharmacologically acceptable salts thereof;

(26) compounds according to any one of (1) to (24) wherein Z is a phenylene group or a thiophenediyl group, or pharmacologically acceptable salts thereof;

(27) compounds according to any one of (1) to (26) wherein $R^5$ is a group having formula: $CONR^aR^b$, a group having formula: $COR^c$, a group having formula: $SO_2NR^aR^b$, a group having formula: $SO_2R^c$ or a group having formula: $SOR^c$ (wherein, $R^a$ and $R^b$ are the same or different and each is independently a hydrogen atom, a lower alkyl group which may optionally be substituted with group(s) selected from Substituent group $\alpha$, a lower alkoxy group, a lower alkenyloxy group, a cycloalkyl group, an amino group, or mono- or di-lower alkylamino group; and $R^c$ is a lower alkyl group), or pharmacologically acceptable salts thereof;

(28) compounds according to any one of (1) to (26) wherein $R^5$ is a group having formula: $CONR^aR^b$, a group having formula: $SO_2NR^aR^b$, a group having formula: $SO_2R^c$ or a group having formula: $SOR^c$ (wherein, $R^a$ and $R^b$ are the same or different and each is independently a hydrogen atom, a lower alkyl group, a halogeno lower alkyl group, a hydroxy lower alkyl group, a lower alkoxy lower alkyl group, a lower alkoxy group or a cycloalkyl group; and $R^c$ is a lower alkyl group), or pharmacologically acceptable salts thereof;

(29) compounds according to any one of (1) to (26) wherein $R^5$ is a carbamoyl, N-methylcarbamoyl, N-ethylcarbamoyl, N-propylcarbamoyl, N-isopropylcarbamoyl, N-cyclopropylcarbamoyl, N-cyclopentylcarbamoyl, N-(2-fluoroethyl)carbamoyl, N-(2-methoxyethyl)carbamoyl, N-methylsulfamoyl, N-ethylsulfamoyl, N-propylsulfamoyl, N-isopropylsulfamoyl, N-cyclopropylsulfamoyl, N-cyclopentylsulfamoyl, N-(2-fluoroethyl)sulfamoyl, N-(methoxyethyl)sulfamoyl, N-methoxysulfamoyl, methanesulfonyl, ethanesulfonyl, propanesulfonyl, methanesulfinyl, ethanesulfinyl or propanesulfinyl group, or pharmacologically acceptable salts thereof;

(30) compounds according to any one of (1) to (29) wherein $R^6$ is 1 to 2 groups selected from the group consisting of a hydrogen atom, a fluorine atom and a methoxy group, or pharmacologically acceptable salts thereof;

(31) compounds according to any one of (1) to (29) wherein $R^6$ is a hydrogen atom, or pharmacologically acceptable salts thereof;

(32) compounds according to any one of (1) to (31) wherein $R^7$ is 1 to 2 groups selected from the group consisting of a hydrogen atom, a hydroxyl group and a lower alkyl group, or pharmacologically acceptable salts thereof;

(33) compounds according to any one of (1) to (31) wherein $R^7$ is a hydrogen atom, or pharmacologically acceptable salts thereof;

(34) compounds according to any one of (1) to (33) in which general formula (I) is any of the structures shown below, or pharmacologically acceptable salts thereof;

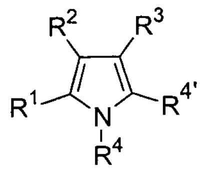, 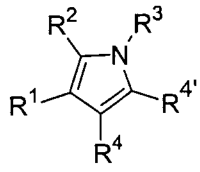, 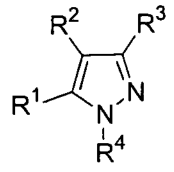 or 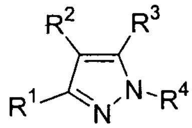

(wherein, $R^4$ and $R^{4'}$ are the same or different and each represents independently a hydrogen atom or a group selected from Substituent group δ), and

(35) compounds according to any one of (1) to (33) in which general formula (I) is either of the structures shown below, or pharmacologically acceptable salts thereof;

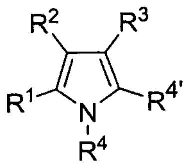 or 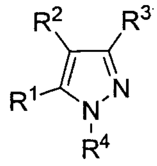

(wherein, $R^4$ and $R^{4'}$ are the same or different and each represents independently a hydrogen atom or a group selected from Substituent group δ).

Additionally, compounds having general formula (I) described in (1) which comprise any combination selected freely from 10 groups consisting of (2) to (4); (5) to (11); (12) to (16); (17); (18) to (21); (25) to (26); (27) to (29); (30) to (31); (32) to (33); and (34) to (35), or pharmacologically acceptable salts thereof, and

compounds which comprise any combination selected freely from the 10 groups consisting of (2) to (4); (5) to (11); (12) to (16); (17); (22) to (24); (25) to (29); (30) to (31); (32) to (33); and (34) to (35), or pharmacologically acceptable salts thereof, are preferred.

Furthermore,

(36) compounds having general formula (I) according to (1) which is selected from the compounds shown below or pharmacologically acceptable salts thereof are more preferred:

4-[1-(4-carbamoylphenethyl)-1,2,3,6-tetrahydropyridin-4-yl]-2-(4-fluorophenyl)-3-(pyridin-4-yl)-1H-pyrrole,
3-[1-(4-carbamoylphenethyl)-1,2,3,6-tetrahydropyridin-4-yl]-5-(4-fluorophenyl)-4-(pyridin-4-yl)-1H-pyrazole,
2-(4-fluorophenyl)-4-[1-[4-(N-methylcarbamoyl)phenethyl]-1,2,3,6-tetrahydropyridin-4-yl]-3-(pyridin-4-yl)-1H-pyrrole,
4-[1-[4-(N-ethylcarbamoyl)phenethyl]-1,2,3,6-tetrahydropyridin-4-yl]-2-(4-fluorophenyl)-3-(pyridin-4-yl)-1H-pyrrole,
4-[1-[(4-cyclopropylcarbamoyl)phenethyl]-1,2,3,6-tetrahydropyridin-4-yl]-2-(4-fluorophenyl)-3-(pyridin-4-yl)-1H-pyrrole,
2-(4-fluorophenyl)-4-[1-[4-(N-isopropylcarbamoyl)phenethyl]-1,2,3,6-tetrahydropyridin-4-yl]-3-(pyridin-4-yl)-1H-pyrrole,
2-(4-fluorophenyl)-4-[1-[4-(N-propylcarbamoyl)phenethyl]-1,2,3,6-tetrahydropyridin-4-yl]-3-(pyridin-4-yl)-1H-pyrrole,
4-[1-[4-[N-(2-fluoroethyl)carbamoyl)phenethyl]-1,2,3,6-tetrahydropyridin-4-yl]-2-(4-fluorophenyl)-3-(pyridin-4-yl)-1H-pyrrole;
4-[1-[4-(N-cyclopentylcarbamoyl)phenethyl]-1,2,3,6-tetrahydropyridin-4-yl]-2-(4-fluorophenyl)-3-(pyridin-4-yl)-1H-pyrrole,
4-[1-[4-[N-(ethoxycarbonylmethyl)carbamoyl]phenethyl]-1,2,3,6-tetrahydropyridin-4-yl]-2-(4-fluorophenyl)-3-(pyridin-4-yl)-1H-pyrrole,
2-(4-fluorophenyl)-4-[1-[4-(N-methylsulfamoyl)phenethyl]-1,2,3,6-tetrahydropyridin-4-yl]-3-(pyridin-4-yl)-1H-pyrrole,
4-[1-[4-(N-ethylsulfamoyl)phenethyl]-1,2,3,6-tetrahydropyridin-4-yl]-2-(4-fluorophenyl)-3-(pyridin-4-yl)-1H-pyrrole,
2-(4-fluorophenyl)-4-[1-[4-(N-propylsulfamoyl)phenethyl]-1,2,3,6-tetrahydropyridin-4-yl]-3-(pyridin-4-yl)-1H-pyrrole,
2-(4-fluorophenyl)-4-[1-[4-(N-isopropylsulfamoyl)phenethyl]-1,2,3,6-tetrahydropyridin-4-yl]-3-(pyridin-4-yl)-1H-pyrrole,
2-(3-chloro-4-fluorophenyl)-4-[1-[4-(N-methylsulfamoyl)phenethyl]-1,2,3,6-tetrahydropyridin-4-yl]-3-(pyridin-4-yl)-1H-pyrrole,
4-[1-[4-(N-cyclopropylsulfamoyl)phenethyl]-1,2,3,6-tetrahydropyridin-4-yl]-2-(4-fluorophenyl)-3-(pyridin-4-yl)-1H-pyrrole,
4-[1-[4-[N-(2-fluoroethyl)sulfamoyl]phenethyl]-1,2,3,6-tetrahydropyridin-4-yl]-2-(4-fluorophenyl)-3-(pyridin-4-yl)-1H-pyrrole,
4-[1-[4-(N,N-dimethylsulfamoyl)phenethyl]-1,2,3,6-tetrahydropyridin-4-yl]-2-(4-fluorophenyl)-3-(pyridin-4-yl)-1H-pyrrole,
2-(4-fluorophenyl)-4-[1-[4-[N-(2-methoxyethyl)sulfamoyl]phenethyl]-1,2,3,6-tetrahydropyridin-4-yl]-3-(pyridin-4-yl)-1H-pyrrole,
4-[1-[4-(N-ethoxysulfamoyl)phenethyl]-1,2,3,6-tetrahydropyridin-4-yl]-2-(4-fluorophenyl)-3-(pyridin-4-yl)-1H-pyrrole,
4-[1-[4-(N-allyloxysulfamoyl)phenethyl]-1,2,3,6-tetrahydropyridin-4-yl]-2-(4-fluorophenyl)-3-(pyridin-4-yl)-1H-pyrrole,
2-(4-fluorophenyl)-4-[1-(4-methanesulfonylphenethyl)-1,2,3,6-tetrahydropyridin-4-yl]-3-(pyridin-4-yl)-1H-pyrrole,
2-(3-chloro-4-fluorophenyl)-4-[1-(4-methanesulfonylphenethyl)-1,2,3,6-tetrahydropyridin-4-yl]-3-(pyridin-4-yl)-1H-pyrrole,
2-(4-fluorophenyl)-4-[1-(4-methanesulfinylphenethyl)-1,2,3,6-tetrahydropyridin-4-yl]-3-(pyridin-4-yl)-1H-pyrrole,
2-(3-chloro-4-fluorophenyl)-4-[1-(4-methanesulfinylphenethyl)-1,2,3,6-tetrahydropyridin-4-yl]-3-(pyridin-4-yl)-1H-pyrrole,
4-[1-(4-acetylphenethyl)-1,2,3,6-tetrahydropyridin-4-yl]-2-(3-chloro-4-fluorophenyl)-3-(pyridin-4-yl)-1H-pyrrole,
2-(3-chloro-4-fluorophenyl)-4-[(2S,8aS)-2-[4-(N-methylcarbamoyl)phenyl]-1,2,3,5,6,8a-hexahydroindolidin-7-yl]-3-(pyridin-4-yl)-1 H-pyrrole,
4-[(2S,8aS)-2-(4-carbamoylphenyl)-1,2,3,5,6,8a-hexahydroindolidin-7-yl]-2-(4-fluorophenyl)-3-(pyridin-4-yl)-1H-pyrrole,
2-(4-fluorophenyl)-4-[(2S,8aS)-2-[4-(N-methylcarbamoyl)phenyl]-1,2,3,5,6,8a-hexahydroindolidin-7-yl]-3-(pyridin-4-yl)-1H-pyrrole,
4-[(2S,8aS)-2-[4-(N-ethylcarbamoyl)phenyl]-1,2,3,5,6,8a-hexahydroindolidin-7-yl]-2-(4-fluorophenyl-3-(pyridin-4-yl)-1H-pyrrole,
4-[(2S,8aS)-2-[4-(N-benzylcarbamoyl)phenyl]-1,2,3,5,6,8a-hexahydroindolidin-7-yl]-2-(4-fluorophenyl-3-(pyridin-4-yl)-1H-pyrrole,
4-[(2S,8aS)-2-[4-(N-cyclopropylcarbamoyl)phenyl]-1,2,3,5,6,8a-hexahydroindolidin-7-yl]-2-(4-fluorophenyl-3-(pyridin-4-yl)-1H-pyrrole,
4-[(2S,8aS)-2-[4-[N-(2-fluoroethyl)carbamoyl]phenyl]-1,2,3,5,6,8a-hexahydroindolidin-7-yl]-2-(4-fluorophenyl-3-(pyridin-4-yl)-1H-pyrrole,
2-(4-fluorophenyl)-4-[(2S,8aS)-2-[4-(N-propylcarbamoyl)phenyl]-1,2,3,5,6,8a-hexahydroindolidin-7-yl]-3-(pyridin-4-yl)-1H-pyrrole,
2-(4-fluorophenyl)-4-[(2S,8aS)-2-[4-(N-isopropylcarbamoyl)phenyl]-1,2,3,5,6,8a-hexahydroindolidin-7-yl]-3-(pyridin-4-yl)-1H-pyrrole,
2-(4-fluorophenyl)-4-[(2S,8aS)-2-[4-[N-(2-methoxyethyl)carbamoyl]phenyl]-1,2,3,5,6,8a-hexahydroindolidin-7-yl]-3-(pyridin-4-yl)-1H-pyrrole,
2-(4-fluorophenyl)-4-[1-[4-(2-methoxyethyl)sulfonylphenethyl]-1,2,3,6-tetrahydropyridin-4-yl]-3-(pyridin-4-yl)-1H-pyrrole,
2-(4-fluorophenyl)-4-[1-[4-(2-hydroxyethyl)sulfonylphenethyl]-1,2,3,6-tetrahydropyridin-4-yl]-3-(pyridin-4-yl)-1H-pyrrole,
2-(3-chloro-4-fluorophenyl)-4-[1-[4-(2-methoxyethyl)sulfonylphenethyl]-1,2,3,6-tetrahydropyridin-4-yl]-3-(pyridin-4-yl)-1H-pyrrole and
2-(3-chloro-4-fluorophenyl)-4-[1-[4-(2-hydroxyethyl)sulfonylphenethyl]-1,2,3,6-tetrahydropyridin-4-yl]-3-(pyridin-4-yl)-1H-pyrrole.

In addition, other objectives of the present invention are to provide a pharmaceutical containing a compound

or pharmacologically acceptable salt thereof, selected from the compounds described in one selected from (1) to (36) as an active ingredient; preferably a pharmaceutical for inhibiting production of inflammatory cytokines; a pharmaceutical for use as a prophylactic or therapeutic agent against diseases mediated by inflammatory cytokines, for example, antipyretic, analgesic and anti-inflammatory drugs that inhibit production of inflammatory cytokines; a prophylactic or therapeutic agent against rheumatoid arthritis, osteoarthritis, septicemic disease, psoriasis, Crohn's disease, chronic ulcerative colitis, diabetes mellitus (particularly type I diabetes mellitus), or hepatitis,

and

(i) methods of inhibiting production of inflammatory cytokines, (ii) prophylactic or therapeutic methods for said diseases associated with inflammatory cytokines [preferably prophylactic or therapeutic methods of relieving fever, pain and/or inflammation; prophylactic or therapeutic methods for rheumatoid arthritis, osteoarthritis, septicemic disease, psoriasis, Crohn's disease, chronic ulcerative colitis, diabetes mellitus (particularly type I diabetes mellitus), or hepatitis, by administration of compounds or pharmacologically acceptable salts thereof as described in one selected from (1) to (36) to warm-blooded animals (preferably humans) at pharmacologically effective doses.

Compounds having the general formula (I) described above include compounds having any one of the following general formulae:

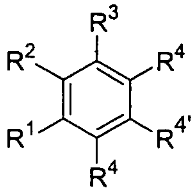
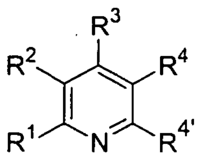
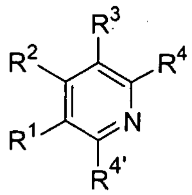
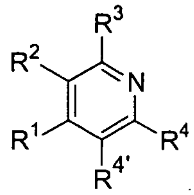

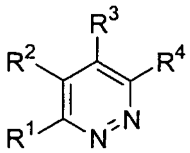
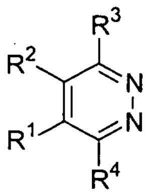
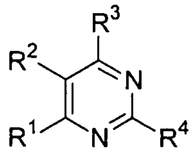
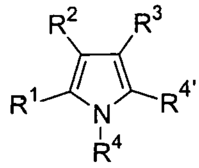

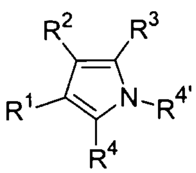
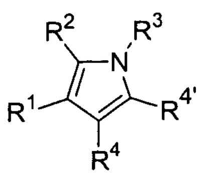
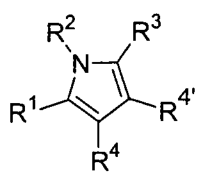
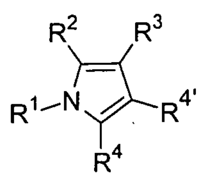

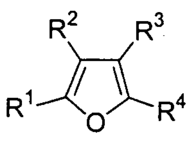
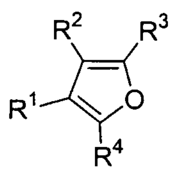
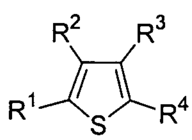
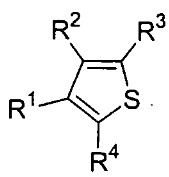

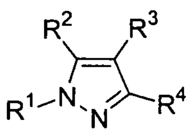
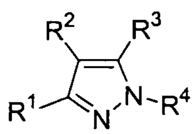
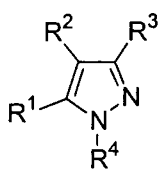
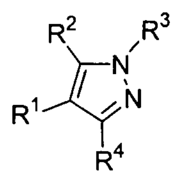

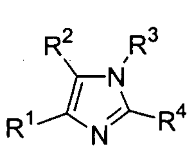
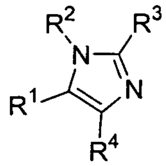
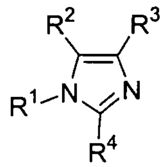
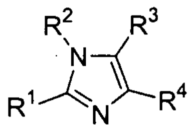

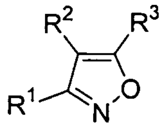
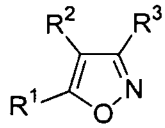
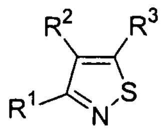
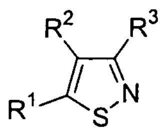

Of the compounds having the general formulae shown above, preferable compounds have any one of the general formulae shown below:

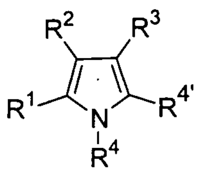
,
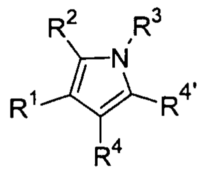
,
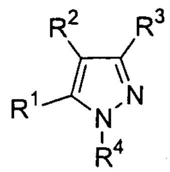
or
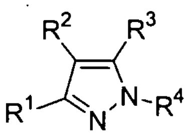

Of the compounds having anyone of the general formulae shown above, more preferable compounds have any one of the general formulae shown below:

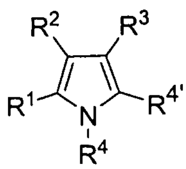
or
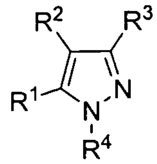

(wherein, $R^1$, $R^2$, $R^3$, $R^4$and $R^{4'}$ have the same meanings as those indicated hereinbefore) .

$R^3$ shown in general formula (I) shown hereinbefore represents a group having general formula (IIa) or (IIb) shown below:

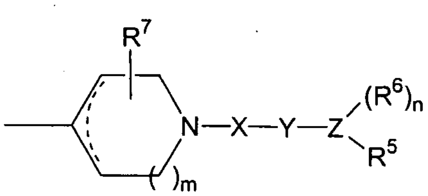
(IIa)

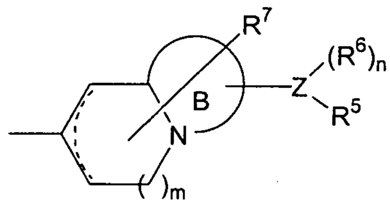
(IIb)

(wherein, B, X, Y, Z, $R^5$, $R^6$, $R^7$, m and n have the same meanings as those indicated hereinbefore).

Of the groups having the general formulae shown above, the preferable groups are groups wherein m represents

an integer of 1.

The group having general formula (IIa) shown above is preferably a group wherein X is an alkylene group having from 1 to 4 carbon atoms; and Y is a group having formula: $C(R^8a)(R^8b)$ ($R^8a$ and $R^8b$ are the same or different and each represents independently a hydrogen atom, a hydroxyl group, a halogen atom, an alkyl group having from 1 to 4 carbon atoms or an alkoxy group having from 1 to 4 carbon atoms, or $R^8a$ and $R^8b$ together form an oxo group or a methylene group, or $R^8a$ and $R^8b$ together with the carbon atom to which they are bonded form a 3- to 6-membered cycloalkyl group) ; more preferably a group wherein X is a methylene group, and Y is a group having formula: $C(R^8a)(R^8b)$ ($R^8a$ and $R^8b$ are the same or different and each represents independently a hydrogen atom, a hydroxyl group, a fluorine atom, a methyl group, an ethyl group, a methoxy group or an ethoxy group, or $R^8a$ and $R^8b$ together form an oxo group or a methylene group, or $R^8a$ and $R^8b$ together with the carbon atom to which they are bonded form a cyclopropyl group) ; still more preferably a group wherein X is a methylene group, and Y is a group having formula: $C(R^8a)(R^8b)$ ($R^8a$ and $R^8b$ are the same or different and each represents independently a hydrogen atom, a fluorine atom, a methyl group, a hydroxyl group or an oxo group); particularly preferably a group wherein X is a methylene group, and Y is a group having formula: $C(R^8a)(R^8b)$ ($R^8a$ and $R^8b$ are the same or different and each represents independently a hydrogen atom, a fluorine atom or a hydroxyl group) ; and most preferably a group wherein X is a methylne group, and Y is a group having formula: $CH_2$, that is, a methylene group.

The group having general formula (IIb) shown above is preferably a group wherein Ring B is a 5- or 6-membered heterocyclyl ring which contains one nitrogen atom and further may optionally contain one atom or group selected from the group consisting of a nitrogen atom, an oxygen atom, a sulfur atom, a group having formula: =SO, and a group having formula: $=SO_2$ (said ring is a saturated or an unsaturated ring which may optionally be fused with an aryl group, a heteroaryl group, a cycloalkyl group or a heterocyclyl group); more preferably a group wherein Ring B is a 5- or 6-membered heterocyclyl ring containing one nitrogen atom (said ring is a saturated or an unsaturated ring which may optionally be fused with an aryl group, a heteroaryl group, a cycloalkyl group or a heterocyclyl group) ; and still more preferably a group wherein Ring B is a pyrrolidine ring or a pyrroline ring.

In the general formula (I) shown hereinbefore, the "aryl group" in the definition of $R^a$, $R^b$ and Ring B; the "aryl group" of the "aryl group which may optionally be substituted with group(s) selected from the group consisting of Substituent group $\alpha$ and Substituent group $\beta$ " in the definition of $R^1$ and the "aryl group" of the "aryl group which may optionally be substituted with group(s) selected from the group consisting of Substituent group $\alpha$, Substituent group $\beta$ and Substituent $\gamma$ in the definition of "Substituent group $\delta$" is an aryl group having from 6 to 14 carbon atoms such as a phenyl, naphthyl, phenanthryl or anthracenyl group; preferably a phenyl group or a naphthyl group; and most preferably a phenyl group.

Furthermore, the "aryl group" shown above may optionally be fused with a cycloalkyl group having from 3 to 10 carbon atoms, and said group is, for example, a 5-indanyl group or the like.

The "arylene group" in the definition of Z is, for example, an arylene group having from 6 to 14 carbon atoms such as a phenylene, naphthalenediyl, phenanthrenediyl or anthracenediyl group, preferably a phenylene or naphthalenediyl group, and most preferably a phenylene group.

The "aryl group which may optionally be substituted with group(s) selected from the group consisting of Substituent group $\alpha$ and Substituent group $\beta$ "in the definition of $R^1$ is preferably an aryl group which may optionally be substituted with from 1 to 4 groups selected from the group consisting of Substituent group $\alpha$ and Substituent group $\beta$, more preferably an aryl group which may optionally be substituted with from 1 to 3 groups selected from the group consisting of Substituent group $\alpha$ and Substituent group $\beta$, and still more preferably a phenyl group which may optionally be substituted with from 1 to 3 groups selected from the group consisting of Substituent group $\alpha$ and Substituent group $\beta$.

A preferable example is a phenyl, 3-cyanophenyl, 4-fluorophenyl, 3-fluorophenyl, 4-chlorophenyl, 3-chlorophenyl, 2,4-difluorophenyl, 3,4-difluorophenyl, 3,4-dichlorophenyl, 3,4,5-trifluorophenyl, 3-chloro-4-fluorophenyl, 3-methoxyphenyl, 3-difluoromethoxyphenyl, 3-trifluoromethoxyphenyl, 3-trifluoromethylphenyl or 4-fluoro-3-methoxyphenyl group, a more preferable example is a phenyl, 3-cyanophenyl, 4-fluorophenyl, 3-fluorophenyl, 3-chlorophenyl, 3,4-difluorophenyl, 3,4,5-trifluorophenyl, 3-chloro-4-fluorophenyl, 3-methoxyphenyl, 3-difluoromethoxyphenyl, 3-trifluoromethylphenyl, 3-cyanophenyl or 4-fluoro-3-methoxyphenyl group, and a still more preferable example is a phenyl, 4-fluorophenyl, 3-fluorophenyl, 3-chlorophenyl, 3,4-difluorophenyl, 3-chloro-4-fluorophenyl or 3-trifluoromethylphenyl group.

The "aryl group which may optionally be substituted with group(s) selected from the group consisting of Substituent group $\alpha$, Substituent group $\beta$ and Substituent group $\gamma$" in the definition of "Substituent group $\delta$" is preferably an aryl group which may optionally be substituted with from 1 to 4 groups selected from the group consisting of Substituent group $\alpha$, Substituent group $\beta$, and Substituent group $\gamma$, more preferably an aryl group which may optionally be substituted with from 1 to 3 groups selected from the group consisting of Substituent group $\alpha$, Substituent group $\beta$, and Substituent group $\gamma$, and still more preferably an aryl group which may optionally be substituted with one group selected from the group consisting of a "lower alkylthio group", a "halogeno lower alkylthio group", a "lower alkylsulfinyl group" and a "lower alkylsulfonyl group". A preferable example of such is a phenyl, 4-methylthiophenyl, 4-ethylthiophenyl, 4-propylthiophenyl, 4-methylsulfinylphenyl, 4-ethylsulfinylphenyl, 4-propylsulfinylphenyl, 4-methanesulfonylphenyl, 4-ethanesulfonylphenyl

or 4-propanesulfonylphenyl group.

The "heteroaryl group" in the definition of $R^a$, $R^b$ and Ring B; the "heteroaryl group" of the "heteroaryl group which may optionally be substituted with group(s) selected from the group consisting of Substituent group α and Substituent group β" in the definition of $R^1$; and the "heteroaryl group" of the "heteroaryl group which may optionally be substituted with group(s) selected from the group consisting of Substituent group α, Substituent group β and Substituent group γ" in the definition of "Substituent group δ" is, for example, a 5- to 7-membered heteroaryl group containing from 1 to 4 heteroatoms selected from the group consisting of a sulfur atom, an oxygen atom and a nitrogen atom such as a furyl, thienyl, pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl or pyrazinyl group, preferably a 5- or 6-membered heteroaryl group containing 1 or 2 heteroatoms selected from the group consisting of a sulfur atom, an oxygen atom and a nitrogen atom such as a furyl, thienyl, pyrrolyl, pyrazolyl, imidazolyl, oxazolyl, isoxazolyl, thiazolyl, isothiazolyl, pyridyl, pyridazinyl, pyrimidinyl or pyrazinyl group, and more preferably a furyl, thienyl, pyridyl, or pyrimidinyl group.

Further, the "heteroaryl group" shown above may optionally be fused with another cyclic group (for example, a cyclic ring such as an aryl group or a cycloalkyl group having from 3 to 10 carbon atoms), and such group is, for example, an indolyl, benzofuranyl, benzothienyl, quinolyl, isoquinolyl, quinazolinyl, tetrahydroquinolyl, or tetrahydroisoquinolyl group.

The "heteroarylene group" in the definition of Z is, for example, a 5- to 7-membered hetreoarylene group containing from 1 to 4 heteroatoms selected from the group consisting of a sulfur atom, an oxygen atom and a nitrogen atom such as a thiophenediyl, furandiyl, pyrrolediyl, pyrazolediyl, imidazolediyl, oxazolediyl, isoxazolediyl, thiazolediyl, isothiazolediyl, triazolediyl, tetrazolediyl, thiadiazolediyl, pyridinediyl, pyridazinediyl, pyrimidinediyl, or pyrazinediyl group, preferably a 5- to 6-membered heteroarylene group containing from 1 to 3 heteroatoms selected from the group consisting of a sulfur atom, an oxygen atom and a nitrogen atom such as a thiophenediyl, furandiyl, pyridinediyl, pyrimidinediyl, thiazolediyl or thiadiazolediyl- group, more preferably a thiophenediyl or pyridinediyl group, and particularly preferably a thiophenediyl group.

The "heteroaryl group which may optionally be substituted with group(s) selected from the group consisting of Substituent group α and Substituent group β "in the definition of $R^1$ is preferably a heteroaryl group which may optionally be substituted with from 1 to 3 groups selected from the group consisting of Substituent group α and Substituent group β, and more preferably a heteroaryl group which is substituted with from 1 to 2 groups selected from the group consisting of Substituent group α and Substituent group β. Such preferable group is, for example, a furyl, thienyl, pyridyl, pyrimidinyl, 5-fluoro-2-furyl, 4-chloro-2-thienyl, 5-difluoromethoxy-3-furyl, 5-trifluoromethyl-3-thienyl or 5-fluoro-2-oxazolyl group.

The "heteroaryl group which may optionally be substituted with group(s) selected from the group consisting of Substituent group α, Substituent group β and Substituent group γ" in the definition of "Substituent group δ" is preferably a heteroaryl group which may optionally be substituted with from 1 to 3 groups selected from the group consisting of Substituent group α, Substituent group β and Substituent group γ, more preferably a heteroaryl group which may optionally be substituted with from 1 to 2 groups selected from the group consisting of Substituent group α, Substituent group β and Substituent group γ. Such preferable group is, for example, the "heteroaryl group" described above, or a 2-methylthio-5-pyridyl, 3-methylthio-6-pyridazinyl, 2-methylthio-5-pyrimidinyl, 2-methylsulfinyl-5-pyridyl, 3-methylsulfinyl-6-pyridazinyl, 2-methylsulfinyl-5-pyrimidinyl, 2-methanesulfonyl-5-pyridyl, 3-methanesulfonyl-6-pyridazinyl or 2-methanesulfonyl-5-pyrimidinyl group.

The "heteroaryl group which contains at least one nitrogen atom" of the "heteroaryl group which contains at least one nitrogen atom which may optionally be substituted with group(s) selected from the group consisting of Substituent group α and Substituent group β" in the definition of $R^2$ is a 5- to 7-membered heteroaryl group which contains at least one nitrogen atom and further may optionally contain from 1 to 3 heteroatoms selected from the group consisting of a sulfur atom, an oxygen atom and a nitrogen atom such as a pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl or pyrazinyl group, preferably a 5- to 6-membered heteroaryl group which contains at least one nitrogen atom and further may optionally contain one heteroatom selected from the group consisting of a sulfur atom, an oxygen atom and a nitrogen atom such as a pyrrolyl, pyrazolyl, imidazolyl, oxazolyl, isoxazolyl, thiazolyl, isothiazolyl, pyridyl, pyridazinyl, pyrimidinyl or pyrazinyl group, more preferably a 5- to 6-membered heteroaryl group containing from 1 to 2 nitrogen atoms such as an imidazolyl, pyridyl, pyridazinyl, pyrimidinyl or pyrazinyl group, particularly preferably a pyridyl or pyrimidinyl group, and most preferably a 4-pyridyl or 4-pyrimidinyl group.

The "heteroaryl group which contains at least one nitrogen atom and further may optionally be substituted with group(s) selected from the group consisting of Substituent group α and Substituent group β" in the definition of $R^2$ is preferably a group which may optionally be substituted with from 1 to 3 groups selected from the group consisting of Substituent group α and Substituent group β, more preferably a group which may optionally be substituted with from 1 to 2 groups selected from the group consisting of Substituent group α and Substituent group β, still more preferably a group which may optionally be substituted with one group selected from the group consisting of Substituent group α and Substituent group β, particularly preferably a 4-pyridyl or 4-pyrimidinyl group, of which the 2-position may optionally be

substituted with one group selected from the group consisting of Substituent group α and Substituent group β, and most preferably a 4-pyridyl or 4-pyrimidinyl group, of which the 2-position may optionally be substituted with one group selected from the group consisting of a group having formula: $-NR^dR^e$ (wherein, $R^d$ and $R^e$ are the same or different and each represents independently a hydrogen atom, a lower alkyl group, a lower alkenyl group, a lower alkynyl group, an aralkyl group or a lower alkylsulfonyl group, or $R^d$ and $R^e$ together with the nitrogen atom to which they are bonded form a heterocyclyl group) and a lower alkyl group which may optionally be substituted with a group having formula: $-NR^dR^e$ (wherein, $R^d$ and $R^e$ have the same meanings as those described above). Such preferable group is, for example, a 4-pyridyl, 4-pyrimidinyl, 2-amino-4-pyridyl, 2-amino-4-pyrimidinyl, 2-methylamino-4-pyridyl, 2-methylamino-4-pyrimidinyl, 2-methoxy-4-pyridyl, 2-methoxy-4-pyrimidinyl, 2-benzylamino-4-pyridyl, 2-benzylamino-4-pyrimidinyl, 2-(α-methylbenzylamino)-4-pyridyl or 2-(α-methylbenzylamino)-4-pyrimidinyl group.

The "lower alkyl group substituted with a heteroaryl group" in the definition of $R^a$ and $R^b$ is a group wherein the "heteroaryl group" shown above is substituted to a lower alkyl group, preferably a group wherein the "heteroaryl group" shown above is substituted to an alkyl group having from 1 to 4 carbon atoms, and more preferably a group wherein the "heteroaryl group" shown above is substituted to an alkyl group having from 1 to 2 carbon atoms. A preferable example of such is a (2 or 3-furyl)methyl, 2-(2 or 3-furyl)ethyl, (2 or 3-thienyl)methyl, 2-(2 or 3-thienyl)ethyl, (2, 3 or 4-pyridyl)methyl, 2-(2, 3 or 4-pyridyl)ethyl, (2, 4 or 5-pyrimidinyl)methyl, or 2-(2, 4 or 5-pyrimidinyl)ethyl group.

The "cycloalkyl group" in the definition of $R^a$, $R^b$, Ring B and "Substituent group β", the "cycloalkyl group" in the definition of $R^8$a and $R^8$b, and the "cycloalkyl group" of the "cycloalkyl group substituted with group(s) selected from the group consisting of Substituent group α, Substituent group β and Substituent group γ" in the definition of "Substituent group δ" is a cycloalkyl group having from 3 to 7 carbon atoms such as a cyclopropyl, cyclobutyl, cyclopentyl, cyclohexyl or cycloheptyl group, preferably a cycloalkyl group having from 3 to 6 carbon atoms, and more preferably a cyclopropyl, cyclopentyl or cyclohexyl group.

The "lower alkyl group substituted with a cycloalkyl group" in the definition of $R^a$ and $R^b$ is a group wherein the "cycloalkyl group" shown above is substituted to a lower alkyl group, preferably a group wherein the "cycloalkyl group" shown above is substituted to an alkyl group having from 1 to 4 carbon atoms, and more preferably a group wherein the "cycloalkyl group" shown above is substituted to an alkyl group having from 1 to 2 carbon atoms. A preferable example of such is a cyclopropylmethyl, 2-cyclopropylethyl, cyclopentylmethyl, 2-cyclopentylethyl, cyclohexylmethyl or 2-cyclohexylethyl group.

The "heterocyclyl group" of the "heterocyclyl group which may optionally be substituted with group(s) selected from the group consisting of Substituent group α, Substituent group β and Substituent group γ" in the definition of "Substituent group δ" is a 4- to 7-membered heterocyclyl group containing from 1 to 3 heteroatoms selected from the group consisting of a sulfur atom, an oxygen atom and a nitrogen atom, preferably a 4- to 7-membered heterocyclyl group containing 1 or 2 heteroatoms selected from the group consisting of a sulfur atom, an oxygen atom and a nitrogen atom, and more preferably a 5- or 6-membered heterocyclyl group which contains one nitrogen atom and further may optionally contain one heteroatom selected from the group consisting of an oxygen atom, a sulfur atom and a nitrogen atom. Such group is, for example, a pyrrolidinyl, pyrrolinyl, imidazolidinyl, imidazolinyl, pyrazolidinyl, pyrazolinyl, oxazolidinyl, thiazolidinyl, piperidyl, tetrahydropyridyl, dihydropyridyl, piperazinyl, morpholinyl, thiomorpholinyl or homopiperidyl group.

The heterocyclyl group formed from $R^d$ and $R^e$ together with the nitrogen atom to which they are bonded of the group having formula: $-NR^dR^e$ included in "Substituent group α" can be a 4- to 7-membered heterocyclyl group which contains one nitrogen atom and further may optionally contain one heteroatom selected from the group consisting of an oxygen atom, a sulfur atom and a nitrogen atom, and is, for example, a 1-azetidinyl, 1-pyrrolidinyl, 1-pyrrolinyl, 1-imidazolidinyl, 1-imidazolinyl, 1-pyrazolidinyl, 1-pyrazolinyl, 3-oxazolidinyl, 3-thiazolidinyl, 1-piperidyl, tetrahydropyridin-1-yl, dihydropyridin-1-yl, 1-piperazinyl, 4-morpholinyl, 4-thiomorpholinyl, 1-homopiperidyl, 8-azabicyclo[3,2,1]octan-8-yl, 8-azabicyclo[3,2,1]octen-8-yl, 9-azabicyclo[3,3,1]nonan-9-yl or 9-azabicyclo[3,3,1]nonen-9-yl group.

Furthermore, these groups may optionally be fused with an aryl group or a heteroaryl group, and such group is, for example, a tetrahydroquinolin-1-yl or tetrahydroisoquinolin-2-yl group.

The "4- to 7-membered heterocyclyl ring " in the definition of Ring B means a 4- to 7-membered heterocyclyl ring consisting of from 2 to 5 atoms or groups selected from the group consisting of a carbon atom, a nitrogen atom, an oxygen atom, a sulfur atom, a group having formula: =SO and a group having formula: $=SO_2$, and can be a 4- to 7-membered heterocyclyl ring containing at least one nitrogen atom (that is, a saturated heterocyclyl ring or an unsaturated heterocyclyl ring), and is preferably a 5- or 6-membered heterocyclyl ring which contains one nitrogen atom and further may optionally contain one atom or group selected from the group consisting of a nitrogen atom, an oxygen atom, a sulfur atom, a group having formula: =SO and a group having formula: $=SO_2$, more preferably a pyrrolidine, pyrroline, imidazolidine, imidazoline, pyrazolidine, pyrazoline, oxazolidine, thiazolidine, piperidine, tetrahydropyridine, dihydropyridine, piperazine, morpholine or thiomorpholine ring, still more preferably a pyrrolidine, pyrroline or imidazolidine ring, and particularly preferably a pyrrolidine ring or a pyrroline ring.

Further, the "heterocyclyl ring" shown above may optionally be fused with the "aryl group" described above,

the "heteroaryl group" described above, the "cycloalkyl group" described above or the "heterocyclyl group" described above, and such ring is, for example, a tetrahydroquinoline, octahydroquinoline, decahydroquinoline, tetrahydroisoquinoline, octahydroisoquinoline, decahydroisoquinoline, indoline, octahydroindole, isoindoline or octahydroisoindole ring.

The "halogen atom" in the definition of $R^6$, $R^7$, $R^8a$, $R^8b$ and "Substituent group α" can be a fluorine atom, a chlorine atom, a bromine atom or an iodine atom, and is preferably a fluorine atom or a chlorine atom.

The "lower alkyl group" in the definition of $R^6$, $R^7$, $R^8a$, $R^8b$, $R^c$, $R^d$ and $R^e$; and the "lower alkyl group" of the "lower alkyl group which may optionally be substituted with group(s) selected from Substituent group α" in the definition of $R^a$, $R^b$ and "Substituent group β" can be a straight or branched alkyl group having from 1 to 6 carbon atoms such as a methyl , ethyl, propyl, isopropyl, butyl, isobutyl, s-butyl, tert-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group, and is preferably an alkyl group having from 1 to 4 carbon atoms, more preferably a methyl, ethyl, propyl, isopropyl or butyl group, and particularly preferably a methyl, ethyl or propyl group.

The "lower alkyl group which may optionally be substituted with group(s) selected from Substituent group α" in the definition of $R^a$, $R^b$ and "Substituent group β" represents a group wherein one or more hydrogen atoms of the "lower alkyl group" described above may optionally be substituted with group(s) selected from "Substituent group α", and is preferably a lower alkyl group which may optionally be substituted with from 1 to 3 groups selected from "Substituent group α", more preferably a lower alkyl group which may optionally be substituted with from 1 to 2 groups selected from "Substituent group α", still more preferably an alkyl group having from 1 to 4 carbon atoms which may optionally be substituted with from 1 to 2 groups selected from "Substituent group α", and particularly preferably a methyl, ethyl, propyl or isopropyl group, of which the end of chain carbon may optionally be substituted with from 1 to 2 groups selected from the "Substituent group α". Such preferable group is, for example, the "lower alkyl group" described above, a hydroxymethyl, 2-hydroxyethyl, 3-hydroxypropyl, 1,3-dihydroxyisopropyl, cyanomethyl, 2-cyyanoethyl, fluoromethyl, 2-fluoroethyl, 3-fluoropropyl, chloromethyl, 2-chloroethyl, 3-chloropropyl, metoxymethyl, 2-methoxyethyl, 3-methoxypropyl, ethoxymethyl, 2-ethoxyethyl, 3-ethoxypropyl, fluoromethoxymethyl, difluoromethoxymethyl, trifluoromethoxymethyl, 2-fluoromethoxyethyl, 2-difluoromethoxyethyl, 2-trifluoromethoxyethyl, carboxymethyl, 2-carboxyethyl, 3-carboxypropyl, methoxycarbonylmethyl, 2-(methoxycarbonyl)ethyl, ethoxycarbonylmethyl, 2-(ethoxycarbonyl)ethyl, carbamoylmethyl, 2-carbamoylethyl, 3-carbamoylpropyl, acetoxymethyl, methylthiomethyl, 2-methylthioethyl, 3-methylthiopropyl, fluoromethylthiomethyl, difluoromethylthiomethyl, trifluoromethylthiomethyl, 2-fluoromethylthioethyl, 2-difluoromethylthioethyl, 2-trifluoromethylthioethyl, aminomethyl, methylaminomethyl, dimethylaminomethyl, 2-aminoethyl, 2-methylaminoethyl, 2-dimethylaminoethyl, 3-aminopropyl, 3-methylaminopropyl or 3-dimethylaminopropyl group, and more preferable group is, for example, a methyl, ethyl, propyl, isopropyl, 2-hydroxyethyl, 2-fluoroethyl, 2-methoxyethyl or 2-dimethylaminoethyl group.

The "lower alkenyl group" in the definition of $R^d$ and $R^e$; and the "lower alkenyl group" of the "lower alkenyl group which may optionally be substituted with group(s) selected from Substituent group α" in the definition of $R^a$, $R^b$ and "Substituent group β" can be a straight or branched alkenyl group having from 2 to 6 carbon atoms such as a vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl or 5-hexenyl group, and is preferably an alkenyl group having from 2 to 4 carbon atoms, and more preferably an alkenyl group having 3 or 4 carbon atoms.

The "lower alkynyl group" in the definition of $R^d$ and $R^e$; and the "lower alkynyl group" of the "lower alkynyl group which may optionally be substituted with group(s) selected from Substituent group α" in the definition of $R^a$, $R^b$ and "Substituent group β" can be a straight or branched alkynyl group having from 2 to 6 carbon atoms such as a ethynyl, 2-propynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl or 5-hexynyl group, and is preferably an alkynyl group having from 2 to 4 carbon atoms, and more preferably an alkynyl group having 3 or 4 carbon atoms.

The "lower alkylene group" in the definition of "Substituent group γ" is a straight or branched alkylene group having from 1 to 6 carbon atoms such as a methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene or hexamethylene group, preferably a straight or branched alkylene group having from 1 to 4 carbon atoms, and more preferably a methylene, ethylene, trimethylene, propylene or tetramethylene group.

The "straight or branched alkylene group having from 1 to 5 carbon atoms" in the definition of X is preferably a straight or branched alkylene group having from 1 to 4 carbon atoms, and more preferably a methlene group.

The "aralkyl group" in the definition of $R^a$, $R^b$, $R^d$ $R^e$ and "Substituent group β" can be a group wherein the "aryl group" described above is bonded to the "lower alkyl group" described above, and such group is, for example, a benzyl, α-naphthylmethyl, β-naphthylmethyl, 1-phenethyl, 2-phenethyl, 2-(α-naphthyl)ethyl, 2-(β-naphthyl)ethyl, 3-phenylpropyl,

4-phenylbutyl, 5-phenylpentyl or 6-phenylhexyl group, and preferably a benzyl group.

Further, the aryl moiety of said "aralkyl group" may optionally be substituted with from 1 to 3 groups selected from the group consisting of "Substituent group α" and "Substituent group β" which are described above, and such substituted aralkyl group is preferably an aralkyl group substituted with a halogen atom, a lower alkyl group or a lower alkoxy group such as a 2-fluorobenzyl, 3-fluorobenzyl, 4-fluorobenzyl, 2-chlorobenzyl, 3-chlorobenzyl, 4-chlorobenzyl, 2-methylbenzyl, 3-methylbenzyl, 4-methylbenzyl, 2-methoxybenzyl, 3-methoxybenzyl or 4-methoxybenzyl group.

The "aralkyl group" is preferably an unsubstituted aralkyl group or an aralkyl group substituted with a halogen atom, a lower alkyl group or a lower alkoxy group, more preferably an unsubstituted aralkyl group or an aralkyl group substituted with a halogen atom or a lower alkyl group, and most preferably an unsubstituted aralkyl group.

The "hydroxy lower alkyl group" in the definition of $R^c$ is a group wherein one or more hydrogen atoms of the "lower alkyl group" described above are substituted with a hydroxyl group, and preferably a hydroxyalkyl group having from 1 to 4 carbon atoms, which is, for example, a hydroxymethyl, 2-hydroxyethyl or 3-hydroxypropyl group.

The "halogeno lower alkyl group" in the definition of $R^6$, $R^7$ and $R^e$ is a group wherein one or more hydrogen atoms of the "lower alkyl group" described above are substituted with a "halogen atom" described above, preferably a halogeno lower alkyl group having from 1 to 4 carbon atoms, more preferably a trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl, and 2,2-dibromoethyl group, still more preferably a trifluoromethyl, trichloromethyl, difluoromethyl and fluoromethyl group, and most preferably a trifluoromethyl group.

The "lower alkoxy group" in the definition of $R^6$, $R^7$, $R^8$a, $R^8$b, $R^a$, $R^b$ and "Substituent group α" is a group wherein the "lower alkyl group" described above is bonded to an oxygen atom, preferably a straight or branched alkoxy group having from 1 to 4 carbon atoms, and more preferably a methoxy, ethoxy, propoxy or isopropoxy group.

The "halogeno lower alkoxy group" in the definition of $R^6$, $R^7$ and "Substituent group α" is a group wherein one or more hydrogen atoms of the "lower alkoxy group" described above are substituted with a "halogen atom" described above, preferably a halogeno lower alkoxy group having from 1 to 4 carbon atoms, more preferably a difluoromethoxy, trifluoromethoxy or 2,2,2-trifluoroethoxy group, and particularly preferably a difluoromethoxy group.

The "lower alkoxy lower alkyl group" in the definition of $R^c$ is a group wherein one or more hydrogen atoms of the "lower alkyl group" described above are substituted with a "lower alkoxy group" described above, preferably an alkoxyalkyl group having from 1 to 4 carbon atoms, and such group is, for example, a methoxymethyl, 1,1-dimethyl-1-methoxymethyl, ethoxymethyl, propoxymethyl, isopropoxymethyl, butoxymethyl or t-butoxymethyl group.

The "lower alkylthio group" in the definition of $R^6$, $R^7$ and "Substituent group α" is a group wherein the "lower alkyl group" described above is bonded to a sulfur atom, preferably a straight or branched alkylthio group having from 1 to 4 carbon atoms, more preferably a methylthio, ethylthio, propylthio, isopropylthio or butylthio group, and particularly preferably a methylthio, ethylthio or propylthio group.

The "halogeno lower alkylthio group" in the definition of $R^6$, $R^7$ and "Substituent group α" is a group wherein one or more hydrogen atoms of the "lower alkylthio group" described above are substituted with a "halogen atom" described above, preferably a halogeno lower alkylthio group having from 1 to 4 carbon atoms, and more preferably a difluoromethylthio, trifluoromethylthio or 2,2,2-trifluoroethylthio group.

The "lower alkenyloxy group" in the definition of $R^a$ and $R^b$ is a group wherein the "lower alkenyl group" described above is bonded to an oxygen atom, preferably a group wherein an alkenyl group having from 2 to 4 carbon atoms is bonded to an oxygen atom, more preferably a group wherein an alkenyl group having from 3 to 4 carbon atoms is bonded to an oxygen atom, which is, for example, a 2-propenyloxy, 2-butenyloxy or 3-butenyloxy group.

The "lower alkynyloxy group" in the definition of $R^a$ and $R^b$ is a group wherein the "lower alkynyl group" described above is bonded to an oxygen atom, preferably a group wherein an alkynyl group having from 2 to 4 carbon atoms is bonded to an oxygen atom, more preferably a group wherein an alkynyl group having from 3 to 4 carbon atoms is bonded to an oxygen atom, which is, for example, a 2-propynyloxy, 2-butynyloxy or 3-butynyloxy group.

The "aralkyloxy group" in the definition of $R^a$ and $R^b$ is a group wherein the "aralkyl group" described above is bonded to an oxygen atom, and said group is, for example, a benzyloxy, α-naphthylmethoxy, β-naphthylmethoxy, 2-phenethyloxy, 3-phenylpropoxy or 4-phenylbutoxy group.

The "lower alkylenedioxy group" in the definition of "Substituent group γ" is an alkylenedioxy group of which the alkylene moiety is a straight or branched alkylene group having from 1 to 6 carbon atoms such as a methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene or hexamethylene group, preferably a straight or branched alkylenedioxy group having from 1 to 4 carbon atoms, and more preferably a methylenedioxy, ethylenedioxy, trimethylenedioxy, propylenedioxy or tetramethylenedioxy group.

The "lower alkoxyimino group" in the definition of "Substituent group γ" is a group wherein the hydrogen atom of a hydroxyimino group is substituted with the "lower alkyl group" described above, preferably an alkoxyimino group having from 1 to 4 carbon atoms, and more preferably a methoxyimino, ethoxyimino or propoxyimino group.

The "mono- or di-lower alkylamino group" in the definition of $R^a$ and $R^b$ is a group wherein one or two hydrogen

atoms of an amino group are substituted with a lower alkyl group. The alkyl moiety of said alkylamino group is preferably an alkyl group having from 1 to 4 carbon atoms, and more preferably an alkyl group having from 1 to 2 carbon atoms. Still more preferably, such alkylamino group is a methylamino, dimethylamino, ethylamino or diethylamino group.

The "lower alkylsulfonyl group" in the definition of $R^d$, $R^e$ and "Substituent group γ" is a group wherein the "lower alkyl" group described above is bonded to a sulfonyl ($-SO_2-$) group, preferably a straight or branched alkylsulfonyl group having from 1 to 4 carbon atoms, more preferably a methanesulfonyl, ethanesulfonyl, propanesulfonyl, isopropanesulfonyl or butanesulfonyl group, and particularly preferably a methanesulfonyl, ethanesulfonyl or propanesulfonyl group.

The "lower alkylsulfinyl group" in the definition of "Substituent group γ" is a group wherein the "lower alkyl" group described above is bonded to a sulfinyl (-SO-) group, preferably a straight or branched alkylsulfinyl group having from 1 to 4 carbon atoms, more preferably a methylsulfinyl, ethylsulfinyl, propylsulfinyl, isopropylsulfinyl or butylsulfinyl group, and particularly preferably a methylsulfinyl, ethylsulfinyl or propylsulfinyl group.

The "group having formula: $CONR^aR^b$" in the definition of $R^5$ is, for example, a carbamoyl group substituted with a lower alkyl group which may optionally be substituted with group(s) selected from Substituent group α such as a carbamoyl, N-hydroxycarbamoyl, N-methylcarbamoyl, N-ethylcarbamoyl, N-propylcarbamoyl, N-isopropylcarbamoyl, N-butylcarbamoyl, N-(s-butyl)carbamoyl, N-(t-butyl)carbamoyl, N-isobutylcarbamoyl, N-pentylcarbamoyl, N-hexylcarbamoyl, N,N-dimethylcabamoyl, N-ethyl-N-methylcarbamoyl, N,N-diethylcarbamoyl, N-(hydroxymethyl)carbamoyl, N-(2-hydroxyethyl)carbamoyl, N-(3-hydroxypropyl)carbamoyl, N-(1,3-dihydroxyisopropyl)carbamoyl, N-(2-nitroethyl) carbamoyl, N-(cyanomethyl)carbamoyl, N-(2-cyanoethyl)carbamoyl, N-(2-fluoroethyl)carbamoyl, N-(2,2,2-trifluoroethyl) carbamoyl, N-(3-fluoropropyl)carbamoyl, N-(4-fluorobutyl)carbamoyl, N-(5-fluoropentyl)carbamoyl, N-(6-fluorohexyl) carbamoyl, N-(2-chloroethyl)carbamoyl, N-(3-chloropropyl)carbamoyl, N-(4-chlorobutyl)carbamoyl, N-(2-bromoethyl) carbamoyl, N-(3-bromopropyl)carbamoyl, N-(4-bromobutyl)carbamoyl, N-(methoxymethyl)carbamoyl, N-(2-methoxyethyl)carbamoyl, N-(3-methoxypropyl)carbamoyl, N-(4-methoxybutyl)carbamoyl, N-(ethoxymethyl)carbamoyl, N-(2-ethoxyethyl)carbamoyl, N-(3-ethoxypropyl)carbamoyl, N-(4-ethoxybutyl)carbamoyl, N-(carboxymethyl)carbamoyl, N-(2-carboxyethyl)carbamoyl, N-(3-carboxypropyl)carbamoyl, N-(methoxycarbonylmethyl)carbamoyl, N-(2-methoxycarbonylethyl)carbamoyl, N-(3-methoxycarbonylpropyl)carbamoyl, N-(ethoxycarbonylmethyl)carbamoyl, N-(1-ethoxycarbonylethyl)carbamoyl, N-(2-ethoxycarbonylethyl)carbamoyl, N-(3-ethoxycarbonylpropyl)carbamoyl, N-(carbamoylmethyl)carbamoyl, N-(2-acetoxyethyl)carbamoyl, N-(2-methylthioethyl)carbamoyl, N-(2-ethylthioethyl)carbamoyl, N-(2-propylthioethyl)carbamoyl, N-(2-fluoromethylthioethyl)carbamoyl, N-(2-difluoromethylthioethyl)carbamoyl, N-(2-trifluoromethylthioethyl)carbamoyl, N-[2-(2-fluoroethylthio)ethyl]carbamoyl, N-[2-(2,2,2-trifluoroethylthio)ethyl]carbamoyl, N-(2-aminoethyl)carbamoyl, N-(2-methylaminoethyl)carbamoyl or N-(2-dimethylaminoethyl)carbamoyl group; a carbamoyl group substituted with a lower alkenyl group which may optionally be substituted with group(s) selected from Substituent group α such as a N-vinylcarbamoyl, N-(2-propenyl)carbamoyl, N-(2-methyl-2-propenyl)carbamoyl, N-(2-butenyl)carbamoyl, N-(2-methyl-2-butenyl)carbamoyl, N-(3-butenyl)carbamoyl, N-(2-hydroxy-3-butenyl)carbamoyl, N-(4-hydroxy-2-butenyl)carbamoyl, N-(2-cyano-2-propenyl)carbamoyl, N-(3-cyano-2-propenyl)carbamoyl, N-(2-fluoro-2-propenyl)carbamoyl, N-(3-fluoro-2-propenyl)carbamoyl, N-(2-chloro-2-propenyl)carbamoyl, N-(3-chloro-2-propenyl)carbamoyl, N-(3,3-difluoro-2-propenyl)carbamoyl, N-(3,3-dichloro-2-propenyl)carbamoyl, N-(3,3-dibromo-2-propenyl)carbamoyl, N-(2-methoxy-3-butenyl)carbamoyl, N-(3-carboxy-2-propenyl)carbamoyl, N-(4-carboxy-3-butenyl)carbamoyl, N-(3-methoxycarbonyl-2-propenyl)carbamoyl, N-(4-methoxycarbonyl-3-butenyl)carbamoyl, N-(3-carbamoyl-2-propenyl)carbamoyl, N-(4-carbamoyl-3-butenyl)carbamoyl, N-(2-methylthio-3-butenyl)carbamoyl, N-(4-methylthio-2-butenyl)carbamoyl, N-(2-trifluoromethylthio-3-butenyl)carbamoyl or N-(4-trifluoromethylthio-2-butenyl)carbamoyl group; a carbamoyl group substituted with a lower alkynyl group which may optionally be substituted with group (s) selected from Substituent group α such as a N-ethynylcarbamoyl, N-(2-propynyl)carbamoyl, N-(1-methyl-2-propynyl) carbamoyl, N-(2-butynyl)carbamoyl, N-(1-methyl-2-butynyl)carbamoyl, N-(3-butynyl)carbamoyl, N-(2-hydroxy-3-butynyl)carbamoyl, N-(4-hydroxy-2-butynyl)carbamoyl, N-(1-cyano-2-propynyl)carbamoyl, N-(3-cyano-2-propynyl)carbamoyl, N-(2-methoxy-3-butynyl)carbamoyl, N-(3-carboxy-2-propynyl)carbamoyl, N-(4-carboxy-3-butynyl)carbamoyl, N-(3-methoxycarbonyl-2-propynyl)carbamoyl, N-(4-methoxycarbonyl-3-butynyl)carbamoyl, N-(3-carbamoyl-2-butynyl) carbamoyl, N-(4-carbamoyl-3-butynyl)carbamoyl, N-(2-methylthio-3-butynyl)carbamoyl, N-(4-methylthio-2-butynyl)carbamoyl, N-(2-trifluoromethylthio-3-butynyl)carbamoyl or N-(4-trifluoromethylthio-2-butynyl)carbamoyl group; a carbamoyl group substituted with a lower alkoxy group such as a N-methoxycarbamoyl, N-methoxy-N-methylcarbamoyl, N-ethoxycarbamoyl, N-propoxycarbamoyl, N-isopropoxycarbamoyl, N-butoxycarbamoyl, N-(s-butoxy)carbamoyl, N-(t-butoxy)carbamoyl or N-isobutoxycarbamoyl group; a carbamoyl group substituted with a lower alkenyloxy group such as a N-(2-propenyloxy)carbamoyl, N-(2-butenyloxy)carbamoyl or N-(3-butenyloxy)carbamoyl group; a carbamoyl group substituted with a lower alkynyloxy group such a N-(2-propynyloxy)carbamoyl, N-(2-butynyloxy)carbamoyl or N-(3-butynyloxy)carbamoyl group; a carbamoyl group substituted with an aralkyloxy group such as a N-benzyloxycarbamoyl, N-benzyloxy-N-methylcarbamoyl or N-phenethyloxycarbamoyl group; a carbamoyl group substituted with a cycloalkyl group such a N-cyclopropylcarbamoyl, N-cyclopropyl-N-methylcarbamoyl, N-cyclobutylcarbamoyl, N-cyclopentylcarbamoyl, N-cyclohexylcarbamoyl or N-cycloheptylcarbamoyl group; a carbamoyl group substituted with a lower alkyl group substituted with a cycloalkyl group such as a N-cyclopropylmethylcarbamoyl, N-cyclopropylmethyl-N-methylcarbamoyl, N-cyclobutylmethylcarbamoyl, N-cyclopentylmethylcarbamoyl, N-cyclohexylmethylcarbamoyl, N-cycloheptylmethylcarbamoyl, N-(2-cyclopropylethyl)carbamoyl, N-(2-cyclobutylethyl)carbamoyl, N-(2-cyclopentylethyl)carbamoyl, N-(2-cyclohexylethyl)carbamoyl, N-(2-cycloheptylethyl)carbamoyl, N-(3-cyclopropylpropyl)carbamoyl, N-(3-cyclopentylpropyl)carbamoyl or N-(3-cyclohexylpropyl)carbamoyl group; a carbamoyl group substituted with an aryl group such as a N-phenylcarbamoyl, N-methyl-N-phenylcarbamoyl, N-(α-naphthyl)carbamoyl or N-(β-naphthyl) carbamoyl group; a carbamoyl group substituted with an aralkyl group such as a N-benzylcarbamoyl, N-(α-naphthylmethyl)carbamoyl, N-(β-naphthylmethyl)carbamoyl, N-(1-phenethyl)carbamoyl, N-(2-phenethyl)carbamoyl, N-[2-(α-naphthyl)ethyl]carbamoyl, N-[2-(β-naphthyl)ethyl]carbamoyl, N-(3-phenylpropyl)carbamoyl, N-(4-phenylbutyl)carbamoyl, N-(5-phenylpentyl)carbamoyl or N-(6-phenylhexyl)carbamoyl group; a carbamoyl group substituted with a heteroaryl group such as a N-(2 or 3-furyl)carbamoyl, N-(2 or 3-thienyl)carbamoyl, N-(2 or 3-pyrrolyl)carbamoyl or N-(2, 3 or 4-pyridyl)carbamoyl group; a carbamoyl group substituted with a lower alkyl group substituted with a heteroaryl group such as a N-(2 or 3-furylmethyl)carbamoyl, N-(2 or 3-furylmethyl)-N-methylcarbamoyl, N-[2-(2 or 3-furyl)ethyl]carbamoyl, N-[3-(2 or 3-furyl)propyl]carbamoyl, N-[4-(2 or 3-furyl)butyl]carbamoyl, N-(2 or 3-thienylmethyl)carbamoyl, N-[2-(2 or 3-thienyl)ethyl]carbamoyl, N-[3-(2 or 3-thienyl)propyl]carbamoyl, N-[4-(2 or 3-thienyl)butyl]carbamoyl, N-(2 or 3-pyrrolylmethyl)carbamoyl, N-[2-(2 or 3-pyrrolyl)ethyl]carbamoyl, N-[3-(2 or 3-pyrrolyl)propyl]carbamoyl, N-[4-(2 or 3-pyrrolyl) butyl]carbamoyl, N-(2, 3 or 4-pyridylmethyl)carbamoyl, N-[2-(2, 3 or 4-pyridyl)ethyl]carbamoyl, N-[3-(2, 3 or 4-pyridyl) propyl]carbamoyl, N-[4-(2, 3 or 4-pyridyl)butyl]carbamoyl, N-(2, 4 or 5-pyrimidinylmethyl)carbamoyl, N-[2-(2, 4 or 5-pyrimidinyl)ethyl]carbamoyl, N-[3-(2, 4 or 5-pyrimidinyl)propyl]carbamoyl or N-[4-(2, 4 or 5-pyrimidinyl)butyl]carbamoyl group; or a carbamoyl group substituted with an amino group, which is a hydrazinocarbonyl group or with a mono- or di-lower alkylamino group such as a hydrazinocarbonyl, 2-methylhydrazinocarbonyl, 2-ethylhydrazinocarbonyl, 2-propylhydrazinocarbonyl, 2-isopropylhydrazinocarbonyl, 2-butylhydrazinocarbonyl, 2,2-dimethylhydrazinocarbonyl, 2-ethyl-2-methylhydrazinocarbonyl or 2,2-diethylhydrazinocarbonyl group; and preferably a carbamoyl group substituted with a lower alkyl group which may optionally be substituted with group(s) selected from Substituent group α such as a carbamoyl, N-methylcarbamoyl, N-ethylcarbamoyl, N-propylcarbamoyl, N-isopropylcarbamoyl, N-dimethylcarbamoyl, N-ethyl-N-methylcarbamoyl, N-(2-hydroxyethyl)carbamoyl, N-(3-hydroxypropyl)carbamoyl, N-(1,3-dihydroxyisopropyl) carbamoyl, N-cyanomethylcarbamoyl, N-(2-cyanomethylethyl)carbamoyl, N-(2-fluoroethyl)carbamoyl, N-(2,2,2-trifluoroethyl)carbamoyl, N-(3-fluoropropyl)carbamoyl or N-(2-methoxyethyl)carbamoyl group; a carbamoyl group substituted with a lower alkenyl group which may optionally be substituted with group(s) selected from Substituent group α such as a N-(2-propenyl)carbamoyl group; a carbamoyl group substituted with a lower alkynyl group which may optionally be substituted with group(s) selected from Substituent group α such as a N-(2-propynyl)carbamoyl group; a carbamoyl group substituted with a lower alkoxy group such as a N-methoxycarbamoyl, N-ethoxycarbamoyl or N-propoxycarbamoyl group; a carbamoyl group substituted with a lower alkenyloxy group such as a N-(2-propenyloxy)carbamoyl group; a carbamoyl group substituted with a lower alkynyloxy group such as a N-(2-propynyloxy)carbamoyl group; a carbamoyl group substituted with a cycloalkyl group such as a N-cyclopropylcarbamoyl, N-cyclobutylcarbamoyl or N-cyclopentylcarbamoyl group; a carbamoyl group substituted with a lower alkyl group which is substituted with a cycloalkyl group such as a N-cyclopropylmethylcarbamoyl group; a carbamoyl group substituted with an aralkyl group such as a N-benzylcarbamoyl or N-(α-naphthyl)carbamoyl group; or a carbamoyl group substituted with an amino group, which is a hydrazinocarbonyl group, or with a mono- or di-lower alkylamino group such as a hydrazinocarbonyl, 2-methylhydrazinocarbonyl or 2,2-dimethylhydrazinocarbonyl group.

The "group having formula: $COR^c$" in the definition of $R^5$ can be, for example, an alkanoyl group such as a formyl, acetyl, propionyl, butylyl, isobutylyl, valeryl, pivaloyl, isovaleryl, hexanoyl or heptanoyl group; a halogeno lower alkylcarbonyl group such as a fluoroacetyl, difluoroacetyl, trifluoroacetyl, chloroacetyl, dichloroacetyl, trichloroacetyl, bromoacetyl, 3-fluoropropionyl, 3,3-difluoropropionyl, 3,3,3-trifluoropropionyl, 3-chloropropionyl, 3,3-dichloropropionyl, 3,3,3-trichloropropionyl or 3-bromopropionyl group; a lower alkoxy lower alkylcarbonyl group such as a methoxymethylcarbonyl, ethoxymethylcarbonyl, 2-methoxyethylcarbonyl, 2-ethoxyethylcarbonyl, 2-propoxyethylcarbonyl, 2-butoxyethylcarbonyl, 3-methoxypropylcarbonyl or 4-methoxybutylcarbonyl group; or a hydroxy lower alkylcarbonyl group such as a hydroxyacetyl, 2-hydroxyethylcarbonyl, 3-hydroxypropylcarbonyl or 4-hydroxybutylcarbonyl group; and is preferably an alkanoyl group such as a formyl, acetyl or propionyl group; a halogeno lower alkylcarbonyl group such as a fluoroacetyl group; a lower alkoxy lower alkylcarbonyl group such as a methoxymethylcarbonyl group; or a hydroxy lower alkylcarbonyl group such as a hydroxyacetyl group.

The "group having formula: $SO_2NR^aR^b$" in the definition of $R^5$ can be, for example, a sulfamoyl group substituted with a lower alkyl group which may optionally be substituted with group(s) selected from Substituent group α such as a sulfamoyl, N-hydroxysulfamoyl, N-methylsulfamoyl, N-ethylsulfamoyl, N-propylsulfamoyl, N-isopropylsulfamoyl, N-butylsulfamoyl, N-(s-butyl)sulfamoyl, N-(t-butyl)sulfamoyl, N-isobutylsulfamoyl, N-pentylsulfamoyl, N-hexylsulfamoyl, N, N-dimethylsulfamoyl, N-ethyl-N-methylsulfamoyl, N,N-diethylsulfamoyl, N-(hydroxymethyl)sulfamoyl, N-(2-hydroxyethyl)sulfamoyl, N-(3-hydroxypropyl)sulfamoyl, N-(1,3-dihydroxyisopropyl)sulfamoyl, N-(2-nitroethyl)sulfamoyl, N-(cyanomethyl)sulfamoyl, N-(2-cyanoethyl)sulfamoyl, N-(2-fluoroethyl)sulfamoyl, N-(2,2,2-trifluoroethyl)sulfamoyl, N-(3-fluoropropyl)sulfamoyl, N-(4-fluorobutyl)sulfamoyl, N-(5-fluoropentyl)sulfamoyl, N-(6-fluorohexyl)sulfamoyl,

N-(2-chloroethyl)sulfamoyl, N-(3-chloropropyl)sulfamoyl, N-(4-chlorobutyl)sulfamoyl, N-(2-bromoethyl)sulfamoyl, N-(3-bromopropyl)sulfamoyl, N-(4-bromobutyl)sulfamoyl, N-(methoxymethyl)sulfamoyl, N-(2-methoxyethyl)sulfamoyl, N-(3-methoxypropyl)sulfamoyl, N-(4-methoxybutyl)sulfamoyl, N-(ethoxymethyl)sulfamoyl, N-(2-ethoxyethyl)sulfamoyl, N-(3-ethoxypropyl)sulfamoyl, N-(4-ethoxybutyl)sulfamoyl, N-(carboxymethyl)sulfamoyl, N-(2-carboxyethyl)sulfamoyl, N-(3-carboxypropyl)sulfamoyl, N-(methoxycarbonylmethyl)sulfamoyl, N-(2-methoxycarbonylethyl)sulfamoyl, N-(3-methoxycarbonylpropyl)sulfamoyl, N-(ethoxycarbonylmethyl)sulfamoyl, N-(1-ethoxycarbonylethyl)sulfamoyl, N-(2-ethoxycarbonylethyl)sulfamoyl, N-(3-ethoxycarbonylpropyl)sulfamoyl, N-(carbamoylmethyl)sulfamoyl, N-(2-acetoxyethyl)sulfamoyl, N-(2-methylthioethyl)sulfamoyl, N-(2-ethylthioethyl)sulfamoyl, N-(2-propylthioethyl)sulfamoyl, N-(2-fluoromethylthioethyl)sulfamoyl, N-(2-difluoromethylthioethyl)sulfamoyl, N-(2-trifluoromethylthioethyl)sulfamoyl, N-[2-(2-fluoroethylthio)ethyl]sulfamoyl, N-[2-(2,2,2-trifluoroethylthio)ethyl]sulfamoyl, N-(2-aminoethyl)sulfamoyl, N-(2-methylaminoethyl)sulfamoyl or N-(2-dimethylaminoethyl)sulfamoyl group; a sulfamoyl group substituted with a lower alkenyl group which may optionally be substituted with group(s) selected from Substituent group $\alpha$ such as a N-vinylsulfamoyl, N-(2-propenyl)sulfamoyl, N-(2-methyl-2-propenyl)sulfamoyl, N-(2-butenyl)sulfamoyl, N-(2-methyl-2-butenyl) sulfamoyl, N-(3-butenyl)sulfamoyl, N-(2-hydroxy-3-butenyl)sulfamoyl, N-(4-hydroxy-2-butenyl)sulfamoyl, N-(2-cyano-2-propenyl)sulfamoyl, N-(3-cyano-2-propenyl)sulfamoyl, N-(2-fluoro-2-propenyl)sulfamoyl, N-(3-fluoro-2-propenyl)sulfamoyl, N-(2-chloro-2-propenyl)sulfamoyl, N-(3-chloro-2-propenyl)sulfamoyl, N-(3,3-difluoro-2-propenyl)sulfamoyl, N-(3,3-dichloro-2-propenyl)sulfamoyl, N-(3,3-dibromo-2-propenyl)sulfamoyl, N-(2-methoxy-3-butenyl)sulfamoyl, N-(3-carboxy-2-propenyl)sulfamoyl, N-(4-carboxy-3-butenyl)sulfamoyl, N-(3-methoxycarbonyl-2-propenyl)sulfamoyl, N-(4-methoxycarbonyl-3-butenyl)sulfamoyl, N-(3-carbamoyl-2-propenyl)sulfamoyl, N-(4-carbamoyl-3-butenyl)sulfamoyl, N-(2-methylthio-3-butenyl)sulfamoyl, N-(4-methylthio-2-butenyl)sulfamoyl, N-(2-trifluoromethylthio-3-butenyl)sulfamoyl or N-(4-trifluoromethylthio-2-butenyl)sulfamoyl group; a sulfamoyl group substituted with a lower alkynyl group which may optionally be substituted with group(s) selected from Substituent group $\alpha$ such as a N-ethynylsulfamoyl, N-(2-propynyl)sulfamoyl, N-(1-methyl-2-propynyl)sulfamoyl, N-(2-butynyl)sulfamoyl, N-(1-methyl-2-butynyl)sulfamoyl, N-(3-butynyl)sulfamoyl, N-(2-hydroxy-3-butynyl)sulfamoyl, N-(4-hydroxy-2-butynyl)sulfamoyl, N-(1-cyano-2-propynyl) sulfamoyl, N-(3-cyano-2-propynyl)sulfamoyl, N-(2-methoxy-3-butynyl)sulfamoyl, N-(3-carboxy-2-propynyl)sulfamoyl, N-(4-carboxy-3-butynyl)sulfamoyl, N-(3-methoxycarbonyl-2-propynyl)sulfamoyl, N-(4-methoxycarbonyl-3-butynyl)sulfamoyl, N-(3-carbamoyl-2-butynyl)sulfamoyl, N-(4-carbamoyl-3-butynyl)sulfamoyl, N-(2-methylthio-3-butynyl)sulfamoyl, N-(4-methylthio-2-butynyl)sulfamoyl, N-(2-trifluoromethylthio-3-butynyl)sulfamoyl or N-(4-trifluoromethylthio-2-butynyl) sulfamoyl group; a sulfamoyl group substituted with a lower alkoxy group such as a N-methoxysulfamoyl, N-methoxy-N-methylsulfamoyl, N-ethoxysulfamoyl, N-propoxysulfamoyl, N-isopropoxysulfamoyl, N-butoxysulfamoyl, N-(s-butoxy)sulfamoyl, N-(t-butoxy)sulfamoylor N-isobutoxysulfamoyl group; a sulfamoyl group substituted with a lower alkenyloxy group such as a N-(2-propenyloxy)sulfamoyl, N-(2-butenyloxy)sulfamoyl or N-(3-butenyloxy)sulfamoyl group; a sulfamoyl group substituted with a lower alkynyloxy group such as a N-(2-propynyloxy)sulfamoyl, N-(2-butynyloxy) sulfamoyl or N-(3-butynyloxy)sulfamoyl group; a sulfamoyl group substituted with an aralkyloxy group such as a N-benzyloxysulfamoyl, N-benzyloxy-N-methylsulfamoyl or N-phenethyloxysulfamoyl group; a sulfamoyl group substituted with a cycloalkyl group such as a N-cyclopropylsulfamoyl, N-cyclopropyl-N-methylsulfamoyl, N-cyclobutylsulfamoyl, N-cyclopentylsulfamoyl, N-cyclohexylsulfamoyl or N-cycloheptylsulfamoyl group; a sulfamoyl group substituted with a lower alkyl group which is substituted with a cycloalkyl group such as a N-cyclopropylmethylsulfamoyl, N-cyclopropylmethyl-N-methylsulfamoyl, N-cyclobutylmethylsulfamoyl, N-cyclopentylmethylsulfamoyl, N-cyclohexylmethylsulfamoyl, N-cycloheptylmethylsulfamoyl, N-(2-cyclopropylethyl)sulfamoyl, N-(2-cyclobutylethyl)sulfamoyl, N-(2-cyclopentylethyl) sulfamoyl, N-(2-cyclohexylethyl)sulfamoyl, N-(2-cycloheptylethyl)sulfamoyl, N-(3-cyclopropylpropyl)sulfamoyl, N-(3-cyclopentylpropyl)sulfamoyl or N-(3-cyclohexylpropyl)sulfamoyl group; a sulfamoyl group substituted with an aryl group such as a N-phenylsulfamoyl, N-methyl-N-phenylsulfamoyl, N-($\alpha$-naphthyl)sulfamoyl or N-($\beta$-naphthyl)sulfamoyl group; a sulfamoyl group substituted with an aralkyl group such as a N-benzylsulfamoyl, N-($\alpha$-naphthylmethyl)sulfamoyl, N-($\beta$-naphthylmethyl)sulfamoyl, N-(1-phenethyl)sulfamoyl, N-(2-phenethyl)sulfamoyl, N-[2-($\alpha$-naphthyl)ethyl]sulfamoyl, N-[2-($\beta$-naphthyl)ethyl]sulfamoyl, N-(3-phenylpropyl)sulfamoyl, N-(4-phenylbutyl)sulfamoyl, N-(5-phenylpentyl)sulfamoyl or N-(6-phenylhexyl)sulfamoyl group; a sulfamoyl group substituted with a heteroaryl group such as a N-(2 or 3-furyl)sulfamoyl, N-(2 or 3-thienyl)sulfamoyl , N-(2 or 3-pyrrolyl)sulfamoyl or N-(2 , 3 or 4-pyridyl)sulfamoyl group; a sulfamoyl group substituted with a lower alkyl group which is substituted with a heteroaryl group such as a N-(2 or 3-furylmethyl)sulfamoyl, N-(2 or 3-furylmethyl)-N-methylsulfamoyl, N-[2-(2 or 3-furyl)ethyl]sulfamoyl, N-[3-(2 or 3-furyl) propyl]sulfamoyl, N-[4-(2 or 3-furyl)butyl]sulfamoyl, N-(2 or 3-thienylmethyl)sulfamoyl, N-[2-(2 or 3-thienyl)ethyl]sulfamoyl, N-[3-(2 or 3-thienyl)propyl]sulfamoyl, N-[4-(2 or 3-thienyl)butyl]sulfamoyl, N-(2 or 3-pyrrolylmethyl)sulfamoyl, N-[2-(2 or 3-pyrrolyl)ethyl]sulfamoyl, N-[3-(2 or 3-pyrrolyl)propyl]sulfamoyl , N-[4-(2 or 3-pyrrolyl)butyl]sulfamoyl, N-(2 or 3-pyridylmethyl)sulfamoyl, N-[2-(2, 3 or 4-pyridyl)ethyl]sulfamoyl, N-[3-(2, 3 or 4-pyridyl)propyl]sulfamoyl, N-[4-(2, 3 or 4-pyridyl)butyl]sulfamoyl, N-(2, 4 or 5-pyrimidinylmethyl)sulfamoyl, N-[2-(2, 4 or 5-pyrimidinyl)ethyl]sulfamoyl, N-[3-(2, 4 or 5-pyrimidinyl)propyl]sulfamoyl or N-[4-(2, 4 or 5-pyrimidinyl)butyl]sulfamoyl group; or a sulfamoyl group substituted with an amino group, which is a hydrazinosulfonyl group, or with a mono- or di-lower alkylamino group such as a hydrazinosulfonyl, 2-methylhydrazinosulfonyl, 2-ethylhydrazinosulfonyl, 2-propylhydrazinosulfonyl, 2-isopropylhydrazinosulfonyl, 2-butylhydrazinosulfonyl, 2,2-dimethylhydrazinosulfonyl, 2-ethyl-2-methylhydrazinosulfonyl or 2,2-diethylhydrazinosulfonyl group; and is preferably a sulfamoyl group substituted with a lower alkyl group which may optionally be substituted with group(s) selected from Substituent group α such as a sulfamoyl, N-methylsulfamoyl, N-ethylsulfamoyl, N-propylsulfamoyl, N-isopropylsulfamoyl, N,N-dimethylsulfamoyl, N-ethyl-N-methylsulfamoyl, N-(2-hydroxyethyl)sulfamoyl, N-(3-hydroxypropyl)sulfamoyl, N-(1,3-dihydroxyisopropyl)sulfamoyl, N-cyanomethylsulfamoyl, N-(2-cyanoethyl) sulfamoyl, N-(2-fluoroethyl)sulfamoyl, N-(2,2,2-trifluoroethyl)sulfamoyl, N-(3-fluoropropyl)sulfamoyl or N-(2-methoxyethyl)sulfamoyl group; a sulfamoyl group substituted with a lower alkenyl group which may optionally be substituted with group(s) selected from Substituent group α such as a N-(2-propenyl)sulfamoyl group; a sulfamoyl group substituted with a lower alkynyl group which may optionally be substituted with a group selected from Substituent group α such as a N-(2-propynyl)sulfamoyl group; a sulfamoyl group substituted with a lower alkoxy group such as a N-methoxysulfamoyl, N-ethoxysulfamoyl or N-propoxysulfamoyl group; a sulfamoyl group substituted with a lower alkenyloxy group such as a N-(2-propenyloxy)sulfamoyl group; a sulfamoyl group substituted with a lower alkynyloxy group such as a N-(2-propynyloxy)sulfamoyl group; a sulfamoyl group substituted with a cycloalkyl group such as a N-cyclopropylsulfamoyl, N-cyclobutylsulfamoyl or N-cyclopentylsulfamoyl group; a sulfamoyl group substituted with a lower alkyl group which is substituted with a cycloalkyl group such as a N-cyclopropylmethylsulfamoyl group; a sulfamoyl group substituted with an aralkyl group such as a N-benzylsulfamoyl or N-(α-naphthyl)sulfamoyl group; or a sulfamoyl group substituted with an amino group, which is a hydrazinosulfonyl group, or with a mono- or di-lower alkylamino group such as a hydrazinosulfonyl, 2-methylhydrazinosulfonyl, or 2,2-dimethylhydrazinosulfonyl group.

The "group having formula: $SO_2R^c$" in the definition of $R^5$ can be, for example, a lower alkylsulfonyl group such as a methanesulfonyl, ethanesulfonyl, propanesulfonyl, isopropanesulfonyl, butanesulfonyl, s-butanesulfonyl, t-butanesulfonyl, isobutanesulfonyl, pentanesulfonyl or hexanesulfonyl group; a halogeno lower alkylsulfonyl group such as a fluoromethanesulfonyl, difluoromethanesulfonyl, trifluoromethanesulfonyl, chloromethanesulfonyl, dichloromethanesulfonyl, trichloromethanesulfonyl, bromomethanesulfonyl, 2-fluoroethanesulfonyl, 3-fluoropropanesulfonyl, 3,3-difluoropropanesulfonyl, 3,3,3-trifluoropropanesulfonyl, 3-chloropropanesulfonyl, 3,3-dichloropropanesulfonyl, 3,3,3-trichloropropanesulfonyl or 3-bromopropanesulfonyl group; a lower alkoxy lower alkylsulfonyl group such as a methoxymethanesulfonyl, ethoxymethanesulfonyl, 2-methoxyethanesulfonyl, 2-ethoxyethanesulfonyl, 2-propoxyethanesulfonyl, 2-butoxyethanesulfonyl, 3-methoxypropanesulfonyl or 4-methoxybutanesulfonyl group; or a hydroxy lower alkylsulfonyl group such as a hydroxymethanesulfonyl, 2-hydroxyethanesulfonyl, 3-hydroxypropanesulfonyl or 4-hydroxybutanesulfonyl group; and is preferably a lower alkylsulfonyl group such as a methanesulfonyl or ethanesulfonyl group; a halogeno lower alkylsulfonyl group such as a fluoromethanesulfonyl or 2-fluoroethanesulfonyl group; a lower alkoxy lower alkylsulfonyl group such as a methoxymethanesulfonyl or 2-methoxyethanesulfonyl group; or a hydroxy lower alkylsulfonyl group such as a hydroxymethanesulfonyl or 2-hydroxyethanesulfonyl group.

The "group having formula: $SOR^c$" in the definition of $R^5$ can be, for example, a lower alkylsulfinyl group such as a methanesulfinyl, ethanesulfinyl, propanesulfinyl, isopropanesulfinyl, butanesulfinyl, s-butanesulfinyl, t-butanesulfinyl, isobutanesulfinyl, pentanesulfinyl or hexanesulfinyl group; a halogeno lower alkylsulfinyl group such as a fluoromethanesulfinyl, difluoromethanesulfinyl, trifluoromethanesulfinyl, chloromethanesulfinyl, dichloromethanesulfinyl, trichloromethanesulfinyl, bromomethanesulfinyl, 2-fluoroethanesulfinyl, 3-fluoropropanesulfinyl, 3,3-difluoropropanesulfinyl, 3,3,3-trifluoropropanesulfinyl, 3-chloropropanesulfinyl, 3,3-dichloropropanesulfinyl, 3,3,3-trichloropropanesulfinyl or 3-bromopropanesulfinyl group; a lower alkoxy lower alkylsulfinyl group such as a methoxymethanesulfinyl, ethoxymethanesulfinyl, 2-methoxyethanesulfinyl, 2-ethoxyethanesulfinyl, 2-propoxyethanesulfinyl, 2-butoxyethanesulfinyl, 3-methoxypropanesulfinyl or 4-methoxybutanesulfinyl group; or a hydroxy lower alkylsulfinyl group such as a hydroxymethanesulfinyl, 2-hydroxyethanesulfinyl, 3-hydroxypropanesulfinyl or 4-hydroxybutanesulfinyl group; and is preferably a lower alkylsulfinyl group such as a methanesulfinyl or ethanesulfinyl group; a hologeno lower alkylsulfinyl group such as a fluoromethanesulfinyl or 2-fluoroethanesulfinyl group; a lower alkoxy lower alkylsulfinyl group such as a methoxymethanesulfinyl or 2-methoxyethanesulfinyl group; or a hydroxy lower alkylsulfinyl group such as a hydroxymethanesulfinyl or 2-hydroxyethanesulfinyl group.

The "lower alkylcarbonyl group" in the definition of $R^d$ and $R^e$ is a group wherein the "lower alkyl group" described above is bonded to a carbonyl group, and preferably a straight or branched chain alkylcarbonyl group having from 1 to 4 carbon atoms, more preferably an alkylcarbonyl group having from 1 to 3 carbon atoms, and particularly preferably an acetyl group.

The "lower acyloxy group" in the definition of "Substituent group α" is a group wherein the "lower alkylcarbonyl group" described above is bonded to an oxygen atom, preferably a straight or branched chain acyloxy group having from 1 to 5 carbon atoms, and more preferably an acetyloxy group, a propionyloxy group, a butylyloxy group or a pivaloyloxy group.

The "lower alkoxycarbonyl group" in the definition of $R^5$ and "Substituent group α" is a group wherein the "lower alkoxy group" described above is bonded to a carbonyl group, and preferably a group wherein an alkoxy group having from 1 to 4 carbon atoms is bonded to a carbonyl group. Such group can be, for example, a methoxycarbonyl, ethoxycarbonyl, propoxycarbonyl, butoxycarbonyl, s-butoxycarbonyl, t-butoxycarbonyl or isobutoxycarbonyl group, and is preferably a methoxycarbonyl, ethoxycarbonyl or propoxycarbonyl group.

The "aryloxycarbonyl group" in the definition of $R^5$ is a group wherein an aryloxy group formed by bonding the "aryl group" described above to an oxygen atom is bonded to a carbonyl group, and such group can be, for example, a phenoxycarbonyl, naphthyloxycarbonyl, phenanthryloxycarbonyl or anthracenyloxycarbonyl group, and is preferably a phenoxycarbonyl or naphtyloxycarbonyl group, and more preferably a phenoxycarbonyl group.

The "aralkyloxycarbonyl group" in the definition of $R^5$ is a group wherein a "aralkyloxy group" described above is bonded to a carbonyl group, and the preferable group is, for example, a benzyloxycarbonyl or phenethyloxycarbonyl group.

Of groups defined as $R^5$, the preferable group is a group having formula: $CONR^aR^b$, a group having formula: $COR^c$, a group having formula: $SO_2NR^aR^b$, a group having formula: $SO_2R^c$ or a group having formula: $SOR^c$ (wherein, $R^a$ and $R^b$ are the same or different and each represents independently a hydrogen atom, a lower alkyl group which may optionally be substituted with group(s) selected from Substituent group $\alpha$, a lower alkoxy group, a lower alkenyloxy group, a cycloalkyl group, an amino group, or mono- or di-lower alkylamino group, and $R^c$ represents a lower alkyl group); the more preferable group is a group having formula: $CONR^aR^b$, a group having formula: $SO_2NR^aR^b$, a group having formula: $SO_2R^c$ or a group having formula: $SOR^c$ (wherein, $R^a$ and $R^b$ are the same or different and each represents independently a hydrogen atom, a lower alkyl group, a halogeno lower alkyl group, a hydroxy lower alkyl group, a lower alkoxy lower alkyl group, a lower alkoxy group or a cycloalkyl group, and $R^c$ represents a lower alkyl group); and the still more preferable group is a carbamoyl, N-methylcarbamoyl, N-ethylcarbamoyl, N-propylcarbamoyl, N-isopropylcarbamoyl, N-cyclopropylcarbamoyl, N-cyclopentylcarbamoyl, N-(2-fluoroethyl)carbamoyl, N-(2-methoxyethy)carbamoyl, N-methylsulfamoyl, N-ethylsulfamoyl, N-propylsulfamoyl, N-isopropylsulfamoyl, N-cyclopropylsulfamoyl, N-cyclopentylsulfamoyl, N-(2-fluoroethyl)sulfamoyl, N-(2-methoxyethy)sulfamoyl, N-methoxysulfamoyl, methanesulfonyl, ethanesulfonyl, propanesulfonyl, methanesulfinyl, ethanesulfinyl or propanesulfinyl group.

Of the groups defined as $R^6$, the preferable group is a hydrogen atom, a fluorine atom or a methoxy group, and the more preferable group is a hydrogen atom.

Of the groups defined as $R^7$, the preferable group is a hydrogen atom, a hydroxyl group or a lower alkyl group; the more preferable group is a hydrogen atom, a hydroxyl group or a methyl group; and the still more preferable group is a hydrogen atom.

Of the groups defined as "Substituent group $\alpha$", the preferable group is shown as "Substituent group $\alpha^1$", in which a hydroxyl group, a cyano group, a halogen atom, a lower alkoxy group and a halogeno lower alkoxy group are included. Of the groups defined as "Substituent group $\alpha^1$", the preferable group is a hydroxyl group, a cyano group, a halogen atom, a lower alkoxy group or a halogeno lower alkoxy group, and the more preferable group is a halogen atom.

Of the groups defined as "Substituent group $\beta$", the preferable group is shown as "Substituent group $\beta^1$", in which a lower alkyl group, a halogeno lower alkyl group and a hydroxy lower alkyl group are included. Of the groups defined as "Substituent group $\beta^1$", the preferable group is a halogeno lower alkyl group.

The "pharmacologically acceptable salts thereof" means salts that can be prepared by reacting the compound having general formula (I) of the present invention with an acid when the compound has a basic group such as an amino group, or can be prepared by reacting the compound having general formula (I) of the present invention with a base when the compound has an acidic group such as a carboxyl group, a carbamoyl group, a sulfamoyl group, or a hydroxyaryl group.

When the compound having general formula (I) of the present invention has a basic group, the salt is preferably an inorganic acid salt consisting of: a hydrohalide such as hydrochloride, hydrobromide or hydroiodide, a nitrate, a perchlorate, a sulfate, a phosphate or the like; an organic acid salt consisting of: a lower alkanesulfonate such as methanesulfonate, trifluoromethanesulfonate or ethanesulfonate, an arylsulfonate such as benzenesulfonate or p-toluenesulfonate, an acetate, a malate, a fumarate, a succinate, a citrate, an ascorbate, a tartrate, an oxalate, a maleate or the like; or an amino acid salt such as glycine salt, lysine salt, arginine salt, ornithine salt, glutamic acid salt, or aspartic acid salt.

On the other hand, when the compound having general formula (I) of the present invention has an acidic group, the salt is preferably a metal salt consisting of: an alkali metal salt such as sodium salts, potassium salts or lithium salts; an alkaline earth metal salts such as calcium salts or magnesium salts; aluminium salts, iron salts, or the like; an amine salt consisting of: inorganic amine salts such as ammonium salts; organic amine salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts, or tris(hydroxymethyl)aminomethane salts; or amino acid salts such as glycine salts, lysine salts, arginine salts, ornithine salts, glutamic acid salts or aspartic acid salts.

Furthermore, when the compounds having general formula (I) of the present invention or pharmacologically acceptable salts thereof are allowed to stand in contact with the atmosphere or to recrystallize, they may absorb water or water may attach to them to form a hydrate. The present invention encompasses such hydrates.

The compounds having general formula (I) of the present invention can exist as geometrical isomers (cis-trans isomers or Z-E isomers) and optical isomers due to the asymmetric centre in their structures. In the present invention, each of geometrical and optical isomers and mixtures of these isomers are represented as a single chemical formula (I). Accordingly, the present invention encompasses both individual isomers and mixtures thereof in any ratio.

Of the compounds having general formula (I), preferable examples are shown in following Tables 1-14. These compounds shown in each of Tables 1-14 have a corresponding formula from (I-1) to (I-14) shown in each table, respectively.

Table 1

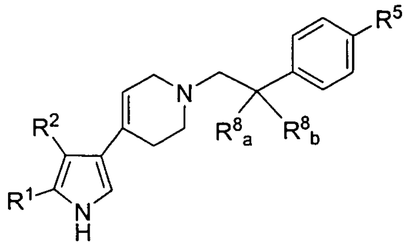

(I-1)

| Compound No. | $R^1$ | $R^2$ | $R^5$ | $R^8{}_a$ | $R^8{}_b$ |
|---|---|---|---|---|---|
| 1-1 | Ph | 4-Pyr | COOH | H | H |
| 1-2 | Ph | 4-Pyr | COOMe | H | H |
| 1-3 | Ph | 4-Pyr | COOEt | H | H |
| 1-4 | Ph | 4-Pyr | COOPr | H | H |
| 1-5 | Ph | 4-Pyr | COO-i-Pr | H | H |
| 1-6 | Ph | 4-Pyr | COOBu | H | H |
| 1-7 | Ph | 4-Pyr | COOBn | H | H |
| 1-8 | Ph | 4-Pyr | COOPh | H | H |
| 1-9 | Ph | 4-Pyr | $CONH_2$ | H | H |
| 1-10 | Ph | 4-Pyr | CONHMe | H | H |
| 1-11 | Ph | 4-Pyr | CONHEt | H | H |
| 1-12 | Ph | 4-Pyr | CONHPr | H | H |
| 1-13 | Ph | 4-Pyr | CONH-i-Pr | H | H |
| 1-14 | Ph | 4-Pyr | CONHBu | H | H |
| 1-15 | Ph | 4-Pyr | CONHBn | H | H |
| 1-16 | Ph | 4-Pyr | $CONMe_2$ | H | H |
| 1-17 | Ph | 4-Pyr | CONH-c-Pr | H | H |
| 1-18 | Ph | 4-Pyr | CONH-c-Bu | H | H |
| 1-19 | Ph | 4-Pyr | CONH-c-Pen | H | H |
| 1-20 | Ph | 4-Pyr | CONH-c-Hex | H | H |
| 1-21 | Ph | 4-Pyr | CONH-c-Hep | H | H |
| 1-22 | Ph | 4-Pyr | $CONHCH_2$-c-Pr | H | H |
| 1-23 | Ph | 4-Pyr | CONH-Allyl | H | H |
| 1-24 | Ph | 4-Pyr | CONH-Propargyl | H | H |
| 1-25 | Ph | 4-Pyr | CONHPh | H | H |
| 1-26 | Ph | 4-Pyr | CONH-3-Pyr | H | H |

| | | | | | |
|---|---|---|---|---|---|
| 1-27 | Ph | 4-Pyr | $CONHCH_2$-4-Pyr | H | H |
| 1-28 | Ph | 4-Pyr | CONH-$(CH_2)_2$-OH | H | H |
| 1-29 | Ph | 4-Pyr | $CONHCH(CH_2OH)_2$ | H | H |
| 1-30 | Ph | 4-Pyr | CONH-$(CH_2)_2$-OAc | H | H |
| 1-31 | Ph | 4-Pyr | $CONHCH_2CN$ | H | H |
| 1-32 | Ph | 4-Pyr | CONH-$(CH_2)_2$-F | H | H |
| 1-33 | Ph | 4-Pyr | CONH-$(CH_2)_2$-OMe | H | H |
| 1-34 | Ph | 4-Pyr | CONH-$(CH_2)_2$-SMe | H | H |
| 1-35 | Ph | 4-Pyr | CONH-$(CH_2)_2$-$NH_2$ | H | H |
| 1-36 | Ph | 4-Pyr | CONH-$(CH_2)_2$-NHMe | H | H |
| 1-37 | Ph | 4-Pyr | CONH-$(CH_2)_2$-$N(Me)_2$ | H | H |
| 1-38 | Ph | 4-Pyr | $CONHCH_2COOH$ | H | H |
| 1-39 | Ph | 4-Pyr | $CONHCH_2COOEt$ | H | H |
| 1-40 | Ph | 4-Pyr | CONHCH(Me)COOEt | H | H |
| 1-41 | Ph | 4-Pyr | $CONHCH_2CONH_2$ | H | H |
| 1-42 | Ph | 4-Pyr | CONHOH | H | H |
| 1-43 | Ph | 4-Pyr | CONHOMe | H | H |
| 1-44 | Ph | 4-Pyr | CONHOEt | H | H |
| 1-45 | Ph | 4-Pyr | CONHOPr | H | H |
| 1-46 | Ph | 4-Pyr | CONHO-Allyl | H | H |
| 1-47 | Ph | 4-Pyr | CONHOBn | H | H |
| 1-48 | Ph | 4-Pyr | $CONHNH_2$ | H | H |
| 1-49 | Ph | 4-Pyr | CONHNHMe | H | H |
| 1-50 | Ph | 4-Pyr | $CONHN(Me)_2$ | H | H |
| 1-51 | Ph | 4-Pyr | COMe | H | H |
| 1-52 | Ph | 4-Pyr | COEt | H | H |
| 1-53 | Ph | 4-Pyr | COPr | H | H |
| 1-54 | Ph | 4-Pyr | CO-i-Pr | H | H |
| 1-55 | Ph | 4-Pyr | COBu | H | H |
| 1-56 | Ph | 4-Pyr | $COCF_3$ | H | H |
| 1-57 | Ph | 4-Pyr | CO-$(CH_2)_2$-F | H | H |
| 1-58 | Ph | 4-Pyr | CO-$(CH_2)_2$-OH | H | H |
| 1-59 | Ph | 4-Pyr | CO-$(CH_2)_2$-OMe | H | H |
| 1-60 | Ph | 4-Pyr | SOMe | H | H |
| 1-61 | Ph | 4-Pyr | SOEt | H | H |
| 1-62 | Ph | 4-Pyr | SOPr | H | H |

| | | | | | |
|---|---|---|---|---|---|
| 1-63 | Ph | 4-Pyr | SO-i-Pr | H | H |
| 1-64 | Ph | 4-Pyr | SOBu | H | H |
| 1-65 | Ph | 4-Pyr | $SOCF_3$ | H | H |
| 1-66 | Ph | 4-Pyr | SO-$(CH_2)_2$-F | H | H |
| 1-67 | Ph | 4-Pyr | SO-$(CH_2)_2$-OH | H | H |
| 1-68 | Ph | 4-Pyr | SO-$(CH_2)_2$-OMe | H | H |
| 1-69 | Ph | 4-Pyr | $SO_2Me$ | H | H |
| 1-70 | Ph | 4-Pyr | $SO_2Et$ | H | H |
| 1-71 | Ph | 4-Pyr | $SO_2Pr$ | H | H |
| 1-72 | Ph | 4-Pyr | $SO_2$-i-Pr | H | H |
| 1-73 | Ph | 4-Pyr | $SO_2Bu$ | H | H |
| 1-74 | Ph | 4-Pyr | $SO_2CF_3$ | H | H |
| 1-75 | Ph | 4-Pyr | $SO_2$-$(CH_2)_2$-F | H | H |
| 1-76 | Ph | 4-Pyr | $SO_2$-$(CH_2)_2$-OH | H | H |
| 1-77 | Ph | 4-Pyr | $SO_2$-$(CH_2)_2$-OMe | H | H |
| 1-78 | Ph | 4-Pyr | $SO_2NH_2$ | H | H |
| 1-79 | Ph | 4-Pyr | $SO_2NHMe$ | H | H |
| 1-80 | Ph | 4-Pyr | $SO_2NHEt$ | H | H |
| 1-81 | Ph | 4-Pyr | $SO_2NHPr$ | H | H |
| 1-82 | Ph | 4-Pyr | $SO_2NH$-i-Pr | H | H |
| 1-83 | Ph | 4-Pyr | $SO_2NHBu$ | H | H |
| 1-84 | Ph | 4-Pyr | $SO_2NHBn$ | H | H |
| 1-85 | Ph | 4-Pyr | $SO_2NMe_2$ | H | H |
| 1-86 | Ph | 4-Pyr | $SO_2NH$-c-Pr | H | H |
| 1-87 | Ph | 4-Pyr | $SO_2NH$-c-Bu | H | H |
| 1-88 | Ph | 4-Pyr | $SO_2NH$-c-Pen | H | H |
| 1-89 | Ph | 4-Pyr | $SO_2NH$-c-Hex | H | H |
| 1-90 | Ph | 4-Pyr | $SO_2NH$-c-Hep | H | H |
| 1-91 | Ph | 4-Pyr | $SO_2NHCH_2$-c-Pr | H | H |
| 1-92 | Ph | 4-Pyr | $SO_2NH$-Allyl | H | H |
| 1-93 | Ph | 4-Pyr | $SO_2NH$-Propargyl | H | H |
| 1-94 | Ph | 4-Pyr | $SO_2NHPh$ | H | H |
| 1-95 | Ph | 4-Pyr | $SO_2NH$-3-Pyr | H | H |
| 1-96 | Ph | 4-Pyr | $SO_2NHCH_2$-4-Pyr | H | H |
| 1-97 | Ph | 4-Pyr | $SO_2NH$-$(CH_2)_2$-OH | H | H |
| 1-98 | Ph | 4-Pyr | $SO_2NHCH(CH_2OH)_2$ | H | H |

| | | | | | |
|---|---|---|---|---|---|
| 1-99 | Ph | 4-Pyr | $SO_2NH-(CH_2)_2-OAc$ | H | H |
| 1-100 | Ph | 4-Pyr | $SO_2NHCH_2CN$ | H | H |
| 1-101 | Ph | 4-Pyr | $SO_2NH-(CH_2)_2-F$ | H | H |
| 1-102 | Ph | 4-Pyr | $SO_2NH-(CH_2)_2-OMe$ | H | H |
| 1-103 | Ph | 4-Pyr | $SO_2NH-(CH_2)_2-SMe$ | H | H |
| 1-104 | Ph | 4-Pyr | $SO_2NH-(CH_2)_2-NH_2$ | H | H |
| 1-105 | Ph | 4-Pyr | $SO_2NH-(CH_2)_2-NHMe$ | H | H |
| 1-106 | Ph | 4-Pyr | $SO_2NH-(CH_2)_2-N(Me)_2$ | H | H |
| 1-107 | Ph | 4-Pyr | $SO_2NHCH_2COOH$ | H | H |
| 1-108 | Ph | 4-Pyr | $SO_2NHCH_2COOEt$ | H | H |
| 1-109 | Ph | 4-Pyr | $SO_2NHCH(Me)COOEt$ | H | H |
| 1-110 | Ph | 4-Pyr | $SO_2NHCH_2CONH_2$ | H | H |
| 1-111 | Ph | 4-Pyr | $SO_2NHOH$ | H | H |
| 1-112 | Ph | 4-Pyr | $SO_2NHOMe$ | H | H |
| 1-113 | Ph | 4-Pyr | $SO_2NHOEt$ | H | H |
| 1-114 | Ph | 4-Pyr | $SO_2NHOPr$ | H | H |
| 1-115 | Ph | 4-Pyr | $SO_2NHOAllyl$ | H | H |
| 1-116 | Ph | 4-Pyr | $SO_2NHOBn$ | H | H |
| 1-117 | Ph | 4-Pyr | $SO_2NHNH_2$ | H | H |
| 1-118 | Ph | 4-Pyr | $SO_2NHNHMe$ | H | H |
| 1-119 | Ph | 4-Pyr | $SO_2NHN(Me)_2$ | H | H |
| 1-120 | 3-F-Ph | 4-Pyr | COOH | H | H |
| 1-121 | 3-F-Ph | 4-Pyr | COOMe | H | H |
| 1-122 | 3-F-Ph | 4-Pyr | COOEt | H | H |
| 1-123 | 3-F-Ph | 4-Pyr | COOPr | H | H |
| 1-124 | 3-F-Ph | 4-Pyr | COO-i-Pr | H | H |
| 1-125 | 3-F-Ph | 4-Pyr | COOBu | H | H |
| 1-126 | 3-F-Ph | 4-Pyr | COOBn | H | H |
| 1-127 | 3-F-Ph | 4-Pyr | COOPh | H | H |
| 1-128 | 3-F-Ph | 4-Pyr | $CONH_2$ | H | H |
| 1-129 | 3-F-Ph | 4-Pyr | CONHMe | H | H |
| 1-130 | 3-F-Ph | 4-Pyr | CONHEt | H | H |
| 1-131 | 3-F-Ph | 4-Pyr | CONHPr | H | H |
| 1-132 | 3-F-Ph | 4-Pyr | CONH-i-Pr | H | H |
| 1-133 | 3-F-Ph | 4-Pyr | CONHBu | H | H |
| 1-134 | 3-F-Ph | 4-Pyr | CONHBn | H | H |

| | | | | | |
|---|---|---|---|---|---|
| 1-135 | 3-F-Ph | 4-Pyr | $CONMe_2$ | H | H |
| 1-136 | 3-F-Ph | 4-Pyr | CONH-c-Pr | H | H |
| 1-137 | 3-F-Ph | 4-Pyr | CONH-c-Bu | H | H |
| 1-138 | 3-F-Ph | 4-Pyr | CONH-c-Pen | H | H |
| 1-139 | 3-F-Ph | 4-Pyr | CONH-c-Hex | H | H |
| 1-140 | 3-F-Ph | 4-Pyr | CONH-c-Hep | H | H |
| 1-141 | 3-F-Ph | 4-Pyr | $CONHCH_2$-c-Pr | H | H |
| 1-142 | 3-F-Ph | 4-Pyr | CONH-Allyl | H | H |
| 1-143 | 3-F-Ph | 4-Pyr | CONH-Propargyl | H | H |
| 1-144 | 3-F-Ph | 4-Pyr | CONHPh | H | H |
| 1-145 | 3-F-Ph | 4-Pyr | CONH-3-Pyr | H | H |
| 1-146 | 3-F-Ph | 4-Pyr | $CONHCH_2$-4-Pyr | H | H |
| 1-147 | 3-F-Ph | 4-Pyr | CONH-$(CH_2)_2$-OH | H | H |
| 1-148 | 3-F-Ph | 4-Pyr | $CONHCH(CH_2OH)_2$ | H | H |
| 1-149 | 3-F-Ph | 4-Pyr | CONH-$(CH_2)_2$-OAc | H | H |
| 1-150 | 3-F-Ph | 4-Pyr | $CONHCH_2CN$ | H | H |
| 1-151 | 3-F-Ph | 4-Pyr | CONH-$(CH_2)_2$-F | H | H |
| 1-152 | 3-F-Ph | 4-Pyr | CONH-$(CH_2)_2$-OMe | H | H |
| 1-153 | 3-F-Ph | 4-Pyr | CONH-$(CH_2)_2$-SMe | H | H |
| 1-154 | 3-F-Ph | 4-Pyr | CONH-$(CH_2)_2$-$NH_2$ | H | H |
| 1-155 | 3-F-Ph | 4-Pyr | CONH-$(CH_2)_2$-NHMe | H | H |
| 1-156 | 3-F-Ph | 4-Pyr | CONH-$(CH_2)_2$-$N(Me)_2$ | H | H |
| 1-157 | 3-F-Ph | 4-Pyr | $CONHCH_2COOH$ | H | H |
| 1-158 | 3-F-Ph | 4-Pyr | $CONHCH_2COOEt$ | H | H |
| 1-159 | 3-F-Ph | 4-Pyr | CONHCH(Me)COOEt | H | H |
| 1-160 | 3-F-Ph | 4-Pyr | $CONHCH_2CONH_2$ | H | H |
| 1-161 | 3-F-Ph | 4-Pyr | CONHOH | H | H |
| 1-162 | 3-F-Ph | 4-Pyr | CONHOMe | H | H |
| 1-163 | 3-F-Ph | 4-Pyr | CONHOEt | H | H |
| 1-164 | 3-F-Ph | 4-Pyr | CONHOPr | H | H |
| 1-165 | 3-F-Ph | 4-Pyr | CONHOAllyl | H | H |
| 1-166 | 3-F-Ph | 4-Pyr | CONHOBn | H | H |
| 1-167 | 3-F-Ph | 4-Pyr | $CONHNH_2$ | H | H |
| 1-168 | 3-F-Ph | 4-Pyr | CONHNHMe | H | H |
| 1-169 | 3-F-Ph | 4-Pyr | $CONHN(Me)_2$ | H | H |
| 1-170 | 3-F-Ph | 4-Pyr | COMe | H | H |

| | | | | | |
|---|---|---|---|---|---|
| 1-171 | 3-F-Ph | 4-Pyr | COEt | H | H |
| 1-172 | 3-F-Ph | 4-Pyr | COPr | H | H |
| 1-173 | 3-F-Ph | 4-Pyr | CO-i-Pr | H | H |
| 1-174 | 3-F-Ph | 4-Pyr | COBu | H | H |
| 1-175 | 3-F-Ph | 4-Pyr | $COCF_3$ | H | H |
| 1-176 | 3-F-Ph | 4-Pyr | $CO-(CH_2)_2-F$ | H | H |
| 1-177 | 3-F-Ph | 4-Pyr | $CO-(CH_2)_2-OH$ | H | H |
| 1-178 | 3-F-Ph | 4-Pyr | $CO-(CH_2)_2-OMe$ | H | H |
| 1-179 | 3-F-Ph | 4-Pyr | SOMe | H | H |
| 1-180 | 3-F-Ph | 4-Pyr | SOEt | H | H |
| 1-181 | 3-F-Ph | 4-Pyr | SOPr | H | H |
| 1-182 | 3-F-Ph | 4-Pyr | SO-i-Pr | H | H |
| 1-183 | 3-F-Ph | 4-Pyr | SOBu | H | H |
| 1-184 | 3-F-Ph | 4-Pyr | $SOCF_3$ | H | H |
| 1-185 | 3-F-Ph | 4-Pyr | $SO-(CH_2)_2-F$ | H | H |
| 1-186 | 3-F-Ph | 4-Pyr | $SO-(CH_2)_2-OH$ | H | H |
| 1-187 | 3-F-Ph | 4-Pyr | $SO-(CH_2)_2-OMe$ | H | H |
| 1-188 | 3-F-Ph | 4-Pyr | $SO_2Me$ | H | H |
| 1-189 | 3-F-Ph | 4-Pyr | $SO_2Et$ | H | H |
| 1-190 | 3-F-Ph | 4-Pyr | $SO_2Pr$ | H | H |
| 1-191 | 3-F-Ph | 4-Pyr | $SO_2$-i-Pr | H | H |
| 1-192 | 3-F-Ph | 4-Pyr | $SO_2Bu$ | H | H |
| 1-193 | 3-F-Ph | 4-Pyr | $SO_2CF_3$ | H | H |
| 1-194 | 3-F-Ph | 4-Pyr | $SO_2-(CH_2)_2-F$ | H | H |
| 1-195 | 3-F-Ph | 4-Pyr | $SO_2-(CH_2)_2-OH$ | H | H |
| 1-196 | 3-F-Ph | 4-Pyr | $SO_2-(CH_2)_2-OMe$ | H | H |
| 1-197 | 3-F-Ph | 4-Pyr | $SO_2NH_2$ | H | H |
| 1-198 | 3-F-Ph | 4-Pyr | $SO_2NHMe$ | H | H |
| 1-199 | 3-F-Ph | 4-Pyr | $SO_2NHEt$ | H | H |
| 1-200 | 3-F-Ph | 4-Pyr | $SO_2NHPr$ | H | H |
| 1-201 | 3-F-Ph | 4-Pyr | $SO_2NH$-i-Pr | H | H |
| 1-202 | 3-F-Ph | 4-Pyr | $SO_2NHBu$ | H | H |
| 1-203 | 3-F-Ph | 4-Pyr | $SO_2NHBn$ | H | H |
| 1-204 | 3-F-Ph | 4-Pyr | $SO_2NMe_2$ | H | H |
| 1-205 | 3-F-Ph | 4-Pyr | $SO_2NH$-c-Pr | H | H |
| 1-206 | 3-F-Ph | 4-Pyr | $SO_2NH$-c-Bu | H | H |

| | | | | | |
|---|---|---|---|---|---|
| 1-207 | 3-F-Ph | 4-Pyr | $SO_2NH$-c-Pen | H | H |
| 1-208 | 3-F-Ph | 4-Pyr | $SO_2NH$-c-Hex | H | H |
| 1-209 | 3-F-Ph | 4-Pyr | $SO_2NH$-c-Hep | H | H |
| 1-210 | 3-F-Ph | 4-Pyr | $SO_2NHCH_2$-c-Pr | H | H |
| 1-211 | 3-F-Ph | 4-Pyr | $SO_2NH$-Allyl | H | H |
| 1-212 | 3-F-Ph | 4-Pyr | $SO_2NH$-Propargyl | H | H |
| 1-213 | 3-F-Ph | 4-Pyr | $SO_2NHPh$ | H | H |
| 1-214 | 3-F-Ph | 4-Pyr | $SO_2NH$-3-Pyr | H | H |
| 1-215 | 3-F-Ph | 4-Pyr | $SO_2NHCH_2$-4-Pyr | H | H |
| 1-216 | 3-F-Ph | 4-Pyr | $SO_2NH-(CH_2)_2-OH$ | H | H |
| 1-217 | 3-F-Ph | 4-Pyr | $SO_2NHCH(CH_2OH)_2$ | H | H |
| 1-218 | 3-F-Ph | 4-Pyr | $SO_2NH-(CH_2)_2-OAc$ | H | H |
| 1-219 | 3-F-Ph | 4-Pyr | $SO_2NHCH_2CN$ | H | H |
| 1-220 | 3-F-Ph | 4-Pyr | $SO_2NH-(CH_2)_2-F$ | H | H |
| 1-221 | 3-F-Ph | 4-Pyr | $SO_2NH-(CH_2)_2-OMe$ | H | H |
| 1-222 | 3-F-Ph | 4-Pyr | $SO_2NH-(CH_2)_2-SMe$ | H | H |
| 1-223 | 3-F-Ph | 4-Pyr | $SO_2NH-(CH_2)_2-NH_2$ | H | H |
| 1-224 | 3-F-Ph | 4-Pyr | $SO_2NH-(CH_2)_2-NHMe$ | H | H |
| 1-225 | 3-F-Ph | 4-Pyr | $SO_2NH-(CH_2)_2-N(Me)_2$ | H | H |
| 1-226 | 3-F-Ph | 4-Pyr | $SO_2NHCH_2COOH$ | H | H |
| 1-227 | 3-F-Ph | 4-Pyr | $SO_2NHCH_2COOEt$ | H | H |
| 1-228 | 3-F-Ph | 4-Pyr | $SO_2NHCH(Me)COOEt$ | H | H |
| 1-229 | 3-F-Ph | 4-Pyr | $SO_2NHCH_2CONH_2$ | H | H |
| 1-230 | 3-F-Ph | 4-Pyr | $SO_2NHOH$ | H | H |
| 1-231 | 3-F-Ph | 4-Pyr | $SO_2NHOMe$ | H | H |
| 1-232 | 3-F-Ph | 4-Pyr | $SO_2NHOEt$ | H | H |
| 1-233 | 3-F-Ph | 4-Pyr | $SO_2NHOPr$ | H | H |
| 1-234 | 3-F-Ph | 4-Pyr | $SO_2NHOAllyl$ | H | H |
| 1-235 | 3-F-Ph | 4-Pyr | $SO_2NHOBn$ | H | H |
| 1-236 | 3-F-Ph | 4-Pyr | $SO_2NHNH_2$ | H | H |
| 1-237 | 3-F-Ph | 4-Pyr | $SO_2NHNHMe$ | H | H |
| 1-238 | 3-F-Ph | 4-Pyr | $SO_2NHN(Me)_2$ | H | H |
| 1-239 | 3-Cl-Ph | 4-Pyr | COOH | H | H |
| 1-240 | 3-Cl-Ph | 4-Pyr | COOMe | H | H |
| 1-241 | 3-Cl-Ph | 4-Pyr | COOEt | H | H |
| 1-242 | 3-Cl-Ph | 4-Pyr | COOPr | H | H |

| | | | | | |
|---|---|---|---|---|---|
| 1-243 | 3-Cl-Ph | 4-Pyr | COO-i-Pr | H | H |
| 1-244 | 3-Cl-Ph | 4-Pyr | COOBu | H | H |
| 1-245 | 3-Cl-Ph | 4-Pyr | COOBn | H | H |
| 1-246 | 3-Cl-Ph | 4-Pyr | COOPh | H | H |
| 1-247 | 3-Cl-Ph | 4-Pyr | $CONH_2$ | H | H |
| 1-248 | 3-Cl-Ph | 4-Pyr | CONHMe | H | H |
| 1-249 | 3-Cl-Ph | 4-Pyr | CONHEt | H | H |
| 1-250 | 3-Cl-Ph | 4-Pyr | CONHPr | H | H |
| 1-251 | 3-Cl-Ph | 4-Pyr | CONH-i-Pr | H | H |
| 1-252 | 3-Cl-Ph | 4-Pyr | CONHBu | H | H |
| 1-253 | 3-Cl-Ph | 4-Pyr | CONHBn | H | H |
| 1-254 | 3-Cl-Ph | 4-Pyr | $CONMe_2$ | H | H |
| 1-255 | 3-Cl-Ph | 4-Pyr | CONH-c-Pr | H | H |
| 1-256 | 3-Cl-Ph | 4-Pyr | CONH-c-Bu | H | H |
| 1-257 | 3-Cl-Ph | 4-Pyr | CONH-c-Pen | H | H |
| 1-258 | 3-Cl-Ph | 4-Pyr | CONH-c-Hex | H | H |
| 1-259 | 3-Cl-Ph | 4-Pyr | CONH-c-Hep | H | H |
| 1-260 | 3-Cl-Ph | 4-Pyr | $CONHCH_2$-c-Pr | H | H |
| 1-261 | 3-Cl-Ph | 4-Pyr | CONH-Allyl | H | H |
| 1-262 | 3-Cl-Ph | 4-Pyr | CONH-Propargyl | H | H |
| 1-263 | 3-Cl-Ph | 4-Pyr | CONHPh | H | H |
| 1-264 | 3-Cl-Ph | 4-Pyr | CONH-3-Pyr | H | H |
| 1-265 | 3-Cl-Ph | 4-Pyr | $CONHCH_2$-4-Pyr | H | H |
| 1-266 | 3-Cl-Ph | 4-Pyr | CONH-$(CH_2)_2$-OH | H | H |
| 1-267 | 3-Cl-Ph | 4-Pyr | $CONHCH(CH_2OH)_2$ | H | H |
| 1-268 | 3-Cl-Ph | 4-Pyr | CONH-$(CH_2)_2$-OAc | H | H |
| 1-269 | 3-Cl-Ph | 4-Pyr | $CONHCH_2CN$ | H | H |
| 1-270 | 3-Cl-Ph | 4-Pyr | CONH-$(CH_2)_2$-F | H | H |
| 1-271 | 3-Cl-Ph | 4-Pyr | CONH-$(CH_2)_2$-OMe | H | H |
| 1-272 | 3-Cl-Ph | 4-Pyr | CONH-$(CH_2)_2$-SMe | H | H |
| 1-273 | 3-Cl-Ph | 4-Pyr | CONH-$(CH_2)_2$-$NH_2$ | H | H |
| 1-274 | 3-Cl-Ph | 4-Pyr | CONH-$(CH_2)_2$-NHMe | H | H |
| 1-275 | 3-Cl-Ph | 4-Pyr | CONH-$(CH_2)_2$-$N(Me)_2$ | H | H |
| 1-276 | 3-Cl-Ph | 4-Pyr | $CONHCH_2COOH$ | H | H |
| 1-277 | 3-Cl-Ph | 4-Pyr | $CONHCH_2COOEt$ | H | H |
| 1-278 | 3-Cl-Ph | 4-Pyr | CONHCH(Me)COOEt | H | H |

| | | | | | |
|---|---|---|---|---|---|
| 1-279 | 3-Cl-Ph | 4-Pyr | $CONHCH_2CONH_2$ | H | H |
| 1-280 | 3-Cl-Ph | 4-Pyr | CONHOH | H | H |
| 1-281 | 3-Cl-Ph | 4-Pyr | CONHOMe | H | H |
| 1-282 | 3-Cl-Ph | 4-Pyr | CONHOEt | H | H |
| 1-283 | 3-Cl-Ph | 4-Pyr | CONHOPr | H | H |
| 1-284 | 3-Cl-Ph | 4-Pyr | CONHOAllyl | H | H |
| 1-285 | 3-Cl-Ph | 4-Pyr | CONHOBn | H | H |
| 1-286 | 3-Cl-Ph | 4-Pyr | $CONHNH_2$ | H | H |
| 1-287 | 3-Cl-Ph | 4-Pyr | CONHNHMe | H | H |
| 1-288 | 3-Cl-Ph | 4-Pyr | $CONHN(Me)_2$ | H | H |
| 1-289 | 3-Cl-Ph | 4-Pyr | COMe | H | H |
| 1-290 | 3-Cl-Ph | 4-Pyr | COEt | H | H |
| 1-291 | 3-Cl-Ph | 4-Pyr | COPr | H | H |
| 1-292 | 3-Cl-Ph | 4-Pyr | CO-i-Pr | H | H |
| 1-293 | 3-Cl-Ph | 4-Pyr | COBu | H | H |
| 1-294 | 3-Cl-Ph | 4-Pyr | $COCF_3$ | H | H |
| 1-295 | 3-Cl-Ph | 4-Pyr | $CO-(CH_2)_2-F$ | H | H |
| 1-296 | 3-Cl-Ph | 4-Pyr | $CO-(CH_2)_2-OH$ | H | H |
| 1-297 | 3-Cl-Ph | 4-Pyr | $CO-(CH_2)_2-OMe$ | H | H |
| 1-298 | 3-Cl-Ph | 4-Pyr | SOMe | H | H |
| 1-299 | 3-Cl-Ph | 4-Pyr | SOEt | H | H |
| 1-300 | 3-Cl-Ph | 4-Pyr | SOPr | H | H |
| 1-301 | 3-Cl-Ph | 4-Pyr | SO-i-Pr | H | H |
| 1-302 | 3-Cl-Ph | 4-Pyr | SOBu | H | H |
| 1-303 | 3-Cl-Ph | 4-Pyr | $SOCF_3$ | H | H |
| 1-304 | 3-Cl-Ph | 4-Pyr | $SO-(CH_2)_2-F$ | H | H |
| 1-305 | 3-Cl-Ph | 4-Pyr | $SO-(CH_2)_2-OH$ | H | H |
| 1-306 | 3-Cl-Ph | 4-Pyr | $SO-(CH_2)_2-OMe$ | H | H |
| 1-307 | 3-Cl-Ph | 4-Pyr | $SO_2Me$ | H | H |
| 1-308 | 3-Cl-Ph | 4-Pyr | $SO_2Et$ | H | H |
| 1-309 | 3-Cl-Ph | 4-Pyr | $SO_2Pr$ | H | H |
| 1-310 | 3-Cl-Ph | 4-Pyr | $SO_2$-i-Pr | H | H |
| 1-311 | 3-Cl-Ph | 4-Pyr | $SO_2Bu$ | H | H |
| 1-312 | 3-Cl-Ph | 4-Pyr | $SO_2CF_3$ | H | H |
| 1-313 | 3-Cl-Ph | 4-Pyr | $SO_2-(CH_2)_2-F$ | H | H |
| 1-314 | 3-Cl-Ph | 4-Pyr | $SO_2-(CH_2)_2-OH$ | H | H |

| | | | | | |
|---|---|---|---|---|---|
| 1-315 | 3-Cl-Ph | 4-Pyr | $SO_2$-$(CH_2)_2$-OMe | H | H |
| 1-316 | 3-Cl-Ph | 4-Pyr | $SO_2NH_2$ | H | H |
| 1-317 | 3-Cl-Ph | 4-Pyr | $SO_2$NHMe | H | H |
| 1-318 | 3-Cl-Ph | 4-Pyr | $SO_2$NHEt | H | H |
| 1-319 | 3-Cl-Ph | 4-Pyr | $SO_2$NHPr | H | H |
| 1-320 | 3-Cl-Ph | 4-Pyr | $SO_2$NH-i-Pr | H | H |
| 1-321 | 3-Cl-Ph | 4-Pyr | $SO_2$NHBu | H | H |
| 1-322 | 3-Cl-Ph | 4-Pyr | $SO_2$NHBn | H | H |
| 1-323 | 3-Cl-Ph | 4-Pyr | $SO_2NMe_2$ | H | H |
| 1-324 | 3-Cl-Ph | 4-Pyr | $SO_2$NH-c-Pr | H | H |
| 1-325 | 3-Cl-Ph | 4-Pyr | $SO_2$NH-c-Bu | H | H |
| 1-326 | 3-Cl-Ph | 4-Pyr | $SO_2$NH-c-Pen | H | H |
| 1-327 | 3-Cl-Ph | 4-Pyr | $SO_2$NH-c-Hex | H | H |
| 1-328 | 3-Cl-Ph | 4-Pyr | $SO_2$NH-c-Hep | H | H |
| 1-329 | 3-Cl-Ph | 4-Pyr | $SO_2NHCH_2$-c-Pr | H | H |
| 1-330 | 3-Cl-Ph | 4-Pyr | $SO_2$NH-Allyl | H | H |
| 1-331 | 3-Cl-Ph | 4-Pyr | $SO_2$NH-Propargyl | H | H |
| 1-332 | 3-Cl-Ph | 4-Pyr | $SO_2$NHPh | H | H |
| 1-333 | 3-Cl-Ph | 4-Pyr | $SO_2$NH-3-Pyr | H | H |
| 1-334 | 3-Cl-Ph | 4-Pyr | $SO_2NHCH_2$-4-Pyr | H | H |
| 1-335 | 3-Cl-Ph | 4-Pyr | $SO_2$NH-$(CH_2)_2$-OH | H | H |
| 1-336 | 3-Cl-Ph | 4-Pyr | $SO_2NHCH(CH_2OH)_2$ | H | H |
| 1-337 | 3-Cl-Ph | 4-Pyr | $SO_2$NH-$(CH_2)_2$-OAc | H | H |
| 1-338 | 3-Cl-Ph | 4-Pyr | $SO_2NHCH_2CN$ | H | H |
| 1-339 | 3-Cl-Ph | 4-Pyr | $SO_2$NH-$(CH_2)_2$-F | H | H |
| 1-340 | 3-Cl-Ph | 4-Pyr | $SO_2$NH-$(CH_2)_2$-OMe | H | H |
| 1-341 | 3-Cl-Ph | 4-Pyr | $SO_2$NH-$(CH_2)_2$-SMe | H | H |
| 1-342 | 3-Cl-Ph | 4-Pyr | $SO_2$NH-$(CH_2)_2$-$NH_2$ | H | H |
| 1-343 | 3-Cl-Ph | 4-Pyr | $SO_2$NH-$(CH_2)_2$-NHMe | H | H |
| 1-344 | 3-Cl-Ph | 4-Pyr | $SO_2$NH-$(CH_2)_2$-$N(Me)_2$ | H | H |
| 1-345 | 3-Cl-Ph | 4-Pyr | $SO_2NHCH_2COOH$ | H | H |
| 1-346 | 3-Cl-Ph | 4-Pyr | $SO_2NHCH_2COOEt$ | H | H |
| 1-347 | 3-Cl-Ph | 4-Pyr | $SO_2$NHCH(Me)COOEt | H | H |
| 1-348 | 3-Cl-Ph | 4-Pyr | $SO_2NHCH_2CONH_2$ | H | H |
| 1-349 | 3-Cl-Ph | 4-Pyr | $SO_2$NHOH | H | H |
| 1-350 | 3-Cl-Ph | 4-Pyr | $SO_2$NHOMe | H | H |

| | | | | | |
|---|---|---|---|---|---|
| 1-351 | 3-Cl-Ph | 4-Pyr | $SO_2NHOEt$ | H | H |
| 1-352 | 3-Cl-Ph | 4-Pyr | $SO_2NHOPr$ | H | H |
| 1-353 | 3-Cl-Ph | 4-Pyr | $SO_2NHOAllyl$ | H | H |
| 1-354 | 3-Cl-Ph | 4-Pyr | $SO_2NHOBn$ | H | H |
| 1-355 | 3-Cl-Ph | 4-Pyr | $SO_2NHNH_2$ | H | H |
| 1-356 | 3-Cl-Ph | 4-Pyr | $SO_2NHNHMe$ | H | H |
| 1-357 | 3-Cl-Ph | 4-Pyr | $SO_2NHN(Me)_2$ | H | H |
| 1-358 | 4-F-Ph | 4-Pyr | COOH | H | H |
| 1-359 | 4-F-Ph | 4-Pyr | COOMe | H | H |
| 1-360 | 4-F-Ph | 4-Pyr | COOEt | H | H |
| 1-361 | 4-F-Ph | 4-Pyr | COOPr | H | H |
| 1-362 | 4-F-Ph | 4-Pyr | COO-i-Pr | H | H |
| 1-363 | 4-F-Ph | 4-Pyr | COOBu | H | H |
| 1-364 | 4-F-Ph | 4-Pyr | COOBn | H | H |
| 1-365 | 4-F-Ph | 4-Pyr | COOPh | H | H |
| 1-366 | 4-F-Ph | 4-Pyr | $CONH_2$ | H | H |
| 1-367 | 4-F-Ph | 4-Pyr | CONHMe | H | H |
| 1-368 | 4-F-Ph | 4-Pyr | CONHEt | H | H |
| 1-369 | 4-F-Ph | 4-Pyr | CONHPr | H | H |
| 1-370 | 4-F-Ph | 4-Pyr | CONH-i-Pr | H | H |
| 1-371 | 4-F-Ph | 4-Pyr | CONHBu | H | H |
| 1-372 | 4-F-Ph | 4-Pyr | CONHBn | H | H |
| 1-373 | 4-F-Ph | 4-Pyr | $CONMe_2$ | H | H |
| 1-374 | 4-F-Ph | 4-Pyr | CONH-c-Pr | H | H |
| 1-375 | 4-F-Ph | 4-Pyr | CONH-c-Bu | H | H |
| 1-376 | 4-F-Ph | 4-Pyr | CONH-c-Pen | H | H |
| 1-377 | 4-F-Ph | 4-Pyr | CONH-c-Hex | H | H |
| 1-378 | 4-F-Ph | 4-Pyr | CONH-c-Hep | H | H |
| 1-379 | 4-F-Ph | 4-Pyr | $CONHCH_2$-c-Pr | H | H |
| 1-380 | 4-F-Ph | 4-Pyr | CONH-Allyl | H | H |
| 1-381 | 4-F-Ph | 4-Pyr | CONH-Propargyl | H | H |
| 1-382 | 4-F-Ph | 4-Pyr | CONHPh | H | H |
| 1-383 | 4-F-Ph | 4-Pyr | CONH-3-Pyr | H | H |
| 1-384 | 4-F-Ph | 4-Pyr | $CONHCH_2$-4-Pyr | H | H |
| 1-385 | 4-F-Ph | 4-Pyr | CONH-$(CH_2)_2$-OH | H | H |
| 1-386 | 4-F-Ph | 4-Pyr | $CONHCH(CH_2OH)_2$ | H | H |

| | | | | | |
|---|---|---|---|---|---|
| 1-387 | 4-F-Ph | 4-Pyr | CONH-$(CH_2)_2$-OAc | H | H |
| 1-388 | 4-F-Ph | 4-Pyr | $CONHCH_2CN$ | H | H |
| 1-389 | 4-F-Ph | 4-Pyr | CONH-$(CH_2)_2$-F | H | H |
| 1-390 | 4-F-Ph | 4-Pyr | CONH-$(CH_2)_2$-OMe | H | H |
| 1-391 | 4-F-Ph | 4-Pyr | CONH-$(CH_2)_2$-SMe | H | H |
| 1-392 | 4-F-Ph | 4-Pyr | CONH-$(CH_2)_2$-$NH_2$ | H | H |
| 1-393 | 4-F-Ph | 4-Pyr | CONH-$(CH_2)_2$-NHMe | H | H |
| 1-394 | 4-F-Ph | 4-Pyr | CONH-$(CH_2)_2$-N$(Me)_2$ | H | H |
| 1-395 | 4-F-Ph | 4-Pyr | $CONHCH_2COOH$ | H | H |
| 1-396 | 4-F-Ph | 4-Pyr | $CONHCH_2COOEt$ | H | H |
| 1-397 | 4-F-Ph | 4-Pyr | CONHCH(Me)COOEt | H | H |
| 1-398 | 4-F-Ph | 4-Pyr | $CONHCH_2CONH_2$ | H | H |
| 1-399 | 4-F-Ph | 4-Pyr | CONHOH | H | H |
| 1-400 | 4-F-Ph | 4-Pyr | CONHOMe | H | H |
| 1-401 | 4-F-Ph | 4-Pyr | CONHOEt | H | H |
| 1-402 | 4-F-Ph | 4-Pyr | CONHOPr | H | H |
| 1-403 | 4-F-Ph | 4-Pyr | CONHOAllyl | H | H |
| 1-404 | 4-F-Ph | 4-Pyr | CONHOBn | H | H |
| 1-405 | 4-F-Ph | 4-Pyr | $CONHNH_2$ | H | H |
| 1-406 | 4-F-Ph | 4-Pyr | CONHNHMe | H | H |
| 1-407 | 4-F-Ph | 4-Pyr | CONHN$(Me)_2$ | H | H |
| 1-408 | 4-F-Ph | 4-Pyr | COMe | H | H |
| 1-409 | 4-F-Ph | 4-Pyr | COEt | H | H |
| 1-410 | 4-F-Ph | 4-Pyr | COPr | H | H |
| 1-411 | 4-F-Ph | 4-Pyr | CO-i-Pr | H | H |
| 1-412 | 4-F-Ph | 4-Pyr | COBu | H | H |
| 1-413 | 4-F-Ph | 4-Pyr | $COCF_3$ | H | H |
| 1-414 | 4-F-Ph | 4-Pyr | CO-$(CH_2)_2$-F | H | H |
| 1-415 | 4-F-Ph | 4-Pyr | CO-$(CH_2)_2$-OH | H | H |
| 1-416 | 4-F-Ph | 4-Pyr | CO-$(CH_2)_2$-OMe | H | H |
| 1-417 | 4-F-Ph | 4-Pyr | SOMe | H | H |
| 1-418 | 4-F-Ph | 4-Pyr | SOEt | H | H |
| 1-419 | 4-F-Ph | 4-Pyr | SOPr | H | H |
| 1-420 | 4-F-Ph | 4-Pyr | SO-i-Pr | H | H |
| 1-421 | 4-F-Ph | 4-Pyr | SOBu | H | H |
| 1-422 | 4-F-Ph | 4-Pyr | $SOCF_3$ | H | H |

| | | | | | |
|---|---|---|---|---|---|
| 1-423 | 4-F-Ph | 4-Pyr | SO-$(CH_2)_2$-F | H | H |
| 1-424 | 4-F-Ph | 4-Pyr | SO-$(CH_2)_2$-OH | H | H |
| 1-425 | 4-F-Ph | 4-Pyr | SO-$(CH_2)_2$-OMe | H | H |
| 1-426 | 4-F-Ph | 4-Pyr | $SO_2Me$ | H | H |
| 1-427 | 4-F-Ph | 4-Pyr | $SO_2Et$ | H | H |
| 1-428 | 4-F-Ph | 4-Pyr | $SO_2Pr$ | H | H |
| 1-429 | 4-F-Ph | 4-Pyr | $SO_2$-i-Pr | H | H |
| 1-430 | 4-F-Ph | 4-Pyr | $SO_2Bu$ | H | H |
| 1-431 | 4-F-Ph | 4-Pyr | $SO_2CF_3$ | H | H |
| 1-432 | 4-F-Ph | 4-Pyr | $SO_2$-$(CH_2)_2$-F | H | H |
| 1-433 | 4-F-Ph | 4-Pyr | $SO_2$-$(CH_2)_2$-OH | H | H |
| 1-434 | 4-F-Ph | 4-Pyr | $SO_2$-$(CH_2)_2$-OMe | H | H |
| 1-435 | 4-F-Ph | 4-Pyr | $SO_2NH_2$ | H | H |
| 1-436 | 4-F-Ph | 4-Pyr | $SO_2NHMe$ | H | H |
| 1-437 | 4-F-Ph | 4-Pyr | $SO_2NHEt$ | H | H |
| 1-438 | 4-F-Ph | 4-Pyr | $SO_2NHPr$ | H | H |
| 1-439 | 4-F-Ph | 4-Pyr | $SO_2NH$-i-Pr | H | H |
| 1-440 | 4-F-Ph | 4-Pyr | $SO_2NHBu$ | H | H |
| 1-441 | 4-F-Ph | 4-Pyr | $SO_2NHBn$ | H | H |
| 1-442 | 4-F-Ph | 4-Pyr | $SO_2NMe_2$ | H | H |
| 1-443 | 4-F-Ph | 4-Pyr | $SO_2NH$-c-Pr | H | H |
| 1-444 | 4-F-Ph | 4-Pyr | $SO_2NH$-c-Bu | H | H |
| 1-445 | 4-F-Ph | 4-Pyr | $SO_2NH$-c-Pen | H | H |
| 1-446 | 4-F-Ph | 4-Pyr | $SO_2NH$-c-Hex | H | H |
| 1-447 | 4-F-Ph | 4-Pyr | $SO_2NH$-c-Hep | H | H |
| 1-448 | 4-F-Ph | 4-Pyr | $SO_2NHCH_2$-c-Pr | H | H |
| 1-449 | 4-F-Ph | 4-Pyr | $SO_2NH$-Allyl | H | H |
| 1-450 | 4-F-Ph | 4-Pyr | $SO_2NH$-Propargyl | H | H |
| 1-451 | 4-F-Ph | 4-Pyr | $SO_2NHPh$ | H | H |
| 1-452 | 4-F-Ph | 4-Pyr | $SO_2NH$-3-Pyr | H | H |
| 1-453 | 4-F-Ph | 4-Pyr | $SO_2NHCH_2$-3-Pyr | H | H |
| 1-454 | 4-F-Ph | 4-Pyr | $SO_2NH$-$(CH_2)_2$-OH | H | H |
| 1-455 | 4-F-Ph | 4-Pyr | $SO_2NHCH(CH_2OH)_2$ | H | H |
| 1-456 | 4-F-Ph | 4-Pyr | $SO_2NH$-$(CH_2)_2$-OAc | H | H |
| 1-457 | 4-F-Ph | 4-Pyr | $SO_2NHCH_2CN$ | H | H |
| 1-458 | 4-F-Ph | 4-Pyr | $SO_2NH$-$(CH_2)_2$-F | H | H |

| | | | | | |
|---|---|---|---|---|---|
| 1-459 | 4-F-Ph | 4-Pyr | $SO_2NH-(CH_2)_2-OMe$ | H | H |
| 1-460 | 4-F-Ph | 4-Pyr | $SO_2NH-(CH_2)_2-SMe$ | H | H |
| 1-461 | 4-F-Ph | 4-Pyr | $SO_2NH-(CH_2)_2-NH_2$ | H | H |
| 1-462 | 4-F-Ph | 4-Pyr | $SO_2NH-(CH_2)_2-NHMe$ | H | H |
| 1-463 | 4-F-Ph | 4-Pyr | $SO_2NH-(CH_2)_2-N(Me)_2$ | H | H |
| 1-464 | 4-F-Ph | 4-Pyr | $SO_2NHCH_2COOH$ | H | H |
| 1-465 | 4-F-Ph | 4-Pyr | $SO_2NHCH_2COOMe$ | H | H |
| 1-466 | 4-F-Ph | 4-Pyr | $SO_2NHCH(Me)COOEt$ | H | H |
| 1-467 | 4-F-Ph | 4-Pyr | $SO_2NHCH_2CONH_2$ | H | H |
| 1-468 | 4-F-Ph | 4-Pyr | $SO_2NHOH$ | H | H |
| 1-469 | 4-F-Ph | 4-Pyr | $SO_2NHOMe$ | H | H |
| 1-470 | 4-F-Ph | 4-Pyr | $SO_2NHOEt$ | H | H |
| 1-471 | 4-F-Ph | 4-Pyr | $SO_2NHOPr$ | H | H |
| 1-472 | 4-F-Ph | 4-Pyr | $SO_2NHOAllyl$ | H | H |
| 1-473 | 4-F-Ph | 4-Pyr | $SO_2NHOBn$ | H | H |
| 1-474 | 4-F-Ph | 4-Pyr | $SO_2NHNH_2$ | H | H |
| 1-475 | 4-F-Ph | 4-Pyr | $SO_2NHNHMe$ | H | H |
| 1-476 | 4-F-Ph | 4-Pyr | $SO_2NHN(Me)_2$ | H | H |
| 1-477 | 3-Cl-4-F-Ph | 4-Pyr | COOH | H | H |
| 1-478 | 3-Cl-4-F-Ph | 4-Pyr | COOMe | H | H |
| 1-479 | 3-Cl-4-F-Ph | 4-Pyr | COOEt | H | H |
| 1-480 | 3-Cl-4-F-Ph | 4-Pyr | COOPr | H | H |
| 1-481 | 3-Cl-4-F-Ph | 4-Pyr | COO-i-Pr | H | H |
| 1-482 | 3-Cl-4-F-Ph | 4-Pyr | COOBu | H | H |
| 1-483 | 3-Cl-4-F-Ph | 4-Pyr | COOBn | H | H |
| 1-484 | 3-Cl-4-F-Ph | 4-Pyr | COOPh | H | H |
| 1-485 | 3-Cl-4-F-Ph | 4-Pyr | $CONH_2$ | H | H |

| | | | | | |
|---|---|---|---|---|---|
| 1-486 | 3-Cl-4-F-Ph | 4-Pyr | CONHMe | H | H |
| 1-487 | 3-Cl-4-F-Ph | 4-Pyr | CONHEt | H | H |
| 1-488 | 3-Cl-4-F-Ph | 4-Pyr | CONHPr | H | H |
| 1-489 | 3-Cl-4-F-Ph | 4-Pyr | CONH-i-Pr | H | H |
| 1-490 | 3-Cl-4-F-Ph | 4-Pyr | CONHBu | H | H |
| 1-491 | 3-Cl-4-F-Ph | 4-Pyr | CONHBn | H | H |
| 1-492 | 3-Cl-4-F-Ph | 4-Pyr | $CONMe_2$ | H | H |
| 1-493 | 3-Cl-4-F-Ph | 4-Pyr | CONH-c-Pr | H | H |
| 1-494 | 3-Cl-4-F-Ph | 4-Pyr | CONH-c-Bu | H | H |
| 1-495 | 3-Cl-4-F-Ph | 4-Pyr | CONH-c-Pen | H | H |
| 1-496 | 3-Cl-4-F-Ph | 4-Pyr | CONH-c-Hex | H | H |
| 1-497 | 3-Cl-4-F-Ph | 4-Pyr | CONH-c-Hep | H | H |
| 1-498 | 3-Cl-4-F-Ph | 4-Pyr | $CONHCH_2$-c-Pr | H | H |
| 1-499 | 3-Cl-4-F-Ph | 4-Pyr | CONH-Allyl | H | H |
| 1-500 | 3-Cl-4-F-Ph | 4-Pyr | CONH-Propargyl | H | H |
| 1-501 | 3-Cl-4-F-Ph | 4-Pyr | CONHPh | H | H |
| 1-502 | 3-Cl-4-F-Ph | 4-Pyr | CONH-3-Pyr | H | H |
| 1-503 | 3-Cl-4-F-Ph | 4-Pyr | $CONHCH_2$-4-Pyr | H | H |

| | | | | | |
|---|---|---|---|---|---|
| 1-504 | 3-Cl-4-F-Ph | 4-Pyr | CONH-$(CH_2)_2$-OH | H | H |
| 1-505 | 3-Cl-4-F-Ph | 4-Pyr | CONHCH$(CH_2OH)_2$ | H | H |
| 1-506 | 3-Cl-4-F-Ph | 4-Pyr | CONH-$(CH_2)_2$-OAc | H | H |
| 1-507 | 3-Cl-4-F-Ph | 4-Pyr | CONH$CH_2$CN | H | H |
| 1-508 | 3-Cl-4-F-Ph | 4-Pyr | CONH-$(CH_2)_2$-F | H | H |
| 1-509 | 3-Cl-4-F-Ph | 4-Pyr | CONH-$(CH_2)_2$-OMe | H | H |
| 1-510 | 3-Cl-4-F-Ph | 4-Pyr | CONH-$(CH_2)_2$-SMe | H | H |
| 1-511 | 3-Cl-4-F-Ph | 4-Pyr | CONH-$(CH_2)_2$-$NH_2$ | H | H |
| 1-512 | 3-Cl-4-F-Ph | 4-Pyr | CONH-$(CH_2)_2$-NHMe | H | H |
| 1-513 | 3-Cl-4-F-Ph | 4-Pyr | CONH-$(CH_2)_2$-N$(Me)_2$ | H | H |
| 1-514 | 3-Cl-4-F-Ph | 4-Pyr | CONH$CH_2$COOH | H | H |
| 1-515 | 3-Cl-4-F-Ph | 4-Pyr | CONH$CH_2$COOEt | H | H |
| 1-516 | 3-Cl-4-F-Ph | 4-Pyr | CONHCH(Me)COOEt | H | H |
| 1-517 | 3-Cl-4-F-Ph | 4-Pyr | CONH$CH_2$CON$H_2$ | H | H |
| 1-518 | 3-Cl-4-F-Ph | 4-Pyr | CONHOH | H | H |
| 1-519 | 3-Cl-4-F-Ph | 4-Pyr | CONHOMe | H | H |
| 1-520 | 3-Cl-4-F-Ph | 4-Pyr | CONHOEt | H | H |
| 1-521 | 3-Cl-4-F-Ph | 4-Pyr | CONHOPr | H | H |

| | | | | | |
|---|---|---|---|---|---|
| 1-522 | 3-Cl-4-F-Ph | 4-Pyr | CONHOAllyl | H | H |
| 1-523 | 3-Cl-4-F-Ph | 4-Pyr | CONHOBn | H | H |
| 1-524 | 3-Cl-4-F-Ph | 4-Pyr | $CONHNH_2$ | H | H |
| 1-525 | 3-Cl-4-F-Ph | 4-Pyr | CONHNHMe | H | H |
| 1-526 | 3-Cl-4-F-Ph | 4-Pyr | $CONHN(Me)_2$ | H | H |
| 1-527 | 3-Cl-4-F-Ph | 4-Pyr | COMe | H | H |
| 1-528 | 3-Cl-4-F-Ph | 4-Pyr | COEt | H | H |
| 1-529 | 3-Cl-4-F-Ph | 4-Pyr | COPr | H | H |
| 1-530 | 3-Cl-4-F-Ph | 4-Pyr | CO-i-Pr | H | H |
| 1-531 | 3-Cl-4-F-Ph | 4-Pyr | COBu | H | H |
| 1-532 | 3-Cl-4-F-Ph | 4-Pyr | $COCF_3$ | H | H |
| 1-533 | 3-Cl-4-F-Ph | 4-Pyr | $CO-(CH_2)_2-F$ | H | H |
| 1-534 | 3-Cl-4-F-Ph | 4-Pyr | $CO-(CH_2)_2-OH$ | H | H |
| 1-535 | 3-Cl-4-F-Ph | 4-Pyr | $CO-(CH_2)_2-OMe$ | H | H |
| 1-536 | 3-Cl-4-F-Ph | 4-Pyr | SOMe | H | H |
| 1-537 | 3-Cl-4-F-Ph | 4-Pyr | SOEt | H | H |
| 1-538 | 3-Cl-4-F-Ph | 4-Pyr | SOPr | H | H |
| 1-539 | 3-Cl-4-F-Ph | 4-Pyr | SO-i-Pr | H | H |

| | | | | | |
|---|---|---|---|---|---|
| 1-540 | 3-Cl-4-F-Ph | 4-Pyr | SOBu | H | H |
| 1-541 | 3-Cl-4-F-Ph | 4-Pyr | $SOCF_3$ | H | H |
| 1-542 | 3-Cl-4-F-Ph | 4-Pyr | $SO-(CH_2)_2-F$ | H | H |
| 1-543 | 3-Cl-4-F-Ph | 4-Pyr | $SO-(CH_2)_2-OH$ | H | H |
| 1-544 | 3-Cl-4-F-Ph | 4-Pyr | $SO-(CH_2)_2-OMe$ | H | H |
| 1-545 | 3-Cl-4-F-Ph | 4-Pyr | $SO_2Me$ | H | H |
| 1-546 | 3-Cl-4-F-Ph | 4-Pyr | $SO_2Et$ | H | H |
| 1-547 | 3-Cl-4-F-Ph | 4-Pyr | $SO_2Pr$ | H | H |
| 1-548 | 3-Cl-4-F-Ph | 4-Pyr | $SO_2$-i-Pr | H | H |
| 1-549 | 3-Cl-4-F-Ph | 4-Pyr | $SO_2Bu$ | H | H |
| 1-550 | 3-Cl-4-F-Ph | 4-Pyr | $SO_2CF_3$ | H | H |
| 1-551 | 3-Cl-4-F-Ph | 4-Pyr | $SO_2-(CH_2)_2-F$ | H | H |
| 1-552 | 3-Cl-4-F-Ph | 4-Pyr | $SO_2-(CH_2)_2-OH$ | H | H |
| 1-553 | 3-Cl-4-F-Ph | 4-Pyr | $SO_2-(CH_2)_2-OMe$ | H | H |
| 1-554 | 3-Cl-4-F-Ph | 4-Pyr | $SO_2NH_2$ | H | H |
| 1-555 | 3-Cl-4-F-Ph | 4-Pyr | $SO_2NHMe$ | H | H |
| 1-556 | 3-Cl-4-F-Ph | 4-Pyr | $SO_2NHEt$ | H | H |
| 1-557 | 3-Cl-4-F-Ph | 4-Pyr | $SO_2NHPr$ | H | H |

| | | | | | |
|---|---|---|---|---|---|
| 1-558 | 3-Cl-4-F-Ph | 4-Pyr | $SO_2NH$-i-Pr | H | H |
| 1-559 | 3-Cl-4-F-Ph | 4-Pyr | $SO_2NHBu$ | H | H |
| 1-560 | 3-Cl-4-F-Ph | 4-Pyr | $SO_2NHBn$ | H | H |
| 1-561 | 3-Cl-4-F-Ph | 4-Pyr | $SO_2NMe_2$ | H | H |
| 1-562 | 3-Cl-4-F-Ph | 4-Pyr | $SO_2NH$-c-Pr | H | H |
| 1-563 | 3-Cl-4-F-Ph | 4-Pyr | $SO_2NH$-c-Bu | H | H |
| 1-564 | 3-Cl-4-F-Ph | 4-Pyr | $SO_2NH$-c-Pen | H | H |
| 1-565 | 3-Cl-4-F-Ph | 4-Pyr | $SO_2NH$-c-Hex | H | H |
| 1-566 | 3-Cl-4-F-Ph | 4-Pyr | $SO_2NH$-c-Hep | H | H |
| 1-567 | 3-Cl-4-F-Ph | 4-Pyr | $SO_2NHCH_2$-c-Pr | H | H |
| 1-568 | 3-Cl-4-F-Ph | 4-Pyr | $SO_2NH$-Allyl | H | H |
| 1-569 | 3-Cl-4-F-Ph | 4-Pyr | $SO_2NH$-Propargyl | H | H |
| 1-570 | 3-Cl-4-F-Ph | 4-Pyr | $SO_2NHPh$ | H | H |
| 1-571 | 3-Cl-4-F-Ph | 4-Pyr | $SO_2NH$-3-Pyr | H | H |
| 1-572 | 3-Cl-4-F-Ph | 4-Pyr | $SO_2NHCH_2$-4-Pyr | H | H |
| 1-573 | 3-Cl-4-F-Ph | 4-Pyr | $SO_2NH-(CH_2)_2-OH$ | H | H |
| 1-574 | 3-Cl-4-F-Ph | 4-Pyr | $SO_2NHCH(CH_2OH)_2$ | H | H |
| 1-575 | 3-Cl-4-F-Ph | 4-Pyr | $SO_2NH-(CH_2)_2-OAc$ | H | H |

| | | | | | |
|---|---|---|---|---|---|
| 1-576 | 3-Cl-4-F-Ph | 4-Pyr | $SO_2NHCH_2CN$ | H | H |
| 1-577 | 3-Cl-4-F-Ph | 4-Pyr | $SO_2NH-(CH_2)_2-F$ | H | H |
| 1-578 | 3-Cl-4-F-Ph | 4-Pyr | $SO_2NH-(CH_2)_2-OMe$ | H | H |
| 1-579 | 3-Cl-4-F-Ph | 4-Pyr | $SO_2NH-(CH_2)_2-SMe$ | H | H |
| 1-580 | 3-Cl-4-F-Ph | 4-Pyr | $SO_2NH-(CH_2)_2-NH_2$ | H | H |
| 1-581 | 3-Cl-4-F-Ph | 4-Pyr | $SO_2NH-(CH_2)_2-NHMe$ | H | H |
| 1-582 | 3-Cl-4-F-Ph | 4-Pyr | $SO_2NH-(CH_2)_2-N(Me)_2$ | H | H |
| 1-583 | 3-Cl-4-F-Ph | 4-Pyr | $SO_2NHCH_2COOH$ | H | H |
| 1-584 | 3-Cl-4-F-Ph | 4-Pyr | $SO_2NHCH_2COOEt$ | H | H |
| 1-585 | 3-Cl-4-F-Ph | 4-Pyr | $SO_2NHCH(Me)COOEt$ | H | H |
| 1-586 | 3-Cl-4-F-Ph | 4-Pyr | $SO_2NHCH_2CONH_2$ | H | H |
| 1-587 | 3-Cl-4-F-Ph | 4-Pyr | $SO_2NHOH$ | H | H |
| 1-588 | 3-Cl-4-F-Ph | 4-Pyr | $SO_2NHOMe$ | H | H |
| 1-589 | 3-Cl-4-F-Ph | 4-Pyr | $SO_2NHOEt$ | H | H |
| 1-590 | 3-Cl-4-F-Ph | 4-Pyr | $SO_2NHOPr$ | H | H |
| 1-591 | 3-Cl-4-F-Ph | 4-Pyr | $SO_2NHOAllyl$ | H | H |
| 1-592 | 3-Cl-4-F-Ph | 4-Pyr | $SO_2NHOBn$ | H | H |
| 1-593 | 3-Cl-4-F-Ph | 4-Pyr | $SO_2NHNH_2$ | H | H |

| | | | | | |
|---|---|---|---|---|---|
| 1-594 | 3-Cl-4-F-Ph | 4-Pyr | $SO_2NHNHMe$ | H | H |
| 1-595 | 3-Cl-4-F-Ph | 4-Pyr | $SO_2NHN(Me)_2$ | H | H |
| 1-596 | 3,4-$F_2$-Ph | 4-Pyr | COOH | H | H |
| 1-597 | 3,4-$F_2$-Ph | 4-Pyr | COOMe | H | H |
| 1-598 | 3,4-$F_2$-Ph | 4-Pyr | COOEt | H | H |
| 1-599 | 3,4-$F_2$-Ph | 4-Pyr | COOPr | H | H |
| 1-600 | 3,4-$F_2$-Ph | 4-Pyr | COO-i-Pr | H | H |
| 1-601 | 3,4-$F_2$-Ph | 4-Pyr | COOBu | H | H |
| 1-602 | 3,4-$F_2$-Ph | 4-Pyr | COOBn | H | H |
| 1-603 | 3,4-$F_2$-Ph | 4-Pyr | COOPh | H | H |
| 1-604 | 3,4-$F_2$-Ph | 4-Pyr | $CONH_2$ | H | H |
| 1-605 | 3,4-$F_2$-Ph | 4-Pyr | CONHMe | H | H |
| 1-606 | 3,4-$F_2$-Ph | 4-Pyr | CONHEt | H | H |
| 1-607 | 3,4-$F_2$-Ph | 4-Pyr | CONHPr | H | H |
| 1-608 | 3,4-$F_2$-Ph | 4-Pyr | CONH-i-Pr | H | H |
| 1-609 | 3,4-$F_2$-Ph | 4-Pyr | CONHBu | H | H |
| 1-610 | 3,4-$F_2$-Ph | 4-Pyr | CONHBn | H | H |
| 1-611 | 3,4-$F_2$-Ph | 4-Pyr | $CONMe_2$ | H | H |

| | | | | | |
|---|---|---|---|---|---|
| 1-612 | 3,4-$F_2$-Ph | 4-Pyr | CONH-c-Pr | H | H |
| 1-613 | 3,4-$F_2$-Ph | 4-Pyr | CONH-c-Bu | H | H |
| 1-614 | 3,4-$F_2$-Ph | 4-Pyr | CONH-c-Pen | H | H |
| 1-615 | 3,4-$F_2$-Ph | 4-Pyr | CONH-c-Hex | H | H |
| 1-616 | 3,4-$F_2$-Ph | 4-Pyr | CONH-c-Hep | H | H |
| 1-617 | 3,4-$F_2$-Ph | 4-Pyr | $CONHCH_2$-c-Pr | H | H |
| 1-618 | 3,4-$F_2$-Ph | 4-Pyr | CONH-Allyl | H | H |
| 1-619 | 3,4-$F_2$-Ph | 4-Pyr | CONH-Propargyl | H | H |
| 1-620 | 3,4-$F_2$-Ph | 4-Pyr | CONHPh | H | H |
| 1-621 | 3,4-$F_2$-Ph | 4-Pyr | CONH-3-Pyr | H | H |
| 1-622 | 3,4-$F_2$-Ph | 4-Pyr | $CONHCH_2$-4-Pyr | H | H |
| 1-623 | 3,4-$F_2$-Ph | 4-Pyr | CONH-$(CH_2)_2$-OH | H | H |
| 1-624 | 3,4-$F_2$-Ph | 4-Pyr | $CONHCH(CH_2OH)_2$ | H | H |
| 1-625 | 3,4-$F_2$-Ph | 4-Pyr | CONH-$(CH_2)_2$-OAc | H | H |
| 1-626 | 3,4-$F_2$-Ph | 4-Pyr | $CONHCH_2CN$ | H | H |
| 1-627 | 3,4-$F_2$-Ph | 4-Pyr | CONH-$(CH_2)_2$-F | H | H |
| 1-628 | 3,4-$F_2$-Ph | 4-Pyr | CONH-$(CH_2)_2$-OMe | H | H |
| 1-629 | 3,4-$F_2$-Ph | 4-Pyr | CONH-$(CH_2)_2$-SMe | H | H |

| | | | | | |
|---|---|---|---|---|---|
| 1-630 | 3,4-$F_2$-Ph | 4-Pyr | CONH-$(CH_2)_2$-$NH_2$ | H | H |
| 1-631 | 3,4-$F_2$-Ph | 4-Pyr | CONH-$(CH_2)_2$-NHMe | H | H |
| 1-632 | 3,4-$F_2$-Ph | 4-Pyr | CONH-$(CH_2)_2$-N(Me)$_2$ | H | H |
| 1-633 | 3,4-$F_2$-Ph | 4-Pyr | $CONHCH_2COOH$ | H | H |
| 1-634 | 3,4-$F_2$-Ph | 4-Pyr | $CONHCH_2COOEt$ | H | H |
| 1-635 | 3,4-$F_2$-Ph | 4-Pyr | CONHCH(Me)COOEt | H | H |
| 1-636 | 3,4-$F_2$-Ph | 4-Pyr | $CONHCH_2CONH_2$ | H | H |
| 1-637 | 3,4-$F_2$-Ph | 4-Pyr | CONHOH | H | H |
| 1-638 | 3,4-$F_2$-Ph | 4-Pyr | CONHOMe | H | H |
| 1-639 | 3,4-$F_2$-Ph | 4-Pyr | CONHOEt | H | H |
| 1-640 | 3,4-$F_2$-Ph | 4-Pyr | CONHOPr | H | H |
| 1-641 | 3,4-$F_2$-Ph | 4-Pyr | CONHOAllyl | H | H |
| 1-642 | 3,4-$F_2$-Ph | 4-Pyr | CONHOBn | H | H |
| 1-643 | 3,4-$F_2$-Ph | 4-Pyr | $CONHNH_2$ | H | H |
| 1-644 | 3,4-$F_2$-Ph | 4-Pyr | CONHNHMe | H | H |
| 1-645 | 3,4-$F_2$-Ph | 4-Pyr | CONHN(Me)$_2$ | H | H |
| 1-646 | 3,4-$F_2$-Ph | 4-Pyr | COMe | H | H |
| 1-647 | 3,4-$F_2$-Ph | 4-Pyr | COEt | H | H |

| | | | | | |
|---|---|---|---|---|---|
| 1-648 | 3,4-$F_2$-Ph | 4-Pyr | COPr | H | H |
| 1-649 | 3,4-$F_2$-Ph | 4-Pyr | CO-i-Pr | H | H |
| 1-650 | 3,4-$F_2$-Ph | 4-Pyr | COBu | H | H |
| 1-651 | 3,4-$F_2$-Ph | 4-Pyr | $COCF_3$ | H | H |
| 1-652 | 3,4-$F_2$-Ph | 4-Pyr | CO-$(CH_2)_2$-F | H | H |
| 1-653 | 3,4-$F_2$-Ph | 4-Pyr | CO-$(CH_2)_2$-OH | H | H |
| 1-654 | 3,4-$F_2$-Ph | 4-Pyr | CO-$(CH_2)_2$-OMe | H | H |
| 1-655 | 3,4-$F_2$-Ph | 4-Pyr | SOMe | H | H |
| 1-656 | 3,4-$F_2$-Ph | 4-Pyr | SOEt | H | H |
| 1-657 | 3,4-$F_2$-Ph | 4-Pyr | SOPr | H | H |
| 1-658 | 3,4-$F_2$-Ph | 4-Pyr | SO-i-Pr | H | H |
| 1-659 | 3,4-$F_2$-Ph | 4-Pyr | SOBu | H | H |
| 1-660 | 3,4-$F_2$-Ph | 4-Pyr | $SOCF_3$ | H | H |
| 1-661 | 3,4-$F_2$-Ph | 4-Pyr | SO-$(CH_2)_2$-F | H | H |
| 1-662 | 3,4-$F_2$-Ph | 4-Pyr | SO-$(CH_2)_2$-OH | H | H |
| 1-663 | 3,4-$F_2$-Ph | 4-Pyr | SO-$(CH_2)_2$-OMe | H | H |
| 1-664 | 3,4-$F_2$-Ph | 4-Pyr | $SO_2Me$ | H | H |
| 1-665 | 3,4-$F_2$-Ph | 4-Pyr | $SO_2Et$ | H | H |

| | | | | | |
|---|---|---|---|---|---|
| 1-666 | 3,4-$F_2$-Ph | 4-Pyr | $SO_2Pr$ | H | H |
| 1-667 | 3,4-$F_2$-Ph | 4-Pyr | $SO_2$-i-Pr | H | H |
| 1-668 | 3,4-$F_2$-Ph | 4-Pyr | $SO_2Bu$ | H | H |
| 1-669 | 3,4-$F_2$-Ph | 4-Pyr | $SO_2CF_3$ | H | H |
| 1-670 | 3,4-$F_2$-Ph | 4-Pyr | $SO_2-(CH_2)_2-F$ | H | H |
| 1-671 | 3,4-$F_2$-Ph | 4-Pyr | $SO_2-(CH_2)_2-OH$ | H | H |
| 1-672 | 3,4-$F_2$-Ph | 4-Pyr | $SO_2-(CH_2)_2-OMe$ | H | H |
| 1-673 | 3,4-$F_2$-Ph | 4-Pyr | $SO_2NH_2$ | H | H |
| 1-674 | 3,4-$F_2$-Ph | 4-Pyr | $SO_2NHMe$ | H | H |
| 1-675 | 3,4-$F_2$-Ph | 4-Pyr | $SO_2NHEt$ | H | H |
| 1-676 | 3,4-$F_2$-Ph | 4-Pyr | $SO_2NHPr$ | H | H |
| 1-677 | 3,4-$F_2$-Ph | 4-Pyr | $SO_2NH$-i-Pr | H | H |
| 1-678 | 3,4-$F_2$-Ph | 4-Pyr | $SO_2NHBu$ | H | H |
| 1-679 | 3,4-$F_2$-Ph | 4-Pyr | $SO_2NHBn$ | H | H |
| 1-680 | 3,4-$F_2$-Ph | 4-Pyr | $SO_2NMe_2$ | H | H |
| 1-681 | 3,4-$F_2$-Ph | 4-Pyr | $SO_2NH$-c-Pr | H | H |
| 1-682 | 3,4-$F_2$-Ph | 4-Pyr | $SO_2NH$-c-Bu | H | H |
| 1-683 | 3,4-$F_2$-Ph | 4-Pyr | $SO_2NH$-c-Pen | H | H |

| | | | | | |
|---|---|---|---|---|---|
| 1-684 | 3,4-$F_2$-Ph | 4-Pyr | $SO_2$NH-c-Hex | H | H |
| 1-685 | 3,4-$F_2$-Ph | 4-Pyr | $SO_2$NH-c-Hep | H | H |
| 1-686 | 3,4-$F_2$-Ph | 4-Pyr | $SO_2NHCH_2$-c-Pr | H | H |
| 1-687 | 3,4-$F_2$-Ph | 4-Pyr | $SO_2$NH-Allyl | H | H |
| 1-688 | 3,4-$F_2$-Ph | 4-Pyr | $SO_2$NH-Propargyl | H | H |
| 1-689 | 3,4-$F_2$-Ph | 4-Pyr | $SO_2$NHPh | H | H |
| 1-690 | 3,4-$F_2$-Ph | 4-Pyr | $SO_2$NH-3-Pyr | H | H |
| 1-691 | 3,4-$F_2$-Ph | 4-Pyr | $SO_2NHCH_2$-4-Pyr | H | H |
| 1-692 | 3,4-$F_2$-Ph | 4-Pyr | $SO_2NH-(CH_2)_2-OH$ | H | H |
| 1-693 | 3,4-$F_2$-Ph | 4-Pyr | $SO_2NHCH(CH_2OH)_2$ | H | H |
| 1-694 | 3,4-$F_2$-Ph | 4-Pyr | $SO_2NH-(CH_2)_2-OAc$ | H | H |
| 1-695 | 3,4-$F_2$-Ph | 4-Pyr | $SO_2NHCH_2CN$ | H | H |
| 1-696 | 3,4-$F_2$-Ph | 4-Pyr | $SO_2NH-(CH_2)_2-F$ | H | H |
| 1-697 | 3,4-$F_2$-Ph | 4-Pyr | $SO_2NH-(CH_2)_2-OMe$ | H | H |
| 1-698 | 3,4-$F_2$-Ph | 4-Pyr | $SO_2NH-(CH_2)_2-SMe$ | H | H |
| 1-699 | 3,4-$F_2$-Ph | 4-Pyr | $SO_2NH-(CH_2)_2-NH_2$ | H | H |
| 1-700 | 3,4-$F_2$-Ph | 4-Pyr | $SO_2NH-(CH_2)_2-NHMe$ | H | H |
| 1-701 | 3,4-$F_2$-Ph | 4-Pyr | $SO_2NH-(CH_2)_2-N(Me)_2$ | H | H |

| | | | | | |
|---|---|---|---|---|---|
| 1-702 | 3,4-$F_2$-Ph | 4-Pyr | $SO_2NHCH_2COOH$ | H | H |
| 1-703 | 3,4-$F_2$-Ph | 4-Pyr | $SO_2NHCH_2COOEt$ | H | H |
| 1-704 | 3,4-$F_2$-Ph | 4-Pyr | $SO_2NHCH(Me)COOEt$ | H | H |
| 1-705 | 3,4-$F_2$-Ph | 4-Pyr | $SO_2NHCH_2CONH_2$ | H | H |
| 1-706 | 3,4-$F_2$-Ph | 4-Pyr | $SO_2NHOH$ | H | H |
| 1-707 | 3,4-$F_2$-Ph | 4-Pyr | $SO_2NHOMe$ | H | H |
| 1-708 | 3,4-$F_2$-Ph | 4-Pyr | $SO_2NHOEt$ | H | H |
| 1-709 | 3,4-$F_2$-Ph | 4-Pyr | $SO_2NHOPr$ | H | H |
| 1-710 | 3,4-$F_2$-Ph | 4-Pyr | $SO_2NHOAllyl$ | H | H |
| 1-711 | 3,4-$F_2$-Ph | 4-Pyr | $SO_2NHOBn$ | H | H |
| 1-712 | 3,4-$F_2$-Ph | 4-Pyr | $SO_2NHNH_2$ | H | H |
| 1-713 | 3,4-$F_2$-Ph | 4-Pyr | $SO_2NHNHMe$ | H | H |
| 1-714 | 3,4-$F_2$-Ph | 4-Pyr | $SO_2NHN(Me)_2$ | H | H |
| 1-715 | 3-$CF_3$-Ph | 4-Pyr | COOH | H | H |
| 1-716 | 3-$CF_3$-Ph | 4-Pyr | COOMe | H | H |
| 1-717 | 3-$CF_3$-Ph | 4-Pyr | COOEt | H | H |
| 1-718 | 3-$CF_3$-Ph | 4-Pyr | COOPr | H | H |
| 1-719 | 3-$CF_3$-Ph | 4-Pyr | COO-i-Pr | H | H |
| 1-720 | 3-$CF_3$-Ph | 4-Pyr | COOBu | H | H |
| 1-721 | 3-$CF_3$-Ph | 4-Pyr | COOBn | H | H |
| 1-722 | 3-$CF_3$-Ph | 4-Pyr | COOPh | H | H |
| 1-723 | 3-$CF_3$-Ph | 4-Pyr | $CONH_2$ | H | H |
| 1-724 | 3-$CF_3$-Ph | 4-Pyr | CONHMe | H | H |

| | | | | | |
|---|---|---|---|---|---|
| 1-725 | 3-$CF_3$-Ph | 4-Pyr | CONHEt | H | H |
| 1-726 | 3-$CF_3$-Ph | 4-Pyr | CONHPr | H | H |
| 1-727 | 3-$CF_3$-Ph | 4-Pyr | CONH-i-Pr | H | H |
| 1-728 | 3-$CF_3$-Ph | 4-Pyr | CONHBu | H | H |
| 1-729 | 3-$CF_3$-Ph | 4-Pyr | CONHBn | H | H |
| 1-730 | 3-$CF_3$-Ph | 4-Pyr | $CONMe_2$ | H | H |
| 1-731 | 3-$CF_3$-Ph | 4-Pyr | CONH-c-Pr | H | H |
| 1-732 | 3-$CF_3$-Ph | 4-Pyr | CONH-c-Bu | H | H |
| 1-733 | 3-$CF_3$-Ph | 4-Pyr | CONH-c-Pen | H | H |
| 1-734 | 3-$CF_3$-Ph | 4-Pyr | CONH-c-Hex | H | H |
| 1-735 | 3-$CF_3$-Ph | 4-Pyr | CONH-c-Hep | H | H |
| 1-736 | 3-$CF_3$-Ph | 4-Pyr | $CONHCH_2$-c-Pr | H | H |
| 1-737 | 3-$CF_3$-Ph | 4-Pyr | CONH-Allyl | H | H |
| 1-738 | 3-$CF_3$-Ph | 4-Pyr | CONH-Propargyl | H | H |
| 1-739 | 3-$CF_3$-Ph | 4-Pyr | CONHPh | H | H |
| 1-740 | 3-$CF_3$-Ph | 4-Pyr | CONH-3-Pyr | H | H |
| 1-741 | 3-$CF_3$-Ph | 4-Pyr | $CONHCH_2$-4-Pyr | H | H |
| 1-742 | 3-$CF_3$-Ph | 4-Pyr | CONH-$(CH_2)_2$-OH | H | H |
| 1-743 | 3-$CF_3$-Ph | 4-Pyr | $CONHCH(CH_2OH)_2$ | H | H |
| 1-744 | 3-$CF_3$-Ph | 4-Pyr | CONH-$(CH_2)_2$-OAc | H | H |
| 1-745 | 3-$CF_3$-Ph | 4-Pyr | $CONHCH_2CN$ | H | H |
| 1-746 | 3-$CF_3$-Ph | 4-Pyr | CONH-$(CH_2)_2$-F | H | H |
| 1-747 | 3-$CF_3$-Ph | 4-Pyr | CONH-$(CH_2)_2$-OMe | H | H |
| 1-748 | 3-$CF_3$-Ph | 4-Pyr | CONH-$(CH_2)_2$-SMe | H | H |
| 1-749 | 3-$CF_3$-Ph | 4-Pyr | CONH-$(CH_2)_2$-$NH_2$ | H | H |
| 1-750 | 3-$CF_3$-Ph | 4-Pyr | CONH-$(CH_2)_2$-NHMe | H | H |
| 1-751 | 3-$CF_3$-Ph | 4-Pyr | CONH-$(CH_2)_2$-$N(Me)_2$ | H | H |
| 1-752 | 3-$CF_3$-Ph | 4-Pyr | $CONHCH_2COOH$ | H | H |
| 1-753 | 3-$CF_3$-Ph | 4-Pyr | $CONHCH_2COOEt$ | H | H |
| 1-754 | 3-$CF_3$-Ph | 4-Pyr | CONHCH(Me)COOEt | H | H |
| 1-755 | 3-$CF_3$-Ph | 4-Pyr | $CONHCH_2CONH_2$ | H | H |
| 1-756 | 3-$CF_3$-Ph | 4-Pyr | CONHOH | H | H |
| 1-757 | 3-$CF_3$-Ph | 4-Pyr | CONHOMe | H | H |
| 1-758 | 3-$CF_3$-Ph | 4-Pyr | CONHOEt | H | H |
| 1-759 | 3-$CF_3$-Ph | 4-Pyr | CONHOPr | H | H |
| 1-760 | 3-$CF_3$-Ph | 4-Pyr | CONHOAllyl | H | H |

| | | | | | |
|---|---|---|---|---|---|
| 1-761 | 3-$CF_3$-Ph | 4-Pyr | CONHOBn | H | H |
| 1-762 | 3-$CF_3$-Ph | 4-Pyr | $CONHNH_2$ | H | H |
| 1-763 | 3-$CF_3$-Ph | 4-Pyr | CONHNHMe | H | H |
| 1-764 | 3-$CF_3$-Ph | 4-Pyr | $CONHN(Me)_2$ | H | H |
| 1-765 | 3-$CF_3$-Ph | 4-Pyr | COMe | H | H |
| 1-766 | 3-$CF_3$-Ph | 4-Pyr | COEt | H | H |
| 1-767 | 3-$CF_3$-Ph | 4-Pyr | COPr | H | H |
| 1-768 | 3-$CF_3$-Ph | 4-Pyr | CO-i-Pr | H | H |
| 1-769 | 3-$CF_3$-Ph | 4-Pyr | COBu | H | H |
| 1-770 | 3-$CF_3$-Ph | 4-Pyr | $COCF_3$ | H | H |
| 1-771 | 3-$CF_3$-Ph | 4-Pyr | CO-$(CH_2)_2$-F | H | H |
| 1-772 | 3-$CF_3$-Ph | 4-Pyr | CO-$(CH_2)_2$-OH | H | H |
| 1-773 | 3-$CF_3$-Ph | 4-Pyr | CO-$(CH_2)_2$-OMe | H | H |
| 1-774 | 3-$CF_3$-Ph | 4-Pyr | SOMe | H | H |
| 1-775 | 3-$CF_3$-Ph | 4-Pyr | SOEt | H | H |
| 1-776 | 3-$CF_3$-Ph | 4-Pyr | SOPr | H | H |
| 1-777 | 3-$CF_3$-Ph | 4-Pyr | SO-i-Pr | H | H |
| 1-778 | 3-$CF_3$-Ph | 4-Pyr | SOBu | H | H |
| 1-779 | 3-$CF_3$-Ph | 4-Pyr | $SOCF_3$ | H | H |
| 1-780 | 3-$CF_3$-Ph | 4-Pyr | SO-$(CH_2)_2$-F | H | H |
| 1-781 | 3-$CF_3$-Ph | 4-Pyr | SO-$(CH_2)_2$-OH | H | H |
| 1-782 | 3-$CF_3$-Ph | 4-Pyr | SO-$(CH_2)_2$-OMe | H | H |
| 1-783 | 3-$CF_3$-Ph | 4-Pyr | $SO_2Me$ | H | H |
| 1-784 | 3-$CF_3$-Ph | 4-Pyr | $SO_2Et$ | H | H |
| 1-785 | 3-$CF_3$-Ph | 4-Pyr | $SO_2Pr$ | H | H |
| 1-786 | 3-$CF_3$-Ph | 4-Pyr | $SO_2$-i-Pr | H | H |
| 1-787 | 3-$CF_3$-Ph | 4-Pyr | $SO_2Bu$ | H | H |
| 1-788 | 3-$CF_3$-Ph | 4-Pyr | $SO_2CF_3$ | H | H |
| 1-789 | 3-$CF_3$-Ph | 4-Pyr | $SO_2$-$(CH_2)_2$-F | H | H |
| 1-790 | 3-$CF_3$-Ph | 4-Pyr | $SO_2$-$(CH_2)_2$-OH | H | H |
| 1-791 | 3-$CF_3$-Ph | 4-Pyr | $SO_2$-$(CH_2)_2$-OMe | H | H |
| 1-792 | 3-$CF_3$-Ph | 4-Pyr | $SO_2NH_2$ | H | H |
| 1-793 | 3-$CF_3$-Ph | 4-Pyr | $SO_2NHMe$ | H | H |
| 1-794 | 3-$CF_3$-Ph | 4-Pyr | $SO_2NHEt$ | H | H |
| 1-795 | 3-$CF_3$-Ph | 4-Pyr | $SO_2NHPr$ | H | H |
| 1-796 | 3-$CF_3$-Ph | 4-Pyr | $SO_2NH$-i-Pr | H | H |

| | | | | | |
|---|---|---|---|---|---|
| 1-797 | 3-$CF_3$-Ph | 4-Pyr | $SO_2NHBu$ | H | H |
| 1-798 | 3-$CF_3$-Ph | 4-Pyr | $SO_2NHBn$ | H | H |
| 1-799 | 3-$CF_3$-Ph | 4-Pyr | $SO_2NMe_2$ | H | H |
| 1-800 | 3-$CF_3$-Ph | 4-Pyr | $SO_2NH$-c-Pr | H | H |
| 1-801 | 3-$CF_3$-Ph | 4-Pyr | $SO_2NH$-c-Bu | H | H |
| 1-802 | 3-$CF_3$-Ph | 4-Pyr | $SO_2NH$-c-Pen | H | H |
| 1-803 | 3-$CF_3$-Ph | 4-Pyr | $SO_2NH$-c-Hex | H | H |
| 1-804 | 3-$CF_3$-Ph | 4-Pyr | $SO_2NH$-c-Hep | H | H |
| 1-805 | 3-$CF_3$-Ph | 4-Pyr | $SO_2NHCH_2$-c-Pr | H | H |
| 1-806 | 3-$CF_3$-Ph | 4-Pyr | $SO_2NH$-Allyl | H | H |
| 1-807 | 3-$CF_3$-Ph | 4-Pyr | $SO_2NH$-Propargyl | H | H |
| 1-808 | 3-$CF_3$-Ph | 4-Pyr | $SO_2NHPh$ | H | H |
| 1-809 | 3-$CF_3$-Ph | 4-Pyr | $SO_2NH$-3-Pyr | H | H |
| 1-810 | 3-$CF_3$-Ph | 4-Pyr | $SO_2NHCH_2$-4-Pyr | H | H |
| 1-811 | 3-$CF_3$-Ph | 4-Pyr | $SO_2NH-(CH_2)_2-OH$ | H | H |
| 1-812 | 3-$CF_3$-Ph | 4-Pyr | $SO_2NHCH(CH_2OH)_2$ | H | H |
| 1-813 | 3-$CF_3$-Ph | 4-Pyr | $SO_2NH-(CH_2)_2-OAc$ | H | H |
| 1-814 | 3-$CF_3$-Ph | 4-Pyr | $SO_2NHCH_2CN$ | H | H |
| 1-815 | 3-$CF_3$-Ph | 4-Pyr | $SO_2NH-(CH_2)_2-F$ | H | H |
| 1-816 | 3-$CF_3$-Ph | 4-Pyr | $SO_2NH-(CH_2)_2-OMe$ | H | H |
| 1-817 | 3-$CF_3$-Ph | 4-Pyr | $SO_2NH-(CH_2)_2-SMe$ | H | H |
| 1-818 | 3-$CF_3$-Ph | 4-Pyr | $SO_2NH-(CH_2)_2-NH_2$ | H | H |
| 1-819 | 3-$CF_3$-Ph | 4-Pyr | $SO_2NH-(CH_2)_2-NHMe$ | H | H |
| 1-820 | 3-$CF_3$-Ph | 4-Pyr | $SO_2NH-(CH_2)_2-N(Me)_2$ | H | H |
| 1-821 | 3-$CF_3$-Ph | 4-Pyr | $SO_2NHCH_2COOH$ | H | H |
| 1-822 | 3-$CF_3$-Ph | 4-Pyr | $SO_2NHCH_2COOEt$ | H | H |
| 1-823 | 3-$CF_3$-Ph | 4-Pyr | $SO_2NHCH(Me)COOEt$ | H | H |
| 1-824 | 3-$CF_3$-Ph | 4-Pyr | $SO_2NHCH_2CONH_2$ | H | H |
| 1-825 | 3-$CF_3$-Ph | 4-Pyr | $SO_2NHOH$ | H | H |
| 1-826 | 3-$CF_3$-Ph | 4-Pyr | $SO_2NHOMe$ | H | H |
| 1-827 | 3-$CF_3$-Ph | 4-Pyr | $SO_2NHOEt$ | H | H |
| 1-828 | 3-$CF_3$-Ph | 4-Pyr | $SO_2NHOPr$ | H | H |
| 1-829 | 3-$CF_3$-Ph | 4-Pyr | $SO_2NHOAllyl$ | H | H |
| 1-830 | 3-$CF_3$-Ph | 4-Pyr | $SO_2NHOBn$ | H | H |
| 1-831 | 3-$CF_3$-Ph | 4-Pyr | $SO_2NHNH_2$ | H | H |
| 1-832 | 3-$CF_3$-Ph | 4-Pyr | $SO_2NHNHMe$ | H | H |

| | | | | | |
|---|---|---|---|---|---|
| 1-833 | 3-$CF_3$-Ph | 4-Pyr | $SO_2NHN(Me)_2$ | H | H |
| 1-834 | 4-F-Ph | 4-Pym | $CONH_2$ | H | H |
| 1-835 | 4-F-Ph | 4-Pym | CONHMe | H | H |
| 1-836 | 4-F-Ph | 4-Pym | CONHEt | H | H |
| 1-837 | 4-F-Ph | 4-Pym | CONHPr | H | H |
| 1-838 | 4-F-Ph | 4-Pym | CONH-i-Pr | H | H |
| 1-839 | 4-F-Ph | 4-Pym | CONHBu | H | H |
| 1-840 | 4-F-Ph | 4-Pym | CONHBn | H | H |
| 1-841 | 4-F-Ph | 4-Pym | $CONMe_2$ | H | H |
| 1-842 | 4-F-Ph | 4-Pym | CONH-c-Pr | H | H |
| 1-843 | 4-F-Ph | 4-Pym | CONH-c-Bu | H | H |
| 1-844 | 4-F-Ph | 4-Pym | CONH-c-Pen | H | H |
| 1-845 | 4-F-Ph | 4-Pym | CONH-c-Hex | H | H |
| 1-846 | 4-F-Ph | 4-Pym | CONH-c-Hep | H | H |
| 1-847 | 4-F-Ph | 4-Pym | $CONHCH_2$-c-Pr | H | H |
| 1-848 | 4-F-Ph | 4-Pym | CONH-Allyl | H | H |
| 1-849 | 4-F-Ph | 4-Pym | CONH-Propargyl | H | H |
| 1-850 | 4-F-Ph | 4-Pym | CONHPh | H | H |
| 1-851 | 4-F-Ph | 4-Pym | CONH-3-Pyr | H | H |
| 1-852 | 4-F-Ph | 4-Pym | $CONHCH_2$-4-Pyr | H | H |
| 1-853 | 4-F-Ph | 4-Pym | CONH-$(CH_2)_2$-OH | H | H |
| 1-854 | 4-F-Ph | 4-Pym | $CONHCH(CH_2OH)_2$ | H | H |
| 1-855 | 4-F-Ph | 4-Pym | CONH-$(CH_2)_2$-OAc | H | H |
| 1-856 | 4-F-Ph | 4-Pym | $CONHCH_2CN$ | H | H |
| 1-857 | 4-F-Ph | 4-Pym | CONH-$(CH_2)_2$-F | H | H |
| 1-858 | 4-F-Ph | 4-Pym | CONH-$(CH_2)_2$-OMe | H | H |
| 1-859 | 4-F-Ph | 4-Pym | CONH-$(CH_2)_2$-SMe | H | H |
| 1-860 | 4-F-Ph | 4-Pym | CONH-$(CH_2)_2$-$NH_2$ | H | H |
| 1-861 | 4-F-Ph | 4-Pym | CONH-$(CH_2)_2$-NHMe | H | H |
| 1-862 | 4-F-Ph | 4-Pym | CONH-$(CH_2)_2$-$N(Me)_2$ | H | H |
| 1-863 | 4-F-Ph | 4-Pym | $CONHCH_2COOH$ | H | H |
| 1-864 | 4-F-Ph | 4-Pym | $CONHCH_2COOEt$ | H | H |
| 1-865 | 4-F-Ph | 4-Pym | CONHCH(Me)COOEt | H | H |
| 1-866 | 4-F-Ph | 4-Pym | $CONHCH_2CONH_2$ | H | H |
| 1-867 | 4-F-Ph | 4-Pym | $SO_2NH_2$ | H | H |
| 1-868 | 4-F-Ph | 4-Pym | $SO_2NHMe$ | H | H |

| | | | | | |
|---|---|---|---|---|---|
| 1-869 | 4-F-Ph | 4-Pym | $SO_2NHEt$ | H | H |
| 1-870 | 4-F-Ph | 4-Pym | $SO_2NHPr$ | H | H |
| 1-871 | 4-F-Ph | 4-Pym | $SO_2NH$-i-Pr | H | H |
| 1-872 | 4-F-Ph | 4-Pym | $SO_2NHBu$ | H | H |
| 1-873 | 4-F-Ph | 4-Pym | $SO_2NHBn$ | H | H |
| 1-874 | 4-F-Ph | 4-Pym | $SO_2NMe_2$ | H | H |
| 1-875 | 4-F-Ph | 4-Pym | $SO_2NH$-c-Pr | H | H |
| 1-876 | 4-F-Ph | 4-Pym | $SO_2NH$-c-Bu | H | H |
| 1-877 | 4-F-Ph | 4-Pym | $SO_2NH$-c-Pen | H | H |
| 1-878 | 4-F-Ph | 4-Pym | $SO_2NH$-c-Hex | H | H |
| 1-879 | 4-F-Ph | 4-Pym | $SO_2NH$-c-Hep | H | H |
| 1-880 | 4-F-Ph | 4-Pym | $SO_2NHCH_2$-c-Pr | H | H |
| 1-881 | 4-F-Ph | 4-Pym | $SO_2NH$-Allyl | H | H |
| 1-882 | 4-F-Ph | 4-Pym | $SO_2NH$-Propargyl | H | H |
| 1-883 | 4-F-Ph | 4-Pym | $SO_2NHPh$ | H | H |
| 1-884 | 4-F-Ph | 4-Pym | $SO_2NH$-3-Pyr | H | H |
| 1-885 | 4-F-Ph | 4-Pym | $SO_2NHCH_2$-4-Pyr | H | H |
| 1-886 | 4-F-Ph | 4-Pym | $SO_2NH-(CH_2)_2-OH$ | H | H |
| 1-887 | 4-F-Ph | 4-Pym | $SO_2NHCH(CH_2OH)_2$ | H | H |
| 1-888 | 4-F-Ph | 4-Pym | $SO_2NH-(CH_2)_2-OAc$ | H | H |
| 1-889 | 4-F-Ph | 4-Pym | $SO_2NHCH_2CN$ | H | H |
| 1-890 | 4-F-Ph | 4-Pym | $SO_2NH-(CH_2)_2-F$ | H | H |
| 1-891 | 4-F-Ph | 4-Pym | $SO_2NH-(CH_2)_2-OMe$ | H | H |
| 1-892 | 4-F-Ph | 4-Pym | $SO_2NH-(CH_2)_2-SMe$ | H | H |
| 1-893 | 4-F-Ph | 4-Pym | $SO_2NH-(CH_2)_2-NH_2$ | H | H |
| 1-894 | 4-F-Ph | 4-Pym | $SO_2NH-(CH_2)_2-NHMe$ | H | H |
| 1-895 | 4-F-Ph | 4-Pym | $SO_2NH-(CH_2)_2-N(Me)_2$ | H | H |
| 1-896 | 4-F-Ph | 4-Pym | $SO_2NHCH_2COOH$ | H | H |
| 1-897 | 4-F-Ph | 4-Pym | $SO_2NHCH_2COOEt$ | H | H |
| 1-898 | 4-F-Ph | 4-Pym | $SO_2NHCH(Me)COOEt$ | H | H |
| 1-899 | 4-F-Ph | 4-Pym | $SO_2NHCH_2CONH_2$ | H | H |
| 1-900 | 4-F-Ph | 4-Pym | $SO_2NHOH$ | H | H |
| 1-901 | 4-F-Ph | 4-Pym | $SO_2NHOMe$ | H | H |
| 1-902 | 4-F-Ph | 4-Pym | $SO_2NHOEt$ | H | H |
| 1-903 | 4-F-Ph | 4-Pym | $SO_2NHOPr$ | H | H |
| 1-904 | 4-F-Ph | 4-Pym | $SO_2NHOAllyl$ | H | H |

| | | | | | |
|---|---|---|---|---|---|
| 1-905 | 4-F-Ph | 4-Pym | $SO_2NHOBn$ | H | H |
| 1-906 | 4-F-Ph | 4-Pym | $SO_2NHNH_2$ | H | H |
| 1-907 | 4-F-Ph | 4-Pym | $SO_2NHNHMe$ | H | H |
| 1-908 | 4-F-Ph | 4-Pym | $SO_2NHN(Me)_2$ | H | H |
| 1-909 | 4-F-Ph | 2-MeO-4-Pym | $CONH_2$ | H | H |
| 1-910 | 4-F-Ph | 2-MeO-4-Pym | CONHMe | H | H |
| 1-911 | 4-F-Ph | 2-MeO-4-Pym | CONHEt | H | H |
| 1-912 | 4-F-Ph | 2-MeO-4-Pym | CONHPr | H | H |
| 1-913 | 4-F-Ph | 2-MeO-4-Pym | CONH-i-Pr | H | H |
| 1-914 | 4-F-Ph | 2-MeO-4-Pym | CONHBu | H | H |
| 1-915 | 4-F-Ph | 2-MeO-4-Pym | CONHBn | H | H |
| 1-916 | 4-F-Ph | 2-MeO-4-Pym | $CONMe_2$ | H | H |
| 1-917 | 4-F-Ph | 2-MeO-4-Pym | CONH-c-Pr | H | H |
| 1-918 | 4-F-Ph | 2-MeO-4-Pym | CONH-c-Bu | H | H |
| 1-919 | 4-F-Ph | 2-MeO-4-Pym | CONH-c-Pen | H | H |
| 1-920 | 4-F-Ph | 2-MeO-4-Pym | CONH-c-Hex | H | H |
| 1-921 | 4-F-Ph | 2-MeO-4-Pym | CONH-c-Hep | H | H |
| 1-922 | 4-F-Ph | 2-MeO-4-Pym | $CONHCH_2$-c-Pr | H | H |
| 1-923 | 4-F-Ph | 2-MeO-4-Pym | CONH-Allyl | H | H |
| 1-924 | 4-F-Ph | 2-MeO-4-Pym | CONH-Propargyl | H | H |
| 1-925 | 4-F-Ph | 2-MeO-4-Pym | CONHPh | H | H |
| 1-926 | 4-F-Ph | 2-MeO-4-Pym | CONH-3-Pyr | H | H |
| 1-927 | 4-F-Ph | 2-MeO-4-Pym | $CONHCH_2$-4-Pyr | H | H |
| 1-928 | 4-F-Ph | 2-MeO-4-Pym | CONH-$(CH_2)_2$-OH | H | H |
| 1-929 | 4-F-Ph | 2-MeO-4-Pym | $CONHCH(CH_2OH)_2$ | H | H |
| 1-930 | 4-F-Ph | 2-MeO-4-Pym | CONH-$(CH_2)_2$-OAc | H | H |
| 1-931 | 4-F-Ph | 2-MeO-4-Pym | $CONHCH_2CN$ | H | H |
| 1-932 | 4-F-Ph | 2-MeO-4-Pym | CONH-$(CH_2)_2$-F | H | H |
| 1-933 | 4-F-Ph | 2-MeO-4-Pym | CONH-$(CH_2)_2$-OMe | H | H |
| 1-934 | 4-F-Ph | 2-MeO-4-Pym | CONH-$(CH_2)_2$-SMe | H | H |
| 1-935 | 4-F-Ph | 2-MeO-4-Pym | CONH-$(CH_2)_2$-$NH_2$ | H | H |
| 1-936 | 4-F-Ph | 2-MeO-4-Pym | CONH-$(CH_2)_2$-NHMe | H | H |
| 1-937 | 4-F-Ph | 2-MeO-4-Pym | CONH-$(CH_2)_2$-$N(Me)_2$ | H | H |
| 1-938 | 4-F-Ph | 2-MeO-4-Pym | $CONHCH_2COOH$ | H | H |
| 1-939 | 4-F-Ph | 2-MeO-4-Pym | $CONHCH_2COOEt$ | H | H |
| 1-940 | 4-F-Ph | 2-MeO-4-Pym | CONHCH(Me)COOEt | H | H |

| | | | | | |
|---|---|---|---|---|---|
| 1-941 | 4-F-Ph | 2-MeO-4-Pym | $CONHCH_2CONH_2$ | H | H |
| 1-942 | 4-F-Ph | 2-MeO-4-Pym | $SO_2NH_2$ | H | H |
| 1-943 | 4-F-Ph | 2-MeO-4-Pym | $SO_2NHMe$ | H | H |
| 1-944 | 4-F-Ph | 2-MeO-4-Pym | $SO_2NHEt$ | H | H |
| 1-945 | 4-F-Ph | 2-MeO-4-Pym | $SO_2NHPr$ | H | H |
| 1-946 | 4-F-Ph | 2-MeO-4-Pym | $SO_2NH$-i-Pr | H | H |
| 1-947 | 4-F-Ph | 2-MeO-4-Pym | $SO_2NHBu$ | H | H |
| 1-948 | 4-F-Ph | 2-MeO-4-Pym | $SO_2NHBn$ | H | H |
| 1-949 | 4-F-Ph | 2-MeO-4-Pym | $SO_2NMe_2$ | H | H |
| 1-950 | 4-F-Ph | 2-MeO-4-Pym | $SO_2NH$-c-Pr | H | H |
| 1-951 | 4-F-Ph | 2-MeO-4-Pym | $SO_2NH$-c-Bu | H | H |
| 1-952 | 4-F-Ph | 2-MeO-4-Pym | $SO_2NH$-c-Pen | H | H |
| 1-953 | 4-F-Ph | 2-MeO-4-Pym | $SO_2NH$-c-Hex | H | H |
| 1-954 | 4-F-Ph | 2-MeO-4-Pym | $SO_2NH$-c-Hep | H | H |
| 1-955 | 4-F-Ph | 2-MeO-4-Pym | $SO_2NHCH_2$-c-Pr | H | H |
| 1-956 | 4-F-Ph | 2-MeO-4-Pym | $SO_2NH$-Allyl | H | H |
| 1-957 | 4-F-Ph | 2-MeO-4-Pym | $SO_2NH$-Propargyl | H | H |
| 1-958 | 4-F-Ph | 2-MeO-4-Pym | $SO_2NHPh$ | H | H |
| 1-959 | 4-F-Ph | 2-MeO-4-Pym | $SO_2NH$-3-Pyr | H | H |
| 1-960 | 4-F-Ph | 2-MeO-4-Pym | $SO_2NHCH_2$-4-Pyr | H | H |
| 1-961 | 4-F-Ph | 2-MeO-4-Pym | $SO_2NH-(CH_2)_2-OH$ | H | H |
| 1-962 | 4-F-Ph | 2-MeO-4-Pym | $SO_2NHCH(CH_2OH)_2$ | H | H |
| 1-963 | 4-F-Ph | 2-MeO-4-Pym | $SO_2NH-(CH_2)_2-OAc$ | H | H |
| 1-964 | 4-F-Ph | 2-MeO-4-Pym | $SO_2NHCH_2CN$ | H | H |
| 1-965 | 4-F-Ph | 2-MeO-4-Pym | $SO_2NH-(CH_2)_2-F$ | H | H |
| 1-966 | 4-F-Ph | 2-MeO-4-Pym | $SO_2NH-(CH_2)_2-OMe$ | H | H |
| 1-967 | 4-F-Ph | 2-MeO-4-Pym | $SO_2NH-(CH_2)_2-SMe$ | H | H |
| 1-968 | 4-F-Ph | 2-MeO-4-Pym | $SO_2NH-(CH_2)_2-NH_2$ | H | H |
| 1-969 | 4-F-Ph | 2-MeO-4-Pym | $SO_2NH-(CH_2)_2-NHMe$ | H | H |
| 1-970 | 4-F-Ph | 2-MeO-4-Pym | $SO_2NH-(CH_2)_2-N(Me)_2$ | H | H |
| 1-971 | 4-F-Ph | 2-MeO-4-Pym | $SO_2NHCH_2COOH$ | H | H |
| 1-972 | 4-F-Ph | 2-MeO-4-Pym | $SO_2NHCH_2COOEt$ | H | H |
| 1-973 | 4-F-Ph | 2-MeO-4-Pym | $SO_2NHCH(Me)COOEt$ | H | H |
| 1-974 | 4-F-Ph | 2-MeO-4-Pym | $SO_2NHCH_2CONH_2$ | H | H |
| 1-975 | 4-F-Ph | 2-MeO-4-Pym | $SO_2NHOH$ | H | H |
| 1-976 | 4-F-Ph | 2-MeO-4-Pym | $SO_2NHOMe$ | H | H |

| | | | | | |
|---|---|---|---|---|---|
| 1-977 | 4-F-Ph | 2-MeO-4-Pym | $SO_2NHOEt$ | H | H |
| 1-978 | 4-F-Ph | 2-MeO-4-Pym | $SO_2NHOPr$ | H | H |
| 1-979 | 4-F-Ph | 2-MeO-4-Pym | $SO_2NHOAllyl$ | H | H |
| 1-980 | 4-F-Ph | 2-MeO-4-Pym | $SO_2NHOBn$ | H | H |
| 1-981 | 4-F-Ph | 2-MeO-4-Pym | $SO_2NHNH_2$ | H | H |
| 1-982 | 4-F-Ph | 2-MeO-4-Pym | $SO_2NHNHMe$ | H | H |
| 1-983 | 4-F-Ph | 2-MeO-4-Pym | $SO_2NHN(Me)_2$ | H | H |
| 1-984 | 4-F-Ph | $2-NH_2-4-Pym$ | $CONH_2$ | H | H |
| 1-985 | 4-F-Ph | $2-NH_2-4-Pym$ | CONHMe | H | H |
| 1-986 | 4-F-Ph | $2-NH_2-4-Pym$ | CONHEt | H | H |
| 1-987 | 4-F-Ph | $2-NH_2-4-Pym$ | CONHPr | H | H |
| 1-988 | 4-F-Ph | $2-NH_2-4-Pym$ | CONH-i-Pr | H | H |
| 1-989 | 4-F-Ph | $2-NH_2-4-Pym$ | CONHBu | H | H |
| 1-990 | 4-F-Ph | $2-NH_2-4-Pym$ | CONHBn | H | H |
| 1-991 | 4-F-Ph | $2-NH_2-4-Pym$ | $CONMe_2$ | H | H |
| 1-992 | 4-F-Ph | $2-NH_2-4-Pym$ | CONH-c-Pr | H | H |
| 1-993 | 4-F-Ph | $2-NH_2-4-Pym$ | CONH-c-Bu | H | H |
| 1-994 | 4-F-Ph | $2-NH_2-4-Pym$ | CONH-c-Pen | H | H |
| 1-995 | 4-F-Ph | $2-NH_2-4-Pym$ | CONH-c-Hex | H | H |
| 1-996 | 4-F-Ph | $2-NH_2-4-Pym$ | CONH-c-Hep | H | H |
| 1-997 | 4-F-Ph | $2-NH_2-4-Pym$ | $CONHCH_2-c-Pr$ | H | H |
| 1-998 | 4-F-Ph | $2-NH_2-4-Pym$ | CONH-Allyl | H | H |
| 1-999 | 4-F-Ph | $2-NH_2-4-Pym$ | CONH-Propargyl | H | H |
| 1-1000 | 4-F-Ph | $2-NH_2-4-Pym$ | CONHPh | H | H |
| 1-1001 | 4-F-Ph | $2-NH_2-4-Pym$ | CONH-3-Pyr | H | H |
| 1-1002 | 4-F-Ph | $2-NH_2-4-Pym$ | $CONHCH_2-4-Pyr$ | H | H |
| 1-1003 | 4-F-Ph | $2-NH_2-4-Pym$ | $CONH-(CH_2)_2-OH$ | H | H |
| 1-1004 | 4-F-Ph | $2-NH_2-4-Pym$ | $CONHCH(CH_2OH)_2$ | H | H |
| 1-1005 | 4-F-Ph | $2-NH_2-4-Pym$ | $CONH-(CH_2)_2-OAc$ | H | H |
| 1-1006 | 4-F-Ph | $2-NH_2-4-Pym$ | $CONHCH_2CN$ | H | H |
| 1-1007 | 4-F-Ph | $2-NH_2-4-Pym$ | $CONH-(CH_2)_2-F$ | H | H |
| 1-1008 | 4-F-Ph | $2-NH_2-4-Pym$ | $CONH-(CH_2)_2-OMe$ | H | H |
| 1-1009 | 4-F-Ph | $2-NH_2-4-Pym$ | $CONH-(CH_2)_2-SMe$ | H | H |
| 1-1010 | 4-F-Ph | $2-NH_2-4-Pym$ | $CONH-(CH_2)_2-NH_2$ | H | H |
| 1-1011 | 4-F-Ph | $2-NH_2-4-Pym$ | $CONH-(CH_2)_2-NHMe$ | H | H |
| 1-1012 | 4-F-Ph | $2-NH_2-4-Pym$ | $CONH-(CH_2)_2-N(Me)_2$ | H | H |

| | | | | | |
|---|---|---|---|---|---|
| 1-1013 | 4-F-Ph | 2-$NH_2$-4-Pym | $CONHCH_2COOH$ | H | H |
| 1-1014 | 4-F-Ph | 2-$NH_2$-4-Pym | $CONHCH_2COOEt$ | H | H |
| 1-1015 | 4-F-Ph | 2-$NH_2$-4-Pym | CONHCH(Me)COOEt | H | H |
| 1-1016 | 4-F-Ph | 2-$NH_2$-4-Pym | $CONHCH_2CONH_2$ | H | H |
| 1-1017 | 4-F-Ph | 2-$NH_2$-4-Pym | $SO_2NH_2$ | H | H |
| 1-1018 | 4-F-Ph | 2-$NH_2$-4-Pym | $SO_2NHMe$ | H | H |
| 1-1019 | 4-F-Ph | 2-$NH_2$-4-Pym | $SO_2NHEt$ | H | H |
| 1-1020 | 4-F-Ph | 2-$NH_2$-4-Pym | $SO_2NHPr$ | H | H |
| 1-1021 | 4-F-Ph | 2-$NH_2$-4-Pym | $SO_2NH$-i-Pr | H | H |
| 1-1022 | 4-F-Ph | 2-$NH_2$-4-Pym | $SO_2NHBu$ | H | H |
| 1-1023 | 4-F-Ph | 2-$NH_2$-4-Pym | $SO_2NHBn$ | H | H |
| 1-1024 | 4-F-Ph | 2-$NH_2$-4-Pym | $SO_2NMe_2$ | H | H |
| 1-1025 | 4-F-Ph | 2-$NH_2$-4-Pym | $SO_2NH$-c-Pr | H | H |
| 1-1026 | 4-F-Ph | 2-$NH_2$-4-Pym | $SO_2NH$-c-Bu | H | H |
| 1-1027 | 4-F-Ph | 2-$NH_2$-4-Pym | $SO_2NH$-c-Pen | H | H |
| 1-1028 | 4-F-Ph | 2-$NH_2$-4-Pym | $SO_2NH$-c-Hex | H | H |
| 1-1029 | 4-F-Ph | 2-$NH_2$-4-Pym | $SO_2NH$-c-Hep | H | H |
| 1-1030 | 4-F-Ph | 2-$NH_2$-4-Pym | $SO_2NHCH_2$-c-Pr | H | H |
| 1-1031 | 4-F-Ph | 2-$NH_2$-4-Pym | $SO_2NH$-Allyl | H | H |
| 1-1032 | 4-F-Ph | 2-$NH_2$-4-Pym | $SO_2NH$-Propargyl | H | H |
| 1-1033 | 4-F-Ph | 2-$NH_2$-4-Pym | $SO_2NHPh$ | H | H |
| 1-1034 | 4-F-Ph | 2-$NH_2$-4-Pym | $SO_2NH$-3-Pyr | H | H |
| 1-1035 | 4-F-Ph | 2-$NH_2$-4-Pym | $SO_2NHCH_2$-4-Pyr | H | H |
| 1-1036 | 4-F-Ph | 2-$NH_2$-4-Pym | $SO_2NH-(CH_2)_2-OH$ | H | H |
| 1-1037 | 4-F-Ph | 2-$NH_2$-4-Pym | $SO_2NHCH(CH_2OH)_2$ | H | H |
| 1-1038 | 4-F-Ph | 2-$NH_2$-4-Pym | $SO_2NH-(CH_2)_2-OAc$ | H | H |
| 1-1039 | 4-F-Ph | 2-$NH_2$-4-Pym | $SO_2NHCH_2CN$ | H | H |
| 1-1040 | 4-F-Ph | 2-$NH_2$-4-Pym | $SO_2NH-(CH_2)_2-F$ | H | H |
| 1-1041 | 4-F-Ph | 2-$NH_2$-4-Pym | $SO_2NH-(CH_2)_2-OMe$ | H | H |
| 1-1042 | 4-F-Ph | 2-$NH_2$-4-Pym | $SO_2NH-(CH_2)_2-SMe$ | H | H |
| 1-1043 | 4-F-Ph | 2-$NH_2$-4-Pym | $SO_2NH-(CH_2)_2-NH_2$ | H | H |
| 1-1044 | 4-F-Ph | 2-$NH_2$-4-Pym | $SO_2NH-(CH_2)_2-NHMe$ | H | H |
| 1-1045 | 4-F-Ph | 2-$NH_2$-4-Pym | $SO_2NH-(CH_2)_2-N(Me)_2$ | H | H |
| 1-1046 | 4-F-Ph | 2-$NH_2$-4-Pym | $SO_2NHCH_2COOH$ | H | H |
| 1-1047 | 4-F-Ph | 2-$NH_2$-4-Pym | $SO_2NHCH_2COOEt$ | H | H |
| 1-1048 | 4-F-Ph | 2-$NH_2$-4-Pym | $SO_2NHCH(Me)COOEt$ | H | H |

| | | | | | |
|---|---|---|---|---|---|
| 1-1049 | 4-F-Ph | 2-$NH_2$-4-Pym | $SO_2NHCH_2CONH_2$ | H | H |
| 1-1050 | 4-F-Ph | 2-$NH_2$-4-Pym | $SO_2NHOH$ | H | H |
| 1-1051 | 4-F-Ph | 2-$NH_2$-4-Pym | $SO_2NHOMe$ | H | H |
| 1-1052 | 4-F-Ph | 2-$NH_2$-4-Pym | $SO_2NHOEt$ | H | H |
| 1-1053 | 4-F-Ph | 2-$NH_2$-4-Pym | $SO_2NHOPr$ | H | H |
| 1-1054 | 4-F-Ph | 2-$NH_2$-4-Pym | $SO_2NHOAllyl$ | H | H |
| 1-1055 | 4-F-Ph | 2-$NH_2$-4-Pym | $SO_2NHOBn$ | H | H |
| 1-1056 | 4-F-Ph | 2-$NH_2$-4-Pym | $SO_2NHNH_2$ | H | H |
| 1-1057 | 4-F-Ph | 2-$NH_2$-4-Pym | $SO_2NHNHMe$ | H | H |
| 1-1058 | 4-F-Ph | 2-$NH_2$-4-Pym | $SO_2NHN(Me)_2$ | H | H |
| 1-1059 | 4-F-Ph | 2-MeNH-4-Pym | $CONH_2$ | H | H |
| 1-1060 | 4-F-Ph | 2-MeNH-4-Pym | CONHMe | H | H |
| 1-1061 | 4-F-Ph | 2-MeNH-4-Pym | CONHEt | H | H |
| 1-1062 | 4-F-Ph | 2-MeNH-4-Pym | CONHPr | H | H |
| 1-1063 | 4-F-Ph | 2-MeNH-4-Pym | CONH-i-Pr | H | H |
| 1-1064 | 4-F-Ph | 2-MeNH-4-Pym | CONHBu | H | H |
| 1-1065 | 4-F-Ph | 2-MeNH-4-Pym | CONHBn | H | H |
| 1-1066 | 4-F-Ph | 2-MeNH-4-Pym | $CONMe_2$ | H | H |
| 1-1067 | 4-F-Ph | 2-MeNH-4-Pym | CONH-c-Pr | H | H |
| 1-1068 | 4-F-Ph | 2-MeNH-4-Pym | CONH-c-Bu | H | H |
| 1-1069 | 4-F-Ph | 2-MeNH-4-Pym | CONH-c-Pen | H | H |
| 1-1070 | 4-F-Ph | 2-MeNH-4-Pym | CONH-c-Hex | H | H |
| 1-1071 | 4-F-Ph | 2-MeNH-4-Pym | CONH-c-Hep | H | H |
| 1-1072 | 4-F-Ph | 2-MeNH-4-Pym | $CONHCH_2$-c-Pr | H | H |
| 1-1073 | 4-F-Ph | 2-MeNH-4-Pym | CONH-Allyl | H | H |
| 1-1074 | 4-F-Ph | 2-MeNH-4-Pym | CONH-Propargyl | H | H |
| 1-1075 | 4-F-Ph | 2-MeNH-4-Pym | CONHPh | H | H |
| 1-1076 | 4-F-Ph | 2-MeNH-4-Pym | CONH-3-Pyr | H | H |
| 1-1077 | 4-F-Ph | 2-MeNH-4-Pym | $CONHCH_2$-4-Pyr | H | H |
| 1-1078 | 4-F-Ph | 2-MeNH-4-Pym | CONH-$(CH_2)_2$-OH | H | H |
| 1-1079 | 4-F-Ph | 2-MeNH-4-Pym | $CONHCH(CH_2OH)_2$ | H | H |
| 1-1080 | 4-F-Ph | 2-MeNH-4-Pym | CONH-$(CH_2)_2$-OAc | H | H |
| 1-1081 | 4-F-Ph | 2-MeNH-4-Pym | $CONHCH_2CN$ | H | H |
| 1-1082 | 4-F-Ph | 2-MeNH-4-Pym | CONH-$(CH_2)_2$-F | H | H |
| 1-1083 | 4-F-Ph | 2-MeNH-4-Pym | CONH-$(CH_2)_2$-OMe | H | H |
| 1-1084 | 4-F-Ph | 2-MeNH-4-Pym | CONH-$(CH_2)_2$-SMe | H | H |

| | | | | | |
|---|---|---|---|---|---|
| 1-1085 | 4-F-Ph | 2-MeNH-4-Pym | CONH-$(CH_2)_2$-$NH_2$ | H | H |
| 1-1086 | 4-F-Ph | 2-MeNH-4-Pym | CONH-$(CH_2)_2$-NHMe | H | H |
| 1-1087 | 4-F-Ph | 2-MeNH-4-Pym | CONH-$(CH_2)_2$-N$(Me)_2$ | H | H |
| 1-1088 | 4-F-Ph | 2-MeNH-4-Pym | $CONHCH_2COOH$ | H | H |
| 1-1089 | 4-F-Ph | 2-MeNH-4-Pym | $CONHCH_2COOEt$ | H | H |
| 1-1090 | 4-F-Ph | 2-MeNH-4-Pym | CONHCH(Me)COOEt | H | H |
| 1-1091 | 4-F-Ph | 2-MeNH-4-Pym | $CONHCH_2CONH_2$ | H | H |
| 1-1092 | 4-F-Ph | 2-MeNH-4-Pym | $SO_2NH_2$ | H | H |
| 1-1093 | 4-F-Ph | 2-MeNH-4-Pym | $SO_2NHMe$ | H | H |
| 1-1094 | 4-F-Ph | 2-MeNH-4-Pym | $SO_2NHEt$ | H | H |
| 1-1095 | 4-F-Ph | 2-MeNH-4-Pym | $SO_2NHPr$ | H | H |
| 1-1096 | 4-F-Ph | 2-MeNH-4-Pym | $SO_2NH$-i-Pr | H | H |
| 1-1097 | 4-F-Ph | 2-MeNH-4-Pym | $SO_2NHBu$ | H | H |
| 1-1098 | 4-F-Ph | 2-MeNH-4-Pym | $SO_2NHBn$ | H | H |
| 1-1099 | 4-F-Ph | 2-MeNH-4-Pym | $SO_2NMe_2$ | H | H |
| 1-1100 | 4-F-Ph | 2-MeNH-4-Pym | $SO_2NH$-c-Pr | H | H |
| 1-1101 | 4-F-Ph | 2-MeNH-4-Pym | $SO_2NH$-c-Bu | H | H |
| 1-1102 | 4-F-Ph | 2-MeNH-4-Pym | $SO_2NH$-c-Pen | H | H |
| 1-1103 | 4-F-Ph | 2-MeNH-4-Pym | $SO_2NH$-c-Hex | H | H |
| 1-1104 | 4-F-Ph | 2-MeNH-4-Pym | $SO_2NH$-c-Hep | H | H |
| 1-1105 | 4-F-Ph | 2-MeNH-4-Pym | $SO_2NHCH_2$-c-Pr | H | H |
| 1-1106 | 4-F-Ph | 2-MeNH-4-Pym | $SO_2NH$-Allyl | H | H |
| 1-1107 | 4-F-Ph | 2-MeNH-4-Pym | $SO_2NH$-Propargyl | H | H |
| 1-1108 | 4-F-Ph | 2-MeNH-4-Pym | $SO_2NHPh$ | H | H |
| 1-1109 | 4-F-Ph | 2-MeNH-4-Pym | $SO_2NH$-3-Pyr | H | H |
| 1-1110 | 4-F-Ph | 2-MeNH-4-Pym | $SO_2NHCH_2$-4-Pyr | H | H |
| 1-1111 | 4-F-Ph | 2-MeNH-4-Pym | $SO_2NH$-$(CH_2)_2$-OH | H | H |
| 1-1112 | 4-F-Ph | 2-MeNH-4-Pym | $SO_2NHCH(CH_2OH)_2$ | H | H |
| 1-1113 | 4-F-Ph | 2-MeNH-4-Pym | $SO_2NH$-$(CH_2)_2$-OAc | H | H |
| 1-1114 | 4-F-Ph | 2-MeNH-4-Pym | $SO_2NHCH_2CN$ | H | H |
| 1-1115 | 4-F-Ph | 2-MeNH-4-Pym | $SO_2NH$-$(CH_2)_2$-F | H | H |
| 1-1116 | 4-F-Ph | 2-MeNH-4-Pym | $SO_2NH$-$(CH_2)_2$-OMe | H | H |
| 1-1117 | 4-F-Ph | 2-MeNH-4-Pym | $SO_2NH$-$(CH_2)_2$-SMe | H | H |
| 1-1118 | 4-F-Ph | 2-MeNH-4-Pym | $SO_2NH$-$(CH_2)_2$-$NH_2$ | H | H |
| 1-1119 | 4-F-Ph | 2-MeNH-4-Pym | $SO_2NH$-$(CH_2)_2$-NHMe | H | H |
| 1-1120 | 4-F-Ph | 2-MeNH-4-Pym | $SO_2NH$-$(CH_2)_2$-N$(Me)_2$ | H | H |

| | | | | | |
|---|---|---|---|---|---|
| 1-1121 | 4-F-Ph | 2-MeNH-4-Pym | $SO_2NHCH_2COOH$ | H | H |
| 1-1122 | 4-F-Ph | 2-MeNH-4-Pym | $SO_2NHCH_2COOEt$ | H | H |
| 1-1123 | 4-F-Ph | 2-MeNH-4-Pym | $SO_2NHCH(Me)COOEt$ | H | H |
| 1-1124 | 4-F-Ph | 2-MeNH-4-Pym | $SO_2NHCH_2CONH_2$ | H | H |
| 1-1125 | 4-F-Ph | 2-MeNH-4-Pym | $SO_2NHOH$ | H | H |
| 1-1126 | 4-F-Ph | 2-MeNH-4-Pym | $SO_2NHOMe$ | H | H |
| 1-1127 | 4-F-Ph | 2-MeNH-4-Pym | $SO_2NHOEt$ | H | H |
| 1-1128 | 4-F-Ph | 2-MeNH-4-Pym | $SO_2NHOPr$ | H | H |
| 1-1129 | 4-F-Ph | 2-MeNH-4-Pym | $SO_2NHOAllyl$ | H | H |
| 1-1130 | 4-F-Ph | 2-MeNH-4-Pym | $SO_2NHOBn$ | H | H |
| 1-1131 | 4-F-Ph | 2-MeNH-4-Pym | $SO_2NHNH_2$ | H | H |
| 1-1132 | 4-F-Ph | 2-MeNH-4-Pym | $SO_2NHNHMe$ | H | H |
| 1-1133 | 4-F-Ph | 2-MeNH-4-Pym | $SO_2NHN(Me)_2$ | H | H |
| 1-1134 | 4-F-Ph | 2-BnNH-4-Pym | $CONH_2$ | H | H |
| 1-1135 | 4-F-Ph | 2-BnNH-4-Pym | CONHMe | H | H |
| 1-1136 | 4-F-Ph | 2-BnNH-4-Pym | CONHEt | H | H |
| 1-1137 | 4-F-Ph | 2-BnNH-4-Pym | CONHPr | H | H |
| 1-1138 | 4-F-Ph | 2-BnNH-4-Pym | CONH-i-Pr | H | H |
| 1-1139 | 4-F-Ph | 2-BnNH-4-Pym | CONHBu | H | H |
| 1-1140 | 4-F-Ph | 2-BnNH-4-Pym | CONHBn | H | H |
| 1-1141 | 4-F-Ph | 2-BnNH-4-Pym | $CONMe_2$ | H | H |
| 1-1142 | 4-F-Ph | 2-BnNH-4-Pym | CONH-c-Pr | H | H |
| 1-1143 | 4-F-Ph | 2-BnNH-4-Pym | CONH-c-Bu | H | H |
| 1-1144 | 4-F-Ph | 2-BnNH-4-Pym | CONH-c-Pen | H | H |
| 1-1145 | 4-F-Ph | 2-BnNH-4-Pym | CONH-c-Hex | H | H |
| 1-1146 | 4-F-Ph | 2-BnNH-4-Pym | CONH-c-Hep | H | H |
| 1-1147 | 4-F-Ph | 2-BnNH-4-Pym | $CONHCH_2$-c-Pr | H | H |
| 1-1148 | 4-F-Ph | 2-BnNH-4-Pym | CONH-Allyl | H | H |
| 1-1149 | 4-F-Ph | 2-BnNH-4-Pym | CONH-Propargyl | H | H |
| 1-1150 | 4-F-Ph | 2-BnNH-4-Pym | CONHPh | H | H |
| 1-1151 | 4-F-Ph | 2-BnNH-4-Pym | CONH-3-Pyr | H | H |
| 1-1152 | 4-F-Ph | 2-BnNH-4-Pym | $CONHCH_2$-4-Pyr | H | H |
| 1-1153 | 4-F-Ph | 2-BnNH-4-Pym | CONH-$(CH_2)_2$-OH | H | H |
| 1-1154 | 4-F-Ph | 2-BnNH-4-Pym | $CONHCH(CH_2OH)_2$ | H | H |
| 1-1155 | 4-F-Ph | 2-BnNH-4-Pym | CONH-$(CH_2)_2$-OAc | H | H |
| 1-1156 | 4-F-Ph | 2-BnNH-4-Pym | $CONHCH_2CN$ | H | H |

| | | | | | |
|---|---|---|---|---|---|
| 1-1157 | 4-F-Ph | 2-BnNH-4-Pym | CONH-$(CH_2)_2$-F | H | H |
| 1-1158 | 4-F-Ph | 2-BnNH-4-Pym | CONH-$(CH_2)_2$-OMe | H | H |
| 1-1159 | 4-F-Ph | 2-BnNH-4-Pym | CONH-$(CH_2)_2$-SMe | H | H |
| 1-1160 | 4-F-Ph | 2-BnNH-4-Pym | CONH-$(CH_2)_2$-$NH_2$ | H | H |
| 1-1161 | 4-F-Ph | 2-BnNH-4-Pym | CONH-$(CH_2)_2$-NHMe | H | H |
| 1-1162 | 4-F-Ph | 2-BnNH-4-Pym | CONH-$(CH_2)_2$-$N(Me)_2$ | H | H |
| 1-1163 | 4-F-Ph | 2-BnNH-4-Pym | $CONHCH_2COOH$ | H | H |
| 1-1164 | 4-F-Ph | 2-BnNH-4-Pym | $CONHCH_2COOEt$ | H | H |
| 1-1165 | 4-F-Ph | 2-BnNH-4-Pym | CONHCH(Me)COOEt | H | H |
| 1-1166 | 4-F-Ph | 2-BnNH-4-Pym | $CONHCH_2CONH_2$ | H | H |
| 1-1167 | 4-F-Ph | 2-BnNH-4-Pym | $SO_2NH_2$ | H | H |
| 1-1168 | 4-F-Ph | 2-BnNH-4-Pym | $SO_2NHMe$ | H | H |
| 1-1169 | 4-F-Ph | 2-BnNH-4-Pym | $SO_2NHEt$ | H | H |
| 1-1170 | 4-F-Ph | 2-BnNH-4-Pym | $SO_2NHPr$ | H | H |
| 1-1171 | 4-F-Ph | 2-BnNH-4-Pym | $SO_2NH$-i-Pr | H | H |
| 1-1172 | 4-F-Ph | 2-BnNH-4-Pym | $SO_2NHBu$ | H | H |
| 1-1173 | 4-F-Ph | 2-BnNH-4-Pym | $SO_2NHBn$ | H | H |
| 1-1174 | 4-F-Ph | 2-BnNH-4-Pym | $SO_2NMe_2$ | H | H |
| 1-1175 | 4-F-Ph | 2-BnNH-4-Pym | $SO_2NH$-c-Pr | H | H |
| 1-1176 | 4-F-Ph | 2-BnNH-4-Pym | $SO_2NH$-c-Bu | H | H |
| 1-1177 | 4-F-Ph | 2-BnNH-4-Pym | $SO_2NH$-c-Pen | H | H |
| 1-1178 | 4-F-Ph | 2-BnNH-4-Pym | $SO_2NH$-c-Hex | H | H |
| 1-1179 | 4-F-Ph | 2-BnNH-4-Pym | $SO_2NH$-c-Hep | H | H |
| 1-1180 | 4-F-Ph | 2-BnNH-4-Pym | $SO_2NHCH_2$-c-Pr | H | H |
| 1-1181 | 4-F-Ph | 2-BnNH-4-Pym | $SO_2NH$-Allyl | H | H |
| 1-1182 | 4-F-Ph | 2-BnNH-4-Pym | $SO_2NH$-Propargyl | H | H |
| 1-1183 | 4-F-Ph | 2-BnNH-4-Pym | $SO_2NHPh$ | H | H |
| 1-1184 | 4-F-Ph | 2-BnNH-4-Pym | $SO_2NH$-3-Pyr | H | H |
| 1-1185 | 4-F-Ph | 2-BnNH-4-Pym | $SO_2NHCH_2$-4-Pyr | H | H |
| 1-1186 | 4-F-Ph | 2-BnNH-4-Pym | $SO_2NH$-$(CH_2)_2$-OH | H | H |
| 1-1187 | 4-F-Ph | 2-BnNH-4-Pym | $SO_2NHCH(CH_2OH)_2$ | H | H |
| 1-1188 | 4-F-Ph | 2-BnNH-4-Pym | $SO_2NH$-$(CH_2)_2$-OAc | H | H |
| 1-1189 | 4-F-Ph | 2-BnNH-4-Pym | $SO_2NHCH_2CN$ | H | H |
| 1-1190 | 4-F-Ph | 2-BnNH-4-Pym | $SO_2NH$-$(CH_2)_2$-F | H | H |
| 1-1191 | 4-F-Ph | 2-BnNH-4-Pym | $SO_2NH$-$(CH_2)_2$-OMe | H | H |
| 1-1192 | 4-F-Ph | 2-BnNH-4-Pym | $SO_2NH$-$(CH_2)_2$-SMe | H | H |

| | | | | | |
|---|---|---|---|---|---|
| 1-1193 | 4-F-Ph | 2-BnNH-4-Pym | $SO_2NH-(CH_2)_2-NH_2$ | H | H |
| 1-1194 | 4-F-Ph | 2-BnNH-4-Pym | $SO_2NH-(CH_2)_2-NHMe$ | H | H |
| 1-1195 | 4-F-Ph | 2-BnNH-4-Pym | $SO_2NH-(CH_2)_2-N(Me)_2$ | H | H |
| 1-1196 | 4-F-Ph | 2-BnNH-4-Pym | $SO_2NHCH_2COOH$ | H | H |
| 1-1197 | 4-F-Ph | 2-BnNH-4-Pym | $SO_2NHCH_2COOEt$ | H | H |
| 1-1198 | 4-F-Ph | 2-BnNH-4-Pym | $SO_2NHCH(Me)COOEt$ | H | H |
| 1-1199 | 4-F-Ph | 2-BnNH-4-Pym | $SO_2NHCH_2CONH_2$ | H | H |
| 1-1200 | 4-F-Ph | 2-BnNH-4-Pym | $SO_2NHOH$ | H | H |
| 1-1201 | 4-F-Ph | 2-BnNH-4-Pym | $SO_2NHOMe$ | H | H |
| 1-1202 | 4-F-Ph | 2-BnNH-4-Pym | $SO_2NHOEt$ | H | H |
| 1-1203 | 4-F-Ph | 2-BnNH-4-Pym | $SO_2NHOPr$ | H | H |
| 1-1204 | 4-F-Ph | 2-BnNH-4-Pym | $SO_2NHOAllyl$ | H | H |
| 1-1205 | 4-F-Ph | 2-BnNH-4-Pym | $SO_2NHOBn$ | H | H |
| 1-1206 | 4-F-Ph | 2-BnNH-4-Pym | $SO_2NHNH_2$ | H | H |
| 1-1207 | 4-F-Ph | 2-BnNH-4-Pym | $SO_2NHNHMe$ | H | H |
| 1-1208 | 4-F-Ph | 2-BnNH-4-Pym | $SO_2NHN(Me)_2$ | H | H |
| 1-1209 | 4-F-Ph | (α-Me-BnNH)-4-Pym | $CONH_2$ | H | H |
| 1-1210 | 4-F-Ph | (α-Me-BnNH)-4-Pym | CONHMe | H | H |
| 1-1211 | 4-F-Ph | (α-Me-BnNH)-4-Pym | CONHEt | H | H |
| 1-1212 | 4-F-Ph | (α-Me-BnNH)-4-Pym | CONHPr | H | H |
| 1-1213 | 4-F-Ph | (α-Me-BnNH)-4-Pym | CONH-i-Pr | H | H |
| 1-1214 | 4-F-Ph | (α-Me-BnNH)-4-Pym | CONHBu | H | H |
| 1-1215 | 4-F-Ph | (α-Me-BnNH)-4-Pym | CONHBn | H | H |
| 1-1216 | 4-F-Ph | (α-Me-BnNH)-4-Pym | $CONMe_2$ | H | H |
| 1-1217 | 4-F-Ph | (α-Me-BnNH)-4-Pym | CONH-c-Pr | H | H |
| 1-1218 | 4-F-Ph | (α-Me-BnNH)-4-Py | CONH-c-Bu | H | H |

| | | | | | |
|---|---|---|---|---|---|
| | | m | | | |
| 1-1219 | 4-F-Ph | (α-Me-BnNH)-4-Py<br>m | CONH-c-Pen | H | H |
| 1-1220 | 4-F-Ph | (α-Me-BnNH)-4-Py<br>m | CONH-c-Hex | H | H |
| 1-1221 | 4-F-Ph | (α-Me-BnNH)-4-Py<br>m | CONH-c-Hep | H | H |
| 1-1222 | 4-F-Ph | (α-Me-BnNH)-4-Py<br>m | $CONHCH_2$-c-Pr | H | H |
| 1-1223 | 4-F-Ph | (α-Me-BnNH)-4-Py<br>m | CONH-Allyl | H | H |
| 1-1224 | 4-F-Ph | (α-Me-BnNH)-4-Py<br>m | CONH-Propargyl | H | H |
| 1-1225 | 4-F-Ph | (α-Me-BnNH)-4-Py<br>m | CONHPh | H | H |
| 1-1226 | 4-F-Ph | (α-Me-BnNH)-4-Py<br>m | CONH-3-Pyr | H | H |
| 1-1227 | 4-F-Ph | (α-Me-BnNH)-4-Py<br>m | $CONHCH_2$-4-Pyr | H | H |
| 1-1228 | 4-F-Ph | (α-Me-BnNH)-4-Py<br>m | CONH-$(CH_2)_2$-OH | H | H |
| 1-1229 | 4-F-Ph | (α-Me-BnNH)-4-Py<br>m | $CONHCH(CH_2OH)_2$ | H | H |
| 1-1230 | 4-F-Ph | (α-Me-BnNH)-4-Py<br>m | CONH-$(CH_2)_2$-OAc | H | H |
| 1-1231 | 4-F-Ph | (α-Me-BnNH)-4-Py<br>m | $CONHCH_2CN$ | H | H |
| 1-1232 | 4-F-Ph | (α-Me-BnNH)-4-Py<br>m | CONH-$(CH_2)_2$-F | H | H |
| 1-1233 | 4-F-Ph | (α-Me-BnNH)-4-Py<br>m | CONH-$(CH_2)_2$-OMe | H | H |
| 1-1234 | 4-F-Ph | (α-Me-BnNH)-4-Py<br>m | CONH-$(CH_2)_2$-SMe | H | H |
| 1-1235 | 4-F-Ph | (α-Me-BnNH)-4-Py | CONH-$(CH_2)_2$-$NH_2$ | H | H |

| | | | | | |
|---|---|---|---|---|---|
| | | m | | | |
| 1-1236 | 4-F-Ph | (α-Me-BnNH)-4-Pym | CONH-$(CH_2)_2$-NHMe | H | H |
| 1-1237 | 4-F-Ph | (α-Me-BnNH)-4-Pym | CONH-$(CH_2)_2$-N(Me)$_2$ | H | H |
| 1-1238 | 4-F-Ph | (α-Me-BnNH)-4-Pym | $CONHCH_2COOH$ | H | H |
| 1-1239 | 4-F-Ph | (α-Me-BnNH)-4-Pym | $CONHCH_2COOEt$ | H | H |
| 1-1240 | 4-F-Ph | (α-Me-BnNH)-4-Pym | CONHCH(Me)COOEt | H | H |
| 1-1241 | 4-F-Ph | (α-Me-BnNH)-4-Pym | $CONHCH_2CONH_2$ | H | H |
| 1-1242 | 4-F-Ph | (α-Me-BnNH)-4-Pym | $SO_2NH_2$ | H | H |
| 1-1243 | 4-F-Ph | (α-Me-BnNH)-4-Pym | $SO_2NHMe$ | H | H |
| 1-1244 | 4-F-Ph | (α-Me-BnNH)-4-Pym | $SO_2NHEt$ | H | H |
| 1-1245 | 4-F-Ph | (α-Me-BnNH)-4-Pym | $SO_2NHPr$ | H | H |
| 1-1246 | 4-F-Ph | (α-Me-BnNH)-4-Pym | $SO_2NH$-i-Pr | H | H |
| 1-1247 | 4-F-Ph | (α-Me-BnNH)-4-Pym | $SO_2NHBu$ | H | H |
| 1-1248 | 4-F-Ph | (α-Me-BnNH)-4-Pym | $SO_2NHBn$ | H | H |
| 1-1249 | 4-F-Ph | (α-Me-BnNH)-4-Pym | $SO_2NMe_2$ | H | H |
| 1-1250 | 4-F-Ph | (α-Me-BnNH)-4-Pym | $SO_2NH$-c-Pr | H | H |
| 1-1251 | 4-F-Ph | (α-Me-BnNH)-4-Pym | $SO_2NH$-c-Bu | H | H |
| 1-1252 | 4-F-Ph | (α-Me-BnNH)-4-Py | $SO_2NH$-c-Pen | H | H |

| | | | | | |
|---|---|---|---|---|---|
| | | m | | | |
| 1-1253 | 4-F-Ph | (α-Me-BnNH)-4-Pym | $SO_2$NH-c-Hex | H | H |
| 1-1254 | 4-F-Ph | (α-Me-BnNH)-4-Pym | $SO_2$NH-c-Hep | H | H |
| 1-1255 | 4-F-Ph | (α-Me-BnNH)-4-Pym | $SO_2NHCH_2$-c-Pr | H | H |
| 1-1256 | 4-F-Ph | (α-Me-BnNH)-4-Pym | $SO_2$NH-Allyl | H | H |
| 1-1257 | 4-F-Ph | (α-Me-BnNH)-4-Pym | $SO_2$NH-Propargyl | H | H |
| 1-1258 | 4-F-Ph | (α-Me-BnNH)-4-Pym | $SO_2$NHPh | H | H |
| 1-1259 | 4-F-Ph | (α-Me-BnNH)-4-Pym | $SO_2$NH-3-Pyr | H | H |
| 1-1260 | 4-F-Ph | (α-Me-BnNH)-4-Pym | $SO_2NHCH_2$-4-Pyr | H | H |
| 1-1261 | 4-F-Ph | (α-Me-BnNH)-4-Pym | $SO_2NH-(CH_2)_2-OH$ | H | H |
| 1-1262 | 4-F-Ph | (α-Me-BnNH)-4-Pym | $SO_2NHCH(CH_2OH)_2$ | H | H |
| 1-1263 | 4-F-Ph | (α-Me-BnNH)-4-Pym | $SO_2NH-(CH_2)_2-OAc$ | H | H |
| 1-1264 | 4-F-Ph | (α-Me-BnNH)-4-Pym | $SO_2NHCH_2CN$ | H | H |
| 1-1265 | 4-F-Ph | (α-Me-BnNH)-4-Pym | $SO_2NH-(CH_2)_2-F$ | H | H |
| 1-1266 | 4-F-Ph | (α-Me-BnNH)-4-Pym | $SO_2NH-(CH_2)_2-OMe$ | H | H |
| 1-1267 | 4-F-Ph | (α-Me-BnNH)-4-Pym | $SO_2NH-(CH_2)_2-SMe$ | H | H |
| 1-1268 | 4-F-Ph | (α-Me-BnNH)-4-Pym | $SO_2NH-(CH_2)_2-NH_2$ | H | H |
| 1-1269 | 4-F-Ph | (α-Me-BnNH)-4-Py | $SO_2NH-(CH_2)_2-NHMe$ | H | H |

| | | | | | |
|---|---|---|---|---|---|
| | | m | | | |
| 1-1270 | 4-F-Ph | (α-Me-BnNH)-4-Pym | $SO_2NH-(CH_2)_2-N(Me)_2$ | H | H |
| 1-1271 | 4-F-Ph | (α-Me-BnNH)-4-Pym | $SO_2NHCH_2COOH$ | H | H |
| 1-1272 | 4-F-Ph | (α-Me-BnNH)-4-Pym | $SO_2NHCH_2COOEt$ | H | H |
| 1-1273 | 4-F-Ph | (α-Me-BnNH)-4-Pym | $SO_2NHCH(Me)COOEt$ | H | H |
| 1-1274 | 4-F-Ph | (α-Me-BnNH)-4-Pym | $SO_2NHCH_2CONH_2$ | H | H |
| 1-1275 | 4-F-Ph | (α-Me-BnNH)-4-Pym | $SO_2NHOH$ | H | H |
| 1-1276 | 4-F-Ph | (α-Me-BnNH)-4-Pym | $SO_2NHOMe$ | H | H |
| 1-1277 | 4-F-Ph | (α-Me-BnNH)-4-Pym | $SO_2NHOEt$ | H | H |
| 1-1278 | 4-F-Ph | (α-Me-BnNH)-4-Pym | $SO_2NHOPr$ | H | H |
| 1-1279 | 4-F-Ph | (α-Me-BnNH)-4-Pym | $SO_2NHOAllyl$ | H | H |
| 1-1280 | 4-F-Ph | (α-Me-BnNH)-4-Pym | $SO_2NHOBn$ | H | H |
| 1-1281 | 4-F-Ph | (α-Me-BnNH)-4-Pym | $SO_2NHNH_2$ | H | H |
| 1-1282 | 4-F-Ph | (α-Me-BnNH)-4-Pym | $SO_2NHNHMe$ | H | H |
| 1-1283 | 4-F-Ph | (α-Me-BnNH)-4-Pym | $SO_2NHN(Me)_2$ | H | H |
| 1-1284 | 4-F-Ph | 4-Pyr | $SO_2NHMe$ | OH | H |
| 1-1285 | 4-F-Ph | 4-Pyr | $SO_2NHMe$ | Me | H |
| 1-1286 | 4-F-Ph | 4-Pyr | $SO_2NHMe$ | Et | H |
| 1-1287 | 4-F-Ph | 4-Pyr | $SO_2NHMe$ | F | H |
| 1-1288 | 4-F-Ph | 4-Pyr | $SO_2NHMe$ | OMe | H |

| | | | | | |
|---|---|---|---|---|---|
| 1-1289 | 4-F-Ph | 4-Pyr | $SO_2NHMe$ | OEt | H |
| 1-1290 | 4-F-Ph | 4-Pyr | $SO_2NHMe$ | =O | |
| 1-1291 | 4-F-Ph | 4-Pyr | $SO_2NHMe$ | $=CH_2$ | |
| 1-1292 | 4-F-Ph | 4-Pyr | $SO_2NHMe$ | $-CH_2CH_2-$ | |
| 1-1293 | 4-F-Ph | 4-Pyr | SOMe | OH | H |
| 1-1294 | 4-F-Ph | 4-Pyr | SOMe | Me | H |
| 1-1295 | 4-F-Ph | 4-Pyr | SOMe | Et | H |
| 1-1296 | 4-F-Ph | 4-Pyr | SOMe | F | H |
| 1-1297 | 4-F-Ph | 4-Pyr | SOMe | OMe | H |
| 1-1298 | 4-F-Ph | 4-Pyr | SOMe | OEt | H |
| 1-1299 | 4-F-Ph | 4-Pyr | SOMe | =O | |
| 1-1300 | 4-F-Ph | 4-Pyr | SOMe | $=CH_2$ | |
| 1-1301 | 4-F-Ph | 4-Pyr | SOMe | $-CH_2CH_2-$ | |
| 1-1302 | 4-F-Ph | 4-Pyr | $SO_2Me$ | OH | H |
| 1-1303 | 4-F-Ph | 4-Pyr | $SO_2Me$ | Me | H |
| 1-1304 | 4-F-Ph | 4-Pyr | $SO_2Me$ | Et | H |
| 1-1305 | 4-F-Ph | 4-Pyr | $SO_2Me$ | F | H |
| 1-1306 | 4-F-Ph | 4-Pyr | $SO_2Me$ | OMe | H |
| 1-1307 | 4-F-Ph | 4-Pyr | $SO_2Me$ | OEt | H |
| 1-1308 | 4-F-Ph | 4-Pyr | $SO_2Me$ | =O | |
| 1-1309 | 4-F-Ph | 4-Pyr | $SO_2Me$ | $=CH_2$ | |
| 1-1310 | 4-F-Ph | 4-Pyr | $SO_2Me$ | $-CH_2CH_2-$ | |
| 1-1311 | 3-Cl-4-F-Ph | 4-Pyr | $SO_2NHMe$ | OH | H |
| 1-1312 | 3-Cl-4-F-Ph | 4-Pyr | $SO_2NHMe$ | Me | H |
| 1-1313 | 3-Cl-4-F-Ph | 4-Pyr | $SO_2NHMe$ | Et | H |
| 1-1314 | 3-Cl-4-F-Ph | 4-Pyr | $SO_2NHMe$ | F | H |
| 1-1315 | 3-Cl-4-F-Ph | 4-Pyr | $SO_2NHMe$ | OMe | H |
| 1-1316 | 3-Cl-4-F-Ph | 4-Pyr | $SO_2NHMe$ | OEt | H |
| 1-1317 | 3-Cl-4-F-Ph | 4-Pyr | $SO_2NHMe$ | =O | |

| | | | | | |
|---|---|---|---|---|---|
| 1-1318 | 3-Cl-4-F-Ph | 4-Pyr | $SO_2NHMe$ | $=CH_2$ | |
| 1-1319 | 3-Cl-4-F-Ph | 4-Pyr | $SO_2NHMe$ | $-CH_2CH_2-$ | |
| 1-1320 | 3-Cl-4-F-Ph | 4-Pyr | SOMe | OH | H |
| 1-1321 | 3-Cl-4-F-Ph | 4-Pyr | SOMe | Me | H |
| 1-1322 | 3-Cl-4-F-Ph | 4-Pyr | SOMe | Et | H |
| 1-1323 | 3-Cl-4-F-Ph | 4-Pyr | SOMe | F | H |
| 1-1324 | 3-Cl-4-F-Ph | 4-Pyr | SOMe | OMe | H |
| 1-1325 | 3-Cl-4-F-Ph | 4-Pyr | SOMe | OEt | H |
| 1-1326 | 3-Cl-4-F-Ph | 4-Pyr | SOMe | =O | |
| 1-1327 | 3-Cl-4-F-Ph | 4-Pyr | SOMe | $=CH_2$ | |
| 1-1328 | 3-Cl-4-F-Ph | 4-Pyr | SOMe | $-CH_2CH_2-$ | |
| 1-1329 | 3-Cl-4-F-Ph | 4-Pyr | $SO_2Me$ | OH | H |
| 1-1330 | 3-Cl-4-F-Ph | 4-Pyr | $SO_2Me$ | Me | H |
| 1-1331 | 3-Cl-4-F-Ph | 4-Pyr | $SO_2Me$ | Et | H |
| 1-1332 | 3-Cl-4-F-Ph | 4-Pyr | $SO_2Me$ | F | H |
| 1-1333 | 3-Cl-4-F-Ph | 4-Pyr | $SO_2Me$ | OMe | H |
| 1-1334 | 3-Cl-4-F-Ph | 4-Pyr | $SO_2Me$ | OEt | H |
| 1-1335 | 3-Cl-4-F-Ph | 4-Pyr | $SO_2Me$ | =O | |

| | | | | | |
|---|---|---|---|---|---|
| 1-1336 | 3-Cl-4-F-Ph | 4-Pyr | $SO_2Me$ | $=CH_2$ | |
| 1-1337 | 3-Cl-4-F-Ph | 4-Pyr | $SO_2Me$ | $-CH_2CH_2-$ | |
| 1-1338 | 4-F-Ph | 4-Pyr | $COO(CH_2)_2N(Me)_2$ | H | H |
| 1-1339 | 4-F-Ph | 4-Pyr | $SO_2NHOCH_2CH(OH)CH_2OH$ | H | H |
| 1-1340 | 4-F-Ph | 4-Pyr | $CONHOCH_2CH(OH)CH_2OH$ | H | H |
| 1-1341 | 4-F-Ph | 4-Pyr | $SO_2CH_2CH_2OMe$ | H | H |
| 1-1342 | 4-F-Ph | 4-Pyr | $SO_2CH_2CH_2OH$ | H | H |
| 1-1343 | 4-F-Ph | 4-Pyr | $SO_2CH_2CH(OH)CH_2OH$ | H | H |
| 1-1344 | 3-Cl-4-F-Ph | 4-Pyr | $SO_2NHOCH_2CH(OH)CH_2OH$ | H | H |
| 1-1345 | 3-Cl-4-F-Ph | 4-Pyr | $CONHOCH_2CH(OH)CH_2OH$ | H | H |
| 1-1346 | 3-Cl-4-F-Ph | 4-Pyr | $SO_2CH_2CH_2OMe$ | H | H |
| 1-1347 | 3-Cl-4-F-Ph | 4-Pyr | $SO_2CH_2CH_2OH$ | H | H |
| 1-1348 | 3-Cl-4-F-Ph | 4-Pyr | $SO_2CH_2CH(OH)CH_2OH$ | H | H |

Table 2

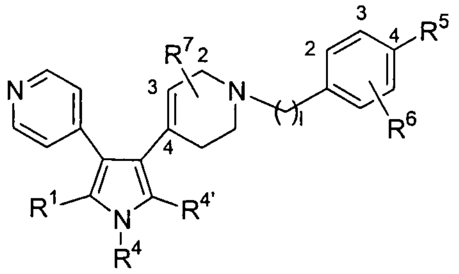

(I-2)

| Compdund No. | $R^1$ | $R^4$ | $R^{4'}$ | $R^5$ | $R^6$ | $R^7$ | l |
|---|---|---|---|---|---|---|---|
| 2-1 | 4-F-Ph | H | H | $SO_2NHMe$ | 3-F | H | 2 |

| | | | | | | | |
|---|---|---|---|---|---|---|---|
| 2-2 | 4-F-Ph | H | H | $SO_2NHMe$ | 3-Cl | H | 2 |
| 2-3 | 4-F-Ph | H | H | $SO_2NHMe$ | 3-OMe | H | 2 |
| 2-4 | 4-F-Ph | H | H | $SO_2NHMe$ | 3-Me | H | 2 |
| 2-5 | 4-F-Ph | Me | H | $SO_2NHMe$ | H | H | 2 |
| 2-6 | 4-F-Ph | H | Me | $SO_2NHMe$ | H | H | 2 |
| 2-7 | 4-F-Ph | Me | Me | $SO_2NHMe$ | H | H | 2 |
| 2-8 | 4-F-Ph | H | H | $SO_2NHMe$ | H | 3-Me | 2 |
| 2-9 | 4-F-Ph | H | H | $SO_2NHMe$ | H | 2-Me | 2 |
| 2-10 | 4-F-Ph | H | H | $SO_2NHMe$ | H | 2-Et | 2 |
| 2-11 | 4-F-Ph | H | H | $SO_2Me$ | 3-F | H | 2 |
| 2-12 | 4-F-Ph | H | H | $SO_2Me$ | 3-Cl | H | 2 |
| 2-13 | 4-F-Ph | H | H | $SO_2Me$ | 3-OMe | H | 2 |
| 2-14 | 4-F-Ph | H | H | $SO_2Me$ | 3-Me | H | 2 |
| 2-15 | 4-F-Ph | Me | H | $SO_2Me$ | H | H | 2 |
| 2-16 | 4-F-Ph | H | Me | $SO_2Me$ | H | H | 2 |
| 2-17 | 4-F-Ph | Me | Me | $SO_2Me$ | H | H | 2 |
| 2-18 | 4-F-Ph | H | H | $SO_2Me$ | H | 3-Me | 2 |
| 2-19 | 4-F-Ph | H | H | $SO_2Me$ | H | 2-Me | 2 |
| 2-20 | 4-F-Ph | H | H | $SO_2Me$ | H | 2-Et | 2 |
| 2-21 | 3-Cl-4-F-Ph | H | H | $SO_2NHMe$ | 3-F | H | 2 |
| 2-22 | 3-Cl-4-F-Ph | H | H | $SO_2NHMe$ | 3-Cl | H | 2 |
| 2-23 | 3-Cl-4-F-Ph | H | H | $SO_2NHMe$ | 3-OMe | H | 2 |
| 2-24 | 3-Cl-4-F-Ph | H | H | $SO_2NHMe$ | 3-Me | H | 2 |
| 2-25 | 3-Cl-4-F-Ph | Me | H | $SO_2NHMe$ | H | H | 2 |
| 2-26 | 3-Cl-4-F-Ph | H | Me | $SO_2NHMe$ | H | H | 2 |
| 2-27 | 3-Cl-4-F-Ph | Me | Me | $SO_2NHMe$ | H | H | 2 |
| 2-28 | 3-Cl-4-F-Ph | H | H | $SO_2NHMe$ | H | 3-Me | 2 |
| 2-29 | 3-Cl-4-F-Ph | H | H | $SO_2NHMe$ | H | 2-Me | 2 |
| 2-30 | 3-Cl-4-F-Ph | H | H | $SO_2NHMe$ | H | 2-Et | 2 |
| 2-31 | 3-Cl-4-F-Ph | H | H | $SO_2Me$ | 3-F | H | 2 |
| 2-32 | 3-Cl-4-F-Ph | H | H | $SO_2Me$ | 3-Cl | H | 2 |
| 2-33 | 3-Cl-4-F-Ph | H | H | $SO_2Me$ | 3-OMe | H | 2 |
| 2-34 | 3-Cl-4-F-Ph | H | H | $SO_2Me$ | 3-Me | H | 2 |
| 2-35 | 3-Cl-4-F-Ph | Me | H | $SO_2Me$ | H | H | 2 |
| 2-36 | 3-Cl-4-F-Ph | H | Me | $SO_2Me$ | H | H | 2 |
| 2-37 | 3-Cl-4-F-Ph | Me | Me | $SO_2Me$ | H | H | 2 |

| | | | | | | | |
|---|---|---|---|---|---|---|---|
| 2-38 | 3-Cl-4-F-Ph | H | H | $SO_2Me$ | H | 3-Me | 2 |
| 2-39 | 3-Cl-4-F-Ph | H | H | $SO_2Me$ | H | 2-Me | 2 |
| 2-40 | 3-Cl-4-F-Ph | H | H | $SO_2Me$ | H | 2-Et | 2 |
| 2-41 | 4-F-Ph | H | H | $SO_2NHMe$ | H | H | 1 |
| 2-42 | 4-F-Ph | H | H | $SO_2NHMe$ | H | H | 3 |
| 2-43 | 4-F-Ph | H | H | CONHMe | H | H | 4 |
| 2-44 | 4-F-Ph | H | H | $SO_2Me$ | H | H | 5 |

Table 3

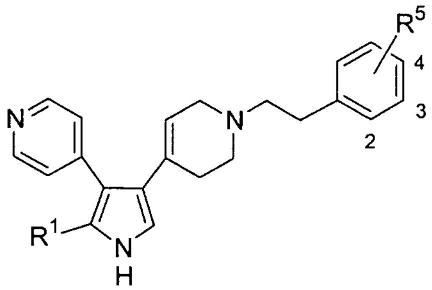

(I-3)

| Compound No. | $R^1$ | $R^5$ |
|---|---|---|
| 3-1 | 4-F-Ph | 2-COOH |
| 3-2 | 4-F-Ph | 3-COOH |
| 3-3 | 4-F-Ph | 2-$CONH_2$ |
| 3-4 | 4-F-Ph | 3-$CONH_2$ |
| 3-5 | 4-F-Ph | 3-CONHMe |
| 3-6 | 4-F-Ph | 3-CONHEt |
| 3-7 | 4-F-Ph | 3-CONHPr |
| 3-8 | 4-F-Ph | 3-CONH-i-Pr |
| 3-9 | 4-F-Ph | 2-$SO_2NH_2$ |
| 3-10 | 4-F-Ph | 3-$SO_2NH_2$ |
| 3-11 | 4-F-Ph | 3-$SO_2$NHMe |
| 3-12 | 4-F-Ph | 3-$SO_2$NHEt |
| 3-13 | 4-F-Ph | 3-$SO_2$NHPr |
| 3-14 | 4-F-Ph | 3-$SO_2$NH-i-Pr |
| 3-15 | 4-F-Ph | 2-SOMe |
| 3-16 | 4-F-Ph | 3-SOMe |
| 3-17 | 4-F-Ph | 2-$SO_2$Me |
| 3-18 | 4-F-Ph | 3-$SO_2$Me |

| | | |
|---|---|---|
| 3-19 | 3-Cl-4-F-Ph | 2-COOH |
| 3-20 | 3-Cl-4-F-Ph | 3-COOH |
| 3-21 | 3-Cl-4-F-Ph | 2-$CONH_2$ |
| 3-22 | 3-Cl-4-F-Ph | 3-$CONH_2$ |
| 3-23 | 3-Cl-4-F-Ph | 3-CONHMe |
| 3-24 | 3-Cl-4-F-Ph | 3-CONHEt |
| 3-25 | 3-Cl-4-F-Ph | 3-CONHPr |
| 3-26 | 3-Cl-4-F-Ph | 3-CONH-i-Pr |
| 3-27 | 3-Cl-4-F-Ph | 2-$SO_2NH_2$ |
| 3-28 | 3-Cl-4-F-Ph | 3-$SO_2NH_2$ |
| 3-29 | 3-Cl-4-F-Ph | 3-$SO_2$NHMe |
| 2-30 | 3-Cl-4-F-Ph | 3-$SO_2$NHEt |
| 3-31 | 3-Cl-4-F-Ph | 3-$SO_2$NHPr |
| 3-32 | 3-Cl-4-F-Ph | 3-$SO_2$NH-i-Pr |
| 3-33 | 3-Cl-4-F-Ph | 2-SOMe |
| 3-34 | 3-Cl-4-F-Ph | 3-SOMe |
| 3-35 | 3-Cl-4-F-Ph | 2-$SO_2$Me |
| 3-36 | 3-Cl-4-F-Ph | 3-$SO_2$Me |
| 3-37 | 3-Me-Ph | 4-$CONH_2$ |
| 3-38 | 3-Me-Ph | 4-CONHMe |
| 3-39 | 3-Me-Ph | 4-CONHEt |
| 3-40 | 3-Me-Ph | 4-CONHPr |
| 3-41 | 3-Me-Ph | 4-CONH-i-Pr |
| 3-42 | 3-Me-Ph | 4-$SO_2NH_2$ |
| 3-43 | 3-Me-Ph | 4-$SO_2$NHMe |
| 3-44 | 3-Me-Ph | 4-$SO_2$NHEt |
| 3-45 | 3-Me-Ph | 4-$SO_2$NHPr |
| 3-46 | 3-Me-Ph | 4-$SO_2$NH-i-Pr |
| 3-47 | 3-Me-Ph | 4-SOMe |
| 3-48 | 3-Me-Ph | 4-$SO_2$Me |
| 3-49 | 3-Me-Ph | 4-$SO_2CH_2CH_2OH$ |
| 3-50 | 3-Me-Ph | 4-$SO_2CH_2CH_2$OMe |

Table 4

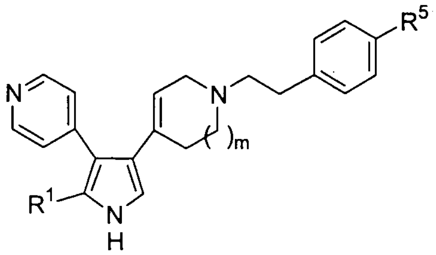

(I-4)

| Compound No. | $R^1$ | $R^5$ | m |
|---|---|---|---|
| 4-1 | 4-F-Ph | $CONH_2$ | 0 |
| 4-2 | 4-F-Ph | CONHMe | 0 |
| 4-3 | 4-F-Ph | CONHEt | 0 |
| 4-4 | 4-F-Ph | CONHPr | 0 |
| 4-5 | 4-F-Ph | CONH-i-Pr | 0 |
| 4-6 | 4-F-Ph | CONHBu | 0 |
| 4-7 | 4-F-Ph | $SO_2NH_2$ | 0 |
| 4-8 | 4-F-Ph | $SO_2NHMe$ | 0 |
| 4-9 | 4-F-Ph | $SO_2NHEt$ | 0 |
| 4-10 | 4-F-Ph | $SO_2NHPr$ | 0 |
| 4-11 | 4-F-Ph | $SO_2NH$-i-Pr | 0 |
| 4-12 | 4-F-Ph | $SO_2NHBu$ | 0 |
| 4-13 | 4-F-Ph | SOMe | 0 |
| 4-14 | 4-F-Ph | SOEt | 0 |
| 4-15 | 4-F-Ph | SOPr | 0 |
| 4-16 | 4-F-Ph | $SO_2Me$ | 0 |
| 4-17 | 4-F-Ph | $SO_2Et$ | 0 |
| 4-18 | 4-F-Ph | $SO_2Pr$ | 0 |
| 4-19 | 4-F-Ph | COMe | 0 |
| 4-20 | 4-F-Ph | COEt | 0 |
| 4-21 | 4-F-Ph | COPr | 0 |
| 4-22 | 3-Cl-4-F-Ph | $CONH_2$ | 0 |
| 4-23 | 3-Cl-4-F-Ph | CONHMe | 0 |
| 4-24 | 3-Cl-4-F-Ph | CONHEt | 0 |
| 4-25 | 3-Cl-4-F-Ph | CONHPr | 0 |
| 4-26 | 3-Cl-4-F-Ph | CONH-i-Pr | 0 |
| 4-27 | 3-Cl-4-F-Ph | CONHBu | 0 |
| 4-28 | 3-Cl-4-F-Ph | $SO_2NH_2$ | 0 |

| | | | |
|---|---|---|---|
| 4-29 | 3-Cl-4-F-Ph | $SO_2NHMe$ | 0 |
| 4-30 | 3-Cl-4-F-Ph | $SO_2NHEt$ | 0 |
| 4-31 | 3-Cl-4-F-Ph | $SO_2NHPr$ | 0 |
| 4-32 | 3-Cl-4-F-Ph | $SO_2NH$-i-Pr | 0 |
| 4-33 | 3-Cl-4-F-Ph | $SO_2NHBu$ | 0 |
| 4-34 | 3-Cl-4-F-Ph | SOMe | 0 |
| 4-35 | 3-Cl-4-F-Ph | SOEt | 0 |
| 4-36 | 3-Cl-4-F-Ph | SOPr | 0 |
| 4-37 | 3-Cl-4-F-Ph | $SO_2Me$ | 0 |
| 4-38 | 3-Cl-4-F-Ph | $SO_2Et$ | 0 |
| 4-39 | 3-Cl-4-F-Ph | $SO_2Pr$ | 0 |
| 4-40 | 3-Cl-4-F-Ph | COMe | 0 |
| 4-41 | 3-Cl-4-F-Ph | COEt | 0 |
| 4-42 | 3-Cl-4-F-Ph | COPr | 0 |
| 4-43 | 4-F-Ph | $CONH_2$ | 2 |
| 4-44 | 4-F-Ph | CONHMe | 2 |
| 4-45 | 4-F-Ph | CONHEt | 2 |
| 4-46 | 4-F-Ph | CONHPr | 2 |
| 4-47 | 4-F-Ph | CONH-i-Pr | 2 |
| 4-48 | 4-F-Ph | CONHBu | 2 |
| 4-49 | 4-F-Ph | $SO_2NH_2$ | 2 |
| 4-50 | 4-F-Ph | $SO_2NHMe$ | 2 |
| 4-51 | 4-F-Ph | $SO_2NHEt$ | 2 |
| 4-52 | 4-F-Ph | $SO_2NHPr$ | 2 |
| 4-53 | 4-F-Ph | $SO_2NH$-i-Pr | 2 |
| 4-54 | 4-F-Ph | $SO_2NHBu$ | 2 |
| 4-55 | 4-F-Ph | SOMe | 2 |
| 4-56 | 4-F-Ph | SOEt | 2 |
| 4-57 | 4-F-Ph | SOPr | 2 |
| 4-58 | 4-F-Ph | $SO_2Me$ | 2 |
| 4-59 | 4-F-Ph | $SO_2Et$ | 2 |
| 4-60 | 4-F-Ph | $SO_2Pr$ | 2 |
| 4-61 | 4-F-Ph | COMe | 2 |
| 4-62 | 4-F-Ph | COEt | 2 |
| 4-63 | 4-F-Ph | COPr | 2 |
| 4-64 | 3-Cl-4-F-Ph | $CONH_2$ | 2 |

| | | | |
|---|---|---|---|
| 4-65 | 3-Cl-4-F-Ph | CONHMe | 2 |
| 4-66 | 3-Cl-4-F-Ph | CONHEt | 2 |
| 4-67 | 3-Cl-4-F-Ph | CONHPr | 2 |
| 4-68 | 3-Cl-4-F-Ph | CONH-i-Pr | 2 |
| 4-69 | 3-Cl-4-F-Ph | CONHBu | 2 |
| 4-70 | 3-Cl-4-F-Ph | $SO_2NH_2$ | 2 |
| 4-71 | 3-Cl-4-F-Ph | $SO_2NHMe$ | 2 |
| 4-72 | 3-Cl-4-F-Ph | $SO_2NHEt$ | 2 |
| 4-73 | 3-Cl-4-F-Ph | $SO_2NHPr$ | 2 |
| 4-74 | 3-Cl-4-F-Ph | $SO_2NH$-i-Pr | 2 |
| 4-75 | 3-Cl-4-F-Ph | $SO_2NHBu$ | 2 |
| 4-76 | 3-Cl-4-F-Ph | SOMe | 2 |
| 4-77 | 3-Cl-4-F-Ph | SOEt | 2 |
| 4-78 | 3-Cl-4-F-Ph | SOPr | 2 |
| 4-79 | 3-Cl-4-F-Ph | $SO_2Me$ | 2 |
| 4-80 | 3-Cl-4-F-Ph | $SO_2Et$ | 2 |
| 4-81 | 3-Cl-4-F-Ph | $SO_2Pr$ | 2 |
| 4-82 | 3-Cl-4-F-Ph | COMe | 2 |
| 4-83 | 3-Cl-4-F-Ph | COEt | 2 |
| 4-84 | 3-Cl-4-F-Ph | COPr | 2 |

Table 5

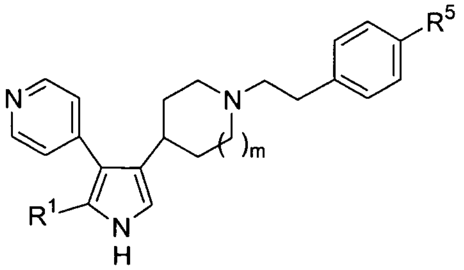

(I-5)

| Compound No. | $R^1$ | $R^5$ | m |
| --- | --- | --- | --- |
| 5-1 | 4-F-Ph | $CONH_2$ | 0 |
| 5-2 | 4-F-Ph | CONHMe | 0 |
| 5-3 | 4-F-Ph | CONHEt | 0 |
| 5-4 | 4-F-Ph | CONHPr | 0 |
| 5-5 | 4-F-Ph | CONH-i-Pr | 0 |
| 5-6 | 4-F-Ph | CONHBu | 0 |

| | | | |
|---|---|---|---|
| 5-7 | 4-F-Ph | $SO_2NH_2$ | 0 |
| 5-8 | 4-F-Ph | $SO_2NHMe$ | 0 |
| 5-9 | 4-F-Ph | $SO_2NHEt$ | 0 |
| 5-10 | 4-F-Ph | $SO_2NHPr$ | 0 |
| 5-11 | 4-F-Ph | $SO_2NH$-i-Pr | 0 |
| 5-12 | 4-F-Ph | $SO_2NHBu$ | 0 |
| 5-13 | 4-F-Ph | SOMe | 0 |
| 5-14 | 4-F-Ph | SOEt | 0 |
| 5-15 | 4-F-Ph | SOPr | 0 |
| 5-16 | 4-F-Ph | $SO_2Me$ | 0 |
| 5-17 | 4-F-Ph | $SO_2Et$ | 0 |
| 5-18 | 4-F-Ph | $SO_2Pr$ | 0 |
| 5-19 | 4-F-Ph | COMe | 0 |
| 5-20 | 4-F-Ph | COEt | 0 |
| 5-21 | 4-F-Ph | COPr | 0 |
| 5-22 | 3-Cl-4-F-Ph | $CONH_2$ | 0 |
| 5-23 | 3-Cl-4-F-Ph | CONHMe | 0 |
| 5-24 | 3-Cl-4-F-Ph | CONHEt | 0 |
| 5-25 | 3-Cl-4-F-Ph | CONHPr | 0 |
| 5-26 | 3-Cl-4-F-Ph | CONH-i-Pr | 0 |
| 5-27 | 3-Cl-4-F-Ph | CONHBu | 0 |
| 5-28 | 3-Cl-4-F-Ph | $SO_2NH_2$ | 0 |
| 5-29 | 3-Cl-4-F-Ph | $SO_2NHMe$ | 0 |
| 5-30 | 3-Cl-4-F-Ph | $SO_2NHEt$ | 0 |
| 5-31 | 3-Cl-4-F-Ph | $SO_2NHPr$ | 0 |
| 5-32 | 3-Cl-4-F-Ph | $SO_2NH$-i-Pr | 0 |
| 5-33 | 3-Cl-4-F-Ph | $SO_2NHBu$ | 0 |
| 5-34 | 3-Cl-4-F-Ph | SOMe | 0 |
| 5-35 | 3-Cl-4-F-Ph | SOEt | 0 |
| 5-36 | 3-Cl-4-F-Ph | SOPr | 0 |
| 5-37 | 3-Cl-4-F-Ph | $SO_2Me$ | 0 |
| 5-38 | 3-Cl-4-F-Ph | $SO_2Et$ | 0 |
| 5-39 | 3-Cl-4-F-Ph | $SO_2Pr$ | 0 |
| 5-40 | 3-Cl-4-F-Ph | COMe | 0 |
| 5-41 | 3-Cl-4-F-Ph | COEt | 0 |
| 5-42 | 3-Cl-4-F-Ph | COPr | 0 |

| | | | |
|---|---|---|---|
| 5-43 | 4-F-Ph | $CONH_2$ | 1 |
| 5-44 | 4-F-Ph | CONHMe | 1 |
| 5-45 | 4-F-Ph | CONHEt | 1 |
| 5-46 | 4-F-Ph | CONHPr | 1 |
| 5-47 | 4-F-Ph | CONH-i-Pr | 1 |
| 5-48 | 4-F-Ph | CONHBu | 1 |
| 5-49 | 4-F-Ph | $SO_2NH_2$ | 1 |
| 5-50 | 4-F-Ph | $SO_2$NHMe | 1 |
| 5-51 | 4-F-Ph | $SO_2$NHEt | 1 |
| 5-52 | 4-F-Ph | $SO_2$NHPr | 1 |
| 5-53 | 4-F-Ph | $SO_2$NH-i-Pr | 1 |
| 5-54 | 4-F-Ph | $SO_2$NHBu | 1 |
| 5-55 | 4-F-Ph | SOMe | 1 |
| 5-56 | 4-F-Ph | SOEt | 1 |
| 5-57 | 4-F-Ph | SOPr | 1 |
| 5-58 | 4-F-Ph | $SO_2$Me | 1 |
| 5-59 | 4-F-Ph | $SO_2$Et | 1 |
| 5-60 | 4-F-Ph | $SO_2$Pr | 1 |
| 5-61 | 4-F-Ph | COMe | 1 |
| 5-62 | 4-F-Ph | COEt | 1 |
| 5-63 | 4-F-Ph | COPr | 1 |
| 5-64 | 3-Cl-4-F-Ph | $CONH_2$ | 1 |
| 5-65 | 3-Cl-4-F-Ph | CONHMe | 1 |
| 5-66 | 3-Cl-4-F-Ph | CONHEt | 1 |
| 5-67 | 3-Cl-4-F-Ph | CONHPr | 1 |
| 5-68 | 3-Cl-4-F-Ph | CONH-i-Pr | 1 |
| 5-69 | 3-Cl-4-F-Ph | CONHBu | 1 |
| 5-70 | 3-Cl-4-F-Ph | $SO_2NH_2$ | 1 |
| 5-71 | 3-Cl-4-F-Ph | $SO_2$NHMe | 1 |
| 5-72 | 3-Cl-4-F-Ph | $SO_2$NHEt | 1 |
| 5-73 | 3-Cl-4-F-Ph | $SO_2$NHPr | 1 |
| 5-74 | 3-Cl-4-F-Ph | $SO_2$NH-i-Pr | 1 |
| 5-75 | 3-Cl-4-F-Ph | $SO_2$NHBu | 1 |
| 5-76 | 3-Cl-4-F-Ph | SOMe | 1 |
| 5-77 | 3-Cl-4-F-Ph | SOEt | 1 |
| 5-78 | 3-Cl-4-F-Ph | SOPr | 1 |

| | | | |
|---|---|---|---|
| 5-79 | 3-Cl-4-F-Ph | $SO_2Me$ | 1 |
| 5-80 | 3-Cl-4-F-Ph | $SO_2Et$ | 1 |
| 5-81 | 3-Cl-4-F-Ph | $SO_2Pr$ | 1 |
| 5-82 | 3-Cl-4-F-Ph | COMe | 1 |
| 5-83 | 3-Cl-4-F-Ph | COEt | 1 |
| 5-84 | 3-Cl-4-F-Ph | COPr | 1 |
| 5-85 | 4-F-Ph | $CONH_2$ | 2 |
| 5-86 | 4-F-Ph | CONHMe | 2 |
| 5-87 | 4-F-Ph | CONHEt | 2 |
| 5-88 | 4-F-Ph | CONHPr | 2 |
| 5-89 | 4-F-Ph | CONH-i-Pr | 2 |
| 5-90 | 4-F-Ph | CONHBu | 2 |
| 5-91 | 4-F-Ph | $SO_2NH_2$ | 2 |
| 5-92 | 4-F-Ph | $SO_2NHMe$ | 2 |
| 5-93 | 4-F-Ph | $SO_2NHEt$ | 2 |
| 5-94 | 4-F-Ph | $SO_2NHPr$ | 2 |
| 5-95 | 4-F-Ph | $SO_2NH$-i-Pr | 2 |
| 5-96 | 4-F-Ph | $SO_2NHBu$ | 2 |
| 5-97 | 4-F-Ph | SOMe | 2 |
| 5-98 | 4-F-Ph | SOEt | 2 |
| 5-99 | 4-F-Ph | SOPr | 2 |
| 5-100 | 4-F-Ph | $SO_2Me$ | 2 |
| 5-101 | 4-F-Ph | $SO_2Et$ | 2 |
| 5-102 | 4-F-Ph | $SO_2Pr$ | 2 |
| 5-103 | 4-F-Ph | COMe | 2 |
| 5-104 | 4-F-Ph | COEt | 2 |
| 5-105 | 4-F-Ph | COPr | 2 |
| 5-106 | 3-Cl-4-F-Ph | $CONH_2$ | 2 |
| 5-107 | 3-Cl-4-F-Ph | CONHMe | 2 |
| 5-108 | 3-Cl-4-F-Ph | CONHEt | 2 |
| 5-109 | 3-Cl-4-F-Ph | CONHPr | 2 |
| 5-110 | 3-Cl-4-F-Ph | CONH-i-Pr | 2 |
| 5-111 | 3-Cl-4-F-Ph | CONHBu | 2 |
| 5-112 | 3-Cl-4-F-Ph | $SO_2NH_2$ | 2 |
| 5-113 | 3-Cl-4-F-Ph | $SO_2NHMe$ | 2 |
| 5-114 | 3-Cl-4-F-Ph | $SO_2NHEt$ | 2 |

| | | | |
|---|---|---|---|
| 5-115 | 3-Cl-4-F-Ph | $SO_2NHPr$ | 2 |
| 5-116 | 3-Cl-4-F-Ph | $SO_2NH$-i-Pr | 2 |
| 5-117 | 3-Cl-4-F-Ph | $SO_2NHBu$ | 2 |
| 5-118 | 3-Cl-4-F-Ph | SOMe | 2 |
| 5-119 | 3-Cl-4-F-Ph | SOEt | 2 |
| 5-120 | 3-Cl-4-F-Ph | SOPr | 2 |
| 5-121 | 3-Cl-4-F-Ph | $SO_2Me$ | 2 |
| 5-122 | 3-Cl-4-F-Ph | $SO_2Et$ | 2 |
| 5-123 | 3-Cl-4-F-Ph | $SO_2Pr$ | 2 |
| 5-124 | 3-Cl-4-F-Ph | COMe | 2 |
| 5-125 | 3-Cl-4-F-Ph | COEt | 2 |
| 5-126 | 3-Cl-4-F-Ph | COPr | 2 |

Table 6

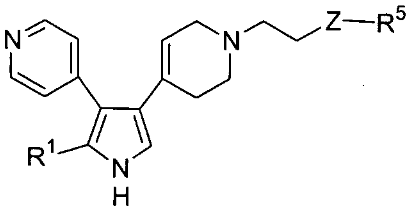

(I-6)

| Compound No. | $R^1$ | Z | $R^5$ |
|---|---|---|---|
| 6-1 | 4-F-Ph | Ring 1 | $CONH_2$ |
| 6-2 | 4-F-Ph | Ring 1 | CONHMe |
| 6-3 | 4-F-Ph | Ring 1 | CONHEt |
| 6-4 | 4-F-Ph | Ring 1 | CONHPr |
| 6-5 | 4-F-Ph | Ring 1 | CONH-i-Pr |
| 6-6 | 4-F-Ph | Ring 1 | CONHBu |
| 6-7 | 4-F-Ph | Ring 1 | $SO_2NH_2$ |
| 6-8 | 4-F-Ph | Ring 1 | $SO_2NHMe$ |
| 6-9 | 4-F-Ph | Ring 1 | $SO_2NHEt$ |
| 6-10 | 4-F-Ph | Ring 1 | $SO_2NHPr$ |
| 6-11 | 4-F-Ph | Ring 1 | $SO_2NH$-i-Pr |
| 6-12 | 4-F-Ph | Ring 1 | $SO_2NHBu$ |
| 6-13 | 4-F-Ph | Ring 1 | SOMe |
| 6-14 | 4-F-Ph | Ring 1 | SOEt |

| | | | |
|---|---|---|---|
| 6-15 | 4-F-Ph | Ring 1 | SOPr |
| 6-16 | 4-F-Ph | Ring 1 | $SO_2Me$ |
| 6-17 | 4-F-Ph | Ring 1 | $SO_2Et$ |
| 6-18 | 4-F-Ph | Ring 1 | $SO_2Pr$ |
| 6-19 | 4-F-Ph | Ring 1 | COMe |
| 6-20 | 4-F-Ph | Ring 1 | COEt |
| 6-21 | 4-F-Ph | Ring 1 | COPr |
| 6-22 | 3-Cl-4-F-Ph | Ring 1 | $CONH_2$ |
| 6-23 | 3-Cl-4-F-Ph | Ring 1 | CONHMe |
| 6-24 | 3-Cl-4-F-Ph | Ring 1 | CONHEt |
| 6-25 | 3-Cl-4-F-Ph | Ring 1 | CONHPr |
| 6-26 | 3-Cl-4-F-Ph | Ring 1 | CONH-i-Pr |
| 6-27 | 3-Cl-4-F-Ph | Ring 1 | CONHBu |
| 6-28 | 3-Cl-4-F-Ph | Ring 1 | $SO_2NH_2$ |
| 6-29 | 3-Cl-4-F-Ph | Ring 1 | $SO_2NHMe$ |
| 6-30 | 3-Cl-4-F-Ph | Ring 1 | $SO_2NHEt$ |
| 6-31 | 3-Cl-4-F-Ph | Ring 1 | $SO_2NHPr$ |
| 6-32 | 3-Cl-4-F-Ph | Ring 1 | $SO_2NH$-i-Pr |
| 6-33 | 3-Cl-4-F-Ph | Ring 1 | $SO_2NHBu$ |
| 6-34 | 3-Cl-4-F-Ph | Ring 1 | SOMe |
| 6-35 | 3-Cl-4-F-Ph | Ring 1 | SOEt |
| 6-36 | 3-Cl-4-F-Ph | Ring 1 | SOPr |
| 6-37 | 3-Cl-4-F-Ph | Ring 1 | $SO_2Me$ |
| 6-38 | 3-Cl-4-F-Ph | Ring 1 | $SO_2Et$ |
| 6-39 | 3-Cl-4-F-Ph | Ring 1 | $SO_2Pr$ |
| 6-40 | 3-Cl-4-F-Ph | Ring 1 | COMe |
| 6-41 | 3-Cl-4-F-Ph | Ring 1 | COEt |
| 6-42 | 3-Cl-4-F-Ph | Ring 1 | COPr |
| 6-43 | 4-F-Ph | Ring 2 | $CONH_2$ |
| 6-44 | 4-F-Ph | Ring 2 | CONHMe |
| 6-45 | 4-F-Ph | Ring 2 | CONHEt |
| 6-46 | 4-F-Ph | Ring 2 | CONHPr |
| 6-47 | 4-F-Ph | Ring 2 | CONH-i-Pr |
| 6-48 | 4-F-Ph | Ring 2 | CONHBu |
| 6-49 | 4-F-Ph | Ring 2 | $SO_2NH_2$ |
| 6-50 | 4-F-Ph | Ring 2 | $SO_2NHMe$ |

| | | | |
|---|---|---|---|
| 6-51 | 4-F-Ph | Ring 2 | $SO_2NHEt$ |
| 6-52 | 4-F-Ph | Ring 2 | $SO_2NHPr$ |
| 6-53 | 4-F-Ph | Ring 2 | $SO_2NH$-i-Pr |
| 6-54 | 4-F-Ph | Ring 2 | $SO_2NHBu$ |
| 6-55 | 4-F-Ph | Ring 2 | SOMe |
| 6-56 | 4-F-Ph | Ring 2 | SOEt |
| 6-57 | 4-F-Ph | Ring 2 | SOPr |
| 6-58 | 4-F-Ph | Ring 2 | $SO_2Me$ |
| 6-59 | 4-F-Ph | Ring 2 | $SO_2Et$ |
| 6-60 | 4-F-Ph | Ring 2 | $SO_2Pr$ |
| 6-61 | 4-F-Ph | Ring 2 | COMe |
| 6-62 | 4-F-Ph | Ring 2 | COEt |
| 6-63 | 4-F-Ph | Ring 2 | COPr |
| 6-64 | 3-Cl-4-F-Ph | Ring 2 | $CONH_2$ |
| 6-65 | 3-Cl-4-F-Ph | Ring 2 | CONHMe |
| 6-66 | 3-Cl-4-F-Ph | Ring 2 | CONHEt |
| 6-67 | 3-Cl-4-F-Ph | Ring 2 | CONHPr |
| 6-68 | 3-Cl-4-F-Ph | Ring 2 | CONH-i-Pr |
| 6-69 | 3-Cl-4-F-Ph | Ring 2 | CONHBu |
| 6-70 | 3-Cl-4-F-Ph | Ring 2 | $SO_2NH_2$ |
| 6-71 | 3-Cl-4-F-Ph | Ring 2 | $SO_2NHMe$ |
| 6-72 | 3-Cl-4-F-Ph | Ring 2 | $SO_2NHEt$ |
| 6-73 | 3-Cl-4-F-Ph | Ring 2 | $SO_2NHPr$ |
| 6-74 | 3-Cl-4-F-Ph | Ring 2 | $SO_2NH$-i-Pr |
| 6-75 | 3-Cl-4-F-Ph | Ring 2 | $SO_2NHBu$ |
| 6-76 | 3-Cl-4-F-Ph | Ring 2 | SOMe |
| 6-77 | 3-Cl-4-F-Ph | Ring 2 | SOEt |
| 6-78 | 3-Cl-4-F-Ph | Ring 2 | SOPr |
| 6-79 | 3-Cl-4-F-Ph | Ring 2 | $SO_2Me$ |
| 6-80 | 3-Cl-4-F-Ph | Ring 2 | $SO_2Et$ |
| 6-81 | 3-Cl-4-F-Ph | Ring 2 | $SO_2Pr$ |
| 6-82 | 3-Cl-4-F-Ph | Ring 2 | COMe |
| 6-83 | 3-Cl-4-F-Ph | Ring 2 | COEt |
| 6-84 | 3-Cl-4-F-Ph | Ring 2 | COPr |
| 6-85 | 4-F-Ph | Ring 3 | $CONH_2$ |
| 6-86 | 4-F-Ph | Ring 3 | CONHMe |

| | | | |
|---|---|---|---|
| 6-87 | 4-F-Ph | Ring 3 | CONHEt |
| 6-88 | 4-F-Ph | Ring 3 | CONHPr |
| 6-89 | 4-F-Ph | Ring 3 | CONH-i-Pr |
| 6-90 | 4-F-Ph | Ring 3 | CONHBu |
| 6-91 | 4-F-Ph | Ring 3 | $SO_2NH_2$ |
| 6-92 | 4-F-Ph | Ring 3 | $SO_2NHMe$ |
| 6-93 | 4-F-Ph | Ring 3 | $SO_2NHEt$ |
| 6-94 | 4-F-Ph | Ring 3 | $SO_2NHPr$ |
| 6-95 | 4-F-Ph | Ring 3 | $SO_2NH$-i-Pr |
| 6-96 | 4-F-Ph | Ring 3 | $SO_2NHBu$ |
| 6-97 | 4-F-Ph | Ring 3 | SOMe |
| 6-98 | 4-F-Ph | Ring 3 | SOEt |
| 6-99 | 4-F-Ph | Ring 3 | SOPr |
| 6-100 | 4-F-Ph | Ring 3 | $SO_2Me$ |
| 6-101 | 4-F-Ph | Ring 3 | $SO_2Et$ |
| 6-102 | 4-F-Ph | Ring 3 | $SO_2Pr$ |
| 6-103 | 4-F-Ph | Ring 3 | COMe |
| 6-104 | 4-F-Ph | Ring 3 | COEt |
| 6-105 | 4-F-Ph | Ring 3 | COPr |
| 6-106 | 3-Cl-4-F-Ph | Ring 3 | $CONH_2$ |
| 6-107 | 3-Cl-4-F-Ph | Ring 3 | CONHMe |
| 6-108 | 3-Cl-4-F-Ph | Ring 3 | CONHEt |
| 6-109 | 3-Cl-4-F-Ph | Ring 3 | CONHPr |
| 6-110 | 3-Cl-4-F-Ph | Ring 3 | CONH-i-Pr |
| 6-111 | 3-Cl-4-F-Ph | Ring 3 | CONHBu |
| 6-112 | 3-Cl-4-F-Ph | Ring 3 | $SO_2NH_2$ |
| 6-113 | 3-Cl-4-F-Ph | Ring 3 | $SO_2NHMe$ |
| 6-114 | 3-Cl-4-F-Ph | Ring 3 | $SO_2NHEt$ |
| 6-115 | 3-Cl-4-F-Ph | Ring 3 | $SO_2NHPr$ |
| 6-116 | 3-Cl-4-F-Ph | Ring 3 | $SO_2NH$-i-Pr |
| 6-117 | 3-Cl-4-F-Ph | Ring 3 | $SO_2NHBu$ |
| 6-118 | 3-Cl-4-F-Ph | Ring 3 | SOMe |
| 6-119 | 3-Cl-4-F-Ph | Ring 3 | SOEt |
| 6-120 | 3-Cl-4-F-Ph | Ring 3 | SOPr |
| 6-121 | 3-Cl-4-F-Ph | Ring 3 | $SO_2Me$ |
| 6-122 | 3-Cl-4-F-Ph | Ring 3 | $SO_2Et$ |

| | | | |
|---|---|---|---|
| 6-123 | 3-Cl-4-F-Ph | Ring 3 | $SO_2Pr$ |
| 6-124 | 3-Cl-4-F-Ph | Ring 3 | COMe |
| 6-125 | 3-Cl-4-F-Ph | Ring 3 | COEt |
| 6-126 | 3-Cl-4-F-Ph | Ring 3 | COPr |
| 6-127 | 4-F-Ph | Ring 4 | $CONH_2$ |
| 6-128 | 4-F-Ph | Ring 4 | CONHMe |
| 6-129 | 4-F-Ph | Ring 4 | CONHEt |
| 6-130 | 4-F-Ph | Ring 4 | CONHPr |
| 6-131 | 4-F-Ph | Ring 4 | CONH-i-Pr |
| 6-132 | 4-F-Ph | Ring 4 | CONHBu |
| 6-133 | 4-F-Ph | Ring 4 | $SO_2NH_2$ |
| 6-134 | 4-F-Ph | Ring 4 | $SO_2NHMe$ |
| 6-135 | 4-F-Ph | Ring 4 | $SO_2NHEt$ |
| 6-136 | 4-F-Ph | Ring 4 | $SO_2NHPr$ |
| 6-137 | 4-F-Ph | Ring 4 | $SO_2NH$-i-Pr |
| 6-138 | 4-F-Ph | Ring 4 | $SO_2NHBu$ |
| 6-139 | 4-F-Ph | Ring 4 | SOMe |
| 6-140 | 4-F-Ph | Ring 4 | SOEt |
| 6-141 | 4-F-Ph | Ring 4 | SOPr |
| 6-142 | 4-F-Ph | Ring 4 | $SO_2Me$ |
| 6-143 | 4-F-Ph | Ring 4 | $SO_2Et$ |
| 6-144 | 4-F-Ph | Ring 4 | $SO_2Pr$ |
| 6-145 | 4-F-Ph | Ring 4 | COMe |
| 6-146 | 4-F-Ph | Ring 4 | COEt |
| 6-147 | 4-F-Ph | Ring 4 | COPr |
| 6-148 | 3-Cl-4-F-Ph | Ring 4 | $CONH_2$ |
| 6-149 | 3-Cl-4-F-Ph | Ring 4 | CONHMe |
| 6-150 | 3-Cl-4-F-Ph | Ring 4 | CONHEt |
| 6-151 | 3-Cl-4-F-Ph | Ring 4 | CONHPr |
| 6-152 | 3-Cl-4-F-Ph | Ring 4 | CONH-i-Pr |
| 6-153 | 3-Cl-4-F-Ph | Ring 4 | CONHBu |
| 6-154 | 3-Cl-4-F-Ph | Ring 4 | $SO_2NH_2$ |
| 6-155 | 3-Cl-4-F-Ph | Ring 4 | $SO_2NHMe$ |
| 6-156 | 3-Cl-4-F-Ph | Ring 4 | $SO_2NHEt$ |
| 6-157 | 3-Cl-4-F-Ph | Ring 4 | $SO_2NHPr$ |
| 6-158 | 3-Cl-4-F-Ph | Ring 4 | $SO_2NH$-i-Pr |

| | | | |
|---|---|---|---|
| 6-159 | 3-Cl-4-F-Ph | Ring 4 | $SO_2NHBu$ |
| 6-160 | 3-Cl-4-F-Ph | Ring 4 | SOMe |
| 6-161 | 3-Cl-4-F-Ph | Ring 4 | SOEt |
| 6-162 | 3-Cl-4-F-Ph | Ring 4 | SOPr |
| 6-163 | 3-Cl-4-F-Ph | Ring 4 | $SO_2Me$ |
| 6-164 | 3-Cl-4-F-Ph | Ring 4 | $SO_2Et$ |
| 6-165 | 3-Cl-4-F-Ph | Ring 4 | $SO_2Pr$ |
| 6-166 | 3-Cl-4-F-Ph | Ring 4 | COMe |
| 6-167 | 3-Cl-4-F-Ph | Ring 4 | COEt |
| 6-168 | 3-Cl-4-F-Ph | Ring 4 | COPr |
| 6-169 | 4-F-Ph | Ring 5 | $CONH_2$ |
| 6-170 | 4-F-Ph | Ring 5 | CONHMe |
| 6-171 | 4-F-Ph | Ring 5 | CONHEt |
| 6-172 | 4-F-Ph | Ring 5 | CONHPr |
| 6-173 | 4-F-Ph | Ring 5 | CONH-i-Pr |
| 6-174 | 4-F-Ph | Ring 5 | CONHBu |
| 6-175 | 4-F-Ph | Ring 5 | $SO_2NH_2$ |
| 6-176 | 4-F-Ph | Ring 5 | $SO_2NHMe$ |
| 6-177 | 4-F-Ph | Ring 5 | $SO_2NHEt$ |
| 6-178 | 4-F-Ph | Ring 5 | $SO_2NHPr$ |
| 6-179 | 4-F-Ph | Ring 5 | $SO_2NH$-i-Pr |
| 6-180 | 4-F-Ph | Ring 5 | $SO_2NHBu$ |
| 6-181 | 4-F-Ph | Ring 5 | SOMe |
| 6-182 | 4-F-Ph | Ring 5 | SOEt |
| 6-183 | 4-F-Ph | Ring 5 | SOPr |
| 6-184 | 4-F-Ph | Ring 5 | $SO_2Me$ |
| 6-185 | 4-F-Ph | Ring 5 | $SO_2Et$ |
| 6-186 | 4-F-Ph | Ring 5 | $SO_2Pr$ |
| 6-187 | 4-F-Ph | Ring 5 | COMe |
| 6-188 | 4-F-Ph | Ring 5 | COEt |
| 6-189 | 4-F-Ph | Ring 5 | COPr |
| 6-190 | 3-Cl-4-F-Ph | Ring 5 | $CONH_2$ |
| 6-191 | 3-Cl-4-F-Ph | Ring 5 | CONHMe |
| 6-192 | 3-Cl-4-F-Ph | Ring 5 | CONHEt |
| 6-193 | 3-Cl-4-F-Ph | Ring 5 | CONHPr |
| 6-194 | 3-Cl-4-F-Ph | Ring 5 | CONH-i-Pr |

| | | | |
|---|---|---|---|
| 6-195 | 3-Cl-4-F-Ph | Ring 5 | CONHBu |
| 6-196 | 3-Cl-4-F-Ph | Ring 5 | $SO_2NH_2$ |
| 6-197 | 3-Cl-4-F-Ph | Ring 5 | $SO_2$NHMe |
| 6-198 | 3-Cl-4-F-Ph | Ring 5 | $SO_2$NHEt |
| 6-199 | 3-Cl-4-F-Ph | Ring 5 | $SO_2$NHPr |
| 6-200 | 3-Cl-4-F-Ph | Ring 5 | $SO_2$NH-i-Pr |
| 6-201 | 3-Cl-4-F-Ph | Ring 5 | $SO_2$NHBu |
| 6-202 | 3-Cl-4-F-Ph | Ring 5 | SOMe |
| 6-203 | 3-Cl-4-F-Ph | Ring 5 | SOEt |
| 6-204 | 3-Cl-4-F-Ph | Ring 5 | SOPr |
| 6-205 | 3-Cl-4-F-Ph | Ring 5 | $SO_2$Me |
| 6-206 | 3-Cl-4-F-Ph | Ring 5 | $SO_2$Et |
| 6-207 | 3-Cl-4-F-Ph | Ring 5 | $SO_2$Pr |
| 6-208 | 3-Cl-4-F-Ph | Ring 5 | COMe |
| 6-209 | 3-Cl-4-F-Ph | Ring 5 | COEt |
| 6-210 | 3-Cl-4-F-Ph | Ring 5 | COPr |
| 6-211 | 4-F-Ph | Ring 6 | $CONH_2$ |
| 6-212 | 4-F-Ph | Ring 6 | CONHMe |
| 6-213 | 4-F-Ph | Ring 6 | CONHEt |
| 6-214 | 4-F-Ph | Ring 6 | CONHPr |
| 6-215 | 4-F-Ph | Ring 6 | CONH-i-Pr |
| 6-216 | 4-F-Ph | Ring 6 | CONHBu |
| 6-217 | 4-F-Ph | Ring 6 | $SO_2NH_2$ |
| 6-218 | 4-F-Ph | Ring 6 | $SO_2$NHMe |
| 6-219 | 4-F-Ph | Ring 6 | $SO_2$NHEt |
| 6-220 | 4-F-Ph | Ring 6 | $SO_2$NHPr |
| 6-221 | 4-F-Ph | Ring 6 | $SO_2$NH-i-Pr |
| 6-222 | 4-F-Ph | Ring 6 | $SO_2$NHBu |
| 6-223 | 4-F-Ph | Ring 6 | SOMe |
| 6-224 | 4-F-Ph | Ring 6 | SOEt |
| 6-225 | 4-F-Ph | Ring 6 | SOPr |
| 6-226 | 4-F-Ph | Ring 6 | $SO_2$Me |
| 6-227 | 4-F-Ph | Ring 6 | $SO_2$Et |
| 6-228 | 4-F-Ph | Ring 6 | $SO_2$Pr |
| 6-229 | 4-F-Ph | Ring 6 | COMe |
| 6-230 | 4-F-Ph | Ring 6 | COEt |

| | | | |
|---|---|---|---|
| 6-231 | 4-F-Ph | Ring 6 | COPr |
| 6-232 | 3-Cl-4-F-Ph | Ring 6 | $CONH_2$ |
| 6-233 | 3-Cl-4-F-Ph | Ring 6 | CONHMe |
| 6-234 | 3-Cl-4-F-Ph | Ring 6 | CONHEt |
| 6-235 | 3-Cl-4-F-Ph | Ring 6 | CONHPr |
| 6-236 | 3-Cl-4-F-Ph | Ring 6 | CONH-i-Pr |
| 6-237 | 3-Cl-4-F-Ph | Ring 6 | CONHBu |
| 6-238 | 3-Cl-4-F-Ph | Ring 6 | $SO_2NH_2$ |
| 6-239 | 3-Cl-4-F-Ph | Ring 6 | $SO_2$NHMe |
| 6-240 | 3-Cl-4-F-Ph | Ring 6 | $SO_2$NHEt |
| 6-241 | 3-Cl-4-F-Ph | Ring 6 | $SO_2$NHPr |
| 6-242 | 3-Cl-4-F-Ph | Ring 6 | $SO_2$NH-i-Pr |
| 6-243 | 3-Cl-4-F-Ph | Ring 6 | $SO_2$NHBu |
| 6-244 | 3-Cl-4-F-Ph | Ring 6 | SOMe |
| 6-245 | 3-Cl-4-F-Ph | Ring 6 | SOEt |
| 6-246 | 3-Cl-4-F-Ph | Ring 6 | SOPr |
| 6-247 | 3-Cl-4-F-Ph | Ring 6 | $SO_2$Me |
| 6-248 | 3-Cl-4-F-Ph | Ring 6 | $SO_2$Et |
| 6-249 | 3-Cl-4-F-Ph | Ring 6 | $SO_2$Pr |
| 6-250 | 3-Cl-4-F-Ph | Ring 6 | COMe |
| 6-251 | 3-Cl-4-F-Ph | Ring 6 | COEt |
| 6-252 | 3-Cl-4-F-Ph | Ring 6 | COPr |
| 6-253 | 4-F-Ph | Ring 7 | $CONH_2$ |
| 6-254 | 4-F-Ph | Ring 7 | CONHMe |
| 6-255 | 4-F-Ph | Ring 7 | CONHEt |
| 6-256 | 4-F-Ph | Ring 7 | CONHPr |
| 6-257 | 4-F-Ph | Ring 7 | CONH-i-Pr |
| 6-258 | 4-F-Ph | Ring 7 | CONHBu |
| 6-259 | 4-F-Ph | Ring 7 | $SO_2NH_2$ |
| 6-260 | 4-F-Ph | Ring 7 | $SO_2$NHMe |
| 6-261 | 4-F-Ph | Ring 7 | $SO_2$NHEt |
| 6-262 | 4-F-Ph | Ring 7 | $SO_2$NHPr |
| 6-263 | 4-F-Ph | Ring 7 | $SO_2$NH-i-Pr |
| 6-264 | 4-F-Ph | Ring 7 | $SO_2$NHBu |
| 6-265 | 4-F-Ph | Ring 7 | SOMe |
| 6-266 | 4-F-Ph | Ring 7 | SOEt |

| | | | |
|---|---|---|---|
| 6-267 | 4-F-Ph | Ring 7 | SOPr |
| 6-268 | 4-F-Ph | Ring 7 | $SO_2Me$ |
| 6-269 | 4-F-Ph | Ring 7 | $SO_2Et$ |
| 6-270 | 4-F-Ph | Ring 7 | $SO_2Pr$ |
| 6-271 | 4-F-Ph | Ring 7 | COMe |
| 6-272 | 4-F-Ph | Ring 7 | COEt |
| 6-273 | 4-F-Ph | Ring 7 | COPr |
| 6-274 | 3-Cl-4-F-Ph | Ring 7 | $CONH_2$ |
| 6-275 | 3-Cl-4-F-Ph | Ring 7 | CONHMe |
| 6-276 | 3-Cl-4-F-Ph | Ring 7 | CONHEt |
| 6-277 | 3-Cl-4-F-Ph | Ring 7 | CONHPr |
| 6-278 | 3-Cl-4-F-Ph | Ring 7 | CONH-i-Pr |
| 6-279 | 3-Cl-4-F-Ph | Ring 7 | CONHBu |
| 6-280 | 3-Cl-4-F-Ph | Ring 7 | $SO_2NH_2$ |
| 6-281 | 3-Cl-4-F-Ph | Ring 7 | $SO_2NHMe$ |
| 6-282 | 3-Cl-4-F-Ph | Ring 7 | $SO_2NHEt$ |
| 6-283 | 3-Cl-4-F-Ph | Ring 7 | $SO_2NHPr$ |
| 6-284 | 3-Cl-4-F-Ph | Ring 7 | $SO_2NH$-i-Pr |
| 6-285 | 3-Cl-4-F-Ph | Ring 7 | $SO_2NHBu$ |
| 6-286 | 3-Cl-4-F-Ph | Ring 7 | SOMe |
| 6-287 | 3-Cl-4-F-Ph | Ring 7 | SOEt |
| 6-288 | 3-Cl-4-F-Ph | Ring 7 | SOPr |
| 6-289 | 3-Cl-4-F-Ph | Ring 7 | $SO_2Me$ |
| 6-290 | 3-Cl-4-F-Ph | Ring 7 | $SO_2Et$ |
| 6-291 | 3-Cl-4-F-Ph | Ring 7 | $SO_2Pr$ |
| 6-292 | 3-Cl-4-F-Ph | Ring 7 | COMe |
| 6-293 | 3-Cl-4-F-Ph | Ring 7 | COEt |
| 6-294 | 3-Cl-4-F-Ph | Ring 7 | COPr |
| 6-295 | 4-F-Ph | Ring 8 | $CONH_2$ |
| 6-296 | 4-F-Ph | Ring 8 | CONHMe |
| 6-297 | 4-F-Ph | Ring 8 | CONHEt |
| 6-298 | 4-F-Ph | Ring 8 | CONHPr |
| 6-299 | 4-F-Ph | Ring 8 | CONH-i-Pr |
| 6-300 | 4-F-Ph | Ring 8 | CONHBu |
| 6-301 | 4-F-Ph | Ring 8 | $SO_2NH_2$ |
| 6-302 | 4-F-Ph | Ring 8 | $SO_2NHMe$ |

| | | | |
|---|---|---|---|
| 6-303 | 4-F-Ph | Ring 8 | $SO_2NHEt$ |
| 6-304 | 4-F-Ph | Ring 8 | $SO_2NHPr$ |
| 6-305 | 4-F-Ph | Ring 8 | $SO_2NH$-i-Pr |
| 6-306 | 4-F-Ph | Ring 8 | $SO_2NHBu$ |
| 6-307 | 4-F-Ph | Ring 8 | SOMe |
| 6-308 | 4-F-Ph | Ring 8 | SOEt |
| 6-309 | 4-F-Ph | Ring 8 | SOPr |
| 6-310 | 4-F-Ph | Ring 8 | $SO_2Me$ |
| 6-311 | 4-F-Ph | Ring 8 | $SO_2Et$ |
| 6-312 | 4-F-Ph | Ring 8 | $SO_2Pr$ |
| 6-313 | 4-F-Ph | Ring 8 | COMe |
| 6-314 | 4-F-Ph | Ring 8 | COEt |
| 6-315 | 4-F-Ph | Ring 8 | COPr |
| 6-316 | 3-Cl-4-F-Ph | Ring 8 | $CONH_2$ |
| 6-317 | 3-Cl-4-F-Ph | Ring 8 | CONHMe |
| 6-318 | 3-Cl-4-F-Ph | Ring 8 | CONHEt |
| 6-319 | 3-Cl-4-F-Ph | Ring 8 | CONHPr |
| 6-320 | 3-Cl-4-F-Ph | Ring 8 | CONH-i-Pr |
| 6-321 | 3-Cl-4-F-Ph | Ring 8 | CONHBu |
| 6-322 | 3-Cl-4-F-Ph | Ring 8 | $SO_2NH_2$ |
| 6-323 | 3-Cl-4-F-Ph | Ring 8 | $SO_2NHMe$ |
| 6-324 | 3-Cl-4-F-Ph | Ring 8 | $SO_2NHEt$ |
| 6-325 | 3-Cl-4-F-Ph | Ring 8 | $SO_2NHPr$ |
| 6-326 | 3-Cl-4-F-Ph | Ring 8 | $SO_2NH$-i-Pr |
| 6-327 | 3-Cl-4-F-Ph | Ring 8 | $SO_2NHBu$ |
| 6-328 | 3-Cl-4-F-Ph | Ring 8 | SOMe |
| 6-329 | 3-Cl-4-F-Ph | Ring 8 | SOEt |
| 6-330 | 3-Cl-4-F-Ph | Ring 8 | SOPr |
| 6-331 | 3-Cl-4-F-Ph | Ring 8 | $SO_2Me$ |
| 6-332 | 3-Cl-4-F-Ph | Ring 8 | $SO_2Et$ |
| 6-333 | 3-Cl-4-F-Ph | Ring 8 | $SO_2Pr$ |
| 6-334 | 3-Cl-4-F-Ph | Ring 8 | COMe |
| 6-335 | 3-Cl-4-F-Ph | Ring 8 | COEt |
| 6-336 | 3-Cl-4-F-Ph | Ring 8 | COPr |
| 6-337 | 4-F-Ph | Ring 9 | $CONH_2$ |
| 6-338 | 4-F-Ph | Ring 9 | CONHMe |

| | | | |
|---|---|---|---|
| 6-339 | 4-F-Ph | Ring 9 | CONHEt |
| 6-340 | 4-F-Ph | Ring 9 | CONHPr |
| 6-341 | 4-F-Ph | Ring 9 | CONH-i-Pr |
| 6-342 | 4-F-Ph | Ring 9 | CONHBu |
| 6-343 | 4-F-Ph | Ring 9 | $SO_2NH_2$ |
| 6-344 | 4-F-Ph | Ring 9 | $SO_2$NHMe |
| 6-345 | 4-F-Ph | Ring 9 | $SO_2$NHEt |
| 6-346 | 4-F-Ph | Ring 9 | $SO_2$NHPr |
| 6-347 | 4-F-Ph | Ring 9 | $SO_2$NH-i-Pr |
| 6-348 | 4-F-Ph | Ring 9 | $SO_2$NHBu |
| 6-349 | 4-F-Ph | Ring 9 | SOMe |
| 6-350 | 4-F-Ph | Ring 9 | SOEt |
| 6-351 | 4-F-Ph | Ring 9 | SOPr |
| 6-352 | 4-F-Ph | Ring 9 | $SO_2$Me |
| 6-353 | 4-F-Ph | Ring 9 | $SO_2$Et |
| 6-354 | 4-F-Ph | Ring 9 | $SO_2$Pr |
| 6-355 | 4-F-Ph | Ring 9 | COMe |
| 6-356 | 4-F-Ph | Ring 9 | COEt |
| 6-357 | 4-F-Ph | Ring 9 | COPr |
| 6-358 | 3-Cl-4-F-Ph | Ring 9 | $CONH_2$ |
| 6-359 | 3-Cl-4-F-Ph | Ring 9 | CONHMe |
| 6-360 | 3-Cl-4-F-Ph | Ring 9 | CONHEt |
| 6-361 | 3-Cl-4-F-Ph | Ring 9 | CONHPr |
| 6-362 | 3-Cl-4-F-Ph | Ring 9 | CONH-i-Pr |
| 6-363 | 3-Cl-4-F-Ph | Ring 9 | CONHBu |
| 6-364 | 3-Cl-4-F-Ph | Ring 9 | $SO_2NH_2$ |
| 6-365 | 3-Cl-4-F-Ph | Ring 9 | $SO_2$NHMe |
| 6-366 | 3-Cl-4-F-Ph | Ring 9 | $SO_2$NHEt |
| 6-367 | 3-Cl-4-F-Ph | Ring 9 | $SO_2$NHPr |
| 6-368 | 3-Cl-4-F-Ph | Ring 9 | $SO_2$NH-i-Pr |
| 6-369 | 3-Cl-4-F-Ph | Ring 9 | $SO_2$NHBu |
| 6-370 | 3-Cl-4-F-Ph | Ring 9 | SOMe |
| 6-371 | 3-Cl-4-F-Ph | Ring 9 | SOEt |
| 6-372 | 3-Cl-4-F-Ph | Ring 9 | SOPr |
| 6-373 | 3-Cl-4-F-Ph | Ring 9 | $SO_2$Me |
| 6-374 | 3-Cl-4-F-Ph | Ring 9 | $SO_2$Et |

| | | | |
|---|---|---|---|
| 6-375 | 3-Cl-4-F-Ph | Ring 9 | $SO_2Pr$ |
| 6-376 | 3-Cl-4-F-Ph | Ring 9 | COMe |
| 6-377 | 3-Cl-4-F-Ph | Ring 9 | COEt |
| 6-378 | 3-Cl-4-F-Ph | Ring 9 | COPr |
| 6-379 | 4-F-Ph | Ring 10 | $CONH_2$ |
| 6-380 | 4-F-Ph | Ring 10 | CONHMe |
| 6-381 | 4-F-Ph | Ring 10 | CONHEt |
| 6-382 | 4-F-Ph | Ring 10 | CONHPr |
| 6-383 | 4-F-Ph | Ring 10 | CONH-i-Pr |
| 6-384 | 4-F-Ph | Ring 10 | CONHBu |
| 6-385 | 4-F-Ph | Ring 10 | $SO_2NH_2$ |
| 6-386 | 4-F-Ph | Ring 10 | $SO_2NHMe$ |
| 6-387 | 4-F-Ph | Ring 10 | $SO_2NHEt$ |
| 6-388 | 4-F-Ph | Ring 10 | $SO_2NHPr$ |
| 6-389 | 4-F-Ph | Ring 10 | $SO_2NH$-i-Pr |
| 6-390 | 4-F-Ph | Ring 10 | $SO_2NHBu$ |
| 6-391 | 4-F-Ph | Ring 10 | SOMe |
| 6-392 | 4-F-Ph | Ring 10 | SOEt |
| 6-393 | 4-F-Ph | Ring 10 | SOPr |
| 6-394 | 4-F-Ph | Ring 10 | $SO_2Me$ |
| 6-395 | 4-F-Ph | Ring 10 | $SO_2Et$ |
| 6-396 | 4-F-Ph | Ring 10 | $SO_2Pr$ |
| 6-397 | 4-F-Ph | Ring 10 | COMe |
| 6-398 | 4-F-Ph | Ring 10 | COEt |
| 6-399 | 4-F-Ph | Ring 10 | COPr |
| 6-400 | 3-Cl-4-F-Ph | Ring 10 | $CONH_2$ |
| 6-401 | 3-Cl-4-F-Ph | Ring 10 | CONHMe |
| 6-402 | 3-Cl-4-F-Ph | Ring 10 | CONHEt |
| 6-403 | 3-Cl-4-F-Ph | Ring 10 | CONHPr |
| 6-404 | 3-Cl-4-F-Ph | Ring 10 | CONH-i-Pr |
| 6-405 | 3-Cl-4-F-Ph | Ring 10 | CONHBu |
| 6-406 | 3-Cl-4-F-Ph | Ring 10 | $SO_2NH_2$ |
| 6-407 | 3-Cl-4-F-Ph | Ring 10 | $SO_2NHMe$ |
| 6-408 | 3-Cl-4-F-Ph | Ring 10 | $SO_2NHEt$ |
| 6-409 | 3-Cl-4-F-Ph | Ring 10 | $SO_2NHPr$ |
| 6-410 | 3-Cl-4-F-Ph | Ring 10 | $SO_2NH$-i-Pr |

| | | | |
|---|---|---|---|
| 6-411 | 3-Cl-4-F-Ph | Ring 10 | $SO_2NHBu$ |
| 6-412 | 3-Cl-4-F-Ph | Ring 10 | SOMe |
| 6-413 | 3-Cl-4-F-Ph | Ring 10 | SOEt |
| 6-414 | 3-Cl-4-F-Ph | Ring 10 | SOPr |
| 6-415 | 3-Cl-4-F-Ph | Ring 10 | $SO_2Me$ |
| 6-416 | 3-Cl-4-F-Ph | Ring 10 | $SO_2Et$ |
| 6-417 | 3-Cl-4-F-Ph | Ring 10 | $SO_2Pr$ |
| 6-418 | 3-Cl-4-F-Ph | Ring 10 | COMe |
| 6-419 | 3-Cl-4-F-Ph | Ring 10 | COEt |
| 6-420 | 3-Cl-4-F-Ph | Ring 10 | COPr |
| 6-421 | 4-F-Ph | Ring 11 | $CONH_2$ |
| 6-422 | 4-F-Ph | Ring 11 | CONHMe |
| 6-423 | 4-F-Ph | Ring 11 | CONHEt |
| 6-424 | 4-F-Ph | Ring 11 | CONHPr |
| 6-425 | 4-F-Ph | Ring 11 | CONH-i-Pr |
| 6-426 | 4-F-Ph | Ring 11 | CONHBu |
| 6-427 | 4-F-Ph | Ring 11 | $SO_2NH_2$ |
| 6-428 | 4-F-Ph | Ring 11 | $SO_2NHMe$ |
| 6-429 | 4-F-Ph | Ring 11 | $SO_2NHEt$ |
| 6-430 | 4-F-Ph | Ring 11 | $SO_2NHPr$ |
| 6-431 | 4-F-Ph | Ring 11 | $SO_2NH$-i-Pr |
| 6-432 | 4-F-Ph | Ring 11 | $SO_2NHBu$ |
| 6-433 | 4-F-Ph | Ring 11 | SOMe |
| 6-434 | 4-F-Ph | Ring 11 | SOEt |
| 6-435 | 4-F-Ph | Ring 11 | SOPr |
| 6-436 | 4-F-Ph | Ring 11 | $SO_2Me$ |
| 6-437 | 4-F-Ph | Ring 11 | $SO_2Et$ |
| 6-438 | 4-F-Ph | Ring 11 | $SO_2Pr$ |
| 6-439 | 4-F-Ph | Ring 11 | COMe |
| 6-440 | 4-F-Ph | Ring 11 | COEt |
| 6-441 | 4-F-Ph | Ring 11 | COPr |
| 6-442 | 3-Cl-4-F-Ph | Ring 11 | $CONH_2$ |
| 6-443 | 3-Cl-4-F-Ph | Ring 11 | CONHMe |
| 6-444 | 3-Cl-4-F-Ph | Ring 11 | CONHEt |
| 6-445 | 3-Cl-4-F-Ph | Ring 11 | CONHPr |
| 6-446 | 3-Cl-4-F-Ph | Ring 11 | CONH-i-Pr |

| | | | |
|---|---|---|---|
| 6-447 | 3-Cl-4-F-Ph | Ring 11 | CONHBu |
| 6-448 | 3-Cl-4-F-Ph | Ring 11 | $SO_2NH_2$ |
| 6-449 | 3-Cl-4-F-Ph | Ring 11 | $SO_2$NHMe |
| 6-450 | 3-Cl-4-F-Ph | Ring 11 | $SO_2$NHEt |
| 6-451 | 3-Cl-4-F-Ph | Ring 11 | $SO_2$NHPr |
| 6-452 | 3-Cl-4-F-Ph | Ring 11 | $SO_2$NH-i-Pr |
| 6-453 | 3-Cl-4-F-Ph | Ring 11 | $SO_2$NHBu |
| 6-454 | 3-Cl-4-F-Ph | Ring 11 | SOMe |
| 6-455 | 3-Cl-4-F-Ph | Ring 11 | SOEt |
| 6-456 | 3-Cl-4-F-Ph | Ring 11 | SOPr |
| 6-457 | 3-Cl-4-F-Ph | Ring 11 | $SO_2$Me |
| 6-458 | 3-Cl-4-F-Ph | Ring 11 | $SO_2$Et |
| 6-459 | 3-Cl-4-F-Ph | Ring 11 | $SO_2$Pr |
| 6-460 | 3-Cl-4-F-Ph | Ring 11 | COMe |
| 6-461 | 3-Cl-4-F-Ph | Ring 11 | COEt |
| 6-462 | 3-Cl-4-F-Ph | Ring 11 | COPr |
| 6-463 | 4-F-Ph | Ring 12 | $CONH_2$ |
| 6-464 | 4-F-Ph | Ring 12 | CONHMe |
| 6-465 | 4-F-Ph | Ring 12 | CONHEt |
| 6-466 | 4-F-Ph | Ring 12 | CONHPr |
| 6-467 | 4-F-Ph | Ring 12 | CONH-i-Pr |
| 6-468 | 4-F-Ph | Ring 12 | CONHBu |
| 6-469 | 4-F-Ph | Ring 12 | $SO_2NH_2$ |
| 6-470 | 4-F-Ph | Ring 12 | $SO_2$NHMe |
| 6-471 | 4-F-Ph | Ring 12 | $SO_2$NHEt |
| 6-472 | 4-F-Ph | Ring 12 | $SO_2$NHPr |
| 6-473 | 4-F-Ph | Ring 12 | $SO_2$NH-i-Pr |
| 6-474 | 4-F-Ph | Ring 12 | $SO_2$NHBu |
| 6-475 | 4-F-Ph | Ring 12 | SOMe |
| 6-476 | 4-F-Ph | Ring 12 | SOEt |
| 6-477 | 4-F-Ph | Ring 12 | SOPr |
| 6-478 | 4-F-Ph | Ring 12 | $SO_2$Me |
| 6-479 | 4-F-Ph | Ring 12 | $SO_2$Et |
| 6-480 | 4-F-Ph | Ring 12 | $SO_2$Pr |
| 6-481 | 4-F-Ph | Ring 12 | COMe |
| 6-482 | 4-F-Ph | Ring 12 | COEt |

| | | | |
|---|---|---|---|
| 6-483 | 4-F-Ph | Ring 12 | COPr |
| 6-484 | 3-Cl-4-F-Ph | Ring 12 | $CONH_2$ |
| 6-485 | 3-Cl-4-F-Ph | Ring 12 | CONHMe |
| 6-486 | 3-Cl-4-F-Ph | Ring 12 | CONHEt |
| 6-487 | 3-Cl-4-F-Ph | Ring 12 | CONHPr |
| 6-488 | 3-Cl-4-F-Ph | Ring 12 | CONH-i-Pr |
| 6-489 | 3-Cl-4-F-Ph | Ring 12 | CONHBu |
| 6-490 | 3-Cl-4-F-Ph | Ring 12 | $SO_2NH_2$ |
| 6-491 | 3-Cl-4-F-Ph | Ring 12 | $SO_2NHMe$ |
| 6-492 | 3-Cl-4-F-Ph | Ring 12 | $SO_2NHEt$ |
| 6-493 | 3-Cl-4-F-Ph | Ring 12 | $SO_2NHPr$ |
| 6-494 | 3-Cl-4-F-Ph | Ring 12 | $SO_2NH$-i-Pr |
| 6-495 | 3-Cl-4-F-Ph | Ring 12 | $SO_2NHBu$ |
| 6-496 | 3-Cl-4-F-Ph | Ring 12 | SOMe |
| 6-497 | 3-Cl-4-F-Ph | Ring 12 | SOEt |
| 6-498 | 3-Cl-4-F-Ph | Ring 12 | SOPr |
| 6-499 | 3-Cl-4-F-Ph | Ring 12 | $SO_2Me$ |
| 6-500 | 3-Cl-4-F-Ph | Ring 12 | $SO_2Et$ |
| 6-501 | 3-Cl-4-F-Ph | Ring 12 | $SO_2Pr$ |
| 6-502 | 3-Cl-4-F-Ph | Ring 12 | COMe |
| 6-503 | 3-Cl-4-F-Ph | Ring 12 | COEt |
| 6-504 | 3-Cl-4-F-Ph | Ring 12 | COPr |
| 6-505 | 4-F-Ph | Ring 13 | $CONH_2$ |
| 6-506 | 4-F-Ph | Ring 13 | CONHMe |
| 6-507 | 4-F-Ph | Ring 13 | CONHEt |
| 6-508 | 4-F-Ph | Ring 13 | CONHPr |
| 6-509 | 4-F-Ph | Ring 13 | CONH-i-Pr |
| 6-510 | 4-F-Ph | Ring 13 | CONHBu |
| 6-511 | 4-F-Ph | Ring 13 | $SO_2NH_2$ |
| 6-512 | 4-F-Ph | Ring 13 | $SO_2NHMe$ |
| 6-513 | 4-F-Ph | Ring 13 | $SO_2NHEt$ |
| 6-514 | 4-F-Ph | Ring 13 | $SO_2NHPr$ |
| 6-515 | 4-F-Ph | Ring 13 | $SO_2NH$-i-Pr |
| 6-516 | 4-F-Ph | Ring 13 | $SO_2NHBu$ |
| 6-517 | 4-F-Ph | Ring 13 | SOMe |
| 6-518 | 4-F-Ph | Ring 13 | SOEt |

| | | | |
|---|---|---|---|
| 6-519 | 4-F-Ph | Ring 13 | SOPr |
| 6-520 | 4-F-Ph | Ring 13 | $SO_2Me$ |
| 6-521 | 4-F-Ph | Ring 13 | $SO_2Et$ |
| 6-522 | 4-F-Ph | Ring 13 | $SO_2Pr$ |
| 6-523 | 4-F-Ph | Ring 13 | COMe |
| 6-524 | 4-F-Ph | Ring 13 | COEt |
| 6-525 | 4-F-Ph | Ring 13 | COPr |
| 6-526 | 3-Cl-4-F-Ph | Ring 13 | $CONH_2$ |
| 6-527 | 3-Cl-4-F-Ph | Ring 13 | CONHMe |
| 6-528 | 3-Cl-4-F-Ph | Ring 13 | CONHEt |
| 6-529 | 3-Cl-4-F-Ph | Ring 13 | CONHPr |
| 6-530 | 3-Cl-4-F-Ph | Ring 13 | CONH-i-Pr |
| 6-531 | 3-Cl-4-F-Ph | Ring 13 | CONHBu |
| 6-532 | 3-Cl-4-F-Ph | Ring 13 | $SO_2NH_2$ |
| 6-533 | 3-Cl-4-F-Ph | Ring 13 | $SO_2NHMe$ |
| 6-534 | 3-Cl-4-F-Ph | Ring 13 | $SO_2NHEt$ |
| 6-535 | 3-Cl-4-F-Ph | Ring 13 | $SO_2NHPr$ |
| 6-536 | 3-Cl-4-F-Ph | Ring 13 | $SO_2NH$-i-Pr |
| 6-537 | 3-Cl-4-F-Ph | Ring 13 | $SO_2NHBu$ |
| 6-538 | 3-Cl-4-F-Ph | Ring 13 | SOMe |
| 6-539 | 3-Cl-4-F-Ph | Ring 13 | SOEt |
| 6-540 | 3-Cl-4-F-Ph | Ring 13 | SOPr |
| 6-541 | 3-Cl-4-F-Ph | Ring 13 | $SO_2Me$ |
| 6-542 | 3-Cl-4-F-Ph | Ring 13 | $SO_2Et$ |
| 6-543 | 3-Cl-4-F-Ph | Ring 13 | $SO_2Pr$ |
| 6-544 | 3-Cl-4-F-Ph | Ring 13 | COMe |
| 6-545 | 3-Cl-4-F-Ph | Ring 13 | COEt |
| 6-546 | 3-Cl-4-F-Ph | Ring 13 | COPr |
| 6-547 | 4-F-Ph | Ring 14 | $CONH_2$ |
| 6-548 | 4-F-Ph | Ring 14 | CONHMe |
| 6-549 | 4-F-Ph | Ring 14 | CONHEt |
| 6-550 | 4-F-Ph | Ring 14 | CONHPr |
| 6-551 | 4-F-Ph | Ring 14 | CONH-i-Pr |
| 6-552 | 4-F-Ph | Ring 14 | CONHBu |
| 6-553 | 4-F-Ph | Ring 14 | $SO_2NH_2$ |
| 6-554 | 4-F-Ph | Ring 14 | $SO_2NHMe$ |

| | | | |
|---|---|---|---|
| 6-555 | 4-F-Ph | Ring 14 | $SO_2NHEt$ |
| 6-556 | 4-F-Ph | Ring 14 | $SO_2NHPr$ |
| 6-557 | 4-F-Ph | Ring 14 | $SO_2NH$-i-Pr |
| 6-558 | 4-F-Ph | Ring 14 | $SO_2NHBu$ |
| 6-559 | 4-F-Ph | Ring 14 | SOMe |
| 6-560 | 4-F-Ph | Ring 14 | SOEt |
| 6-561 | 4-F-Ph | Ring 14 | SOPr |
| 6-562 | 4-F-Ph | Ring 14 | $SO_2Me$ |
| 6-563 | 4-F-Ph | Ring 14 | $SO_2Et$ |
| 6-564 | 4-F-Ph | Ring 14 | $SO_2Pr$ |
| 6-565 | 4-F-Ph | Ring 14 | COMe |
| 6-566 | 4-F-Ph | Ring 14 | COEt |
| 6-567 | 4-F-Ph | Ring 14 | COPr |
| 6-568 | 3-Cl-4-F-Ph | Ring 14 | $CONH_2$ |
| 6-569 | 3-Cl-4-F-Ph | Ring 14 | CONHMe |
| 6-570 | 3-Cl-4-F-Ph | Ring 14 | CONHEt |
| 6-571 | 3-Cl-4-F-Ph | Ring 14 | CONHPr |
| 6-572 | 3-Cl-4-F-Ph | Ring 14 | CONH-i-Pr |
| 6-573 | 3-Cl-4-F-Ph | Ring 14 | CONHBu |
| 6-574 | 3-Cl-4-F-Ph | Ring 14 | $SO_2NH_2$ |
| 6-575 | 3-Cl-4-F-Ph | Ring 14 | $SO_2NHMe$ |
| 6-576 | 3-Cl-4-F-Ph | Ring 14 | $SO_2NHEt$ |
| 6-577 | 3-Cl-4-F-Ph | Ring 14 | $SO_2NHPr$ |
| 6-578 | 3-Cl-4-F-Ph | Ring 14 | $SO_2NH$-i-Pr |
| 6-579 | 3-Cl-4-F-Ph | Ring 14 | $SO_2NHBu$ |
| 6-580 | 3-Cl-4-F-Ph | Ring 14 | SOMe |
| 6-581 | 3-Cl-4-F-Ph | Ring 14 | SOEt |
| 6-582 | 3-Cl-4-F-Ph | Ring 14 | SOPr |
| 6-583 | 3-Cl-4-F-Ph | Ring 14 | $SO_2Me$ |
| 6-584 | 3-Cl-4-F-Ph | Ring 14 | $SO_2Et$ |
| 6-585 | 3-Cl-4-F-Ph | Ring 14 | $SO_2Pr$ |
| 6-586 | 3-Cl-4-F-Ph | Ring 14 | COMe |
| 6-587 | 3-Cl-4-F-Ph | Ring 14 | COEt |
| 6-588 | 3-Cl-4-F-Ph | Ring 14 | COPr |

Table 7

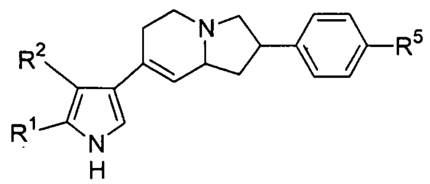

(I-7)

| Compound No. | $R^1$ | $R^2$ | $R^5$ |
|---|---|---|---|
| 7-1 | Ph | 4-Pyr | COOH |
| 7-2 | Ph | 4-Pyr | COOMe |
| 7-3 | Ph | 4-Pyr | COOEt |
| 7-4 | Ph | 4-Pyr | COOPr |
| 7-5 | Ph | 4-Pyr | COO-i-Pr |
| 7-6 | Ph | 4-Pyr | COOBu |
| 7-7 | Ph | 4-Pyr | COOBn |
| 7-8 | Ph | 4-Pyr | COOPh |
| 7-9 | Ph | 4-Pyr | $CONH_2$ |
| 7-10 | Ph | 4-Pyr | CONHMe |
| 7-11 | Ph | 4-Pyr | CONHEt |
| 7-12 | Ph | 4-Pyr | CONHPr |
| 7-13 | Ph | 4-Pyr | CONH-i-Pr |
| 7-14 | Ph | 4-Pyr | CONHBu |
| 7-15 | Ph | 4-Pyr | CONHBn |
| 7-16 | Ph | 4-Pyr | $CONMe_2$ |
| 7-17 | Ph | 4-Pyr | CONH-c-Pr |
| 7-18 | Ph | 4-Pyr | CONH-c-Bu |
| 7-19 | Ph | 4-Pyr | CONH-c-Pen |
| 7-20 | Ph | 4-Pyr | CONH-c-Hex |
| 7-21 | Ph | 4-Pyr | CONH-c-Hep |
| 7-22 | Ph | 4-Pyr | $CONHCH_2$-c-Pr |
| 7-23 | Ph | 4-Pyr | CONH-Allyl |
| 7-24 | Ph | 4-Pyr | CONH-Propargyl |
| 7-25 | Ph | 4-Pyr | CONHPh |
| 7-26 | Ph | 4-Pyr | CONH-3-Pyr |
| 7-27 | Ph | 4-Pyr | $CONHCH_2$-4-Pyr |
| 7-28 | Ph | 4-Pyr | CONH-$(CH_2)_2$-OH |

| | | | |
|---|---|---|---|
| 7-29 | Ph | 4-Pyr | CONHCH$(CH_2OH)_2$ |
| 7-30 | Ph | 4-Pyr | CONH-$(CH_2)_2$-OAc |
| 7-31 | Ph | 4-Pyr | $CONHCH_2CN$ |
| 7-32 | Ph | 4-Pyr | CONH-$(CH_2)_2$-F |
| 7-33 | Ph | 4-Pyr | CONH-$(CH_2)_2$-OMe |
| 7-34 | Ph | 4-Pyr | CONH-$(CH_2)_2$-SMe |
| 7-35 | Ph | 4-Pyr | CONH-$(CH_2)_2$-$NH_2$ |
| 7-36 | Ph | 4-Pyr | CONH-$(CH_2)_2$-NHMe |
| 7-37 | Ph | 4-Pyr | CONH-$(CH_2)_2$-N$(Me)_2$ |
| 7-38 | Ph | 4-Pyr | $CONHCH_2COOH$ |
| 7-39 | Ph | 4-Pyr | $CONHCH_2COOEt$ |
| 7-40 | Ph | 4-Pyr | CONHCH(Me)COOEt |
| 7-41 | Ph | 4-Pyr | $CONHCH_2CONH_2$ |
| 7-42 | Ph | 4-Pyr | CONHOH |
| 7-43 | Ph | 4-Pyr | CONHOMe |
| 7-44 | Ph | 4-Pyr | CONHOEt |
| 7-45 | Ph | 4-Pyr | CONHOPr |
| 7-46 | Ph | 4-Pyr | CONHO-Allyl |
| 7-47 | Ph | 4-Pyr | CONHOBn |
| 7-48 | Ph | 4-Pyr | $CONHNH_2$ |
| 7-49 | Ph | 4-Pyr | CONHNHMe |
| 7-50 | Ph | 4-Pyr | CONHN$(Me)_2$ |
| 7-51 | Ph | 4-Pyr | COMe |
| 7-52 | Ph | 4-Pyr | COEt |
| 7-53 | Ph | 4-Pyr | COPr |
| 7-54 | Ph | 4-Pyr | CO-i-Pr |
| 7-55 | Ph | 4-Pyr | COBu |
| 7-56 | Ph | 4-Pyr | $COCF_3$ |
| 7-57 | Ph | 4-Pyr | CO-$(CH_2)_2$-F |
| 7-58 | Ph | 4-Pyr | CO-$(CH_2)_2$-OH |
| 7-59 | Ph | 4-Pyr | CO-$(CH_2)_2$-OMe |
| 7-60 | Ph | 4-Pyr | SOMe |
| 7-61 | Ph | 4-Pyr | SOEt |
| 7-62 | Ph | 4-Pyr | SOPr |
| 7-63 | Ph | 4-Pyr | SO-i-Pr |
| 7-64 | Ph | 4-Pyr | SOBu |

| | | | |
|---|---|---|---|
| 7-65 | Ph | 4-Pyr | $SOCF_3$ |
| 7-66 | Ph | 4-Pyr | $SO-(CH_2)_2-F$ |
| 7-67 | Ph | 4-Pyr | $SO-(CH_2)_2-OH$ |
| 7-68 | Ph | 4-Pyr | $SO-(CH_2)_2-OMe$ |
| 7-69 | Ph | 4-Pyr | $SO_2Me$ |
| 7-70 | Ph | 4-Pyr | $SO_2Et$ |
| 7-71 | Ph | 4-Pyr | $SO_2Pr$ |
| 7-72 | Ph | 4-Pyr | $SO_2$-i-Pr |
| 7-73 | Ph | 4-Pyr | $SO_2Bu$ |
| 7-74 | Ph | 4-Pyr | $SO_2CF_3$ |
| 7-75 | Ph | 4-Pyr | $SO_2-(CH_2)_2-F$ |
| 7-76 | Ph | 4-Pyr | $SO_2-(CH_2)_2-OH$ |
| 7-77 | Ph | 4-Pyr | $SO_2-(CH_2)_2-OMe$ |
| 7-78 | Ph | 4-Pyr | $SO_2NH_2$ |
| 7-79 | Ph | 4-Pyr | $SO_2NHMe$ |
| 7-80 | Ph | 4-Pyr | $SO_2NHEt$ |
| 7-81 | Ph | 4-Pyr | $SO_2NHPr$ |
| 7-82 | Ph | 4-Pyr | $SO_2NH$-i-Pr |
| 7-83 | Ph | 4-Pyr | $SO_2NHBu$ |
| 7-84 | Ph | 4-Pyr | $SO_2NHBn$ |
| 7-85 | Ph | 4-Pyr | $SO_2NMe_2$ |
| 7-86 | Ph | 4-Pyr | $SO_2NH$-c-Pr |
| 7-87 | Ph | 4-Pyr | $SO_2NH$-c-Bu |
| 7-88 | Ph | 4-Pyr | $SO_2NH$-c-Pen |
| 7-89 | Ph | 4-Pyr | $SO_2NH$-c-Hex |
| 7-90 | Ph | 4-Pyr | $SO_2NH$-c-Hep |
| 7-91 | Ph | 4-Pyr | $SO_2NHCH_2$-c-Pr |
| 7-92 | Ph | 4-Pyr | $SO_2NH$-Allyl |
| 7-93 | Ph | 4-Pyr | $SO_2NH$-Propargyl |
| 7-94 | Ph | 4-Pyr | $SO_2NHPh$ |
| 7-95 | Ph | 4-Pyr | $SO_2NH$-3-Pyr |
| 7-96 | Ph | 4-Pyr | $SO_2NHCH_2$-4-Pyr |
| 7-97 | Ph | 4-Pyr | $SO_2NH-(CH_2)_2-OH$ |
| 7-98 | Ph | 4-Pyr | $SO_2NHCH(CH_2OH)_2$ |
| 7-99 | Ph | 4-Pyr | $SO_2NH-(CH_2)_2-OAc$ |
| 7-100 | Ph | 4-Pyr | $SO_2NHCH_2CN$ |

| | | | |
|---|---|---|---|
| 7-101 | Ph | 4-Pyr | $SO_2NH-(CH_2)_2-F$ |
| 7-102 | Ph | 4-Pyr | $SO_2NH-(CH_2)_2-OMe$ |
| 7-103 | Ph | 4-Pyr | $SO_2NH-(CH_2)_2-SMe$ |
| 7-104 | Ph | 4-Pyr | $SO_2NH-(CH_2)_2-NH_2$ |
| 7-105 | Ph | 4-Pyr | $SO_2NH-(CH_2)_2-NHMe$ |
| 7-106 | Ph | 4-Pyr | $SO_2NH-(CH_2)_2-N(Me)_2$ |
| 7-107 | Ph | 4-Pyr | $SO_2NHCH_2COOH$ |
| 7-108 | Ph | 4-Pyr | $SO_2NHCH_2COOEt$ |
| 7-109 | Ph | 4-Pyr | $SO_2NHCH(Me)COOEt$ |
| 7-110 | Ph | 4-Pyr | $SO_2NHCH_2CONH_2$ |
| 7-111 | Ph | 4-Pyr | $SO_2NHOH$ |
| 7-112 | Ph | 4-Pyr | $SO_2NHOMe$ |
| 7-113 | Ph | 4-Pyr | $SO_2NHOEt$ |
| 7-114 | Ph | 4-Pyr | $SO_2NHOPr$ |
| 7-115 | Ph | 4-Pyr | $SO_2NHOAllyl$ |
| 7-116 | Ph | 4-Pyr | $SO_2NHOBn$ |
| 7-117 | Ph | 4-Pyr | $SO_2NHNH_2$ |
| 7-118 | Ph | 4-Pyr | $SO_2NHNHMe$ |
| 7-119 | Ph | 4-Pyr | $SO_2NHN(Me)_2$ |
| 7-120 | 3-F-Ph | 4-Pyr | COOH |
| 7-121 | 3-F-Ph | 4-Pyr | COOMe |
| 7-122 | 3-F-Ph | 4-Pyr | COOEt |
| 7-123 | 3-F-Ph | 4-Pyr | COOPr |
| 7-124 | 3-F-Ph | 4-Pyr | COO-i-Pr |
| 7-125 | 3-F-Ph | 4-Pyr | COOBu |
| 7-126 | 3-F-Ph | 4-Pyr | COOBn |
| 7-127 | 3-F-Ph | 4-Pyr | COOPh |
| 7-128 | 3-F-Ph | 4-Pyr | $CONH_2$ |
| 7-129 | 3-F-Ph | 4-Pyr | CONHMe |
| 7-130 | 3-F-Ph | 4-Pyr | CONHEt |
| 7-131 | 3-F-Ph | 4-Pyr | CONHPr |
| 7-132 | 3-F-Ph | 4-Pyr | CONH-i-Pr |
| 7-133 | 3-F-Ph | 4-Pyr | CONHBu |
| 7-134 | 3-F-Ph | 4-Pyr | CONHBn |
| 7-135 | 3-F-Ph | 4-Pyr | $CONMe_2$ |
| 7-136 | 3-F-Ph | 4-Pyr | CONH-c-Pr |

| | | | |
|---|---|---|---|
| 7-137 | 3-F-Ph | 4-Pyr | CONH-c-Bu |
| 7-138 | 3-F-Ph | 4-Pyr | CONH-c-Pen |
| 7-139 | 3-F-Ph | 4-Pyr | CONH-c-Hex |
| 7-140 | 3-F-Ph | 4-Pyr | CONH-c-Hep |
| 7-141 | 3-F-Ph | 4-Pyr | $CONHCH_2$-c-Pr |
| 7-142 | 3-F-Ph | 4-Pyr | CONH-Allyl |
| 7-143 | 3-F-Ph | 4-Pyr | CONH-Propargyl |
| 7-144 | 3-F-Ph | 4-Pyr | CONHPh |
| 7-145 | 3-F-Ph | 4-Pyr | CONH-3-Pyr |
| 7-146 | 3-F-Ph | 4-Pyr | $CONHCH_2$-4-Pyr |
| 7-147 | 3-F-Ph | 4-Pyr | CONH-$(CH_2)_2$-OH |
| 7-148 | 3-F-Ph | 4-Pyr | $CONHCH(CH_2OH)_2$ |
| 7-149 | 3-F-Ph | 4-Pyr | CONH-$(CH_2)_2$-OAc |
| 7-150 | 3-F-Ph | 4-Pyr | $CONHCH_2CN$ |
| 7-151 | 3-F-Ph | 4-Pyr | CONH-$(CH_2)_2$-F |
| 7-152 | 3-F-Ph | 4-Pyr | CONH-$(CH_2)_2$-OMe |
| 7-153 | 3-F-Ph | 4-Pyr | CONH-$(CH_2)_2$-SMe |
| 7-154 | 3-F-Ph | 4-Pyr | CONH-$(CH_2)_2$-$NH_2$ |
| 7-155 | 3-F-Ph | 4-Pyr | CONH-$(CH_2)_2$-NHMe |
| 7-156 | 3-F-Ph | 4-Pyr | CONH-$(CH_2)_2$-$N(Me)_2$ |
| 7-157 | 3-F-Ph | 4-Pyr | $CONHCH_2COOH$ |
| 7-158 | 3-F-Ph | 4-Pyr | $CONHCH_2COOEt$ |
| 7-159 | 3-F-Ph | 4-Pyr | CONHCH(Me)COOEt |
| 7-160 | 3-F-Ph | 4-Pyr | $CONHCH_2CONH_2$ |
| 7-161 | 3-F-Ph | 4-Pyr | CONHOH |
| 7-162 | 3-F-Ph | 4-Pyr | CONHOMe |
| 7-163 | 3-F-Ph | 4-Pyr | CONHOEt |
| 7-164 | 3-F-Ph | 4-Pyr | CONHOPr |
| 7-165 | 3-F-Ph | 4-Pyr | CONHOAllyl |
| 7-166 | 3-F-Ph | 4-Pyr | CONHOBn |
| 7-167 | 3-F-Ph | 4-Pyr | $CONHNH_2$ |
| 7-168 | 3-F-Ph | 4-Pyr | CONHNHMe |
| 7-169 | 3-F-Ph | 4-Pyr | $CONHN(Me)_2$ |
| 7-170 | 3-F-Ph | 4-Pyr | COMe |
| 7-171 | 3-F-Ph | 4-Pyr | COEt |
| 7-172 | 3-F-Ph | 4-Pyr | COPr |

| | | | |
|---|---|---|---|
| 7-173 | 3-F-Ph | 4-Pyr | CO-i-Pr |
| 7-174 | 3-F-Ph | 4-Pyr | COBu |
| 7-175 | 3-F-Ph | 4-Pyr | $COCF_3$ |
| 7-176 | 3-F-Ph | 4-Pyr | CO-$(CH_2)_2$-F |
| 7-177 | 3-F-Ph | 4-Pyr | CO-$(CH_2)_2$-OH |
| 7-178 | 3-F-Ph | 4-Pyr | CO-$(CH_2)_2$-OMe |
| 7-179 | 3-F-Ph | 4-Pyr | SOMe |
| 7-180 | 3-F-Ph | 4-Pyr | SOEt |
| 7-181 | 3-F-Ph | 4-Pyr | SOPr |
| 7-182 | 3-F-Ph | 4-Pyr | SO-i-Pr |
| 7-183 | 3-F-Ph | 4-Pyr | SOBu |
| 7-184 | 3-F-Ph | 4-Pyr | $SOCF_3$ |
| 7-185 | 3-F-Ph | 4-Pyr | SO-$(CH_2)_2$-F |
| 7-186 | 3-F-Ph | 4-Pyr | SO-$(CH_2)_2$-OH |
| 7-187 | 3-F-Ph | 4-Pyr | SO-$(CH_2)_2$-OMe |
| 7-188 | 3-F-Ph | 4-Pyr | $SO_2Me$ |
| 7-189 | 3-F-Ph | 4-Pyr | $SO_2Et$ |
| 7-190 | 3-F-Ph | 4-Pyr | $SO_2Pr$ |
| 7-191 | 3-F-Ph | 4-Pyr | $SO_2$-i-Pr |
| 7-192 | 3-F-Ph | 4-Pyr | $SO_2Bu$ |
| 7-193 | 3-F-Ph | 4-Pyr | $SO_2CF_3$ |
| 7-194 | 3-F-Ph | 4-Pyr | $SO_2$-$(CH_2)_2$-F |
| 7-195 | 3-F-Ph | 4-Pyr | $SO_2$-$(CH_2)_2$-OH |
| 7-196 | 3-F-Ph | 4-Pyr | $SO_2$-$(CH_2)_2$-OMe |
| 7-197 | 3-F-Ph | 4-Pyr | $SO_2NH_2$ |
| 7-198 | 3-F-Ph | 4-Pyr | $SO_2NHMe$ |
| 7-199 | 3-F-Ph | 4-Pyr | $SO_2NHEt$ |
| 7-200 | 3-F-Ph | 4-Pyr | $SO_2NHPr$ |
| 7-201 | 3-F-Ph | 4-Pyr | $SO_2NH$-i-Pr |
| 7-202 | 3-F-Ph | 4-Pyr | $SO_2NHBu$ |
| 7-203 | 3-F-Ph | 4-Pyr | $SO_2NHBn$ |
| 7-204 | 3-F-Ph | 4-Pyr | $SO_2NMe_2$ |
| 7-205 | 3-F-Ph | 4-Pyr | $SO_2NH$-c-Pr |
| 7-206 | 3-F-Ph | 4-Pyr | $SO_2NH$-c-Bu |
| 7-207 | 3-F-Ph | 4-Pyr | $SO_2NH$-c-Pen |
| 7-208 | 3-F-Ph | 4-Pyr | $SO_2NH$-c-Hex |

| | | | |
|---|---|---|---|
| 7-209 | 3-F-Ph | 4-Pyr | $SO_2NH$-c-Hep |
| 7-210 | 3-F-Ph | 4-Pyr | $SO_2NHCH_2$-c-Pr |
| 7-211 | 3-F-Ph | 4-Pyr | $SO_2NH$-Allyl |
| 7-212 | 3-F-Ph | 4-Pyr | $SO_2NH$-Propargyl |
| 7-213 | 3-F-Ph | 4-Pyr | $SO_2NHPh$ |
| 7-214 | 3-F-Ph | 4-Pyr | $SO_2NH$-3-Pyr |
| 7-215 | 3-F-Ph | 4-Pyr | $SO_2NHCH_2$-4-Pyr |
| 7-216 | 3-F-Ph | 4-Pyr | $SO_2NH-(CH_2)_2-OH$ |
| 7-217 | 3-F-Ph | 4-Pyr | $SO_2NHCH(CH_2OH)_2$ |
| 7-218 | 3-F-Ph | 4-Pyr | $SO_2NH-(CH_2)_2-OAc$ |
| 7-219 | 3-F-Ph | 4-Pyr | $SO_2NHCH_2CN$ |
| 7-220 | 3-F-Ph | 4-Pyr | $SO_2NH-(CH_2)_2-F$ |
| 7-221 | 3-F-Ph | 4-Pyr | $SO_2NH-(CH_2)_2-OMe$ |
| 7-222 | 3-F-Ph | 4-Pyr | $SO_2NH-(CH_2)_2-SMe$ |
| 7-223 | 3-F-Ph | 4-Pyr | $SO_2NH-(CH_2)_2-NH_2$ |
| 7-224 | 3-F-Ph | 4-Pyr | $SO_2NH-(CH_2)_2-NHMe$ |
| 7-225 | 3-F-Ph | 4-Pyr | $SO_2NH-(CH_2)_2-N(Me)_2$ |
| 7-226 | 3-F-Ph | 4-Pyr | $SO_2NHCH_2COOH$ |
| 7-227 | 3-F-Ph | 4-Pyr | $SO_2NHCH_2COOEt$ |
| 7-228 | 3-F-Ph | 4-Pyr | $SO_2NHCH(Me)COOEt$ |
| 7-229 | 3-F-Ph | 4-Pyr | $SO_2NHCH_2CONH_2$ |
| 7-230 | 3-F-Ph | 4-Pyr | $SO_2NHOH$ |
| 7-231 | 3-F-Ph | 4-Pyr | $SO_2NHOMe$ |
| 7-232 | 3-F-Ph | 4-Pyr | $SO_2NHOEt$ |
| 7-233 | 3-F-Ph | 4-Pyr | $SO_2NHOPr$ |
| 7-234 | 3-F-Ph | 4-Pyr | $SO_2NHOAllyl$ |
| 7-235 | 3-F-Ph | 4-Pyr | $SO_2NHOBn$ |
| 7-236 | 3-F-Ph | 4-Pyr | $SO_2NHNH_2$ |
| 7-237 | 3-F-Ph | 4-Pyr | $SO_2NHNHMe$ |
| 7-238 | 3-F-Ph | 4-Pyr | $SO_2NHN(Me)_2$ |
| 7-239 | 3-Cl-Ph | 4-Pyr | COOH |
| 7-240 | 3-Cl-Ph | 4-Pyr | COOMe |
| 7-241 | 3-Cl-Ph | 4-Pyr | COOEt |
| 7-242 | 3-Cl-Ph | 4-Pyr | COOPr |
| 7-243 | 3-Cl-Ph | 4-Pyr | COO-i-Pr |
| 7-244 | 3-Cl-Ph | 4-Pyr | COOBu |

| | | | |
|---|---|---|---|
| 7-245 | 3-Cl-Ph | 4-Pyr | COOBn |
| 7-246 | 3-Cl-Ph | 4-Pyr | COOPh |
| 7-247 | 3-Cl-Ph | 4-Pyr | $CONH_2$ |
| 7-248 | 3-Cl-Ph | 4-Pyr | CONHMe |
| 7-249 | 3-Cl-Ph | 4-Pyr | CONHEt |
| 7-250 | 3-Cl-Ph | 4-Pyr | CONHPr |
| 7-251 | 3-Cl-Ph | 4-Pyr | CONH-i-Pr |
| 7-252 | 3-Cl-Ph | 4-Pyr | CONHBu |
| 7-253 | 3-Cl-Ph | 4-Pyr | CONHBn |
| 7-254 | 3-Cl-Ph | 4-Pyr | $CONMe_2$ |
| 7-255 | 3-Cl-Ph | 4-Pyr | CONH-c-Pr |
| 7-256 | 3-Cl-Ph | 4-Pyr | CONH-c-Bu |
| 7-257 | 3-Cl-Ph | 4-Pyr | CONH-c-Pen |
| 7-258 | 3-Cl-Ph | 4-Pyr | CONH-c-Hex |
| 7-259 | 3-Cl-Ph | 4-Pyr | CONH-c-Hep |
| 7-260 | 3-Cl-Ph | 4-Pyr | $CONHCH_2$-c-Pr |
| 7-261 | 3-Cl-Ph | 4-Pyr | CONH-Allyl |
| 7-262 | 3-Cl-Ph | 4-Pyr | CONH-Propargyl |
| 7-263 | 3-Cl-Ph | 4-Pyr | CONHPh |
| 7-264 | 3-Cl-Ph | 4-Pyr | CONH-3-Pyr |
| 7-265 | 3-Cl-Ph | 4-Pyr | $CONHCH_2$-4-Pyr |
| 7-266 | 3-Cl-Ph | 4-Pyr | CONH-$(CH_2)_2$-OH |
| 7-267 | 3-Cl-Ph | 4-Pyr | $CONHCH(CH_2OH)_2$ |
| 7-268 | 3-Cl-Ph | 4-Pyr | CONH-$(CH_2)_2$-OAc |
| 7-269 | 3-Cl-Ph | 4-Pyr | $CONHCH_2CN$ |
| 7-270 | 3-Cl-Ph | 4-Pyr | CONH-$(CH_2)_2$-F |
| 7-271 | 3-Cl-Ph | 4-Pyr | CONH-$(CH_2)_2$-OMe |
| 7-272 | 3-Cl-Ph | 4-Pyr | CONH-$(CH_2)_2$-SMe |
| 7-273 | 3-Cl-Ph | 4-Pyr | CONH-$(CH_2)_2$-$NH_2$ |
| 7-274 | 3-Cl-Ph | 4-Pyr | CONH-$(CH_2)_2$-NHMe |
| 7-275 | 3-Cl-Ph | 4-Pyr | CONH-$(CH_2)_2$-$N(Me)_2$ |
| 7-276 | 3-Cl-Ph | 4-Pyr | $CONHCH_2COOH$ |
| 7-277 | 3-Cl-Ph | 4-Pyr | $CONHCH_2COOEt$ |
| 7-278 | 3-Cl-Ph | 4-Pyr | CONHCH(Me)COOEt |
| 7-279 | 3-Cl-Ph | 4-Pyr | $CONHCH_2CONH_2$ |
| 7-280 | 3-Cl-Ph | 4-Pyr | CONHOH |

| | | | |
|---|---|---|---|
| 7-281 | 3-Cl-Ph | 4-Pyr | CONHOMe |
| 7-282 | 3-Cl-Ph | 4-Pyr | CONHOEt |
| 7-283 | 3-Cl-Ph | 4-Pyr | CONHOPr |
| 7-284 | 3-Cl-Ph | 4-Pyr | CONHOAllyl |
| 7-285 | 3-Cl-Ph | 4-Pyr | CONHOBn |
| 7-286 | 3-Cl-Ph | 4-Pyr | $CONHNH_2$ |
| 7-287 | 3-Cl-Ph | 4-Pyr | CONHNHMe |
| 7-288 | 3-Cl-Ph | 4-Pyr | $CONHN(Me)_2$ |
| 7-289 | 3-Cl-Ph | 4-Pyr | COMe |
| 7-290 | 3-Cl-Ph | 4-Pyr | COEt |
| 7-291 | 3-Cl-Ph | 4-Pyr | COPr |
| 7-292 | 3-Cl-Ph | 4-Pyr | CO-i-Pr |
| 7-293 | 3-Cl-Ph | 4-Pyr | COBu |
| 7-294 | 3-Cl-Ph | 4-Pyr | $COCF_3$ |
| 7-295 | 3-Cl-Ph | 4-Pyr | $CO-(CH_2)_2-F$ |
| 7-296 | 3-Cl-Ph | 4-Pyr | $CO-(CH_2)_2-OH$ |
| 7-297 | 3-Cl-Ph | 4-Pyr | $CO-(CH_2)_2-OMe$ |
| 7-298 | 3-Cl-Ph | 4-Pyr | SOMe |
| 7-299 | 3-Cl-Ph | 4-Pyr | SOEt |
| 7-300 | 3-Cl-Ph | 4-Pyr | SOPr |
| 7-301 | 3-Cl-Ph | 4-Pyr | SO-i-Pr |
| 7-302 | 3-Cl-Ph | 4-Pyr | SOBu |
| 7-303 | 3-Cl-Ph | 4-Pyr | $SOCF_3$ |
| 7-304 | 3-Cl-Ph | 4-Pyr | $SO-(CH_2)_2-F$ |
| 7-305 | 3-Cl-Ph | 4-Pyr | $SO-(CH_2)_2-OH$ |
| 7-306 | 3-Cl-Ph | 4-Pyr | $SO-(CH_2)_2-OMe$ |
| 7-307 | 3-Cl-Ph | 4-Pyr | $SO_2Me$ |
| 7-308 | 3-Cl-Ph | 4-Pyr | $SO_2Et$ |
| 7-309 | 3-Cl-Ph | 4-Pyr | $SO_2Pr$ |
| 7-310 | 3-Cl-Ph | 4-Pyr | $SO_2$-i-Pr |
| 7-311 | 3-Cl-Ph | 4-Pyr | $SO_2Bu$ |
| 7-312 | 3-Cl-Ph | 4-Pyr | $SO_2CF_3$ |
| 7-313 | 3-Cl-Ph | 4-Pyr | $SO_2-(CH_2)_2-F$ |
| 7-314 | 3-Cl-Ph | 4-Pyr | $SO_2-(CH_2)_2-OH$ |
| 7-315 | 3-Cl-Ph | 4-Pyr | $SO_2-(CH_2)_2-OMe$ |
| 7-316 | 3-Cl-Ph | 4-Pyr | $SO_2NH_2$ |

| | | | |
|---|---|---|---|
| 7-317 | 3-Cl-Ph | 4-Pyr | $SO_2NHMe$ |
| 7-318 | 3-Cl-Ph | 4-Pyr | $SO_2NHEt$ |
| 7-319 | 3-Cl-Ph | 4-Pyr | $SO_2NHPr$ |
| 7-320 | 3-Cl-Ph | 4-Pyr | $SO_2NH$-i-Pr |
| 7-321 | 3-Cl-Ph | 4-Pyr | $SO_2NHBu$ |
| 7-322 | 3-Cl-Ph | 4-Pyr | $SO_2NHBn$ |
| 7-323 | 3-Cl-Ph | 4-Pyr | $SO_2NMe_2$ |
| 7-324 | 3-Cl-Ph | 4-Pyr | $SO_2NH$-c-Pr |
| 7-325 | 3-Cl-Ph | 4-Pyr | $SO_2NH$-c-Bu |
| 7-326 | 3-Cl-Ph | 4-Pyr | $SO_2NH$-c-Pen |
| 7-327 | 3-Cl-Ph | 4-Pyr | $SO_2NH$-c-Hex |
| 7-328 | 3-Cl-Ph | 4-Pyr | $SO_2NH$-c-Hep |
| 7-329 | 3-Cl-Ph | 4-Pyr | $SO_2NHCH_2$-c-Pr |
| 7-330 | 3-Cl-Ph | 4-Pyr | $SO_2NH$-Allyl |
| 7-331 | 3-Cl-Ph | 4-Pyr | $SO_2NH$-Propargyl |
| 7-332 | 3-Cl-Ph | 4-Pyr | $SO_2NHPh$ |
| 7-333 | 3-Cl-Ph | 4-Pyr | $SO_2NH$-3-Pyr |
| 7-334 | 3-Cl-Ph | 4-Pyr | $SO_2NHCH_2$-4-Pyr |
| 7-335 | 3-Cl-Ph | 4-Pyr | $SO_2NH-(CH_2)_2-OH$ |
| 7-336 | 3-Cl-Ph | 4-Pyr | $SO_2NHCH(CH_2OH)_2$ |
| 7-337 | 3-Cl-Ph | 4-Pyr | $SO_2NH-(CH_2)_2-OAc$ |
| 7-338 | 3-Cl-Ph | 4-Pyr | $SO_2NHCH_2CN$ |
| 7-339 | 3-Cl-Ph | 4-Pyr | $SO_2NH-(CH_2)_2-F$ |
| 7-340 | 3-Cl-Ph | 4-Pyr | $SO_2NH-(CH_2)_2-OMe$ |
| 7-341 | 3-Cl-Ph | 4-Pyr | $SO_2NH-(CH_2)_2-SMe$ |
| 7-342 | 3-Cl-Ph | 4-Pyr | $SO_2NH-(CH_2)_2-NH_2$ |
| 7-343 | 3-Cl-Ph | 4-Pyr | $SO_2NH-(CH_2)_2-NHMe$ |
| 7-344 | 3-Cl-Ph | 4-Pyr | $SO_2NH-(CH_2)_2-N(Me)_2$ |
| 7-345 | 3-Cl-Ph | 4-Pyr | $SO_2NHCH_2COOH$ |
| 7-346 | 3-Cl-Ph | 4-Pyr | $SO_2NHCH_2COOEt$ |
| 7-347 | 3-Cl-Ph | 4-Pyr | $SO_2NHCH(Me)COOEt$ |
| 7-348 | 3-Cl-Ph | 4-Pyr | $SO_2NHCH_2CONH_2$ |
| 7-349 | 3-Cl-Ph | 4-Pyr | $SO_2NHOH$ |
| 7-350 | 3-Cl-Ph | 4-Pyr | $SO_2NHOMe$ |
| 7-351 | 3-Cl-Ph | 4-Pyr | $SO_2NHOEt$ |
| 7-352 | 3-Cl-Ph | 4-Pyr | $SO_2NHOPr$ |

| | | | |
|---|---|---|---|
| 7-353 | 3-Cl-Ph | 4-Pyr | $SO_2NHOAllyl$ |
| 7-354 | 3-Cl-Ph | 4-Pyr | $SO_2NHOBn$ |
| 7-355 | 3-Cl-Ph | 4-Pyr | $SO_2NHNH_2$ |
| 7-356 | 3-Cl-Ph | 4-Pyr | $SO_2NHNHMe$ |
| 7-357 | 3-Cl-Ph | 4-Pyr | $SO_2NHN(Me)_2$ |
| 7-358 | 4-F-Ph | 4-Pyr | COOH |
| 7-359 | 4-F-Ph | 4-Pyr | COOMe |
| 7-360 | 4-F-Ph | 4-Pyr | COOEt |
| 7-361 | 4-F-Ph | 4-Pyr | COOPr |
| 7-362 | 4-F-Ph | 4-Pyr | COO-i-Pr |
| 7-363 | 4-F-Ph | 4-Pyr | COOBu |
| 7-364 | 4-F-Ph | 4-Pyr | COOBn |
| 7-365 | 4-F-Ph | 4-Pyr | COOPh |
| 7-366 | 4-F-Ph | 4-Pyr | $CONH_2$ |
| 7-367 | 4-F-Ph | 4-Pyr | CONHMe |
| 7-368 | 4-F-Ph | 4-Pyr | CONHEt |
| 7-369 | 4-F-Ph | 4-Pyr | CONHPr |
| 7-370 | 4-F-Ph | 4-Pyr | CONH-i-Pr |
| 7-371 | 4-F-Ph | 4-Pyr | CONHBu |
| 7-372 | 4-F-Ph | 4-Pyr | CONHBn |
| 7-373 | 4-F-Ph | 4-Pyr | $CONMe_2$ |
| 7-374 | 4-F-Ph | 4-Pyr | CONH-c-Pr |
| 7-375 | 4-F-Ph | 4-Pyr | CONH-c-Bu |
| 7-376 | 4-F-Ph | 4-Pyr | CONH-c-Pen |
| 7-377 | 4-F-Ph | 4-Pyr | CONH-c-Hex |
| 7-378 | 4-F-Ph | 4-Pyr | CONH-c-Hep |
| 7-379 | 4-F-Ph | 4-Pyr | $CONHCH_2$-c-Pr |
| 7-380 | 4-F-Ph | 4-Pyr | CONH-Allyl |
| 7-381 | 4-F-Ph | 4-Pyr | CONH-Propargyl |
| 7-382 | 4-F-Ph | 4-Pyr | CONHPh |
| 7-383 | 4-F-Ph | 4-Pyr | CONH-3-Pyr |
| 7-384 | 4-F-Ph | 4-Pyr | $CONHCH_2$-4-Pyr |
| 7-385 | 4-F-Ph | 4-Pyr | $CONH-(CH_2)_2-OH$ |
| 7-386 | 4-F-Ph | 4-Pyr | $CONHCH(CH_2OH)_2$ |
| 7-387 | 4-F-Ph | 4-Pyr | $CONH-(CH_2)_2-OAc$ |
| 7-388 | 4-F-Ph | 4-Pyr | $CONHCH_2CN$ |

| | | | |
|---|---|---|---|
| 7-389 | 4-F-Ph | 4-Pyr | CONH-$(CH_2)_2$-F |
| 7-390 | 4-F-Ph | 4-Pyr | CONH-$(CH_2)_2$-OMe |
| 7-391 | 4-F-Ph | 4-Pyr | CONH-$(CH_2)_2$-SMe |
| 7-392 | 4-F-Ph | 4-Pyr | CONH-$(CH_2)_2$-$NH_2$ |
| 7-393 | 4-F-Ph | 4-Pyr | CONH-$(CH_2)_2$-NHMe |
| 7-394 | 4-F-Ph | 4-Pyr | CONH-$(CH_2)_2$-$N(Me)_2$ |
| 7-395 | 4-F-Ph | 4-Pyr | $CONHCH_2COOH$ |
| 7-396 | 4-F-Ph | 4-Pyr | $CONHCH_2COOEt$ |
| 7-397 | 4-F-Ph | 4-Pyr | CONHCH(Me)COOEt |
| 7-398 | 4-F-Ph | 4-Pyr | $CONHCH_2CONH_2$ |
| 7-399 | 4-F-Ph | 4-Pyr | CONHOH |
| 7-400 | 4-F-Ph | 4-Pyr | CONHOMe |
| 7-401 | 4-F-Ph | 4-Pyr | CONHOEt |
| 7-402 | 4-F-Ph | 4-Pyr | CONHOPr |
| 7-403 | 4-F-Ph | 4-Pyr | CONHOAllyl |
| 7-404 | 4-F-Ph | 4-Pyr | CONHOBn |
| 7-405 | 4-F-Ph | 4-Pyr | $CONHNH_2$ |
| 7-406 | 4-F-Ph | 4-Pyr | CONHNHMe |
| 7-407 | 4-F-Ph | 4-Pyr | $CONHN(Me)_2$ |
| 7-408 | 4-F-Ph | 4-Pyr | COMe |
| 7-409 | 4-F-Ph | 4-Pyr | COEt |
| 7-410 | 4-F-Ph | 4-Pyr | COPr |
| 7-411 | 4-F-Ph | 4-Pyr | CO-i-Pr |
| 7-412 | 4-F-Ph | 4-Pyr | COBu |
| 7-413 | 4-F-Ph | 4-Pyr | $COCF_3$ |
| 7-414 | 4-F-Ph | 4-Pyr | CO-$(CH_2)_2$-F |
| 7-415 | 4-F-Ph | 4-Pyr | CO-$(CH_2)_2$-OH |
| 7-416 | 4-F-Ph | 4-Pyr | CO-$(CH_2)_2$-OMe |
| 7-417 | 4-F-Ph | 4-Pyr | SOMe |
| 7-418 | 4-F-Ph | 4-Pyr | SOEt |
| 7-419 | 4-F-Ph | 4-Pyr | SOPr |
| 7-420 | 4-F-Ph | 4-Pyr | SO-i-Pr |
| 7-421 | 4-F-Ph | 4-Pyr | SOBu |
| 7-422 | 4-F-Ph | 4-Pyr | $SOCF_3$ |
| 7-423 | 4-F-Ph | 4-Pyr | SO-$(CH_2)_2$-F |
| 7-424 | 4-F-Ph | 4-Pyr | SO-$(CH_2)_2$-OH |

| | | | |
|---|---|---|---|
| 7-425 | 4-F-Ph | 4-Pyr | SO-$(CH_2)_2$-OMe |
| 7-426 | 4-F-Ph | 4-Pyr | $SO_2$Me |
| 7-427 | 4-F-Ph | 4-Pyr | $SO_2$Et |
| 7-428 | 4-F-Ph | 4-Pyr | $SO_2$Pr |
| 7-429 | 4-F-Ph | 4-Pyr | $SO_2$-i-Pr |
| 7-430 | 4-F-Ph | 4-Pyr | $SO_2$Bu |
| 7-431 | 4-F-Ph | 4-Pyr | $SO_2CF_3$ |
| 7-432 | 4-F-Ph | 4-Pyr | $SO_2$-$(CH_2)_2$-F |
| 7-433 | 4-F-Ph | 4-Pyr | $SO_2$-$(CH_2)_2$-OH |
| 7-434 | 4-F-Ph | 4-Pyr | $SO_2$-$(CH_2)_2$-OMe |
| 7-435 | 4-F-Ph | 4-Pyr | $SO_2NH_2$ |
| 7-436 | 4-F-Ph | 4-Pyr | $SO_2$NHMe |
| 7-437 | 4-F-Ph | 4-Pyr | $SO_2$NHEt |
| 7-438 | 4-F-Ph | 4-Pyr | $SO_2$NHPr |
| 7-439 | 4-F-Ph | 4-Pyr | $SO_2$NH-i-Pr |
| 7-440 | 4-F-Ph | 4-Pyr | $SO_2$NHBu |
| 7-441 | 4-F-Ph | 4-Pyr | $SO_2$NHBn |
| 7-442 | 4-F-Ph | 4-Pyr | $SO_2NMe_2$ |
| 7-443 | 4-F-Ph | 4-Pyr | $SO_2$NH-c-Pr |
| 7-444 | 4-F-Ph | 4-Pyr | $SO_2$NH-c-Bu |
| 7-445 | 4-F-Ph | 4-Pyr | $SO_2$NH-c-Pen |
| 7-446 | 4-F-Ph | 4-Pyr | $SO_2$NH-c-Hex |
| 7-447 | 4-F-Ph | 4-Pyr | $SO_2$NH-c-Hep |
| 7-448 | 4-F-Ph | 4-Pyr | $SO_2NHCH_2$-c-Pr |
| 7-449 | 4-F-Ph | 4-Pyr | $SO_2$NH-Allyl |
| 7-450 | 4-F-Ph | 4-Pyr | $SO_2$NH-Propargyl |
| 7-451 | 4-F-Ph | 4-Pyr | $SO_2$NHPh |
| 7-452 | 4-F-Ph | 4-Pyr | $SO_2$NH-3-Pyr |
| 7-453 | 4-F-Ph | 4-Pyr | $SO_2NHCH_2$-4-Pyr |
| 7-454 | 4-F-Ph | 4-Pyr | $SO_2$NH-$(CH_2)_2$-OH |
| 7-455 | 4-F-Ph | 4-Pyr | $SO_2NHCH(CH_2OH)_2$ |
| 7-456 | 4-F-Ph | 4-Pyr | $SO_2$NH-$(CH_2)_2$-OAc |
| 7-457 | 4-F-Ph | 4-Pyr | $SO_2NHCH_2CN$ |
| 7-458 | 4-F-Ph | 4-Pyr | $SO_2$NH-$(CH_2)_2$-F |
| 7-459 | 4-F-Ph | 4-Pyr | $SO_2$NH-$(CH_2)_2$-OMe |
| 7-460 | 4-F-Ph | 4-Pyr | $SO_2$NH-$(CH_2)_2$-SMe |

| | | | |
|---|---|---|---|
| 7-461 | 4-F-Ph | 4-Pyr | $SO_2NH-(CH_2)_2-NH_2$ |
| 7-462 | 4-F-Ph | 4-Pyr | $SO_2NH-(CH_2)_2-NHMe$ |
| 7-463 | 4-F-Ph | 4-Pyr | $SO_2NH-(CH_2)_2-N(Me)_2$ |
| 7-464 | 4-F-Ph | 4-Pyr | $SO_2NHCH_2COOH$ |
| 7-465 | 4-F-Ph | 4-Pyr | $SO_2NHCH_2COOEt$ |
| 7-466 | 4-F-Ph | 4-Pyr | $SO_2NHCH(Me)COOEt$ |
| 7-467 | 4-F-Ph | 4-Pyr | $SO_2NHCH_2CONH_2$ |
| 7-468 | 4-F-Ph | 4-Pyr | $SO_2NHOH$ |
| 7-469 | 4-F-Ph | 4-Pyr | $SO_2NHOMe$ |
| 7-470 | 4-F-Ph | 4-Pyr | $SO_2NHOEt$ |
| 7-471 | 4-F-Ph | 4-Pyr | $SO_2NHOPr$ |
| 7-472 | 4-F-Ph | 4-Pyr | $SO_2NHOAllyl$ |
| 7-473 | 4-F-Ph | 4-Pyr | $SO_2NHOBn$ |
| 7-474 | 4-F-Ph | 4-Pyr | $SO_2NHNH_2$ |
| 7-475 | 4-F-Ph | 4-Pyr | $SO_2NHNHMe$ |
| 7-476 | 4-F-Ph | 4-Pyr | $SO_2NHN(Me)_2$ |
| 7-477 | 3-Cl-4-F-Ph | 4-Pyr | COOH |
| 7-478 | 3-Cl-4-F-Ph | 4-Pyr | COOMe |
| 7-479 | 3-Cl-4-F-Ph | 4-Pyr | COOEt |
| 7-480 | 3-Cl-4-F-Ph | 4-Pyr | COOPr |
| 7-481 | 3-Cl-4-F-Ph | 4-Pyr | COO-i-Pr |
| 7-482 | 3-Cl-4-F-Ph | 4-Pyr | COOBu |
| 7-483 | 3-Cl-4-F-Ph | 4-Pyr | COOBn |
| 7-484 | 3-Cl-4-F-Ph | 4-Pyr | COOPh |
| 7-485 | 3-Cl-4-F-Ph | 4-Pyr | $CONH_2$ |
| 7-486 | 3-Cl-4-F-Ph | 4-Pyr | CONHMe |
| 7-487 | 3-Cl-4-F-Ph | 4-Pyr | CONHEt |
| 7-488 | 3-Cl-4-F-Ph | 4-Pyr | CONHPr |
| 7-489 | 3-Cl-4-F-Ph | 4-Pyr | CONH-i-Pr |
| 7-490 | 3-Cl-4-F-Ph | 4-Pyr | CONHBu |
| 7-491 | 3-Cl-4-F-Ph | 4-Pyr | CONHBn |
| 7-492 | 3-Cl-4-F-Ph | 4-Pyr | $CONMe_2$ |
| 7-493 | 3-Cl-4-F-Ph | 4-Pyr | CONH-c-Pr |
| 7-494 | 3-Cl-4-F-Ph | 4-Pyr | CONH-c-Bu |
| 7-495 | 3-Cl-4-F-Ph | 4-Pyr | CONH-c-Pen |
| 7-496 | 3-Cl-4-F-Ph | 4-Pyr | CONH-c-Hex |

| | | | |
|---|---|---|---|
| 7-497 | 3-Cl-4-F-Ph | 4-Pyr | CONH-c-Hep |
| 7-498 | 3-Cl-4-F-Ph | 4-Pyr | $CONHCH_2$-c-Pr |
| 7-499 | 3-Cl-4-F-Ph | 4-Pyr | CONH-Allyl |
| 7-500 | 3-Cl-4-F-Ph | 4-Pyr | CONH-Propargyl |
| 7-501 | 3-Cl-4-F-Ph | 4-Pyr | CONHPh |
| 7-502 | 3-Cl-4-F-Ph | 4-Pyr | CONH-3-Pyr |
| 7-503 | 3-Cl-4-F-Ph | 4-Pyr | $CONHCH_2$-4-Pyr |
| 7-504 | 3-Cl-4-F-Ph | 4-Pyr | CONH-$(CH_2)_2$-OH |
| 7-505 | 3-Cl-4-F-Ph | 4-Pyr | $CONHCH(CH_2OH)_2$ |
| 7-506 | 3-Cl-4-F-Ph | 4-Pyr | CONH-$(CH_2)_2$-OAc |
| 7-507 | 3-Cl-4-F-Ph | 4-Pyr | $CONHCH_2CN$ |
| 7-508 | 3-Cl-4-F-Ph | 4-Pyr | CONH-$(CH_2)_2$-F |
| 7-509 | 3-Cl-4-F-Ph | 4-Pyr | CONH-$(CH_2)_2$-OMe |
| 7-510 | 3-Cl-4-F-Ph | 4-Pyr | CONH-$(CH_2)_2$-SMe |
| 7-511 | 3-Cl-4-F-Ph | 4-Pyr | CONH-$(CH_2)_2$-$NH_2$ |
| 7-512 | 3-Cl-4-F-Ph | 4-Pyr | CONH-$(CH_2)_2$-NHMe |
| 7-513 | 3-Cl-4-F-Ph | 4-Pyr | CONH-$(CH_2)_2$-$N(Me)_2$ |
| 7-514 | 3-Cl-4-F-Ph | 4-Pyr | $CONHCH_2COOH$ |
| 7-515 | 3-Cl-4-F-Ph | 4-Pyr | $CONHCH_2COOEt$ |
| 7-516 | 3-Cl-4-F-Ph | 4-Pyr | CONHCH(Me)COOEt |
| 7-517 | 3-Cl-4-F-Ph | 4-Pyr | $CONHCH_2CONH_2$ |
| 7-518 | 3-Cl-4-F-Ph | 4-Pyr | CONHOH |
| 7-519 | 3-Cl-4-F-Ph | 4-Pyr | CONHOMe |
| 7-520 | 3-Cl-4-F-Ph | 4-Pyr | CONHOEt |
| 7-521 | 3-Cl-4-F-Ph | 4-Pyr | CONHOPr |
| 7-522 | 3-Cl-4-F-Ph | 4-Pyr | CONHOAllyl |
| 7-523 | 3-Cl-4-F-Ph | 4-Pyr | CONHOBn |
| 7-524 | 3-Cl-4-F-Ph | 4-Pyr | $CONHNH_2$ |
| 7-525 | 3-Cl-4-F-Ph | 4-Pyr | CONHNHMe |
| 7-526 | 3-Cl-4-F-Ph | 4-Pyr | $CONHN(Me)_2$ |
| 7-527 | 3-Cl-4-F-Ph | 4-Pyr | COMe |
| 7-528 | 3-Cl-4-F-Ph | 4-Pyr | COEt |
| 7-529 | 3-Cl-4-F-Ph | 4-Pyr | COPr |
| 7-530 | 3-Cl-4-F-Ph | 4-Pyr | CO-i-Pr |
| 7-531 | 3-Cl-4-F-Ph | 4-Pyr | COBu |
| 7-532 | 3-Cl-4-F-Ph | 4-Pyr | $COCF_3$ |

| | | | |
|---|---|---|---|
| 7-533 | 3-Cl-4-F-Ph | 4-Pyr | CO-$(CH_2)_2$-F |
| 7-534 | 3-Cl-4-F-Ph | 4-Pyr | CO-$(CH_2)_2$-OH |
| 7-535 | 3-Cl-4-F-Ph | 4-Pyr | CO-$(CH_2)_2$-OMe |
| 7-536 | 3-Cl-4-F-Ph | 4-Pyr | SOMe |
| 7-537 | 3-Cl-4-F-Ph | 4-Pyr | SOEt |
| 7-538 | 3-Cl-4-F-Ph | 4-Pyr | SOPr |
| 7-539 | 3-Cl-4-F-Ph | 4-Pyr | SO-i-Pr |
| 7-540 | 3-Cl-4-F-Ph | 4-Pyr | SOBu |
| 7-541 | 3-Cl-4-F-Ph | 4-Pyr | $SOCF_3$ |
| 7-542 | 3-Cl-4-F-Ph | 4-Pyr | SO-$(CH_2)_2$-F |
| 7-543 | 3-Cl-4-F-Ph | 4-Pyr | SO-$(CH_2)_2$-OH |
| 7-544 | 3-Cl-4-F-Ph | 4-Pyr | SO-$(CH_2)_2$-OMe |
| 7-545 | 3-Cl-4-F-Ph | 4-Pyr | $SO_2Me$ |
| 7-546 | 3-Cl-4-F-Ph | 4-Pyr | $SO_2Et$ |
| 7-547 | 3-Cl-4-F-Ph | 4-Pyr | $SO_2Pr$ |
| 7-548 | 3-Cl-4-F-Ph | 4-Pyr | $SO_2$-i-Pr |
| 7-549 | 3-Cl-4-F-Ph | 4-Pyr | $SO_2Bu$ |
| 7-550 | 3-Cl-4-F-Ph | 4-Pyr | $SO_2CF_3$ |
| 7-551 | 3-Cl-4-F-Ph | 4-Pyr | $SO_2$-$(CH_2)_2$-F |
| 7-552 | 3-Cl-4-F-Ph | 4-Pyr | $SO_2$-$(CH_2)_2$-OH |
| 7-553 | 3-Cl-4-F-Ph | 4-Pyr | $SO_2$-$(CH_2)_2$-OMe |
| 7-554 | 3-Cl-4-F-Ph | 4-Pyr | $SO_2NH_2$ |
| 7-555 | 3-Cl-4-F-Ph | 4-Pyr | $SO_2NHMe$ |
| 7-556 | 3-Cl-4-F-Ph | 4-Pyr | $SO_2NHEt$ |
| 7-557 | 3-Cl-4-F-Ph | 4-Pyr | $SO_2NHPr$ |
| 7-558 | 3-Cl-4-F-Ph | 4-Pyr | $SO_2NH$-i-Pr |
| 7-559 | 3-Cl-4-F-Ph | 4-Pyr | $SO_2NHBu$ |
| 7-560 | 3-Cl-4-F-Ph | 4-Pyr | $SO_2NHBn$ |
| 7-561 | 3-Cl-4-F-Ph | 4-Pyr | $SO_2NMe_2$ |
| 7-562 | 3-Cl-4-F-Ph | 4-Pyr | $SO_2NH$-c-Pr |
| 7-563 | 3-Cl-4-F-Ph | 4-Pyr | $SO_2NH$-c-Bu |
| 7-564 | 3-Cl-4-F-Ph | 4-Pyr | $SO_2NH$-c-Pen |
| 7-565 | 3-Cl-4-F-Ph | 4-Pyr | $SO_2NH$-c-Hex |
| 7-566 | 3-Cl-4-F-Ph | 4-Pyr | $SO_2NH$-c-Hep |
| 7-567 | 3-Cl-4-F-Ph | 4-Pyr | $SO_2NHCH_2$-c-Pr |
| 7-568 | 3-Cl-4-F-Ph | 4-Pyr | $SO_2NH$-Allyl |

| | | | |
|---|---|---|---|
| 7-569 | 3-Cl-4-F-Ph | 4-Pyr | $SO_2NH$-Propargyl |
| 7-570 | 3-Cl-4-F-Ph | 4-Pyr | $SO_2NHPh$ |
| 7-571 | 3-Cl-4-F-Ph | 4-Pyr | $SO_2NH$-3-Pyr |
| 7-572 | 3-Cl-4-F-Ph | 4-Pyr | $SO_2NHCH_2$-4-Pyr |
| 7-573 | 3-Cl-4-F-Ph | 4-Pyr | $SO_2NH-(CH_2)_2-OH$ |
| 7-574 | 3-Cl-4-F-Ph | 4-Pyr | $SO_2NHCH(CH_2OH)_2$ |
| 7-575 | 3-Cl-4-F-Ph | 4-Pyr | $SO_2NH-(CH_2)_2-OAc$ |
| 7-576 | 3-Cl-4-F-Ph | 4-Pyr | $SO_2NHCH_2CN$ |
| 7-577 | 3-Cl-4-F-Ph | 4-Pyr | $SO_2NH-(CH_2)_2-F$ |
| 7-578 | 3-Cl-4-F-Ph | 4-Pyr | $SO_2NH-(CH_2)_2-OMe$ |
| 7-579 | 3-Cl-4-F-Ph | 4-Pyr | $SO_2NH-(CH_2)_2-SMe$ |
| 7-580 | 3-Cl-4-F-Ph | 4-Pyr | $SO_2NH-(CH_2)_2-NH_2$ |
| 7-581 | 3-Cl-4-F-Ph | 4-Pyr | $SO_2NH-(CH_2)_2-NHMe$ |
| 7-582 | 3-Cl-4-F-Ph | 4-Pyr | $SO_2NH-(CH_2)_2-N(Me)_2$ |
| 7-583 | 3-Cl-4-F-Ph | 4-Pyr | $SO_2NHCH_2COOH$ |
| 7-584 | 3-Cl-4-F-Ph | 4-Pyr | $SO_2NHCH_2COOEt$ |
| 7-585 | 3-Cl-4-F-Ph | 4-Pyr | $SO_2NHCH(Me)COOEt$ |
| 7-586 | 3-Cl-4-F-Ph | 4-Pyr | $SO_2NHCH_2CONH_2$ |
| 7-587 | 3-Cl-4-F-Ph | 4-Pyr | $SO_2NHOH$ |
| 7-588 | 3-Cl-4-F-Ph | 4-Pyr | $SO_2NHOMe$ |
| 7-589 | 3-Cl-4-F-Ph | 4-Pyr | $SO_2NHOEt$ |
| 7-590 | 3-Cl-4-F-Ph | 4-Pyr | $SO_2NHOPr$ |
| 7-591 | 3-Cl-4-F-Ph | 4-Pyr | $SO_2NHOAllyl$ |
| 7-592 | 3-Cl-4-F-Ph | 4-Pyr | $SO_2NHOBn$ |
| 7-593 | 3-Cl-4-F-Ph | 4-Pyr | $SO_2NHNH_2$ |
| 7-594 | 3-Cl-4-F-Ph | 4-Pyr | $SO_2NHNHMe$ |
| 7-595 | 3-Cl-4-F-Ph | 4-Pyr | $SO_2NHN(Me)_2$ |
| 7-596 | 3,4-$F_2$-Ph | 4-Pyr | COOH |
| 7-597 | 3,4-$F_2$-Ph | 4-Pyr | COOMe |
| 7-598 | 3,4-$F_2$-Ph | 4-Pyr | COOEt |
| 7-599 | 3,4-$F_2$-Ph | 4-Pyr | COOPr |
| 7-600 | 3,4-$F_2$-Ph | 4-Pyr | COO-i-Pr |
| 7-601 | 3,4-$F_2$-Ph | 4-Pyr | COOBu |
| 7-602 | 3,4-$F_2$-Ph | 4-Pyr | COOBn |
| 7-603 | 3,4-$F_2$-Ph | 4-Pyr | COOPh |
| 7-604 | 3,4-$F_2$-Ph | 4-Pyr | $CONH_2$ |

| | | | |
|---|---|---|---|
| 7-605 | 3,4-$F_2$-Ph | 4-Pyr | CONHMe |
| 7-606 | 3,4-$F_2$-Ph | 4-Pyr | CONHEt |
| 7-607 | 3,4-$F_2$-Ph | 4-Pyr | CONHPr |
| 7-608 | 3,4-$F_2$-Ph | 4-Pyr | CONH-i-Pr |
| 7-609 | 3,4-$F_2$-Ph | 4-Pyr | CONHBu |
| 7-610 | 3,4-$F_2$-Ph | 4-Pyr | CONHBn |
| 7-611 | 3,4-$F_2$-Ph | 4-Pyr | $CONMe_2$ |
| 7-612 | 3,4-$F_2$-Ph | 4-Pyr | CONH-c-Pr |
| 7-613 | 3,4-$F_2$-Ph | 4-Pyr | CONH-c-Bu |
| 7-614 | 3,4-$F_2$-Ph | 4-Pyr | CONH-c-Pen |
| 7-615 | 3,4-$F_2$-Ph | 4-Pyr | CONH-c-Hex |
| 7-616 | 3,4-$F_2$-Ph | 4-Pyr | CONH-c-Hep |
| 7-617 | 3,4-$F_2$-Ph | 4-Pyr | $CONHCH_2$-c-Pr |
| 7-618 | 3,4-$F_2$-Ph | 4-Pyr | CONH-Allyl |
| 7-619 | 3,4-$F_2$-Ph | 4-Pyr | CONH-Propargyl |
| 7-620 | 3,4-$F_2$-Ph | 4-Pyr | CONHPh |
| 7-621 | 3,4-$F_2$-Ph | 4-Pyr | CONH-3-Pyr |
| 7-622 | 3,4-$F_2$-Ph | 4-Pyr | $CONHCH_2$-4-Pyr |
| 7-623 | 3,4-$F_2$-Ph | 4-Pyr | CONH-$(CH_2)_2$-OH |
| 7-624 | 3,4-$F_2$-Ph | 4-Pyr | $CONHCH(CH_2OH)_2$ |
| 7-625 | 3,4-$F_2$-Ph | 4-Pyr | CONH-$(CH_2)_2$-OAc |
| 7-626 | 3,4-$F_2$-Ph | 4-Pyr | $CONHCH_2CN$ |
| 7-627 | 3,4-$F_2$-Ph | 4-Pyr | CONH-$(CH_2)_2$-F |
| 7-628 | 3,4-$F_2$-Ph | 4-Pyr | CONH-$(CH_2)_2$-OMe |
| 7-629 | 3,4-$F_2$-Ph | 4-Pyr | CONH-$(CH_2)_2$-SMe |
| 7-630 | 3,4-$F_2$-Ph | 4-Pyr | CONH-$(CH_2)_2$-$NH_2$ |
| 7-631 | 3,4-$F_2$-Ph | 4-Pyr | CONH-$(CH_2)_2$-NHMe |
| 7-632 | 3,4-$F_2$-Ph | 4-Pyr | CONH-$(CH_2)_2$-$N(Me)_2$ |
| 7-633 | 3,4-$F_2$-Ph | 4-Pyr | $CONHCH_2COOH$ |
| 7-634 | 3,4-$F_2$-Ph | 4-Pyr | $CONHCH_2COOEt$ |
| 7-635 | 3,4-$F_2$-Ph | 4-Pyr | CONHCH(Me)COOEt |
| 7-636 | 3,4-$F_2$-Ph | 4-Pyr | $CONHCH_2CONH_2$ |
| 7-637 | 3,4-$F_2$-Ph | 4-Pyr | CONHOH |
| 7-638 | 3,4-$F_2$-Ph | 4-Pyr | CONHOMe |
| 7-639 | 3,4-$F_2$-Ph | 4-Pyr | CONHOEt |
| 7-640 | 3,4-$F_2$-Ph | 4-Pyr | CONHOPr |

| | | | |
|---|---|---|---|
| 7-641 | 3,4-$F_2$-Ph | 4-Pyr | CONHOAllyl |
| 7-642 | 3,4-$F_2$-Ph | 4-Pyr | CONHOBn |
| 7-643 | 3,4-$F_2$-Ph | 4-Pyr | $CONHNH_2$ |
| 7-644 | 3,4-$F_2$-Ph | 4-Pyr | CONHNHMe |
| 7-645 | 3,4-$F_2$-Ph | 4-Pyr | $CONHN(Me)_2$ |
| 7-646 | 3,4-$F_2$-Ph | 4-Pyr | COMe |
| 7-647 | 3,4-$F_2$-Ph | 4-Pyr | COEt |
| 7-648 | 3,4-$F_2$-Ph | 4-Pyr | COPr |
| 7-649 | 3,4-$F_2$-Ph | 4-Pyr | CO-i-Pr |
| 7-650 | 3,4-$F_2$-Ph | 4-Pyr | COBu |
| 7-651 | 3,4-$F_2$-Ph | 4-Pyr | $COCF_3$ |
| 7-652 | 3,4-$F_2$-Ph | 4-Pyr | CO-$(CH_2)_2$-F |
| 7-653 | 3,4-$F_2$-Ph | 4-Pyr | CO-$(CH_2)_2$-OH |
| 7-654 | 3,4-$F_2$-Ph | 4-Pyr | CO-$(CH_2)_2$-OMe |
| 7-655 | 3,4-$F_2$-Ph | 4-Pyr | SOMe |
| 7-656 | 3,4-$F_2$-Ph | 4-Pyr | SOEt |
| 7-657 | 3,4-$F_2$-Ph | 4-Pyr | SOPr |
| 7-658 | 3,4-$F_2$-Ph | 4-Pyr | SO-i-Pr |
| 7-659 | 3,4-$F_2$-Ph | 4-Pyr | SOBu |
| 7-660 | 3,4-$F_2$-Ph | 4-Pyr | $SOCF_3$ |
| 7-661 | 3,4-$F_2$-Ph | 4-Pyr | SO-$(CH_2)_2$-F |
| 7-662 | 3,4-$F_2$-Ph | 4-Pyr | SO-$(CH_2)_2$-OH |
| 7-663 | 3,4-$F_2$-Ph | 4-Pyr | SO-$(CH_2)_2$-OMe |
| 7-664 | 3,4-$F_2$-Ph | 4-Pyr | $SO_2Me$ |
| 7-665 | 3,4-$F_2$-Ph | 4-Pyr | $SO_2Et$ |
| 7-666 | 3,4-$F_2$-Ph | 4-Pyr | $SO_2Pr$ |
| 7-667 | 3,4-$F_2$-Ph | 4-Pyr | $SO_2$-i-Pr |
| 7-668 | 3,4-$F_2$-Ph | 4-Pyr | $SO_2Bu$ |
| 7-669 | 3,4-$F_2$-Ph | 4-Pyr | $SO_2CF_3$ |
| 7-670 | 3,4-$F_2$-Ph | 4-Pyr | $SO_2$-$(CH_2)_2$-F |
| 7-671 | 3,4-$F_2$-Ph | 4-Pyr | $SO_2$-$(CH_2)_2$-OH |
| 7-672 | 3,4-$F_2$-Ph | 4-Pyr | $SO_2$-$(CH_2)_2$-OMe |
| 7-673 | 3,4-$F_2$-Ph | 4-Pyr | $SO_2NH_2$ |
| 7-674 | 3,4-$F_2$-Ph | 4-Pyr | $SO_2NHMe$ |
| 7-675 | 3,4-$F_2$-Ph | 4-Pyr | $SO_2NHEt$ |
| 7-676 | 3,4-$F_2$-Ph | 4-Pyr | $SO_2NHPr$ |

| | | | |
|---|---|---|---|
| 7-677 | 3,4-$F_2$-Ph | 4-Pyr | $SO_2$NH-i-Pr |
| 7-678 | 3,4-$F_2$-Ph | 4-Pyr | $SO_2$NHBu |
| 7-679 | 3,4-$F_2$-Ph | 4-Pyr | $SO_2$NHBn |
| 7-680 | 3,4-$F_2$-Ph | 4-Pyr | $SO_2NMe_2$ |
| 7-681 | 3,4-$F_2$-Ph | 4-Pyr | $SO_2$NH-c-Pr |
| 7-682 | 3,4-$F_2$-Ph | 4-Pyr | $SO_2$NH-c-Bu |
| 7-683 | 3,4-$F_2$-Ph | 4-Pyr | $SO_2$NH-c-Pen |
| 7-684 | 3,4-$F_2$-Ph | 4-Pyr | $SO_2$NH-c-Hex |
| 7-685 | 3,4-$F_2$-Ph | 4-Pyr | $SO_2$NH-c-Hep |
| 7-686 | 3,4-$F_2$-Ph | 4-Pyr | $SO_2NHCH_2$-c-Pr |
| 7-687 | 3,4-$F_2$-Ph | 4-Pyr | $SO_2$NH-Allyl |
| 7-688 | 3,4-$F_2$-Ph | 4-Pyr | $SO_2$NH-Propargyl |
| 7-689 | 3,4-$F_2$-Ph | 4-Pyr | $SO_2$NHPh |
| 7-690 | 3,4-$F_2$-Ph | 4-Pyr | $SO_2$NH-3-Pyr |
| 7-691 | 3,4-$F_2$-Ph | 4-Pyr | $SO_2NHCH_2$-4-Pyr |
| 7-692 | 3,4-$F_2$-Ph | 4-Pyr | $SO_2NH-(CH_2)_2-OH$ |
| 7-693 | 3,4-$F_2$-Ph | 4-Pyr | $SO_2NHCH(CH_2OH)_2$ |
| 7-694 | 3,4-$F_2$-Ph | 4-Pyr | $SO_2NH-(CH_2)_2-OAc$ |
| 7-695 | 3,4-$F_2$-Ph | 4-Pyr | $SO_2NHCH_2CN$ |
| 7-696 | 3,4-$F_2$-Ph | 4-Pyr | $SO_2NH-(CH_2)_2-F$ |
| 7-697 | 3,4-$F_2$-Ph | 4-Pyr | $SO_2NH-(CH_2)_2-OMe$ |
| 7-698 | 3,4-$F_2$-Ph | 4-Pyr | $SO_2NH-(CH_2)_2-SMe$ |
| 7-699 | 3,4-$F_2$-Ph | 4-Pyr | $SO_2NH-(CH_2)_2-NH_2$ |
| 7-700 | 3,4-$F_2$-Ph | 4-Pyr | $SO_2NH-(CH_2)_2-NHMe$ |
| 7-701 | 3,4-$F_2$-Ph | 4-Pyr | $SO_2NH-(CH_2)_2-N(Me)_2$ |
| 7-702 | 3,4-$F_2$-Ph | 4-Pyr | $SO_2NHCH_2COOH$ |
| 7-703 | 3,4-$F_2$-Ph | 4-Pyr | $SO_2NHCH_2COOEt$ |
| 7-704 | 3,4-$F_2$-Ph | 4-Pyr | $SO_2$NHCH(Me)COOEt |
| 7-705 | 3,4-$F_2$-Ph | 4-Pyr | $SO_2NHCH_2CONH_2$ |
| 7-706 | 3,4-$F_2$-Ph | 4-Pyr | $SO_2$NHOH |
| 7-707 | 3,4-$F_2$-Ph | 4-Pyr | $SO_2$NHOMe |
| 7-708 | 3,4-$F_2$-Ph | 4-Pyr | $SO_2$NHOEt |
| 7-709 | 3,4-$F_2$-Ph | 4-Pyr | $SO_2$NHOPr |
| 7-710 | 3,4-$F_2$-Ph | 4-Pyr | $SO_2$NHOAllyl |
| 7-711 | 3,4-$F_2$-Ph | 4-Pyr | $SO_2$NHOBn |
| 7-712 | 3,4-$F_2$-Ph | 4-Pyr | $SO_2NHNH_2$ |

| | | | |
|---|---|---|---|
| 7-713 | 3,4-$F_2$-Ph | 4-Pyr | $SO_2$NHNHMe |
| 7-714 | 3,4-$F_2$-Ph | 4-Pyr | $SO_2$NHN(Me)$_2$ |
| 7-715 | 3-$CF_3$-Ph | 4-Pyr | COOH |
| 7-716 | 3-$CF_3$-Ph | 4-Pyr | COOMe |
| 7-717 | 3-$CF_3$-Ph | 4-Pyr | COOEt |
| 7-718 | 3-$CF_3$-Ph | 4-Pyr | COOPr |
| 7-719 | 3-$CF_3$-Ph | 4-Pyr | COO-i-Pr |
| 7-720 | 3-$CF_3$-Ph | 4-Pyr | COOBu |
| 7-721 | 3-$CF_3$-Ph | 4-Pyr | COOBn |
| 7-722 | 3-$CF_3$-Ph | 4-Pyr | COOPh |
| 7-723 | 3-$CF_3$-Ph | 4-Pyr | $CONH_2$ |
| 7-724 | 3-$CF_3$-Ph | 4-Pyr | CONHMe |
| 7-725 | 3-$CF_3$-Ph | 4-Pyr | CONHEt |
| 7-726 | 3-$CF_3$-Ph | 4-Pyr | CONHPr |
| 7-727 | 3-$CF_3$-Ph | 4-Pyr | CONH-i-Pr |
| 7-728 | 3-$CF_3$-Ph | 4-Pyr | CONHBu |
| 7-729 | 3-$CF_3$-Ph | 4-Pyr | CONHBn |
| 7-730 | 3-$CF_3$-Ph | 4-Pyr | $CONMe_2$ |
| 7-731 | 3-$CF_3$-Ph | 4-Pyr | CONH-c-Pr |
| 7-732 | 3-$CF_3$-Ph | 4-Pyr | CONH-c-Bu |
| 7-733 | 3-$CF_3$-Ph | 4-Pyr | CONH-c-Pen |
| 7-734 | 3-$CF_3$-Ph | 4-Pyr | CONH-c-Hex |
| 7-735 | 3-$CF_3$-Ph | 4-Pyr | CONH-c-Hep |
| 7-736 | 3-$CF_3$-Ph | 4-Pyr | $CONHCH_2$-c-Pr |
| 7-737 | 3-$CF_3$-Ph | 4-Pyr | CONH-Allyl |
| 7-738 | 3-$CF_3$-Ph | 4-Pyr | CONH-Propargyl |
| 7-739 | 3-$CF_3$-Ph | 4-Pyr | CONHPh |
| 7-740 | 3-$CF_3$-Ph | 4-Pyr | CONH-3-Pyr |
| 7-741 | 3-$CF_3$-Ph | 4-Pyr | $CONHCH_2$-4-Pyr |
| 7-742 | 3-$CF_3$-Ph | 4-Pyr | CONH-$(CH_2)_2$-OH |
| 7-743 | 3-$CF_3$-Ph | 4-Pyr | $CONHCH(CH_2OH)_2$ |
| 7-744 | 3-$CF_3$-Ph | 4-Pyr | CONH-$(CH_2)_2$-OAc |
| 7-745 | 3-$CF_3$-Ph | 4-Pyr | $CONHCH_2CN$ |
| 7-746 | 3-$CF_3$-Ph | 4-Pyr | CONH-$(CH_2)_2$-F |
| 7-747 | 3-$CF_3$-Ph | 4-Pyr | CONH-$(CH_2)_2$-OMe |
| 7-748 | 3-$CF_3$-Ph | 4-Pyr | CONH-$(CH_2)_2$-SMe |

| | | | |
|---|---|---|---|
| 7-749 | 3-$CF_3$-Ph | 4-Pyr | CONH-$(CH_2)_2$-$NH_2$ |
| 7-750 | 3-$CF_3$-Ph | 4-Pyr | CONH-$(CH_2)_2$-NHMe |
| 7-751 | 3-$CF_3$-Ph | 4-Pyr | CONH-$(CH_2)_2$-N(Me)$_2$ |
| 7-752 | 3-$CF_3$-Ph | 4-Pyr | $CONHCH_2COOH$ |
| 7-753 | 3-$CF_3$-Ph | 4-Pyr | $CONHCH_2COOEt$ |
| 7-754 | 3-$CF_3$-Ph | 4-Pyr | CONHCH(Me)COOEt |
| 7-755 | 3-$CF_3$-Ph | 4-Pyr | $CONHCH_2CONH_2$ |
| 7-756 | 3-$CF_3$-Ph | 4-Pyr | CONHOH |
| 7-757 | 3-$CF_3$-Ph | 4-Pyr | CONHOMe |
| 7-758 | 3-$CF_3$-Ph | 4-Pyr | CONHOEt |
| 7-759 | 3-$CF_3$-Ph | 4-Pyr | CONHOPr |
| 7-760 | 3-$CF_3$-Ph | 4-Pyr | CONHOAllyl |
| 7-761 | 3-$CF_3$-Ph | 4-Pyr | CONHOBn |
| 7-762 | 3-$CF_3$-Ph | 4-Pyr | $CONHNH_2$ |
| 7-763 | 3-$CF_3$-Ph | 4-Pyr | CONHNHMe |
| 7-764 | 3-$CF_3$-Ph | 4-Pyr | CONHN(Me)$_2$ |
| 7-765 | 3-$CF_3$-Ph | 4-Pyr | COMe |
| 7-766 | 3-$CF_3$-Ph | 4-Pyr | COEt |
| 7-767 | 3-$CF_3$-Ph | 4-Pyr | COPr |
| 7-768 | 3-$CF_3$-Ph | 4-Pyr | CO-i-Pr |
| 7-769 | 3-$CF_3$-Ph | 4-Pyr | COBu |
| 7-770 | 3-$CF_3$-Ph | 4-Pyr | $COCF_3$ |
| 7-771 | 3-$CF_3$-Ph | 4-Pyr | CO-$(CH_2)_2$-F |
| 7-772 | 3-$CF_3$-Ph | 4-Pyr | CO-$(CH_2)_2$-OH |
| 7-773 | 3-$CF_3$-Ph | 4-Pyr | CO-$(CH_2)_2$-OMe |
| 7-774 | 3-$CF_3$-Ph | 4-Pyr | SOMe |
| 7-775 | 3-$CF_3$-Ph | 4-Pyr | SOEt |
| 7-776 | 3-$CF_3$-Ph | 4-Pyr | SOPr |
| 7-777 | 3-$CF_3$-Ph | 4-Pyr | SO-i-Pr |
| 7-778 | 3-$CF_3$-Ph | 4-Pyr | SOBu |
| 7-779 | 3-$CF_3$-Ph | 4-Pyr | $SOCF_3$ |
| 7-780 | 3-$CF_3$-Ph | 4-Pyr | SO-$(CH_2)_2$-F |
| 7-781 | 3-$CF_3$-Ph | 4-Pyr | SO-$(CH_2)_2$-OH |
| 7-782 | 3-$CF_3$-Ph | 4-Pyr | SO-$(CH_2)_2$-OMe |
| 7-783 | 3-$CF_3$-Ph | 4-Pyr | $SO_2Me$ |
| 7-784 | 3-$CF_3$-Ph | 4-Pyr | $SO_2Et$ |

| | | | |
|---|---|---|---|
| 7-785 | 3-$CF_3$-Ph | 4-Pyr | $SO_2$Pr |
| 7-786 | 3-$CF_3$-Ph | 4-Pyr | $SO_2$-i-Pr |
| 7-787 | 3-$CF_3$-Ph | 4-Pyr | $SO_2$Bu |
| 7-788 | 3-$CF_3$-Ph | 4-Pyr | $SO_2CF_3$ |
| 7-789 | 3-$CF_3$-Ph | 4-Pyr | $SO_2$-$(CH_2)_2$-F |
| 7-790 | 3-$CF_3$-Ph | 4-Pyr | $SO_2$-$(CH_2)_2$-OH |
| 7-791 | 3-$CF_3$-Ph | 4-Pyr | $SO_2$-$(CH_2)_2$-OMe |
| 7-792 | 3-$CF_3$-Ph | 4-Pyr | $SO_2NH_2$ |
| 7-793 | 3-$CF_3$-Ph | 4-Pyr | $SO_2$NHMe |
| 7-794 | 3-$CF_3$-Ph | 4-Pyr | $SO_2$NHEt |
| 7-795 | 3-$CF_3$-Ph | 4-Pyr | $SO_2$NHPr |
| 7-796 | 3-$CF_3$-Ph | 4-Pyr | $SO_2$NH-i-Pr |
| 7-797 | 3-$CF_3$-Ph | 4-Pyr | $SO_2$NHBu |
| 7-798 | 3-$CF_3$-Ph | 4-Pyr | $SO_2$NHBn |
| 7-799 | 3-$CF_3$-Ph | 4-Pyr | $SO_2NMe_2$ |
| 7-800 | 3-$CF_3$-Ph | 4-Pyr | $SO_2$NH-c-Pr |
| 7-801 | 3-$CF_3$-Ph | 4-Pyr | $SO_2$NH-c-Bu |
| 7-802 | 3-$CF_3$-Ph | 4-Pyr | $SO_2$NH-c-Pen |
| 7-803 | 3-$CF_3$-Ph | 4-Pyr | $SO_2$NH-c-Hex |
| 7-804 | 3-$CF_3$-Ph | 4-Pyr | $SO_2$NH-c-Hep |
| 7-805 | 3-$CF_3$-Ph | 4-Pyr | $SO_2NHCH_2$-c-Pr |
| 7-806 | 3-$CF_3$-Ph | 4-Pyr | $SO_2$NH-Allyl |
| 7-807 | 3-$CF_3$-Ph | 4-Pyr | $SO_2$NH-Propargyl |
| 7-808 | 3-$CF_3$-Ph | 4-Pyr | $SO_2$NHPh |
| 7-809 | 3-$CF_3$-Ph | 4-Pyr | $SO_2$NH-3-Pyr |
| 7-810 | 3-$CF_3$-Ph | 4-Pyr | $SO_2NHCH_2$-4-Pyr |
| 7-811 | 3-$CF_3$-Ph | 4-Pyr | $SO_2$NH-$(CH_2)_2$-OH |
| 7-812 | 3-$CF_3$-Ph | 4-Pyr | $SO_2NHCH(CH_2OH)_2$ |
| 7-813 | 3-$CF_3$-Ph | 4-Pyr | $SO_2$NH-$(CH_2)_2$-OAc |
| 7-814 | 3-$CF_3$-Ph | 4-Pyr | $SO_2NHCH_2CN$ |
| 7-815 | 3-$CF_3$-Ph | 4-Pyr | $SO_2$NH-$(CH_2)_2$-F |
| 7-816 | 3-$CF_3$-Ph | 4-Pyr | $SO_2$NH-$(CH_2)_2$-OMe |
| 7-817 | 3-$CF_3$-Ph | 4-Pyr | $SO_2$NH-$(CH_2)_2$-SMe |
| 7-818 | 3-$CF_3$-Ph | 4-Pyr | $SO_2$NH-$(CH_2)_2$-$NH_2$ |
| 7-819 | 3-$CF_3$-Ph | 4-Pyr | $SO_2$NH-$(CH_2)_2$-NHMe |
| 7-820 | 3-$CF_3$-Ph | 4-Pyr | $SO_2$NH-$(CH_2)_2$-N$(Me)_2$ |

| | | | |
|---|---|---|---|
| 7-821 | 3-$CF_3$-Ph | 4-Pyr | $SO_2NHCH_2COOH$ |
| 7-822 | 3-$CF_3$-Ph | 4-Pyr | $SO_2NHCH_2COOEt$ |
| 7-823 | 3-$CF_3$-Ph | 4-Pyr | $SO_2NHCH(Me)COOEt$ |
| 7-824 | 3-$CF_3$-Ph | 4-Pyr | $SO_2NHCH_2CONH_2$ |
| 7-825 | 3-$CF_3$-Ph | 4-Pyr | $SO_2NHOH$ |
| 7-826 | 3-$CF_3$-Ph | 4-Pyr | $SO_2NHOMe$ |
| 7-827 | 3-$CF_3$-Ph | 4-Pyr | $SO_2NHOEt$ |
| 7-828 | 3-$CF_3$-Ph | 4-Pyr | $SO_2NHOPr$ |
| 7-829 | 3-$CF_3$-Ph | 4-Pyr | $SO_2NHOAllyl$ |
| 7-830 | 3-$CF_3$-Ph | 4-Pyr | $SO_2NHOBn$ |
| 7-831 | 3-$CF_3$-Ph | 4-Pyr | $SO_2NHNH_2$ |
| 7-832 | 3-$CF_3$-Ph | 4-Pyr | $SO_2NHNHMe$ |
| 7-833 | 3-$CF_3$-Ph | 4-Pyr | $SO_2NHN(Me)_2$ |
| 7-834 | 4-F-Ph | 4-Pym | $CONH_2$ |
| 7-835 | 4-F-Ph | 4-Pym | CONHMe |
| 7-836 | 4-F-Ph | 4-Pym | CONHEt |
| 7-837 | 4-F-Ph | 4-Pym | CONHPr |
| 7-838 | 4-F-Ph | 4-Pym | CONH-i-Pr |
| 7-839 | 4-F-Ph | 4-Pym | CONHBu |
| 7-840 | 4-F-Ph | 4-Pym | CONHBn |
| 7-841 | 4-F-Ph | 4-Pym | $CONMe_2$ |
| 7-842 | 4-F-Ph | 4-Pym | CONH-c-Pr |
| 7-843 | 4-F-Ph | 4-Pym | CONH-c-Bu |
| 7-844 | 4-F-Ph | 4-Pym | CONH-c-Pen |
| 7-845 | 4-F-Ph | 4-Pym | CONH-c-Hex |
| 7-846 | 4-F-Ph | 4-Pym | CONH-c-Hep |
| 7-847 | 4-F-Ph | 4-Pym | $CONHCH_2$-c-Pr |
| 7-848 | 4-F-Ph | 4-Pym | CONH-Allyl |
| 7-849 | 4-F-Ph | 4-Pym | CONH-Propargyl |
| 7-850 | 4-F-Ph | 4-Pym | CONHPh |
| 7-851 | 4-F-Ph | 4-Pym | CONH-3-Pyr |
| 7-852 | 4-F-Ph | 4-Pym | $CONHCH_2$-4-Pyr |
| 7-853 | 4-F-Ph | 4-Pym | CONH-$(CH_2)_2$-OH |
| 7-854 | 4-F-Ph | 4-Pym | $CONHCH(CH_2OH)_2$ |
| 7-855 | 4-F-Ph | 4-Pym | CONH-$(CH_2)_2$-OAc |
| 7-856 | 4-F-Ph | 4-Pym | $CONHCH_2CN$ |

| | | | |
|---|---|---|---|
| 7-857 | 4-F-Ph | 4-Pym | CONH-$(CH_2)_2$-F |
| 7-858 | 4-F-Ph | 4-Pym | CONH-$(CH_2)_2$-OMe |
| 7-859 | 4-F-Ph | 4-Pym | CONH-$(CH_2)_2$-SMe |
| 7-860 | 4-F-Ph | 4-Pym | CONH-$(CH_2)_2$-$NH_2$ |
| 7-861 | 4-F-Ph | 4-Pym | CONH-$(CH_2)_2$-NHMe |
| 7-862 | 4-F-Ph | 4-Pym | CONH-$(CH_2)_2$-N$(Me)_2$ |
| 7-863 | 4-F-Ph | 4-Pym | $CONHCH_2COOH$ |
| 7-864 | 4-F-Ph | 4-Pym | $CONHCH_2COOEt$ |
| 7-865 | 4-F-Ph | 4-Pym | CONHCH(Me)COOEt |
| 7-866 | 4-F-Ph | 4-Pym | $CONHCH_2CONH_2$ |
| 7-867 | 4-F-Ph | 4-Pym | $SO_2NH_2$ |
| 7-868 | 4-F-Ph | 4-Pym | $SO_2NHMe$ |
| 7-869 | 4-F-Ph | 4-Pym | $SO_2NHEt$ |
| 7-870 | 4-F-Ph | 4-Pym | $SO_2NHPr$ |
| 7-871 | 4-F-Ph | 4-Pym | $SO_2NH$-i-Pr |
| 7-872 | 4-F-Ph | 4-Pym | $SO_2NHBu$ |
| 7-873 | 4-F-Ph | 4-Pym | $SO_2NHBn$ |
| 7-874 | 4-F-Ph | 4-Pym | $SO_2NMe_2$ |
| 7-875 | 4-F-Ph | 4-Pym | $SO_2NH$-c-Pr |
| 7-876 | 4-F-Ph | 4-Pym | $SO_2NH$-c-Bu |
| 7-877 | 4-F-Ph | 4-Pym | $SO_2NH$-c-Pen |
| 7-878 | 4-F-Ph | 4-Pym | $SO_2NH$-c-Hex |
| 7-879 | 4-F-Ph | 4-Pym | $SO_2NH$-c-Hep |
| 7-880 | 4-F-Ph | 4-Pym | $SO_2NHCH_2$-c-Pr |
| 7-881 | 4-F-Ph | 4-Pym | $SO_2NH$-Allyl |
| 7-882 | 4-F-Ph | 4-Pym | $SO_2NH$-Propargyl |
| 7-883 | 4-F-Ph | 4-Pym | $SO_2NHPh$ |
| 7-884 | 4-F-Ph | 4-Pym | $SO_2NH$-3-Pyr |
| 7-885 | 4-F-Ph | 4-Pym | $SO_2NHCH_2$-4-Pyr |
| 7-886 | 4-F-Ph | 4-Pym | $SO_2NH$-$(CH_2)_2$-OH |
| 7-887 | 4-F-Ph | 4-Pym | $SO_2NHCH(CH_2OH)_2$ |
| 7-888 | 4-F-Ph | 4-Pym | $SO_2NH$-$(CH_2)_2$-OAc |
| 7-889 | 4-F-Ph | 4-Pym | $SO_2NHCH_2CN$ |
| 7-890 | 4-F-Ph | 4-Pym | $SO_2NH$-$(CH_2)_2$-F |
| 7-891 | 4-F-Ph | 4-Pym | $SO_2NH$-$(CH_2)_2$-OMe |
| 7-892 | 4-F-Ph | 4-Pym | $SO_2NH$-$(CH_2)_2$-SMe |

| | | | |
|---|---|---|---|
| 7-893 | 4-F-Ph | 4-Pym | $SO_2NH-(CH_2)_2-NH_2$ |
| 7-894 | 4-F-Ph | 4-Pym | $SO_2NH-(CH_2)_2-NHMe$ |
| 7-895 | 4-F-Ph | 4-Pym | $SO_2NH-(CH_2)_2-N(Me)_2$ |
| 7-896 | 4-F-Ph | 4-Pym | $SO_2NHCH_2COOH$ |
| 7-897 | 4-F-Ph | 4-Pym | $SO_2NHCH_2COOEt$ |
| 7-898 | 4-F-Ph | 4-Pym | $SO_2NHCH(Me)COOEt$ |
| 7-899 | 4-F-Ph | 4-Pym | $SO_2NHCH_2CONH_2$ |
| 7-900 | 4-F-Ph | 4-Pym | $SO_2NHOH$ |
| 7-901 | 4-F-Ph | 4-Pym | $SO_2NHOMe$ |
| 7-902 | 4-F-Ph | 4-Pym | $SO_2NHOEt$ |
| 7-903 | 4-F-Ph | 4-Pym | $SO_2NHOPr$ |
| 7-904 | 4-F-Ph | 4-Pym | $SO_2NHOAllyl$ |
| 7-905 | 4-F-Ph | 4-Pym | $SO_2NHOBn$ |
| 7-906 | 4-F-Ph | 4-Pym | $SO_2NHNH_2$ |
| 7-907 | 4-F-Ph | 4-Pym | $SO_2NHNHMe$ |
| 7-908 | 4-F-Ph | 4-Pym | $SO_2NHN(Me)_2$ |
| 7-909 | 4-F-Ph | 2-MeO-4-Pym | $CONH_2$ |
| 7-910 | 4-F-Ph | 2-MeO-4-Pym | CONHMe |
| 7-911 | 4-F-Ph | 2-MeO-4-Pym | CONHEt |
| 7-912 | 4-F-Ph | 2-MeO-4-Pym | CONHPr |
| 7-913 | 4-F-Ph | 2-MeO-4-Pym | CONH-i-Pr |
| 7-914 | 4-F-Ph | 2-MeO-4-Pym | CONHBu |
| 7-915 | 4-F-Ph | 2-MeO-4-Pym | CONHBn |
| 7-916 | 4-F-Ph | 2-MeO-4-Pym | $CONMe_2$ |
| 7-917 | 4-F-Ph | 2-MeO-4-Pym | CONH-c-Pr |
| 7-918 | 4-F-Ph | 2-MeO-4-Pym | CONH-c-Bu |
| 7-919 | 4-F-Ph | 2-MeO-4-Pym | CONH-c-Pen |
| 7-920 | 4-F-Ph | 2-MeO-4-Pym | CONH-c-Hex |
| 7-921 | 4-F-Ph | 2-MeO-4-Pym | CONH-c-Hep |
| 7-922 | 4-F-Ph | 2-MeO-4-Pym | $CONHCH_2$-c-Pr |
| 7-923 | 4-F-Ph | 2-MeO-4-Pym | CONH-Allyl |
| 7-924 | 4-F-Ph | 2-MeO-4-Pym | CONH-Propargyl |
| 7-925 | 4-F-Ph | 2-MeO-4-Pym | CONHPh |
| 7-926 | 4-F-Ph | 2-MeO-4-Pym | CONH-3-Pyr |
| 7-927 | 4-F-Ph | 2-MeO-4-Pym | $CONHCH_2$-4-Pyr |
| 7-928 | 4-F-Ph | 2-MeO-4-Pym | $CONH-(CH_2)_2-OH$ |

| | | | |
|---|---|---|---|
| 7-929 | 4-F-Ph | 2-MeO-4-Pym | $CONHCH(CH_2OH)_2$ |
| 7-930 | 4-F-Ph | 2-MeO-4-Pym | $CONH-(CH_2)_2$-OAc |
| 7-931 | 4-F-Ph | 2-MeO-4-Pym | $CONHCH_2CN$ |
| 7-932 | 4-F-Ph | 2-MeO-4-Pym | $CONH-(CH_2)_2$-F |
| 7-933 | 4-F-Ph | 2-MeO-4-Pym | $CONH-(CH_2)_2$-OMe |
| 7-934 | 4-F-Ph | 2-MeO-4-Pym | $CONH-(CH_2)_2$-SMe |
| 7-935 | 4-F-Ph | 2-MeO-4-Pym | $CONH-(CH_2)_2-NH_2$ |
| 7-936 | 4-F-Ph | 2-MeO-4-Pym | $CONH-(CH_2)_2$-NHMe |
| 7-937 | 4-F-Ph | 2-MeO-4-Pym | $CONH-(CH_2)_2-N(Me)_2$ |
| 7-938 | 4-F-Ph | 2-MeO-4-Pym | $CONHCH_2COOH$ |
| 7-939 | 4-F-Ph | 2-MeO-4-Pym | $CONHCH_2COOEt$ |
| 7-940 | 4-F-Ph | 2-MeO-4-Pym | CONHCH(Me)COOEt |
| 7-941 | 4-F-Ph | 2-MeO-4-Pym | $CONHCH_2CONH_2$ |
| 7-942 | 4-F-Ph | 2-MeO-4-Pym | $SO_2NH_2$ |
| 7-943 | 4-F-Ph | 2-MeO-4-Pym | $SO_2NHMe$ |
| 7-944 | 4-F-Ph | 2-MeO-4-Pym | $SO_2NHEt$ |
| 7-945 | 4-F-Ph | 2-MeO-4-Pym | $SO_2NHPr$ |
| 7-946 | 4-F-Ph | 2-MeO-4-Pym | $SO_2NH$-i-Pr |
| 7-947 | 4-F-Ph | 2-MeO-4-Pym | $SO_2NHBu$ |
| 7-948 | 4-F-Ph | 2-MeO-4-Pym | $SO_2NHBn$ |
| 7-949 | 4-F-Ph | 2-MeO-4-Pym | $SO_2NMe_2$ |
| 7-950 | 4-F-Ph | 2-MeO-4-Pym | $SO_2NH$-c-Pr |
| 7-951 | 4-F-Ph | 2-MeO-4-Pym | $SO_2NH$-c-Bu |
| 7-952 | 4-F-Ph | 2-MeO-4-Pym | $SO_2NH$-c-Pen |
| 7-953 | 4-F-Ph | 2-MeO-4-Pym | $SO_2NH$-c-Hex |
| 7-954 | 4-F-Ph | 2-MeO-4-Pym | $SO_2NH$-c-Hep |
| 7-955 | 4-F-Ph | 2-MeO-4-Pym | $SO_2NHCH_2$-c-Pr |
| 7-956 | 4-F-Ph | 2-MeO-4-Pym | $SO_2NH$-Allyl |
| 7-957 | 4-F-Ph | 2-MeO-4-Pym | $SO_2NH$-Propargyl |
| 7-958 | 4-F-Ph | 2-MeO-4-Pym | $SO_2NHPh$ |
| 7-959 | 4-F-Ph | 2-MeO-4-Pym | $SO_2NH$-3-Pyr |
| 7-960 | 4-F-Ph | 2-MeO-4-Pym | $SO_2NHCH_2$-4-Pyr |
| 7-961 | 4-F-Ph | 2-MeO-4-Pym | $SO_2NH-(CH_2)_2$-OH |
| 7-962 | 4-F-Ph | 2-MeO-4-Pym | $SO_2NHCH(CH_2OH)_2$ |
| 7-963 | 4-F-Ph | 2-MeO-4-Pym | $SO_2NH-(CH_2)_2$-OAc |
| 7-964 | 4-F-Ph | 2-MeO-4-Pym | $SO_2NHCH_2CN$ |

| | | | |
|---|---|---|---|
| 7-965 | 4-F-Ph | 2-MeO-4-Pym | $SO_2NH-(CH_2)_2-F$ |
| 7-966 | 4-F-Ph | 2-MeO-4-Pym | $SO_2NH-(CH_2)_2-OMe$ |
| 7-967 | 4-F-Ph | 2-MeO-4-Pym | $SO_2NH-(CH_2)_2-SMe$ |
| 7-968 | 4-F-Ph | 2-MeO-4-Pym | $SO_2NH-(CH_2)_2-NH_2$ |
| 7-969 | 4-F-Ph | 2-MeO-4-Pym | $SO_2NH-(CH_2)_2-NHMe$ |
| 7-970 | 4-F-Ph | 2-MeO-4-Pym | $SO_2NH-(CH_2)_2-N(Me)_2$ |
| 7-971 | 4-F-Ph | 2-MeO-4-Pym | $SO_2NHCH_2COOH$ |
| 7-972 | 4-F-Ph | 2-MeO-4-Pym | $SO_2NHCH_2COOEt$ |
| 7-973 | 4-F-Ph | 2-MeO-4-Pym | $SO_2NHCH(Me)COOEt$ |
| 7-974 | 4-F-Ph | 2-MeO-4-Pym | $SO_2NHCH_2CONH_2$ |
| 7-975 | 4-F-Ph | 2-MeO-4-Pym | $SO_2NHOH$ |
| 7-976 | 4-F-Ph | 2-MeO-4-Pym | $SO_2NHOMe$ |
| 7-977 | 4-F-Ph | 2-MeO-4-Pym | $SO_2NHOEt$ |
| 7-978 | 4-F-Ph | 2-MeO-4-Pym | $SO_2NHOPr$ |
| 7-979 | 4-F-Ph | 2-MeO-4-Pym | $SO_2NHOAllyl$ |
| 7-980 | 4-F-Ph | 2-MeO-4-Pym | $SO_2NHOBn$ |
| 7-981 | 4-F-Ph | 2-MeO-4-Pym | $SO_2NHNH_2$ |
| 7-982 | 4-F-Ph | 2-MeO-4-Pym | $SO_2NHNHMe$ |
| 7-983 | 4-F-Ph | 2-MeO-4-Pym | $SO_2NHN(Me)_2$ |
| 7-984 | 4-F-Ph | $2-NH_2-4-Pym$ | $CONH_2$ |
| 7-985 | 4-F-Ph | $2-NH_2-4-Pym$ | CONHMe |
| 7-986 | 4-F-Ph | $2-NH_2-4-Pym$ | CONHEt |
| 7-987 | 4-F-Ph | $2-NH_2-4-Pym$ | CONHPr |
| 7-988 | 4-F-Ph | $2-NH_2-4-Pym$ | CONH-i-Pr |
| 7-989 | 4-F-Ph | $2-NH_2-4-Pym$ | CONHBu |
| 7-990 | 4-F-Ph | $2-NH_2-4-Pym$ | CONHBn |
| 7-991 | 4-F-Ph | $2-NH_2-4-Pym$ | $CONMe_2$ |
| 7-992 | 4-F-Ph | $2-NH_2-4-Pym$ | CONH-c-Pr |
| 7-993 | 4-F-Ph | $2-NH_2-4-Pym$ | CONH-c-Bu |
| 7-994 | 4-F-Ph | $2-NH_2-4-Pym$ | CONH-c-Pen |
| 7-995 | 4-F-Ph | $2-NH_2-4-Pym$ | CONH-c-Hex |
| 7-996 | 4-F-Ph | $2-NH_2-4-Pym$ | CONH-c-Hep |
| 7-997 | 4-F-Ph | $2-NH_2-4-Pym$ | $CONHCH_2-c-Pr$ |
| 7-998 | 4-F-Ph | $2-NH_2-4-Pym$ | CONH-Allyl |
| 7-999 | 4-F-Ph | $2-NH_2-4-Pym$ | CONH-Propargyl |
| 7-1000 | 4-F-Ph | $2-NH_2-4-Pym$ | CONHPh |

| | | | |
|---|---|---|---|
| 7-1001 | 4-F-Ph | 2-$NH_2$-4-Pym | CONH-3-Pyr |
| 7-1002 | 4-F-Ph | 2-$NH_2$-4-Pym | $CONHCH_2$-4-Pyr |
| 7-1003 | 4-F-Ph | 2-$NH_2$-4-Pym | CONH-$(CH_2)_2$-OH |
| 7-1004 | 4-F-Ph | 2-$NH_2$-4-Pym | $CONHCH(CH_2OH)_2$ |
| 7-1005 | 4-F-Ph | 2-$NH_2$-4-Pym | CONH-$(CH_2)_2$-OAc |
| 7-1006 | 4-F-Ph | 2-$NH_2$-4-Pym | $CONHCH_2CN$ |
| 7-1007 | 4-F-Ph | 2-$NH_2$-4-Pym | CONH-$(CH_2)_2$-F |
| 7-1008 | 4-F-Ph | 2-$NH_2$-4-Pym | CONH-$(CH_2)_2$-OMe |
| 7-1009 | 4-F-Ph | 2-$NH_2$-4-Pym | CONH-$(CH_2)_2$-SMe |
| 7-1010 | 4-F-Ph | 2-$NH_2$-4-Pym | CONH-$(CH_2)_2$-$NH_2$ |
| 7-1011 | 4-F-Ph | 2-$NH_2$-4-Pym | CONH-$(CH_2)_2$-NHMe |
| 7-1012 | 4-F-Ph | 2-$NH_2$-4-Pym | CONH-$(CH_2)_2$-$N(Me)_2$ |
| 7-1013 | 4-F-Ph | 2-$NH_2$-4-Pym | $CONHCH_2COOH$ |
| 7-1014 | 4-F-Ph | 2-$NH_2$-4-Pym | $CONHCH_2COOEt$ |
| 7-1015 | 4-F-Ph | 2-$NH_2$-4-Pym | CONHCH(Me)COOEt |
| 7-1016 | 4-F-Ph | 2-$NH_2$-4-Pym | $CONHCH_2CONH_2$ |
| 7-1017 | 4-F-Ph | 2-$NH_2$-4-Pym | $SO_2NH_2$ |
| 7-1018 | 4-F-Ph | 2-$NH_2$-4-Pym | $SO_2NHMe$ |
| 7-1019 | 4-F-Ph | 2-$NH_2$-4-Pym | $SO_2NHEt$ |
| 7-1020 | 4-F-Ph | 2-$NH_2$-4-Pym | $SO_2NHPr$ |
| 7-1021 | 4-F-Ph | 2-$NH_2$-4-Pym | $SO_2NH$-i-Pr |
| 7-1022 | 4-F-Ph | 2-$NH_2$-4-Pym | $SO_2NHBu$ |
| 7-1023 | 4-F-Ph | 2-$NH_2$-4-Pym | $SO_2NHBn$ |
| 7-1024 | 4-F-Ph | 2-$NH_2$-4-Pym | $SO_2NMe_2$ |
| 7-1025 | 4-F-Ph | 2-$NH_2$-4-Pym | $SO_2NH$-c-Pr |
| 7-1026 | 4-F-Ph | 2-$NH_2$-4-Pym | $SO_2NH$-c-Bu |
| 7-1027 | 4-F-Ph | 2-$NH_2$-4-Pym | $SO_2NH$-c-Pen |
| 7-1028 | 4-F-Ph | 2-$NH_2$-4-Pym | $SO_2NH$-c-Hex |
| 7-1029 | 4-F-Ph | 2-$NH_2$-4-Pym | $SO_2NH$-c-Hep |
| 7-1030 | 4-F-Ph | 2-$NH_2$-4-Pym | $SO_2NHCH_2$-c-Pr |
| 7-1031 | 4-F-Ph | 2-$NH_2$-4-Pym | $SO_2NH$-Allyl |
| 7-1032 | 4-F-Ph | 2-$NH_2$-4-Pym | $SO_2NH$-Propargyl |
| 7-1033 | 4-F-Ph | 2-$NH_2$-4-Pym | $SO_2NHPh$ |
| 7-1034 | 4-F-Ph | 2-$NH_2$-4-Pym | $SO_2NH$-3-Pyr |
| 7-1035 | 4-F-Ph | 2-$NH_2$-4-Pym | $SO_2NHCH_2$-4-Pyr |
| 7-1036 | 4-F-Ph | 2-$NH_2$-4-Pym | $SO_2NH$-$(CH_2)_2$-OH |

| | | | |
|---|---|---|---|
| 7-1037 | 4-F-Ph | 2-$NH_2$-4-Pym | $SO_2NHCH(CH_2OH)_2$ |
| 7-1038 | 4-F-Ph | 2-$NH_2$-4-Pym | $SO_2NH-(CH_2)_2$-OAc |
| 7-1039 | 4-F-Ph | 2-$NH_2$-4-Pym | $SO_2NHCH_2CN$ |
| 7-1040 | 4-F-Ph | 2-$NH_2$-4-Pym | $SO_2NH-(CH_2)_2$-F |
| 7-1041 | 4-F-Ph | 2-$NH_2$-4-Pym | $SO_2NH-(CH_2)_2$-OMe |
| 7-1042 | 4-F-Ph | 2-$NH_2$-4-Pym | $SO_2NH-(CH_2)_2$-SMe |
| 7-1043 | 4-F-Ph | 2-$NH_2$-4-Pym | $SO_2NH-(CH_2)_2-NH_2$ |
| 7-1044 | 4-F-Ph | 2-$NH_2$-4-Pym | $SO_2NH-(CH_2)_2$-NHMe |
| 7-1045 | 4-F-Ph | 2-$NH_2$-4-Pym | $SO_2NH-(CH_2)_2-N(Me)_2$ |
| 7-1046 | 4-F-Ph | 2-$NH_2$-4-Pym | $SO_2NHCH_2COOH$ |
| 7-1047 | 4-F-Ph | 2-$NH_2$-4-Pym | $SO_2NHCH_2COOEt$ |
| 7-1048 | 4-F-Ph | 2-$NH_2$-4-Pym | $SO_2NHCH(Me)COOEt$ |
| 7-1049 | 4-F-Ph | 2-$NH_2$-4-Pym | $SO_2NHCH_2CONH_2$ |
| 7-1050 | 4-F-Ph | 2-$NH_2$-4-Pym | $SO_2NHOH$ |
| 7-1051 | 4-F-Ph | 2-$NH_2$-4-Pym | $SO_2NHOMe$ |
| 7-1052 | 4-F-Ph | 2-$NH_2$-4-Pym | $SO_2NHOEt$ |
| 7-1053 | 4-F-Ph | 2-$NH_2$-4-Pym | $SO_2NHOPr$ |
| 7-1054 | 4-F-Ph | 2-$NH_2$-4-Pym | $SO_2NHOAllyl$ |
| 7-1055 | 4-F-Ph | 2-$NH_2$-4-Pym | $SO_2NHOBn$ |
| 7-1056 | 4-F-Ph | 2-$NH_2$-4-Pym | $SO_2NHNH_2$ |
| 7-1057 | 4-F-Ph | 2-$NH_2$-4-Pym | $SO_2NHNHMe$ |
| 7-1058 | 4-F-Ph | 2-$NH_2$-4-Pym | $SO_2NHN(Me)_2$ |
| 7-1059 | 4-F-Ph | 2-MeNH-4-Pym | $CONH_2$ |
| 7-1060 | 4-F-Ph | 2-MeNH-4-Pym | CONHMe |
| 7-1061 | 4-F-Ph | 2-MeNH-4-Pym | CONHEt |
| 7-1062 | 4-F-Ph | 2-MeNH-4-Pym | CONHPr |
| 7-1063 | 4-F-Ph | 2-MeNH-4-Pym | CONH-i-Pr |
| 7-1064 | 4-F-Ph | 2-MeNH-4-Pym | CONHBu |
| 7-1065 | 4-F-Ph | 2-MeNH-4-Pym | CONHBn |
| 7-1066 | 4-F-Ph | 2-MeNH-4-Pym | $CONMe_2$ |
| 7-1067 | 4-F-Ph | 2-MeNH-4-Pym | CONH-c-Pr |
| 7-1068 | 4-F-Ph | 2-MeNH-4-Pym | CONH-c-Bu |
| 7-1069 | 4-F-Ph | 2-MeNH-4-Pym | CONH-c-Pen |
| 7-1070 | 4-F-Ph | 2-MeNH-4-Pym | CONH-c-Hex |
| 7-1071 | 4-F-Ph | 2-MeNH-4-Pym | CONH-c-Hep |
| 7-1072 | 4-F-Ph | 2-MeNH-4-Pym | $CONHCH_2$-c-Pr |

| | | | |
|---|---|---|---|
| 7-1073 | 4-F-Ph | 2-MeNH-4-Pym | CONH-Allyl |
| 7-1074 | 4-F-Ph | 2-MeNH-4-Pym | CONH-Propargyl |
| 7-1075 | 4-F-Ph | 2-MeNH-4-Pym | CONHPh |
| 7-1076 | 4-F-Ph | 2-MeNH-4-Pym | CONH-3-Pyr |
| 7-1077 | 4-F-Ph | 2-MeNH-4-Pym | $CONHCH_2$-4-Pyr |
| 7-1078 | 4-F-Ph | 2-MeNH-4-Pym | CONH-$(CH_2)_2$-OH |
| 7-1079 | 4-F-Ph | 2-MeNH-4-Pym | $CONHCH(CH_2OH)_2$ |
| 7-1080 | 4-F-Ph | 2-MeNH-4-Pym | CONH-$(CH_2)_2$-OAc |
| 7-1081 | 4-F-Ph | 2-MeNH-4-Pym | $CONHCH_2CN$ |
| 7-1082 | 4-F-Ph | 2-MeNH-4-Pym | CONH-$(CH_2)_2$-F |
| 7-1083 | 4-F-Ph | 2-MeNH-4-Pym | CONH-$(CH_2)_2$-OMe |
| 7-1084 | 4-F-Ph | 2-MeNH-4-Pym | CONH-$(CH_2)_2$-SMe |
| 7-1085 | 4-F-Ph | 2-MeNH-4-Pym | CONH-$(CH_2)_2$-$NH_2$ |
| 7-1086 | 4-F-Ph | 2-MeNH-4-Pym | CONH-$(CH_2)_2$-NHMe |
| 7-1087 | 4-F-Ph | 2-MeNH-4-Pym | CONH-$(CH_2)_2$-$N(Me)_2$ |
| 7-1088 | 4-F-Ph | 2-MeNH-4-Pym | $CONHCH_2COOH$ |
| 7-1089 | 4-F-Ph | 2-MeNH-4-Pym | $CONHCH_2COOEt$ |
| 7-1090 | 4-F-Ph | 2-MeNH-4-Pym | CONHCH(Me)COOEt |
| 7-1091 | 4-F-Ph | 2-MeNH-4-Pym | $CONHCH_2CONH_2$ |
| 7-1092 | 4-F-Ph | 2-MeNH-4-Pym | $SO_2NH_2$ |
| 7-1093 | 4-F-Ph | 2-MeNH-4-Pym | $SO_2NHMe$ |
| 7-1094 | 4-F-Ph | 2-MeNH-4-Pym | $SO_2NHEt$ |
| 7-1095 | 4-F-Ph | 2-MeNH-4-Pym | $SO_2NHPr$ |
| 7-1096 | 4-F-Ph | 2-MeNH-4-Pym | $SO_2NH$-i-Pr |
| 7-1097 | 4-F-Ph | 2-MeNH-4-Pym | $SO_2NHBu$ |
| 7-1098 | 4-F-Ph | 2-MeNH-4-Pym | $SO_2NHBn$ |
| 7-1099 | 4-F-Ph | 2-MeNH-4-Pym | $SO_2NMe_2$ |
| 7-1100 | 4-F-Ph | 2-MeNH-4-Pym | $SO_2NH$-c-Pr |
| 7-1101 | 4-F-Ph | 2-MeNH-4-Pym | $SO_2NH$-c-Bu |
| 7-1102 | 4-F-Ph | 2-MeNH-4-Pym | $SO_2NH$-c-Pen |
| 7-1103 | 4-F-Ph | 2-MeNH-4-Pym | $SO_2NH$-c-Hex |
| 7-1104 | 4-F-Ph | 2-MeNH-4-Pym | $SO_2NH$-c-Hep |
| 7-1105 | 4-F-Ph | 2-MeNH-4-Pym | $SO_2NHCH_2$-c-Pr |
| 7-1106 | 4-F-Ph | 2-MeNH-4-Pym | $SO_2NH$-Allyl |
| 7-1107 | 4-F-Ph | 2-MeNH-4-Pym | $SO_2NH$-Propargyl |
| 7-1108 | 4-F-Ph | 2-MeNH-4-Pym | $SO_2NHPh$ |

| | | | |
|---|---|---|---|
| 7-1109 | 4-F-Ph | 2-MeNH-4-Pym | $SO_2NH$-3-Pyr |
| 7-1110 | 4-F-Ph | 2-MeNH-4-Pym | $SO_2NHCH_2$-4-Pyr |
| 7-1111 | 4-F-Ph | 2-MeNH-4-Pym | $SO_2NH-(CH_2)_2-OH$ |
| 7-1112 | 4-F-Ph | 2-MeNH-4-Pym | $SO_2NHCH(CH_2OH)_2$ |
| 7-1113 | 4-F-Ph | 2-MeNH-4-Pym | $SO_2NH-(CH_2)_2-OAc$ |
| 7-1114 | 4-F-Ph | 2-MeNH-4-Pym | $SO_2NHCH_2CN$ |
| 7-1115 | 4-F-Ph | 2-MeNH-4-Pym | $SO_2NH-(CH_2)_2-F$ |
| 7-1116 | 4-F-Ph | 2-MeNH-4-Pym | $SO_2NH-(CH_2)_2-OMe$ |
| 7-1117 | 4-F-Ph | 2-MeNH-4-Pym | $SO_2NH-(CH_2)_2-SMe$ |
| 7-1118 | 4-F-Ph | 2-MeNH-4-Pym | $SO_2NH-(CH_2)_2-NH_2$ |
| 7-1119 | 4-F-Ph | 2-MeNH-4-Pym | $SO_2NH-(CH_2)_2-NHMe$ |
| 7-1120 | 4-F-Ph | 2-MeNH-4-Pym | $SO_2NH-(CH_2)_2-N(Me)_2$ |
| 7-1121 | 4-F-Ph | 2-MeNH-4-Pym | $SO_2NHCH_2COOH$ |
| 7-1122 | 4-F-Ph | 2-MeNH-4-Pym | $SO_2NHCH_2COOEt$ |
| 7-1123 | 4-F-Ph | 2-MeNH-4-Pym | $SO_2NHCH(Me)COOEt$ |
| 7-1124 | 4-F-Ph | 2-MeNH-4-Pym | $SO_2NHCH_2CONH_2$ |
| 7-1125 | 4-F-Ph | 2-MeNH-4-Pym | $SO_2NHOH$ |
| 7-1126 | 4-F-Ph | 2-MeNH-4-Pym | $SO_2NHOMe$ |
| 7-1127 | 4-F-Ph | 2-MeNH-4-Pym | $SO_2NHOEt$ |
| 7-1128 | 4-F-Ph | 2-MeNH-4-Pym | $SO_2NHOPr$ |
| 7-1129 | 4-F-Ph | 2-MeNH-4-Pym | $SO_2NHOAllyl$ |
| 7-1130 | 4-F-Ph | 2-MeNH-4-Pym | $SO_2NHOBn$ |
| 7-1131 | 4-F-Ph | 2-MeNH-4-Pym | $SO_2NHNH_2$ |
| 7-1132 | 4-F-Ph | 2-MeNH-4-Pym | $SO_2NHNHMe$ |
| 7-1133 | 4-F-Ph | 2-MeNH-4-Pym | $SO_2NHN(Me)_2$ |
| 7-1134 | 4-F-Ph | 2-BnNH-4-Pym | $CONH_2$ |
| 7-1135 | 4-F-Ph | 2-BnNH-4-Pym | CONHMe |
| 7-1136 | 4-F-Ph | 2-BnNH-4-Pym | CONHEt |
| 7-1137 | 4-F-Ph | 2-BnNH-4-Pym | CONHPr |
| 7-1138 | 4-F-Ph | 2-BnNH-4-Pym | CONH-i-Pr |
| 7-1139 | 4-F-Ph | 2-BnNH-4-Pym | CONHBu |
| 7-1140 | 4-F-Ph | 2-BnNH-4-Pym | CONHBn |
| 7-1141 | 4-F-Ph | 2-BnNH-4-Pym | $CONMe_2$ |
| 7-1142 | 4-F-Ph | 2-BnNH-4-Pym | CONH-c-Pr |
| 7-1143 | 4-F-Ph | 2-BnNH-4-Pym | CONH-c-Bu |
| 7-1144 | 4-F-Ph | 2-BnNH-4-Pym | CONH-c-Pen |

| | | | |
|---|---|---|---|
| 7-1145 | 4-F-Ph | 2-BnNH-4-Pym | CONH-c-Hex |
| 7-1146 | 4-F-Ph | 2-BnNH-4-Pym | CONH-c-Hep |
| 7-1147 | 4-F-Ph | 2-BnNH-4-Pym | $CONHCH_2$-c-Pr |
| 7-1148 | 4-F-Ph | 2-BnNH-4-Pym | CONH-Allyl |
| 7-1149 | 4-F-Ph | 2-BnNH-4-Pym | CONH-Propargyl |
| 7-1150 | 4-F-Ph | 2-BnNH-4-Pym | CONHPh |
| 7-1151 | 4-F-Ph | 2-BnNH-4-Pym | CONH-3-Pyr |
| 7-1152 | 4-F-Ph | 2-BnNH-4-Pym | $CONHCH_2$-4-Pyr |
| 7-1153 | 4-F-Ph | 2-BnNH-4-Pym | CONH-$(CH_2)_2$-OH |
| 7-1154 | 4-F-Ph | 2-BnNH-4-Pym | $CONHCH(CH_2OH)_2$ |
| 7-1155 | 4-F-Ph | 2-BnNH-4-Pym | CONH-$(CH_2)_2$-OAc |
| 7-1156 | 4-F-Ph | 2-BnNH-4-Pym | $CONHCH_2CN$ |
| 7-1157 | 4-F-Ph | 2-BnNH-4-Pym | CONH-$(CH_2)_2$-F |
| 7-1158 | 4-F-Ph | 2-BnNH-4-Pym | CONH-$(CH_2)_2$-OMe |
| 7-1159 | 4-F-Ph | 2-BnNH-4-Pym | CONH-$(CH_2)_2$-SMe |
| 7-1160 | 4-F-Ph | 2-BnNH-4-Pym | CONH-$(CH_2)_2$-$NH_2$ |
| 7-1161 | 4-F-Ph | 2-BnNH-4-Pym | CONH-$(CH_2)_2$-NHMe |
| 7-1162 | 4-F-Ph | 2-BnNH-4-Pym | CONH-$(CH_2)_2$-$N(Me)_2$ |
| 7-1163 | 4-F-Ph | 2-BnNH-4-Pym | $CONHCH_2COOH$ |
| 7-1164 | 4-F-Ph | 2-BnNH-4-Pym | $CONHCH_2COOEt$ |
| 7-1165 | 4-F-Ph | 2-BnNH-4-Pym | CONHCH(Me)COOEt |
| 7-1166 | 4-F-Ph | 2-BnNH-4-Pym | $CONHCH_2CONH_2$ |
| 7-1167 | 4-F-Ph | 2-BnNH-4-Pym | $SO_2NH_2$ |
| 7-1168 | 4-F-Ph | 2-BnNH-4-Pym | $SO_2NHMe$ |
| 7-1169 | 4-F-Ph | 2-BnNH-4-Pym | $SO_2NHEt$ |
| 7-1170 | 4-F-Ph | 2-BnNH-4-Pym | $SO_2NHPr$ |
| 7-1171 | 4-F-Ph | 2-BnNH-4-Pym | $SO_2NH$-i-Pr |
| 7-1172 | 4-F-Ph | 2-BnNH-4-Pym | $SO_2NHBu$ |
| 7-1173 | 4-F-Ph | 2-BnNH-4-Pym | $SO_2NHBn$ |
| 7-1174 | 4-F-Ph | 2-BnNH-4-Pym | $SO_2NMe_2$ |
| 7-1175 | 4-F-Ph | 2-BnNH-4-Pym | $SO_2NH$-c-Pr |
| 7-1176 | 4-F-Ph | 2-BnNH-4-Pym | $SO_2NH$-c-Bu |
| 7-1177 | 4-F-Ph | 2-BnNH-4-Pym | $SO_2NH$-c-Pen |
| 7-1178 | 4-F-Ph | 2-BnNH-4-Pym | $SO_2NH$-c-Hex |
| 7-1179 | 4-F-Ph | 2-BnNH-4-Pym | $SO_2NH$-c-Hep |
| 7-1180 | 4-F-Ph | 2-BnNH-4-Pym | $SO_2NHCH_2$-c-Pr |

| | | | |
|---|---|---|---|
| 7-1181 | 4-F-Ph | 2-BnNH-4-Pym | $SO_2$NH-Allyl |
| 7-1182 | 4-F-Ph | 2-BnNH-4-Pym | $SO_2$NH-Propargyl |
| 7-1183 | 4-F-Ph | 2-BnNH-4-Pym | $SO_2$NHPh |
| 7-1184 | 4-F-Ph | 2-BnNH-4-Pym | $SO_2$NH-3-Pyr |
| 7-1185 | 4-F-Ph | 2-BnNH-4-Pym | $SO_2NHCH_2$-4-Pyr |
| 7-1186 | 4-F-Ph | 2-BnNH-4-Pym | $SO_2NH-(CH_2)_2-OH$ |
| 7-1187 | 4-F-Ph | 2-BnNH-4-Pym | $SO_2NHCH(CH_2OH)_2$ |
| 7-1188 | 4-F-Ph | 2-BnNH-4-Pym | $SO_2NH-(CH_2)_2-OAc$ |
| 7-1189 | 4-F-Ph | 2-BnNH-4-Pym | $SO_2NHCH_2CN$ |
| 7-1190 | 4-F-Ph | 2-BnNH-4-Pym | $SO_2NH-(CH_2)_2-F$ |
| 7-1191 | 4-F-Ph | 2-BnNH-4-Pym | $SO_2NH-(CH_2)_2-OMe$ |
| 7-1192 | 4-F-Ph | 2-BnNH-4-Pym | $SO_2NH-(CH_2)_2-SMe$ |
| 7-1193 | 4-F-Ph | 2-BnNH-4-Pym | $SO_2NH-(CH_2)_2-NH_2$ |
| 7-1194 | 4-F-Ph | 2-BnNH-4-Pym | $SO_2NH-(CH_2)_2-NHMe$ |
| 7-1195 | 4-F-Ph | 2-BnNH-4-Pym | $SO_2NH-(CH_2)_2-N(Me)_2$ |
| 7-1196 | 4-F-Ph | 2-BnNH-4-Pym | $SO_2NHCH_2COOH$ |
| 7-1197 | 4-F-Ph | 2-BnNH-4-Pym | $SO_2NHCH_2COOEt$ |
| 7-1198 | 4-F-Ph | 2-BnNH-4-Pym | $SO_2NHCH(Me)COOEt$ |
| 7-1199 | 4-F-Ph | 2-BnNH-4-Pym | $SO_2NHCH_2CONH_2$ |
| 7-1200 | 4-F-Ph | 2-BnNH-4-Pym | $SO_2NHOH$ |
| 7-1201 | 4-F-Ph | 2-BnNH-4-Pym | $SO_2NHOMe$ |
| 7-1202 | 4-F-Ph | 2-BnNH-4-Pym | $SO_2NHOEt$ |
| 7-1203 | 4-F-Ph | 2-BnNH-4-Pym | $SO_2NHOPr$ |
| 7-1204 | 4-F-Ph | 2-BnNH-4-Pym | $SO_2$NHOAllyl |
| 7-1205 | 4-F-Ph | 2-BnNH-4-Pym | $SO_2NHOBn$ |
| 7-1206 | 4-F-Ph | 2-BnNH-4-Pym | $SO_2NHNH_2$ |
| 7-1207 | 4-F-Ph | 2-BnNH-4-Pym | $SO_2NHNHMe$ |
| 7-1208 | 4-F-Ph | 2-BnNH-4-Pym | $SO_2NHN(Me)_2$ |
| 7-1209 | 4-F-Ph | (α-Me-BnNH)-4-Pym | $CONH_2$ |
| 7-1210 | 4-F-Ph | (α-Me-BnNH)-4-Pym | CONHMe |
| 7-1211 | 4-F-Ph | (α-Me-BnNH)-4-Pym | CONHEt |
| 7-1212 | 4-F-Ph | (α-Me-BnNH)-4-Pym | CONHPr |
| 7-1213 | 4-F-Ph | (α-Me-BnNH)-4-Pym | CONH-i-Pr |
| 7-1214 | 4-F-Ph | (α-Me-BnNH)-4-Pym | CONHBu |
| 7-1215 | 4-F-Ph | (α-Me-BnNH)-4-Pym | CONHBn |

| | | | |
|---|---|---|---|
| 7-1216 | 4-F-Ph | (α-Me-BnNH)-4-Pym | $CONMe_2$ |
| 7-1217 | 4-F-Ph | (α-Me-BnNH)-4-Pym | CONH-c-Pr |
| 7-1218 | 4-F-Ph | (α-Me-BnNH)-4-Pym | CONH-c-Bu |
| 7-1219 | 4-F-Ph | (α-Me-BnNH)-4-Pym | CONH-c-Pen |
| 7-1220 | 4-F-Ph | (α-Me-BnNH)-4-Pym | CONH-c-Hex |
| 7-1221 | 4-F-Ph | (α-Me-BnNH)-4-Pym | CONH-c-Hep |
| 7-1222 | 4-F-Ph | (α-Me-BnNH)-4-Pym | $CONHCH_2$-c-Pr |
| 7-1223 | 4-F-Ph | (α-Me-BnNH)-4-Pym | CONH-Allyl |
| 7-1224 | 4-F-Ph | (α-Me-BnNH)-4-Pym | CONH-Propargyl |
| 7-1225 | 4-F-Ph | (α-Me-BnNH)-4-Pym | CONHPh |
| 7-1226 | 4-F-Ph | (α-Me-BnNH)-4-Pym | CONH-3-Pyr |
| 7-1227 | 4-F-Ph | (α-Me-BnNH)-4-Pym | $CONHCH_2$-4-Pyr |
| 7-1228 | 4-F-Ph | (α-Me-BnNH)-4-Pym | CONH-$(CH_2)_2$-OH |
| 7-1229 | 4-F-Ph | (α-Me-BnNH)-4-Pym | $CONHCH(CH_2OH)_2$ |
| 7-1230 | 4-F-Ph | (α-Me-BnNH)-4-Pym | CONH-$(CH_2)_2$-OAc |
| 7-1231 | 4-F-Ph | (α-Me-BnNH)-4-Pym | $CONHCH_2CN$ |
| 7-1232 | 4-F-Ph | (α-Me-BnNH)-4-Pym | CONH-$(CH_2)_2$-F |
| 7-1233 | 4-F-Ph | (α-Me-BnNH)-4-Pym | CONH-$(CH_2)_2$-OMe |
| 7-1234 | 4-F-Ph | (α-Me-BnNH)-4-Pym | CONH-$(CH_2)_2$-SMe |
| 7-1235 | 4-F-Ph | (α-Me-BnNH)-4-Pym | CONH-$(CH_2)_2$-$NH_2$ |
| 7-1236 | 4-F-Ph | (α-Me-BnNH)-4-Pym | CONH-$(CH_2)_2$-NHMe |
| 7-1237 | 4-F-Ph | (α-Me-BnNH)-4-Pym | CONH-$(CH_2)_2$-$N(Me)_2$ |
| 7-1238 | 4-F-Ph | (α-Me-BnNH)-4-Pym | $CONHCH_2COOH$ |
| 7-1239 | 4-F-Ph | (α-Me-BnNH)-4-Pym | $CONHCH_2COOEt$ |
| 7-1240 | 4-F-Ph | (α-Me-BnNH)-4-Pym | CONHCH(Me)COOEt |
| 7-1241 | 4-F-Ph | (α-Me-BnNH)-4-Pym | $CONHCH_2CONH_2$ |
| 7-1242 | 4-F-Ph | (α-Me-BnNH)-4-Pym | $SO_2NH_2$ |
| 7-1243 | 4-F-Ph | (α-Me-BnNH)-4-Pym | $SO_2NHMe$ |
| 7-1244 | 4-F-Ph | (α-Me-BnNH)-4-Pym | $SO_2NHEt$ |
| 7-1245 | 4-F-Ph | (α-Me-BnNH)-4-Pym | $SO_2NHPr$ |
| 7-1246 | 4-F-Ph | (α-Me-BnNH)-4-Pym | $SO_2NH$-i-Pr |
| 7-1247 | 4-F-Ph | (α-Me-BnNH)-4-Pym | $SO_2NHBu$ |

| | | | |
|---|---|---|---|
| 7-1248 | 4-F-Ph | (α-Me-BnNH)-4-Pym | $SO_2NHBn$ |
| 7-1249 | 4-F-Ph | (α-Me-BnNH)-4-Pym | $SO_2NMe_2$ |
| 7-1250 | 4-F-Ph | (α-Me-BnNH)-4-Pym | $SO_2NH$-c-Pr |
| 7-1251 | 4-F-Ph | (α-Me-BnNH)-4-Pym | $SO_2NH$-c-Bu |
| 7-1252 | 4-F-Ph | (α-Me-BnNH)-4-Pym | $SO_2NH$-c-Pen |
| 7-1253 | 4-F-Ph | (α-Me-BnNH)-4-Pym | $SO_2NH$-c-Hex |
| 7-1254 | 4-F-Ph | (α-Me-BnNH)-4-Pym | $SO_2NH$-c-Hep |
| 7-1255 | 4-F-Ph | (α-Me-BnNH)-4-Pym | $SO_2NHCH_2$-c-Pr |
| 7-1256 | 4-F-Ph | (α-Me-BnNH)-4-Pym | $SO_2NH$-Allyl |
| 7-1257 | 4-F-Ph | (α-Me-BnNH)-4-Pym | $SO_2NH$-Propargyl |
| 7-1258 | 4-F-Ph | (α-Me-BnNH)-4-Pym | $SO_2NHPh$ |
| 7-1259 | 4-F-Ph | (α-Me-BnNH)-4-Pym | $SO_2NH$-3-Pyr |
| 7-1260 | 4-F-Ph | (α-Me-BnNH)-4-Pym | $SO_2NHCH_2$-4-Pyr |
| 7-1261 | 4-F-Ph | (α-Me-BnNH)-4-Pym | $SO_2NH-(CH_2)_2-OH$ |
| 7-1262 | 4-F-Ph | (α-Me-BnNH)-4-Pym | $SO_2NHCH(CH_2OH)_2$ |
| 7-1263 | 4-F-Ph | (α-Me-BnNH)-4-Pym | $SO_2NH-(CH_2)_2-OAc$ |
| 7-1264 | 4-F-Ph | (α-Me-BnNH)-4-Pym | $SO_2NHCH_2CN$ |
| 7-1265 | 4-F-Ph | (α-Me-BnNH)-4-Pym | $SO_2NH-(CH_2)_2-F$ |
| 7-1266 | 4-F-Ph | (α-Me-BnNH)-4-Pym | $SO_2NH-(CH_2)_2-OMe$ |
| 7-1267 | 4-F-Ph | (α-Me-BnNH)-4-Pym | $SO_2NH-(CH_2)_2-SMe$ |
| 7-1268 | 4-F-Ph | (α-Me-BnNH)-4-Pym | $SO_2NH-(CH_2)_2-NH_2$ |
| 7-1269 | 4-F-Ph | (α-Me-BnNH)-4-Pym | $SO_2NH-(CH_2)_2-NHMe$ |
| 7-1270 | 4-F-Ph | (α-Me-BnNH)-4-Pym | $SO_2NH-(CH_2)_2-N(Me)_2$ |
| 7-1271 | 4-F-Ph | (α-Me-BnNH)-4-Pym | $SO_2NHCH_2COOH$ |
| 7-1272 | 4-F-Ph | (α-Me-BnNH)-4-Pym | $SO_2NHCH_2COOEt$ |
| 7-1273 | 4-F-Ph | (α-Me-BnNH)-4-Pym | $SO_2NHCH(Me)COOEt$ |
| 7-1274 | 4-F-Ph | (α-Me-BnNH)-4-Pym | $SO_2NHCH_2CONH_2$ |
| 7-1275 | 4-F-Ph | (α-Me-BnNH)-4-Pym | $SO_2NHOH$ |
| 7-1276 | 4-F-Ph | (α-Me-BnNH)-4-Pym | $SO_2NHOMe$ |
| 7-1277 | 4-F-Ph | (α-Me-BnNH)-4-Pym | $SO_2NHOEt$ |
| 7-1278 | 4-F-Ph | (α-Me-BnNH)-4-Pym | $SO_2NHOPr$ |
| 7-1279 | 4-F-Ph | (α-Me-BnNH)-4-Pym | $SO_2NHOAllyl$ |

| | | | |
|---|---|---|---|
| 7-1280 | 4-F-Ph | (α-Me-BnNH)-4-Pym | $SO_2NHOBn$ |
| 7-1281 | 4-F-Ph | (α-Me-BnNH)-4-Pym | $SO_2NHNH_2$ |
| 7-1282 | 4-F-Ph | (α-Me-BnNH)-4-Pym | $SO_2NHNHMe$ |
| 7-1283 | 4-F-Ph | (α-Me-BnNH)-4-Pym | $SO_2NHN(Me)_2$ |
| 7-1284 | 4-F-Ph | 4-Pyr | $SO_2NHMe$ |
| 7-1285 | 4-F-Ph | 4-Pyr | SOMe |
| 7-1286 | 4-F-Ph | 4-Pyr | $SO_2Me$ |
| 7-1287 | 3-Cl-4-F-Ph | 4-Pyr | $SO_2NHMe$ |
| 7-1288 | 3-Cl-4-F-Ph | 4-Pyr | SOMe |
| 7-1289 | 3-Cl-4-F-Ph | 4-Pyr | $SO_2Me$ |

Table 8

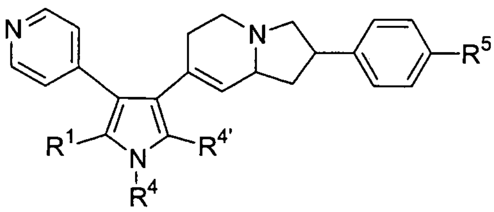

(I-8)

| Compound No. | $R^1$ | $R^4$ | $R^{4'}$ | $R^5$ |
|---|---|---|---|---|
| 8-1 | 4-F-Ph | Me | H | $CONH_2$ |
| 8-2 | 4-F-Ph | Me | H | CONHMe |
| 8-3 | 4-F-Ph | Me | H | CONHEt |
| 8-4 | 4-F-Ph | Me | H | CONHPr |
| 8-5 | 4-F-Ph | Me | H | CONH-i-Pr |
| 8-6 | 4-F-Ph | Me | H | CONHBu |
| 8-7 | 4-F-Ph | Me | H | $SO_2NH_2$ |
| 8-8 | 4-F-Ph | Me | H | $SO_2NHMe$ |
| 8-9 | 4-F-Ph | Me | H | $SO_2NHEt$ |
| 8-10 | 4-F-Ph | Me | H | $SO_2NHPr$ |
| 8-11 | 4-F-Ph | Me | H | $SO_2NH$-i-Pr |
| 8-12 | 4-F-Ph | Me | H | $SO_2NHBu$ |
| 8-13 | 4-F-Ph | Me | H | SOMe |
| 8-14 | 4-F-Ph | Me | H | SOEt |
| 8-15 | 4-F-Ph | Me | H | SOPr |
| 8-16 | 4-F-Ph | Me | H | $SO_2Me$ |
| 8-17 | 4-F-Ph | Me | H | $SO_2Et$ |

| | | | | |
|---|---|---|---|---|
| 8-18 | 4-F-Ph | Me | H | $SO_2Pr$ |
| 8-19 | 4-F-Ph | Me | H | COMe |
| 8-20 | 4-F-Ph | Me | H | COEt |
| 8-21 | 4-F-Ph | Me | H | COPr |
| 8-22 | 4-F-Ph | H | Me | $CONH_2$ |
| 8-23 | 4-F-Ph | H | Me | CONHMe |
| 8-24 | 4-F-Ph | H | Me | CONHEt |
| 8-25 | 4-F-Ph | H | Me | CONHPr |
| 8-26 | 4-F-Ph | H | Me | CONH-i-Pr |
| 8-27 | 4-F-Ph | H | Me | CONHBu |
| 8-28 | 4-F-Ph | H | Me | $SO_2NH_2$ |
| 8-29 | 4-F-Ph | H | Me | $SO_2NHMe$ |
| 8-30 | 4-F-Ph | H | Me | $SO_2NHEt$ |
| 8-31 | 4-F-Ph | H | Me | $SO_2NHPr$ |
| 8-32 | 4-F-Ph | H | Me | $SO_2NH$-i-Pr |
| 8-33 | 4-F-Ph | H | Me | $SO_2NHBu$ |
| 8-34 | 4-F-Ph | H | Me | SOMe |
| 8-35 | 4-F-Ph | H | Me | SOEt |
| 8-36 | 4-F-Ph | H | Me | SOPr |
| 8-37 | 4-F-Ph | H | Me | $SO_2Me$ |
| 8-38 | 4-F-Ph | H | Me | $SO_2Et$ |
| 8-39 | 4-F-Ph | H | Me | $SO_2Pr$ |
| 8-40 | 4-F-Ph | H | Me | COMe |
| 8-41 | 4-F-Ph | H | Me | COEt |
| 8-42 | 4-F-Ph | H | Me | COPr |
| 8-43 | 4-F-Ph | Me | Me | $CONH_2$ |
| 8-44 | 4-F-Ph | Me | Me | CONHMe |
| 8-45 | 4-F-Ph | Me | Me | CONHEt |
| 8-46 | 4-F-Ph | Me | Me | CONHPr |
| 8-47 | 4-F-Ph | Me | Me | CONH-i-Pr |
| 8-48 | 4-F-Ph | Me | Me | CONHBu |
| 8-49 | 4-F-Ph | Me | Me | $SO_2NH_2$ |
| 8-50 | 4-F-Ph | Me | Me | $SO_2NHMe$ |
| 8-51 | 4-F-Ph | Me | Me | $SO_2NHEt$ |
| 8-52 | 4-F-Ph | Me | Me | $SO_2NHPr$ |
| 8-53 | 4-F-Ph | Me | Me | $SO_2NH$-i-Pr |

| | | | | |
|---|---|---|---|---|
| 8-54 | 4-F-Ph | Me | Me | $SO_2NHBu$ |
| 8-55 | 4-F-Ph | Me | Me | SOMe |
| 8-56 | 4-F-Ph | Me | Me | SOEt |
| 8-57 | 4-F-Ph | Me | Me | SOPr |
| 8-58 | 4-F-Ph | Me | Me | $SO_2Me$ |
| 8-59 | 4-F-Ph | Me | Me | $SO_2Et$ |
| 8-60 | 4-F-Ph | Me | Me | $SO_2Pr$ |
| 8-61 | 4-F-Ph | Me | Me | COMe |
| 8-62 | 4-F-Ph | Me | Me | COEt |
| 8-63 | 4-F-Ph | Me | Me | COPr |

Table 9

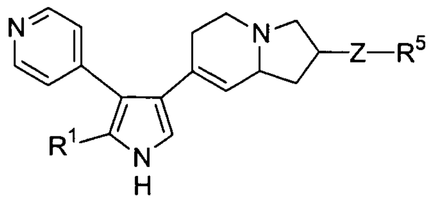

(I-9)

| Compound No. | $R^1$ | Z | $R^5$ |
|---|---|---|---|
| 9-1 | 4-F-Ph | Ring 1 | $CONH_2$ |
| 9-2 | 4-F-Ph | Ring 1 | CONHMe |
| 9-3 | 4-F-Ph | Ring 1 | CONHEt |
| 9-4 | 4-F-Ph | Ring 1 | CONHPr |
| 9-5 | 4-F-Ph | Ring 1 | CONH-i-Pr |
| 9-6 | 4-F-Ph | Ring 1 | CONHBu |
| 9-7 | 4-F-Ph | Ring 1 | $SO_2NH_2$ |
| 9-8 | 4-F-Ph | Ring 1 | $SO_2NHMe$ |
| 9-9 | 4-F-Ph | Ring 1 | $SO_2NHEt$ |
| 9-10 | 4-F-Ph | Ring 1 | $SO_2NHPr$ |
| 9-11 | 4-F-Ph | Ring 1 | $SO_2NH$-i-Pr |
| 9-12 | 4-F-Ph | Ring 1 | $SO_2NHBu$ |
| 9-13 | 4-F-Ph | Ring 1 | SOMe |
| 9-14 | 4-F-Ph | Ring 1 | SOEt |
| 9-15 | 4-F-Ph | Ring 1 | SOPr |
| 9-16 | 4-F-Ph | Ring 1 | $SO_2Me$ |
| 9-17 | 4-F-Ph | Ring 1 | $SO_2Et$ |

| | | | |
|---|---|---|---|
| 9-18 | 4-F-Ph | Ring 1 | $SO_2Pr$ |
| 9-19 | 4-F-Ph | Ring 1 | COMe |
| 9-20 | 4-F-Ph | Ring 1 | COEt |
| 9-21 | 4-F-Ph | Ring 1 | COPr |
| 9-22 | 3-Cl-4-F-Ph | Ring 1 | $CONH_2$ |
| 9-23 | 3-Cl-4-F-Ph | Ring 1 | CONHMe |
| 9-24 | 3-Cl-4-F-Ph | Ring 1 | CONHEt |
| 9-25 | 3-Cl-4-F-Ph | Ring 1 | CONHPr |
| 9-26 | 3-Cl-4-F-Ph | Ring 1 | CONH-i-Pr |
| 9-27 | 3-Cl-4-F-Ph | Ring 1 | CONHBu |
| 9-28 | 3-Cl-4-F-Ph | Ring 1 | $SO_2NH_2$ |
| 9-29 | 3-Cl-4-F-Ph | Ring 1 | $SO_2NHMe$ |
| 9-30 | 3-Cl-4-F-Ph | Ring 1 | $SO_2NHEt$ |
| 9-31 | 3-Cl-4-F-Ph | Ring 1 | $SO_2NHPr$ |
| 9-32 | 3-Cl-4-F-Ph | Ring 1 | $SO_2NH$-i-Pr |
| 9-33 | 3-Cl-4-F-Ph | Ring 1 | $SO_2NHBu$ |
| 9-34 | 3-Cl-4-F-Ph | Ring 1 | SOMe |
| 9-35 | 3-Cl-4-F-Ph | Ring 1 | SOEt |
| 9-36 | 3-Cl-4-F-Ph | Ring 1 | SOPr |
| 9-37 | 3-Cl-4-F-Ph | Ring 1 | $SO_2Me$ |
| 9-38 | 3-Cl-4-F-Ph | Ring 1 | $SO_2Et$ |
| 9-39 | 3-Cl-4-F-Ph | Ring 1 | $SO_2Pr$ |
| 9-40 | 3-Cl-4-F-Ph | Ring 1 | COMe |
| 9-41 | 3-Cl-4-F-Ph | Ring 1 | COEt |
| 9-42 | 3-Cl-4-F-Ph | Ring 1 | COPr |
| 9-43 | 4-F-Ph | Ring 2 | $CONH_2$ |
| 9-44 | 4-F-Ph | Ring 2 | CONHMe |
| 9-45 | 4-F-Ph | Ring 2 | CONHEt |
| 9-46 | 4-F-Ph | Ring 2 | CONHPr |
| 9-47 | 4-F-Ph | Ring 2 | CONH-i-Pr |
| 9-48 | 4-F-Ph | Ring 2 | CONHBu |
| 9-49 | 4-F-Ph | Ring 2 | $SO_2NH_2$ |
| 9-50 | 4-F-Ph | Ring 2 | $SO_2NHMe$ |
| 9-51 | 4-F-Ph | Ring 2 | $SO_2NHEt$ |
| 9-52 | 4-F-Ph | Ring 2 | $SO_2NHPr$ |
| 9-53 | 4-F-Ph | Ring 2 | $SO_2NH$-i-Pr |

| | | | |
|---|---|---|---|
| 9-54 | 4-F-Ph | Ring 2 | $SO_2NHBu$ |
| 9-55 | 4-F-Ph | Ring 2 | SOMe |
| 9-56 | 4-F-Ph | Ring 2 | SOEt |
| 9-57 | 4-F-Ph | Ring 2 | SOPr |
| 9-58 | 4-F-Ph | Ring 2 | $SO_2Me$ |
| 9-59 | 4-F-Ph | Ring 2 | $SO_2Et$ |
| 9-60 | 4-F-Ph | Ring 2 | $SO_2Pr$ |
| 9-61 | 4-F-Ph | Ring 2 | COMe |
| 9-62 | 4-F-Ph | Ring 2 | COEt |
| 9-63 | 4-F-Ph | Ring 2 | COPr |
| 9-64 | 3-Cl-4-F-Ph | Ring 2 | $CONH_2$ |
| 9-65 | 3-Cl-4-F-Ph | Ring 2 | CONHMe |
| 9-66 | 3-Cl-4-F-Ph | Ring 2 | CONHEt |
| 9-67 | 3-Cl-4-F-Ph | Ring 2 | CONHPr |
| 9-68 | 3-Cl-4-F-Ph | Ring 2 | CONH-i-Pr |
| 9-69 | 3-Cl-4-F-Ph | Ring 2 | CONHBu |
| 9-70 | 3-Cl-4-F-Ph | Ring 2 | $SO_2NH_2$ |
| 9-71 | 3-Cl-4-F-Ph | Ring 2 | $SO_2NHMe$ |
| 9-72 | 3-Cl-4-F-Ph | Ring 2 | $SO_2NHEt$ |
| 9-73 | 3-Cl-4-F-Ph | Ring 2 | $SO_2NHPr$ |
| 9-74 | 3-Cl-4-F-Ph | Ring 2 | $SO_2NH$-i-Pr |
| 9-75 | 3-Cl-4-F-Ph | Ring 2 | $SO_2NHBu$ |
| 9-76 | 3-Cl-4-F-Ph | Ring 2 | SOMe |
| 9-77 | 3-Cl-4-F-Ph | Ring 2 | SOEt |
| 9-78 | 3-Cl-4-F-Ph | Ring 2 | SOPr |
| 9-79 | 3-Cl-4-F-Ph | Ring 2 | $SO_2Me$ |
| 9-80 | 3-Cl-4-F-Ph | Ring 2 | $SO_2Et$ |
| 9-81 | 3-Cl-4-F-Ph | Ring 2 | $SO_2Pr$ |
| 9-82 | 3-Cl-4-F-Ph | Ring 2 | COMe |
| 9-83 | 3-Cl-4-F-Ph | Ring 2 | COEt |
| 9-84 | 3-Cl-4-F-Ph | Ring 2 | COPr |
| 9-85 | 4-F-Ph | Ring 3 | $CONH_2$ |
| 9-86 | 4-F-Ph | Ring 3 | CONHMe |
| 9-87 | 4-F-Ph | Ring 3 | CONHEt |
| 9-88 | 4-F-Ph | Ring 3 | CONHPr |
| 9-89 | 4-F-Ph | Ring 3 | CONH-i-Pr |

| | | | |
|---|---|---|---|
| 9-90 | 4-F-Ph | Ring 3 | CONHBu |
| 9-91 | 4-F-Ph | Ring 3 | $SO_2NH_2$ |
| 9-92 | 4-F-Ph | Ring 3 | $SO_2NHMe$ |
| 9-93 | 4-F-Ph | Ring 3 | $SO_2NHEt$ |
| 9-94 | 4-F-Ph | Ring 3 | $SO_2NHPr$ |
| 9-95 | 4-F-Ph | Ring 3 | $SO_2NH$-i-Pr |
| 9-96 | 4-F-Ph | Ring 3 | $SO_2NHBu$ |
| 9-97 | 4-F-Ph | Ring 3 | SOMe |
| 9-98 | 4-F-Ph | Ring 3 | SOEt |
| 9-99 | 4-F-Ph | Ring 3 | SOPr |
| 9-100 | 4-F-Ph | Ring 3 | $SO_2Me$ |
| 9-101 | 4-F-Ph | Ring 3 | $SO_2Et$ |
| 9-102 | 4-F-Ph | Ring 3 | $SO_2Pr$ |
| 9-103 | 4-F-Ph | Ring 3 | COMe |
| 9-104 | 4-F-Ph | Ring 3 | COEt |
| 9-105 | 4-F-Ph | Ring 3 | COPr |
| 9-106 | 3-Cl-4-F-Ph | Ring 3 | $CONH_2$ |
| 9-107 | 3-Cl-4-F-Ph | Ring 3 | CONHMe |
| 9-108 | 3-Cl-4-F-Ph | Ring 3 | CONHEt |
| 9-109 | 3-Cl-4-F-Ph | Ring 3 | CONHPr |
| 9-110 | 3-Cl-4-F-Ph | Ring 3 | CONH-i-Pr |
| 9-111 | 3-Cl-4-F-Ph | Ring 3 | CONHBu |
| 9-112 | 3-Cl-4-F-Ph | Ring 3 | $SO_2NH_2$ |
| 9-113 | 3-Cl-4-F-Ph | Ring 3 | $SO_2NHMe$ |
| 9-114 | 3-Cl-4-F-Ph | Ring 3 | $SO_2NHEt$ |
| 9-115 | 3-Cl-4-F-Ph | Ring 3 | $SO_2NHPr$ |
| 9-116 | 3-Cl-4-F-Ph | Ring 3 | $SO_2NH$-i-Pr |
| 9-117 | 3-Cl-4-F-Ph | Ring 3 | $SO_2NHBu$ |
| 9-118 | 3-Cl-4-F-Ph | Ring 3 | SOMe |
| 9-119 | 3-Cl-4-F-Ph | Ring 3 | SOEt |
| 9-120 | 3-Cl-4-F-Ph | Ring 3 | SOPr |
| 9-121 | 3-Cl-4-F-Ph | Ring 3 | $SO_2Me$ |
| 9-122 | 3-Cl-4-F-Ph | Ring 3 | $SO_2Et$ |
| 9-123 | 3-Cl-4-F-Ph | Ring 3 | $SO_2Pr$ |
| 9-124 | 3-Cl-4-F-Ph | Ring 3 | COMe |
| 9-125 | 3-Cl-4-F-Ph | Ring 3 | COEt |

| | | | |
|---|---|---|---|
| 9-126 | 3-Cl-4-F-Ph | Ring 3 | COPr |
| 9-127 | 4-F-Ph | Ring 4 | $CONH_2$ |
| 9-128 | 4-F-Ph | Ring 4 | CONHMe |
| 9-129 | 4-F-Ph | Ring 4 | CONHEt |
| 9-130 | 4-F-Ph | Ring 4 | CONHPr |
| 9-131 | 4-F-Ph | Ring 4 | CONH-i-Pr |
| 9-132 | 4-F-Ph | Ring 4 | CONHBu |
| 9-133 | 4-F-Ph | Ring 4 | $SO_2NH_2$ |
| 9-134 | 4-F-Ph | Ring 4 | $SO_2NHMe$ |
| 9-135 | 4-F-Ph | Ring 4 | $SO_2NHEt$ |
| 9-136 | 4-F-Ph | Ring 4 | $SO_2NHPr$ |
| 9-137 | 4-F-Ph | Ring 4 | $SO_2NH$-i-Pr |
| 9-138 | 4-F-Ph | Ring 4 | $SO_2NHBu$ |
| 9-139 | 4-F-Ph | Ring 4 | SOMe |
| 9-140 | 4-F-Ph | Ring 4 | SOEt |
| 9-141 | 4-F-Ph | Ring 4 | SOPr |
| 9-142 | 4-F-Ph | Ring 4 | $SO_2Me$ |
| 9-143 | 4-F-Ph | Ring 4 | $SO_2Et$ |
| 9-144 | 4-F-Ph | Ring 4 | $SO_2Pr$ |
| 9-145 | 4-F-Ph | Ring 4 | COMe |
| 9-146 | 4-F-Ph | Ring 4 | COEt |
| 9-147 | 4-F-Ph | Ring 4 | COPr |
| 9-148 | 3-Cl-4-F-Ph | Ring 4 | $CONH_2$ |
| 9-149 | 3-Cl-4-F-Ph | Ring 4 | CONHMe |
| 9-150 | 3-Cl-4-F-Ph | Ring 4 | CONHEt |
| 9-151 | 3-Cl-4-F-Ph | Ring 4 | CONHPr |
| 9-152 | 3-Cl-4-F-Ph | Ring 4 | CONH-i-Pr |
| 9-153 | 3-Cl-4-F-Ph | Ring 4 | CONHBu |
| 9-154 | 3-Cl-4-F-Ph | Ring 4 | $SO_2NH_2$ |
| 9-155 | 3-Cl-4-F-Ph | Ring 4 | $SO_2NHMe$ |
| 9-156 | 3-Cl-4-F-Ph | Ring 4 | $SO_2NHEt$ |
| 9-157 | 3-Cl-4-F-Ph | Ring 4 | $SO_2NHPr$ |
| 9-158 | 3-Cl-4-F-Ph | Ring 4 | $SO_2NH$-i-Pr |
| 9-159 | 3-Cl-4-F-Ph | Ring 4 | $SO_2NHBu$ |
| 9-160 | 3-Cl-4-F-Ph | Ring 4 | SOMe |
| 9-161 | 3-Cl-4-F-Ph | Ring 4 | SOEt |

| | | | |
|---|---|---|---|
| 9-162 | 3-Cl-4-F-Ph | Ring 4 | SOPr |
| 9-163 | 3-Cl-4-F-Ph | Ring 4 | $SO_2Me$ |
| 9-164 | 3-Cl-4-F-Ph | Ring 4 | $SO_2Et$ |
| 9-165 | 3-Cl-4-F-Ph | Ring 4 | $SO_2Pr$ |
| 9-166 | 3-Cl-4-F-Ph | Ring 4 | COMe |
| 9-167 | 3-Cl-4-F-Ph | Ring 4 | COEt |
| 9-168 | 3-Cl-4-F-Ph | Ring 4 | COPr |
| 9-169 | 4-F-Ph | Ring 5 | $CONH_2$ |
| 9-170 | 4-F-Ph | Ring 5 | CONHMe |
| 9-171 | 4-F-Ph | Ring 5 | CONHEt |
| 9-172 | 4-F-Ph | Ring 5 | CONHPr |
| 9-173 | 4-F-Ph | Ring 5 | CONH-i-Pr |
| 9-174 | 4-F-Ph | Ring 5 | CONHBu |
| 9-175 | 4-F-Ph | Ring 5 | $SO_2NH_2$ |
| 9-176 | 4-F-Ph | Ring 5 | $SO_2NHMe$ |
| 9-177 | 4-F-Ph | Ring 5 | $SO_2NHEt$ |
| 9-178 | 4-F-Ph | Ring 5 | $SO_2NHPr$ |
| 9-179 | 4-F-Ph | Ring 5 | $SO_2NH$-i-Pr |
| 9-180 | 4-F-Ph | Ring 5 | $SO_2NHBu$ |
| 9-181 | 4-F-Ph | Ring 5 | SOMe |
| 9-182 | 4-F-Ph | Ring 5 | SOEt |
| 9-183 | 4-F-Ph | Ring 5 | SOPr |
| 9-184 | 4-F-Ph | Ring 5 | $SO_2Me$ |
| 9-185 | 4-F-Ph | Ring 5 | $SO_2Et$ |
| 9-186 | 4-F-Ph | Ring 5 | $SO_2Pr$ |
| 9-187 | 4-F-Ph | Ring 5 | COMe |
| 9-188 | 4-F-Ph | Ring 5 | COEt |
| 9-189 | 4-F-Ph | Ring 5 | COPr |
| 9-190 | 3-Cl-4-F-Ph | Ring 5 | $CONH_2$ |
| 9-191 | 3-Cl-4-F-Ph | Ring 5 | CONHMe |
| 9-192 | 3-Cl-4-F-Ph | Ring 5 | CONHEt |
| 9-193 | 3-Cl-4-F-Ph | Ring 5 | CONHPr |
| 9-194 | 3-Cl-4-F-Ph | Ring 5 | CONH-i-Pr |
| 9-195 | 3-Cl-4-F-Ph | Ring 5 | CONHBu |
| 9-196 | 3-Cl-4-F-Ph | Ring 5 | $SO_2NH_2$ |
| 9-197 | 3-Cl-4-F-Ph | Ring 5 | $SO_2NHMe$ |

| | | | |
|---|---|---|---|
| 9-198 | 3-Cl-4-F-Ph | Ring 5 | $SO_2NHEt$ |
| 9-199 | 3-Cl-4-F-Ph | Ring 5 | $SO_2NHPr$ |
| 9-200 | 3-Cl-4-F-Ph | Ring 5 | $SO_2NH$-i-Pr |
| 9-201 | 3-Cl-4-F-Ph | Ring 5 | $SO_2NHBu$ |
| 9-202 | 3-Cl-4-F-Ph | Ring 5 | SOMe |
| 9-203 | 3-Cl-4-F-Ph | Ring 5 | SOEt |
| 9-204 | 3-Cl-4-F-Ph | Ring 5 | SOPr |
| 9-205 | 3-Cl-4-F-Ph | Ring 5 | $SO_2Me$ |
| 9-206 | 3-Cl-4-F-Ph | Ring 5 | $SO_2Et$ |
| 9-207 | 3-Cl-4-F-Ph | Ring 5 | $SO_2Pr$ |
| 9-208 | 3-Cl-4-F-Ph | Ring 5 | COMe |
| 9-209 | 3-Cl-4-F-Ph | Ring 5 | COEt |
| 9-210 | 3-Cl-4-F-Ph | Ring 5 | COPr |
| 9-211 | 4-F-Ph | Ring 6 | $CONH_2$ |
| 9-212 | 4-F-Ph | Ring 6 | CONHMe |
| 9-213 | 4-F-Ph | Ring 6 | CONHEt |
| 9-214 | 4-F-Ph | Ring 6 | CONHPr |
| 9-215 | 4-F-Ph | Ring 6 | CONH-i-Pr |
| 9-216 | 4-F-Ph | Ring 6 | CONHBu |
| 9-217 | 4-F-Ph | Ring 6 | $SO_2NH_2$ |
| 9-218 | 4-F-Ph | Ring 6 | $SO_2NHMe$ |
| 9-219 | 4-F-Ph | Ring 6 | $SO_2NHEt$ |
| 9-220 | 4-F-Ph | Ring 6 | $SO_2NHPr$ |
| 9-221 | 4-F-Ph | Ring 6 | $SO_2NH$-i-Pr |
| 9-222 | 4-F-Ph | Ring 6 | $SO_2NHBu$ |
| 9-223 | 4-F-Ph | Ring 6 | SOMe |
| 9-224 | 4-F-Ph | Ring 6 | SOEt |
| 9-225 | 4-F-Ph | Ring 6 | SOPr |
| 9-226 | 4-F-Ph | Ring 6 | $SO_2Me$ |
| 9-227 | 4-F-Ph | Ring 6 | $SO_2Et$ |
| 9-228 | 4-F-Ph | Ring 6 | $SO_2Pr$ |
| 9-229 | 4-F-Ph | Ring 6 | COMe |
| 9-230 | 4-F-Ph | Ring 6 | COEt |
| 9-231 | 4-F-Ph | Ring 6 | COPr |
| 9-232 | 3-Cl-4-F-Ph | Ring 6 | $CONH_2$ |
| 9-233 | 3-Cl-4-F-Ph | Ring 6 | CONHMe |

| | | | |
|---|---|---|---|
| 9-234 | 3-Cl-4-F-Ph | Ring 6 | CONHEt |
| 9-235 | 3-Cl-4-F-Ph | Ring 6 | CONHPr |
| 9-236 | 3-Cl-4-F-Ph | Ring 6 | CONH-i-Pr |
| 9-237 | 3-Cl-4-F-Ph | Ring 6 | CONHBu |
| 9-238 | 3-Cl-4-F-Ph | Ring 6 | $SO_2NH_2$ |
| 9-239 | 3-Cl-4-F-Ph | Ring 6 | $SO_2NHMe$ |
| 9-240 | 3-Cl-4-F-Ph | Ring 6 | $SO_2NHEt$ |
| 9-241 | 3-Cl-4-F-Ph | Ring 6 | $SO_2NHPr$ |
| 9-242 | 3-Cl-4-F-Ph | Ring 6 | $SO_2NH$-i-Pr |
| 9-243 | 3-Cl-4-F-Ph | Ring 6 | $SO_2NHBu$ |
| 9-244 | 3-Cl-4-F-Ph | Ring 6 | SOMe |
| 9-245 | 3-Cl-4-F-Ph | Ring 6 | SOEt |
| 9-246 | 3-Cl-4-F-Ph | Ring 6 | SOPr |
| 9-247 | 3-Cl-4-F-Ph | Ring 6 | $SO_2Me$ |
| 9-248 | 3-Cl-4-F-Ph | Ring 6 | $SO_2Et$ |
| 9-249 | 3-Cl-4-F-Ph | Ring 6 | $SO_2Pr$ |
| 9-250 | 3-Cl-4-F-Ph | Ring 6 | COMe |
| 9-251 | 3-Cl-4-F-Ph | Ring 6 | COEt |
| 9-252 | 3-Cl-4-F-Ph | Ring 6 | COPr |
| 9-253 | 4-F-Ph | Ring 7 | $CONH_2$ |
| 9-254 | 4-F-Ph | Ring 7 | CONHMe |
| 9-255 | 4-F-Ph | Ring 7 | CONHEt |
| 9-256 | 4-F-Ph | Ring 7 | CONHPr |
| 9-257 | 4-F-Ph | Ring 7 | CONH-i-Pr |
| 9-258 | 4-F-Ph | Ring 7 | CONHBu |
| 9-259 | 4-F-Ph | Ring 7 | $SO_2NH_2$ |
| 9-260 | 4-F-Ph | Ring 7 | $SO_2NHMe$ |
| 9-261 | 4-F-Ph | Ring 7 | $SO_2NHEt$ |
| 9-262 | 4-F-Ph | Ring 7 | $SO_2NHPr$ |
| 9-263 | 4-F-Ph | Ring 7 | $SO_2NH$-i-Pr |
| 9-264 | 4-F-Ph | Ring 7 | $SO_2NHBu$ |
| 9-265 | 4-F-Ph | Ring 7 | SOMe |
| 9-266 | 4-F-Ph | Ring 7 | SOEt |
| 9-267 | 4-F-Ph | Ring 7 | SOPr |
| 9-268 | 4-F-Ph | Ring 7 | $SO_2Me$ |
| 9-269 | 4-F-Ph | Ring 7 | $SO_2Et$ |

| | | | |
|---|---|---|---|
| 9-270 | 4-F-Ph | Ring 7 | $SO_2Pr$ |
| 9-271 | 4-F-Ph | Ring 7 | COMe |
| 9-272 | 4-F-Ph | Ring 7 | COEt |
| 9-273 | 4-F-Ph | Ring 7 | COPr |
| 9-274 | 3-Cl-4-F-Ph | Ring 7 | $CONH_2$ |
| 9-275 | 3-Cl-4-F-Ph | Ring 7 | CONHMe |
| 9-276 | 3-Cl-4-F-Ph | Ring 7 | CONHEt |
| 9-277 | 3-Cl-4-F-Ph | Ring 7 | CONHPr |
| 9-278 | 3-Cl-4-F-Ph | Ring 7 | CONH-i-Pr |
| 9-279 | 3-Cl-4-F-Ph | Ring 7 | CONHBu |
| 9-280 | 3-Cl-4-F-Ph | Ring 7 | $SO_2NH_2$ |
| 9-281 | 3-Cl-4-F-Ph | Ring 7 | $SO_2NHMe$ |
| 9-282 | 3-Cl-4-F-Ph | Ring 7 | $SO_2NHEt$ |
| 9-283 | 3-Cl-4-F-Ph | Ring 7 | $SO_2NHPr$ |
| 9-284 | 3-Cl-4-F-Ph | Ring 7 | $SO_2NH$-i-Pr |
| 9-285 | 3-Cl-4-F-Ph | Ring 7 | $SO_2NHBu$ |
| 9-286 | 3-Cl-4-F-Ph | Ring 7 | SOMe |
| 9-287 | 3-Cl-4-F-Ph | Ring 7 | SOEt |
| 9-288 | 3-Cl-4-F-Ph | Ring 7 | SOPr |
| 9-289 | 3-Cl-4-F-Ph | Ring 7 | $SO_2Me$ |
| 9-290 | 3-Cl-4-F-Ph | Ring 7 | $SO_2Et$ |
| 9-291 | 3-Cl-4-F-Ph | Ring 7 | $SO_2Pr$ |
| 9-292 | 3-Cl-4-F-Ph | Ring 7 | COMe |
| 9-293 | 3-Cl-4-F-Ph | Ring 7 | COEt |
| 9-294 | 3-Cl-4-F-Ph | Ring 7 | COPr |
| 9-295 | 4-F-Ph | Ring 8 | $CONH_2$ |
| 9-296 | 4-F-Ph | Ring 8 | CONHMe |
| 9-297 | 4-F-Ph | Ring 8 | CONHEt |
| 9-298 | 4-F-Ph | Ring 8 | CONHPr |
| 9-299 | 4-F-Ph | Ring 8 | CONH-i-Pr |
| 9-300 | 4-F-Ph | Ring 8 | CONHBu |
| 9-301 | 4-F-Ph | Ring 8 | $SO_2NH_2$ |
| 9-302 | 4-F-Ph | Ring 8 | $SO_2NHMe$ |
| 9-303 | 4-F-Ph | Ring 8 | $SO_2NHEt$ |
| 9-304 | 4-F-Ph | Ring 8 | $SO_2NHPr$ |
| 9-305 | 4-F-Ph | Ring 8 | $SO_2NH$-i-Pr |

| | | | |
|---|---|---|---|
| 9-306 | 4-F-Ph | Ring 8 | $SO_2NHBu$ |
| 9-307 | 4-F-Ph | Ring 8 | SOMe |
| 9-308 | 4-F-Ph | Ring 8 | SOEt |
| 9-309 | 4-F-Ph | Ring 8 | SOPr |
| 9-310 | 4-F-Ph | Ring 8 | $SO_2Me$ |
| 9-311 | 4-F-Ph | Ring 8 | $SO_2Et$ |
| 9-312 | 4-F-Ph | Ring 8 | $SO_2Pr$ |
| 9-313 | 4-F-Ph | Ring 8 | COMe |
| 9-314 | 4-F-Ph | Ring 8 | COEt |
| 9-315 | 4-F-Ph | Ring 8 | COPr |
| 9-316 | 3-Cl-4-F-Ph | Ring 8 | $CONH_2$ |
| 9-317 | 3-Cl-4-F-Ph | Ring 8 | CONHMe |
| 9-318 | 3-Cl-4-F-Ph | Ring 8 | CONHEt |
| 9-319 | 3-Cl-4-F-Ph | Ring 8 | CONHPr |
| 9-320 | 3-Cl-4-F-Ph | Ring 8 | CONH-i-Pr |
| 9-321 | 3-Cl-4-F-Ph | Ring 8 | CONHBu |
| 9-322 | 3-Cl-4-F-Ph | Ring 8 | $SO_2NH_2$ |
| 9-323 | 3-Cl-4-F-Ph | Ring 8 | $SO_2NHMe$ |
| 9-324 | 3-Cl-4-F-Ph | Ring 8 | $SO_2NHEt$ |
| 9-325 | 3-Cl-4-F-Ph | Ring 8 | $SO_2NHPr$ |
| 9-326 | 3-Cl-4-F-Ph | Ring 8 | $SO_2NH$-i-Pr |
| 9-327 | 3-Cl-4-F-Ph | Ring 8 | $SO_2NHBu$ |
| 9-328 | 3-Cl-4-F-Ph | Ring 8 | SOMe |
| 9-329 | 3-Cl-4-F-Ph | Ring 8 | SOEt |
| 9-330 | 3-Cl-4-F-Ph | Ring 8 | SOPr |
| 9-331 | 3-Cl-4-F-Ph | Ring 8 | $SO_2Me$ |
| 9-332 | 3-Cl-4-F-Ph | Ring 8 | $SO_2Et$ |
| 9-333 | 3-Cl-4-F-Ph | Ring 8 | $SO_2Pr$ |
| 9-334 | 3-Cl-4-F-Ph | Ring 8 | COMe |
| 9-335 | 3-Cl-4-F-Ph | Ring 8 | COEt |
| 9-336 | 3-Cl-4-F-Ph | Ring 8 | COPr |
| 9-337 | 4-F-Ph | Ring 9 | $CONH_2$ |
| 9-338 | 4-F-Ph | Ring 9 | CONHMe |
| 9-339 | 4-F-Ph | Ring 9 | CONHEt |
| 9-340 | 4-F-Ph | Ring 9 | CONHPr |
| 9-341 | 4-F-Ph | Ring 9 | CONH-i-Pr |

| | | | |
|---|---|---|---|
| 9-342 | 4-F-Ph | Ring 9 | CONHBu |
| 9-343 | 4-F-Ph | Ring 9 | $SO_2NH_2$ |
| 9-344 | 4-F-Ph | Ring 9 | $SO_2NHMe$ |
| 9-345 | 4-F-Ph | Ring 9 | $SO_2NHEt$ |
| 9-346 | 4-F-Ph | Ring 9 | $SO_2NHPr$ |
| 9-347 | 4-F-Ph | Ring 9 | $SO_2NH$-i-Pr |
| 9-348 | 4-F-Ph | Ring 9 | $SO_2NHBu$ |
| 9-349 | 4-F-Ph | Ring 9 | SOMe |
| 9-350 | 4-F-Ph | Ring 9 | SOEt |
| 9-351 | 4-F-Ph | Ring 9 | SOPr |
| 9-352 | 4-F-Ph | Ring 9 | $SO_2Me$ |
| 9-353 | 4-F-Ph | Ring 9 | $SO_2Et$ |
| 9-354 | 4-F-Ph | Ring 9 | $SO_2Pr$ |
| 9-355 | 4-F-Ph | Ring 9 | COMe |
| 9-356 | 4-F-Ph | Ring 9 | COEt |
| 9-357 | 4-F-Ph | Ring 9 | COPr |
| 9-358 | 3-Cl-4-F-Ph | Ring 9 | $CONH_2$ |
| 9-359 | 3-Cl-4-F-Ph | Ring 9 | CONHMe |
| 9-360 | 3-Cl-4-F-Ph | Ring 9 | CONHEt |
| 9-361 | 3-Cl-4-F-Ph | Ring 9 | CONHPr |
| 9-362 | 3-Cl-4-F-Ph | Ring 9 | CONH-i-Pr |
| 9-363 | 3-Cl-4-F-Ph | Ring 9 | CONHBu |
| 9-364 | 3-Cl-4-F-Ph | Ring 9 | $SO_2NH_2$ |
| 9-365 | 3-Cl-4-F-Ph | Ring 9 | $SO_2NHMe$ |
| 9-366 | 3-Cl-4-F-Ph | Ring 9 | $SO_2NHEt$ |
| 9-367 | 3-Cl-4-F-Ph | Ring 9 | $SO_2NHPr$ |
| 9-368 | 3-Cl-4-F-Ph | Ring 9 | $SO_2NH$-i-Pr |
| 9-369 | 3-Cl-4-F-Ph | Ring 9 | $SO_2NHBu$ |
| 9-370 | 3-Cl-4-F-Ph | Ring 9 | SOMe |
| 9-371 | 3-Cl-4-F-Ph | Ring 9 | SOEt |
| 9-372 | 3-Cl-4-F-Ph | Ring 9 | SOPr |
| 9-373 | 3-Cl-4-F-Ph | Ring 9 | $SO_2Me$ |
| 9-374 | 3-Cl-4-F-Ph | Ring 9 | $SO_2Et$ |
| 9-375 | 3-Cl-4-F-Ph | Ring 9 | $SO_2Pr$ |
| 9-376 | 3-Cl-4-F-Ph | Ring 9 | COMe |
| 9-377 | 3-Cl-4-F-Ph | Ring 9 | COEt |

| | | | |
|---|---|---|---|
| 9-378 | 3-Cl-4-F-Ph | Ring 9 | COPr |
| 9-379 | 4-F-Ph | Ring 10 | $CONH_2$ |
| 9-380 | 4-F-Ph | Ring 10 | CONHMe |
| 9-381 | 4-F-Ph | Ring 10 | CONHEt |
| 9-382 | 4-F-Ph | Ring 10 | CONHPr |
| 9-383 | 4-F-Ph | Ring 10 | CONH-i-Pr |
| 9-384 | 4-F-Ph | Ring 10 | CONHBu |
| 9-385 | 4-F-Ph | Ring 10 | $SO_2NH_2$ |
| 9-386 | 4-F-Ph | Ring 10 | $SO_2$NHMe |
| 9-387 | 4-F-Ph | Ring 10 | $SO_2$NHEt |
| 9-388 | 4-F-Ph | Ring 10 | $SO_2$NHPr |
| 9-389 | 4-F-Ph | Ring 10 | $SO_2$NH-i-Pr |
| 9-390 | 4-F-Ph | Ring 10 | $SO_2$NHBu |
| 9-391 | 4-F-Ph | Ring 10 | SOMe |
| 9-392 | 4-F-Ph | Ring 10 | SOEt |
| 9-393 | 4-F-Ph | Ring 10 | SOPr |
| 9-394 | 4-F-Ph | Ring 10 | $SO_2$Me |
| 9-395 | 4-F-Ph | Ring 10 | $SO_2$Et |
| 9-396 | 4-F-Ph | Ring 10 | $SO_2$Pr |
| 9-397 | 4-F-Ph | Ring 10 | COMe |
| 9-398 | 4-F-Ph | Ring 10 | COEt |
| 9-399 | 4-F-Ph | Ring 10 | COPr |
| 9-400 | 3-Cl-4-F-Ph | Ring 10 | $CONH_2$ |
| 9-401 | 3-Cl-4-F-Ph | Ring 10 | CONHMe |
| 9-402 | 3-Cl-4-F-Ph | Ring 10 | CONHEt |
| 9-403 | 3-Cl-4-F-Ph | Ring 10 | CONHPr |
| 9-404 | 3-Cl-4-F-Ph | Ring 10 | CONH-i-Pr |
| 9-405 | 3-Cl-4-F-Ph | Ring 10 | CONHBu |
| 9-406 | 3-Cl-4-F-Ph | Ring 10 | $SO_2NH_2$ |
| 9-407 | 3-Cl-4-F-Ph | Ring 10 | $SO_2$NHMe |
| 9-408 | 3-Cl-4-F-Ph | Ring 10 | $SO_2$NHEt |
| 9-409 | 3-Cl-4-F-Ph | Ring 10 | $SO_2$NHPr |
| 9-410 | 3-Cl-4-F-Ph | Ring 10 | $SO_2$NH-i-Pr |
| 9-411 | 3-Cl-4-F-Ph | Ring 10 | $SO_2$NHBu |
| 9-412 | 3-Cl-4-F-Ph | Ring 10 | SOMe |
| 9-413 | 3-Cl-4-F-Ph | Ring 10 | SOEt |

| | | | |
|---|---|---|---|
| 9-414 | 3-Cl-4-F-Ph | Ring 10 | SOPr |
| 9-415 | 3-Cl-4-F-Ph | Ring 10 | $SO_2Me$ |
| 9-416 | 3-Cl-4-F-Ph | Ring 10 | $SO_2Et$ |
| 9-417 | 3-Cl-4-F-Ph | Ring 10 | $SO_2Pr$ |
| 9-418 | 3-Cl-4-F-Ph | Ring 10 | COMe |
| 9-419 | 3-Cl-4-F-Ph | Ring 10 | COEt |
| 9-420 | 3-Cl-4-F-Ph | Ring 10 | COPr |
| 9-421 | 4-F-Ph | Ring 11 | $CONH_2$ |
| 9-422 | 4-F-Ph | Ring 11 | CONHMe |
| 9-423 | 4-F-Ph | Ring 11 | CONHEt |
| 9-424 | 4-F-Ph | Ring 11 | CONHPr |
| 9-425 | 4-F-Ph | Ring 11 | CONH-i-Pr |
| 9-426 | 4-F-Ph | Ring 11 | CONHBu |
| 9-427 | 4-F-Ph | Ring 11 | $SO_2NH_2$ |
| 9-428 | 4-F-Ph | Ring 11 | $SO_2NHMe$ |
| 9-429 | 4-F-Ph | Ring 11 | $SO_2NHEt$ |
| 9-430 | 4-F-Ph | Ring 11 | $SO_2NHPr$ |
| 9-431 | 4-F-Ph | Ring 11 | $SO_2NH$-i-Pr |
| 9-432 | 4-F-Ph | Ring 11 | $SO_2NHBu$ |
| 9-433 | 4-F-Ph | Ring 11 | SOMe |
| 9-434 | 4-F-Ph | Ring 11 | SOEt |
| 9-435 | 4-F-Ph | Ring 11 | SOPr |
| 9-436 | 4-F-Ph | Ring 11 | $SO_2Me$ |
| 9-437 | 4-F-Ph | Ring 11 | $SO_2Et$ |
| 9-438 | 4-F-Ph | Ring 11 | $SO_2Pr$ |
| 9-439 | 4-F-Ph | Ring 11 | COMe |
| 9-440 | 4-F-Ph | Ring 11 | COEt |
| 9-441 | 4-F-Ph | Ring 11 | COPr |
| 9-442 | 3-Cl-4-F-Ph | Ring 11 | $CONH_2$ |
| 9-443 | 3-Cl-4-F-Ph | Ring 11 | CONHMe |
| 9-444 | 3-Cl-4-F-Ph | Ring 11 | CONHEt |
| 9-445 | 3-Cl-4-F-Ph | Ring 11 | CONHPr |
| 9-446 | 3-Cl-4-F-Ph | Ring 11 | CONH-i-Pr |
| 9-447 | 3-Cl-4-F-Ph | Ring 11 | CONHBu |
| 9-448 | 3-Cl-4-F-Ph | Ring 11 | $SO_2NH_2$ |
| 9-449 | 3-Cl-4-F-Ph | Ring 11 | $SO_2NHMe$ |

| | | | |
|---|---|---|---|
| 9-450 | 3-Cl-4-F-Ph | Ring 11 | $SO_2NHEt$ |
| 9-451 | 3-Cl-4-F-Ph | Ring 11 | $SO_2NHPr$ |
| 9-452 | 3-Cl-4-F-Ph | Ring 11 | $SO_2NH$-i-Pr |
| 9-453 | 3-Cl-4-F-Ph | Ring 11 | $SO_2NHBu$ |
| 9-454 | 3-Cl-4-F-Ph | Ring 11 | SOMe |
| 9-455 | 3-Cl-4-F-Ph | Ring 11 | SOEt |
| 9-456 | 3-Cl-4-F-Ph | Ring 11 | SOPr |
| 9-457 | 3-Cl-4-F-Ph | Ring 11 | $SO_2Me$ |
| 9-458 | 3-Cl-4-F-Ph | Ring 11 | $SO_2Et$ |
| 9-459 | 3-Cl-4-F-Ph | Ring 11 | $SO_2Pr$ |
| 9-460 | 3-Cl-4-F-Ph | Ring 11 | COMe |
| 9-461 | 3-Cl-4-F-Ph | Ring 11 | COEt |
| 9-462 | 3-Cl-4-F-Ph | Ring 11 | COPr |
| 9-463 | 4-F-Ph | Ring 12 | $CONH_2$ |
| 9-464 | 4-F-Ph | Ring 12 | CONHMe |
| 9-465 | 4-F-Ph | Ring 12 | CONHEt |
| 9-466 | 4-F-Ph | Ring 12 | CONHPr |
| 9-467 | 4-F-Ph | Ring 12 | CONH-i-Pr |
| 9-468 | 4-F-Ph | Ring 12 | CONHBu |
| 9-469 | 4-F-Ph | Ring 12 | $SO_2NH_2$ |
| 9-470 | 4-F-Ph | Ring 12 | $SO_2NHMe$ |
| 9-471 | 4-F-Ph | Ring 12 | $SO_2NHEt$ |
| 9-472 | 4-F-Ph | Ring 12 | $SO_2NHPr$ |
| 9-473 | 4-F-Ph | Ring 12 | $SO_2NH$-i-Pr |
| 9-474 | 4-F-Ph | Ring 12 | $SO_2NHBu$ |
| 9-475 | 4-F-Ph | Ring 12 | SOMe |
| 9-476 | 4-F-Ph | Ring 12 | SOEt |
| 9-477 | 4-F-Ph | Ring 12 | SOPr |
| 9-478 | 4-F-Ph | Ring 12 | $SO_2Me$ |
| 9-479 | 4-F-Ph | Ring 12 | $SO_2Et$ |
| 9-480 | 4-F-Ph | Ring 12 | $SO_2Pr$ |
| 9-481 | 4-F-Ph | Ring 12 | COMe |
| 9-482 | 4-F-Ph | Ring 12 | COEt |
| 9-483 | 4-F-Ph | Ring 12 | COPr |
| 9-484 | 3-Cl-4-F-Ph | Ring 12 | $CONH_2$ |
| 9-485 | 3-Cl-4-F-Ph | Ring 12 | CONHMe |

| | | | |
|---|---|---|---|
| 9-486 | 3-Cl-4-F-Ph | Ring 12 | CONHEt |
| 9-487 | 3-Cl-4-F-Ph | Ring 12 | CONHPr |
| 9-488 | 3-Cl-4-F-Ph | Ring 12 | CONH-i-Pr |
| 9-489 | 3-Cl-4-F-Ph | Ring 12 | CONHBu |
| 9-490 | 3-Cl-4-F-Ph | Ring 12 | $SO_2NH_2$ |
| 9-491 | 3-Cl-4-F-Ph | Ring 12 | $SO_2NHMe$ |
| 9-492 | 3-Cl-4-F-Ph | Ring 12 | $SO_2NHEt$ |
| 9-493 | 3-Cl-4-F-Ph | Ring 12 | $SO_2NHPr$ |
| 9-494 | 3-Cl-4-F-Ph | Ring 12 | $SO_2NH$-i-Pr |
| 9-495 | 3-Cl-4-F-Ph | Ring 12 | $SO_2NHBu$ |
| 9-496 | 3-Cl-4-F-Ph | Ring 12 | SOMe |
| 9-497 | 3-Cl-4-F-Ph | Ring 12 | SOEt |
| 9-498 | 3-Cl-4-F-Ph | Ring 12 | SOPr |
| 9-499 | 3-Cl-4-F-Ph | Ring 12 | $SO_2Me$ |
| 9-500 | 3-Cl-4-F-Ph | Ring 12 | $SO_2Et$ |
| 9-501 | 3-Cl-4-F-Ph | Ring 12 | $SO_2Pr$ |
| 9-502 | 3-Cl-4-F-Ph | Ring 12 | COMe |
| 9-503 | 3-Cl-4-F-Ph | Ring 12 | COEt |
| 9-504 | 3-Cl-4-F-Ph | Ring 12 | COPr |
| 9-505 | 4-F-Ph | Ring 13 | $CONH_2$ |
| 9-506 | 4-F-Ph | Ring 13 | CONHMe |
| 9-507 | 4-F-Ph | Ring 13 | CONHEt |
| 9-508 | 4-F-Ph | Ring 13 | CONHPr |
| 9-509 | 4-F-Ph | Ring 13 | CONH-i-Pr |
| 9-510 | 4-F-Ph | Ring 13 | CONHBu |
| 9-511 | 4-F-Ph | Ring 13 | $SO_2NH_2$ |
| 9-512 | 4-F-Ph | Ring 13 | $SO_2NHMe$ |
| 9-513 | 4-F-Ph | Ring 13 | $SO_2NHEt$ |
| 9-514 | 4-F-Ph | Ring 13 | $SO_2NHPr$ |
| 9-515 | 4-F-Ph | Ring 13 | $SO_2NH$-i-Pr |
| 9-516 | 4-F-Ph | Ring 13 | $SO_2NHBu$ |
| 9-517 | 4-F-Ph | Ring 13 | SOMe |
| 9-518 | 4-F-Ph | Ring 13 | SOEt |
| 9-519 | 4-F-Ph | Ring 13 | SOPr |
| 9-520 | 4-F-Ph | Ring 13 | $SO_2Me$ |
| 9-521 | 4-F-Ph | Ring 13 | $SO_2Et$ |

| | | | |
|---|---|---|---|
| 9-522 | 4-F-Ph | Ring 13 | $SO_2$Pr |
| 9-523 | 4-F-Ph | Ring 13 | COMe |
| 9-524 | 4-F-Ph | Ring 13 | COEt |
| 9-525 | 4-F-Ph | Ring 13 | COPr |
| 9-526 | 3-Cl-4-F-Ph | Ring 13 | $CONH_2$ |
| 9-527 | 3-Cl-4-F-Ph | Ring 13 | CONHMe |
| 9-528 | 3-Cl-4-F-Ph | Ring 13 | CONHEt |
| 9-529 | 3-Cl-4-F-Ph | Ring 13 | CONHPr |
| 9-530 | 3-Cl-4-F-Ph | Ring 13 | CONH-i-Pr |
| 9-531 | 3-Cl-4-F-Ph | Ring 13 | CONHBu |
| 9-532 | 3-Cl-4-F-Ph | Ring 13 | $SO_2NH_2$ |
| 9-533 | 3-Cl-4-F-Ph | Ring 13 | $SO_2$NHMe |
| 9-534 | 3-Cl-4-F-Ph | Ring 13 | $SO_2$NHEt |
| 9-535 | 3-Cl-4-F-Ph | Ring 13 | $SO_2$NHPr |
| 9-536 | 3-Cl-4-F-Ph | Ring 13 | $SO_2$NH-i-Pr |
| 9-537 | 3-Cl-4-F-Ph | Ring 13 | $SO_2$NHBu |
| 9-538 | 3-Cl-4-F-Ph | Ring 13 | SOMe |
| 9-539 | 3-Cl-4-F-Ph | Ring 13 | SOEt |
| 9-540 | 3-Cl-4-F-Ph | Ring 13 | SOPr |
| 9-541 | 3-Cl-4-F-Ph | Ring 13 | $SO_2$Me |
| 9-542 | 3-Cl-4-F-Ph | Ring 13 | $SO_2$Et |
| 9-543 | 3-Cl-4-F-Ph | Ring 13 | $SO_2$Pr |
| 9-544 | 3-Cl-4-F-Ph | Ring 13 | COMe |
| 9-545 | 3-Cl-4-F-Ph | Ring 13 | COEt |
| 9-546 | 3-Cl-4-F-Ph | Ring 13 | COPr |
| 9-547 | 4-F-Ph | Ring 14 | $CONH_2$ |
| 9-548 | 4-F-Ph | Ring 14 | CONHMe |
| 9-549 | 4-F-Ph | Ring 14 | CONHEt |
| 9-550 | 4-F-Ph | Ring 14 | CONHPr |
| 9-551 | 4-F-Ph | Ring 14 | CONH-i-Pr |
| 9-552 | 4-F-Ph | Ring 14 | CONHBu |
| 9-553 | 4-F-Ph | Ring 14 | $SO_2NH_2$ |
| 9-554 | 4-F-Ph | Ring 14 | $SO_2$NHMe |
| 9-555 | 4-F-Ph | Ring 14 | $SO_2$NHEt |
| 9-556 | 4-F-Ph | Ring 14 | $SO_2$NHPr |
| 9-557 | 4-F-Ph | Ring 14 | $SO_2$NH-i-Pr |

| | | | |
|---|---|---|---|
| 9-558 | 4-F-Ph | Ring 14 | $SO_2NHBu$ |
| 9-559 | 4-F-Ph | Ring 14 | SOMe |
| 9-560 | 4-F-Ph | Ring 14 | SOEt |
| 9-561 | 4-F-Ph | Ring 14 | SOPr |
| 9-562 | 4-F-Ph | Ring 14 | $SO_2Me$ |
| 9-563 | 4-F-Ph | Ring 14 | $SO_2Et$ |
| 9-564 | 4-F-Ph | Ring 14 | $SO_2Pr$ |
| 9-565 | 4-F-Ph | Ring 14 | COMe |
| 9-566 | 4-F-Ph | Ring 14 | COEt |
| 9-567 | 4-F-Ph | Ring 14 | COPr |
| 9-568 | 3-Cl-4-F-Ph | Ring 14 | $CONH_2$ |
| 9-569 | 3-Cl-4-F-Ph | Ring 14 | CONHMe |
| 9-570 | 3-Cl-4-F-Ph | Ring 14 | CONHEt |
| 9-571 | 3-Cl-4-F-Ph | Ring 14 | CONHPr |
| 9-572 | 3-Cl-4-F-Ph | Ring 14 | CONH-i-Pr |
| 9-573 | 3-Cl-4-F-Ph | Ring 14 | CONHBu |
| 9-574 | 3-Cl-4-F-Ph | Ring 14 | $SO_2NH_2$ |
| 9-575 | 3-Cl-4-F-Ph | Ring 14 | $SO_2NHMe$ |
| 9-576 | 3-Cl-4-F-Ph | Ring 14 | $SO_2NHEt$ |
| 9-577 | 3-Cl-4-F-Ph | Ring 14 | $SO_2NHPr$ |
| 9-578 | 3-Cl-4-F-Ph | Ring 14 | $SO_2NH$-i-Pr |
| 9-579 | 3-Cl-4-F-Ph | Ring 14 | $SO_2NHBu$ |
| 9-580 | 3-Cl-4-F-Ph | Ring 14 | SOMe |
| 9-581 | 3-Cl-4-F-Ph | Ring 14 | SOEt |
| 9-582 | 3-Cl-4-F-Ph | Ring 14 | SOPr |
| 9-583 | 3-Cl-4-F-Ph | Ring 14 | $SO_2Me$ |
| 9-584 | 3-Cl-4-F-Ph | Ring 14 | $SO_2Et$ |
| 9-585 | 3-Cl-4-F-Ph | Ring 14 | $SO_2Pr$ |
| 9-586 | 3-Cl-4-F-Ph | Ring 14 | COMe |
| 9-587 | 3-Cl-4-F-Ph | Ring 14 | COEt |
| 9-588 | 3-Cl-4-F-Ph | Ring 14 | COPr |

Table 10

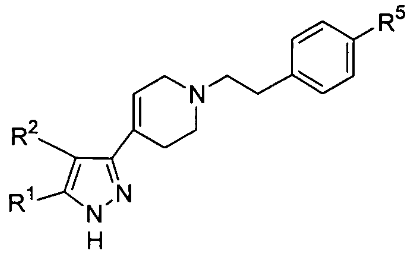

(I-10)

| Compound No. | $R^1$ | $R^2$ | $R^5$ |
|---|---|---|---|
| 10-1 | Ph | 4-Pyr | COOH |
| 10-2 | Ph | 4-Pyr | COOMe |
| 10-3 | Ph | 4-Pyr | COOEt |
| 10-4 | Ph | 4-Pyr | COOPr |
| 10-5 | Ph | 4-Pyr | COO-i-Pr |
| 10-6 | Ph | 4-Pyr | COOBu |
| 10-7 | Ph | 4-Pyr | COOBn |
| 10-8 | Ph | 4-Pyr | COOPh |
| 10-9 | Ph | 4-Pyr | $CONH_2$ |
| 10-10 | Ph | 4-Pyr | CONHMe |
| 10-11 | Ph | 4-Pyr | CONHEt |
| 10-12 | Ph | 4-Pyr | CONHPr |
| 10-13 | Ph | 4-Pyr | CONH-i-Pr |
| 10-14 | Ph | 4-Pyr | CONHBu |
| 10-15 | Ph | 4-Pyr | CONHBn |
| 10-16 | Ph | 4-Pyr | $CONMe_2$ |
| 10-17 | Ph | 4-Pyr | CONH-c-Pr |
| 10-18 | Ph | 4-Pyr | CONH-c-Bu |
| 10-19 | Ph | 4-Pyr | CONH-c-Pen |
| 10-20 | Ph | 4-Pyr | CONH-c-Hex |
| 10-21 | Ph | 4-Pyr | CONH-c-Hep |
| 10-22 | Ph | 4-Pyr | $CONHCH_2$-c-Pr |
| 10-23 | Ph | 4-Pyr | CONH-Allyl |
| 10-24 | Ph | 4-Pyr | CONH-Propargyl |
| 10-25 | Ph | 4-Pyr | CONHPh |
| 10-26 | Ph | 4-Pyr | CONH-3-Pyr |

| | | | |
|---|---|---|---|
| 10-27 | Ph | 4-Pyr | CONHCH$_2$-4-Pyr |
| 10-28 | Ph | 4-Pyr | CONH-(CH$_2$)$_2$-OH |
| 10-29 | Ph | 4-Pyr | CONHCH(CH$_2$OH)$_2$ |
| 10-30 | Ph | 4-Pyr | CONH-(CH$_2$)$_2$-OAc |
| 10-31 | Ph | 4-Pyr | CONHCH$_2$CN |
| 10-32 | Ph | 4-Pyr | CONH-(CH$_2$)$_2$-F |
| 10-33 | Ph | 4-Pyr | CONH-(CH$_2$)$_2$-OMe |
| 10-34 | Ph | 4-Pyr | CONH-(CH$_2$)$_2$-SMe |
| 10-35 | Ph | 4-Pyr | CONH-(CH$_2$)$_2$-NH$_2$ |
| 10-36 | Ph | 4-Pyr | CONH-(CH$_2$)$_2$-NHMe |
| 10-37 | Ph | 4-Pyr | CONH-(CH$_2$)$_2$-N(Me)$_2$ |
| 10-38 | Ph | 4-Pyr | CONHCH$_2$COOH |
| 10-39 | Ph | 4-Pyr | CONHCH$_2$COOEt |
| 10-40 | Ph | 4-Pyr | CONHCH(Me)COOEt |
| 10-41 | Ph | 4-Pyr | CONHCH$_2$CONH$_2$ |
| 10-42 | Ph | 4-Pyr | CONHOH |
| 10-43 | Ph | 4-Pyr | CONHOMe |
| 10-44 | Ph | 4-Pyr | CONHOEt |
| 10-45 | Ph | 4-Pyr | CONHOPr |
| 10-46 | Ph | 4-Pyr | CONHO-Allyl |
| 10-47 | Ph | 4-Pyr | CONHOBn |
| 10-48 | Ph | 4-Pyr | CONHNH$_2$ |
| 10-49 | Ph | 4-Pyr | CONHNHMe |
| 10-50 | Ph | 4-Pyr | CONHN(Me)$_2$ |
| 10-51 | Ph | 4-Pyr | COMe |
| 10-52 | Ph | 4-Pyr | COEt |
| 10-53 | Ph | 4-Pyr | COPr |
| 10-54 | Ph | 4-Pyr | CO-i-Pr |
| 10-55 | Ph | 4-Pyr | COBu |
| 10-56 | Ph | 4-Pyr | COCF$_3$ |
| 10-57 | Ph | 4-Pyr | CO-(CH$_2$)$_2$-F |
| 10-58 | Ph | 4-Pyr | CO-(CH$_2$)$_2$-OH |
| 10-59 | Ph | 4-Pyr | CO-(CH$_2$)$_2$-OMe |
| 10-60 | Ph | 4-Pyr | SOMe |
| 10-61 | Ph | 4-Pyr | SOEt |
| 10-62 | Ph | 4-Pyr | SOPr |

| | | | |
|---|---|---|---|
| 10-63 | Ph | 4-Pyr | SO-i-Pr |
| 10-64 | Ph | 4-Pyr | SOBu |
| 10-65 | Ph | 4-Pyr | $SOCF_3$ |
| 10-66 | Ph | 4-Pyr | SO-$(CH_2)_2$-F |
| 10-67 | Ph | 4-Pyr | SO-$(CH_2)_2$-OH |
| 10-68 | Ph | 4-Pyr | SO-$(CH_2)_2$-OMe |
| 10-69 | Ph | 4-Pyr | $SO_2Me$ |
| 10-70 | Ph | 4-Pyr | $SO_2Et$ |
| 10-71 | Ph | 4-Pyr | $SO_2Pr$ |
| 10-72 | Ph | 4-Pyr | $SO_2$-i-Pr |
| 10-73 | Ph | 4-Pyr | $SO_2Bu$ |
| 10-74 | Ph | 4-Pyr | $SO_2CF_3$ |
| 10-75 | Ph | 4-Pyr | $SO_2$-$(CH_2)_2$-F |
| 10-76 | Ph | 4-Pyr | $SO_2$-$(CH_2)_2$-OH |
| 10-77 | Ph | 4-Pyr | $SO_2$-$(CH_2)_2$-OMe |
| 10-78 | Ph | 4-Pyr | $SO_2NH_2$ |
| 10-79 | Ph | 4-Pyr | $SO_2NHMe$ |
| 10-80 | Ph | 4-Pyr | $SO_2NHEt$ |
| 10-81 | Ph | 4-Pyr | $SO_2NHPr$ |
| 10-82 | Ph | 4-Pyr | $SO_2NH$-i-Pr |
| 10-83 | Ph | 4-Pyr | $SO_2NHBu$ |
| 10-84 | Ph | 4-Pyr | $SO_2NHBn$ |
| 10-85 | Ph | 4-Pyr | $SO_2NMe_2$ |
| 10-86 | Ph | 4-Pyr | $SO_2NH$-c-Pr |
| 10-87 | Ph | 4-Pyr | $SO_2NH$-c-Bu |
| 10-88 | Ph | 4-Pyr | $SO_2NH$-c-Pen |
| 10-89 | Ph | 4-Pyr | $SO_2NH$-c-Hex |
| 10-90 | Ph | 4-Pyr | $SO_2NH$-c-Hep |
| 10-91 | Ph | 4-Pyr | $SO_2NHCH_2$-c-Pr |
| 10-92 | Ph | 4-Pyr | $SO_2NH$-Allyl |
| 10-93 | Ph | 4-Pyr | $SO_2NH$-Propargyl |
| 10-94 | Ph | 4-Pyr | $SO_2NHPh$ |
| 10-95 | Ph | 4-Pyr | $SO_2NH$-3-Pyr |
| 10-96 | Ph | 4-Pyr | $SO_2NHCH_2$-4-Pyr |
| 10-97 | Ph | 4-Pyr | $SO_2NH$-$(CH_2)_2$-OH |
| 10-98 | Ph | 4-Pyr | $SO_2NHCH(CH_2OH)_2$ |

| | | | |
|---|---|---|---|
| 10-99 | Ph | 4-Pyr | $SO_2NH-(CH_2)_2-OAc$ |
| 10-100 | Ph | 4-Pyr | $SO_2NHCH_2CN$ |
| 10-101 | Ph | 4-Pyr | $SO_2NH-(CH_2)_2-F$ |
| 10-102 | Ph | 4-Pyr | $SO_2NH-(CH_2)_2-OMe$ |
| 10-103 | Ph | 4-Pyr | $SO_2NH-(CH_2)_2-SMe$ |
| 10-104 | Ph | 4-Pyr | $SO_2NH-(CH_2)_2-NH_2$ |
| 10-105 | Ph | 4-Pyr | $SO_2NH-(CH_2)_2-NHMe$ |
| 10-106 | Ph | 4-Pyr | $SO_2NH-(CH_2)_2-N(Me)_2$ |
| 10-107 | Ph | 4-Pyr | $SO_2NHCH_2COOH$ |
| 10-108 | Ph | 4-Pyr | $SO_2NHCH_2COOEt$ |
| 10-109 | Ph | 4-Pyr | $SO_2NHCH(Me)COOEt$ |
| 10-110 | Ph | 4-Pyr | $SO_2NHCH_2CONH_2$ |
| 10-111 | Ph | 4-Pyr | $SO_2NHOH$ |
| 10-112 | Ph | 4-Pyr | $SO_2NHOMe$ |
| 10-113 | Ph | 4-Pyr | $SO_2NHOEt$ |
| 10-114 | Ph | 4-Pyr | $SO_2NHOPr$ |
| 10-115 | Ph | 4-Pyr | $SO_2NHOAllyl$ |
| 10-116 | Ph | 4-Pyr | $SO_2NHOBn$ |
| 10-117 | Ph | 4-Pyr | $SO_2NHNH_2$ |
| 10-118 | Ph | 4-Pyr | $SO_2NHNHMe$ |
| 10-119 | Ph | 4-Pyr | $SO_2NHN(Me)_2$ |
| 10-120 | 3-F-Ph | 4-Pyr | COOH |
| 10-121 | 3-F-Ph | 4-Pyr | COOMe |
| 10-122 | 3-F-Ph | 4-Pyr | COOEt |
| 10-123 | 3-F-Ph | 4-Pyr | COOPr |
| 10-124 | 3-F-Ph | 4-Pyr | COO-i-Pr |
| 10-125 | 3-F-Ph | 4-Pyr | COOBu |
| 10-126 | 3-F-Ph | 4-Pyr | COOBn |
| 10-127 | 3-F-Ph | 4-Pyr | COOPh |
| 10-128 | 3-F-Ph | 4-Pyr | $CONH_2$ |
| 10-129 | 3-F-Ph | 4-Pyr | CONHMe |
| 10-130 | 3-F-Ph | 4-Pyr | CONHEt |
| 10-131 | 3-F-Ph | 4-Pyr | CONHPr |
| 10-132 | 3-F-Ph | 4-Pyr | CONH-i-Pr |
| 10-133 | 3-F-Ph | 4-Pyr | CONHBu |
| 10-134 | 3-F-Ph | 4-Pyr | CONHBn |

| | | | |
|---|---|---|---|
| 10-135 | 3-F-Ph | 4-Pyr | $CONMe_2$ |
| 10-136 | 3-F-Ph | 4-Pyr | CONH-c-Pr |
| 10-137 | 3-F-Ph | 4-Pyr | CONH-c-Bu |
| 10-138 | 3-F-Ph | 4-Pyr | CONH-c-Pen |
| 10-139 | 3-F-Ph | 4-Pyr | CONH-c-Hex |
| 10-140 | 3-F-Ph | 4-Pyr | CONH-c-Hep |
| 10-141 | 3-F-Ph | 4-Pyr | $CONHCH_2$-c-Pr |
| 10-142 | 3-F-Ph | 4-Pyr | CONH-Allyl |
| 10-143 | 3-F-Ph | 4-Pyr | CONH-Propargyl |
| 10-144 | 3-F-Ph | 4-Pyr | CONHPh |
| 10-145 | 3-F-Ph | 4-Pyr | CONH-3-Pyr |
| 10-146 | 3-F-Ph | 4-Pyr | $CONHCH_2$-4-Pyr |
| 10-147 | 3-F-Ph | 4-Pyr | CONH-$(CH_2)_2$-OH |
| 10-148 | 3-F-Ph | 4-Pyr | $CONHCH(CH_2OH)_2$ |
| 10-149 | 3-F-Ph | 4-Pyr | CONH-$(CH_2)_2$-OAc |
| 10-150 | 3-F-Ph | 4-Pyr | $CONHCH_2CN$ |
| 10-151 | 3-F-Ph | 4-Pyr | CONH-$(CH_2)_2$-F |
| 10-152 | 3-F-Ph | 4-Pyr | CONH-$(CH_2)_2$-OMe |
| 10-153 | 3-F-Ph | 4-Pyr | CONH-$(CH_2)_2$-SMe |
| 10-154 | 3-F-Ph | 4-Pyr | CONH-$(CH_2)_2$-$NH_2$ |
| 10-155 | 3-F-Ph | 4-Pyr | CONH-$(CH_2)_2$-NHMe |
| 10-156 | 3-F-Ph | 4-Pyr | CONH-$(CH_2)_2$-$N(Me)_2$ |
| 10-157 | 3-F-Ph | 4-Pyr | $CONHCH_2COOH$ |
| 10-158 | 3-F-Ph | 4-Pyr | $CONHCH_2COOEt$ |
| 10-159 | 3-F-Ph | 4-Pyr | CONHCH(Me)COOEt |
| 10-160 | 3-F-Ph | 4-Pyr | $CONHCH_2CONH_2$ |
| 10-161 | 3-F-Ph | 4-Pyr | CONHOH |
| 10-162 | 3-F-Ph | 4-Pyr | CONHOMe |
| 10-163 | 3-F-Ph | 4-Pyr | CONHOEt |
| 10-164 | 3-F-Ph | 4-Pyr | CONHOPr |
| 10-165 | 3-F-Ph | 4-Pyr | CONHOAllyl |
| 10-166 | 3-F-Ph | 4-Pyr | CONHOBn |
| 10-167 | 3-F-Ph | 4-Pyr | $CONHNH_2$ |
| 10-168 | 3-F-Ph | 4-Pyr | CONHNHMe |
| 10-169 | 3-F-Ph | 4-Pyr | $CONHN(Me)_2$ |
| 10-170 | 3-F-Ph | 4-Pyr | COMe |

| | | | |
|---|---|---|---|
| 10-171 | 3-F-Ph | 4-Pyr | COEt |
| 10-172 | 3-F-Ph | 4-Pyr | COPr |
| 10-173 | 3-F-Ph | 4-Pyr | CO-i-Pr |
| 10-174 | 3-F-Ph | 4-Pyr | COBu |
| 10-175 | 3-F-Ph | 4-Pyr | $COCF_3$ |
| 10-176 | 3-F-Ph | 4-Pyr | $CO-(CH_2)_2-F$ |
| 10-177 | 3-F-Ph | 4-Pyr | $CO-(CH_2)_2-OH$ |
| 10-178 | 3-F-Ph | 4-Pyr | $CO-(CH_2)_2-OMe$ |
| 10-179 | 3-F-Ph | 4-Pyr | SOMe |
| 10-180 | 3-F-Ph | 4-Pyr | SOEt |
| 10-181 | 3-F-Ph | 4-Pyr | SOPr |
| 10-182 | 3-F-Ph | 4-Pyr | SO-i-Pr |
| 10-183 | 3-F-Ph | 4-Pyr | SOBu |
| 10-184 | 3-F-Ph | 4-Pyr | $SOCF_3$ |
| 10-185 | 3-F-Ph | 4-Pyr | $SO-(CH_2)_2-F$ |
| 10-186 | 3-F-Ph | 4-Pyr | $SO-(CH_2)_2-OH$ |
| 10-187 | 3-F-Ph | 4-Pyr | $SO-(CH_2)_2-OMe$ |
| 10-188 | 3-F-Ph | 4-Pyr | $SO_2Me$ |
| 10-189 | 3-F-Ph | 4-Pyr | $SO_2Et$ |
| 10-190 | 3-F-Ph | 4-Pyr | $SO_2Pr$ |
| 10-191 | 3-F-Ph | 4-Pyr | $SO_2$-i-Pr |
| 10-192 | 3-F-Ph | 4-Pyr | $SO_2Bu$ |
| 10-193 | 3-F-Ph | 4-Pyr | $SO_2CF_3$ |
| 10-194 | 3-F-Ph | 4-Pyr | $SO_2-(CH_2)_2-F$ |
| 10-195 | 3-F-Ph | 4-Pyr | $SO_2-(CH_2)_2-OH$ |
| 10-196 | 3-F-Ph | 4-Pyr | $SO_2-(CH_2)_2-OMe$ |
| 10-197 | 3-F-Ph | 4-Pyr | $SO_2NH_2$ |
| 10-198 | 3-F-Ph | 4-Pyr | $SO_2NHMe$ |
| 10-199 | 3-F-Ph | 4-Pyr | $SO_2NHEt$ |
| 10-200 | 3-F-Ph | 4-Pyr | $SO_2NHPr$ |
| 10-201 | 3-F-Ph | 4-Pyr | $SO_2NH$-i-Pr |
| 10-202 | 3-F-Ph | 4-Pyr | $SO_2NHBu$ |
| 10-203 | 3-F-Ph | 4-Pyr | $SO_2NHBn$ |
| 10-204 | 3-F-Ph | 4-Pyr | $SO_2NMe_2$ |
| 10-205 | 3-F-Ph | 4-Pyr | $SO_2NH$-c-Pr |
| 10-206 | 3-F-Ph | 4-Pyr | $SO_2NH$-c-Bu |

| | | | |
|---|---|---|---|
| 10-207 | 3-F-Ph | 4-Pyr | $SO_2$NH-c-Pen |
| 10-208 | 3-F-Ph | 4-Pyr | $SO_2$NH-c-Hex |
| 10-209 | 3-F-Ph | 4-Pyr | $SO_2$NH-c-Hep |
| 10-210 | 3-F-Ph | 4-Pyr | $SO_2NHCH_2$-c-Pr |
| 10-211 | 3-F-Ph | 4-Pyr | $SO_2$NH-Allyl |
| 10-212 | 3-F-Ph | 4-Pyr | $SO_2$NH-Propargyl |
| 10-213 | 3-F-Ph | 4-Pyr | $SO_2$NHPh |
| 10-214 | 3-F-Ph | 4-Pyr | $SO_2$NH-3-Pyr |
| 10-215 | 3-F-Ph | 4-Pyr | $SO_2NHCH_2$-4-Pyr |
| 10-216 | 3-F-Ph | 4-Pyr | $SO_2NH-(CH_2)_2-OH$ |
| 10-217 | 3-F-Ph | 4-Pyr | $SO_2NHCH(CH_2OH)_2$ |
| 10-218 | 3-F-Ph | 4-Pyr | $SO_2NH-(CH_2)_2-OAc$ |
| 10-219 | 3-F-Ph | 4-Pyr | $SO_2NHCH_2CN$ |
| 10-220 | 3-F-Ph | 4-Pyr | $SO_2NH-(CH_2)_2-F$ |
| 10-221 | 3-F-Ph | 4-Pyr | $SO_2NH-(CH_2)_2-OMe$ |
| 10-222 | 3-F-Ph | 4-Pyr | $SO_2NH-(CH_2)_2-SMe$ |
| 10-223 | 3-F-Ph | 4-Pyr | $SO_2NH-(CH_2)_2-NH_2$ |
| 10-224 | 3-F-Ph | 4-Pyr | $SO_2NH-(CH_2)_2-NHMe$ |
| 10-225 | 3-F-Ph | 4-Pyr | $SO_2NH-(CH_2)_2-N(Me)_2$ |
| 10-226 | 3-F-Ph | 4-Pyr | $SO_2NHCH_2COOH$ |
| 10-227 | 3-F-Ph | 4-Pyr | $SO_2NHCH_2COOEt$ |
| 10-228 | 3-F-Ph | 4-Pyr | $SO_2$NHCH(Me)COOEt |
| 10-229 | 3-F-Ph | 4-Pyr | $SO_2NHCH_2CONH_2$ |
| 10-230 | 3-F-Ph | 4-Pyr | $SO_2$NHOH |
| 10-231 | 3-F-Ph | 4-Pyr | $SO_2$NHOMe |
| 10-232 | 3-F-Ph | 4-Pyr | $SO_2$NHOEt |
| 10-233 | 3-F-Ph | 4-Pyr | $SO_2$NHOPr |
| 10-234 | 3-F-Ph | 4-Pyr | $SO_2$NHOAllyl |
| 10-235 | 3-F-Ph | 4-Pyr | $SO_2$NHOBn |
| 10-236 | 3-F-Ph | 4-Pyr | $SO_2NHNH_2$ |
| 10-237 | 3-F-Ph | 4-Pyr | $SO_2$NHNHMe |
| 10-238 | 3-F-Ph | 4-Pyr | $SO_2NHN(Me)_2$ |
| 10-239 | 3-Cl-Ph | 4-Pyr | COOH |
| 10-240 | 3-Cl-Ph | 4-Pyr | COOMe |
| 10-241 | 3-Cl-Ph | 4-Pyr | COOEt |
| 10-242 | 3-Cl-Ph | 4-Pyr | COOPr |

| | | | |
|---|---|---|---|
| 10-243 | 3-Cl-Ph | 4-Pyr | COO-i-Pr |
| 10-244 | 3-Cl-Ph | 4-Pyr | COOBu |
| 10-245 | 3-Cl-Ph | 4-Pyr | COOBn |
| 10-246 | 3-Cl-Ph | 4-Pyr | COOPh |
| 10-247 | 3-Cl-Ph | 4-Pyr | $CONH_2$ |
| 10-248 | 3-Cl-Ph | 4-Pyr | CONHMe |
| 10-249 | 3-Cl-Ph | 4-Pyr | CONHEt |
| 10-250 | 3-Cl-Ph | 4-Pyr | CONHPr |
| 10-251 | 3-Cl-Ph | 4-Pyr | CONH-i-Pr |
| 10-252 | 3-Cl-Ph | 4-Pyr | CONHBu |
| 10-253 | 3-Cl-Ph | 4-Pyr | CONHBn |
| 10-254 | 3-Cl-Ph | 4-Pyr | $CONMe_2$ |
| 10-255 | 3-Cl-Ph | 4-Pyr | CONH-c-Pr |
| 10-256 | 3-Cl-Ph | 4-Pyr | CONH-c-Bu |
| 10-257 | 3-Cl-Ph | 4-Pyr | CONH-c-Pen |
| 10-258 | 3-Cl-Ph | 4-Pyr | CONH-c-Hex |
| 10-259 | 3-Cl-Ph | 4-Pyr | CONH-c-Hep |
| 10-260 | 3-Cl-Ph | 4-Pyr | $CONHCH_2$-c-Pr |
| 10-261 | 3-Cl-Ph | 4-Pyr | CONH-Allyl |
| 10-262 | 3-Cl-Ph | 4-Pyr | CONH-Propargyl |
| 10-263 | 3-Cl-Ph | 4-Pyr | CONHPh |
| 10-264 | 3-Cl-Ph | 4-Pyr | CONH-3-Pyr |
| 10-265 | 3-Cl-Ph | 4-Pyr | $CONHCH_2$-4-Pyr |
| 10-266 | 3-Cl-Ph | 4-Pyr | CONH-$(CH_2)_2$-OH |
| 10-267 | 3-Cl-Ph | 4-Pyr | $CONHCH(CH_2OH)_2$ |
| 10-268 | 3-Cl-Ph | 4-Pyr | CONH-$(CH_2)_2$-OAc |
| 10-269 | 3-Cl-Ph | 4-Pyr | $CONHCH_2CN$ |
| 10-270 | 3-Cl-Ph | 4-Pyr | CONH-$(CH_2)_2$-F |
| 10-271 | 3-Cl-Ph | 4-Pyr | CONH-$(CH_2)_2$-OMe |
| 10-272 | 3-Cl-Ph | 4-Pyr | CONH-$(CH_2)_2$-SMe |
| 10-273 | 3-Cl-Ph | 4-Pyr | CONH-$(CH_2)_2$-$NH_2$ |
| 10-274 | 3-Cl-Ph | 4-Pyr | CONH-$(CH_2)_2$-NHMe |
| 10-275 | 3-Cl-Ph | 4-Pyr | CONH-$(CH_2)_2$-$N(Me)_2$ |
| 10-276 | 3-Cl-Ph | 4-Pyr | $CONHCH_2COOH$ |
| 10-277 | 3-Cl-Ph | 4-Pyr | $CONHCH_2COOEt$ |
| 10-278 | 3-Cl-Ph | 4-Pyr | CONHCH(Me)COOEt |

| | | | |
|---|---|---|---|
| 10-279 | 3-Cl-Ph | 4-Pyr | $CONHCH_2CONH_2$ |
| 10-280 | 3-Cl-Ph | 4-Pyr | CONHOH |
| 10-281 | 3-Cl-Ph | 4-Pyr | CONHOMe |
| 10-282 | 3-Cl-Ph | 4-Pyr | CONHOEt |
| 10-283 | 3-Cl-Ph | 4-Pyr | CONHOPr |
| 10-284 | 3-Cl-Ph | 4-Pyr | CONHOAllyl |
| 10-285 | 3-Cl-Ph | 4-Pyr | CONHOBn |
| 10-286 | 3-Cl-Ph | 4-Pyr | $CONHNH_2$ |
| 10-287 | 3-Cl-Ph | 4-Pyr | CONHNHMe |
| 10-288 | 3-Cl-Ph | 4-Pyr | $CONHN(Me)_2$ |
| 10-289 | 3-Cl-Ph | 4-Pyr | COMe |
| 10-290 | 3-Cl-Ph | 4-Pyr | COEt |
| 10-291 | 3-Cl-Ph | 4-Pyr | COPr |
| 10-292 | 3-Cl-Ph | 4-Pyr | CO-i-Pr |
| 10-293 | 3-Cl-Ph | 4-Pyr | COBu |
| 10-294 | 3-Cl-Ph | 4-Pyr | $COCF_3$ |
| 10-295 | 3-Cl-Ph | 4-Pyr | $CO-(CH_2)_2-F$ |
| 10-296 | 3-Cl-Ph | 4-Pyr | $CO-(CH_2)_2-OH$ |
| 10-297 | 3-Cl-Ph | 4-Pyr | $CO-(CH_2)_2-OMe$ |
| 10-298 | 3-Cl-Ph | 4-Pyr | SOMe |
| 10-299 | 3-Cl-Ph | 4-Pyr | SOEt |
| 10-300 | 3-Cl-Ph | 4-Pyr | SOPr |
| 10-301 | 3-Cl-Ph | 4-Pyr | SO-i-Pr |
| 10-302 | 3-Cl-Ph | 4-Pyr | SOBu |
| 10-303 | 3-Cl-Ph | 4-Pyr | $SOCF_3$ |
| 10-304 | 3-Cl-Ph | 4-Pyr | $SO-(CH_2)_2-F$ |
| 10-305 | 3-Cl-Ph | 4-Pyr | $SO-(CH_2)_2-OH$ |
| 10-306 | 3-Cl-Ph | 4-Pyr | $SO-(CH_2)_2-OMe$ |
| 10-307 | 3-Cl-Ph | 4-Pyr | $SO_2Me$ |
| 10-308 | 3-Cl-Ph | 4-Pyr | $SO_2Et$ |
| 10-309 | 3-Cl-Ph | 4-Pyr | $SO_2Pr$ |
| 10-310 | 3-Cl-Ph | 4-Pyr | $SO_2$-i-Pr |
| 10-311 | 3-Cl-Ph | 4-Pyr | $SO_2Bu$ |
| 10-312 | 3-Cl-Ph | 4-Pyr | $SO_2CF_3$ |
| 10-313 | 3-Cl-Ph | 4-Pyr | $SO_2-(CH_2)_2-F$ |
| 10-314 | 3-Cl-Ph | 4-Pyr | $SO_2-(CH_2)_2-OH$ |

| | | | |
|---|---|---|---|
| 10-315 | 3-Cl-Ph | 4-Pyr | $SO_2$-$(CH_2)_2$-OMe |
| 10-316 | 3-Cl-Ph | 4-Pyr | $SO_2NH_2$ |
| 10-317 | 3-Cl-Ph | 4-Pyr | $SO_2$NHMe |
| 10-318 | 3-Cl-Ph | 4-Pyr | $SO_2$NHEt |
| 10-319 | 3-Cl-Ph | 4-Pyr | $SO_2$NHPr |
| 10-320 | 3-Cl-Ph | 4-Pyr | $SO_2$NH-i-Pr |
| 10-321 | 3-Cl-Ph | 4-Pyr | $SO_2$NHBu |
| 10-322 | 3-Cl-Ph | 4-Pyr | $SO_2$NHBn |
| 10-323 | 3-Cl-Ph | 4-Pyr | $SO_2NMe_2$ |
| 10-324 | 3-Cl-Ph | 4-Pyr | $SO_2$NH-c-Pr |
| 10-325 | 3-Cl-Ph | 4-Pyr | $SO_2$NH-c-Bu |
| 10-326 | 3-Cl-Ph | 4-Pyr | $SO_2$NH-c-Pen |
| 10-327 | 3-Cl-Ph | 4-Pyr | $SO_2$NH-c-Hex |
| 10-328 | 3-Cl-Ph | 4-Pyr | $SO_2$NH-c-Hep |
| 10-329 | 3-Cl-Ph | 4-Pyr | $SO_2NHCH_2$-c-Pr |
| 10-330 | 3-Cl-Ph | 4-Pyr | $SO_2$NH-Allyl |
| 10-331 | 3-Cl-Ph | 4-Pyr | $SO_2$NH-Propargyl |
| 10-332 | 3-Cl-Ph | 4-Pyr | $SO_2$NHPh |
| 10-333 | 3-Cl-Ph | 4-Pyr | $SO_2$NH-3-Pyr |
| 10-334 | 3-Cl-Ph | 4-Pyr | $SO_2NHCH_2$-4-Pyr |
| 10-335 | 3-Cl-Ph | 4-Pyr | $SO_2$NH-$(CH_2)_2$-OH |
| 10-336 | 3-Cl-Ph | 4-Pyr | $SO_2NHCH(CH_2OH)_2$ |
| 10-337 | 3-Cl-Ph | 4-Pyr | $SO_2$NH-$(CH_2)_2$-OAc |
| 10-338 | 3-Cl-Ph | 4-Pyr | $SO_2NHCH_2CN$ |
| 10-339 | 3-Cl-Ph | 4-Pyr | $SO_2$NH-$(CH_2)_2$-F |
| 10-340 | 3-Cl-Ph | 4-Pyr | $SO_2$NH-$(CH_2)_2$-OMe |
| 10-341 | 3-Cl-Ph | 4-Pyr | $SO_2$NH-$(CH_2)_2$-SMe |
| 10-342 | 3-Cl-Ph | 4-Pyr | $SO_2$NH-$(CH_2)_2$-$NH_2$ |
| 10-343 | 3-Cl-Ph | 4-Pyr | $SO_2$NH-$(CH_2)_2$-NHMe |
| 10-344 | 3-Cl-Ph | 4-Pyr | $SO_2$NH-$(CH_2)_2$-$N(Me)_2$ |
| 10-345 | 3-Cl-Ph | 4-Pyr | $SO_2NHCH_2COOH$ |
| 10-346 | 3-Cl-Ph | 4-Pyr | $SO_2NHCH_2COOEt$ |
| 10-347 | 3-Cl-Ph | 4-Pyr | $SO_2$NHCH(Me)COOEt |
| 10-348 | 3-Cl-Ph | 4-Pyr | $SO_2NHCH_2CONH_2$ |
| 10-349 | 3-Cl-Ph | 4-Pyr | $SO_2$NHOH |
| 10-350 | 3-Cl-Ph | 4-Pyr | $SO_2$NHOMe |

| | | | |
|---|---|---|---|
| 10-351 | 3-Cl-Ph | 4-Pyr | $SO_2NHOEt$ |
| 10-352 | 3-Cl-Ph | 4-Pyr | $SO_2NHOPr$ |
| 10-353 | 3-Cl-Ph | 4-Pyr | $SO_2NHOAllyl$ |
| 10-354 | 3-Cl-Ph | 4-Pyr | $SO_2NHOBn$ |
| 10-355 | 3-Cl-Ph | 4-Pyr | $SO_2NHNH_2$ |
| 10-356 | 3-Cl-Ph | 4-Pyr | $SO_2NHNHMe$ |
| 10-357 | 3-Cl-Ph | 4-Pyr | $SO_2NHN(Me)_2$ |
| 10-358 | 4-F-Ph | 4-Pyr | COOH |
| 10-359 | 4-F-Ph | 4-Pyr | COOMe |
| 10-360 | 4-F-Ph | 4-Pyr | COOEt |
| 10-361 | 4-F-Ph | 4-Pyr | COOPr |
| 10-362 | 4-F-Ph | 4-Pyr | COO-i-Pr |
| 10-363 | 4-F-Ph | 4-Pyr | COOBu |
| 10-364 | 4-F-Ph | 4-Pyr | COOBn |
| 10-365 | 4-F-Ph | 4-Pyr | COOPh |
| 10-366 | 4-F-Ph | 4-Pyr | $CONH_2$ |
| 10-367 | 4-F-Ph | 4-Pyr | CONHMe |
| 10-368 | 4-F-Ph | 4-Pyr | CONHEt |
| 10-369 | 4-F-Ph | 4-Pyr | CONHPr |
| 10-370 | 4-F-Ph | 4-Pyr | CONH-i-Pr |
| 10-371 | 4-F-Ph | 4-Pyr | CONHBu |
| 10-372 | 4-F-Ph | 4-Pyr | CONHBn |
| 10-373 | 4-F-Ph | 4-Pyr | $CONMe_2$ |
| 10-374 | 4-F-Ph | 4-Pyr | CONH-c-Pr |
| 10-375 | 4-F-Ph | 4-Pyr | CONH-c-Bu |
| 10-376 | 4-F-Ph | 4-Pyr | CONH-c-Pen |
| 10-377 | 4-F-Ph | 4-Pyr | CONH-c-Hex |
| 10-378 | 4-F-Ph | 4-Pyr | CONH-c-Hep |
| 10-379 | 4-F-Ph | 4-Pyr | $CONHCH_2$-c-Pr |
| 10-380 | 4-F-Ph | 4-Pyr | CONH-Allyl |
| 10-381 | 4-F-Ph | 4-Pyr | CONH-Propargyl |
| 10-382 | 4-F-Ph | 4-Pyr | CONHPh |
| 10-383 | 4-F-Ph | 4-Pyr | CONH-3-Pyr |
| 10-384 | 4-F-Ph | 4-Pyr | $CONHCH_2$-4-Pyr |
| 10-385 | 4-F-Ph | 4-Pyr | CONH-$(CH_2)_2$-OH |
| 10-386 | 4-F-Ph | 4-Pyr | $CONHCH(CH_2OH)_2$ |

| | | | |
|---|---|---|---|
| 10-387 | 4-F-Ph | 4-Pyr | CONH-$(CH_2)_2$-OAc |
| 10-388 | 4-F-Ph | 4-Pyr | $CONHCH_2CN$ |
| 10-389 | 4-F-Ph | 4-Pyr | CONH-$(CH_2)_2$-F |
| 10-390 | 4-F-Ph | 4-Pyr | CONH-$(CH_2)_2$-OMe |
| 10-391 | 4-F-Ph | 4-Pyr | CONH-$(CH_2)_2$-SMe |
| 10-392 | 4-F-Ph | 4-Pyr | CONH-$(CH_2)_2$-$NH_2$ |
| 10-393 | 4-F-Ph | 4-Pyr | CONH-$(CH_2)_2$-NHMe |
| 10-394 | 4-F-Ph | 4-Pyr | CONH-$(CH_2)_2$-$N(Me)_2$ |
| 10-395 | 4-F-Ph | 4-Pyr | $CONHCH_2COOH$ |
| 10-396 | 4-F-Ph | 4-Pyr | $CONHCH_2COOEt$ |
| 10-397 | 4-F-Ph | 4-Pyr | CONHCH(Me)COOEt |
| 10-398 | 4-F-Ph | 4-Pyr | $CONHCH_2CONH_2$ |
| 10-399 | 4-F-Ph | 4-Pyr | CONHOH |
| 10-400 | 4-F-Ph | 4-Pyr | CONHOMe |
| 10-401 | 4-F-Ph | 4-Pyr | CONHOEt |
| 10-402 | 4-F-Ph | 4-Pyr | CONHOPr |
| 10-403 | 4-F-Ph | 4-Pyr | CONHOAllyl |
| 10-404 | 4-F-Ph | 4-Pyr | CONHOBn |
| 10-405 | 4-F-Ph | 4-Pyr | $CONHNH_2$ |
| 10-406 | 4-F-Ph | 4-Pyr | CONHNHMe |
| 10-407 | 4-F-Ph | 4-Pyr | $CONHN(Me)_2$ |
| 10-408 | 4-F-Ph | 4-Pyr | COMe |
| 10-409 | 4-F-Ph | 4-Pyr | COEt |
| 10-410 | 4-F-Ph | 4-Pyr | COPr |
| 10-411 | 4-F-Ph | 4-Pyr | CO-i-Pr |
| 10-412 | 4-F-Ph | 4-Pyr | COBu |
| 10-413 | 4-F-Ph | 4-Pyr | $COCF_3$ |
| 10-414 | 4-F-Ph | 4-Pyr | CO-$(CH_2)_2$-F |
| 10-415 | 4-F-Ph | 4-Pyr | CO-$(CH_2)_2$-OH |
| 10-416 | 4-F-Ph | 4-Pyr | CO-$(CH_2)_2$-OMe |
| 10-417 | 4-F-Ph | 4-Pyr | SOMe |
| 10-418 | 4-F-Ph | 4-Pyr | SOEt |
| 10-419 | 4-F-Ph | 4-Pyr | SOPr |
| 10-420 | 4-F-Ph | 4-Pyr | SO-i-Pr |
| 10-421 | 4-F-Ph | 4-Pyr | SOBu |
| 10-422 | 4-F-Ph | 4-Pyr | $SOCF_3$ |

| | | | |
|---|---|---|---|
| 10-423 | 4-F-Ph | 4-Pyr | SO-$(CH_2)_2$-F |
| 10-424 | 4-F-Ph | 4-Pyr | SO-$(CH_2)_2$-OH |
| 10-425 | 4-F-Ph | 4-Pyr | SO-$(CH_2)_2$-OMe |
| 10-426 | 4-F-Ph | 4-Pyr | $SO_2Me$ |
| 10-427 | 4-F-Ph | 4-Pyr | $SO_2Et$ |
| 10-428 | 4-F-Ph | 4-Pyr | $SO_2Pr$ |
| 10-429 | 4-F-Ph | 4-Pyr | $SO_2$-i-Pr |
| 10-430 | 4-F-Ph | 4-Pyr | $SO_2Bu$ |
| 10-431 | 4-F-Ph | 4-Pyr | $SO_2CF_3$ |
| 10-432 | 4-F-Ph | 4-Pyr | $SO_2$-$(CH_2)_2$-F |
| 10-433 | 4-F-Ph | 4-Pyr | $SO_2$-$(CH_2)_2$-OH |
| 10-434 | 4-F-Ph | 4-Pyr | $SO_2$-$(CH_2)_2$-OMe |
| 10-435 | 4-F-Ph | 4-Pyr | $SO_2NH_2$ |
| 10-436 | 4-F-Ph | 4-Pyr | $SO_2NHMe$ |
| 10-437 | 4-F-Ph | 4-Pyr | $SO_2NHEt$ |
| 10-438 | 4-F-Ph | 4-Pyr | $SO_2NHPr$ |
| 10-439 | 4-F-Ph | 4-Pyr | $SO_2NH$-i-Pr |
| 10-440 | 4-F-Ph | 4-Pyr | $SO_2NHBu$ |
| 10-441 | 4-F-Ph | 4-Pyr | $SO_2NHBn$ |
| 10-442 | 4-F-Ph | 4-Pyr | $SO_2NMe_2$ |
| 10-443 | 4-F-Ph | 4-Pyr | $SO_2NH$-c-Pr |
| 10-444 | 4-F-Ph | 4-Pyr | $SO_2NH$-c-Bu |
| 10-445 | 4-F-Ph | 4-Pyr | $SO_2NH$-c-Pen |
| 10-446 | 4-F-Ph | 4-Pyr | $SO_2NH$-c-Hex |
| 10-447 | 4-F-Ph | 4-Pyr | $SO_2NH$-c-Hep |
| 10-448 | 4-F-Ph | 4-Pyr | $SO_2NHCH_2$-c-Pr |
| 10-449 | 4-F-Ph | 4-Pyr | $SO_2NH$-Allyl |
| 10-450 | 4-F-Ph | 4-Pyr | $SO_2NH$-Propargyl |
| 10-451 | 4-F-Ph | 4-Pyr | $SO_2NHPh$ |
| 10-452 | 4-F-Ph | 4-Pyr | $SO_2NH$-3-Pyr |
| 10-453 | 4-F-Ph | 4-Pyr | $SO_2NHCH_2$-4-Pyr |
| 10-454 | 4-F-Ph | 4-Pyr | $SO_2NH$-$(CH_2)_2$-OH |
| 10-455 | 4-F-Ph | 4-Pyr | $SO_2NHCH(CH_2OH)_2$ |
| 10-456 | 4-F-Ph | 4-Pyr | $SO_2NH$-$(CH_2)_2$-OAc |
| 10-457 | 4-F-Ph | 4-Pyr | $SO_2NHCH_2CN$ |
| 10-458 | 4-F-Ph | 4-Pyr | $SO_2NH$-$(CH_2)_2$-F |

| | | | |
|---|---|---|---|
| 10-459 | 4-F-Ph | 4-Pyr | $SO_2NH-(CH_2)_2-OMe$ |
| 10-460 | 4-F-Ph | 4-Pyr | $SO_2NH-(CH_2)_2-SMe$ |
| 10-461 | 4-F-Ph | 4-Pyr | $SO_2NH-(CH_2)_2-NH_2$ |
| 10-462 | 4-F-Ph | 4-Pyr | $SO_2NH-(CH_2)_2-NHMe$ |
| 10-463 | 4-F-Ph | 4-Pyr | $SO_2NH-(CH_2)_2-N(Me)_2$ |
| 10-464 | 4-F-Ph | 4-Pyr | $SO_2NHCH_2COOH$ |
| 10-465 | 4-F-Ph | 4-Pyr | $SO_2NHCH_2COOEt$ |
| 10-466 | 4-F-Ph | 4-Pyr | $SO_2NHCH(Me)COOEt$ |
| 10-467 | 4-F-Ph | 4-Pyr | $SO_2NHCH_2CONH_2$ |
| 10-468 | 4-F-Ph | 4-Pyr | $SO_2NHOH$ |
| 10-469 | 4-F-Ph | 4-Pyr | $SO_2NHOMe$ |
| 10-470 | 4-F-Ph | 4-Pyr | $SO_2NHOEt$ |
| 10-471 | 4-F-Ph | 4-Pyr | $SO_2NHOPr$ |
| 10-472 | 4-F-Ph | 4-Pyr | $SO_2NHOAllyl$ |
| 10-473 | 4-F-Ph | 4-Pyr | $SO_2NHOBn$ |
| 10-474 | 4-F-Ph | 4-Pyr | $SO_2NHNH_2$ |
| 10-475 | 4-F-Ph | 4-Pyr | $SO_2NHNHMe$ |
| 10-476 | 4-F-Ph | 4-Pyr | $SO_2NHN(Me)_2$ |
| 10-477 | 3-Cl-4-F-Ph | 4-Pyr | COOH |
| 10-478 | 3-Cl-4-F-Ph | 4-Pyr | COOMe |
| 10-479 | 3-Cl-4-F-Ph | 4-Pyr | COOEt |
| 10-480 | 3-Cl-4-F-Ph | 4-Pyr | COOPr |
| 10-481 | 3-Cl-4-F-Ph | 4-Pyr | COO-i-Pr |
| 10-482 | 3-Cl-4-F-Ph | 4-Pyr | COOBu |
| 10-483 | 3-Cl-4-F-Ph | 4-Pyr | COOBn |
| 10-484 | 3-Cl-4-F-Ph | 4-Pyr | COOPh |
| 10-485 | 3-Cl-4-F-Ph | 4-Pyr | $CONH_2$ |
| 10-486 | 3-Cl-4-F-Ph | 4-Pyr | CONHMe |
| 10-487 | 3-Cl-4-F-Ph | 4-Pyr | CONHEt |
| 10-488 | 3-Cl-4-F-Ph | 4-Pyr | CONHPr |
| 10-489 | 3-Cl-4-F-Ph | 4-Pyr | CONH-i-Pr |
| 10-490 | 3-Cl-4-F-Ph | 4-Pyr | CONHBu |
| 10-491 | 3-Cl-4-F-Ph | 4-Pyr | CONHBn |
| 10-492 | 3-Cl-4-F-Ph | 4-Pyr | $CONMe_2$ |
| 10-493 | 3-Cl-4-F-Ph | 4-Pyr | CONH-c-Pr |
| 10-494 | 3-Cl-4-F-Ph | 4-Pyr | CONH-c-Bu |

| | | | |
|---|---|---|---|
| 10-495 | 3-Cl-4-F-Ph | 4-Pyr | CONH-c-Pen |
| 10-496 | 3-Cl-4-F-Ph | 4-Pyr | CONH-c-Hex |
| 10-497 | 3-Cl-4-F-Ph | 4-Pyr | CONH-c-Hep |
| 10-498 | 3-Cl-4-F-Ph | 4-Pyr | $CONHCH_2$-c-Pr |
| 10-499 | 3-Cl-4-F-Ph | 4-Pyr | CONH-Allyl |
| 10-500 | 3-Cl-4-F-Ph | 4-Pyr | CONH-Propargyl |
| 10-501 | 3-Cl-4-F-Ph | 4-Pyr | CONHPh |
| 10-502 | 3-Cl-4-F-Ph | 4-Pyr | CONH-3-Pyr |
| 10-503 | 3-Cl-4-F-Ph | 4-Pyr | $CONHCH_2$-4-Pyr |
| 10-504 | 3-Cl-4-F-Ph | 4-Pyr | CONH-$(CH_2)_2$-OH |
| 10-505 | 3-Cl-4-F-Ph | 4-Pyr | $CONHCH(CH_2OH)_2$ |
| 10-506 | 3-Cl-4-F-Ph | 4-Pyr | CONH-$(CH_2)_2$-OAc |
| 10-507 | 3-Cl-4-F-Ph | 4-Pyr | $CONHCH_2CN$ |
| 10-508 | 3-Cl-4-F-Ph | 4-Pyr | CONH-$(CH_2)_2$-F |
| 10-509 | 3-Cl-4-F-Ph | 4-Pyr | CONH-$(CH_2)_2$-OMe |
| 10-510 | 3-Cl-4-F-Ph | 4-Pyr | CONH-$(CH_2)_2$-SMe |
| 10-511 | 3-Cl-4-F-Ph | 4-Pyr | CONH-$(CH_2)_2$-$NH_2$ |
| 10-512 | 3-Cl-4-F-Ph | 4-Pyr | CONH-$(CH_2)_2$-NHMe |
| 10-513 | 3-Cl-4-F-Ph | 4-Pyr | CONH-$(CH_2)_2$-$N(Me)_2$ |
| 10-514 | 3-Cl-4-F-Ph | 4-Pyr | $CONHCH_2COOH$ |
| 10-515 | 3-Cl-4-F-Ph | 4-Pyr | $CONHCH_2COOEt$ |
| 10-516 | 3-Cl-4-F-Ph | 4-Pyr | CONHCH(Me)COOEt |
| 10-517 | 3-Cl-4-F-Ph | 4-Pyr | $CONHCH_2CONH_2$ |
| 10-518 | 3-Cl-4-F-Ph | 4-Pyr | CONHOH |
| 10-519 | 3-Cl-4-F-Ph | 4-Pyr | CONHOMe |
| 10-520 | 3-Cl-4-F-Ph | 4-Pyr | CONHOEt |
| 10-521 | 3-Cl-4-F-Ph | 4-Pyr | CONHOPr |
| 10-522 | 3-Cl-4-F-Ph | 4-Pyr | CONHOAllyl |
| 10-523 | 3-Cl-4-F-Ph | 4-Pyr | CONHOBn |
| 10-524 | 3-Cl-4-F-Ph | 4-Pyr | $CONHNH_2$ |
| 10-525 | 3-Cl-4-F-Ph | 4-Pyr | CONHNHMe |
| 10-526 | 3-Cl-4-F-Ph | 4-Pyr | $CONHN(Me)_2$ |
| 10-527 | 3-Cl-4-F-Ph | 4-Pyr | COMe |
| 10-528 | 3-Cl-4-F-Ph | 4-Pyr | COEt |
| 10-529 | 3-Cl-4-F-Ph | 4-Pyr | COPr |
| 10-530 | 3-Cl-4-F-Ph | 4-Pyr | CO-i-Pr |

| | | | |
|---|---|---|---|
| 10-531 | 3-Cl-4-F-Ph | 4-Pyr | COBu |
| 10-532 | 3-Cl-4-F-Ph | 4-Pyr | $COCF_3$ |
| 10-533 | 3-Cl-4-F-Ph | 4-Pyr | CO-$(CH_2)_2$-F |
| 10-534 | 3-Cl-4-F-Ph | 4-Pyr | CO-$(CH_2)_2$-OH |
| 10-535 | 3-Cl-4-F-Ph | 4-Pyr | CO-$(CH_2)_2$-OMe |
| 10-536 | 3-Cl-4-F-Ph | 4-Pyr | SOMe |
| 10-537 | 3-Cl-4-F-Ph | 4-Pyr | SOEt |
| 10-538 | 3-Cl-4-F-Ph | 4-Pyr | SOPr |
| 10-539 | 3-Cl-4-F-Ph | 4-Pyr | SO-i-Pr |
| 10-540 | 3-Cl-4-F-Ph | 4-Pyr | SOBu |
| 10-541 | 3-Cl-4-F-Ph | 4-Pyr | $SOCF_3$ |
| 10-542 | 3-Cl-4-F-Ph | 4-Pyr | SO-$(CH_2)_2$-F |
| 10-543 | 3-Cl-4-F-Ph | 4-Pyr | SO-$(CH_2)_2$-OH |
| 10-544 | 3-Cl-4-F-Ph | 4-Pyr | SO-$(CH_2)_2$-OMe |
| 10-545 | 3-Cl-4-F-Ph | 4-Pyr | $SO_2Me$ |
| 10-546 | 3-Cl-4-F-Ph | 4-Pyr | $SO_2Et$ |
| 10-547 | 3-Cl-4-F-Ph | 4-Pyr | $SO_2Pr$ |
| 10-548 | 3-Cl-4-F-Ph | 4-Pyr | $SO_2$-i-Pr |
| 10-549 | 3-Cl-4-F-Ph | 4-Pyr | $SO_2Bu$ |
| 10-550 | 3-Cl-4-F-Ph | 4-Pyr | $SO_2CF_3$ |
| 10-551 | 3-Cl-4-F-Ph | 4-Pyr | $SO_2$-$(CH_2)_2$-F |
| 10-552 | 3-Cl-4-F-Ph | 4-Pyr | $SO_2$-$(CH_2)_2$-OH |
| 10-553 | 3-Cl-4-F-Ph | 4-Pyr | $SO_2$-$(CH_2)_2$-OMe |
| 10-554 | 3-Cl-4-F-Ph | 4-Pyr | $SO_2NH_2$ |
| 10-555 | 3-Cl-4-F-Ph | 4-Pyr | $SO_2NHMe$ |
| 10-556 | 3-Cl-4-F-Ph | 4-Pyr | $SO_2NHEt$ |
| 10-557 | 3-Cl-4-F-Ph | 4-Pyr | $SO_2NHPr$ |
| 10-558 | 3-Cl-4-F-Ph | 4-Pyr | $SO_2NH$-i-Pr |
| 10-559 | 3-Cl-4-F-Ph | 4-Pyr | $SO_2NHBu$ |
| 10-560 | 3-Cl-4-F-Ph | 4-Pyr | $SO_2NHBn$ |
| 10-561 | 3-Cl-4-F-Ph | 4-Pyr | $SO_2NMe_2$ |
| 10-562 | 3-Cl-4-F-Ph | 4-Pyr | $SO_2NH$-c-Pr |
| 10-563 | 3-Cl-4-F-Ph | 4-Pyr | $SO_2NH$-c-Bu |
| 10-564 | 3-Cl-4-F-Ph | 4-Pyr | $SO_2NH$-c-Pen |
| 10-565 | 3-Cl-4-F-Ph | 4-Pyr | $SO_2NH$-c-Hex |
| 10-566 | 3-Cl-4-F-Ph | 4-Pyr | $SO_2NH$-c-Hep |

| | | | |
|---|---|---|---|
| 10-567 | 3-Cl-4-F-Ph | 4-Pyr | $SO_2NHCH_2$-c-Pr |
| 10-568 | 3-Cl-4-F-Ph | 4-Pyr | $SO_2NH$-Allyl |
| 10-569 | 3-Cl-4-F-Ph | 4-Pyr | $SO_2NH$-Propargyl |
| 10-570 | 3-Cl-4-F-Ph | 4-Pyr | $SO_2NHPh$ |
| 10-571 | 3-Cl-4-F-Ph | 4-Pyr | $SO_2NH$-3-Pyr |
| 10-572 | 3-Cl-4-F-Ph | 4-Pyr | $SO_2NHCH_2$-4-Pyr |
| 10-573 | 3-Cl-4-F-Ph | 4-Pyr | $SO_2NH-(CH_2)_2-OH$ |
| 10-574 | 3-Cl-4-F-Ph | 4-Pyr | $SO_2NHCH(CH_2OH)_2$ |
| 10-575 | 3-Cl-4-F-Ph | 4-Pyr | $SO_2NH-(CH_2)_2-OAc$ |
| 10-576 | 3-Cl-4-F-Ph | 4-Pyr | $SO_2NHCH_2CN$ |
| 10-577 | 3-Cl-4-F-Ph | 4-Pyr | $SO_2NH-(CH_2)_2-F$ |
| 10-578 | 3-Cl-4-F-Ph | 4-Pyr | $SO_2NH-(CH_2)_2-OMe$ |
| 10-579 | 3-Cl-4-F-Ph | 4-Pyr | $SO_2NH-(CH_2)_2-SMe$ |
| 10-580 | 3-Cl-4-F-Ph | 4-Pyr | $SO_2NH-(CH_2)_2-NH_2$ |
| 10-581 | 3-Cl-4-F-Ph | 4-Pyr | $SO_2NH-(CH_2)_2-NHMe$ |
| 10-582 | 3-Cl-4-F-Ph | 4-Pyr | $SO_2NH-(CH_2)_2-N(Me)_2$ |
| 10-583 | 3-Cl-4-F-Ph | 4-Pyr | $SO_2NHCH_2COOH$ |
| 10-584 | 3-Cl-4-F-Ph | 4-Pyr | $SO_2NHCH_2COOEt$ |
| 10-585 | 3-Cl-4-F-Ph | 4-Pyr | $SO_2NHCH(Me)COOEt$ |
| 10-586 | 3-Cl-4-F-Ph | 4-Pyr | $SO_2NHCH_2CONH_2$ |
| 10-587 | 3-Cl-4-F-Ph | 4-Pyr | $SO_2NHOH$ |
| 10-588 | 3-Cl-4-F-Ph | 4-Pyr | $SO_2NHOMe$ |
| 10-589 | 3-Cl-4-F-Ph | 4-Pyr | $SO_2NHOEt$ |
| 10-590 | 3-Cl-4-F-Ph | 4-Pyr | $SO_2NHOPr$ |
| 10-591 | 3-Cl-4-F-Ph | 4-Pyr | $SO_2NHOAllyl$ |
| 10-592 | 3-Cl-4-F-Ph | 4-Pyr | $SO_2NHOBn$ |
| 10-593 | 3-Cl-4-F-Ph | 4-Pyr | $SO_2NHNH_2$ |
| 10-594 | 3-Cl-4-F-Ph | 4-Pyr | $SO_2NHNHMe$ |
| 10-595 | 3-Cl-4-F-Ph | 4-Pyr | $SO_2NHN(Me)_2$ |
| 10-596 | 3,4-$F_2$-Ph | 4-Pyr | COOH |
| 10-597 | 3,4-$F_2$-Ph | 4-Pyr | COOMe |
| 10-598 | 3,4-$F_2$-Ph | 4-Pyr | COOEt |
| 10-599 | 3,4-$F_2$-Ph | 4-Pyr | COOPr |
| 10-600 | 3,4-$F_2$-Ph | 4-Pyr | COO-i-Pr |
| 10-601 | 3,4-$F_2$-Ph | 4-Pyr | COOBu |
| 10-602 | 3,4-$F_2$-Ph | 4-Pyr | COOBn |

| | | | |
|---|---|---|---|
| 10-603 | 3,4-$F_2$-Ph | 4-Pyr | COOPh |
| 10-604 | 3,4-$F_2$-Ph | 4-Pyr | $CONH_2$ |
| 10-605 | 3,4-$F_2$-Ph | 4-Pyr | CONHMe |
| 10-606 | 3,4-$F_2$-Ph | 4-Pyr | CONHEt |
| 10-607 | 3,4-$F_2$-Ph | 4-Pyr | CONHPr |
| 10-608 | 3,4-$F_2$-Ph | 4-Pyr | CONH-i-Pr |
| 10-609 | 3,4-$F_2$-Ph | 4-Pyr | CONHBu |
| 10-610 | 3,4-$F_2$-Ph | 4-Pyr | CONHBn |
| 10-611 | 3,4-$F_2$-Ph | 4-Pyr | $CONMe_2$ |
| 10-612 | 3,4-$F_2$-Ph | 4-Pyr | CONH-c-Pr |
| 10-613 | 3,4-$F_2$-Ph | 4-Pyr | CONH-c-Bu |
| 10-614 | 3,4-$F_2$-Ph | 4-Pyr | CONH-c-Pen |
| 10-615 | 3,4-$F_2$-Ph | 4-Pyr | CONH-c-Hex |
| 10-616 | 3,4-$F_2$-Ph | 4-Pyr | CONH-c-Hep |
| 10-617 | 3,4-$F_2$-Ph | 4-Pyr | $CONHCH_2$-c-Pr |
| 10-618 | 3,4-$F_2$-Ph | 4-Pyr | CONH-Allyl |
| 10-619 | 3,4-$F_2$-Ph | 4-Pyr | CONH-Propargyl |
| 10-620 | 3,4-$F_2$-Ph | 4-Pyr | CONHPh |
| 10-621 | 3,4-$F_2$-Ph | 4-Pyr | CONH-3-Pyr |
| 10-622 | 3,4-$F_2$-Ph | 4-Pyr | $CONHCH_2$-4-Pyr |
| 10-623 | 3,4-$F_2$-Ph | 4-Pyr | CONH-$(CH_2)_2$-OH |
| 10-624 | 3,4-$F_2$-Ph | 4-Pyr | $CONHCH(CH_2OH)_2$ |
| 10-625 | 3,4-$F_2$-Ph | 4-Pyr | CONH-$(CH_2)_2$-OAc |
| 10-626 | 3,4-$F_2$-Ph | 4-Pyr | $CONHCH_2CN$ |
| 10-627 | 3,4-$F_2$-Ph | 4-Pyr | CONH-$(CH_2)_2$-F |
| 10-628 | 3,4-$F_2$-Ph | 4-Pyr | CONH-$(CH_2)_2$-OMe |
| 10-629 | 3,4-$F_2$-Ph | 4-Pyr | CONH-$(CH_2)_2$-SMe |
| 10-630 | 3,4-$F_2$-Ph | 4-Pyr | CONH-$(CH_2)_2$-$NH_2$ |
| 10-631 | 3,4-$F_2$-Ph | 4-Pyr | CONH-$(CH_2)_2$-NHMe |
| 10-632 | 3,4-$F_2$-Ph | 4-Pyr | CONH-$(CH_2)_2$-$N(Me)_2$ |
| 10-633 | 3,4-$F_2$-Ph | 4-Pyr | $CONHCH_2COOH$ |
| 10-634 | 3,4-$F_2$-Ph | 4-Pyr | $CONHCH_2COOEt$ |
| 10-635 | 3,4-$F_2$-Ph | 4-Pyr | CONHCH(Me)COOEt |
| 10-636 | 3,4-$F_2$-Ph | 4-Pyr | $CONHCH_2CONH_2$ |
| 10-637 | 3,4-$F_2$-Ph | 4-Pyr | CONHOH |
| 10-638 | 3,4-$F_2$-Ph | 4-Pyr | CONHOMe |

| | | | |
|---|---|---|---|
| 10-639 | 3,4-$F_2$-Ph | 4-Pyr | CONHOEt |
| 10-640 | 3,4-$F_2$-Ph | 4-Pyr | CONHOPr |
| 10-641 | 3,4-$F_2$-Ph | 4-Pyr | CONHOAllyl |
| 10-642 | 3,4-$F_2$-Ph | 4-Pyr | CONHOBn |
| 10-643 | 3,4-$F_2$-Ph | 4-Pyr | $CONHNH_2$ |
| 10-644 | 3,4-$F_2$-Ph | 4-Pyr | CONHNHMe |
| 10-645 | 3,4-$F_2$-Ph | 4-Pyr | $CONHN(Me)_2$ |
| 10-646 | 3,4-$F_2$-Ph | 4-Pyr | COMe |
| 10-647 | 3,4-$F_2$-Ph | 4-Pyr | COEt |
| 10-648 | 3,4-$F_2$-Ph | 4-Pyr | COPr |
| 10-649 | 3,4-$F_2$-Ph | 4-Pyr | CO-i-Pr |
| 10-650 | 3,4-$F_2$-Ph | 4-Pyr | COBu |
| 10-651 | 3,4-$F_2$-Ph | 4-Pyr | $COCF_3$ |
| 10-652 | 3,4-$F_2$-Ph | 4-Pyr | CO-$(CH_2)_2$-F |
| 10-653 | 3,4-$F_2$-Ph | 4-Pyr | CO-$(CH_2)_2$-OH |
| 10-654 | 3,4-$F_2$-Ph | 4-Pyr | CO-$(CH_2)_2$-OMe |
| 10-655 | 3,4-$F_2$-Ph | 4-Pyr | SOMe |
| 10-656 | 3,4-$F_2$-Ph | 4-Pyr | SOEt |
| 10-657 | 3,4-$F_2$-Ph | 4-Pyr | SOPr |
| 10-658 | 3,4-$F_2$-Ph | 4-Pyr | SO-i-Pr |
| 10-659 | 3,4-$F_2$-Ph | 4-Pyr | SOBu |
| 10-660 | 3,4-$F_2$-Ph | 4-Pyr | $SOCF_3$ |
| 10-661 | 3,4-$F_2$-Ph | 4-Pyr | SO-$(CH_2)_2$-F |
| 10-662 | 3,4-$F_2$-Ph | 4-Pyr | SO-$(CH_2)_2$-OH |
| 10-663 | 3,4-$F_2$-Ph | 4-Pyr | SO-$(CH_2)_2$-OMe |
| 10-664 | 3,4-$F_2$-Ph | 4-Pyr | $SO_2Me$ |
| 10-665 | 3,4-$F_2$-Ph | 4-Pyr | $SO_2Et$ |
| 10-666 | 3,4-$F_2$-Ph | 4-Pyr | $SO_2Pr$ |
| 10-667 | 3,4-$F_2$-Ph | 4-Pyr | $SO_2$-i-Pr |
| 10-668 | 3,4-$F_2$-Ph | 4-Pyr | $SO_2Bu$ |
| 10-669 | 3,4-$F_2$-Ph | 4-Pyr | $SO_2CF_3$ |
| 10-670 | 3,4-$F_2$-Ph | 4-Pyr | $SO_2$-$(CH_2)_2$-F |
| 10-671 | 3,4-$F_2$-Ph | 4-Pyr | $SO_2$-$(CH_2)_2$-OH |
| 10-672 | 3,4-$F_2$-Ph | 4-Pyr | $SO_2$-$(CH_2)_2$-OMe |
| 10-673 | 3,4-$F_2$-Ph | 4-Pyr | $SO_2NH_2$ |
| 10-674 | 3,4-$F_2$-Ph | 4-Pyr | $SO_2NHMe$ |

| | | | |
|---|---|---|---|
| 10-675 | 3,4-$F_2$-Ph | 4-Pyr | $SO_2$NHEt |
| 10-676 | 3,4-$F_2$-Ph | 4-Pyr | $SO_2$NHPr |
| 10-677 | 3,4-$F_2$-Ph | 4-Pyr | $SO_2$NH-i-Pr |
| 10-678 | 3,4-$F_2$-Ph | 4-Pyr | $SO_2$NHBu |
| 10-679 | 3,4-$F_2$-Ph | 4-Pyr | $SO_2$NHBn |
| 10-680 | 3,4-$F_2$-Ph | 4-Pyr | $SO_2NMe_2$ |
| 10-681 | 3,4-$F_2$-Ph | 4-Pyr | $SO_2$NH-c-Pr |
| 10-682 | 3,4-$F_2$-Ph | 4-Pyr | $SO_2$NH-c-Bu |
| 10-683 | 3,4-$F_2$-Ph | 4-Pyr | $SO_2$NH-c-Pen |
| 10-684 | 3,4-$F_2$-Ph | 4-Pyr | $SO_2$NH-c-Hex |
| 10-685 | 3,4-$F_2$-Ph | 4-Pyr | $SO_2$NH-c-Hep |
| 10-686 | 3,4-$F_2$-Ph | 4-Pyr | $SO_2NHCH_2$-c-Pr |
| 10-687 | 3,4-$F_2$-Ph | 4-Pyr | $SO_2$NH-Allyl |
| 10-688 | 3,4-$F_2$-Ph | 4-Pyr | $SO_2$NH-Propargyl |
| 10-689 | 3,4-$F_2$-Ph | 4-Pyr | $SO_2$NHPh |
| 10-690 | 3,4-$F_2$-Ph | 4-Pyr | $SO_2$NH-3-Pyr |
| 10-691 | 3,4-$F_2$-Ph | 4-Pyr | $SO_2NHCH_2$-4-Pyr |
| 10-692 | 3,4-$F_2$-Ph | 4-Pyr | $SO_2NH-(CH_2)_2$-OH |
| 10-693 | 3,4-$F_2$-Ph | 4-Pyr | $SO_2NHCH(CH_2OH)_2$ |
| 10-694 | 3,4-$F_2$-Ph | 4-Pyr | $SO_2NH-(CH_2)_2$-OAc |
| 10-695 | 3,4-$F_2$-Ph | 4-Pyr | $SO_2NHCH_2CN$ |
| 10-696 | 3,4-$F_2$-Ph | 4-Pyr | $SO_2NH-(CH_2)_2$-F |
| 10-697 | 3,4-$F_2$-Ph | 4-Pyr | $SO_2NH-(CH_2)_2$-OMe |
| 10-698 | 3,4-$F_2$-Ph | 4-Pyr | $SO_2NH-(CH_2)_2$-SMe |
| 10-699 | 3,4-$F_2$-Ph | 4-Pyr | $SO_2NH-(CH_2)_2-NH_2$ |
| 10-700 | 3,4-$F_2$-Ph | 4-Pyr | $SO_2NH-(CH_2)_2$-NHMe |
| 10-701 | 3,4-$F_2$-Ph | 4-Pyr | $SO_2NH-(CH_2)_2-N(Me)_2$ |
| 10-702 | 3,4-$F_2$-Ph | 4-Pyr | $SO_2NHCH_2COOH$ |
| 10-703 | 3,4-$F_2$-Ph | 4-Pyr | $SO_2NHCH_2COOEt$ |
| 10-704 | 3,4-$F_2$-Ph | 4-Pyr | $SO_2$NHCH(Me)COOEt |
| 10-705 | 3,4-$F_2$-Ph | 4-Pyr | $SO_2NHCH_2CONH_2$ |
| 10-706 | 3,4-$F_2$-Ph | 4-Pyr | $SO_2$NHOH |
| 10-707 | 3,4-$F_2$-Ph | 4-Pyr | $SO_2$NHOMe |
| 10-708 | 3,4-$F_2$-Ph | 4-Pyr | $SO_2$NHOEt |
| 10-709 | 3,4-$F_2$-Ph | 4-Pyr | $SO_2$NHOPr |
| 10-710 | 3,4-$F_2$-Ph | 4-Pyr | $SO_2$NHOAllyl |

| | | | |
|---|---|---|---|
| 10-711 | 3,4-$F_2$-Ph | 4-Pyr | $SO_2$NHOBn |
| 10-712 | 3,4-$F_2$-Ph | 4-Pyr | $SO_2NHNH_2$ |
| 10-713 | 3,4-$F_2$-Ph | 4-Pyr | $SO_2$NHNHMe |
| 10-714 | 3,4-$F_2$-Ph | 4-Pyr | $SO_2$NHN(Me)$_2$ |
| 10-715 | 3-$CF_3$-Ph | 4-Pyr | COOH |
| 10-716 | 3-$CF_3$-Ph | 4-Pyr | COOMe |
| 10-717 | 3-$CF_3$-Ph | 4-Pyr | COOEt |
| 10-718 | 3-$CF_3$-Ph | 4-Pyr | COOPr |
| 10-719 | 3-$CF_3$-Ph | 4-Pyr | COO-i-Pr |
| 10-720 | 3-$CF_3$-Ph | 4-Pyr | COOBu |
| 10-721 | 3-$CF_3$-Ph | 4-Pyr | COOBn |
| 10-722 | 3-$CF_3$-Ph | 4-Pyr | COOPh |
| 10-723 | 3-$CF_3$-Ph | 4-Pyr | $CONH_2$ |
| 10-724 | 3-$CF_3$-Ph | 4-Pyr | CONHMe |
| 10-725 | 3-$CF_3$-Ph | 4-Pyr | CONHEt |
| 10-726 | 3-$CF_3$-Ph | 4-Pyr | CONHPr |
| 10-727 | 3-$CF_3$-Ph | 4-Pyr | CONH-i-Pr |
| 10-728 | 3-$CF_3$-Ph | 4-Pyr | CONHBu |
| 10-729 | 3-$CF_3$-Ph | 4-Pyr | CONHBn |
| 10-730 | 3-$CF_3$-Ph | 4-Pyr | $CONMe_2$ |
| 10-731 | 3-$CF_3$-Ph | 4-Pyr | CONH-c-Pr |
| 10-732 | 3-$CF_3$-Ph | 4-Pyr | CONH-c-Bu |
| 10-733 | 3-$CF_3$-Ph | 4-Pyr | CONH-c-Pen |
| 10-734 | 3-$CF_3$-Ph | 4-Pyr | CONH-c-Hex |
| 10-735 | 3-$CF_3$-Ph | 4-Pyr | CONH-c-Hep |
| 10-736 | 3-$CF_3$-Ph | 4-Pyr | $CONHCH_2$-c-Pr |
| 10-737 | 3-$CF_3$-Ph | 4-Pyr | CONH-Allyl |
| 10-738 | 3-$CF_3$-Ph | 4-Pyr | CONH-Propargyl |
| 10-739 | 3-$CF_3$-Ph | 4-Pyr | CONHPh |
| 10-740 | 3-$CF_3$-Ph | 4-Pyr | CONH-3-Pyr |
| 10-741 | 3-$CF_3$-Ph | 4-Pyr | $CONHCH_2$-4-Pyr |
| 10-742 | 3-$CF_3$-Ph | 4-Pyr | CONH-$(CH_2)_2$-OH |
| 10-743 | 3-$CF_3$-Ph | 4-Pyr | $CONHCH(CH_2OH)_2$ |
| 10-744 | 3-$CF_3$-Ph | 4-Pyr | CONH-$(CH_2)_2$-OAc |
| 10-745 | 3-$CF_3$-Ph | 4-Pyr | $CONHCH_2CN$ |
| 10-746 | 3-$CF_3$-Ph | 4-Pyr | CONH-$(CH_2)_2$-F |

| | | | |
|---|---|---|---|
| 10-747 | 3-$CF_3$-Ph | 4-Pyr | CONH-$(CH_2)_2$-OMe |
| 10-748 | 3-$CF_3$-Ph | 4-Pyr | CONH-$(CH_2)_2$-SMe |
| 10-749 | 3-$CF_3$-Ph | 4-Pyr | CONH-$(CH_2)_2$-$NH_2$ |
| 10-750 | 3-$CF_3$-Ph | 4-Pyr | CONH-$(CH_2)_2$-NHMe |
| 10-751 | 3-$CF_3$-Ph | 4-Pyr | CONH-$(CH_2)_2$-N$(Me)_2$ |
| 10-752 | 3-$CF_3$-Ph | 4-Pyr | $CONHCH_2COOH$ |
| 10-753 | 3-$CF_3$-Ph | 4-Pyr | $CONHCH_2COOEt$ |
| 10-754 | 3-$CF_3$-Ph | 4-Pyr | CONHCH(Me)COOEt |
| 10-755 | 3-$CF_3$-Ph | 4-Pyr | $CONHCH_2CONH_2$ |
| 10-756 | 3-$CF_3$-Ph | 4-Pyr | CONHOH |
| 10-757 | 3-$CF_3$-Ph | 4-Pyr | CONHOMe |
| 10-758 | 3-$CF_3$-Ph | 4-Pyr | CONHOEt |
| 10-759 | 3-$CF_3$-Ph | 4-Pyr | CONHOPr |
| 10-760 | 3-$CF_3$-Ph | 4-Pyr | CONHOAllyl |
| 10-761 | 3-$CF_3$-Ph | 4-Pyr | CONHOBn |
| 10-762 | 3-$CF_3$-Ph | 4-Pyr | $CONHNH_2$ |
| 10-763 | 3-$CF_3$-Ph | 4-Pyr | CONHNHMe |
| 10-764 | 3-$CF_3$-Ph | 4-Pyr | CONHN$(Me)_2$ |
| 10-765 | 3-$CF_3$-Ph | 4-Pyr | COMe |
| 10-766 | 3-$CF_3$-Ph | 4-Pyr | COEt |
| 10-767 | 3-$CF_3$-Ph | 4-Pyr | COPr |
| 10-768 | 3-$CF_3$-Ph | 4-Pyr | CO-i-Pr |
| 10-769 | 3-$CF_3$-Ph | 4-Pyr | COBu |
| 10-770 | 3-$CF_3$-Ph | 4-Pyr | $COCF_3$ |
| 10-771 | 3-$CF_3$-Ph | 4-Pyr | CO-$(CH_2)_2$-F |
| 10-772 | 3-$CF_3$-Ph | 4-Pyr | CO-$(CH_2)_2$-OH |
| 10-773 | 3-$CF_3$-Ph | 4-Pyr | CO-$(CH_2)_2$-OMe |
| 10-774 | 3-$CF_3$-Ph | 4-Pyr | SOMe |
| 10-775 | 3-$CF_3$-Ph | 4-Pyr | SOEt |
| 10-776 | 3-$CF_3$-Ph | 4-Pyr | SOPr |
| 10-777 | 3-$CF_3$-Ph | 4-Pyr | SO-i-Pr |
| 10-778 | 3-$CF_3$-Ph | 4-Pyr | SOBu |
| 10-779 | 3-$CF_3$-Ph | 4-Pyr | $SOCF_3$ |
| 10-780 | 3-$CF_3$-Ph | 4-Pyr | SO-$(CH_2)_2$-F |
| 10-781 | 3-$CF_3$-Ph | 4-Pyr | SO-$(CH_2)_2$-OH |
| 10-782 | 3-$CF_3$-Ph | 4-Pyr | SO-$(CH_2)_2$-OMe |

| | | | |
|---|---|---|---|
| 10-783 | 3-$CF_3$-Ph | 4-Pyr | $SO_2Me$ |
| 10-784 | 3-$CF_3$-Ph | 4-Pyr | $SO_2Et$ |
| 10-785 | 3-$CF_3$-Ph | 4-Pyr | $SO_2Pr$ |
| 10-786 | 3-$CF_3$-Ph | 4-Pyr | $SO_2$-i-Pr |
| 10-787 | 3-$CF_3$-Ph | 4-Pyr | $SO_2Bu$ |
| 10-788 | 3-$CF_3$-Ph | 4-Pyr | $SO_2CF_3$ |
| 10-789 | 3-$CF_3$-Ph | 4-Pyr | $SO_2$-$(CH_2)_2$-F |
| 10-790 | 3-$CF_3$-Ph | 4-Pyr | $SO_2$-$(CH_2)_2$-OH |
| 10-791 | 3-$CF_3$-Ph | 4-Pyr | $SO_2$-$(CH_2)_2$-OMe |
| 10-792 | 3-$CF_3$-Ph | 4-Pyr | $SO_2NH_2$ |
| 10-793 | 3-$CF_3$-Ph | 4-Pyr | $SO_2NHMe$ |
| 10-794 | 3-$CF_3$-Ph | 4-Pyr | $SO_2NHEt$ |
| 10-795 | 3-$CF_3$-Ph | 4-Pyr | $SO_2NHPr$ |
| 10-796 | 3-$CF_3$-Ph | 4-Pyr | $SO_2NH$-i-Pr |
| 10-797 | 3-$CF_3$-Ph | 4-Pyr | $SO_2NHBu$ |
| 10-798 | 3-$CF_3$-Ph | 4-Pyr | $SO_2NHBn$ |
| 10-799 | 3-$CF_3$-Ph | 4-Pyr | $SO_2NMe_2$ |
| 10-800 | 3-$CF_3$-Ph | 4-Pyr | $SO_2NH$-c-Pr |
| 10-801 | 3-$CF_3$-Ph | 4-Pyr | $SO_2NH$-c-Bu |
| 10-802 | 3-$CF_3$-Ph | 4-Pyr | $SO_2NH$-c-Pen |
| 10-803 | 3-$CF_3$-Ph | 4-Pyr | $SO_2NH$-c-Hex |
| 10-804 | 3-$CF_3$-Ph | 4-Pyr | $SO_2NH$-c-Hep |
| 10-805 | 3-$CF_3$-Ph | 4-Pyr | $SO_2NHCH_2$-c-Pr |
| 10-806 | 3-$CF_3$-Ph | 4-Pyr | $SO_2NH$-Allyl |
| 10-807 | 3-$CF_3$-Ph | 4-Pyr | $SO_2NH$-Propargyl |
| 10-808 | 3-$CF_3$-Ph | 4-Pyr | $SO_2NHPh$ |
| 10-809 | 3-$CF_3$-Ph | 4-Pyr | $SO_2NH$-3-Pyr |
| 10-810 | 3-$CF_3$-Ph | 4-Pyr | $SO_2NHCH_2$-4-Pyr |
| 10-811 | 3-$CF_3$-Ph | 4-Pyr | $SO_2NH$-$(CH_2)_2$-OH |
| 10-812 | 3-$CF_3$-Ph | 4-Pyr | $SO_2NHCH(CH_2OH)_2$ |
| 10-813 | 3-$CF_3$-Ph | 4-Pyr | $SO_2NH$-$(CH_2)_2$-OAc |
| 10-814 | 3-$CF_3$-Ph | 4-Pyr | $SO_2NHCH_2CN$ |
| 10-815 | 3-$CF_3$-Ph | 4-Pyr | $SO_2NH$-$(CH_2)_2$-F |
| 10-816 | 3-$CF_3$-Ph | 4-Pyr | $SO_2NH$-$(CH_2)_2$-OMe |
| 10-817 | 3-$CF_3$-Ph | 4-Pyr | $SO_2NH$-$(CH_2)_2$-SMe |
| 10-818 | 3-$CF_3$-Ph | 4-Pyr | $SO_2NH$-$(CH_2)_2$-$NH_2$ |

| | | | |
|---|---|---|---|
| 10-819 | 3-$CF_3$-Ph | 4-Pyr | $SO_2NH-(CH_2)_2$-NHMe |
| 10-820 | 3-$CF_3$-Ph | 4-Pyr | $SO_2NH-(CH_2)_2$-N(Me)$_2$ |
| 10-821 | 3-$CF_3$-Ph | 4-Pyr | $SO_2NHCH_2COOH$ |
| 10-822 | 3-$CF_3$-Ph | 4-Pyr | $SO_2NHCH_2COOEt$ |
| 10-823 | 3-$CF_3$-Ph | 4-Pyr | $SO_2NHCH(Me)COOEt$ |
| 10-824 | 3-$CF_3$-Ph | 4-Pyr | $SO_2NHCH_2CONH_2$ |
| 10-825 | 3-$CF_3$-Ph | 4-Pyr | $SO_2NHOH$ |
| 10-826 | 3-$CF_3$-Ph | 4-Pyr | $SO_2NHOMe$ |
| 10-827 | 3-$CF_3$-Ph | 4-Pyr | $SO_2NHOEt$ |
| 10-828 | 3-$CF_3$-Ph | 4-Pyr | $SO_2NHOPr$ |
| 10-829 | 3-$CF_3$-Ph | 4-Pyr | $SO_2NHOAllyl$ |
| 10-830 | 3-$CF_3$-Ph | 4-Pyr | $SO_2NHOBn$ |
| 10-831 | 3-$CF_3$-Ph | 4-Pyr | $SO_2NHNH_2$ |
| 10-832 | 3-$CF_3$-Ph | 4-Pyr | $SO_2NHNHMe$ |
| 10-833 | 3-$CF_3$-Ph | 4-Pyr | $SO_2NHN(Me)_2$ |
| 10-834 | 4-F-Ph | 4-Pym | $CONH_2$ |
| 10-835 | 4-F-Ph | 4-Pym | CONHMe |
| 10-836 | 4-F-Ph | 4-Pym | CONHEt |
| 10-837 | 4-F-Ph | 4-Pym | CONHPr |
| 10-838 | 4-F-Ph | 4-Pym | CONH-i-Pr |
| 10-839 | 4-F-Ph | 4-Pym | CONHBu |
| 10-840 | 4-F-Ph | 4-Pym | CONHBn |
| 10-841 | 4-F-Ph | 4-Pym | $CONMe_2$ |
| 10-842 | 4-F-Ph | 4-Pym | CONH-c-Pr |
| 10-843 | 4-F-Ph | 4-Pym | CONH-c-Bu |
| 10-844 | 4-F-Ph | 4-Pym | CONH-c-Pen |
| 10-845 | 4-F-Ph | 4-Pym | CONH-c-Hex |
| 10-846 | 4-F-Ph | 4-Pym | CONH-c-Hep |
| 10-847 | 4-F-Ph | 4-Pym | $CONHCH_2$-c-Pr |
| 10-848 | 4-F-Ph | 4-Pym | CONH-Allyl |
| 10-849 | 4-F-Ph | 4-Pym | CONH-Propargyl |
| 10-850 | 4-F-Ph | 4-Pym | CONHPh |
| 10-851 | 4-F-Ph | 4-Pym | CONH-3-Pyr |
| 10-852 | 4-F-Ph | 4-Pym | $CONHCH_2$-4-Pyr |
| 10-853 | 4-F-Ph | 4-Pym | CONH-$(CH_2)_2$-OH |
| 10-854 | 4-F-Ph | 4-Pym | $CONHCH(CH_2OH)_2$ |

| | | | |
|---|---|---|---|
| 10-855 | 4-F-Ph | 4-Pym | CONH-$(CH_2)_2$-OAc |
| 10-856 | 4-F-Ph | 4-Pym | $CONHCH_2CN$ |
| 10-857 | 4-F-Ph | 4-Pym | CONH-$(CH_2)_2$-F |
| 10-858 | 4-F-Ph | 4-Pym | CONH-$(CH_2)_2$-OMe |
| 10-859 | 4-F-Ph | 4-Pym | CONH-$(CH_2)_2$-SMe |
| 10-860 | 4-F-Ph | 4-Pym | CONH-$(CH_2)_2$-$NH_2$ |
| 10-861 | 4-F-Ph | 4-Pym | CONH-$(CH_2)_2$-NHMe |
| 10-862 | 4-F-Ph | 4-Pym | CONH-$(CH_2)_2$-N$(Me)_2$ |
| 10-863 | 4-F-Ph | 4-Pym | $CONHCH_2COOH$ |
| 10-864 | 4-F-Ph | 4-Pym | $CONHCH_2COOEt$ |
| 10-865 | 4-F-Ph | 4-Pym | CONHCH(Me)COOEt |
| 10-866 | 4-F-Ph | 4-Pym | $CONHCH_2CONH_2$ |
| 10-867 | 4-F-Ph | 4-Pym | $SO_2NH_2$ |
| 10-868 | 4-F-Ph | 4-Pym | $SO_2NHMe$ |
| 10-869 | 4-F-Ph | 4-Pym | $SO_2NHEt$ |
| 10-870 | 4-F-Ph | 4-Pym | $SO_2NHPr$ |
| 10-871 | 4-F-Ph | 4-Pym | $SO_2NH$-i-Pr |
| 10-872 | 4-F-Ph | 4-Pym | $SO_2NHBu$ |
| 10-873 | 4-F-Ph | 4-Pym | $SO_2NHBn$ |
| 10-874 | 4-F-Ph | 4-Pym | $SO_2NMe_2$ |
| 10-875 | 4-F-Ph | 4-Pym | $SO_2NH$-c-Pr |
| 10-876 | 4-F-Ph | 4-Pym | $SO_2NH$-c-Bu |
| 10-877 | 4-F-Ph | 4-Pym | $SO_2NH$-c-Pen |
| 10-878 | 4-F-Ph | 4-Pym | $SO_2NH$-c-Hex |
| 10-879 | 4-F-Ph | 4-Pym | $SO_2NH$-c-Hep |
| 10-880 | 4-F-Ph | 4-Pym | $SO_2NHCH_2$-c-Pr |
| 10-881 | 4-F-Ph | 4-Pym | $SO_2NH$-Allyl |
| 10-882 | 4-F-Ph | 4-Pym | $SO_2NH$-Propargyl |
| 10-883 | 4-F-Ph | 4-Pym | $SO_2NHPh$ |
| 10-884 | 4-F-Ph | 4-Pym | $SO_2NH$-3-Pyr |
| 10-885 | 4-F-Ph | 4-Pym | $SO_2NHCH_2$-4-Pyr |
| 10-886 | 4-F-Ph | 4-Pym | $SO_2NH$-$(CH_2)_2$-OH |
| 10-887 | 4-F-Ph | 4-Pym | $SO_2NHCH(CH_2OH)_2$ |
| 10-888 | 4-F-Ph | 4-Pym | $SO_2NH$-$(CH_2)_2$-OAc |
| 10-889 | 4-F-Ph | 4-Pym | $SO_2NHCH_2CN$ |
| 10-890 | 4-F-Ph | 4-Pym | $SO_2NH$-$(CH_2)_2$-F |

| | | | |
|---|---|---|---|
| 10-891 | 4-F-Ph | 4-Pym | $SO_2NH-(CH_2)_2-OMe$ |
| 10-892 | 4-F-Ph | 4-Pym | $SO_2NH-(CH_2)_2-SMe$ |
| 10-893 | 4-F-Ph | 4-Pym | $SO_2NH-(CH_2)_2-NH_2$ |
| 10-894 | 4-F-Ph | 4-Pym | $SO_2NH-(CH_2)_2-NHMe$ |
| 10-895 | 4-F-Ph | 4-Pym | $SO_2NH-(CH_2)_2-N(Me)_2$ |
| 10-896 | 4-F-Ph | 4-Pym | $SO_2NHCH_2COOH$ |
| 10-897 | 4-F-Ph | 4-Pym | $SO_2NHCH_2COOEt$ |
| 10-898 | 4-F-Ph | 4-Pym | $SO_2NHCH(Me)COOEt$ |
| 10-899 | 4-F-Ph | 4-Pym | $SO_2NHCH_2CONH_2$ |
| 10-900 | 4-F-Ph | 4-Pym | $SO_2NHOH$ |
| 10-901 | 4-F-Ph | 4-Pym | $SO_2NHOMe$ |
| 10-902 | 4-F-Ph | 4-Pym | $SO_2NHOEt$ |
| 10-903 | 4-F-Ph | 4-Pym | $SO_2NHOPr$ |
| 10-904 | 4-F-Ph | 4-Pym | $SO_2NHOAllyl$ |
| 10-905 | 4-F-Ph | 4-Pym | $SO_2NHOBn$ |
| 10-906 | 4-F-Ph | 4-Pym | $SO_2NHNH_2$ |
| 10-907 | 4-F-Ph | 4-Pym | $SO_2NHNHMe$ |
| 10-908 | 4-F-Ph | 4-Pym | $SO_2NHN(Me)_2$ |
| 10-909 | 4-F-Ph | 2-MeO-4-Pym | $CONH_2$ |
| 10-910 | 4-F-Ph | 2-MeO-4-Pym | CONHMe |
| 10-911 | 4-F-Ph | 2-MeO-4-Pym | CONHEt |
| 10-912 | 4-F-Ph | 2-MeO-4-Pym | CONHPr |
| 10-913 | 4-F-Ph | 2-MeO-4-Pym | CONH-i-Pr |
| 10-914 | 4-F-Ph | 2-MeO-4-Pym | CONHBu |
| 10-915 | 4-F-Ph | 2-MeO-4-Pym | CONHBn |
| 10-916 | 4-F-Ph | 2-MeO-4-Pym | $CONMe_2$ |
| 10-917 | 4-F-Ph | 2-MeO-4-Pym | CONH-c-Pr |
| 10-918 | 4-F-Ph | 2-MeO-4-Pym | CONH-c-Bu |
| 10-919 | 4-F-Ph | 2-MeO-4-Pym | CONH-c-Pen |
| 10-920 | 4-F-Ph | 2-MeO-4-Pym | CONH-c-Hex |
| 10-921 | 4-F-Ph | 2-MeO-4-Pym | CONH-c-Hep |
| 10-922 | 4-F-Ph | 2-MeO-4-Pym | $CONHCH_2$-c-Pr |
| 10-923 | 4-F-Ph | 2-MeO-4-Pym | CONH-Allyl |
| 10-924 | 4-F-Ph | 2-MeO-4-Pym | CONH-Propargyl |
| 10-925 | 4-F-Ph | 2-MeO-4-Pym | CONHPh |
| 10-926 | 4-F-Ph | 2-MeO-4-Pym | CONH-3-Pyr |

| | | | |
|---|---|---|---|
| 10-927 | 4-F-Ph | 2-MeO-4-Pym | $CONHCH_2$-4-Pyr |
| 10-928 | 4-F-Ph | 2-MeO-4-Pym | CONH-$(CH_2)_2$-OH |
| 10-929 | 4-F-Ph | 2-MeO-4-Pym | $CONHCH(CH_2OH)_2$ |
| 10-930 | 4-F-Ph | 2-MeO-4-Pym | CONH-$(CH_2)_2$-OAc |
| 10-931 | 4-F-Ph | 2-MeO-4-Pym | $CONHCH_2CN$ |
| 10-932 | 4-F-Ph | 2-MeO-4-Pym | CONH-$(CH_2)_2$-F |
| 10-933 | 4-F-Ph | 2-MeO-4-Pym | CONH-$(CH_2)_2$-OMe |
| 10-934 | 4-F-Ph | 2-MeO-4-Pym | CONH-$(CH_2)_2$-SMe |
| 10-935 | 4-F-Ph | 2-MeO-4-Pym | CONH-$(CH_2)_2$-$NH_2$ |
| 10-936 | 4-F-Ph | 2-MeO-4-Pym | CONH-$(CH_2)_2$-NHMe |
| 10-937 | 4-F-Ph | 2-MeO-4-Pym | CONH-$(CH_2)_2$-$N(Me)_2$ |
| 10-938 | 4-F-Ph | 2-MeO-4-Pym | $CONHCH_2COOH$ |
| 10-939 | 4-F-Ph | 2-MeO-4-Pym | $CONHCH_2COOEt$ |
| 10-940 | 4-F-Ph | 2-MeO-4-Pym | CONHCH(Me)COOEt |
| 10-941 | 4-F-Ph | 2-MeO-4-Pym | $CONHCH_2CONH_2$ |
| 10-942 | 4-F-Ph | 2-MeO-4-Pym | $SO_2NH_2$ |
| 10-943 | 4-F-Ph | 2-MeO-4-Pym | $SO_2NHMe$ |
| 10-944 | 4-F-Ph | 2-MeO-4-Pym | $SO_2NHEt$ |
| 10-945 | 4-F-Ph | 2-MeO-4-Pym | $SO_2NHPr$ |
| 10-946 | 4-F-Ph | 2-MeO-4-Pym | $SO_2NH$-i-Pr |
| 10-947 | 4-F-Ph | 2-MeO-4-Pym | $SO_2NHBu$ |
| 10-948 | 4-F-Ph | 2-MeO-4-Pym | $SO_2NHBn$ |
| 10-949 | 4-F-Ph | 2-MeO-4-Pym | $SO_2NMe_2$ |
| 10-950 | 4-F-Ph | 2-MeO-4-Pym | $SO_2NH$-c-Pr |
| 10-951 | 4-F-Ph | 2-MeO-4-Pym | $SO_2NH$-c-Bu |
| 10-952 | 4-F-Ph | 2-MeO-4-Pym | $SO_2NH$-c-Pen |
| 10-953 | 4-F-Ph | 2-MeO-4-Pym | $SO_2NH$-c-Hex |
| 10-954 | 4-F-Ph | 2-MeO-4-Pym | $SO_2NH$-c-Hep |
| 10-955 | 4-F-Ph | 2-MeO-4-Pym | $SO_2NHCH_2$-c-Pr |
| 10-956 | 4-F-Ph | 2-MeO-4-Pym | $SO_2NH$-Allyl |
| 10-957 | 4-F-Ph | 2-MeO-4-Pym | $SO_2NH$-Propargyl |
| 10-958 | 4-F-Ph | 2-MeO-4-Pym | $SO_2NHPh$ |
| 10-959 | 4-F-Ph | 2-MeO-4-Pym | $SO_2NH$-3-Pyr |
| 10-960 | 4-F-Ph | 2-MeO-4-Pym | $SO_2NHCH_2$-4-Pyr |
| 10-961 | 4-F-Ph | 2-MeO-4-Pym | $SO_2NH$-$(CH_2)_2$-OH |
| 10-962 | 4-F-Ph | 2-MeO-4-Pym | $SO_2NHCH(CH_2OH)_2$ |

| | | | |
|---|---|---|---|
| 10-963 | 4-F-Ph | 2-MeO-4-Pym | $SO_2NH-(CH_2)_2-OAc$ |
| 10-964 | 4-F-Ph | 2-MeO-4-Pym | $SO_2NHCH_2CN$ |
| 10-965 | 4-F-Ph | 2-MeO-4-Pym | $SO_2NH-(CH_2)_2-F$ |
| 10-966 | 4-F-Ph | 2-MeO-4-Pym | $SO_2NH-(CH_2)_2-OMe$ |
| 10-967 | 4-F-Ph | 2-MeO-4-Pym | $SO_2NH-(CH_2)_2-SMe$ |
| 10-968 | 4-F-Ph | 2-MeO-4-Pym | $SO_2NH-(CH_2)_2-NH_2$ |
| 10-969 | 4-F-Ph | 2-MeO-4-Pym | $SO_2NH-(CH_2)_2-NHMe$ |
| 10-970 | 4-F-Ph | 2-MeO-4-Pym | $SO_2NH-(CH_2)_2-N(Me)_2$ |
| 10-971 | 4-F-Ph | 2-MeO-4-Pym | $SO_2NHCH_2COOH$ |
| 10-972 | 4-F-Ph | 2-MeO-4-Pym | $SO_2NHCH_2COOEt$ |
| 10-973 | 4-F-Ph | 2-MeO-4-Pym | $SO_2NHCH(Me)COOEt$ |
| 10-974 | 4-F-Ph | 2-MeO-4-Pym | $SO_2NHCH_2CONH_2$ |
| 10-975 | 4-F-Ph | 2-MeO-4-Pym | $SO_2NHOH$ |
| 10-976 | 4-F-Ph | 2-MeO-4-Pym | $SO_2NHOMe$ |
| 10-977 | 4-F-Ph | 2-MeO-4-Pym | $SO_2NHOEt$ |
| 10-978 | 4-F-Ph | 2-MeO-4-Pym | $SO_2NHOPr$ |
| 10-979 | 4-F-Ph | 2-MeO-4-Pym | $SO_2NHOAllyl$ |
| 10-980 | 4-F-Ph | 2-MeO-4-Pym | $SO_2NHOBn$ |
| 10-981 | 4-F-Ph | 2-MeO-4-Pym | $SO_2NHNH_2$ |
| 10-982 | 4-F-Ph | 2-MeO-4-Pym | $SO_2NHNHMe$ |
| 10-983 | 4-F-Ph | 2-MeO-4-Pym | $SO_2NHN(Me)_2$ |
| 10-984 | 4-F-Ph | $2-NH_2-4-Pym$ | $CONH_2$ |
| 10-985 | 4-F-Ph | $2-NH_2-4-Pym$ | CONHMe |
| 10-986 | 4-F-Ph | $2-NH_2-4-Pym$ | CONHEt |
| 10-987 | 4-F-Ph | $2-NH_2-4-Pym$ | CONHPr |
| 10-988 | 4-F-Ph | $2-NH_2-4-Pym$ | CONH-i-Pr |
| 10-989 | 4-F-Ph | $2-NH_2-4-Pym$ | CONHBu |
| 10-990 | 4-F-Ph | $2-NH_2-4-Pym$ | CONHBn |
| 10-991 | 4-F-Ph | $2-NH_2-4-Pym$ | $CONMe_2$ |
| 10-992 | 4-F-Ph | $2-NH_2-4-Pym$ | CONH-c-Pr |
| 10-993 | 4-F-Ph | $2-NH_2-4-Pym$ | CONH-c-Bu |
| 10-994 | 4-F-Ph | $2-NH_2-4-Pym$ | CONH-c-Pen |
| 10-995 | 4-F-Ph | $2-NH_2-4-Pym$ | CONH-c-Hex |
| 10-996 | 4-F-Ph | $2-NH_2-4-Pym$ | CONH-c-Hep |
| 10-997 | 4-F-Ph | $2-NH_2-4-Pym$ | $CONHCH_2-c-Pr$ |
| 10-998 | 4-F-Ph | $2-NH_2-4-Pym$ | CONH-Allyl |

| | | | |
|---|---|---|---|
| 10-999 | 4-F-Ph | $2\text{-}NH_2\text{-}4\text{-}Pym$ | CONH-Propargyl |
| 10-1000 | 4-F-Ph | $2\text{-}NH_2\text{-}4\text{-}Pym$ | CONHPh |
| 10-1001 | 4-F-Ph | $2\text{-}NH_2\text{-}4\text{-}Pym$ | CONH-3-Pyr |
| 10-1002 | 4-F-Ph | $2\text{-}NH_2\text{-}4\text{-}Pym$ | $CONHCH_2\text{-}4\text{-}Pyr$ |
| 10-1003 | 4-F-Ph | $2\text{-}NH_2\text{-}4\text{-}Pym$ | $CONH\text{-}(CH_2)_2\text{-}OH$ |
| 10-1004 | 4-F-Ph | $2\text{-}NH_2\text{-}4\text{-}Pym$ | $CONHCH(CH_2OH)_2$ |
| 10-1005 | 4-F-Ph | $2\text{-}NH_2\text{-}4\text{-}Pym$ | $CONH\text{-}(CH_2)_2\text{-}OAc$ |
| 10-1006 | 4-F-Ph | $2\text{-}NH_2\text{-}4\text{-}Pym$ | $CONHCH_2CN$ |
| 10-1007 | 4-F-Ph | $2\text{-}NH_2\text{-}4\text{-}Pym$ | $CONH\text{-}(CH_2)_2\text{-}F$ |
| 10-1008 | 4-F-Ph | $2\text{-}NH_2\text{-}4\text{-}Pym$ | $CONH\text{-}(CH_2)_2\text{-}OMe$ |
| 10-1009 | 4-F-Ph | $2\text{-}NH_2\text{-}4\text{-}Pym$ | $CONH\text{-}(CH_2)_2\text{-}SMe$ |
| 10-1010 | 4-F-Ph | $2\text{-}NH_2\text{-}4\text{-}Pym$ | $CONH\text{-}(CH_2)_2\text{-}NH_2$ |
| 10-1011 | 4-F-Ph | $2\text{-}NH_2\text{-}4\text{-}Pym$ | $CONH\text{-}(CH_2)_2\text{-}NHMe$ |
| 10-1012 | 4-F-Ph | $2\text{-}NH_2\text{-}4\text{-}Pym$ | $CONH\text{-}(CH_2)_2\text{-}N(Me)_2$ |
| 10-1013 | 4-F-Ph | $2\text{-}NH_2\text{-}4\text{-}Pym$ | $CONHCH_2COOH$ |
| 10-1014 | 4-F-Ph | $2\text{-}NH_2\text{-}4\text{-}Pym$ | $CONHCH_2COOEt$ |
| 10-1015 | 4-F-Ph | $2\text{-}NH_2\text{-}4\text{-}Pym$ | CONHCH(Me)COOEt |
| 10-1016 | 4-F-Ph | $2\text{-}NH_2\text{-}4\text{-}Pym$ | $CONHCH_2CONH_2$ |
| 10-1017 | 4-F-Ph | $2\text{-}NH_2\text{-}4\text{-}Pym$ | $SO_2NH_2$ |
| 10-1018 | 4-F-Ph | $2\text{-}NH_2\text{-}4\text{-}Pym$ | $SO_2NHMe$ |
| 10-1019 | 4-F-Ph | $2\text{-}NH_2\text{-}4\text{-}Pym$ | $SO_2NHEt$ |
| 10-1020 | 4-F-Ph | $2\text{-}NH_2\text{-}4\text{-}Pym$ | $SO_2NHPr$ |
| 10-1021 | 4-F-Ph | $2\text{-}NH_2\text{-}4\text{-}Pym$ | $SO_2NH\text{-}i\text{-}Pr$ |
| 10-1022 | 4-F-Ph | $2\text{-}NH_2\text{-}4\text{-}Pym$ | $SO_2NHBu$ |
| 10-1023 | 4-F-Ph | $2\text{-}NH_2\text{-}4\text{-}Pym$ | $SO_2NHBn$ |
| 10-1024 | 4-F-Ph | $2\text{-}NH_2\text{-}4\text{-}Pym$ | $SO_2NMe_2$ |
| 10-1025 | 4-F-Ph | $2\text{-}NH_2\text{-}4\text{-}Pym$ | $SO_2NH\text{-}c\text{-}Pr$ |
| 10-1026 | 4-F-Ph | $2\text{-}NH_2\text{-}4\text{-}Pym$ | $SO_2NH\text{-}c\text{-}Bu$ |
| 10-1027 | 4-F-Ph | $2\text{-}NH_2\text{-}4\text{-}Pym$ | $SO_2NH\text{-}c\text{-}Pen$ |
| 10-1028 | 4-F-Ph | $2\text{-}NH_2\text{-}4\text{-}Pym$ | $SO_2NH\text{-}c\text{-}Hex$ |
| 10-1029 | 4-F-Ph | $2\text{-}NH_2\text{-}4\text{-}Pym$ | $SO_2NH\text{-}c\text{-}Hep$ |
| 10-1030 | 4-F-Ph | $2\text{-}NH_2\text{-}4\text{-}Pym$ | $SO_2NHCH_2\text{-}c\text{-}Pr$ |
| 10-1031 | 4-F-Ph | $2\text{-}NH_2\text{-}4\text{-}Pym$ | $SO_2NH\text{-}Allyl$ |
| 10-1032 | 4-F-Ph | $2\text{-}NH_2\text{-}4\text{-}Pym$ | $SO_2NH\text{-}Propargyl$ |
| 10-1033 | 4-F-Ph | $2\text{-}NH_2\text{-}4\text{-}Pym$ | $SO_2NHPh$ |
| 10-1034 | 4-F-Ph | $2\text{-}NH_2\text{-}4\text{-}Pym$ | $SO_2NH\text{-}3\text{-}Pyr$ |

| | | | |
|---|---|---|---|
| 10-1035 | 4-F-Ph | 2-$NH_2$-4-Pym | $SO_2NHCH_2$-4-Pyr |
| 10-1036 | 4-F-Ph | 2-$NH_2$-4-Pym | $SO_2NH-(CH_2)_2-OH$ |
| 10-1037 | 4-F-Ph | 2-$NH_2$-4-Pym | $SO_2NHCH(CH_2OH)_2$ |
| 10-1038 | 4-F-Ph | 2-$NH_2$-4-Pym | $SO_2NH-(CH_2)_2-OAc$ |
| 10-1039 | 4-F-Ph | 2-$NH_2$-4-Pym | $SO_2NHCH_2CN$ |
| 10-1040 | 4-F-Ph | 2-$NH_2$-4-Pym | $SO_2NH-(CH_2)_2-F$ |
| 10-1041 | 4-F-Ph | 2-$NH_2$-4-Pym | $SO_2NH-(CH_2)_2-OMe$ |
| 10-1042 | 4-F-Ph | 2-$NH_2$-4-Pym | $SO_2NH-(CH_2)_2-SMe$ |
| 10-1043 | 4-F-Ph | 2-$NH_2$-4-Pym | $SO_2NH-(CH_2)_2-NH_2$ |
| 10-1044 | 4-F-Ph | 2-$NH_2$-4-Pym | $SO_2NH-(CH_2)_2-NHMe$ |
| 10-1045 | 4-F-Ph | 2-$NH_2$-4-Pym | $SO_2NH-(CH_2)_2-N(Me)_2$ |
| 10-1046 | 4-F-Ph | 2-$NH_2$-4-Pym | $SO_2NHCH_2COOH$ |
| 10-1047 | 4-F-Ph | 2-$NH_2$-4-Pym | $SO_2NHCH_2COOEt$ |
| 10-1048 | 4-F-Ph | 2-$NH_2$-4-Pym | $SO_2NHCH(Me)COOEt$ |
| 10-1049 | 4-F-Ph | 2-$NH_2$-4-Pym | $SO_2NHCH_2CONH_2$ |
| 10-1050 | 4-F-Ph | 2-$NH_2$-4-Pym | $SO_2NHOH$ |
| 10-1051 | 4-F-Ph | 2-$NH_2$-4-Pym | $SO_2NHOMe$ |
| 10-1052 | 4-F-Ph | 2-$NH_2$-4-Pym | $SO_2NHOEt$ |
| 10-1053 | 4-F-Ph | 2-$NH_2$-4-Pym | $SO_2NHOPr$ |
| 10-1054 | 4-F-Ph | 2-$NH_2$-4-Pym | $SO_2NHOAllyl$ |
| 10-1055 | 4-F-Ph | 2-$NH_2$-4-Pym | $SO_2NHOBn$ |
| 10-1056 | 4-F-Ph | 2-$NH_2$-4-Pym | $SO_2NHNH_2$ |
| 10-1057 | 4-F-Ph | 2-$NH_2$-4-Pym | $SO_2NHNHMe$ |
| 10-1058 | 4-F-Ph | 2-$NH_2$-4-Pym | $SO_2NHN(Me)_2$ |
| 10-1059 | 4-F-Ph | 2-MeNH-4-Pym | $CONH_2$ |
| 10-1060 | 4-F-Ph | 2-MeNH-4-Pym | CONHMe |
| 10-1061 | 4-F-Ph | 2-MeNH-4-Pym | CONHEt |
| 10-1062 | 4-F-Ph | 2-MeNH-4-Pym | CONHPr |
| 10-1063 | 4-F-Ph | 2-MeNH-4-Pym | CONH-i-Pr |
| 10-1064 | 4-F-Ph | 2-MeNH-4-Pym | CONHBu |
| 10-1065 | 4-F-Ph | 2-MeNH-4-Pym | CONHBn |
| 10-1066 | 4-F-Ph | 2-MeNH-4-Pym | $CONMe_2$ |
| 10-1067 | 4-F-Ph | 2-MeNH-4-Pym | CONH-c-Pr |
| 10-1068 | 4-F-Ph | 2-MeNH-4-Pym | CONH-c-Bu |
| 10-1069 | 4-F-Ph | 2-MeNH-4-Pym | CONH-c-Pen |
| 10-1070 | 4-F-Ph | 2-MeNH-4-Pym | CONH-c-Hex |

| | | | |
|---|---|---|---|
| 10-1071 | 4-F-Ph | 2-MeNH-4-Pym | CONH-c-Hep |
| 10-1072 | 4-F-Ph | 2-MeNH-4-Pym | $CONHCH_2$-c-Pr |
| 10-1073 | 4-F-Ph | 2-MeNH-4-Pym | CONH-Allyl |
| 10-1074 | 4-F-Ph | 2-MeNH-4-Pym | CONH-Propargyl |
| 10-1075 | 4-F-Ph | 2-MeNH-4-Pym | CONHPh |
| 10-1076 | 4-F-Ph | 2-MeNH-4-Pym | CONH-3-Pyr |
| 10-1077 | 4-F-Ph | 2-MeNH-4-Pym | $CONHCH_2$-4-Pyr |
| 10-1078 | 4-F-Ph | 2-MeNH-4-Pym | CONH-$(CH_2)_2$-OH |
| 10-1079 | 4-F-Ph | 2-MeNH-4-Pym | $CONHCH(CH_2OH)_2$ |
| 10-1080 | 4-F-Ph | 2-MeNH-4-Pym | CONH-$(CH_2)_2$-OAc |
| 10-1081 | 4-F-Ph | 2-MeNH-4-Pym | $CONHCH_2CN$ |
| 10-1082 | 4-F-Ph | 2-MeNH-4-Pym | CONH-$(CH_2)_2$-F |
| 10-1083 | 4-F-Ph | 2-MeNH-4-Pym | CONH-$(CH_2)_2$-OMe |
| 10-1084 | 4-F-Ph | 2-MeNH-4-Pym | CONH-$(CH_2)_2$-SMe |
| 10-1085 | 4-F-Ph | 2-MeNH-4-Pym | CONH-$(CH_2)_2$-$NH_2$ |
| 10-1086 | 4-F-Ph | 2-MeNH-4-Pym | CONH-$(CH_2)_2$-NHMe |
| 10-1087 | 4-F-Ph | 2-MeNH-4-Pym | CONH-$(CH_2)_2$-$N(Me)_2$ |
| 10-1088 | 4-F-Ph | 2-MeNH-4-Pym | $CONHCH_2COOH$ |
| 10-1089 | 4-F-Ph | 2-MeNH-4-Pym | $CONHCH_2COOEt$ |
| 10-1090 | 4-F-Ph | 2-MeNH-4-Pym | CONHCH(Me)COOEt |
| 10-1091 | 4-F-Ph | 2-MeNH-4-Pym | $CONHCH_2CONH_2$ |
| 10-1092 | 4-F-Ph | 2-MeNH-4-Pym | $SO_2NH_2$ |
| 10-1093 | 4-F-Ph | 2-MeNH-4-Pym | $SO_2NHMe$ |
| 10-1094 | 4-F-Ph | 2-MeNH-4-Pym | $SO_2NHEt$ |
| 10-1095 | 4-F-Ph | 2-MeNH-4-Pym | $SO_2NHPr$ |
| 10-1096 | 4-F-Ph | 2-MeNH-4-Pym | $SO_2NH$-i-Pr |
| 10-1097 | 4-F-Ph | 2-MeNH-4-Pym | $SO_2NHBu$ |
| 10-1098 | 4-F-Ph | 2-MeNH-4-Pym | $SO_2NHBn$ |
| 10-1099 | 4-F-Ph | 2-MeNH-4-Pym | $SO_2NMe_2$ |
| 10-1100 | 4-F-Ph | 2-MeNH-4-Pym | $SO_2NH$-c-Pr |
| 10-1101 | 4-F-Ph | 2-MeNH-4-Pym | $SO_2NH$-c-Bu |
| 10-1102 | 4-F-Ph | 2-MeNH-4-Pym | $SO_2NH$-c-Pen |
| 10-1103 | 4-F-Ph | 2-MeNH-4-Pym | $SO_2NH$-c-Hex |
| 10-1104 | 4-F-Ph | 2-MeNH-4-Pym | $SO_2NH$-c-Hep |
| 10-1105 | 4-F-Ph | 2-MeNH-4-Pym | $SO_2NHCH_2$-c-Pr |
| 10-1106 | 4-F-Ph | 2-MeNH-4-Pym | $SO_2NH$-Allyl |

| | | | |
|---|---|---|---|
| 10-1107 | 4-F-Ph | 2-MeNH-4-Pym | $SO_2$NH-Propargyl |
| 10-1108 | 4-F-Ph | 2-MeNH-4-Pym | $SO_2$NHPh |
| 10-1109 | 4-F-Ph | 2-MeNH-4-Pym | $SO_2$NH-3-Pyr |
| 10-1110 | 4-F-Ph | 2-MeNH-4-Pym | $SO_2NHCH_2$-4-Pyr |
| 10-1111 | 4-F-Ph | 2-MeNH-4-Pym | $SO_2NH-(CH_2)_2-OH$ |
| 10-1112 | 4-F-Ph | 2-MeNH-4-Pym | $SO_2NHCH(CH_2OH)_2$ |
| 10-1113 | 4-F-Ph | 2-MeNH-4-Pym | $SO_2NH-(CH_2)_2-OAc$ |
| 10-1114 | 4-F-Ph | 2-MeNH-4-Pym | $SO_2NHCH_2CN$ |
| 10-1115 | 4-F-Ph | 2-MeNH-4-Pym | $SO_2NH-(CH_2)_2-F$ |
| 10-1116 | 4-F-Ph | 2-MeNH-4-Pym | $SO_2NH-(CH_2)_2-OMe$ |
| 10-1117 | 4-F-Ph | 2-MeNH-4-Pym | $SO_2NH-(CH_2)_2-SMe$ |
| 10-1118 | 4-F-Ph | 2-MeNH-4-Pym | $SO_2NH-(CH_2)_2-NH_2$ |
| 10-1119 | 4-F-Ph | 2-MeNH-4-Pym | $SO_2NH-(CH_2)_2-NHMe$ |
| 10-1120 | 4-F-Ph | 2-MeNH-4-Pym | $SO_2NH-(CH_2)_2-N(Me)_2$ |
| 10-1121 | 4-F-Ph | 2-MeNH-4-Pym | $SO_2NHCH_2COOH$ |
| 10-1122 | 4-F-Ph | 2-MeNH-4-Pym | $SO_2NHCH_2COOEt$ |
| 10-1123 | 4-F-Ph | 2-MeNH-4-Pym | $SO_2NHCH(Me)COOEt$ |
| 10-1124 | 4-F-Ph | 2-MeNH-4-Pym | $SO_2NHCH_2CONH_2$ |
| 10-1125 | 4-F-Ph | 2-MeNH-4-Pym | $SO_2NHOH$ |
| 10-1126 | 4-F-Ph | 2-MeNH-4-Pym | $SO_2NHOMe$ |
| 10-1127 | 4-F-Ph | 2-MeNH-4-Pym | $SO_2NHOEt$ |
| 10-1128 | 4-F-Ph | 2-MeNH-4-Pym | $SO_2NHOPr$ |
| 10-1129 | 4-F-Ph | 2-MeNH-4-Pym | $SO_2$NHOAllyl |
| 10-1130 | 4-F-Ph | 2-MeNH-4-Pym | $SO_2NHOBn$ |
| 10-1131 | 4-F-Ph | 2-MeNH-4-Pym | $SO_2NHNH_2$ |
| 10-1132 | 4-F-Ph | 2-MeNH-4-Pym | $SO_2NHNHMe$ |
| 10-1133 | 4-F-Ph | 2-MeNH-4-Pym | $SO_2NHN(Me)_2$ |
| 10-1134 | 4-F-Ph | 2-BnNH-4-Pym | $CONH_2$ |
| 10-1135 | 4-F-Ph | 2-BnNH-4-Pym | CONHMe |
| 10-1136 | 4-F-Ph | 2-BnNH-4-Pym | CONHEt |
| 10-1137 | 4-F-Ph | 2-BnNH-4-Pym | CONHPr |
| 10-1138 | 4-F-Ph | 2-BnNH-4-Pym | CONH-i-Pr |
| 10-1139 | 4-F-Ph | 2-BnNH-4-Pym | CONHBu |
| 10-1140 | 4-F-Ph | 2-BnNH-4-Pym | CONHBn |
| 10-1141 | 4-F-Ph | 2-BnNH-4-Pym | $CONMe_2$ |
| 10-1142 | 4-F-Ph | 2-BnNH-4-Pym | CONH-c-Pr |

| | | | |
|---|---|---|---|
| 10-1143 | 4-F-Ph | 2-BnNH-4-Pym | CONH-c-Bu |
| 10-1144 | 4-F-Ph | 2-BnNH-4-Pym | CONH-c-Pen |
| 10-1145 | 4-F-Ph | 2-BnNH-4-Pym | CONH-c-Hex |
| 10-1146 | 4-F-Ph | 2-BnNH-4-Pym | CONH-c-Hep |
| 10-1147 | 4-F-Ph | 2-BnNH-4-Pym | $CONHCH_2$-c-Pr |
| 10-1148 | 4-F-Ph | 2-BnNH-4-Pym | CONH-Allyl |
| 10-1149 | 4-F-Ph | 2-BnNH-4-Pym | CONH-Propargyl |
| 10-1150 | 4-F-Ph | 2-BnNH-4-Pym | CONHPh |
| 10-1151 | 4-F-Ph | 2-BnNH-4-Pym | CONH-3-Pyr |
| 10-1152 | 4-F-Ph | 2-BnNH-4-Pym | $CONHCH_2$-4-Pyr |
| 10-1153 | 4-F-Ph | 2-BnNH-4-Pym | CONH-$(CH_2)_2$-OH |
| 10-1154 | 4-F-Ph | 2-BnNH-4-Pym | $CONHCH(CH_2OH)_2$ |
| 10-1155 | 4-F-Ph | 2-BnNH-4-Pym | CONH-$(CH_2)_2$-OAc |
| 10-1156 | 4-F-Ph | 2-BnNH-4-Pym | $CONHCH_2CN$ |
| 10-1157 | 4-F-Ph | 2-BnNH-4-Pym | CONH-$(CH_2)_2$-F |
| 10-1158 | 4-F-Ph | 2-BnNH-4-Pym | CONH-$(CH_2)_2$-OMe |
| 10-1159 | 4-F-Ph | 2-BnNH-4-Pym | CONH-$(CH_2)_2$-SMe |
| 10-1160 | 4-F-Ph | 2-BnNH-4-Pym | CONH-$(CH_2)_2$-$NH_2$ |
| 10-1161 | 4-F-Ph | 2-BnNH-4-Pym | CONH-$(CH_2)_2$-NHMe |
| 10-1162 | 4-F-Ph | 2-BnNH-4-Pym | CONH-$(CH_2)_2$-$N(Me)_2$ |
| 10-1163 | 4-F-Ph | 2-BnNH-4-Pym | $CONHCH_2COOH$ |
| 10-1164 | 4-F-Ph | 2-BnNH-4-Pym | $CONHCH_2COOEt$ |
| 10-1165 | 4-F-Ph | 2-BnNH-4-Pym | CONHCH(Me)COOEt |
| 10-1166 | 4-F-Ph | 2-BnNH-4-Pym | $CONHCH_2CONH_2$ |
| 10-1167 | 4-F-Ph | 2-BnNH-4-Pym | $SO_2NH_2$ |
| 10-1168 | 4-F-Ph | 2-BnNH-4-Pym | $SO_2NHMe$ |
| 10-1169 | 4-F-Ph | 2-BnNH-4-Pym | $SO_2NHEt$ |
| 10-1170 | 4-F-Ph | 2-BnNH-4-Pym | $SO_2NHPr$ |
| 10-1171 | 4-F-Ph | 2-BnNH-4-Pym | $SO_2NH$-i-Pr |
| 10-1172 | 4-F-Ph | 2-BnNH-4-Pym | $SO_2NHBu$ |
| 10-1173 | 4-F-Ph | 2-BnNH-4-Pym | $SO_2NHBn$ |
| 10-1174 | 4-F-Ph | 2-BnNH-4-Pym | $SO_2NMe_2$ |
| 10-1175 | 4-F-Ph | 2-BnNH-4-Pym | $SO_2NH$-c-Pr |
| 10-1176 | 4-F-Ph | 2-BnNH-4-Pym | $SO_2NH$-c-Bu |
| 10-1177 | 4-F-Ph | 2-BnNH-4-Pym | $SO_2NH$-c-Pen |
| 10-1178 | 4-F-Ph | 2-BnNH-4-Pym | $SO_2NH$-c-Hex |

| | | | |
|---|---|---|---|
| 10-1179 | 4-F-Ph | 2-BnNH-4-Pym | $SO_2$NH-c-Hep |
| 10-1180 | 4-F-Ph | 2-BnNH-4-Pym | $SO_2NHCH_2$-c-Pr |
| 10-1181 | 4-F-Ph | 2-BnNH-4-Pym | $SO_2$NH-Allyl |
| 10-1182 | 4-F-Ph | 2-BnNH-4-Pym | $SO_2$NH-Propargyl |
| 10-1183 | 4-F-Ph | 2-BnNH-4-Pym | $SO_2$NHPh |
| 10-1184 | 4-F-Ph | 2-BnNH-4-Pym | $SO_2$NH-3-Pyr |
| 10-1185 | 4-F-Ph | 2-BnNH-4-Pym | $SO_2NHCH_2$-4-Pyr |
| 10-1186 | 4-F-Ph | 2-BnNH-4-Pym | $SO_2NH-(CH_2)_2-OH$ |
| 10-1187 | 4-F-Ph | 2-BnNH-4-Pym | $SO_2NHCH(CH_2OH)_2$ |
| 10-1188 | 4-F-Ph | 2-BnNH-4-Pym | $SO_2NH-(CH_2)_2-OAc$ |
| 10-1189 | 4-F-Ph | 2-BnNH-4-Pym | $SO_2NHCH_2CN$ |
| 10-1190 | 4-F-Ph | 2-BnNH-4-Pym | $SO_2NH-(CH_2)_2-F$ |
| 10-1191 | 4-F-Ph | 2-BnNH-4-Pym | $SO_2NH-(CH_2)_2-OMe$ |
| 10-1192 | 4-F-Ph | 2-BnNH-4-Pym | $SO_2NH-(CH_2)_2-SMe$ |
| 10-1193 | 4-F-Ph | 2-BnNH-4-Pym | $SO_2NH-(CH_2)_2-NH_2$ |
| 10-1194 | 4-F-Ph | 2-BnNH-4-Pym | $SO_2NH-(CH_2)_2-NHMe$ |
| 10-1195 | 4-F-Ph | 2-BnNH-4-Pym | $SO_2NH-(CH_2)_2-N(Me)_2$ |
| 10-1196 | 4-F-Ph | 2-BnNH-4-Pym | $SO_2NHCH_2COOH$ |
| 10-1197 | 4-F-Ph | 2-BnNH-4-Pym | $SO_2NHCH_2COOEt$ |
| 10-1198 | 4-F-Ph | 2-BnNH-4-Pym | $SO_2$NHCH(Me)COOEt |
| 10-1199 | 4-F-Ph | 2-BnNH-4-Pym | $SO_2NHCH_2CONH_2$ |
| 10-1200 | 4-F-Ph | 2-BnNH-4-Pym | $SO_2$NHOH |
| 10-1201 | 4-F-Ph | 2-BnNH-4-Pym | $SO_2$NHOMe |
| 10-1202 | 4-F-Ph | 2-BnNH-4-Pym | $SO_2$NHOEt |
| 10-1203 | 4-F-Ph | 2-BnNH-4-Pym | $SO_2$NHOPr |
| 10-1204 | 4-F-Ph | 2-BnNH-4-Pym | $SO_2$NHOAllyl |
| 10-1205 | 4-F-Ph | 2-BnNH-4-Pym | $SO_2$NHOBn |
| 10-1206 | 4-F-Ph | 2-BnNH-4-Pym | $SO_2NHNH_2$ |
| 10-1207 | 4-F-Ph | 2-BnNH-4-Pym | $SO_2$NHNHMe |
| 10-1208 | 4-F-Ph | 2-BnNH-4-Pym | $SO_2NHN(Me)_2$ |
| 10-1209 | 4-F-Ph | (α-Me-BnNH)-4-Pym | $CONH_2$ |
| 10-1210 | 4-F-Ph | (α-Me-BnNH)-4-Pym | CONHMe |
| 10-1211 | 4-F-Ph | (α-Me-BnNH)-4-Pym | CONHEt |
| 10-1212 | 4-F-Ph | (α-Me-BnNH)-4-Pym | CONHPr |
| 10-1213 | 4-F-Ph | (α-Me-BnNH)-4-Pym | CONH-i-Pr |

| | | | |
|---|---|---|---|
| 10-1214 | 4-F-Ph | (α-Me-BnNH)-4-Pym | CONHBu |
| 10-1215 | 4-F-Ph | (α-Me-BnNH)-4-Pym | CONHBn |
| 10-1216 | 4-F-Ph | (α-Me-BnNH)-4-Pym | $CONMe_2$ |
| 10-1217 | 4-F-Ph | (α-Me-BnNH)-4-Pym | CONH-c-Pr |
| 10-1218 | 4-F-Ph | (α-Me-BnNH)-4-Pym | CONH-c-Bu |
| 10-1219 | 4-F-Ph | (α-Me-BnNH)-4-Pym | CONH-c-Pen |
| 10-1220 | 4-F-Ph | (α-Me-BnNH)-4-Pym | CONH-c-Hex |
| 10-1221 | 4-F-Ph | (α-Me-BnNH)-4-Pym | CONH-c-Hep |
| 10-1222 | 4-F-Ph | (α-Me-BnNH)-4-Pym | $CONHCH_2$-c-Pr |
| 10-1223 | 4-F-Ph | (α-Me-BnNH)-4-Pym | CONH-Allyl |
| 10-1224 | 4-F-Ph | (α-Me-BnNH)-4-Pym | CONH-Propargyl |
| 10-1225 | 4-F-Ph | (α-Me-BnNH)-4-Pym | CONHPh |
| 10-1226 | 4-F-Ph | (α-Me-BnNH)-4-Pym | CONH-3-Pyr |
| 10-1227 | 4-F-Ph | (α-Me-BnNH)-4-Pym | $CONHCH_2$-4-Pyr |
| 10-1228 | 4-F-Ph | (α-Me-BnNH)-4-Pym | CONH-$(CH_2)_2$-OH |
| 10-1229 | 4-F-Ph | (α-Me-BnNH)-4-Pym | $CONHCH(CH_2OH)_2$ |
| 10-1230 | 4-F-Ph | (α-Me-BnNH)-4-Pym | CONH-$(CH_2)_2$-OAc |
| 10-1231 | 4-F-Ph | (α-Me-BnNH)-4-Pym | $CONHCH_2CN$ |
| 10-1232 | 4-F-Ph | (α-Me-BnNH)-4-Pym | CONH-$(CH_2)_2$-F |
| 10-1233 | 4-F-Ph | (α-Me-BnNH)-4-Pym | CONH-$(CH_2)_2$-OMe |
| 10-1234 | 4-F-Ph | (α-Me-BnNH)-4-Pym | CONH-$(CH_2)_2$-SMe |
| 10-1235 | 4-F-Ph | (α-Me-BnNH)-4-Pym | CONH-$(CH_2)_2$-$NH_2$ |
| 10-1236 | 4-F-Ph | (α-Me-BnNH)-4-Pym | CONH-$(CH_2)_2$-NHMe |
| 10-1237 | 4-F-Ph | (α-Me-BnNH)-4-Pym | CONH-$(CH_2)_2$-$N(Me)_2$ |
| 10-1238 | 4-F-Ph | (α-Me-BnNH)-4-Pym | $CONHCH_2COOH$ |
| 10-1239 | 4-F-Ph | (α-Me-BnNH)-4-Pym | $CONHCH_2COOEt$ |
| 10-1240 | 4-F-Ph | (α-Me-BnNH)-4-Pym | CONHCH(Me)COOEt |
| 10-1241 | 4-F-Ph | (α-Me-BnNH)-4-Pym | $CONHCH_2CONH_2$ |
| 10-1242 | 4-F-Ph | (α-Me-BnNH)-4-Pym | $SO_2NH_2$ |
| 10-1243 | 4-F-Ph | (α-Me-BnNH)-4-Pym | $SO_2NHMe$ |
| 10-1244 | 4-F-Ph | (α-Me-BnNH)-4-Pym | $SO_2NHEt$ |
| 10-1245 | 4-F-Ph | (α-Me-BnNH)-4-Pym | $SO_2NHPr$ |

| | | | |
|---|---|---|---|
| 10-1246 | 4-F-Ph | (α-Me-BnNH)-4-Pym | $SO_2NH$-i-Pr |
| 10-1247 | 4-F-Ph | (α-Me-BnNH)-4-Pym | $SO_2NHBu$ |
| 10-1248 | 4-F-Ph | (α-Me-BnNH)-4-Pym | $SO_2NHBn$ |
| 10-1249 | 4-F-Ph | (α-Me-BnNH)-4-Pym | $SO_2NMe_2$ |
| 10-1250 | 4-F-Ph | (α-Me-BnNH)-4-Pym | $SO_2NH$-c-Pr |
| 10-1251 | 4-F-Ph | (α-Me-BnNH)-4-Pym | $SO_2NH$-c-Bu |
| 10-1252 | 4-F-Ph | (α-Me-BnNH)-4-Pym | $SO_2NH$-c-Pen |
| 10-1253 | 4-F-Ph | (α-Me-BnNH)-4-Pym | $SO_2NH$-c-Hex |
| 10-1254 | 4-F-Ph | (α-Me-BnNH)-4-Pym | $SO_2NH$-c-Hep |
| 10-1255 | 4-F-Ph | (α-Me-BnNH)-4-Pym | $SO_2NHCH_2$-c-Pr |
| 10-1256 | 4-F-Ph | (α-Me-BnNH)-4-Pym | $SO_2NH$-Allyl |
| 10-1257 | 4-F-Ph | (α-Me-BnNH)-4-Pym | $SO_2NH$-Propargyl |
| 10-1258 | 4-F-Ph | (α-Me-BnNH)-4-Pym | $SO_2NHPh$ |
| 10-1259 | 4-F-Ph | (α-Me-BnNH)-4-Pym | $SO_2NH$-3-Pyr |
| 10-1260 | 4-F-Ph | (α-Me-BnNH)-4-Pym | $SO_2NHCH_2$-4-Pyr |
| 10-1261 | 4-F-Ph | (α-Me-BnNH)-4-Pym | $SO_2NH-(CH_2)_2-OH$ |
| 10-1262 | 4-F-Ph | (α-Me-BnNH)-4-Pym | $SO_2NHCH(CH_2OH)_2$ |
| 10-1263 | 4-F-Ph | (α-Me-BnNH)-4-Pym | $SO_2NH-(CH_2)_2-OAc$ |
| 10-1264 | 4-F-Ph | (α-Me-BnNH)-4-Pym | $SO_2NHCH_2CN$ |
| 10-1265 | 4-F-Ph | (α-Me-BnNH)-4-Pym | $SO_2NH-(CH_2)_2-F$ |
| 10-1266 | 4-F-Ph | (α-Me-BnNH)-4-Pym | $SO_2NH-(CH_2)_2-OMe$ |
| 10-1267 | 4-F-Ph | (α-Me-BnNH)-4-Pym | $SO_2NH-(CH_2)_2-SMe$ |
| 10-1268 | 4-F-Ph | (α-Me-BnNH)-4-Pym | $SO_2NH-(CH_2)_2-NH_2$ |
| 10-1269 | 4-F-Ph | (α-Me-BnNH)-4-Pym | $SO_2NH-(CH_2)_2-NHMe$ |
| 10-1270 | 4-F-Ph | (α-Me-BnNH)-4-Pym | $SO_2NH-(CH_2)_2-N(Me)_2$ |
| 10-1271 | 4-F-Ph | (α-Me-BnNH)-4-Pym | $SO_2NHCH_2COOH$ |
| 10-1272 | 4-F-Ph | (α-Me-BnNH)-4-Pym | $SO_2NHCH_2COOEt$ |
| 10-1273 | 4-F-Ph | (α-Me-BnNH)-4-Pym | $SO_2NHCH(Me)COOEt$ |
| 10-1274 | 4-F-Ph | (α-Me-BnNH)-4-Pym | $SO_2NHCH_2CONH_2$ |
| 10-1275 | 4-F-Ph | (α-Me-BnNH)-4-Pym | $SO_2NHOH$ |
| 10-1276 | 4-F-Ph | (α-Me-BnNH)-4-Pym | $SO_2NHOMe$ |
| 10-1277 | 4-F-Ph | (α-Me-BnNH)-4-Pym | $SO_2NHOEt$ |

| | | | |
|---|---|---|---|
| 10-1278 | 4-F-Ph | (α-Me-BnNH)-4-Pym | $SO_2$NHOPr |
| 10-1279 | 4-F-Ph | (α-Me-BnNH)-4-Pym | $SO_2$NHOAllyl |
| 10-1280 | 4-F-Ph | (α-Me-BnNH)-4-Pym | $SO_2$NHOBn |
| 10-1281 | 4-F-Ph | (α-Me-BnNH)-4-Pym | $SO_2NHNH_2$ |
| 10-1282 | 4-F-Ph | (α-Me-BnNH)-4-Pym | $SO_2$NHNHMe |
| 10-1283 | 4-F-Ph | (α-Me-BnNH)-4-Pym | $SO_2NHN(Me)_2$ |
| 10-1284 | 4-F-Ph | 4-Pyr | $SO_2$NHMe |
| 10-1285 | 4-F-Ph | 4-Pyr | SOMe |
| 10-1286 | 4-F-Ph | 4-Pyr | $SO_2$Me |
| 10-1287 | 3-Cl-4-F-Ph | 4-Pyr | $SO_2$NHMe |
| 10-1288 | 3-Cl-4-F-Ph | 4-Pyr | SOMe |
| 10-1289 | 3-Cl-4-F-Ph | 4-Pyr | $SO_2$Me |

Table 11

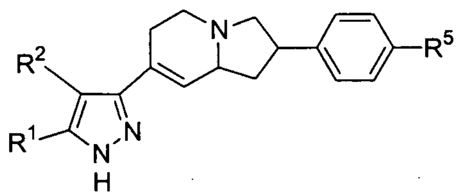

(I-1 1)

| Compound No. | $R^1$ | $R^2$ | $R^5$ |
|---|---|---|---|
| 11-1 | Ph | 4-Pyr | COOH |
| 11-2 | Ph | 4-Pyr | COOMe |
| 11-3 | Ph | 4-Pyr | COOEt |
| 11-4 | Ph | 4-Pyr | COOPr |
| 11-5 | Ph | 4-Pyr | COO-i-Pr |
| 11-6 | Ph | 4-Pyr | COOBu |
| 11-7 | Ph | 4-Pyr | COOBn |
| 11-8 | Ph | 4-Pyr | COOPh |
| 11-9 | Ph | 4-Pyr | $CONH_2$ |
| 11-10 | Ph | 4-Pyr | CONHMe |
| 11-11 | Ph | 4-Pyr | CONHEt |
| 11-12 | Ph | 4-Pyr | CONHPr |

| | | | |
|---|---|---|---|
| 11-13 | Ph | 4-Pyr | CONH-i-Pr |
| 11-14 | Ph | 4-Pyr | CONHBu |
| 11-15 | Ph | 4-Pyr | CONHBn |
| 11-16 | Ph | 4-Pyr | $CONMe_2$ |
| 11-17 | Ph | 4-Pyr | CONH-c-Pr |
| 11-18 | Ph | 4-Pyr | CONH-c-Bu |
| 11-19 | Ph | 4-Pyr | CONH-c-Pen |
| 11-20 | Ph | 4-Pyr | CONH-c-Hex |
| 11-21 | Ph | 4-Pyr | CONH-c-Hep |
| 11-22 | Ph | 4-Pyr | $CONHCH_2$-c-Pr |
| 11-23 | Ph | 4-Pyr | CONH-Allyl |
| 11-24 | Ph | 4-Pyr | CONH-Propargyl |
| 11-25 | Ph | 4-Pyr | CONHPh |
| 11-26 | Ph | 4-Pyr | CONH-3-Pyr |
| 11-27 | Ph | 4-Pyr | $CONHCH_2$-4-Pyr |
| 11-28 | Ph | 4-Pyr | CONH-$(CH_2)_2$-OH |
| 11-29 | Ph | 4-Pyr | $CONHCH(CH_2OH)_2$ |
| 11-30 | Ph | 4-Pyr | CONH-$(CH_2)_2$-OAc |
| 11-31 | Ph | 4-Pyr | $CONHCH_2CN$ |
| 11-32 | Ph | 4-Pyr | CONH-$(CH_2)_2$-F |
| 11-33 | Ph | 4-Pyr | CONH-$(CH_2)_2$-OMe |
| 11-34 | Ph | 4-Pyr | CONH-$(CH_2)_2$-SMe |
| 11-35 | Ph | 4-Pyr | CONH-$(CH_2)_2$-$NH_2$ |
| 11-36 | Ph | 4-Pyr | CONH-$(CH_2)_2$-NHMe |
| 11-37 | Ph | 4-Pyr | CONH-$(CH_2)_2$-$N(Me)_2$ |
| 11-38 | Ph | 4-Pyr | $CONHCH_2COOH$ |
| 11-39 | Ph | 4-Pyr | $CONHCH_2COOEt$ |
| 11-40 | Ph | 4-Pyr | CONHCH(Me)COOEt |
| 11-41 | Ph | 4-Pyr | $CONHCH_2CONH_2$ |
| 11-42 | Ph | 4-Pyr | CONHOH |
| 11-43 | Ph | 4-Pyr | CONHOMe |
| 11-44 | Ph | 4-Pyr | CONHOEt |
| 11-45 | Ph | 4-Pyr | CONHOPr |
| 11-46 | Ph | 4-Pyr | CONHO-Allyl |
| 11-47 | Ph | 4-Pyr | CONHOBn |
| 11-48 | Ph | 4-Pyr | $CONHNH_2$ |

| | | | |
|---|---|---|---|
| 11-49 | Ph | 4-Pyr | CONHNHMe |
| 11-50 | Ph | 4-Pyr | CONHN(Me)$_2$ |
| 11-51 | Ph | 4-Pyr | COMe |
| 11-52 | Ph | 4-Pyr | COEt |
| 11-53 | Ph | 4-Pyr | COPr |
| 11-54 | Ph | 4-Pyr | CO-i-Pr |
| 11-55 | Ph | 4-Pyr | COBu |
| 11-56 | Ph | 4-Pyr | $COCF_3$ |
| 11-57 | Ph | 4-Pyr | CO-$(CH_2)_2$-F |
| 11-58 | Ph | 4-Pyr | CO-$(CH_2)_2$-OH |
| 11-59 | Ph | 4-Pyr | CO-$(CH_2)_2$-OMe |
| 11-60 | Ph | 4-Pyr | SOMe |
| 11-61 | Ph | 4-Pyr | SOEt |
| 11-62 | Ph | 4-Pyr | SOPr |
| 11-63 | Ph | 4-Pyr | SO-i-Pr |
| 11-64 | Ph | 4-Pyr | SOBu |
| 11-65 | Ph | 4-Pyr | $SOCF_3$ |
| 11-66 | Ph | 4-Pyr | SO-$(CH_2)_2$-F |
| 11-67 | Ph | 4-Pyr | SO-$(CH_2)_2$-OH |
| 11-68 | Ph | 4-Pyr | SO-$(CH_2)_2$-OMe |
| 11-69 | Ph | 4-Pyr | $SO_2$Me |
| 11-70 | Ph | 4-Pyr | $SO_2$Et |
| 11-71 | Ph | 4-Pyr | $SO_2$Pr |
| 11-72 | Ph | 4-Pyr | $SO_2$-i-Pr |
| 11-73 | Ph | 4-Pyr | $SO_2$Bu |
| 11-74 | Ph | 4-Pyr | $SO_2CF_3$ |
| 11-75 | Ph | 4-Pyr | $SO_2$-$(CH_2)_2$-F |
| 11-76 | Ph | 4-Pyr | $SO_2$-$(CH_2)_2$-OH |
| 11-77 | Ph | 4-Pyr | $SO_2$-$(CH_2)_2$-OMe |
| 11-78 | Ph | 4-Pyr | $SO_2NH_2$ |
| 11-79 | Ph | 4-Pyr | $SO_2$NHMe |
| 11-80 | Ph | 4-Pyr | $SO_2$NHEt |
| 11-81 | Ph | 4-Pyr | $SO_2$NHPr |
| 11-82 | Ph | 4-Pyr | $SO_2$NH-i-Pr |
| 11-83 | Ph | 4-Pyr | $SO_2$NHBu |
| 11-84 | Ph | 4-Pyr | $SO_2$NHBn |

| | | | |
|---|---|---|---|
| 11-85 | Ph | 4-Pyr | $SO_2NMe_2$ |
| 11-86 | Ph | 4-Pyr | $SO_2NH$-c-Pr |
| 11-87 | Ph | 4-Pyr | $SO_2NH$-c-Bu |
| 11-88 | Ph | 4-Pyr | $SO_2NH$-c-Pen |
| 11-89 | Ph | 4-Pyr | $SO_2NH$-c-Hex |
| 11-90 | Ph | 4-Pyr | $SO_2NH$-c-Hep |
| 11-91 | Ph | 4-Pyr | $SO_2NHCH_2$-c-Pr |
| 11-92 | Ph | 4-Pyr | $SO_2NH$-Allyl |
| 11-93 | Ph | 4-Pyr | $SO_2NH$-Propargyl |
| 11-94 | Ph | 4-Pyr | $SO_2NHPh$ |
| 11-95 | Ph | 4-Pyr | $SO_2NH$-3-Pyr |
| 11-96 | Ph | 4-Pyr | $SO_2NHCH_2$-4-Pyr |
| 11-97 | Ph | 4-Pyr | $SO_2NH-(CH_2)_2-OH$ |
| 11-98 | Ph | 4-Pyr | $SO_2NHCH(CH_2OH)_2$ |
| 11-99 | Ph | 4-Pyr | $SO_2NH-(CH_2)_2-OAc$ |
| 11-100 | Ph | 4-Pyr | $SO_2NHCH_2CN$ |
| 11-101 | Ph | 4-Pyr | $SO_2NH-(CH_2)_2-F$ |
| 11-102 | Ph | 4-Pyr | $SO_2NH-(CH_2)_2-OMe$ |
| 11-103 | Ph | 4-Pyr | $SO_2NH-(CH_2)_2-SMe$ |
| 11-104 | Ph | 4-Pyr | $SO_2NH-(CH_2)_2-NH_2$ |
| 11-105 | Ph | 4-Pyr | $SO_2NH-(CH_2)_2-NHMe$ |
| 11-106 | Ph | 4-Pyr | $SO_2NH-(CH_2)_2-N(Me)_2$ |
| 11-107 | Ph | 4-Pyr | $SO_2NHCH_2COOH$ |
| 11-108 | Ph | 4-Pyr | $SO_2NHCH_2COOEt$ |
| 11-109 | Ph | 4-Pyr | $SO_2NHCH(Me)COOEt$ |
| 11-110 | Ph | 4-Pyr | $SO_2NHCH_2CONH_2$ |
| 11-111 | Ph | 4-Pyr | $SO_2NHOH$ |
| 11-112 | Ph | 4-Pyr | $SO_2NHOMe$ |
| 11-113 | Ph | 4-Pyr | $SO_2NHOEt$ |
| 11-114 | Ph | 4-Pyr | $SO_2NHOPr$ |
| 11-115 | Ph | 4-Pyr | $SO_2NHOAllyl$ |
| 11-116 | Ph | 4-Pyr | $SO_2NHOBn$ |
| 11-117 | Ph | 4-Pyr | $SO_2NHNH_2$ |
| 11-118 | Ph | 4-Pyr | $SO_2NHNHMe$ |
| 11-119 | Ph | 4-Pyr | $SO_2NHN(Me)_2$ |
| 11-120 | 3-F-Ph | 4-Pyr | COOH |

| | | | |
|---|---|---|---|
| 11-121 | 3-F-Ph | 4-Pyr | COOMe |
| 11-122 | 3-F-Ph | 4-Pyr | COOEt |
| 11-123 | 3-F-Ph | 4-Pyr | COOPr |
| 11-124 | 3-F-Ph | 4-Pyr | COO-i-Pr |
| 11-125 | 3-F-Ph | 4-Pyr | COOBu |
| 11-126 | 3-F-Ph | 4-Pyr | COOBn |
| 11-127 | 3-F-Ph | 4-Pyr | COOPh |
| 11-128 | 3-F-Ph | 4-Pyr | $CONH_2$ |
| 11-129 | 3-F-Ph | 4-Pyr | CONHMe |
| 11-130 | 3-F-Ph | 4-Pyr | CONHEt |
| 11-131 | 3-F-Ph | 4-Pyr | CONHPr |
| 11-132 | 3-F-Ph | 4-Pyr | CONH-i-Pr |
| 11-133 | 3-F-Ph | 4-Pyr | CONHBu |
| 11-134 | 3-F-Ph | 4-Pyr | CONHBn |
| 11-135 | 3-F-Ph | 4-Pyr | $CONMe_2$ |
| 11-136 | 3-F-Ph | 4-Pyr | CONH-c-Pr |
| 11-137 | 3-F-Ph | 4-Pyr | CONH-c-Bu |
| 11-138 | 3-F-Ph | 4-Pyr | CONH-c-Pen |
| 11-139 | 3-F-Ph | 4-Pyr | CONH-c-Hex |
| 11-140 | 3-F-Ph | 4-Pyr | CONH-c-Hep |
| 11-141 | 3-F-Ph | 4-Pyr | $CONHCH_2$-c-Pr |
| 11-142 | 3-F-Ph | 4-Pyr | CONH-Allyl |
| 11-143 | 3-F-Ph | 4-Pyr | CONH-Propargyl |
| 11-144 | 3-F-Ph | 4-Pyr | CONHPh |
| 11-145 | 3-F-Ph | 4-Pyr | CONH-3-Pyr |
| 11-146 | 3-F-Ph | 4-Pyr | $CONHCH_2$-4-Pyr |
| 11-147 | 3-F-Ph | 4-Pyr | CONH-$(CH_2)_2$-OH |
| 11-148 | 3-F-Ph | 4-Pyr | $CONHCH(CH_2OH)_2$ |
| 11-149 | 3-F-Ph | 4-Pyr | CONH-$(CH_2)_2$-OAc |
| 11-150 | 3-F-Ph | 4-Pyr | $CONHCH_2CN$ |
| 11-151 | 3-F-Ph | 4-Pyr | CONH-$(CH_2)_2$-F |
| 11-152 | 3-F-Ph | 4-Pyr | CONH-$(CH_2)_2$-OMe |
| 11-153 | 3-F-Ph | 4-Pyr | CONH-$(CH_2)_2$-SMe |
| 11-154 | 3-F-Ph | 4-Pyr | CONH-$(CH_2)_2$-$NH_2$ |
| 11-155 | 3-F-Ph | 4-Pyr | CONH-$(CH_2)_2$-NHMe |
| 11-156 | 3-F-Ph | 4-Pyr | CONH-$(CH_2)_2$-$N(Me)_2$ |

| | | | |
|---|---|---|---|
| 11-157 | 3-F-Ph | 4-Pyr | $CONHCH_2COOH$ |
| 11-158 | 3-F-Ph | 4-Pyr | $CONHCH_2COOEt$ |
| 11-159 | 3-F-Ph | 4-Pyr | CONHCH(Me)COOEt |
| 11-160 | 3-F-Ph | 4-Pyr | $CONHCH_2CONH_2$ |
| 11-161 | 3-F-Ph | 4-Pyr | CONHOH |
| 11-162 | 3-F-Ph | 4-Pyr | CONHOMe |
| 11-163 | 3-F-Ph | 4-Pyr | CONHOEt |
| 11-164 | 3-F-Ph | 4-Pyr | CONHOPr |
| 11-165 | 3-F-Ph | 4-Pyr | CONHOAllyl |
| 11-166 | 3-F-Ph | 4-Pyr | CONHOBn |
| 11-167 | 3-F-Ph | 4-Pyr | $CONHNH_2$ |
| 11-168 | 3-F-Ph | 4-Pyr | CONHNHMe |
| 11-169 | 3-F-Ph | 4-Pyr | $CONHN(Me)_2$ |
| 11-170 | 3-F-Ph | 4-Pyr | COMe |
| 11-171 | 3-F-Ph | 4-Pyr | COEt |
| 11-172 | 3-F-Ph | 4-Pyr | COPr |
| 11-173 | 3-F-Ph | 4-Pyr | CO-i-Pr |
| 11-174 | 3-F-Ph | 4-Pyr | COBu |
| 11-175 | 3-F-Ph | 4-Pyr | $COCF_3$ |
| 11-176 | 3-F-Ph | 4-Pyr | $CO-(CH_2)_2-F$ |
| 11-177 | 3-F-Ph | 4-Pyr | $CO-(CH_2)_2-OH$ |
| 11-178 | 3-F-Ph | 4-Pyr | $CO-(CH_2)_2-OMe$ |
| 11-179 | 3-F-Ph | 4-Pyr | SOMe |
| 11-180 | 3-F-Ph | 4-Pyr | SOEt |
| 11-181 | 3-F-Ph | 4-Pyr | SOPr |
| 11-182 | 3-F-Ph | 4-Pyr | SO-i-Pr |
| 11-183 | 3-F-Ph | 4-Pyr | SOBu |
| 11-184 | 3-F-Ph | 4-Pyr | $SOCF_3$ |
| 11-185 | 3-F-Ph | 4-Pyr | $SO-(CH_2)_2-F$ |
| 11-186 | 3-F-Ph | 4-Pyr | $SO-(CH_2)_2-OH$ |
| 11-187 | 3-F-Ph | 4-Pyr | $SO-(CH_2)_2-OMe$ |
| 11-188 | 3-F-Ph | 4-Pyr | $SO_2Me$ |
| 11-189 | 3-F-Ph | 4-Pyr | $SO_2Et$ |
| 11-190 | 3-F-Ph | 4-Pyr | $SO_2Pr$ |
| 11-191 | 3-F-Ph | 4-Pyr | $SO_2-i-Pr$ |
| 11-192 | 3-F-Ph | 4-Pyr | $SO_2Bu$ |

| | | | |
|---|---|---|---|
| 11-193 | 3-F-Ph | 4-Pyr | $SO_2CF_3$ |
| 11-194 | 3-F-Ph | 4-Pyr | $SO_2-(CH_2)_2-F$ |
| 11-195 | 3-F-Ph | 4-Pyr | $SO_2-(CH_2)_2-OH$ |
| 11-196 | 3-F-Ph | 4-Pyr | $SO_2-(CH_2)_2-OMe$ |
| 11-197 | 3-F-Ph | 4-Pyr | $SO_2NH_2$ |
| 11-198 | 3-F-Ph | 4-Pyr | $SO_2NHMe$ |
| 11-199 | 3-F-Ph | 4-Pyr | $SO_2NHEt$ |
| 11-200 | 3-F-Ph | 4-Pyr | $SO_2NHPr$ |
| 11-201 | 3-F-Ph | 4-Pyr | $SO_2NH$-i-Pr |
| 11-202 | 3-F-Ph | 4-Pyr | $SO_2NHBu$ |
| 11-203 | 3-F-Ph | 4-Pyr | $SO_2NHBn$ |
| 11-204 | 3-F-Ph | 4-Pyr | $SO_2NMe_2$ |
| 11-205 | 3-F-Ph | 4-Pyr | $SO_2NH$-c-Pr |
| 11-206 | 3-F-Ph | 4-Pyr | $SO_2NH$-c-Bu |
| 11-207 | 3-F-Ph | 4-Pyr | $SO_2NH$-c-Pen |
| 11-208 | 3-F-Ph | 4-Pyr | $SO_2NH$-c-Hex |
| 11-209 | 3-F-Ph | 4-Pyr | $SO_2NH$-c-Hep |
| 11-210 | 3-F-Ph | 4-Pyr | $SO_2NHCH_2$-c-Pr |
| 11-211 | 3-F-Ph | 4-Pyr | $SO_2NH$-Allyl |
| 11-212 | 3-F-Ph | 4-Pyr | $SO_2NH$-Propargyl |
| 11-213 | 3-F-Ph | 4-Pyr | $SO_2NHPh$ |
| 11-214 | 3-F-Ph | 4-Pyr | $SO_2NH$-3-Pyr |
| 11-215 | 3-F-Ph | 4-Pyr | $SO_2NHCH_2$-4-Pyr |
| 11-216 | 3-F-Ph | 4-Pyr | $SO_2NH-(CH_2)_2-OH$ |
| 11-217 | 3-F-Ph | 4-Pyr | $SO_2NHCH(CH_2OH)_2$ |
| 11-218 | 3-F-Ph | 4-Pyr | $SO_2NH-(CH_2)_2-OAc$ |
| 11-219 | 3-F-Ph | 4-Pyr | $SO_2NHCH_2CN$ |
| 11-220 | 3-F-Ph | 4-Pyr | $SO_2NH-(CH_2)_2-F$ |
| 11-221 | 3-F-Ph | 4-Pyr | $SO_2NH-(CH_2)_2-OMe$ |
| 11-222 | 3-F-Ph | 4-Pyr | $SO_2NH-(CH_2)_2-SMe$ |
| 11-223 | 3-F-Ph | 4-Pyr | $SO_2NH-(CH_2)_2-NH_2$ |
| 11-224 | 3-F-Ph | 4-Pyr | $SO_2NH-(CH_2)_2-NHMe$ |
| 11-225 | 3-F-Ph | 4-Pyr | $SO_2NH-(CH_2)_2-N(Me)_2$ |
| 11-226 | 3-F-Ph | 4-Pyr | $SO_2NHCH_2COOH$ |
| 11-227 | 3-F-Ph | 4-Pyr | $SO_2NHCH_2COOEt$ |
| 11-228 | 3-F-Ph | 4-Pyr | $SO_2NHCH(Me)COOEt$ |

| | | | |
|---|---|---|---|
| 11-229 | 3-F-Ph | 4-Pyr | $SO_2NHCH_2CONH_2$ |
| 11-230 | 3-F-Ph | 4-Pyr | $SO_2NHOH$ |
| 11-231 | 3-F-Ph | 4-Pyr | $SO_2NHOMe$ |
| 11-232 | 3-F-Ph | 4-Pyr | $SO_2NHOEt$ |
| 11-233 | 3-F-Ph | 4-Pyr | $SO_2NHOPr$ |
| 11-234 | 3-F-Ph | 4-Pyr | $SO_2NHOAllyl$ |
| 11-235 | 3-F-Ph | 4-Pyr | $SO_2NHOBn$ |
| 11-236 | 3-F-Ph | 4-Pyr | $SO_2NHNH_2$ |
| 11-237 | 3-F-Ph | 4-Pyr | $SO_2NHNHMe$ |
| 11-238 | 3-F-Ph | 4-Pyr | $SO_2NHN(Me)_2$ |
| 11-239 | 3-Cl-Ph | 4-Pyr | COOH |
| 11-240 | 3-Cl-Ph | 4-Pyr | COOMe |
| 11-241 | 3-Cl-Ph | 4-Pyr | COOEt |
| 11-242 | 3-Cl-Ph | 4-Pyr | COOPr |
| 11-243 | 3-Cl-Ph | 4-Pyr | COO-i-Pr |
| 11-244 | 3-Cl-Ph | 4-Pyr | COOBu |
| 11-245 | 3-Cl-Ph | 4-Pyr | COOBn |
| 11-246 | 3-Cl-Ph | 4-Pyr | COOPh |
| 11-247 | 3-Cl-Ph | 4-Pyr | $CONH_2$ |
| 11-248 | 3-Cl-Ph | 4-Pyr | CONHMe |
| 11-249 | 3-Cl-Ph | 4-Pyr | CONHEt |
| 11-250 | 3-Cl-Ph | 4-Pyr | CONHPr |
| 11-251 | 3-Cl-Ph | 4-Pyr | CONH-i-Pr |
| 11-252 | 3-Cl-Ph | 4-Pyr | CONHBu |
| 11-253 | 3-Cl-Ph | 4-Pyr | CONHBn |
| 11-254 | 3-Cl-Ph | 4-Pyr | $CONMe_2$ |
| 11-255 | 3-Cl-Ph | 4-Pyr | CONH-c-Pr |
| 11-256 | 3-Cl-Ph | 4-Pyr | CONH-c-Bu |
| 11-257 | 3-Cl-Ph | 4-Pyr | CONH-c-Pen |
| 11-258 | 3-Cl-Ph | 4-Pyr | CONH-c-Hex |
| 11-259 | 3-Cl-Ph | 4-Pyr | CONH-c-Hep |
| 11-260 | 3-Cl-Ph | 4-Pyr | $CONHCH_2$-c-Pr |
| 11-261 | 3-Cl-Ph | 4-Pyr | CONH-Allyl |
| 11-262 | 3-Cl-Ph | 4-Pyr | CONH-Propargyl |
| 11-263 | 3-Cl-Ph | 4-Pyr | CONHPh |
| 11-264 | 3-Cl-Ph | 4-Pyr | CONH-3-Pyr |

| | | | |
|---|---|---|---|
| 11-265 | 3-Cl-Ph | 4-Pyr | $CONHCH_2$-4-Pyr |
| 11-266 | 3-Cl-Ph | 4-Pyr | CONH-$(CH_2)_2$-OH |
| 11-267 | 3-Cl-Ph | 4-Pyr | $CONHCH(CH_2OH)_2$ |
| 11-268 | 3-Cl-Ph | 4-Pyr | CONH-$(CH_2)_2$-OAc |
| 11-269 | 3-Cl-Ph | 4-Pyr | $CONHCH_2CN$ |
| 11-270 | 3-Cl-Ph | 4-Pyr | CONH-$(CH_2)_2$-F |
| 11-271 | 3-Cl-Ph | 4-Pyr | CONH-$(CH_2)_2$-OMe |
| 11-272 | 3-Cl-Ph | 4-Pyr | CONH-$(CH_2)_2$-SMe |
| 11-273 | 3-Cl-Ph | 4-Pyr | CONH-$(CH_2)_2$-$NH_2$ |
| 11-274 | 3-Cl-Ph | 4-Pyr | CONH-$(CH_2)_2$-NHMe |
| 11-275 | 3-Cl-Ph | 4-Pyr | CONH-$(CH_2)_2$-$N(Me)_2$ |
| 11-276 | 3-Cl-Ph | 4-Pyr | $CONHCH_2COOH$ |
| 11-277 | 3-Cl-Ph | 4-Pyr | $CONHCH_2COOEt$ |
| 11-278 | 3-Cl-Ph | 4-Pyr | CONHCH(Me)COOEt |
| 11-279 | 3-Cl-Ph | 4-Pyr | $CONHCH_2CONH_2$ |
| 11-280 | 3-Cl-Ph | 4-Pyr | CONHOH |
| 11-281 | 3-Cl-Ph | 4-Pyr | CONHOMe |
| 11-282 | 3-Cl-Ph | 4-Pyr | CONHOEt |
| 11-283 | 3-Cl-Ph | 4-Pyr | CONHOPr |
| 11-284 | 3-Cl-Ph | 4-Pyr | CONHOAllyl |
| 11-285 | 3-Cl-Ph | 4-Pyr | CONHOBn |
| 11-286 | 3-Cl-Ph | 4-Pyr | $CONHNH_2$ |
| 11-287 | 3-Cl-Ph | 4-Pyr | CONHNHMe |
| 11-288 | 3-Cl-Ph | 4-Pyr | $CONHN(Me)_2$ |
| 11-289 | 3-Cl-Ph | 4-Pyr | COMe |
| 11-290 | 3-Cl-Ph | 4-Pyr | COEt |
| 11-291 | 3-Cl-Ph | 4-Pyr | COPr |
| 11-292 | 3-Cl-Ph | 4-Pyr | CO-i-Pr |
| 11-293 | 3-Cl-Ph | 4-Pyr | COBu |
| 11-294 | 3-Cl-Ph | 4-Pyr | $COCF_3$ |
| 11-295 | 3-Cl-Ph | 4-Pyr | CO-$(CH_2)_2$-F |
| 11-296 | 3-Cl-Ph | 4-Pyr | CO-$(CH_2)_2$-OH |
| 11-297 | 3-Cl-Ph | 4-Pyr | CO-$(CH_2)_2$-OMe |
| 11-298 | 3-Cl-Ph | 4-Pyr | SOMe |
| 11-299 | 3-Cl-Ph | 4-Pyr | SOEt |
| 11-300 | 3-Cl-Ph | 4-Pyr | SOPr |

| | | | |
|---|---|---|---|
| 11-301 | 3-Cl-Ph | 4-Pyr | SO-i-Pr |
| 11-302 | 3-Cl-Ph | 4-Pyr | SOBu |
| 11-303 | 3-Cl-Ph | 4-Pyr | $SOCF_3$ |
| 11-304 | 3-Cl-Ph | 4-Pyr | $SO-(CH_2)_2-F$ |
| 11-305 | 3-Cl-Ph | 4-Pyr | $SO-(CH_2)_2-OH$ |
| 11-306 | 3-Cl-Ph | 4-Pyr | $SO-(CH_2)_2-OMe$ |
| 11-307 | 3-Cl-Ph | 4-Pyr | $SO_2Me$ |
| 11-308 | 3-Cl-Ph | 4-Pyr | $SO_2Et$ |
| 11-309 | 3-Cl-Ph | 4-Pyr | $SO_2Pr$ |
| 11-310 | 3-Cl-Ph | 4-Pyr | $SO_2$-i-Pr |
| 11-311 | 3-Cl-Ph | 4-Pyr | $SO_2Bu$ |
| 11-312 | 3-Cl-Ph | 4-Pyr | $SO_2CF_3$ |
| 11-313 | 3-Cl-Ph | 4-Pyr | $SO_2-(CH_2)_2-F$ |
| 11-314 | 3-Cl-Ph | 4-Pyr | $SO_2-(CH_2)_2-OH$ |
| 11-315 | 3-Cl-Ph | 4-Pyr | $SO_2-(CH_2)_2-OMe$ |
| 11-316 | 3-Cl-Ph | 4-Pyr | $SO_2NH_2$ |
| 11-317 | 3-Cl-Ph | 4-Pyr | $SO_2NHMe$ |
| 11-318 | 3-Cl-Ph | 4-Pyr | $SO_2NHEt$ |
| 11-319 | 3-Cl-Ph | 4-Pyr | $SO_2NHPr$ |
| 11-320 | 3-Cl-Ph | 4-Pyr | $SO_2NH$-i-Pr |
| 11-321 | 3-Cl-Ph | 4-Pyr | $SO_2NHBu$ |
| 11-322 | 3-Cl-Ph | 4-Pyr | $SO_2NHBn$ |
| 11-323 | 3-Cl-Ph | 4-Pyr | $SO_2NMe_2$ |
| 11-324 | 3-Cl-Ph | 4-Pyr | $SO_2NH$-c-Pr |
| 11-325 | 3-Cl-Ph | 4-Pyr | $SO_2NH$-c-Bu |
| 11-326 | 3-Cl-Ph | 4-Pyr | $SO_2NH$-c-Pen |
| 11-327 | 3-Cl-Ph | 4-Pyr | $SO_2NH$-c-Hex |
| 11-328 | 3-Cl-Ph | 4-Pyr | $SO_2NH$-c-Hep |
| 11-329 | 3-Cl-Ph | 4-Pyr | $SO_2NHCH_2$-c-Pr |
| 11-330 | 3-Cl-Ph | 4-Pyr | $SO_2NH$-Allyl |
| 11-331 | 3-Cl-Ph | 4-Pyr | $SO_2NH$-Propargyl |
| 11-332 | 3-Cl-Ph | 4-Pyr | $SO_2NHPh$ |
| 11-333 | 3-Cl-Ph | 4-Pyr | $SO_2NH$-3-Pyr |
| 11-334 | 3-Cl-Ph | 4-Pyr | $SO_2NHCH_2$-4-Pyr |
| 11-335 | 3-Cl-Ph | 4-Pyr | $SO_2NH-(CH_2)_2-OH$ |
| 11-336 | 3-Cl-Ph | 4-Pyr | $SO_2NHCH(CH_2OH)_2$ |

| | | | |
|---|---|---|---|
| 11-337 | 3-Cl-Ph | 4-Pyr | $SO_2NH-(CH_2)_2-OAc$ |
| 11-338 | 3-Cl-Ph | 4-Pyr | $SO_2NHCH_2CN$ |
| 11-339 | 3-Cl-Ph | 4-Pyr | $SO_2NH-(CH_2)_2-F$ |
| 11-340 | 3-Cl-Ph | 4-Pyr | $SO_2NH-(CH_2)_2-OMe$ |
| 11-341 | 3-Cl-Ph | 4-Pyr | $SO_2NH-(CH_2)_2-SMe$ |
| 11-342 | 3-Cl-Ph | 4-Pyr | $SO_2NH-(CH_2)_2-NH_2$ |
| 11-343 | 3-Cl-Ph | 4-Pyr | $SO_2NH-(CH_2)_2-NHMe$ |
| 11-344 | 3-Cl-Ph | 4-Pyr | $SO_2NH-(CH_2)_2-N(Me)_2$ |
| 11-345 | 3-Cl-Ph | 4-Pyr | $SO_2NHCH_2COOH$ |
| 11-346 | 3-Cl-Ph | 4-Pyr | $SO_2NHCH_2COOEt$ |
| 11-347 | 3-Cl-Ph | 4-Pyr | $SO_2NHCH(Me)COOEt$ |
| 11-348 | 3-Cl-Ph | 4-Pyr | $SO_2NHCH_2CONH_2$ |
| 11-349 | 3-Cl-Ph | 4-Pyr | $SO_2NHOH$ |
| 11-350 | 3-Cl-Ph | 4-Pyr | $SO_2NHOMe$ |
| 11-351 | 3-Cl-Ph | 4-Pyr | $SO_2NHOEt$ |
| 11-352 | 3-Cl-Ph | 4-Pyr | $SO_2NHOPr$ |
| 11-353 | 3-Cl-Ph | 4-Pyr | $SO_2NHOAllyl$ |
| 11-354 | 3-Cl-Ph | 4-Pyr | $SO_2NHOBn$ |
| 11-355 | 3-Cl-Ph | 4-Pyr | $SO_2NHNH_2$ |
| 11-356 | 3-Cl-Ph | 4-Pyr | $SO_2NHNHMe$ |
| 11-357 | 3-Cl-Ph | 4-Pyr | $SO_2NHN(Me)_2$ |
| 11-358 | 4-F-Ph | 4-Pyr | COOH |
| 11-359 | 4-F-Ph | 4-Pyr | COOMe |
| 11-360 | 4-F-Ph | 4-Pyr | COOEt |
| 11-361 | 4-F-Ph | 4-Pyr | COOPr |
| 11-362 | 4-F-Ph | 4-Pyr | COO-i-Pr |
| 11-363 | 4-F-Ph | 4-Pyr | COOBu |
| 11-364 | 4-F-Ph | 4-Pyr | COOBn |
| 11-365 | 4-F-Ph | 4-Pyr | COOPh |
| 11-366 | 4-F-Ph | 4-Pyr | $CONH_2$ |
| 11-367 | 4-F-Ph | 4-Pyr | CONHMe |
| 11-368 | 4-F-Ph | 4-Pyr | CONHEt |
| 11-369 | 4-F-Ph | 4-Pyr | CONHPr |
| 11-370 | 4-F-Ph | 4-Pyr | CONH-i-Pr |
| 11-371 | 4-F-Ph | 4-Pyr | CONHBu |
| 11-372 | 4-F-Ph | 4-Pyr | CONHBn |

| | | | |
|---|---|---|---|
| 11-373 | 4-F-Ph | 4-Pyr | $CONMe_2$ |
| 11-374 | 4-F-Ph | 4-Pyr | CONH-c-Pr |
| 11-375 | 4-F-Ph | 4-Pyr | CONH-c-Bu |
| 11-376 | 4-F-Ph | 4-Pyr | CONH-c-Pen |
| 11-377 | 4-F-Ph | 4-Pyr | CONH-c-Hex |
| 11-378 | 4-F-Ph | 4-Pyr | CONH-c-Hep |
| 11-379 | 4-F-Ph | 4-Pyr | $CONHCH_2$-c-Pr |
| 11-380 | 4-F-Ph | 4-Pyr | CONH-Allyl |
| 11-381 | 4-F-Ph | 4-Pyr | CONH-Propargyl |
| 11-382 | 4-F-Ph | 4-Pyr | CONHPh |
| 11-383 | 4-F-Ph | 4-Pyr | CONH-3-Pyr |
| 11-384 | 4-F-Ph | 4-Pyr | $CONHCH_2$-4-Pyr |
| 11-385 | 4-F-Ph | 4-Pyr | CONH-$(CH_2)_2$-OH |
| 11-386 | 4-F-Ph | 4-Pyr | $CONHCH(CH_2OH)_2$ |
| 11-387 | 4-F-Ph | 4-Pyr | CONH-$(CH_2)_2$-OAc |
| 11-388 | 4-F-Ph | 4-Pyr | $CONHCH_2CN$ |
| 11-389 | 4-F-Ph | 4-Pyr | CONH-$(CH_2)_2$-F |
| 11-390 | 4-F-Ph | 4-Pyr | CONH-$(CH_2)_2$-OMe |
| 11-391 | 4-F-Ph | 4-Pyr | CONH-$(CH_2)_2$-SMe |
| 11-392 | 4-F-Ph | 4-Pyr | CONH-$(CH_2)_2$-$NH_2$ |
| 11-393 | 4-F-Ph | 4-Pyr | CONH-$(CH_2)_2$-NHMe |
| 11-394 | 4-F-Ph | 4-Pyr | CONH-$(CH_2)_2$-$N(Me)_2$ |
| 11-395 | 4-F-Ph | 4-Pyr | $CONHCH_2COOH$ |
| 11-396 | 4-F-Ph | 4-Pyr | $CONHCH_2COOEt$ |
| 11-397 | 4-F-Ph | 4-Pyr | CONHCH(Me)COOEt |
| 11-398 | 4-F-Ph | 4-Pyr | $CONHCH_2CONH_2$ |
| 11-399 | 4-F-Ph | 4-Pyr | CONHOH |
| 11-400 | 4-F-Ph | 4-Pyr | CONHOMe |
| 11-401 | 4-F-Ph | 4-Pyr | CONHOEt |
| 11-402 | 4-F-Ph | 4-Pyr | CONHOPr |
| 11-403 | 4-F-Ph | 4-Pyr | CONHOAllyl |
| 11-404 | 4-F-Ph | 4-Pyr | CONHOBn |
| 11-405 | 4-F-Ph | 4-Pyr | $CONHNH_2$ |
| 11-406 | 4-F-Ph | 4-Pyr | CONHNHMe |
| 11-407 | 4-F-Ph | 4-Pyr | $CONHN(Me)_2$ |
| 11-408 | 4-F-Ph | 4-Pyr | COMe |

| | | | |
|---|---|---|---|
| 11-409 | 4-F-Ph | 4-Pyr | COEt |
| 11-410 | 4-F-Ph | 4-Pyr | COPr |
| 11-411 | 4-F-Ph | 4-Pyr | CO-i-Pr |
| 11-412 | 4-F-Ph | 4-Pyr | COBu |
| 11-413 | 4-F-Ph | 4-Pyr | $COCF_3$ |
| 11-414 | 4-F-Ph | 4-Pyr | CO-$(CH_2)_2$-F |
| 11-415 | 4-F-Ph | 4-Pyr | CO-$(CH_2)_2$-OH |
| 11-416 | 4-F-Ph | 4-Pyr | CO-$(CH_2)_2$-OMe |
| 11-417 | 4-F-Ph | 4-Pyr | SOMe |
| 11-418 | 4-F-Ph | 4-Pyr | SOEt |
| 11-419 | 4-F-Ph | 4-Pyr | SOPr |
| 11-420 | 4-F-Ph | 4-Pyr | SO-i-Pr |
| 11-421 | 4-F-Ph | 4-Pyr | SOBu |
| 11-422 | 4-F-Ph | 4-Pyr | $SOCF_3$ |
| 11-423 | 4-F-Ph | 4-Pyr | SO-$(CH_2)_2$-F |
| 11-424 | 4-F-Ph | 4-Pyr | SO-$(CH_2)_2$-OH |
| 11-425 | 4-F-Ph | 4-Pyr | SO-$(CH_2)_2$-OMe |
| 11-426 | 4-F-Ph | 4-Pyr | $SO_2Me$ |
| 11-427 | 4-F-Ph | 4-Pyr | $SO_2Et$ |
| 11-428 | 4-F-Ph | 4-Pyr | $SO_2Pr$ |
| 11-429 | 4-F-Ph | 4-Pyr | $SO_2$-i-Pr |
| 11-430 | 4-F-Ph | 4-Pyr | $SO_2Bu$ |
| 11-431 | 4-F-Ph | 4-Pyr | $SO_2CF_3$ |
| 11-432 | 4-F-Ph | 4-Pyr | $SO_2$-$(CH_2)_2$-F |
| 11-433 | 4-F-Ph | 4-Pyr | $SO_2$-$(CH_2)_2$-OH |
| 11-434 | 4-F-Ph | 4-Pyr | $SO_2$-$(CH_2)_2$-OMe |
| 11-435 | 4-F-Ph | 4-Pyr | $SO_2NH_2$ |
| 11-436 | 4-F-Ph | 4-Pyr | $SO_2NHMe$ |
| 11-437 | 4-F-Ph | 4-Pyr | $SO_2NHEt$ |
| 11-438 | 4-F-Ph | 4-Pyr | $SO_2NHPr$ |
| 11-439 | 4-F-Ph | 4-Pyr | $SO_2NH$-i-Pr |
| 11-440 | 4-F-Ph | 4-Pyr | $SO_2NHBu$ |
| 11-441 | 4-F-Ph | 4-Pyr | $SO_2NHBn$ |
| 11-442 | 4-F-Ph | 4-Pyr | $SO_2NMe_2$ |
| 11-443 | 4-F-Ph | 4-Pyr | $SO_2NH$-c-Pr |
| 11-444 | 4-F-Ph | 4-Pyr | $SO_2NH$-c-Bu |

| | | | |
|---|---|---|---|
| 11-445 | 4-F-Ph | 4-Pyr | $SO_2NH$-c-Pen |
| 11-446 | 4-F-Ph | 4-Pyr | $SO_2NH$-c-Hex |
| 11-447 | 4-F-Ph | 4-Pyr | $SO_2NH$-c-Hep |
| 11-448 | 4-F-Ph | 4-Pyr | $SO_2NHCH_2$-c-Pr |
| 11-449 | 4-F-Ph | 4-Pyr | $SO_2NH$-Allyl |
| 11-450 | 4-F-Ph | 4-Pyr | $SO_2NH$-Propargyl |
| 11-451 | 4-F-Ph | 4-Pyr | $SO_2NHPh$ |
| 11-452 | 4-F-Ph | 4-Pyr | $SO_2NH$-3-Pyr |
| 11-453 | 4-F-Ph | 4-Pyr | $SO_2NHCH_2$-4-Pyr |
| 11-454 | 4-F-Ph | 4-Pyr | $SO_2NH-(CH_2)_2-OH$ |
| 11-455 | 4-F-Ph | 4-Pyr | $SO_2NHCH(CH_2OH)_2$ |
| 11-456 | 4-F-Ph | 4-Pyr | $SO_2NH-(CH_2)_2-OAc$ |
| 11-457 | 4-F-Ph | 4-Pyr | $SO_2NHCH_2CN$ |
| 11-458 | 4-F-Ph | 4-Pyr | $SO_2NH-(CH_2)_2-F$ |
| 11-459 | 4-F-Ph | 4-Pyr | $SO_2NH-(CH_2)_2-OMe$ |
| 11-460 | 4-F-Ph | 4-Pyr | $SO_2NH-(CH_2)_2-SMe$ |
| 11-461 | 4-F-Ph | 4-Pyr | $SO_2NH-(CH_2)_2-NH_2$ |
| 11-462 | 4-F-Ph | 4-Pyr | $SO_2NH-(CH_2)_2-NHMe$ |
| 11-463 | 4-F-Ph | 4-Pyr | $SO_2NH-(CH_2)_2-N(Me)_2$ |
| 11-464 | 4-F-Ph | 4-Pyr | $SO_2NHCH_2COOH$ |
| 11-465 | 4-F-Ph | 4-Pyr | $SO_2NHCH_2COOEt$ |
| 11-466 | 4-F-Ph | 4-Pyr | $SO_2NHCH(Me)COOEt$ |
| 11-467 | 4-F-Ph | 4-Pyr | $SO_2NHCH_2CONH_2$ |
| 11-468 | 4-F-Ph | 4-Pyr | $SO_2NHOH$ |
| 11-469 | 4-F-Ph | 4-Pyr | $SO_2NHOMe$ |
| 11-470 | 4-F-Ph | 4-Pyr | $SO_2NHOEt$ |
| 11-471 | 4-F-Ph | 4-Pyr | $SO_2NHOPr$ |
| 11-472 | 4-F-Ph | 4-Pyr | $SO_2NHOAllyl$ |
| 11-473 | 4-F-Ph | 4-Pyr | $SO_2NHOBn$ |
| 11-474 | 4-F-Ph | 4-Pyr | $SO_2NHNH_2$ |
| 11-475 | 4-F-Ph | 4-Pyr | $SO_2NHNHMe$ |
| 11-476 | 4-F-Ph | 4-Pyr | $SO_2NHN(Me)_2$ |
| 11-477 | 3-Cl-4-F-Ph | 4-Pyr | COOH |
| 11-478 | 3-Cl-4-F-Ph | 4-Pyr | COOMe |
| 11-479 | 3-Cl-4-F-Ph | 4-Pyr | COOEt |
| 11-480 | 3-Cl-4-F-Ph | 4-Pyr | COOPr |

| | | | |
|---|---|---|---|
| 11-481 | 3-Cl-4-F-Ph | 4-Pyr | COO-i-Pr |
| 11-482 | 3-Cl-4-F-Ph | 4-Pyr | COOBu |
| 11-483 | 3-Cl-4-F-Ph | 4-Pyr | COOBn |
| 11-484 | 3-Cl-4-F-Ph | 4-Pyr | COOPh |
| 11-485 | 3-Cl-4-F-Ph | 4-Pyr | $CONH_2$ |
| 11-486 | 3-Cl-4-F-Ph | 4-Pyr | CONHMe |
| 11-487 | 3-Cl-4-F-Ph | 4-Pyr | CONHEt |
| 11-488 | 3-Cl-4-F-Ph | 4-Pyr | CONHPr |
| 11-489 | 3-Cl-4-F-Ph | 4-Pyr | CONH-i-Pr |
| 11-490 | 3-Cl-4-F-Ph | 4-Pyr | CONHBu |
| 11-491 | 3-Cl-4-F-Ph | 4-Pyr | CONHBn |
| 11-492 | 3-Cl-4-F-Ph | 4-Pyr | $CONMe_2$ |
| 11-493 | 3-Cl-4-F-Ph | 4-Pyr | CONH-c-Pr |
| 11-494 | 3-Cl-4-F-Ph | 4-Pyr | CONH-c-Bu |
| 11-495 | 3-Cl-4-F-Ph | 4-Pyr | CONH-c-Pen |
| 11-496 | 3-Cl-4-F-Ph | 4-Pyr | CONH-c-Hex |
| 11-497 | 3-Cl-4-F-Ph | 4-Pyr | CONH-c-Hep |
| 11-498 | 3-Cl-4-F-Ph | 4-Pyr | $CONHCH_2$-c-Pr |
| 11-499 | 3-Cl-4-F-Ph | 4-Pyr | CONH-Allyl |
| 11-500 | 3-Cl-4-F-Ph | 4-Pyr | CONH-Propargyl |
| 11-501 | 3-Cl-4-F-Ph | 4-Pyr | CONHPh |
| 11-502 | 3-Cl-4-F-Ph | 4-Pyr | CONH-3-Pyr |
| 11-503 | 3-Cl-4-F-Ph | 4-Pyr | $CONHCH_2$-4-Pyr |
| 11-504 | 3-Cl-4-F-Ph | 4-Pyr | CONH-$(CH_2)_2$-OH |
| 11-505 | 3-Cl-4-F-Ph | 4-Pyr | $CONHCH(CH_2OH)_2$ |
| 11-506 | 3-Cl-4-F-Ph | 4-Pyr | CONH-$(CH_2)_2$-OAc |
| 11-507 | 3-Cl-4-F-Ph | 4-Pyr | $CONHCH_2CN$ |
| 11-508 | 3-Cl-4-F-Ph | 4-Pyr | CONH-$(CH_2)_2$-F |
| 11-509 | 3-Cl-4-F-Ph | 4-Pyr | CONH-$(CH_2)_2$-OMe |
| 11-510 | 3-Cl-4-F-Ph | 4-Pyr | CONH-$(CH_2)_2$-SMe |
| 11-511 | 3-Cl-4-F-Ph | 4-Pyr | CONH-$(CH_2)_2$-$NH_2$ |
| 11-512 | 3-Cl-4-F-Ph | 4-Pyr | CONH-$(CH_2)_2$-NHMe |
| 11-513 | 3-Cl-4-F-Ph | 4-Pyr | CONH-$(CH_2)_2$-$N(Me)_2$ |
| 11-514 | 3-Cl-4-F-Ph | 4-Pyr | $CONHCH_2COOH$ |
| 11-515 | 3-Cl-4-F-Ph | 4-Pyr | $CONHCH_2COOEt$ |
| 11-516 | 3-Cl-4-F-Ph | 4-Pyr | CONHCH(Me)COOEt |

| | | | |
|---|---|---|---|
| 11-517 | 3-Cl-4-F-Ph | 4-Pyr | $CONHCH_2CONH_2$ |
| 11-518 | 3-Cl-4-F-Ph | 4-Pyr | CONHOH |
| 11-519 | 3-Cl-4-F-Ph | 4-Pyr | CONHOMe |
| 11-520 | 3-Cl-4-F-Ph | 4-Pyr | CONHOEt |
| 11-521 | 3-Cl-4-F-Ph | 4-Pyr | CONHOPr |
| 11-522 | 3-Cl-4-F-Ph | 4-Pyr | CONHOAllyl |
| 11-523 | 3-Cl-4-F-Ph | 4-Pyr | CONHOBn |
| 11-524 | 3-Cl-4-F-Ph | 4-Pyr | $CONHNH_2$ |
| 11-525 | 3-Cl-4-F-Ph | 4-Pyr | CONHNHMe |
| 11-526 | 3-Cl-4-F-Ph | 4-Pyr | $CONHN(Me)_2$ |
| 11-527 | 3-Cl-4-F-Ph | 4-Pyr | COMe |
| 11-528 | 3-Cl-4-F-Ph | 4-Pyr | COEt |
| 11-529 | 3-Cl-4-F-Ph | 4-Pyr | COPr |
| 11-530 | 3-Cl-4-F-Ph | 4-Pyr | CO-i-Pr |
| 11-531 | 3-Cl-4-F-Ph | 4-Pyr | COBu |
| 11-532 | 3-Cl-4-F-Ph | 4-Pyr | $COCF_3$ |
| 11-533 | 3-Cl-4-F-Ph | 4-Pyr | $CO-(CH_2)_2-F$ |
| 11-534 | 3-Cl-4-F-Ph | 4-Pyr | $CO-(CH_2)_2-OH$ |
| 11-535 | 3-Cl-4-F-Ph | 4-Pyr | $CO-(CH_2)_2-OMe$ |
| 11-536 | 3-Cl-4-F-Ph | 4-Pyr | SOMe |
| 11-537 | 3-Cl-4-F-Ph | 4-Pyr | SOEt |
| 11-538 | 3-Cl-4-F-Ph | 4-Pyr | SOPr |
| 11-539 | 3-Cl-4-F-Ph | 4-Pyr | SO-i-Pr |
| 11-540 | 3-Cl-4-F-Ph | 4-Pyr | SOBu |
| 11-541 | 3-Cl-4-F-Ph | 4-Pyr | $SOCF_3$ |
| 11-542 | 3-Cl-4-F-Ph | 4-Pyr | $SO-(CH_2)_2-F$ |
| 11-543 | 3-Cl-4-F-Ph | 4-Pyr | $SO-(CH_2)_2-OH$ |
| 11-544 | 3-Cl-4-F-Ph | 4-Pyr | $SO-(CH_2)_2-OMe$ |
| 11-545 | 3-Cl-4-F-Ph | 4-Pyr | $SO_2Me$ |
| 11-546 | 3-Cl-4-F-Ph | 4-Pyr | $SO_2Et$ |
| 11-547 | 3-Cl-4-F-Ph | 4-Pyr | $SO_2Pr$ |
| 11-548 | 3-Cl-4-F-Ph | 4-Pyr | $SO_2$-i-Pr |
| 11-549 | 3-Cl-4-F-Ph | 4-Pyr | $SO_2Bu$ |
| 11-550 | 3-Cl-4-F-Ph | 4-Pyr | $SO_2CF_3$ |
| 11-551 | 3-Cl-4-F-Ph | 4-Pyr | $SO_2-(CH_2)_2-F$ |
| 11-552 | 3-Cl-4-F-Ph | 4-Pyr | $SO_2-(CH_2)_2-OH$ |

| | | | |
|---|---|---|---|
| 11-553 | 3-Cl-4-F-Ph | 4-Pyr | $SO_2$-$(CH_2)_2$-OMe |
| 11-554 | 3-Cl-4-F-Ph | 4-Pyr | $SO_2NH_2$ |
| 11-555 | 3-Cl-4-F-Ph | 4-Pyr | $SO_2$NHMe |
| 11-556 | 3-Cl-4-F-Ph | 4-Pyr | $SO_2$NHEt |
| 11-557 | 3-Cl-4-F-Ph | 4-Pyr | $SO_2$NHPr |
| 11-558 | 3-Cl-4-F-Ph | 4-Pyr | $SO_2$NH-i-Pr |
| 11-559 | 3-Cl-4-F-Ph | 4-Pyr | $SO_2$NHBu |
| 11-560 | 3-Cl-4-F-Ph | 4-Pyr | $SO_2$NHBn |
| 11-561 | 3-Cl-4-F-Ph | 4-Pyr | $SO_2NMe_2$ |
| 11-562 | 3-Cl-4-F-Ph | 4-Pyr | $SO_2$NH-c-Pr |
| 11-563 | 3-Cl-4-F-Ph | 4-Pyr | $SO_2$NH-c-Bu |
| 11-564 | 3-Cl-4-F-Ph | 4-Pyr | $SO_2$NH-c-Pen |
| 11-565 | 3-Cl-4-F-Ph | 4-Pyr | $SO_2$NH-c-Hex |
| 11-566 | 3-Cl-4-F-Ph | 4-Pyr | $SO_2$NH-c-Hep |
| 11-567 | 3-Cl-4-F-Ph | 4-Pyr | $SO_2NHCH_2$-c-Pr |
| 11-568 | 3-Cl-4-F-Ph | 4-Pyr | $SO_2$NH-Allyl |
| 11-569 | 3-Cl-4-F-Ph | 4-Pyr | $SO_2$NH-Propargyl |
| 11-570 | 3-Cl-4-F-Ph | 4-Pyr | $SO_2$NHPh |
| 11-571 | 3-Cl-4-F-Ph | 4-Pyr | $SO_2$NH-3-Pyr |
| 11-572 | 3-Cl-4-F-Ph | 4-Pyr | $SO_2NHCH_2$-4-Pyr |
| 11-573 | 3-Cl-4-F-Ph | 4-Pyr | $SO_2$NH-$(CH_2)_2$-OH |
| 11-574 | 3-Cl-4-F-Ph | 4-Pyr | $SO_2NHCH(CH_2OH)_2$ |
| 11-575 | 3-Cl-4-F-Ph | 4-Pyr | $SO_2$NH-$(CH_2)_2$-OAc |
| 11-576 | 3-Cl-4-F-Ph | 4-Pyr | $SO_2NHCH_2CN$ |
| 11-577 | 3-Cl-4-F-Ph | 4-Pyr | $SO_2$NH-$(CH_2)_2$-F |
| 11-578 | 3-Cl-4-F-Ph | 4-Pyr | $SO_2$NH-$(CH_2)_2$-OMe |
| 11-579 | 3-Cl-4-F-Ph | 4-Pyr | $SO_2$NH-$(CH_2)_2$-SMe |
| 11-580 | 3-Cl-4-F-Ph | 4-Pyr | $SO_2$NH-$(CH_2)_2$-$NH_2$ |
| 11-581 | 3-Cl-4-F-Ph | 4-Pyr | $SO_2$NH-$(CH_2)_2$-NHMe |
| 11-582 | 3-Cl-4-F-Ph | 4-Pyr | $SO_2$NH-$(CH_2)_2$-$N(Me)_2$ |
| 11-583 | 3-Cl-4-F-Ph | 4-Pyr | $SO_2NHCH_2COOH$ |
| 11-584 | 3-Cl-4-F-Ph | 4-Pyr | $SO_2NHCH_2COOEt$ |
| 11-585 | 3-Cl-4-F-Ph | 4-Pyr | $SO_2$NHCH(Me)COOEt |
| 11-586 | 3-Cl-4-F-Ph | 4-Pyr | $SO_2NHCH_2CONH_2$ |
| 11-587 | 3-Cl-4-F-Ph | 4-Pyr | $SO_2$NHOH |
| 11-588 | 3-Cl-4-F-Ph | 4-Pyr | $SO_2$NHOMe |

| | | | |
|---|---|---|---|
| 11-589 | 3-Cl-4-F-Ph | 4-Pyr | $SO_2NHOEt$ |
| 11-590 | 3-Cl-4-F-Ph | 4-Pyr | $SO_2NHOPr$ |
| 11-591 | 3-Cl-4-F-Ph | 4-Pyr | $SO_2NHOAllyl$ |
| 11-592 | 3-Cl-4-F-Ph | 4-Pyr | $SO_2NHOBn$ |
| 11-593 | 3-Cl-4-F-Ph | 4-Pyr | $SO_2NHNH_2$ |
| 11-594 | 3-Cl-4-F-Ph | 4-Pyr | $SO_2NHNHMe$ |
| 11-595 | 3-Cl-4-F-Ph | 4-Pyr | $SO_2NHN(Me)_2$ |
| 11-596 | 3,4-$F_2$-Ph | 4-Pyr | COOH |
| 11-597 | 3,4-$F_2$-Ph | 4-Pyr | COOMe |
| 11-598 | 3,4-$F_2$-Ph | 4-Pyr | COOEt |
| 11-599 | 3,4-$F_2$-Ph | 4-Pyr | COOPr |
| 11-600 | 3,4-$F_2$-Ph | 4-Pyr | COO-i-Pr |
| 11-601 | 3,4-$F_2$-Ph | 4-Pyr | COOBu |
| 11-602 | 3,4-$F_2$-Ph | 4-Pyr | COOBn |
| 11-603 | 3,4-$F_2$-Ph | 4-Pyr | COOPh |
| 11-604 | 3,4-$F_2$-Ph | 4-Pyr | $CONH_2$ |
| 11-605 | 3,4-$F_2$-Ph | 4-Pyr | CONHMe |
| 11-606 | 3,4-$F_2$-Ph | 4-Pyr | CONHEt |
| 11-607 | 3,4-$F_2$-Ph | 4-Pyr | CONHPr |
| 11-608 | 3,4-$F_2$-Ph | 4-Pyr | CONH-i-Pr |
| 11-609 | 3,4-$F_2$-Ph | 4-Pyr | CONHBu |
| 11-610 | 3,4-$F_2$-Ph | 4-Pyr | CONHBn |
| 11-611 | 3,4-$F_2$-Ph | 4-Pyr | $CONMe_2$ |
| 11-612 | 3,4-$F_2$-Ph | 4-Pyr | CONH-c-Pr |
| 11-613 | 3,4-$F_2$-Ph | 4-Pyr | CONH-c-Bu |
| 11-614 | 3,4-$F_2$-Ph | 4-Pyr | CONH-c-Pen |
| 11-615 | 3,4-$F_2$-Ph | 4-Pyr | CONH-c-Hex |
| 11-616 | 3,4-$F_2$-Ph | 4-Pyr | CONH-c-Hep |
| 11-617 | 3,4-$F_2$-Ph | 4-Pyr | $CONHCH_2$-c-Pr |
| 11-618 | 3,4-$F_2$-Ph | 4-Pyr | CONH-Allyl |
| 11-619 | 3,4-$F_2$-Ph | 4-Pyr | CONH-Propargyl |
| 11-620 | 3,4-$F_2$-Ph | 4-Pyr | CONHPh |
| 11-621 | 3,4-$F_2$-Ph | 4-Pyr | CONH-3-Pyr |
| 11-622 | 3,4-$F_2$-Ph | 4-Pyr | $CONHCH_2$-4-Pyr |
| 11-623 | 3,4-$F_2$-Ph | 4-Pyr | CONH-$(CH_2)_2$-OH |
| 11-624 | 3,4-$F_2$-Ph | 4-Pyr | $CONHCH(CH_2OH)_2$ |

| | | | |
|---|---|---|---|
| 11-625 | 3,4-$F_2$-Ph | 4-Pyr | CONH-$(CH_2)_2$-OAc |
| 11-626 | 3,4-$F_2$-Ph | 4-Pyr | $CONHCH_2CN$ |
| 11-627 | 3,4-$F_2$-Ph | 4-Pyr | CONH-$(CH_2)_2$-F |
| 11-628 | 3,4-$F_2$-Ph | 4-Pyr | CONH-$(CH_2)_2$-OMe |
| 11-629 | 3,4-$F_2$-Ph | 4-Pyr | CONH-$(CH_2)_2$-SMe |
| 11-630 | 3,4-$F_2$-Ph | 4-Pyr | CONH-$(CH_2)_2$-$NH_2$ |
| 11-631 | 3,4-$F_2$-Ph | 4-Pyr | CONH-$(CH_2)_2$-NHMe |
| 11-632 | 3,4-$F_2$-Ph | 4-Pyr | CONH-$(CH_2)_2$-N$(Me)_2$ |
| 11-633 | 3,4-$F_2$-Ph | 4-Pyr | $CONHCH_2COOH$ |
| 11-634 | 3,4-$F_2$-Ph | 4-Pyr | $CONHCH_2COOEt$ |
| 11-635 | 3,4-$F_2$-Ph | 4-Pyr | CONHCH(Me)COOEt |
| 11-636 | 3,4-$F_2$-Ph | 4-Pyr | $CONHCH_2CONH_2$ |
| 11-637 | 3,4-$F_2$-Ph | 4-Pyr | CONHOH |
| 11-638 | 3,4-$F_2$-Ph | 4-Pyr | CONHOMe |
| 11-639 | 3,4-$F_2$-Ph | 4-Pyr | CONHOEt |
| 11-640 | 3,4-$F_2$-Ph | 4-Pyr | CONHOPr |
| 11-641 | 3,4-$F_2$-Ph | 4-Pyr | CONHOAllyl |
| 11-642 | 3,4-$F_2$-Ph | 4-Pyr | CONHOBn |
| 11-643 | 3,4-$F_2$-Ph | 4-Pyr | $CONHNH_2$ |
| 11-644 | 3,4-$F_2$-Ph | 4-Pyr | CONHNHMe |
| 11-645 | 3,4-$F_2$-Ph | 4-Pyr | CONHN$(Me)_2$ |
| 11-646 | 3,4-$F_2$-Ph | 4-Pyr | COMe |
| 11-647 | 3,4-$F_2$-Ph | 4-Pyr | COEt |
| 11-648 | 3,4-$F_2$-Ph | 4-Pyr | COPr |
| 11-649 | 3,4-$F_2$-Ph | 4-Pyr | CO-i-Pr |
| 11-650 | 3,4-$F_2$-Ph | 4-Pyr | COBu |
| 11-651 | 3,4-$F_2$-Ph | 4-Pyr | $COCF_3$ |
| 11-652 | 3,4-$F_2$-Ph | 4-Pyr | CO-$(CH_2)_2$-F |
| 11-653 | 3,4-$F_2$-Ph | 4-Pyr | CO-$(CH_2)_2$-OH |
| 11-654 | 3,4-$F_2$-Ph | 4-Pyr | CO-$(CH_2)_2$-OMe |
| 11-655 | 3,4-$F_2$-Ph | 4-Pyr | SOMe |
| 11-656 | 3,4-$F_2$-Ph | 4-Pyr | SOEt |
| 11-657 | 3,4-$F_2$-Ph | 4-Pyr | SOPr |
| 11-658 | 3,4-$F_2$-Ph | 4-Pyr | SO-i-Pr |
| 11-659 | 3,4-$F_2$-Ph | 4-Pyr | SOBu |
| 11-660 | 3,4-$F_2$-Ph | 4-Pyr | $SOCF_3$ |

| | | | |
|---|---|---|---|
| 11-661 | 3,4-$F_2$-Ph | 4-Pyr | SO-$(CH_2)_2$-F |
| 11-662 | 3,4-$F_2$-Ph | 4-Pyr | SO-$(CH_2)_2$-OH |
| 11-663 | 3,4-$F_2$-Ph | 4-Pyr | SO-$(CH_2)_2$-OMe |
| 11-664 | 3,4-$F_2$-Ph | 4-Pyr | $SO_2$Me |
| 11-665 | 3,4-$F_2$-Ph | 4-Pyr | $SO_2$Et |
| 11-666 | 3,4-$F_2$-Ph | 4-Pyr | $SO_2$Pr |
| 11-667 | 3,4-$F_2$-Ph | 4-Pyr | $SO_2$-i-Pr |
| 11-668 | 3,4-$F_2$-Ph | 4-Pyr | $SO_2$Bu |
| 11-669 | 3,4-$F_2$-Ph | 4-Pyr | $SO_2CF_3$ |
| 11-670 | 3,4-$F_2$-Ph | 4-Pyr | $SO_2$-$(CH_2)_2$-F |
| 11-671 | 3,4-$F_2$-Ph | 4-Pyr | $SO_2$-$(CH_2)_2$-OH |
| 11-672 | 3,4-$F_2$-Ph | 4-Pyr | $SO_2$-$(CH_2)_2$-OMe |
| 11-673 | 3,4-$F_2$-Ph | 4-Pyr | $SO_2NH_2$ |
| 11-674 | 3,4-$F_2$-Ph | 4-Pyr | $SO_2$NHMe |
| 11-675 | 3,4-$F_2$-Ph | 4-Pyr | $SO_2$NHEt |
| 11-676 | 3,4-$F_2$-Ph | 4-Pyr | $SO_2$NHPr |
| 11-677 | 3,4-$F_2$-Ph | 4-Pyr | $SO_2$NH-i-Pr |
| 11-678 | 3,4-$F_2$-Ph | 4-Pyr | $SO_2$NHBu |
| 11-679 | 3,4-$F_2$-Ph | 4-Pyr | $SO_2$NHBn |
| 11-680 | 3,4-$F_2$-Ph | 4-Pyr | $SO_2NMe_2$ |
| 11-681 | 3,4-$F_2$-Ph | 4-Pyr | $SO_2$NH-c-Pr |
| 11-682 | 3,4-$F_2$-Ph | 4-Pyr | $SO_2$NH-c-Bu |
| 11-683 | 3,4-$F_2$-Ph | 4-Pyr | $SO_2$NH-c-Pen |
| 11-684 | 3,4-$F_2$-Ph | 4-Pyr | $SO_2$NH-c-Hex |
| 11-685 | 3,4-$F_2$-Ph | 4-Pyr | $SO_2$NH-c-Hep |
| 11-686 | 3,4-$F_2$-Ph | 4-Pyr | $SO_2NHCH_2$-c-Pr |
| 11-687 | 3,4-$F_2$-Ph | 4-Pyr | $SO_2$NH-Allyl |
| 11-688 | 3,4-$F_2$-Ph | 4-Pyr | $SO_2$NH-Propargyl |
| 11-689 | 3,4-$F_2$-Ph | 4-Pyr | $SO_2$NHPh |
| 11-690 | 3,4-$F_2$-Ph | 4-Pyr | $SO_2$NH-3-Pyr |
| 11-691 | 3,4-$F_2$-Ph | 4-Pyr | $SO_2NHCH_2$-4-Pyr |
| 11-692 | 3,4-$F_2$-Ph | 4-Pyr | $SO_2$NH-$(CH_2)_2$-OH |
| 11-693 | 3,4-$F_2$-Ph | 4-Pyr | $SO_2NHCH(CH_2OH)_2$ |
| 11-694 | 3,4-$F_2$-Ph | 4-Pyr | $SO_2$NH-$(CH_2)_2$-OAc |
| 11-695 | 3,4-$F_2$-Ph | 4-Pyr | $SO_2NHCH_2CN$ |
| 11-696 | 3,4-$F_2$-Ph | 4-Pyr | $SO_2$NH-$(CH_2)_2$-F |

| | | | |
|---|---|---|---|
| 11-697 | 3,4-$F_2$-Ph | 4-Pyr | $SO_2NH-(CH_2)_2$-OMe |
| 11-698 | 3,4-$F_2$-Ph | 4-Pyr | $SO_2NH-(CH_2)_2$-SMe |
| 11-699 | 3,4-$F_2$-Ph | 4-Pyr | $SO_2NH-(CH_2)_2-NH_2$ |
| 11-700 | 3,4-$F_2$-Ph | 4-Pyr | $SO_2NH-(CH_2)_2$-NHMe |
| 11-701 | 3,4-$F_2$-Ph | 4-Pyr | $SO_2NH-(CH_2)_2-N(Me)_2$ |
| 11-702 | 3,4-$F_2$-Ph | 4-Pyr | $SO_2NHCH_2COOH$ |
| 11-703 | 3,4-$F_2$-Ph | 4-Pyr | $SO_2NHCH_2COOEt$ |
| 11-704 | 3,4-$F_2$-Ph | 4-Pyr | $SO_2NHCH(Me)COOEt$ |
| 11-705 | 3,4-$F_2$-Ph | 4-Pyr | $SO_2NHCH_2CONH_2$ |
| 11-706 | 3,4-$F_2$-Ph | 4-Pyr | $SO_2NHOH$ |
| 11-707 | 3,4-$F_2$-Ph | 4-Pyr | $SO_2NHOMe$ |
| 11-708 | 3,4-$F_2$-Ph | 4-Pyr | $SO_2NHOEt$ |
| 11-709 | 3,4-$F_2$-Ph | 4-Pyr | $SO_2NHOPr$ |
| 11-710 | 3,4-$F_2$-Ph | 4-Pyr | $SO_2NHOAllyl$ |
| 11-711 | 3,4-$F_2$-Ph | 4-Pyr | $SO_2NHOBn$ |
| 11-712 | 3,4-$F_2$-Ph | 4-Pyr | $SO_2NHNH_2$ |
| 11-713 | 3,4-$F_2$-Ph | 4-Pyr | $SO_2NHNHMe$ |
| 11-714 | 3,4-$F_2$-Ph | 4-Pyr | $SO_2NHN(Me)_2$ |
| 11-715 | 3-$CF_3$-Ph | 4-Pyr | COOH |
| 11-716 | 3-$CF_3$-Ph | 4-Pyr | COOMe |
| 11-717 | 3-$CF_3$-Ph | 4-Pyr | COOEt |
| 11-718 | 3-$CF_3$-Ph | 4-Pyr | COOPr |
| 11-719 | 3-$CF_3$-Ph | 4-Pyr | COO-i-Pr |
| 11-720 | 3-$CF_3$-Ph | 4-Pyr | COOBu |
| 11-721 | 3-$CF_3$-Ph | 4-Pyr | COOBn |
| 11-722 | 3-$CF_3$-Ph | 4-Pyr | COOPh |
| 11-723 | 3-$CF_3$-Ph | 4-Pyr | $CONH_2$ |
| 11-724 | 3-$CF_3$-Ph | 4-Pyr | CONHMe |
| 11-725 | 3-$CF_3$-Ph | 4-Pyr | CONHEt |
| 11-726 | 3-$CF_3$-Ph | 4-Pyr | CONHPr |
| 11-727 | 3-$CF_3$-Ph | 4-Pyr | CONH-i-Pr |
| 11-728 | 3-$CF_3$-Ph | 4-Pyr | CONHBu |
| 11-729 | 3-$CF_3$-Ph | 4-Pyr | CONHBn |
| 11-730 | 3-$CF_3$-Ph | 4-Pyr | $CONMe_2$ |
| 11-731 | 3-$CF_3$-Ph | 4-Pyr | CONH-c-Pr |
| 11-732 | 3-$CF_3$-Ph | 4-Pyr | CONH-c-Bu |

| | | | |
|---|---|---|---|
| 11-733 | 3-$CF_3$-Ph | 4-Pyr | CONH-c-Pen |
| 11-734 | 3-$CF_3$-Ph | 4-Pyr | CONH-c-Hex |
| 11-735 | 3-$CF_3$-Ph | 4-Pyr | CONH-c-Hep |
| 11-736 | 3-$CF_3$-Ph | 4-Pyr | $CONHCH_2$-c-Pr |
| 11-737 | 3-$CF_3$-Ph | 4-Pyr | CONH-Allyl |
| 11-738 | 3-$CF_3$-Ph | 4-Pyr | CONH-Propargyl |
| 11-739 | 3-$CF_3$-Ph | 4-Pyr | CONHPh |
| 11-740 | 3-$CF_3$-Ph | 4-Pyr | CONH-3-Pyr |
| 11-741 | 3-$CF_3$-Ph | 4-Pyr | $CONHCH_2$-4-Pyr |
| 11-742 | 3-$CF_3$-Ph | 4-Pyr | CONH-$(CH_2)_2$-OH |
| 11-743 | 3-$CF_3$-Ph | 4-Pyr | $CONHCH(CH_2OH)_2$ |
| 11-744 | 3-$CF_3$-Ph | 4-Pyr | CONH-$(CH_2)_2$-OAc |
| 11-745 | 3-$CF_3$-Ph | 4-Pyr | $CONHCH_2CN$ |
| 11-746 | 3-$CF_3$-Ph | 4-Pyr | CONH-$(CH_2)_2$-F |
| 11-747 | 3-$CF_3$-Ph | 4-Pyr | CONH-$(CH_2)_2$-OMe |
| 11-748 | 3-$CF_3$-Ph | 4-Pyr | CONH-$(CH_2)_2$-SMe |
| 11-749 | 3-$CF_3$-Ph | 4-Pyr | CONH-$(CH_2)_2$-$NH_2$ |
| 11-750 | 3-$CF_3$-Ph | 4-Pyr | CONH-$(CH_2)_2$-NHMe |
| 11-751 | 3-$CF_3$-Ph | 4-Pyr | CONH-$(CH_2)_2$-$N(Me)_2$ |
| 11-752 | 3-$CF_3$-Ph | 4-Pyr | $CONHCH_2COOH$ |
| 11-753 | 3-$CF_3$-Ph | 4-Pyr | $CONHCH_2COOEt$ |
| 11-754 | 3-$CF_3$-Ph | 4-Pyr | CONHCH(Me)COOEt |
| 11-755 | 3-$CF_3$-Ph | 4-Pyr | $CONHCH_2CONH_2$ |
| 11-756 | 3-$CF_3$-Ph | 4-Pyr | CONHOH |
| 11-757 | 3-$CF_3$-Ph | 4-Pyr | CONHOMe |
| 11-758 | 3-$CF_3$-Ph | 4-Pyr | CONHOEt |
| 11-759 | 3-$CF_3$-Ph | 4-Pyr | CONHOPr |
| 11-760 | 3-$CF_3$-Ph | 4-Pyr | CONHOAllyl |
| 11-761 | 3-$CF_3$-Ph | 4-Pyr | CONHOBn |
| 11-762 | 3-$CF_3$-Ph | 4-Pyr | $CONHNH_2$ |
| 11-763 | 3-$CF_3$-Ph | 4-Pyr | CONHNHMe |
| 11-764 | 3-$CF_3$-Ph | 4-Pyr | $CONHN(Me)_2$ |
| 11-765 | 3-$CF_3$-Ph | 4-Pyr | COMe |
| 11-766 | 3-$CF_3$-Ph | 4-Pyr | COEt |
| 11-767 | 3-$CF_3$-Ph | 4-Pyr | COPr |
| 11-768 | 3-$CF_3$-Ph | 4-Pyr | CO-i-Pr |

| | | | |
|---|---|---|---|
| 11-769 | 3-$CF_3$-Ph | 4-Pyr | COBu |
| 11-770 | 3-$CF_3$-Ph | 4-Pyr | $COCF_3$ |
| 11-771 | 3-$CF_3$-Ph | 4-Pyr | CO-$(CH_2)_2$-F |
| 11-772 | 3-$CF_3$-Ph | 4-Pyr | CO-$(CH_2)_2$-OH |
| 11-773 | 3-$CF_3$-Ph | 4-Pyr | CO-$(CH_2)_2$-OMe |
| 11-774 | 3-$CF_3$-Ph | 4-Pyr | SOMe |
| 11-775 | 3-$CF_3$-Ph | 4-Pyr | SOEt |
| 11-776 | 3-$CF_3$-Ph | 4-Pyr | SOPr |
| 11-777 | 3-$CF_3$-Ph | 4-Pyr | SO-i-Pr |
| 11-778 | 3-$CF_3$-Ph | 4-Pyr | SOBu |
| 11-779 | 3-$CF_3$-Ph | 4-Pyr | $SOCF_3$ |
| 11-780 | 3-$CF_3$-Ph | 4-Pyr | SO-$(CH_2)_2$-F |
| 11-781 | 3-$CF_3$-Ph | 4-Pyr | SO-$(CH_2)_2$-OH |
| 11-782 | 3-$CF_3$-Ph | 4-Pyr | SO-$(CH_2)_2$-OMe |
| 11-783 | 3-$CF_3$-Ph | 4-Pyr | $SO_2Me$ |
| 11-784 | 3-$CF_3$-Ph | 4-Pyr | $SO_2Et$ |
| 11-785 | 3-$CF_3$-Ph | 4-Pyr | $SO_2Pr$ |
| 11-786 | 3-$CF_3$-Ph | 4-Pyr | $SO_2$-i-Pr |
| 11-787 | 3-$CF_3$-Ph | 4-Pyr | $SO_2Bu$ |
| 11-788 | 3-$CF_3$-Ph | 4-Pyr | $SO_2CF_3$ |
| 11-789 | 3-$CF_3$-Ph | 4-Pyr | $SO_2$-$(CH_2)_2$-F |
| 11-790 | 3-$CF_3$-Ph | 4-Pyr | $SO_2$-$(CH_2)_2$-OH |
| 11-791 | 3-$CF_3$-Ph | 4-Pyr | $SO_2$-$(CH_2)_2$-OMe |
| 11-792 | 3-$CF_3$-Ph | 4-Pyr | $SO_2NH_2$ |
| 11-793 | 3-$CF_3$-Ph | 4-Pyr | $SO_2NHMe$ |
| 11-794 | 3-$CF_3$-Ph | 4-Pyr | $SO_2NHEt$ |
| 11-795 | 3-$CF_3$-Ph | 4-Pyr | $SO_2NHPr$ |
| 11-796 | 3-$CF_3$-Ph | 4-Pyr | $SO_2NH$-i-Pr |
| 11-797 | 3-$CF_3$-Ph | 4-Pyr | $SO_2NHBu$ |
| 11-798 | 3-$CF_3$-Ph | 4-Pyr | $SO_2NHBn$ |
| 11-799 | 3-$CF_3$-Ph | 4-Pyr | $SO_2NMe_2$ |
| 11-800 | 3-$CF_3$-Ph | 4-Pyr | $SO_2NH$-c-Pr |
| 11-801 | 3-$CF_3$-Ph | 4-Pyr | $SO_2NH$-c-Bu |
| 11-802 | 3-$CF_3$-Ph | 4-Pyr | $SO_2NH$-c-Pen |
| 11-803 | 3-$CF_3$-Ph | 4-Pyr | $SO_2NH$-c-Hex |
| 11-804 | 3-$CF_3$-Ph | 4-Pyr | $SO_2NH$-c-Hep |

| | | | |
|---|---|---|---|
| 11-805 | 3-$CF_3$-Ph | 4-Pyr | $SO_2NHCH_2$-c-Pr |
| 11-806 | 3-$CF_3$-Ph | 4-Pyr | $SO_2NH$-Allyl |
| 11-807 | 3-$CF_3$-Ph | 4-Pyr | $SO_2NH$-Propargyl |
| 11-808 | 3-$CF_3$-Ph | 4-Pyr | $SO_2NHPh$ |
| 11-809 | 3-$CF_3$-Ph | 4-Pyr | $SO_2NH$-3-Pyr |
| 11-810 | 3-$CF_3$-Ph | 4-Pyr | $SO_2NHCH_2$-4-Pyr |
| 11-811 | 3-$CF_3$-Ph | 4-Pyr | $SO_2NH-(CH_2)_2-OH$ |
| 11-812 | 3-$CF_3$-Ph | 4-Pyr | $SO_2NHCH(CH_2OH)_2$ |
| 11-813 | 3-$CF_3$-Ph | 4-Pyr | $SO_2NH-(CH_2)_2-OAc$ |
| 11-814 | 3-$CF_3$-Ph | 4-Pyr | $SO_2NHCH_2CN$ |
| 11-815 | 3-$CF_3$-Ph | 4-Pyr | $SO_2NH-(CH_2)_2-F$ |
| 11-816 | 3-$CF_3$-Ph | 4-Pyr | $SO_2NH-(CH_2)_2-OMe$ |
| 11-817 | 3-$CF_3$-Ph | 4-Pyr | $SO_2NH-(CH_2)_2-SMe$ |
| 11-818 | 3-$CF_3$-Ph | 4-Pyr | $SO_2NH-(CH_2)_2-NH_2$ |
| 11-819 | 3-$CF_3$-Ph | 4-Pyr | $SO_2NH-(CH_2)_2-NHMe$ |
| 11-820 | 3-$CF_3$-Ph | 4-Pyr | $SO_2NH-(CH_2)_2-N(Me)_2$ |
| 11-821 | 3-$CF_3$-Ph | 4-Pyr | $SO_2NHCH_2COOH$ |
| 11-822 | 3-$CF_3$-Ph | 4-Pyr | $SO_2NHCH_2COOEt$ |
| 11-823 | 3-$CF_3$-Ph | 4-Pyr | $SO_2NHCH(Me)COOEt$ |
| 11-824 | 3-$CF_3$-Ph | 4-Pyr | $SO_2NHCH_2CONH_2$ |
| 11-825 | 3-$CF_3$-Ph | 4-Pyr | $SO_2NHOH$ |
| 11-826 | 3-$CF_3$-Ph | 4-Pyr | $SO_2NHOMe$ |
| 11-827 | 3-$CF_3$-Ph | 4-Pyr | $SO_2NHOEt$ |
| 11-828 | 3-$CF_3$-Ph | 4-Pyr | $SO_2NHOPr$ |
| 11-829 | 3-$CF_3$-Ph | 4-Pyr | $SO_2NHOAllyl$ |
| 11-830 | 3-$CF_3$-Ph | 4-Pyr | $SO_2NHOBn$ |
| 11-831 | 3-$CF_3$-Ph | 4-Pyr | $SO_2NHNH_2$ |
| 11-832 | 3-$CF_3$-Ph | 4-Pyr | $SO_2NHNHMe$ |
| 11-833 | 3-$CF_3$-Ph | 4-Pyr | $SO_2NHN(Me)_2$ |
| 11-834 | 4-F-Ph | 4-Pym | $CONH_2$ |
| 11-835 | 4-F-Ph | 4-Pym | CONHMe |
| 11-836 | 4-F-Ph | 4-Pym | CONHEt |
| 11-837 | 4-F-Ph | 4-Pym | CONHPr |
| 11-838 | 4-F-Ph | 4-Pym | CONH-i-Pr |
| 11-839 | 4-F-Ph | 4-Pym | CONHBu |
| 11-840 | 4-F-Ph | 4-Pym | CONHBn |

| | | | |
|---|---|---|---|
| 11-841 | 4-F-Ph | 4-Pym | $CONMe_2$ |
| 11-842 | 4-F-Ph | 4-Pym | CONH-c-Pr |
| 11-843 | 4-F-Ph | 4-Pym | CONH-c-Bu |
| 11-844 | 4-F-Ph | 4-Pym | CONH-c-Pen |
| 11-845 | 4-F-Ph | 4-Pym | CONH-c-Hex |
| 11-846 | 4-F-Ph | 4-Pym | CONH-c-Hep |
| 11-847 | 4-F-Ph | 4-Pym | $CONHCH_2$-c-Pr |
| 11-848 | 4-F-Ph | 4-Pym | CONH-Allyl |
| 11-849 | 4-F-Ph | 4-Pym | CONH-Propargyl |
| 11-850 | 4-F-Ph | 4-Pym | CONHPh |
| 11-851 | 4-F-Ph | 4-Pym | CONH-3-Pyr |
| 11-852 | 4-F-Ph | 4-Pym | $CONHCH_2$-4-Pyr |
| 11-853 | 4-F-Ph | 4-Pym | CONH-$(CH_2)_2$-OH |
| 11-854 | 4-F-Ph | 4-Pym | $CONHCH(CH_2OH)_2$ |
| 11-855 | 4-F-Ph | 4-Pym | CONH-$(CH_2)_2$-OAc |
| 11-856 | 4-F-Ph | 4-Pym | $CONHCH_2CN$ |
| 11-857 | 4-F-Ph | 4-Pym | CONH-$(CH_2)_2$-F |
| 11-858 | 4-F-Ph | 4-Pym | CONH-$(CH_2)_2$-OMe |
| 11-859 | 4-F-Ph | 4-Pym | CONH-$(CH_2)_2$-SMe |
| 11-860 | 4-F-Ph | 4-Pym | CONH-$(CH_2)_2$-$NH_2$ |
| 11-861 | 4-F-Ph | 4-Pym | CONH-$(CH_2)_2$-NHMe |
| 11-862 | 4-F-Ph | 4-Pym | CONH-$(CH_2)_2$-$N(Me)_2$ |
| 11-863 | 4-F-Ph | 4-Pym | $CONHCH_2COOH$ |
| 11-864 | 4-F-Ph | 4-Pym | $CONHCH_2COOEt$ |
| 11-865 | 4-F-Ph | 4-Pym | CONHCH(Me)COOEt |
| 11-866 | 4-F-Ph | 4-Pym | $CONHCH_2CONH_2$ |
| 11-867 | 4-F-Ph | 4-Pym | $SO_2NH_2$ |
| 11-868 | 4-F-Ph | 4-Pym | $SO_2NHMe$ |
| 11-869 | 4-F-Ph | 4-Pym | $SO_2NHEt$ |
| 11-870 | 4-F-Ph | 4-Pym | $SO_2NHPr$ |
| 11-871 | 4-F-Ph | 4-Pym | $SO_2NH$-i-Pr |
| 11-872 | 4-F-Ph | 4-Pym | $SO_2NHBu$ |
| 11-873 | 4-F-Ph | 4-Pym | $SO_2NHBn$ |
| 11-874 | 4-F-Ph | 4-Pym | $SO_2NMe_2$ |
| 11-875 | 4-F-Ph | 4-Pym | $SO_2NH$-c-Pr |
| 11-876 | 4-F-Ph | 4-Pym | $SO_2NH$-c-Bu |

| | | | |
|---|---|---|---|
| 11-877 | 4-F-Ph | 4-Pym | $SO_2NH$-c-Pen |
| 11-878 | 4-F-Ph | 4-Pym | $SO_2NH$-c-Hex |
| 11-879 | 4-F-Ph | 4-Pym | $SO_2NH$-c-Hep |
| 11-880 | 4-F-Ph | 4-Pym | $SO_2NHCH_2$-c-Pr |
| 11-881 | 4-F-Ph | 4-Pym | $SO_2NH$-Allyl |
| 11-882 | 4-F-Ph | 4-Pym | $SO_2NH$-Propargyl |
| 11-883 | 4-F-Ph | 4-Pym | $SO_2NHPh$ |
| 11-884 | 4-F-Ph | 4-Pym | $SO_2NH$-3-Pyr |
| 11-885 | 4-F-Ph | 4-Pym | $SO_2NHCH_2$-4-Pyr |
| 11-886 | 4-F-Ph | 4-Pym | $SO_2NH-(CH_2)_2-OH$ |
| 11-887 | 4-F-Ph | 4-Pym | $SO_2NHCH(CH_2OH)_2$ |
| 11-888 | 4-F-Ph | 4-Pym | $SO_2NH-(CH_2)_2-OAc$ |
| 11-889 | 4-F-Ph | 4-Pym | $SO_2NHCH_2CN$ |
| 11-890 | 4-F-Ph | 4-Pym | $SO_2NH-(CH_2)_2-F$ |
| 11-891 | 4-F-Ph | 4-Pym | $SO_2NH-(CH_2)_2-OMe$ |
| 11-892 | 4-F-Ph | 4-Pym | $SO_2NH-(CH_2)_2-SMe$ |
| 11-893 | 4-F-Ph | 4-Pym | $SO_2NH-(CH_2)_2-NH_2$ |
| 11-894 | 4-F-Ph | 4-Pym | $SO_2NH-(CH_2)_2-NHMe$ |
| 11-895 | 4-F-Ph | 4-Pym | $SO_2NH-(CH_2)_2-N(Me)_2$ |
| 11-896 | 4-F-Ph | 4-Pym | $SO_2NHCH_2COOH$ |
| 11-897 | 4-F-Ph | 4-Pym | $SO_2NHCH_2COOEt$ |
| 11-898 | 4-F-Ph | 4-Pym | $SO_2NHCH(Me)COOEt$ |
| 11-899 | 4-F-Ph | 4-Pym | $SO_2NHCH_2CONH_2$ |
| 11-900 | 4-F-Ph | 4-Pym | $SO_2NHOH$ |
| 11-901 | 4-F-Ph | 4-Pym | $SO_2NHOMe$ |
| 11-902 | 4-F-Ph | 4-Pym | $SO_2NHOEt$ |
| 11-903 | 4-F-Ph | 4-Pym | $SO_2NHOPr$ |
| 11-904 | 4-F-Ph | 4-Pym | $SO_2NHOAllyl$ |
| 11-905 | 4-F-Ph | 4-Pym | $SO_2NHOBn$ |
| 11-906 | 4-F-Ph | 4-Pym | $SO_2NHNH_2$ |
| 11-907 | 4-F-Ph | 4-Pym | $SO_2NHNHMe$ |
| 11-908 | 4-F-Ph | 4-Pym | $SO_2NHN(Me)_2$ |
| 11-909 | 4-F-Ph | 2-MeO-4-Pym | $CONH_2$ |
| 11-910 | 4-F-Ph | 2-MeO-4-Pym | CONHMe |
| 11-911 | 4-F-Ph | 2-MeO-4-Pym | CONHEt |
| 11-912 | 4-F-Ph | 2-MeO-4-Pym | CONHPr |

| | | | |
|---|---|---|---|
| 11-913 | 4-F-Ph | 2-MeO-4-Pym | CONH-i-Pr |
| 11-914 | 4-F-Ph | 2-MeO-4-Pym | CONHBu |
| 11-915 | 4-F-Ph | 2-MeO-4-Pym | CONHBn |
| 11-916 | 4-F-Ph | 2-MeO-4-Pym | $CONMe_2$ |
| 11-917 | 4-F-Ph | 2-MeO-4-Pym | CONH-c-Pr |
| 11-918 | 4-F-Ph | 2-MeO-4-Pym | CONH-c-Bu |
| 11-919 | 4-F-Ph | 2-MeO-4-Pym | CONH-c-Pen |
| 11-920 | 4-F-Ph | 2-MeO-4-Pym | CONH-c-Hex |
| 11-921 | 4-F-Ph | 2-MeO-4-Pym | CONH-c-Hep |
| 11-922 | 4-F-Ph | 2-MeO-4-Pym | $CONHCH_2$-c-Pr |
| 11-923 | 4-F-Ph | 2-MeO-4-Pym | CONH-Allyl |
| 11-924 | 4-F-Ph | 2-MeO-4-Pym | CONH-Propargyl |
| 11-925 | 4-F-Ph | 2-MeO-4-Pym | CONHPh |
| 11-926 | 4-F-Ph | 2-MeO-4-Pym | CONH-3-Pyr |
| 11-927 | 4-F-Ph | 2-MeO-4-Pym | $CONHCH_2$-4-Pyr |
| 11-928 | 4-F-Ph | 2-MeO-4-Pym | CONH-$(CH_2)_2$-OH |
| 11-929 | 4-F-Ph | 2-MeO-4-Pym | $CONHCH(CH_2OH)_2$ |
| 11-930 | 4-F-Ph | 2-MeO-4-Pym | CONH-$(CH_2)_2$-OAc |
| 11-931 | 4-F-Ph | 2-MeO-4-Pym | $CONHCH_2CN$ |
| 11-932 | 4-F-Ph | 2-MeO-4-Pym | CONH-$(CH_2)_2$-F |
| 11-933 | 4-F-Ph | 2-MeO-4-Pym | CONH-$(CH_2)_2$-OMe |
| 11-934 | 4-F-Ph | 2-MeO-4-Pym | CONH-$(CH_2)_2$-SMe |
| 11-935 | 4-F-Ph | 2-MeO-4-Pym | CONH-$(CH_2)_2$-$NH_2$ |
| 11-936 | 4-F-Ph | 2-MeO-4-Pym | CONH-$(CH_2)_2$-NHMe |
| 11-937 | 4-F-Ph | 2-MeO-4-Pym | CONH-$(CH_2)_2$-$N(Me)_2$ |
| 11-938 | 4-F-Ph | 2-MeO-4-Pym | $CONHCH_2COOH$ |
| 11-939 | 4-F-Ph | 2-MeO-4-Pym | $CONHCH_2COOEt$ |
| 11-940 | 4-F-Ph | 2-MeO-4-Pym | CONHCH(Me)COOEt |
| 11-941 | 4-F-Ph | 2-MeO-4-Pym | $CONHCH_2CONH_2$ |
| 11-942 | 4-F-Ph | 2-MeO-4-Pym | $SO_2NH_2$ |
| 11-943 | 4-F-Ph | 2-MeO-4-Pym | $SO_2NHMe$ |
| 11-944 | 4-F-Ph | 2-MeO-4-Pym | $SO_2NHEt$ |
| 11-945 | 4-F-Ph | 2-MeO-4-Pym | $SO_2NHPr$ |
| 11-946 | 4-F-Ph | 2-MeO-4-Pym | $SO_2NH$-i-Pr |
| 11-947 | 4-F-Ph | 2-MeO-4-Pym | $SO_2NHBu$ |
| 11-948 | 4-F-Ph | 2-MeO-4-Pym | $SO_2NHBn$ |

| | | | |
|---|---|---|---|
| 11-949 | 4-F-Ph | 2-MeO-4-Pym | $SO_2NMe_2$ |
| 11-950 | 4-F-Ph | 2-MeO-4-Pym | $SO_2NH$-c-Pr |
| 11-951 | 4-F-Ph | 2-MeO-4-Pym | $SO_2NH$-c-Bu |
| 11-952 | 4-F-Ph | 2-MeO-4-Pym | $SO_2NH$-c-Pen |
| 11-953 | 4-F-Ph | 2-MeO-4-Pym | $SO_2NH$-c-Hex |
| 11-954 | 4-F-Ph | 2-MeO-4-Pym | $SO_2NH$-c-Hep |
| 11-955 | 4-F-Ph | 2-MeO-4-Pym | $SO_2NHCH_2$-c-Pr |
| 11-956 | 4-F-Ph | 2-MeO-4-Pym | $SO_2NH$-Allyl |
| 11-957 | 4-F-Ph | 2-MeO-4-Pym | $SO_2NH$-Propargyl |
| 11-958 | 4-F-Ph | 2-MeO-4-Pym | $SO_2NHPh$ |
| 11-959 | 4-F-Ph | 2-MeO-4-Pym | $SO_2NH$-3-Pyr |
| 11-960 | 4-F-Ph | 2-MeO-4-Pym | $SO_2NHCH_2$-4-Pyr |
| 11-961 | 4-F-Ph | 2-MeO-4-Pym | $SO_2NH-(CH_2)_2-OH$ |
| 11-962 | 4-F-Ph | 2-MeO-4-Pym | $SO_2NHCH(CH_2OH)_2$ |
| 11-963 | 4-F-Ph | 2-MeO-4-Pym | $SO_2NH-(CH_2)_2-OAc$ |
| 11-964 | 4-F-Ph | 2-MeO-4-Pym | $SO_2NHCH_2CN$ |
| 11-965 | 4-F-Ph | 2-MeO-4-Pym | $SO_2NH-(CH_2)_2-F$ |
| 11-966 | 4-F-Ph | 2-MeO-4-Pym | $SO_2NH-(CH_2)_2-OMe$ |
| 11-967 | 4-F-Ph | 2-MeO-4-Pym | $SO_2NH-(CH_2)_2-SMe$ |
| 11-968 | 4-F-Ph | 2-MeO-4-Pym | $SO_2NH-(CH_2)_2-NH_2$ |
| 11-969 | 4-F-Ph | 2-MeO-4-Pym | $SO_2NH-(CH_2)_2-NHMe$ |
| 11-970 | 4-F-Ph | 2-MeO-4-Pym | $SO_2NH-(CH_2)_2-N(Me)_2$ |
| 11-971 | 4-F-Ph | 2-MeO-4-Pym | $SO_2NHCH_2COOH$ |
| 11-972 | 4-F-Ph | 2-MeO-4-Pym | $SO_2NHCH_2COOEt$ |
| 11-973 | 4-F-Ph | 2-MeO-4-Pym | $SO_2NHCH(Me)COOEt$ |
| 11-974 | 4-F-Ph | 2-MeO-4-Pym | $SO_2NHCH_2CONH_2$ |
| 11-975 | 4-F-Ph | 2-MeO-4-Pym | $SO_2NHOH$ |
| 11-976 | 4-F-Ph | 2-MeO-4-Pym | $SO_2NHOMe$ |
| 11-977 | 4-F-Ph | 2-MeO-4-Pym | $SO_2NHOEt$ |
| 11-978 | 4-F-Ph | 2-MeO-4-Pym | $SO_2NHOPr$ |
| 11-979 | 4-F-Ph | 2-MeO-4-Pym | $SO_2NHOAllyl$ |
| 11-980 | 4-F-Ph | 2-MeO-4-Pym | $SO_2NHOBn$ |
| 11-981 | 4-F-Ph | 2-MeO-4-Pym | $SO_2NHNH_2$ |
| 11-982 | 4-F-Ph | 2-MeO-4-Pym | $SO_2NHNHMe$ |
| 11-983 | 4-F-Ph | 2-MeO-4-Pym | $SO_2NHN(Me)_2$ |
| 11-984 | 4-F-Ph | $2-NH_2$-4-Pym | $CONH_2$ |

| | | | |
|---|---|---|---|
| 11-985 | 4-F-Ph | $2\text{-}NH_2\text{-}4\text{-}Pym$ | CONHMe |
| 11-986 | 4-F-Ph | $2\text{-}NH_2\text{-}4\text{-}Pym$ | CONHEt |
| 11-987 | 4-F-Ph | $2\text{-}NH_2\text{-}4\text{-}Pym$ | CONHPr |
| 11-988 | 4-F-Ph | $2\text{-}NH_2\text{-}4\text{-}Pym$ | CONH-i-Pr |
| 11-989 | 4-F-Ph | $2\text{-}NH_2\text{-}4\text{-}Pym$ | CONHBu |
| 11-990 | 4-F-Ph | $2\text{-}NH_2\text{-}4\text{-}Pym$ | CONHBn |
| 11-991 | 4-F-Ph | $2\text{-}NH_2\text{-}4\text{-}Pym$ | $CONMe_2$ |
| 11-992 | 4-F-Ph | $2\text{-}NH_2\text{-}4\text{-}Pym$ | CONH-c-Pr |
| 11-993 | 4-F-Ph | $2\text{-}NH_2\text{-}4\text{-}Pym$ | CONH-c-Bu |
| 11-994 | 4-F-Ph | $2\text{-}NH_2\text{-}4\text{-}Pym$ | CONH-c-Pen |
| 11-995 | 4-F-Ph | $2\text{-}NH_2\text{-}4\text{-}Pym$ | CONH-c-Hex |
| 11-996 | 4-F-Ph | $2\text{-}NH_2\text{-}4\text{-}Pym$ | CONH-c-Hep |
| 11-997 | 4-F-Ph | $2\text{-}NH_2\text{-}4\text{-}Pym$ | $CONHCH_2\text{-}c\text{-}Pr$ |
| 11-998 | 4-F-Ph | $2\text{-}NH_2\text{-}4\text{-}Pym$ | CONH-Allyl |
| 11-999 | 4-F-Ph | $2\text{-}NH_2\text{-}4\text{-}Pym$ | CONH-Propargyl |
| 11-1000 | 4-F-Ph | $2\text{-}NH_2\text{-}4\text{-}Pym$ | CONHPh |
| 11-1001 | 4-F-Ph | $2\text{-}NH_2\text{-}4\text{-}Pym$ | CONH-3-Pyr |
| 11-1002 | 4-F-Ph | $2\text{-}NH_2\text{-}4\text{-}Pym$ | $CONHCH_2\text{-}4\text{-}Pyr$ |
| 11-1003 | 4-F-Ph | $2\text{-}NH_2\text{-}4\text{-}Pym$ | $CONH\text{-}(CH_2)_2\text{-}OH$ |
| 11-1004 | 4-F-Ph | $2\text{-}NH_2\text{-}4\text{-}Pym$ | $CONHCH(CH_2OH)_2$ |
| 11-1005 | 4-F-Ph | $2\text{-}NH_2\text{-}4\text{-}Pym$ | $CONH\text{-}(CH_2)_2\text{-}OAc$ |
| 11-1006 | 4-F-Ph | $2\text{-}NH_2\text{-}4\text{-}Pym$ | $CONHCH_2CN$ |
| 11-1007 | 4-F-Ph | $2\text{-}NH_2\text{-}4\text{-}Pym$ | $CONH\text{-}(CH_2)_2\text{-}F$ |
| 11-1008 | 4-F-Ph | $2\text{-}NH_2\text{-}4\text{-}Pym$ | $CONH\text{-}(CH_2)_2\text{-}OMe$ |
| 11-1009 | 4-F-Ph | $2\text{-}NH_2\text{-}4\text{-}Pym$ | $CONH\text{-}(CH_2)_2\text{-}SMe$ |
| 11-1010 | 4-F-Ph | $2\text{-}NH_2\text{-}4\text{-}Pym$ | $CONH\text{-}(CH_2)_2\text{-}NH_2$ |
| 11-1011 | 4-F-Ph | $2\text{-}NH_2\text{-}4\text{-}Pym$ | $CONH\text{-}(CH_2)_2\text{-}NHMe$ |
| 11-1012 | 4-F-Ph | $2\text{-}NH_2\text{-}4\text{-}Pym$ | $CONH\text{-}(CH_2)_2\text{-}N(Me)_2$ |
| 11-1013 | 4-F-Ph | $2\text{-}NH_2\text{-}4\text{-}Pym$ | $CONHCH_2COOH$ |
| 11-1014 | 4-F-Ph | $2\text{-}NH_2\text{-}4\text{-}Pym$ | $CONHCH_2COOEt$ |
| 11-1015 | 4-F-Ph | $2\text{-}NH_2\text{-}4\text{-}Pym$ | CONHCH(Me)COOEt |
| 11-1016 | 4-F-Ph | $2\text{-}NH_2\text{-}4\text{-}Pym$ | $CONHCH_2CONH_2$ |
| 11-1017 | 4-F-Ph | $2\text{-}NH_2\text{-}4\text{-}Pym$ | $SO_2NH_2$ |
| 11-1018 | 4-F-Ph | $2\text{-}NH_2\text{-}4\text{-}Pym$ | $SO_2NHMe$ |
| 11-1019 | 4-F-Ph | $2\text{-}NH_2\text{-}4\text{-}Pym$ | $SO_2NHEt$ |
| 11-1020 | 4-F-Ph | $2\text{-}NH_2\text{-}4\text{-}Pym$ | $SO_2NHPr$ |

| | | | |
|---|---|---|---|
| 11-1021 | 4-F-Ph | 2-$NH_2$-4-Pym | $SO_2$NH-i-Pr |
| 11-1022 | 4-F-Ph | 2-$NH_2$-4-Pym | $SO_2$NHBu |
| 11-1023 | 4-F-Ph | 2-$NH_2$-4-Pym | $SO_2$NHBn |
| 11-1024 | 4-F-Ph | 2-$NH_2$-4-Pym | $SO_2NMe_2$ |
| 11-1025 | 4-F-Ph | 2-$NH_2$-4-Pym | $SO_2$NH-c-Pr |
| 11-1026 | 4-F-Ph | 2-$NH_2$-4-Pym | $SO_2$NH-c-Bu |
| 11-1027 | 4-F-Ph | 2-$NH_2$-4-Pym | $SO_2$NH-c-Pen |
| 11-1028 | 4-F-Ph | 2-$NH_2$-4-Pym | $SO_2$NH-c-Hex |
| 11-1029 | 4-F-Ph | 2-$NH_2$-4-Pym | $SO_2$NH-c-Hep |
| 11-1030 | 4-F-Ph | 2-$NH_2$-4-Pym | $SO_2NHCH_2$-c-Pr |
| 11-1031 | 4-F-Ph | 2-$NH_2$-4-Pym | $SO_2$NH-Allyl |
| 11-1032 | 4-F-Ph | 2-$NH_2$-4-Pym | $SO_2$NH-Propargyl |
| 11-1033 | 4-F-Ph | 2-$NH_2$-4-Pym | $SO_2$NHPh |
| 11-1034 | 4-F-Ph | 2-$NH_2$-4-Pym | $SO_2$NH-3-Pyr |
| 11-1035 | 4-F-Ph | 2-$NH_2$-4-Pym | $SO_2NHCH_2$-4-Pyr |
| 11-1036 | 4-F-Ph | 2-$NH_2$-4-Pym | $SO_2NH-(CH_2)_2$-OH |
| 11-1037 | 4-F-Ph | 2-$NH_2$-4-Pym | $SO_2NHCH(CH_2OH)_2$ |
| 11-1038 | 4-F-Ph | 2-$NH_2$-4-Pym | $SO_2NH-(CH_2)_2$-OAc |
| 11-1039 | 4-F-Ph | 2-$NH_2$-4-Pym | $SO_2NHCH_2CN$ |
| 11-1040 | 4-F-Ph | 2-$NH_2$-4-Pym | $SO_2NH-(CH_2)_2$-F |
| 11-1041 | 4-F-Ph | 2-$NH_2$-4-Pym | $SO_2NH-(CH_2)_2$-OMe |
| 11-1042 | 4-F-Ph | 2-$NH_2$-4-Pym | $SO_2NH-(CH_2)_2$-SMe |
| 11-1043 | 4-F-Ph | 2-$NH_2$-4-Pym | $SO_2NH-(CH_2)_2-NH_2$ |
| 11-1044 | 4-F-Ph | 2-$NH_2$-4-Pym | $SO_2NH-(CH_2)_2$-NHMe |
| 11-1045 | 4-F-Ph | 2-$NH_2$-4-Pym | $SO_2NH-(CH_2)_2-N(Me)_2$ |
| 11-1046 | 4-F-Ph | 2-$NH_2$-4-Pym | $SO_2NHCH_2COOH$ |
| 11-1047 | 4-F-Ph | 2-$NH_2$-4-Pym | $SO_2NHCH_2COOEt$ |
| 11-1048 | 4-F-Ph | 2-$NH_2$-4-Pym | $SO_2$NHCH(Me)COOEt |
| 11-1049 | 4-F-Ph | 2-$NH_2$-4-Pym | $SO_2NHCH_2CONH_2$ |
| 11-1050 | 4-F-Ph | 2-$NH_2$-4-Pym | $SO_2$NHOH |
| 11-1051 | 4-F-Ph | 2-$NH_2$-4-Pym | $SO_2$NHOMe |
| 11-1052 | 4-F-Ph | 2-$NH_2$-4-Pym | $SO_2$NHOEt |
| 11-1053 | 4-F-Ph | 2-$NH_2$-4-Pym | $SO_2$NHOPr |
| 11-1054 | 4-F-Ph | 2-$NH_2$-4-Pym | $SO_2$NHOAllyl |
| 11-1055 | 4-F-Ph | 2-$NH_2$-4-Pym | $SO_2$NHOBn |
| 11-1056 | 4-F-Ph | 2-$NH_2$-4-Pym | $SO_2NHNH_2$ |

| | | | |
|---|---|---|---|
| 11-1057 | 4-F-Ph | 2-$NH_2$-4-Pym | $SO_2NHNHMe$ |
| 11-1058 | 4-F-Ph | 2-$NH_2$-4-Pym | $SO_2NHN(Me)_2$ |
| 11-1059 | 4-F-Ph | 2-MeNH-4-Pym | $CONH_2$ |
| 11-1060 | 4-F-Ph | 2-MeNH-4-Pym | CONHMe |
| 11-1061 | 4-F-Ph | 2-MeNH-4-Pym | CONHEt |
| 11-1062 | 4-F-Ph | 2-MeNH-4-Pym | CONHPr |
| 11-1063 | 4-F-Ph | 2-MeNH-4-Pym | CONH-i-Pr |
| 11-1064 | 4-F-Ph | 2-MeNH-4-Pym | CONHBu |
| 11-1065 | 4-F-Ph | 2-MeNH-4-Pym | CONHBn |
| 11-1066 | 4-F-Ph | 2-MeNH-4-Pym | $CONMe_2$ |
| 11-1067 | 4-F-Ph | 2-MeNH-4-Pym | CONH-c-Pr |
| 11-1068 | 4-F-Ph | 2-MeNH-4-Pym | CONH-c-Bu |
| 11-1069 | 4-F-Ph | 2-MeNH-4-Pym | CONH-c-Pen |
| 11-1070 | 4-F-Ph | 2-MeNH-4-Pym | CONH-c-Hex |
| 11-1071 | 4-F-Ph | 2-MeNH-4-Pym | CONH-c-Hep |
| 11-1072 | 4-F-Ph | 2-MeNH-4-Pym | $CONHCH_2$-c-Pr |
| 11-1073 | 4-F-Ph | 2-MeNH-4-Pym | CONH-Allyl |
| 11-1074 | 4-F-Ph | 2-MeNH-4-Pym | CONH-Propargyl |
| 11-1075 | 4-F-Ph | 2-MeNH-4-Pym | CONHPh |
| 11-1076 | 4-F-Ph | 2-MeNH-4-Pym | CONH-3-Pyr |
| 11-1077 | 4-F-Ph | 2-MeNH-4-Pym | $CONHCH_2$-4-Pyr |
| 11-1078 | 4-F-Ph | 2-MeNH-4-Pym | CONH-$(CH_2)_2$-OH |
| 11-1079 | 4-F-Ph | 2-MeNH-4-Pym | $CONHCH(CH_2OH)_2$ |
| 11-1080 | 4-F-Ph | 2-MeNH-4-Pym | CONH-$(CH_2)_2$-OAc |
| 11-1081 | 4-F-Ph | 2-MeNH-4-Pym | $CONHCH_2CN$ |
| 11-1082 | 4-F-Ph | 2-MeNH-4-Pym | CONH-$(CH_2)_2$-F |
| 11-1083 | 4-F-Ph | 2-MeNH-4-Pym | CONH-$(CH_2)_2$-OMe |
| 11-1084 | 4-F-Ph | 2-MeNH-4-Pym | CONH-$(CH_2)_2$-SMe |
| 11-1085 | 4-F-Ph | 2-MeNH-4-Pym | CONH-$(CH_2)_2$-$NH_2$ |
| 11-1086 | 4-F-Ph | 2-MeNH-4-Pym | CONH-$(CH_2)_2$-NHMe |
| 11-1087 | 4-F-Ph | 2-MeNH-4-Pym | CONH-$(CH_2)_2$-$N(Me)_2$ |
| 11-1088 | 4-F-Ph | 2-MeNH-4-Pym | $CONHCH_2COOH$ |
| 11-1089 | 4-F-Ph | 2-MeNH-4-Pym | $CONHCH_2COOEt$ |
| 11-1090 | 4-F-Ph | 2-MeNH-4-Pym | CONHCH(Me)COOEt |
| 11-1091 | 4-F-Ph | 2-MeNH-4-Pym | $CONHCH_2CONH_2$ |
| 11-1092 | 4-F-Ph | 2-MeNH-4-Pym | $SO_2NH_2$ |

| | | | |
|---|---|---|---|
| 11-1093 | 4-F-Ph | 2-MeNH-4-Pym | $SO_2NHMe$ |
| 11-1094 | 4-F-Ph | 2-MeNH-4-Pym | $SO_2NHEt$ |
| 11-1095 | 4-F-Ph | 2-MeNH-4-Pym | $SO_2NHPr$ |
| 11-1096 | 4-F-Ph | 2-MeNH-4-Pym | $SO_2NH$-i-Pr |
| 11-1097 | 4-F-Ph | 2-MeNH-4-Pym | $SO_2NHBu$ |
| 11-1098 | 4-F-Ph | 2-MeNH-4-Pym | $SO_2NHBn$ |
| 11-1099 | 4-F-Ph | 2-MeNH-4-Pym | $SO_2NMe_2$ |
| 11-1100 | 4-F-Ph | 2-MeNH-4-Pym | $SO_2NH$-c-Pr |
| 11-1101 | 4-F-Ph | 2-MeNH-4-Pym | $SO_2NH$-c-Bu |
| 11-1102 | 4-F-Ph | 2-MeNH-4-Pym | $SO_2NH$-c-Pen |
| 11-1103 | 4-F-Ph | 2-MeNH-4-Pym | $SO_2NH$-c-Hex |
| 11-1104 | 4-F-Ph | 2-MeNH-4-Pym | $SO_2NH$-c-Hep |
| 11-1105 | 4-F-Ph | 2-MeNH-4-Pym | $SO_2NHCH_2$-c-Pr |
| 11-1106 | 4-F-Ph | 2-MeNH-4-Pym | $SO_2NH$-Allyl |
| 11-1107 | 4-F-Ph | 2-MeNH-4-Pym | $SO_2NH$-Propargyl |
| 11-1108 | 4-F-Ph | 2-MeNH-4-Pym | $SO_2NHPh$ |
| 11-1109 | 4-F-Ph | 2-MeNH-4-Pym | $SO_2NH$-3-Pyr |
| 11-1110 | 4-F-Ph | 2-MeNH-4-Pym | $SO_2NHCH_2$-4-Pyr |
| 11-1111 | 4-F-Ph | 2-MeNH-4-Pym | $SO_2NH-(CH_2)_2-OH$ |
| 11-1112 | 4-F-Ph | 2-MeNH-4-Pym | $SO_2NHCH(CH_2OH)_2$ |
| 11-1113 | 4-F-Ph | 2-MeNH-4-Pym | $SO_2NH-(CH_2)_2-OAc$ |
| 11-1114 | 4-F-Ph | 2-MeNH-4-Pym | $SO_2NHCH_2CN$ |
| 11-1115 | 4-F-Ph | 2-MeNH-4-Pym | $SO_2NH-(CH_2)_2-F$ |
| 11-1116 | 4-F-Ph | 2-MeNH-4-Pym | $SO_2NH-(CH_2)_2-OMe$ |
| 11-1117 | 4-F-Ph | 2-MeNH-4-Pym | $SO_2NH-(CH_2)_2-SMe$ |
| 11-1118 | 4-F-Ph | 2-MeNH-4-Pym | $SO_2NH-(CH_2)_2-NH_2$ |
| 11-1119 | 4-F-Ph | 2-MeNH-4-Pym | $SO_2NH-(CH_2)_2-NHMe$ |
| 11-1120 | 4-F-Ph | 2-MeNH-4-Pym | $SO_2NH-(CH_2)_2-N(Me)_2$ |
| 11-1121 | 4-F-Ph | 2-MeNH-4-Pym | $SO_2NHCH_2COOH$ |
| 11-1122 | 4-F-Ph | 2-MeNH-4-Pym | $SO_2NHCH_2COOEt$ |
| 11-1123 | 4-F-Ph | 2-MeNH-4-Pym | $SO_2NHCH(Me)COOEt$ |
| 11-1124 | 4-F-Ph | 2-MeNH-4-Pym | $SO_2NHCH_2CONH_2$ |
| 11-1125 | 4-F-Ph | 2-MeNH-4-Pym | $SO_2NHOH$ |
| 11-1126 | 4-F-Ph | 2-MeNH-4-Pym | $SO_2NHOMe$ |
| 11-1127 | 4-F-Ph | 2-MeNH-4-Pym | $SO_2NHOEt$ |
| 11-1128 | 4-F-Ph | 2-MeNH-4-Pym | $SO_2NHOPr$ |

| | | | |
|---|---|---|---|
| 11-1129 | 4-F-Ph | 2-MeNH-4-Pym | $SO_2NHOAllyl$ |
| 11-1130 | 4-F-Ph | 2-MeNH-4-Pym | $SO_2NHOBn$ |
| 11-1131 | 4-F-Ph | 2-MeNH-4-Pym | $SO_2NHNH_2$ |
| 11-1132 | 4-F-Ph | 2-MeNH-4-Pym | $SO_2NHNHMe$ |
| 11-1133 | 4-F-Ph | 2-MeNH-4-Pym | $SO_2NHN(Me)_2$ |
| 11-1134 | 4-F-Ph | 2-BnNH-4-Pym | $CONH_2$ |
| 11-1135 | 4-F-Ph | 2-BnNH-4-Pym | CONHMe |
| 11-1136 | 4-F-Ph | 2-BnNH-4-Pym | CONHEt |
| 11-1137 | 4-F-Ph | 2-BnNH-4-Pym | CONHPr |
| 11-1138 | 4-F-Ph | 2-BnNH-4-Pym | CONH-i-Pr |
| 11-1139 | 4-F-Ph | 2-BnNH-4-Pym | CONHBu |
| 11-1140 | 4-F-Ph | 2-BnNH-4-Pym | CONHBn |
| 11-1141 | 4-F-Ph | 2-BnNH-4-Pym | $CONMe_2$ |
| 11-1142 | 4-F-Ph | 2-BnNH-4-Pym | CONH-c-Pr |
| 11-1143 | 4-F-Ph | 2-BnNH-4-Pym | CONH-c-Bu |
| 11-1144 | 4-F-Ph | 2-BnNH-4-Pym | CONH-c-Pen |
| 11-1145 | 4-F-Ph | 2-BnNH-4-Pym | CONH-c-Hex |
| 11-1146 | 4-F-Ph | 2-BnNH-4-Pym | CONH-c-Hep |
| 11-1147 | 4-F-Ph | 2-BnNH-4-Pym | $CONHCH_2$-c-Pr |
| 11-1148 | 4-F-Ph | 2-BnNH-4-Pym | CONH-Allyl |
| 11-1149 | 4-F-Ph | 2-BnNH-4-Pym | CONH-Propargyl |
| 11-1150 | 4-F-Ph | 2-BnNH-4-Pym | CONHPh |
| 11-1151 | 4-F-Ph | 2-BnNH-4-Pym | CONH-3-Pyr |
| 11-1152 | 4-F-Ph | 2-BnNH-4-Pym | $CONHCH_2$-4-Pyr |
| 11-1153 | 4-F-Ph | 2-BnNH-4-Pym | CONH-$(CH_2)_2$-OH |
| 11-1154 | 4-F-Ph | 2-BnNH-4-Pym | $CONHCH(CH_2OH)_2$ |
| 11-1155 | 4-F-Ph | 2-BnNH-4-Pym | CONH-$(CH_2)_2$-OAc |
| 11-1156 | 4-F-Ph | 2-BnNH-4-Pym | $CONHCH_2CN$ |
| 11-1157 | 4-F-Ph | 2-BnNH-4-Pym | CONH-$(CH_2)_2$-F |
| 11-1158 | 4-F-Ph | 2-BnNH-4-Pym | CONH-$(CH_2)_2$-OMe |
| 11-1159 | 4-F-Ph | 2-BnNH-4-Pym | CONH-$(CH_2)_2$-SMe |
| 11-1160 | 4-F-Ph | 2-BnNH-4-Pym | CONH-$(CH_2)_2$-$NH_2$ |
| 11-1161 | 4-F-Ph | 2-BnNH-4-Pym | CONH-$(CH_2)_2$-NHMe |
| 11-1162 | 4-F-Ph | 2-BnNH-4-Pym | CONH-$(CH_2)_2$-$N(Me)_2$ |
| 11-1163 | 4-F-Ph | 2-BnNH-4-Pym | $CONHCH_2COOH$ |
| 11-1164 | 4-F-Ph | 2-BnNH-4-Pym | $CONHCH_2COOEt$ |

| | | | |
|---|---|---|---|
| 11-1165 | 4-F-Ph | 2-BnNH-4-Pym | CONHCH(Me)COOEt |
| 11-1166 | 4-F-Ph | 2-BnNH-4-Pym | $CONHCH_2CONH_2$ |
| 11-1167 | 4-F-Ph | 2-BnNH-4-Pym | $SO_2NH_2$ |
| 11-1168 | 4-F-Ph | 2-BnNH-4-Pym | $SO_2NHMe$ |
| 11-1169 | 4-F-Ph | 2-BnNH-4-Pym | $SO_2NHEt$ |
| 11-1170 | 4-F-Ph | 2-BnNH-4-Pym | $SO_2NHPr$ |
| 11-1171 | 4-F-Ph | 2-BnNH-4-Pym | $SO_2NH$-i-Pr |
| 11-1172 | 4-F-Ph | 2-BnNH-4-Pym | $SO_2NHBu$ |
| 11-1173 | 4-F-Ph | 2-BnNH-4-Pym | $SO_2NHBn$ |
| 11-1174 | 4-F-Ph | 2-BnNH-4-Pym | $SO_2NMe_2$ |
| 11-1175 | 4-F-Ph | 2-BnNH-4-Pym | $SO_2NH$-c-Pr |
| 11-1176 | 4-F-Ph | 2-BnNH-4-Pym | $SO_2NH$-c-Bu |
| 11-1177 | 4-F-Ph | 2-BnNH-4-Pym | $SO_2NH$-c-Pen |
| 11-1178 | 4-F-Ph | 2-BnNH-4-Pym | $SO_2NH$-c-Hex |
| 11-1179 | 4-F-Ph | 2-BnNH-4-Pym | $SO_2NH$-c-Hep |
| 11-1180 | 4-F-Ph | 2-BnNH-4-Pym | $SO_2NHCH_2$-c-Pr |
| 11-1181 | 4-F-Ph | 2-BnNH-4-Pym | $SO_2NH$-Allyl |
| 11-1182 | 4-F-Ph | 2-BnNH-4-Pym | $SO_2NH$-Propargyl |
| 11-1183 | 4-F-Ph | 2-BnNH-4-Pym | $SO_2NHPh$ |
| 11-1184 | 4-F-Ph | 2-BnNH-4-Pym | $SO_2NH$-3-Pyr |
| 11-1185 | 4-F-Ph | 2-BnNH-4-Pym | $SO_2NHCH_2$-4-Pyr |
| 11-1186 | 4-F-Ph | 2-BnNH-4-Pym | $SO_2NH-(CH_2)_2-OH$ |
| 11-1187 | 4-F-Ph | 2-BnNH-4-Pym | $SO_2NHCH(CH_2OH)_2$ |
| 11-1188 | 4-F-Ph | 2-BnNH-4-Pym | $SO_2NH-(CH_2)_2-OAc$ |
| 11-1189 | 4-F-Ph | 2-BnNH-4-Pym | $SO_2NHCH_2CN$ |
| 11-1190 | 4-F-Ph | 2-BnNH-4-Pym | $SO_2NH-(CH_2)_2-F$ |
| 11-1191 | 4-F-Ph | 2-BnNH-4-Pym | $SO_2NH-(CH_2)_2-OMe$ |
| 11-1192 | 4-F-Ph | 2-BnNH-4-Pym | $SO_2NH-(CH_2)_2-SMe$ |
| 11-1193 | 4-F-Ph | 2-BnNH-4-Pym | $SO_2NH-(CH_2)_2-NH_2$ |
| 11-1194 | 4-F-Ph | 2-BnNH-4-Pym | $SO_2NH-(CH_2)_2-NHMe$ |
| 11-1195 | 4-F-Ph | 2-BnNH-4-Pym | $SO_2NH-(CH_2)_2-N(Me)_2$ |
| 11-1196 | 4-F-Ph | 2-BnNH-4-Pym | $SO_2NHCH_2COOH$ |
| 11-1197 | 4-F-Ph | 2-BnNH-4-Pym | $SO_2NHCH_2COOEt$ |
| 11-1198 | 4-F-Ph | 2-BnNH-4-Pym | $SO_2NHCH(Me)COOEt$ |
| 11-1199 | 4-F-Ph | 2-BnNH-4-Pym | $SO_2NHCH_2CONH_2$ |
| 11-1200 | 4-F-Ph | 2-BnNH-4-Pym | $SO_2NHOH$ |

| | | | |
|---|---|---|---|
| 11-1201 | 4-F-Ph | 2-BnNH-4-Pym | $SO_2NHOMe$ |
| 11-1202 | 4-F-Ph | 2-BnNH-4-Pym | $SO_2NHOEt$ |
| 11-1203 | 4-F-Ph | 2-BnNH-4-Pym | $SO_2NHOPr$ |
| 11-1204 | 4-F-Ph | 2-BnNH-4-Pym | $SO_2NHOAllyl$ |
| 11-1205 | 4-F-Ph | 2-BnNH-4-Pym | $SO_2NHOBn$ |
| 11-1206 | 4-F-Ph | 2-BnNH-4-Pym | $SO_2NHNH_2$ |
| 11-1207 | 4-F-Ph | 2-BnNH-4-Pym | $SO_2NHNHMe$ |
| 11-1208 | 4-F-Ph | 2-BnNH-4-Pym | $SO_2NHN(Me)_2$ |
| 11-1209 | 4-F-Ph | (α-Me-BnNH)-4-Pym | $CONH_2$ |
| 11-1210 | 4-F-Ph | (α-Me-BnNH)-4-Pym | CONHMe |
| 11-1211 | 4-F-Ph | (α-Me-BnNH)-4-Pym | CONHEt |
| 11-1212 | 4-F-Ph | (α-Me-BnNH)-4-Pym | CONHPr |
| 11-1213 | 4-F-Ph | (α-Me-BnNH)-4-Pym | CONH-i-Pr |
| 11-1214 | 4-F-Ph | (α-Me-BnNH)-4-Pym | CONHBu |
| 11-1215 | 4-F-Ph | (α-Me-BnNH)-4-Pym | CONHBn |
| 11-1216 | 4-F-Ph | (α-Me-BnNH)-4-Pym | $CONMe_2$ |
| 11-1217 | 4-F-Ph | (α-Me-BnNH)-4-Pym | CONH-c-Pr |
| 11-1218 | 4-F-Ph | (α-Me-BnNH)-4-Pym | CONH-c-Bu |
| 11-1219 | 4-F-Ph | (α-Me-BnNH)-4-Pym | CONH-c-Pen |
| 11-1220 | 4-F-Ph | (α-Me-BnNH)-4-Pym | CONH-c-Hex |
| 11-1221 | 4-F-Ph | (α-Me-BnNH)-4-Pym | CONH-c-Hep |
| 11-1222 | 4-F-Ph | (α-Me-BnNH)-4-Pym | $CONHCH_2$-c-Pr |
| 11-1223 | 4-F-Ph | (α-Me-BnNH)-4-Pym | CONH-Allyl |
| 11-1224 | 4-F-Ph | (α-Me-BnNH)-4-Pym | CONH-Propargyl |
| 11-1225 | 4-F-Ph | (α-Me-BnNH)-4-Pym | CONHPh |
| 11-1226 | 4-F-Ph | (α-Me-BnNH)-4-Pym | CONH-3-Pyr |
| 11-1227 | 4-F-Ph | (α-Me-BnNH)-4-Pym | $CONHCH_2$-4-Pyr |
| 11-1228 | 4-F-Ph | (α-Me-BnNH)-4-Pym | CONH-$(CH_2)_2$-OH |
| 11-1229 | 4-F-Ph | (α-Me-BnNH)-4-Pym | $CONHCH(CH_2OH)_2$ |
| 11-1230 | 4-F-Ph | (α-Me-BnNH)-4-Pym | CONH-$(CH_2)_2$-OAc |
| 11-1231 | 4-F-Ph | (α-Me-BnNH)-4-Pym | $CONHCH_2CN$ |
| 11-1232 | 4-F-Ph | (α-Me-BnNH)-4-Pym | CONH-$(CH_2)_2$-F |
| 11-1233 | 4-F-Ph | (α-Me-BnNH)-4-Pym | CONH-$(CH_2)_2$-OMe |

| | | | |
|---|---|---|---|
| 11-1234 | 4-F-Ph | (α-Me-BnNH)-4-Pym | CONH-$(CH_2)_2$-SMe |
| 11-1235 | 4-F-Ph | (α-Me-BnNH)-4-Pym | CONH-$(CH_2)_2$-$NH_2$ |
| 11-1236 | 4-F-Ph | (α-Me-BnNH)-4-Pym | CONH-$(CH_2)_2$-NHMe |
| 11-1237 | 4-F-Ph | (α-Me-BnNH)-4-Pym | CONH-$(CH_2)_2$-$N(Me)_2$ |
| 11-1238 | 4-F-Ph | (α-Me-BnNH)-4-Pym | $CONHCH_2COOH$ |
| 11-1239 | 4-F-Ph | (α-Me-BnNH)-4-Pym | $CONHCH_2COOEt$ |
| 11-1240 | 4-F-Ph | (α-Me-BnNH)-4-Pym | CONHCH(Me)COOEt |
| 11-1241 | 4-F-Ph | (α-Me-BnNH)-4-Pym | $CONHCH_2CONH_2$ |
| 11-1242 | 4-F-Ph | (α-Me-BnNH)-4-Pym | $SO_2NH_2$ |
| 11-1243 | 4-F-Ph | (α-Me-BnNH)-4-Pym | $SO_2NHMe$ |
| 11-1244 | 4-F-Ph | (α-Me-BnNH)-4-Pym | $SO_2NHEt$ |
| 11-1245 | 4-F-Ph | (α-Me-BnNH)-4-Pym | $SO_2NHPr$ |
| 11-1246 | 4-F-Ph | (α-Me-BnNH)-4-Pym | $SO_2NH$-i-Pr |
| 11-1247 | 4-F-Ph | (α-Me-BnNH)-4-Pym | $SO_2NHBu$ |
| 11-1248 | 4-F-Ph | (α-Me-BnNH)-4-Pym | $SO_2NHBn$ |
| 11-1249 | 4-F-Ph | (α-Me-BnNH)-4-Pym | $SO_2NMe_2$ |
| 11-1250 | 4-F-Ph | (α-Me-BnNH)-4-Pym | $SO_2NH$-c-Pr |
| 11-1251 | 4-F-Ph | (α-Me-BnNH)-4-Pym | $SO_2NH$-c-Bu |
| 11-1252 | 4-F-Ph | (α-Me-BnNH)-4-Pym | $SO_2NH$-c-Pen |
| 11-1253 | 4-F-Ph | (α-Me-BnNH)-4-Pym | $SO_2NH$-c-Hex |
| 11-1254 | 4-F-Ph | (α-Me-BnNH)-4-Pym | $SO_2NH$-c-Hep |
| 11-1255 | 4-F-Ph | (α-Me-BnNH)-4-Pym | $SO_2NHCH_2$-c-Pr |
| 11-1256 | 4-F-Ph | (α-Me-BnNH)-4-Pym | $SO_2NH$-Allyl |
| 11-1257 | 4-F-Ph | (α-Me-BnNH)-4-Pym | $SO_2NH$-Propargyl |
| 11-1258 | 4-F-Ph | (α-Me-BnNH)-4-Pym | $SO_2NHPh$ |
| 11-1259 | 4-F-Ph | (α-Me-BnNH)-4-Pym | $SO_2NH$-3-Pyr |
| 11-1260 | 4-F-Ph | (α-Me-BnNH)-4-Pym | $SO_2NHCH_2$-4-Pyr |
| 11-1261 | 4-F-Ph | (α-Me-BnNH)-4-Pym | $SO_2NH$-$(CH_2)_2$-OH |
| 11-1262 | 4-F-Ph | (α-Me-BnNH)-4-Pym | $SO_2NHCH(CH_2OH)_2$ |
| 11-1263 | 4-F-Ph | (α-Me-BnNH)-4-Pym | $SO_2NH$-$(CH_2)_2$-OAc |
| 11-1264 | 4-F-Ph | (α-Me-BnNH)-4-Pym | $SO_2NHCH_2CN$ |
| 11-1265 | 4-F-Ph | (α-Me-BnNH)-4-Pym | $SO_2NH$-$(CH_2)_2$-F |

| | | | |
|---|---|---|---|
| 11-1266 | 4-F-Ph | (α-Me-BnNH)-4-Pym | $SO_2NH-(CH_2)_2-OMe$ |
| 11-1267 | 4-F-Ph | (α-Me-BnNH)-4-Pym | $SO_2NH-(CH_2)_2-SMe$ |
| 11-1268 | 4-F-Ph | (α-Me-BnNH)-4-Pym | $SO_2NH-(CH_2)_2-NH_2$ |
| 11-1269 | 4-F-Ph | (α-Me-BnNH)-4-Pym | $SO_2NH-(CH_2)_2-NHMe$ |
| 11-1270 | 4-F-Ph | (α-Me-BnNH)-4-Pym | $SO_2NH-(CH_2)_2-N(Me)_2$ |
| 11-1271 | 4-F-Ph | (α-Me-BnNH)-4-Pym | $SO_2NHCH_2COOH$ |
| 11-1272 | 4-F-Ph | (α-Me-BnNH)-4-Pym | $SO_2NHCH_2COOEt$ |
| 11-1273 | 4-F-Ph | (α-Me-BnNH)-4-Pym | $SO_2NHCH(Me)COOEt$ |
| 11-1274 | 4-F-Ph | (α-Me-BnNH)-4-Pym | $SO_2NHCH_2CONH_2$ |
| 11-1275 | 4-F-Ph | (α-Me-BnNH)-4-Pym | $SO_2NHOH$ |
| 11-1276 | 4-F-Ph | (α-Me-BnNH)-4-Pym | $SO_2NHOMe$ |
| 11-1277 | 4-F-Ph | (α-Me-BnNH)-4-Pym | $SO_2NHOEt$ |
| 11-1278 | 4-F-Ph | (α-Me-BnNH)-4-Pym | $SO_2NHOPr$ |
| 11-1279 | 4-F-Ph | (α-Me-BnNH)-4-Pym | $SO_2NHOAllyl$ |
| 11-1280 | 4-F-Ph | (α-Me-BnNH)-4-Pym | $SO_2NHOBn$ |
| 11-1281 | 4-F-Ph | (α-Me-BnNH)-4-Pym | $SO_2NHNH_2$ |
| 11-1282 | 4-F-Ph | (α-Me-BnNH)-4-Pym | $SO_2NHNHMe$ |
| 11-1283 | 4-F-Ph | (α-Me-BnNH)-4-Pym | $SO_2NHN(Me)_2$ |
| 11-1284 | 4-F-Ph | 4-Pyr | $SO_2NHMe$ |
| 11-1285 | 4-F-Ph | 4-Pyr | SOMe |
| 11-1286 | 4-F-Ph | 4-Pyr | $SO_2Me$ |
| 11-1287 | 3-Cl-4-F-Ph | 4-Pyr | $SO_2NHMe$ |
| 11-1288 | 3-Cl-4-F-Ph | 4-Pyr | SOMe |
| 11-1289 | 3-Cl-4-F-Ph | 4-Pyr | $SO_2Me$ |

Table 12

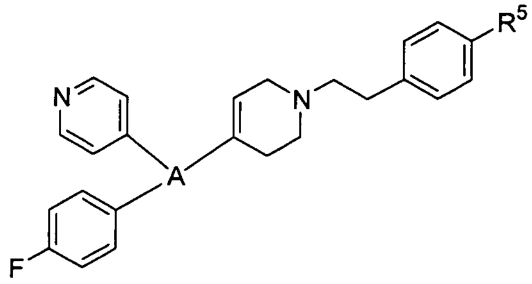

(I-12)

| Compound No. | A | $R^5$ |
|---|---|---|
| 12-1 | Ring 15 | $CONH_2$ |
| 12-2 | Ring 15 | $SO_2NHMe$ |
| 12-3 | Ring 15 | $SO_2Me$ |
| 12-4 | Ring 16 | $CONH_2$ |
| 12-5 | Ring 16 | $SO_2NHMe$ |
| 12-6 | Ring 16 | $SO_2Me$ |
| 12-7 | Ring 17 | $CONH_2$ |
| 12-8 | Ring 17 | $SO_2NHMe$ |
| 12-9 | Ring 17 | $SO_2Me$ |
| 12-10 | Ring 18 | $CONH_2$ |
| 12-11 | Ring 18 | $SO_2NHMe$ |
| 12-12 | Ring 18 | $SO_2Me$ |
| 12-13 | Ring 19 | $CONH_2$ |
| 12-14 | Ring 19 | $SO_2NHMe$ |
| 12-15 | Ring 19 | $SO_2Me$ |
| 12-16 | Ring 20 | $CONH_2$ |
| 12-17 | Ring 20 | $SO_2NHMe$ |
| 12-18 | Ring 20 | $SO_2Me$ |
| 12-19 | Ring 21 | $CONH_2$ |
| 12-20 | Ring 21 | $SO_2NHMe$ |
| 12-21 | Ring 21 | $SO_2Me$ |
| 12-22 | Ring 22 | $CONH_2$ |
| 12-23 | Ring 22 | $SO_2NHMe$ |
| 12-24 | Ring 22 | $SO_2Me$ |
| 12-25 | Ring 23 | $CONH_2$ |
| 12-26 | Ring 23 | $SO_2NHMe$ |
| 12-27 | Ring 23 | $SO_2Me$ |
| 12-28 | Ring 24 | $CONH_2$ |
| 12-29 | Ring 24 | $SO_2NHMe$ |
| 12-30 | Ring 24 | $SO_2Me$ |
| 12-31 | Ring 25 | $CONH_2$ |
| 12-32 | Ring 25 | $SO_2NHMe$ |
| 12-33 | Ring 25 | $SO_2Me$ |
| 12-34 | Ring 26 | $CONH_2$ |
| 12-35 | Ring 26 | $SO_2NHMe$ |

| | | |
|---|---|---|
| 12-36 | Ring 26 | $SO_2Me$ |
| 12-37 | Ring 27 | $CONH_2$ |
| 12-38 | Ring 27 | $SO_2NHMe$ |
| 12-39 | Ring 27 | $SO_2Me$ |
| 12-40 | Ring 28 | $CONH_2$ |
| 12-41 | Ring 28 | $SO_2NHMe$ |
| 12-42 | Ring 28 | $SO_2Me$ |
| 12-43 | Ring 29 | $CONH_2$ |
| 12-44 | Ring 29 | $SO_2NHMe$ |
| 12-45 | Ring 29 | $SO_2Me$ |
| 12-46 | Ring 30 | $CONH_2$ |
| 12-47 | Ring 30 | $SO_2NHMe$ |
| 12-48 | Ring 30 | $SO_2Me$ |
| 12-49 | Ring 31 | $CONH_2$ |
| 12-50 | Ring 31 | $SO_2NHMe$ |
| 12-51 | Ring 31 | $SO_2Me$ |
| 12-52 | Ring 32 | $CONH_2$ |
| 12-53 | Ring 32 | $SO_2NHMe$ |
| 12-54 | Ring 32 | $SO_2Me$ |
| 12-55 | Ring 33 | $CONH_2$ |
| 12-56 | Ring 33 | $SO_2NHMe$ |
| 12-57 | Ring 33 | $SO_2Me$ |
| 12-58 | Ring 34 | $CONH_2$ |
| 12-59 | Ring 34 | $SO_2NHMe$ |
| 12-60 | Ring 34 | $SO_2Me$ |
| 12-61 | Ring 35 | $CONH_2$ |
| 12-62 | Ring 35 | $SO_2NHMe$ |
| 12-63 | Ring 35 | $SO_2Me$ |
| 12-64 | Ring 36 | $CONH_2$ |
| 12-65 | Ring 36 | $SO_2NHMe$ |
| 12-66 | Ring 36 | $SO_2Me$ |
| 12-67 | Ring 37 | $CONH_2$ |
| 12-68 | Ring 37 | $SO_2NHMe$ |
| 12-69 | Ring 37 | $SO_2Me$ |
| 12-70 | Ring 38 | $CONH_2$ |
| 12-71 | Ring 38 | $SO_2NHMe$ |

| | | |
|---|---|---|
| 12-72 | Ring 38 | $SO_2Me$ |
| 12-73 | Ring 39 | $CONH_2$ |
| 12-74 | Ring 39 | $SO_2NHMe$ |
| 12-75 | Ring 39 | $SO_2Me$ |
| 12-76 | Ring 40 | $CONH_2$ |
| 12-77 | Ring 40 | $SO_2NHMe$ |
| 12-78 | Ring 40 | $SO_2Me$ |
| 12-79 | Ring 41 | $CONH_2$ |
| 12-80 | Ring 41 | $SO_2NHMe$ |
| 12-81 | Ring 41 | $SO_2Me$ |
| 12-82 | Ring 42 | $CONH_2$ |
| 12-83 | Ring 42 | $SO_2NHMe$ |
| 12-84 | Ring 42 | $SO_2Me$ |
| 12-85 | Ring 43 | $CONH_2$ |
| 12-86 | Ring 43 | $SO_2NHMe$ |
| 12-87 | Ring 43 | $SO_2Me$ |
| 12-88 | Ring 44 | $CONH_2$ |
| 12-89 | Ring 44 | $SO_2NHMe$ |
| 12-90 | Ring 44 | $SO_2Me$ |
| 12-91 | Ring 45 | $CONH_2$ |
| 12-92 | Ring 45 | $SO_2NHMe$ |
| 12-93 | Ring 45 | $SO_2Me$ |
| 12-94 | Ring 46 | $CONH_2$ |
| 12-95 | Ring 46 | $SO_2NHMe$ |
| 12-96 | Ring 46 | $SO_2Me$ |
| 12-97 | Ring 47 | $CONH_2$ |
| 12-98 | Ring 47 | $SO_2NHMe$ |
| 12-99 | Ring 47 | $SO_2Me$ |
| 12-100 | Ring 48 | $CONH_2$ |
| 12-101 | Ring 48 | $SO_2NHMe$ |
| 12-102 | Ring 48 | $SO_2Me$ |
| 12-103 | Ring 49 | $CONH_2$ |
| 12-104 | Ring 49 | $SO_2NHMe$ |
| 12-105 | Ring 49 | $SO_2Me$ |
| 12-106 | Ring 50 | $CONH_2$ |
| 12-107 | Ring 50 | $SO_2NHMe$ |

| | | |
|---|---|---|
| 12-108 | Ring 50 | $SO_2Me$ |
| 12-109 | Ring 51 | $CONH_2$ |
| 12-109 | Ring 51 | $SO_2NHMe$ |
| 12-110 | Ring 51 | $SO_2Me$ |

Table 13

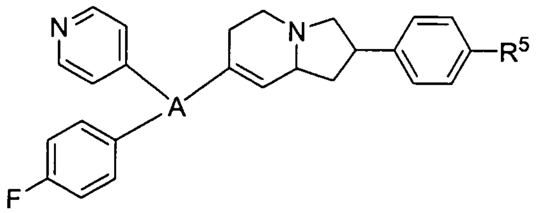

(I-13)

| Compound No. | A | $R^5$ |
|---|---|---|
| 13-1 | Ring 15 | $CONH_2$ |
| 13-2 | Ring 15 | $SO_2NHMe$ |
| 13-3 | Ring 15 | $SO_2Me$ |
| 13-4 | Ring 16 | $CONH_2$ |
| 13-5 | Ring 16 | $SO_2NHMe$ |
| 13-6 | Ring 16 | $SO_2Me$ |
| 13-7 | Ring 17 | $CONH_2$ |
| 13-8 | Ring 17 | $SO_2NHMe$ |
| 13-9 | Ring 17 | $SO_2Me$ |
| 13-10 | Ring 18 | $CONH_2$ |
| 13-11 | Ring 18 | $SO_2NHMe$ |
| 13-12 | Ring 18 | $SO_2Me$ |
| 13-13 | Ring 19 | $CONH_2$ |
| 13-14 | Ring 19 | $SO_2NHMe$ |
| 13-15 | Ring 19 | $SO_2Me$ |
| 13-16 | Ring 20 | $CONH_2$ |
| 13-17 | Ring 20 | $SO_2NHMe$ |
| 13-18 | Ring 20 | $SO_2Me$ |
| 13-19 | Ring 21 | $CONH_2$ |
| 13-20 | Ring 21 | $SO_2NHMe$ |
| 13-21 | Ring 21 | $SO_2Me$ |
| 13-22 | Ring 22 | $CONH_2$ |
| 13-23 | Ring 22 | $SO_2NHMe$ |

| | | |
|---|---|---|
| 13-24 | Ring 22 | $SO_2Me$ |
| 13-25 | Ring 23 | $CONH_2$ |
| 13-26 | Ring 23 | $SO_2NHMe$ |
| 13-27 | Ring 23 | $SO_2Me$ |
| 13-28 | Ring 24 | $CONH_2$ |
| 13-29 | Ring 24 | $SO_2NHMe$ |
| 13-30 | Ring 24 | $SO_2Me$ |
| 13-31 | Ring 25 | $CONH_2$ |
| 13-32 | Ring 25 | $SO_2NHMe$ |
| 13-33 | Ring 25 | $SO_2Me$ |
| 13-34 | Ring 26 | $CONH_2$ |
| 13-35 | Ring 26 | $SO_2NHMe$ |
| 13-36 | Ring 26 | $SO_2Me$ |
| 13-37 | Ring 27 | $CONH_2$ |
| 13-38 | Ring 27 | $SO_2NHMe$ |
| 13-39 | Ring 27 | $SO_2Me$ |
| 13-40 | Ring 28 | $CONH_2$ |
| 13-41 | Ring 28 | $SO_2NHMe$ |
| 13-42 | Ring 28 | $SO_2Me$ |
| 13-43 | Ring 29 | $CONH_2$ |
| 13-44 | Ring 29 | $SO_2NHMe$ |
| 13-45 | Ring 29 | $SO_2Me$ |
| 13-46 | Ring 30 | $CONH_2$ |
| 13-47 | Ring 30 | $SO_2NHMe$ |
| 13-48 | Ring 30 | $SO_2Me$ |
| 13-49 | Ring 31 | $CONH_2$ |
| 13-50 | Ring 31 | $SO_2NHMe$ |
| 13-51 | Ring 31 | $SO_2Me$ |
| 13-52 | Ring 32 | $CONH_2$ |
| 13-53 | Ring 32 | $SO_2NHMe$ |
| 13-54 | Ring 32 | $SO_2Me$ |
| 13-55 | Ring 33 | $CONH_2$ |
| 13-56 | Ring 33 | $SO_2NHMe$ |
| 13-57 | Ring 33 | $SO_2Me$ |
| 13-58 | Ring 34 | $CONH_2$ |
| 13-59 | Ring 34 | $SO_2NHMe$ |

| | | |
|---|---|---|
| 13-60 | Ring 34 | $SO_2Me$ |
| 13-61 | Ring 35 | $CONH_2$ |
| 13-62 | Ring 35 | $SO_2NHMe$ |
| 13-63 | Ring 35 | $SO_2Me$ |
| 13-64 | Ring 36 | $CONH_2$ |
| 13-65 | Ring 36 | $SO_2NHMe$ |
| 13-66 | Ring 36 | $SO_2Me$ |
| 13-67 | Ring 37 | $CONH_2$ |
| 13-68 | Ring 37 | $SO_2NHMe$ |
| 13-69 | Ring 37 | $SO_2Me$ |
| 13-70 | Ring 38 | $CONH_2$ |
| 13-71 | Ring 38 | $SO_2NHMe$ |
| 13-72 | Ring 38 | $SO_2Me$ |
| 13-73 | Ring 39 | $CONH_2$ |
| 13-74 | Ring 39 | $SO_2NHMe$ |
| 13-75 | Ring 39 | $SO_2Me$ |
| 13-76 | Ring 40 | $CONH_2$ |
| 13-77 | Ring 40 | $SO_2NHMe$ |
| 13-78 | Ring 40 | $SO_2Me$ |
| 13-79 | Ring 41 | $CONH_2$ |
| 13-80 | Ring 41 | $SO_2NHMe$ |
| 13-81 | Ring 41 | $SO_2Me$ |
| 13-82 | Ring 42 | $CONH_2$ |
| 13-83 | Ring 42 | $SO_2NHMe$ |
| 13-84 | Ring 42 | $SO_2Me$ |
| 13-85 | Ring 43 | $CONH_2$ |
| 13-86 | Ring 43 | $SO_2NHMe$ |
| 13-87 | Ring 43 | $SO_2Me$ |
| 13-88 | Ring 44 | $CONH_2$ |
| 13-89 | Ring 44 | $SO_2NHMe$ |
| 13-90 | Ring 44 | $SO_2Me$ |
| 13-91 | Ring 45 | $CONH_2$ |
| 13-92 | Ring 45 | $SO_2NHMe$ |
| 13-93 | Ring 45 | $SO_2Me$ |
| 13-94 | Ring 46 | $CONH_2$ |
| 13-95 | Ring 46 | $SO_2NHMe$ |

| | | |
|---|---|---|
| 13-96 | Ring 46 | $SO_2Me$ |
| 13-97 | Ring 47 | $CONH_2$ |
| 13-98 | Ring 47 | $SO_2NHMe$ |
| 13-99 | Ring 47 | $SO_2Me$ |
| 13-100 | Ring 48 | $CONH_2$ |
| 13-101 | Ring 48 | $SO_2NHMe$ |
| 13-102 | Ring 48 | $SO_2Me$ |
| 13-103 | Ring 49 | $CONH_2$ |
| 13-104 | Ring 49 | $SO_2NHMe$ |
| 13-105 | Ring 49 | $SO_2Me$ |
| 13-106 | Ring 50 | $CONH_2$ |
| 13-107 | Ring 50 | $SO_2NHMe$ |
| 13-108 | Ring 50 | $SO_2Me$ |
| 13-109 | Ring 51 | $CONH_2$ |
| 13-110 | Ring 51 | $SO_2NHMe$ |
| 13-111 | Ring 51 | $SO_2Me$ |

Table 14

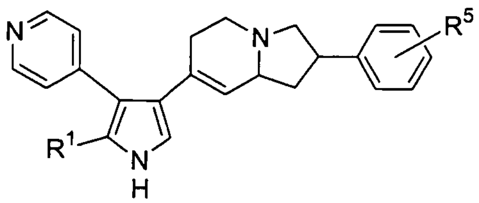

(I-14)

| Compound No. | $R^1$ | $R^5$ |
|---|---|---|
| 14-1 | 4-F-Ph | 2-COOH |
| 14-2 | 4-F-Ph | 3-COOH |
| 14-3 | 4-F-Ph | 2-$CONH_2$ |
| 14-4 | 4-F-Ph | 3-$CONH_2$ |
| 14-5 | 4-F-Ph | 3-CONHMe |
| 14-6 | 4-F-Ph | 3-CONHEt |
| 14-7 | 4-F-Ph | 3-CONHPr |
| 14-8 | 4-F-Ph | 3-CONH-i-Pr |
| 14-9 | 4-F-Ph | 2-$SO_2NH_2$ |
| 14-10 | 4-F-Ph | 3-$SO_2NH_2$ |
| 14-11 | 4-F-Ph | 3-$SO_2NHMe$ |
| 14-12 | 4-F-Ph | 3-$SO_2NHEt$ |

| | | |
|---|---|---|
| 14-13 | 4-F-Ph | 3-$SO_2$NHPr |
| 14-14 | 4-F-Ph | 3-$SO_2$NH-i-Pr |
| 14-15 | 4-F-Ph | 2-SOMe |
| 14-16 | 4-F-Ph | 3-SOMe |
| 14-17 | 4-F-Ph | 2-$SO_2$Me |
| 14-18 | 4-F-Ph | 3-$SO_2$Me |
| 14-19 | 3-Cl-4-F-Ph | 2-COOH |
| 14-20 | 3-Cl-4-F-Ph | 3-COOH |
| 14-21 | 3-Cl-4-F-Ph | 2-$CONH_2$ |
| 14-22 | 3-Cl-4-F-Ph | 3-$CONH_2$ |
| 14-23 | 3-Cl-4-F-Ph | 3-CONHMe |
| 14-24 | 3-Cl-4-F-Ph | 3-CONHEt |
| 14-25 | 3-Cl-4-F-Ph | 3-CONHPr |
| 14-26 | 3-Cl-4-F-Ph | 3-CONH-i-Pr |
| 14-27 | 3-Cl-4-F-Ph | 2-$SO_2NH_2$ |
| 14-28 | 3-Cl-4-F-Ph | 3-$SO_2NH_2$ |
| 14-29 | 3-Cl-4-F-Ph | 3-$SO_2$NHMe |
| 14-30 | 3-Cl-4-F-Ph | 3-$SO_2$NHEt |
| 14-31 | 3-Cl-4-F-Ph | 3-$SO_2$NHPr |
| 14-32 | 3-Cl-4-F-Ph | 3-$SO_2$NH-i-Pr |
| 14-33 | 3-Cl-4-F-Ph | 2-SOMe |
| 14-34 | 3-Cl-4-F-Ph | 3-SOMe |
| 14-35 | 3-Cl-4-F-Ph | 2-$SO_2$Me |
| 14-36 | 3-Cl-4-F-Ph | 3-$SO_2$Me |

In the table shown above, "Ac" represents an acetyl group; "Allyl" represents an allyl group; "Bn" represents a benzyl group; "Bu" represents a butyl group; "c-Bu" represents a cyclobutyl group; "Et" represents an ethyl group; "c-Hep" represents a cyclohexyl group; "c-Hex" represents a cyclohexyl group; "Me" represents a methyl group; "c-Pen" represents a cyclopentyl group; "Ph" represents a phenyl group; "Propargyl" represents a propargyl group; "Pr" represents a propyl group; "i-Pr" represents an isopropyl group; "c-Pr" represents a cyclopropyl group; "Pym" represents a pyrimidinyl group; "Pyr" represents a pyridyl group; "=$CH_2$" represents a methylidenyl group and "=O" represents a carbonyl group, respectively.

The indications of "Ring 1" to "Ring 14" shown in column A in Table 6 represent the ring corresponding to the relevant number shown in following Table 15, respectively.

Table 15

| | | | | | |
|---|---|---|---|---|---|
| Ring 1: | 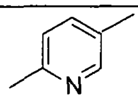 | Ring 2: | 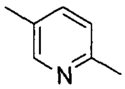 | Ring 3: | 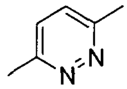 |
| Ring 4: | 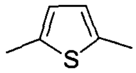 | Ring 5: | 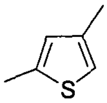 | Ring 6: | 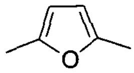 |
| Ring 7: | 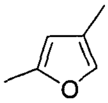 | Ring 8: | 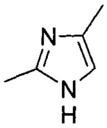 | Ring 9: | 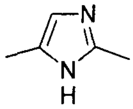 |
| Ring 10: | 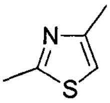 | Ring11: | 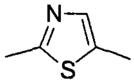 | Ring12: | 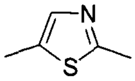 |
| Ring 13: | 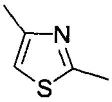 | Ring 14: | 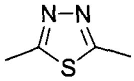 | | |

In each ring shown in above Table 15, $R^5$ is bonded to each ring at the rightmost bonding position.

The indications of "Ring 15" to "Ring 51" shown in column A in Tables 12 and 13 represent the ring corresponding to the relevant number shown in following Table 16, respectively.

Table 16

| | | | | | |
|---|---|---|---|---|---|
| Ring 15: | 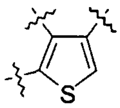 | Ring 16: | 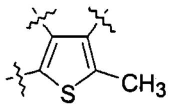 | Ring 17: | 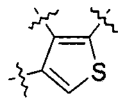 |
| Ring 18: | 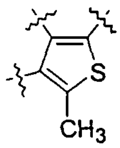 | Ring 19: | 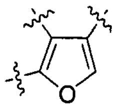 | Ring 20: | 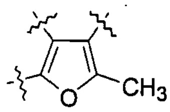 |
| Ring 21: | 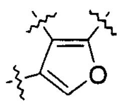 | Ring 22: | 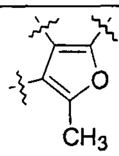 | Ring 23: | 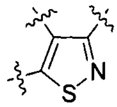 |

| | | | | | |
|---|---|---|---|---|---|
| Ring 24: | 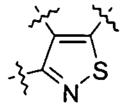 | Ring 25: | 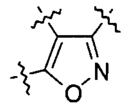 | Ring 26: | 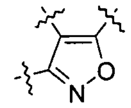 |
| Ring 27: | 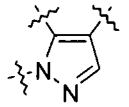 | Ring 28: | 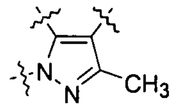 | Ring 29: | 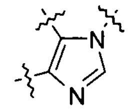 |
| Ring 30: | 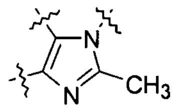 | Ring 31: | 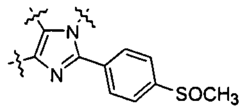 | Ring 32: | 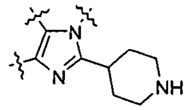 |
| Ring 33: | 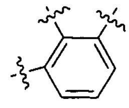 | Ring 34: | 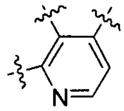 | Ring 35: | 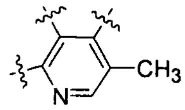 |
| Ring 36: | 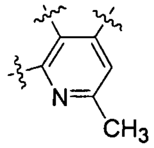 | Ring 37: | 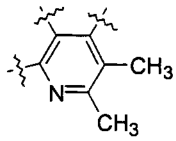 | Ring 38: | 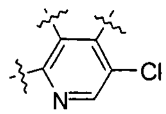 |
| Ring 39: | 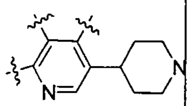 | Ring 40: | 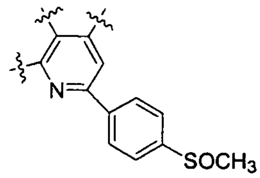 | Ring 41: | 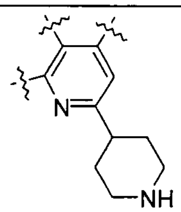 |
| Ring 42: | 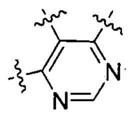 | Ring 43: | 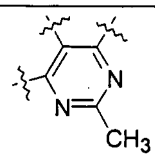 | Ring 44: | 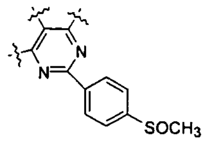 |
| Ring 45: | 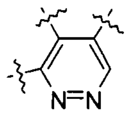 | Ring 46: | 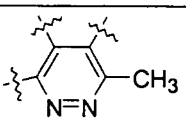 | Ring 47: | 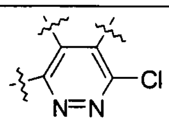 |
| Ring 48: | 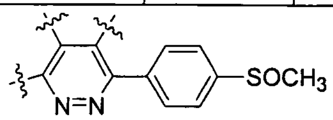 | Ring 49: | 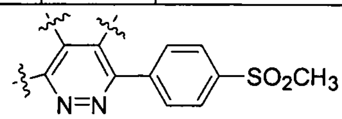 | | |
| Ring 50: | 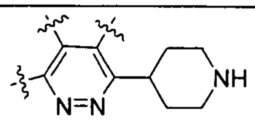 | Ring 51: | 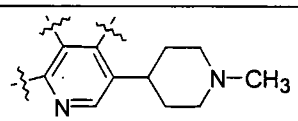 | | |

In each ring shown in above Table 16, $R^1$ (4-F-Ph group) is bonded to each ring at the leftmost bonding position.

In Tables 10 and 11 shown above, compounds wherein the substituent on the nitrogen atom of pyrazole ring corresponding to A shown in the general formula (1) is a hydrogen atom can exist as a tautomer as shown below.

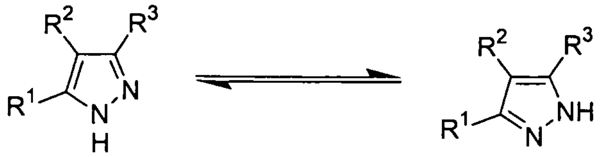

Exemplified compounds shown in Tables 10 and 11 are all intended compounds in the present invention.

Among the said compounds, preferable compounds are Example Compound Nos. 1-1 to 1-4, 1-9 to 1-14, 1-17 to 1-20, 1-23 to 1-24, 1-28 to 1-30, 1-32 to 1-34, 1-39 to 1-40, 1-43 to 1-46, 1-51 to 1-53, 1-57 to 1-63, 1-66 to 1-72, 1-75 to 1-93,1-96 to 1-103, 1-108 to 1-110, 1-112 to 1-115, 1-120 to 1-123, 1-128 to 1-133, 1-136 to 1-139, 1-142 to 1-143, 1-147 to 1-149, 1-151 to 1-153, 1-158 to 1-159, 1-162 to 1-165, 1-170 to 1-172, 1-176 to 1-182, 1-185 to 1-191, 1-194 to 1-212, 1-215 to 1-222, 1-227 to 1-229, 1-231 to 1-234, 1-239 to 1-242, 1-247 to 1-252, 1-255 to 1-258,1-261 to 1-262, 1-266 to 1-268, 1-270 to 1-272, 1-277 to 1-278, 1-261 to 1-284, 1-289 to 1-291, 1-295 to 1-301, 1-304 to 1-310, 1-313 to 1-331, 1-334 to 1-341, 1-346 to 1-348, 1-350 to 1-353, 1-358 to 1-361, 1-366 to 1-371, 1-374 to 1-377, 1-380 to 1-381, 1-385 to 1-387, 1-389 to 1-391, 1-396 to 1-397, 1-400 to 1-403, 1-408 to 1-410, 1-414- to 1-420, 1-423 to 1-429, 1-432 to 1-450, 1-453 to 1-460, 1-465 to 1-467, 1-469 to 1-472, 1-477 to 1-480, 1-485 to 1-490, 1-493 to 1-496, 1-499 to 1-500, 1-504 to 1-506, 1-508 to 1-510, 1-515 to 1-516, 1-519 to 1-522, 1-527 to 1-529, 1-533 to 1-539, 1-542 to 1-548, 1-551 to 1-569, 1-572 to 1-579, 1-584 to 1-586, 1-588 to 1-591, 1-596 to 1-599, 1-604 to 1-609, 1-612 to 1-615, 1-618 to 1-619, 1-623 to 1-625, 1-627 to 1-629, 1-634 to 1-635, 1-638 to 1-641, 1-646 to 1-648, 1-652 to 1-658, 1-661 to 1-667, 1-670 to 1-688, 1-691 to 1-698, 1-703 to 1-705, 1-707 to 1-710, 1-715 to 1-718,1-723 to 1-728, 1-731 to 1-734, 1-737 to 1-738, 1-742 to 1-744, 1-746 to 1-748, 1-753 to 1-754, 1-757 to 1-760, 1-765 to 1-767, 1-771 to 1-777, 1-780 to 1-786, 1-789 to 1-807, 1-810 to 1-817, 1-822 to 1-824, 1-826 to 1-829, 1-834 to 1-839, 1-842 to 1-845, 1-848 to 1-849, 1-853 to 1-855, 1-857 to 1-859, 1-864 to 1-865, 1-867 to 1-882, 1-885 to 1-892, 1-897 to 1-899, 1-901 to 1-904, 1-909 to 1-914, 1-917 to 1-920, 1-923 to 1-924, 1-928 to 1-930, 1-932 to 1-934, 1-939 to 1-940, 1-942 to 1-957, 1-960 to 1-967, 1-972 to 1-974, 1-976 to 1-979, 1-984 to 1-989, 1-992 to 1-995, 1-998 to 1-999, 1-1003 to 1-1005, 1-1007 to 1-1009, 1-1014 to 1015, 1-1017 to 1-1032, 1-1035 to 1-1042, 1-1047 to 1-1049, 1-1051 to 1-1054, 1-1059 to 1-1064, 1-1067 to 1-1070, 1-1073 to 1-1074, 1-1078 to 1-1080, 1-1082 to 1-1084, 1-1089 to 1-1090, 1-1092 to 1-1107, 1-1110 to 1-1117, 1-1122 to 1-1124, 1-1126 to 1-1129, 1-1134 to 1-1139, 1-1142 to 1-1145, 1-1148 to 1-1149, 1-1153 to 1-1155, 1-1157 to 1-1159, 1-1164 to 1-1165, 1-1167 to 1-1182, 1-1185 to 1-1192, 1-1197 to 1-1199, 1-1201 to 1-1204, 1-1209 to 1-1214, 1-1217 to 1-1220, 1-1223 to 1-1224, 1-1228 to 1-1230, 1-1232 to 1-1234, 1-1239 to 1-1240, 1-1242 to 1-1257, 1-1260 to 1-1267, 1-1272 to 1-1274, 1-1276 to 1-1279, 1-1284 to 1-1285, 1-1287, 1-1290, 1-1293 to 1-1294, 1-1296, 1-1299, 1-1302 to 1-1303, 1-1305, 1-1308, 1-1311 to 1-1312, 1-1314, 1-1317, 1-1320 to 1-1321, 1-1323, 1-1326, 1-1329 to 1-1330, 1-1332, 1-1335, 1-1338, 1-1341 to 1-1343, 1-1346 to 1-1348,
2-1 to 2-6, 2-11 to 2-16, 2-21 to 2-26, 2-31 to 2-36,
3-5, 3-11, 3-16, 3-18, 3-23, 3-29, 3-34, 3-36, 3-38, 3-43, 3-47 to 3-50,
6-2, 6-8, 6-13, 6-16, 6-19, 6-23, 6-29, 6-34, 6-37, 6-40, 6-44, 6-50, 6-55, 6-58, 6-61, 6-65, 6-71, 6-76, 6-79, 6-82, 6-86, 6-92, 6-97, 6-100, 6-103, 6-107, 6-113, 6-118, 6-121, 6-124, 6-128, 6-134, 6-139, 6-142, 6-145, 6-149, 6-155, 6-160, 6-163, 6-166, 6-170, 6-176, 6-181, 6-184, 6-187, 6-191, 6-197, 6-202, 6-205, 6-208, 6-212, 6-218, 6-223, 6-226, 6-229, 6-233, 6-239, 6-244, 6-247, 6-250, 6-254, 6-260, 6-265, 6-268, 6-271, 6-275, 6-281, 6-286, 6-289, 6-292, 6-296, 6-302, 6-307, 6-310, 6-313, 6-317, 6-323, 6-328, 6-331, 6-334, 6-338, 6-344, 6-349, 6-352, 6-355, 6-359, 6-365, 6-370, 6-373, 6-376, 6-380, 6-386, 6-391, 6-394, 6-397, 6-401, 6-407, 6-412, 6-415, 6-418, 6-422, 6-428, 6-433, 6-436, 6-439, 6-443, 6-449, 6-454, 6-457, 6-460, 6-464, 6-470, 6-475, 6-478, 6-481, 6-485, 6-491, 6-496, 6-499, 6-502, 6-506, 6-512, 6-517, 6-520, 6-523, 6-527, 6-533, 6-538, 6-541, 6-544, 6-548, 6-554, 6-559, 6-562, 6-565, 6-569, 6-575, 6-580, 6-583, 6-586,
7-1 to 7-4, 7-9 to 7-14, 7-17 to 7-20, 7-23 to 7-24, 7-28 to 7-30, 7-32 to 7-34, 7-39 to 7-40, 7-43 to 7-46, 7-51 to 7-53, 7-57 to 7-63, 7-66 to 7-72, 7-75 to 7-93, 7-96 to 7-103, 7-108 to 7-110, 7-112 to 7-115, 7-120 to 7-123, 7-128 to 7-133, 7-136 to 7-139, 7-142 to 7-143, 7-147 to 71-149, 7-151 to 7-153, 7-158 to 7-159, 7-162 to 7-165, 7-170 to 7-172, 7-176 to 7-182, 7-185 to 7-191, 7-194 to 71-212, 7-215 to 7-222, 7-227 to 7-229, 7-231 to 7-234, 7-239 to 7-242, 7-247 to 7-252, 7-255 to 7-258, 7-261 to 7-262, 7-266 to 7-268, 7-270 to 7-272, 7-277 to 7-278, 7-281 to 7-284, 7-289 to 7-291, 7-295 to 7-301, 7-304 to 7-310, 7-313 to 7-331, 7-334 to 7-341, 7-346 to 7-348, 7-350 to 7-353, 7-358 to 7-361, 7-366 to 7-371, 7-374 to 7-377, 7-380 to 7-381, 7-385 to 7-387, 7-389 to 7-391, 7-396 to 7-397, 7-400 to 7-403, 7-408 to 7-410, 7-414 to 7-420, 7-423 to 7-429, 7-432 to 7-450, 7-453 to 7-460, 7-465 to 7-467, 7-469 to 7-472, 7-477 to 7-480, 7-485 to 7-490, 7-493 to 7-496, 7-499 to 7-500, 7-504 to 7-506, 7-508 to 7-510, 7-515 to 7-516, 7-519 to 7-522, 7-527 to 7-529, 7-533 to 7-539, 7-542 to 7-548, 7-551 to 7-569, 7-572 to 7-579, 7-584 to 7-586, 7-588 to 7-591, 7-596 to 7-599, 7-604 to 7-609, 7-612 to 7-615, 7-618 to 7-619, 7-623 to 7-625, 7-627 to 7-629, 7-634 to 7-635, 7-638 to 7-641, 7-646 to 7-648, 7-652 to 7-658, 7-661 to 7-667, 7-670 to 7-688, 7-691 to 7-698,7-703 to 7-705, 7-707 to 7-710, 7-715 to 7-718, 7-723 to 7-728, 7-731 to 7-734, 7-737 to 7-738, 7-742 to 7-744, 7-746 to 7-748, 7-753 to 7-754, 7-757 to 7-760, 7-765 to 7-767,

7-771 to 7-777, 7-780 to 7-786, 7-789 to 7-807, 7-810 to 7-817, 7-822 to 7-824, 7-826 to 7-829, 7-834 to 7-839, 7-842 to 7-845, 7-848 to 7-849, 7-853 to 7-855, 7-857 to 7-859, 7-864 to 7-865, 7-867 to 7-882, 7-885 to 7-892, 7-897 to 7-899, 7-901 to 7-904, 7-909 to 7-914, 7-917 to 7-920, 7-923 to 7-924, 7-928 to 7-930, 7-932 to 7-934, 7-939 to 7-940, 7-942 to 7-957, 7-960 to 7-967, 7-972 to 7-974, 7-976 to 7-979, 7-984 to 7-989, 7-992 to 7-995, 7-998 to 7-999, 7-1003 to 7-1005, 7-1007 to 7-100 9, 7-1014 to 7-1015, 7-1017 to 7-1032, 7-1035 to 7-1042, 7-1047 to 7-1049, 7-1051 to 7-1054, 7-1059 to 7-1064, 7-1067 to 7-1070, 7-1073 to 7-1074, 7-1078 to 7-1080, 7-1082 to 7-1084, 7-1089 to 7-1090, 7-1092 to 7-1107, 7-1110 to 7-1117, 7-1122 to 7-1124, 7-1126 to 7-1129, 7-1134 to 7-1139, 7-1142 to 7-1145, 7-1148 to 7-1149, 7-1153 to 7-1155, 7-1157 to 7-1159, 7-1164 to 7-1165, 7-1 167 to 7-1182, 7-1185 to 7-1192, 7-1197 to 7-1199, 7-1201 to 7-1 204, 7-1209 to 7-1214, 7-1217 to 7-1220, 7-1223 to 7-1224, 7-1228 to 7-1230, 7-1232 to 7-1234, 7-1239 to 7-1240, 7-1242 to 7-1257, 7-1260 to 7-1267, 7-1272 to 7-1274, 7-1276 to 7-1279, 7-1284 to 7-1289,

8-2, 8-8, 8-13, 8-16, 8-19, 8-23, 8-29, 8-34, 8-37, 8-40,

9-2, 9-8, 9-13, 9-16, 9-19, 9-23, 9-29, 9-34, 9-37, 9-40, 9-44, 9-50, 9-55, 9-58, 9-61, 9-65, 9-71, 9-76, 9-79, 9-82, 9-86, 9-92, 9-97, 9-100, 9-103, 9-107, 9-113, 9-118, 9-121, 9-124, 9-128, 9-134, 9-139, 9-142, 9-145, 9-149, 9-155, 9-160, 9-163, 9-166, 9-170, 9-176, 9-181, 9-184, 9-187, 9-191, 9-197, 9-202, 9-205, 9-208, 9-212, 9-218, 9-223, 9-226, 9-229, 9-233, 9-239, 9-244, 9-247, 9-250, 9-254, 9-260, 9-265, 9-268, 9-271, 9-275, 9-281, 9-286, 9-289, 9-292, 9-296, 9-302, 9-307, 9-310, 9-313, 9-317, 9-323, 9-328, 9-331, 9-334, 9-338, 9-344, 9-349, 9-352, 9-355, 9-359, 9-365, 9-370, 9-373, 9-376, 9-380, 9-386, 9-391, 9-394, 9-397, 9-401, 9-407, 9-412, 9-415, 9-418, 9-422, 9-428, 9-433, 9-436, 9-439, 9-443, 9-449, 9-454, 9-457, 9-460, 9-464, 9-470, 9-475, 9-478, 9-481, 9-485, 9-491, 9-496, 9-499 9-502, 9-506, 9-512, 9-517, 9-520, 9-523, 9-527, 9-533, 9-538, 9-541, 9-544, 9-548, 9-554, 9-559, 9-562, 9-565, 9-569, 9-575, 9-580, 9-583, 9-586,

10-1 to 10-4, 10-9 to 10-14, 10-17 to 10-20, 10-23 to 10-24, 10-28 to 10-30, 10-32 to 10-34, 10-39 to 10-40, 10-43 to 10-46, 10-51 to 10-53, 10-57 to 10-63, 10-66 to 10-72, 10-75 to 10-93, 10-96 to 10-103, 10-108 to 10-110, 10-112 to 10-115, 10-120 to 10-123, 10-128 to 10-133, 10-136 to 10-139, 10-142 to 10-143, 10-147 to 10-149, 10-151 to 10-153, 10-158 to 10-159, 10-162 to 10-165, 10-170 to 10-172, 10-176 to 10-182, 10-185 to 10-191, 10-194 to 10-212, 10-215 to 10-222, 10-227 to 10-229, 10-231 to 10-234, 10-239 to 10-242, 10-247 to 10-252, 10-255 to 10-258, 10-261 to 10-262, 10-266 to 10-268, 10-270 to 10-272, 10-277 to 10-278, 10-281 to 10-284, 10-289 to 10-291, 10-295 to 10-301, 10-304 to 10-310, 10-313 to 10-331, 10-334 to 10-341, 10.-346 to 10-348, 10-350 to 10-353, 10-358 to 10-361, 10-366 to 10-371, 10-374 to 10-377, 10-380 to 10-381, 10-385 to 10-387, 10-389 to 10-391, 10-396 to 10-397, 10-400 to 10-403, 10-408 to 10-410, 10-414 to 10-420, 10-423 to 10-429, 10-432 to 10-450, 10-453 to 10-460, 10-465 to 10-467, 10-469 to 10-472, 10-477 to 10-480, 10-485 to 10-490, 10-493 to 10-496, 10-499 to 10-500, 10-504 to 10-506, 10-508 to 10-510, 10-515 to 10-516, 10-519 to 10-522, 10-527 to 10-529, 10-533 to 10-539, 10-542 to 10-548, 10-551 to 10-569, 10-572 to 10-579, 10-584 to 10-586, 10-588 to 10-591, 10-596 to.10-599, 10-604 to 10-609, 10-612 to 10-615, 10-618 to 10-619, 10-623 to 10-625, 10-627 to 10-629, 10-634 to 10-635, 10-638 to 10-641, 10-646 to 10-648, 10-652 to 10-658, 10-661 to 10-667, 10-670 to 10-688, 10-691 to 10-698, 10-703 to 10-705, 10-707 to 10-710, 10-715 to 10-718, 10-723 to 10-728, 10-731 to 10-734, 10-737 to 10-738, 10-742 to 10-744, 10-746 to 10-748, 10-753 to 10-754, 10-757 to 10-760, 10-765 to 10-767, 10-771 to 10-777, 10-780 to 10-786, 10-789 to 10-807, 10-810 to 10-817, 10-822 to 10-824, 10-826 to 10-829, 10-834 to 10-839, 10-842 to 10-845, 10-848 to 10-849, 10-853 to 10-855, 10-857 to 10-859, 10-864 to 10-865, 10-867 to 10-882, 10-885 to 10-892, 10-897 to 10-899, 10-901 to 10-904, 10-909 to 10-914, 10-917 to 10-920, 10-923 to 10-924, 10-928 to 10-930, 10-932 to 10-934, 10-939 to 10-940, 10-942 to 10-957, 10-960 to 10-967, 10-972 to 10-974, 10-976 to 10-979, 10-984 to 10-989, 10-992 to 10-995, 10-998 to 10-999, 10-1003 to 10-1005, 10-1007 to 10-1009, 10-1014 to 10-1015, 10-1017 to 10-1032, 10-1035 to 10-1042, 10-1047 to 10-1049, 10-1051 to 10-1054, 10-1059 to 10-1064, 10-1067 to 10-1070, 10-1073 to 10-1074, 10-1078 to 10-1080, 10-1082 to 10-1084, 10-1089 to 10-1090, 10-1092 to 10-1107, 10-1110 to 10-1117, 10-1122 to 10-1124, 10-1126 to 10-1129, 10-1134 to 10-1139, 10-1142 to 10-1145, 10-1148 to 10-1149, 10-1153 to 10-1155, 10-1157 to 10-1159, 10-1164 to 10-1165, 10-1167 to 10-1182, 10-1185 to 10-1192, 10-1197 to 10-1199, 10-1201 to 10-1204, 10-1209 to 10-1214, 10-1217 to 10-1220, 10-1223 to 10-1224, 10-1228 to 10-1230, 10-1232 to 10-1234, 10-1239 to 10-1240, 10-1242 to 10-1257, 10-1260 to 10-1267, 10-1272 to 10-1274, 10-1276 to 10-1279, 10-1284 to 10-1289,

11-1 to 11-4, 11-9 to 11-14, 11-17 to 11-20, 11-23 to 11-24, 11-28 to 11-30, 11-32 to 11-34, 11-39 to 11-40, 11-43 to 11-46, 11-51 to 11-53, 11-57 to 11-63, 11-66 to 11-72, 11-75 to 11-93, 11-96 to 11-103, 11-108 to 11-110, 11-112 to 11-115, 11-120 to 11-123, 11-128 to 11-133, 11-136 to 11-139, 11-142 to 11-143, 11-147 to 11-149, 11-151 to 11-153, 11-158 to 11-159, 11-162 to 11-165, 11-170 to 11-172, 11-176 to 11-182, 11-185 to 11-191, 11-194 to 11-212, 11-215 to 11-222, 11-227 to 11-229, 11-231 to 11-234, 11-239 to 11-242, 11-247 to 11-252, 11-255 to 11-258, 11-261 to 11-262, 11-266 to 11-268, 11-270 to 11-272, 11-277 to 11-278, 11-281 to 11-284, 11-289 to 11-291, 11-295 to 11-301, 11-304 to 11-310, 11-313 to 11-331, 11-334 to 11-341, 11-346 to 11-348, 11-350 to 11-353, 11-358 to 11-361, 11-366 to 11-371, 11-374 to 11-377, 11-380 to 11-381, 11-385 to 11-387, 11-389 to 11-391, 11-396 to 11-397, 11-400 to 11-403, 11-408 to 11-410, 11-414 to 11-420, 11-423 to 11-429, 11-432 to 11-450, 11-453 to 11-460, 11-465 to 11-467, 11-469 to 11-472, 11-477 to 11-480, 11-485 to 11-490, 11-493 to 11-496, 11-499 to 11-500, 11-504 to 11-506, 11-508 to 11-510, 11-515 to 11-516, 11-519 to 11-522, 11-527 to 11-529, 11-533 to 11-539, 11-542 to 11-548, 11-551 to 11-569, 11-572 to 11-579, 11-584 to 11-586, 11-588 to 11-591, 11-596 to 11-599, 11-604 to 11-609, 11-612 to 11-615, 11-618 to 11-619, 11-623

to 11-625, 11-627 to 11-629, 11-634 to 11-635, 11-638 to 11-641, 11-646 to 11-648, 11-652 to 11-658, 11-661 to 11-667, 11-670 to 11-688, 11-691 to 11-698, 11-703 to 11-705, 11-707 to 11-710, 11-715 to 11-718, 11-723 to 11-728, 11-731 to 11-734, 11-737 to 11-738, 11-742 to 11-744, 11-746 to 11-748, 11-753 to 11-754, 11-757 to 11-760, 11-765 to 11-767, 11-771 to 11-777, 11-780 to 11-786, 11-789 to 11-807, 11-810 to 11-817, 11-822 to 11-824, 11-826 to 11-829, 11-834 to 11-839, 11-842 to 11-845, 11-848 to 11-849, 11-853 to 11-855, 11-857 to 11-859, 11-864 to 11-865, 11-867 to 11-882, 11-885 to 11-892, 11-897 to 11-899, 11-901 to 11-904, 11-909 to 11-914, 11-917 to 11-920, 11-923 to 11-924, 11-928 to 11-930, 11-932 to 11-934, 11-939 to 11-940, 11-942 to 11-957, 11-960 to 11-967, 11-972 to 11-974, 11-976 to 11-979, 11-984 to 11-989, 11-992 to 11-995, 11-998 to 11-999, 11-1003 to 11-1005, 11-1007 to 11-1009, 11-1014 to 11-1015, 11-1017 to 11-1032, 11-1035 to 11-1042, 11-1047 to 11-1049, 11-1051 to 11-1054, 11-1059 to 11-1064, 11-1067 to 11-1070, 11-1073 to 11-1074, 11-1078 to 11-1080, 11-1082 to 11-1084, 11-1089 to 11-1090, 11-1092 to 11-1107, 11-1110 to 11-1117, 11-1122 to 11-1124, 11-1126 to 11-1129, 11-1134 to 11-1139, 11-1142 to 11-1145, 11-1148 to 11-1149, 11-1153 to 11-1155, 11-1157 to 11-1159, 11-1164 to 11-1165, 11-1167 to 11-1182, 11-1185 to 11-1192, 11-1197 to 11-1199, 11-1201 to 11-1204, 11-1209 to 11-1214, 11-1217 to 11-1220, 11-1223 to 11-1224, 11-1228 to 11-1230, 11-1232 to 11-1234, 11-1239 to 11-1240, 11-1242 to 11-1257, 11-1260 to 11-1267, 11-1272 to 11-1274, 11-1276 to 11-1279, 11-1284 to 11-1289,

14-5,14-11,14-16,14-18,14-23,14-29,14-34, and 14-36,

more preferable compounds are Example Compound Nos. 1-9 to 1-13, 1-17 to 1-19, 1-23 to 1-24, 1-32 to 1-33, 1-39, 1-51, 1-60 to 1-62, 1-69 to 1-72, 1-79 to 1-82, 1-85 to 1-86, 1-101 to 1-102, 1-112 to 1-115, 1-128 to 1-132, 1-136 to 1-138, 1-142 to 1-143, 1-151 to 1-152, 1-158, 1-170, 1-179 to 1-181, 1-188 to 1-191, 1-198 to 1-201, 1-204 to 1-205, 1-220 to 1-221, 1-231 to 1-234, 1-247 to 1-251, 1-255 to 1-257, 1-261 to 1-262, 1-270 to 1-271, 1-277, 1-289, 1-298 to 1-300, 1-307 to 1-310, 1-317 to 1-320, 1-323 to 1-324, 1-339 to 1-340, 1-350 to 1-353, 1-366 to 1-370, 1-374 to 1-376, 1-380 to 1-381, 1-389 to 1-390, 1-396, 1-408, 1-417 to 1-419, 1-426 to 1-429, 1-436 to 1-439, 1-442 to 1-443, 1-458 to 1-459, 1-469 to 1-472, 1-485 to 1-489, 1-493 to 1-495, 1-499 to 1-500, 1-508 to 1-509, 1-515, 1-527, 1-536 to 1-538, 1-545 to 1-548, 1-555 to 1-558, 1-561 to 1-562, 1-577 to 1-578, 1-588 to 1-591, 1-604 to 1-608, 1-612 to 1-614, 1-618 to 1-619, 1-627 to 1-628, 1-634, 1-646, 1-655 to 1-657, 1-664 to 1-667, 1-674 to 1-677, 1-680 to 1-681, 1-696 to 1-697, 1-707 to 1-710, 1-723 to 1-727, 1-731 to 1-733, 1-737 to 1-738, 1-746 to 1-747, 1-753, 1-765, 1-774 to 1-776, 1-783 to 1-786, 1-793 to 1-796, 1-799 to 1-800, 1-815 to 1-816, 1-826 to 1-829, 1-834 to 1-838, 1-842 to 1-844, 1-848 to 1-849, 1-857 to 1-858, 1-864, 1-868 to 1-871, 1-874 to 1-875, 1-890 to 1-891, 1-901 to 1-904, 1-909 to 1-913, 1-917 to 1-919, 1-923 to 1-924, 1-932 to 1-933, 1-939, 1-943 to 1-946, 1-949 to 1-950, 1-965 to 1-966, 1-976 to 1-979, 1-984 to 1-988, 1-992 to 1-994, 1-998 to 1-999, 1-1007 to 1-1008, 1-1014, 1-1018 to 1-1021, 1-1024 to 1-1025, 1-1040 to 1-1041, 1-1051 to 1-1054, 1-1059 to 1-1063, 1-1067 to 1-1069, 1-1073 to 1-1074, 1-1082 to 1-1083, 1-1089, 1-1093 to 1-1096, 1-1099 to 1-1100, 1-1115 to 1-1116, 1-1126 to 1-1129, 1-1134 to 1-1138, 1-1142 to 1-1144, 1-1148 to 1-1149, 1-1157 to 1-1158, 1-1164, 1-1168 to 1-1171, 1-1174 to 1-1175, 1-1190 to 1-1191, 1-1201 to 1-1204, 1-1209 to 1-1213, 1-1217 to 1-1219, 1-1223 to 1-1224, 1-1232 to 1-1233, 1-1239, 1-1243 to 1-1246, 1-1249 to 1-1250, 1-1265 to 1-1266, 1-1276 to 1-1279, 1-1341, 1-1342, 1-1346, 1-1347,

2-1 to 2-4, 2-11 to 2-14, 2-21 to 2-24, 2-31 to 2-34,

3-48 to 3-50,

7-9 to 7-13, 7-17 to 7-19, 7-23 to 7-24, 7-32 to 7-33, 7-39, 7-51, 7-60 to 7-62, 7-69 to 7-72, 7-79 to 7-82, 7-85 to 7-86, 7-101 to 7-102, 7-112 to 7-115, 7-128 to 7-132, 7-136 to 7-138, 7-142 to 7-143, 7-151 to 7-152, 7-158, 7-170, 7-179 to 7-181, 7-188 to 7-191, 7-198 to 7-201, 7-204 to 7-205, 7-220 to 7-221, 7-231 to 7-234, 7-247 to 7-251, 7-255 to 7-257, 7-261 to 7-262, 7-270 to 7-271, 7-277, 7-289, 7-298 to 7-300, 7-307 to 7-310, 7-317 to 7-320, 7-323 to 7-324, 7-339 to 7-340, 7-350 to 7-353, 7-366 to 7-370, 7-374 to 7-376, 7-380 to 7-381, 7-389 to 7-390, 7-396, 7-408, 7-417 to 7-419, 7-426 to 7-429, 7-436 to 7-439, 7-442 to 7-443, 7-458 to 7-459, 7-469 to 7-472, 7-485 to 7-489, 7-493 to 7-495, 7-499 to 7-500, 7-508 to 7-509, 7-515, 7-527, 7-536 to 7-538, 7-545 to 7-548, 7-555 to 7-558, 7-561 to 7-562,7-577to7-578,7-588to7-591,7-604to7-608,7-612to7-614,7-618 to 7-619, 7-627 to 7-628, 7-634, 7-646, 7-655 to 7-657, 7-664 to 7-667, 7-674 to 7-677, 7-680 to 7-681, 7-696 to 7-697, 7-707 to 7-710, 7-723 to 7-727, 7-731 to 7-733, 7-737 to 7-738, 7-746 to 7-747, 7-753, 7-765, 7-774 to 7-776, 7-783 to 7-786, 7-793 to 7-796, 7-799 to 7-800, 7-815 to 7-816, 7-826 to 7-829, 7-834 to 7-838, 7-842 to 7-844, 7-848 to 7-849, 7-857 to 7-858, 7-864, 7-868 to 7-871, 7-874 to 7-875, 7-890 to 7-891, 7-901 to 7-904, 7-909 to 7-913, 7-917 to 7-919, 7-923 to 7-924, 7-932 to 7-933, 7-939, 7-943 to 7-946, 7-949 to 7-950, 7-965 to 7-966, 7-976 to 7-979, 7-984 to 7-988, 7-992 to 7-994, 7-998 to 7-999, 7-1007 to 7-1008, 7-1014, 7-1018 to 7-1021, 7-1024 to 7-1025, 7-1040 to 7-1041, 7-1051 to 7-1054, 7-1059 to 7-1063 ,7-1067 to 7-1069, 7-1073 to 7-1074, 7-1082 to 7-1083, 7-1089, 7-1093 to 7-1096, 7-1099 to 7-1100, 7-1115 to 7-1116, 7-1126 to 7-1129, 7-1134 to 7-1138, 7-1142 to 7-1144, 7-1148 to 7-1149, 7-1157 to 7-1158, 7-1164, 7-1168 to 7-1171, 7-1174 to 7-1175, 7-1190 to 7-1191, 7-1201 to 7-1204, 7-1209 to 7-1213, 7-1217 to 7-1219, 7-1223 to 7-1224, 7-1232 to 7-1233, 7-1239, 7-1243 to 7-1246, 7-1249 to 7-1250, 7-1265 to 7-1266, 7-1276 to 7-1279,

10-9 to 10-13, 10-17 to 10-19, 10-23 to 10-24, 10-32 to 10-33, 10-39, 10-51, 10-60 to 10-62, 10-69 to 10-72, 10-79 to 10-82, 10-85 to 10-86, 10-101 to 10-102, 10-112 to 10-115, 10-128 to 10-132, 10-136 to 10-138, 10-142 to 10-143, 10-151 to 10-152, 10-158, 10-170, 10-179 to 10-181, 10-188 to 10-191, 10-198 to 10-201, 10-204 to 10-205, 10-220 to

10-221, 10-231 to 10-234, 10-247 to 10-251, 10-255 to 10-257, 10-261 to 10-262, 10-270 to 10-271, 10-277, 10-289, 10-298 to 10-300, 10-307 to 10-310, 10-317 to 10-320, 10-323 to 10-324, 10-339 to 10-340, 10-350 to 10-353, 10-366 to 10-370, 10-374 to 10-376, 10-380 to 10-381, 10-389 to 10-390, 10-396, 10-408, 10-417 to 10-419, 10-426 to 10-429, 10-436 to 10-439, 10-442 to 10-443, 10-458 to 10-459, 10-469 to 10-472, 10-485 to 10-489, 10-493 to 10-495, 10-499 to 10-500, 10-508 to 10-509, 10-515, 10-527, 10-536 to 10-538, 10-545 to 10-548, 10-555 to 10-558, 10-561 to 10-562, 10-577 to 10-578, 10-588 to 10-591, 10-604 to 10-608, 10-612 to 10-614, 10-618 to10-619, 10-627 to 10-628, 10-634, 10-646, 10-655 to 10-657, 10-664 to 10-667, 10-674 to 10-677, 10-680 to 10-681, 10-696 to 10-697, 10-707 to 10-710, 10-723 to 10-727, 10-731 to 10-733, 10-737 to 10-738, 10-746 to 10-747, 10-753, 10-765, 10-774 to 10-776, 10-783 to 10-786, 10-793 to 10-796, 10-799 to 10-800, 10-815 to 10-816, 10-826 to 10-829, 10-834 to 10-838, 10-842 to 10-844, 10-848 to 10-849, 10-857 to 10-858, 10-864, 10-868 to 10-871, 10-874 to 10-875, 10-890 to 10-891, 10-901 to 10-904, 10-909 to 10-913, 10-917 to 10-919, 10-923 to 10-924, 10-932 to 10-933, 10-939, 10-943 to 10-946, 10-949 to 10-950, 10-965 to 10-966, 10-976 to 10-979, 10-984 to 10-988, 10-992 to 10-994, 10-998 to 10-999, 10-1007 to 10-1008, 10-1014, 10-1018 to 10-1021, 10-1024 to 10-1025, 10-1040 to 10-1041, 10-1051 to 10-1054, 10-1059 to 10-1063, 10-1067 to 10-1069, 10-1073 to 10-1074, 10-1082 to 10-1083, 10-1089, 10-1093 to 10-1096, 10-1099 to 10-1100, 10-1115 to 10-1116, 10-1126 to 10-1129, 10-1134 to 10-1138, 10-1142 to 10-1144, 10-1148 to 10-1149, 10-1157 to 10-1158, 10-1164, 10-1168 to 10-1171, 10-1174 to 10-1175, 10-1190 to 10-1191, 10-1201 to 10-1204, 10-1209 to 10-1213, 10-1217 to 10-1219, 10-1223 to 10-1224, 10-1232 to 10-1233, 10-1239, 10-1243 to 10-1246, 10-1249 to 10-1250, 10-1265 to 10-1266, 10-1276 to 10-1279,

11-9 to 11-13, 11-17 to 11-19, 11-23 to 11-24, 11-32 to 11-33, 11-39, 11-51, 11-60 to 11-62, 11-69 to 11-72, 11-79 to 11-82, 11-85 to 11-86, 11-101 to 11-102, 11-112 to 11-115, 11-128 to 11-132, 11-136 to 11-138, 11-142 to 11-143, 11-151 to 11-152, 11-158, 11-170, 11-179 to 11-181, 11-188 to 11-191, 11-198 to 11-201, 11-204 to 11-205, 11-220 to 11-221, 11-231 to 11-234, 11-247 to 11-251, 11-255 to 11-257, 11-261 to 11-262, 11-270 to 11-271, 11-277, 11-289, 11-298 to 11-300, 11-307 to 11-310, 11-317 to 11-320, 11-323 to 11-324, 11-339 to 11-340, 11-350 to 11-353, 11-366 to 11-370, 11-374 to 11-376, 11-380 to 11-381, 11-389 to 11-390, 11-396, 11-408, 11-417 to 11-419, 11-426 to 11-429, 11-436 to 11-439, 11-442 to 11-443, 11-458 to 11-459, 11-469 to 11-472, 11-485 to 11-489, 11-493 to 11-495, 11-499 to 11-500, 11-508 to 11-509, 11-515, 11-527, 11-536 to 11-538, 11-545 to 11-548, 11-555 to 11-558, 11-561 to 11-562, 11-577 to 11-578, 11-588 to 11-591, 11-604 to 11-608, 11-612 to 11-614, 11-618 to 11-619, 11-627 to 11-628, 11-634, 11-646, 11-655 to 11-657, 11-664 to 11-667, 11-674 to 11-677, 11-680 to 11-681, 11-696 to 11-697, 11-707 to 11-710, 11-723 to 11-727, 11-731 to 11-733, 11-737 to 11-738, 11-746 to 11-747, 11-753, 11-765, 11-774 to 11-776, 11-783 to 11-786, 11-793 to 11-796, 11-799 to 11-800, 11-815 to 11-816, 11-826 to 11-829, 11-834 to 11-838, 11-842 to 11-844, 11-848 to 11-849, 11-857 to 11-858, 11-864, 11-868 to 11-871, 11-874 to 11-875, 11-890 to 11-891, 11-901 to 11-904, 11-909 to 11-913, 11-917 to 11-919, 11-923 to 11-924, 11-932 to 11-933, 11-939, 11-943 to 11-946, 11-949 to 11-950, 11-965 to 11-966, 11-976 to 11-979, 11-984 to 11-988, 11-992 to 11-994, 11-998 to 11-999, 11-1007 to 11-1008, 11-1014, 11-1018 to 11-1021, 11-1024 to 11-1025, 11-1040 to 11-1041, 11-1051 to 11-1054, 11-1059 to 11-1063, 11-1067 to 11-1069, 11-1073 to 11-1074, 11-1082 to 11-1083, 11-1089, 11-1093 to 11-1096, 11-1099 to 11-1100, 11-1115 to 11-1116, 11-1126 to 11-1129, 11-1134 to 11-1138, 11-1142 to 11-1144, 11-1148 to 11-1149, 11-1157 to 11-1158, 11-1164, 11-1168 to 11-1171, 11-1174 to 11-1175, 11-1190 to 11-1191, 11-1201 to 11-1204, 11-1209 to 11-1213, 11-1217 to 11-1219, 11-1223 to 11-1224, 11-1232 to 11-1233, 11-1239, 11-1243 to 11-1246, 11-1249 to 11-1250, 11-1265 to 11-1266, and 11-1276 to 11-1279, and

further more preferable compounds are Example Compound Nos. 1-11 to 1-13; 1-19, 1-32, 1-60, 1-69, 1-79, 1-130 to 1-132, 1-138, 1-151, 1-179, 1-188, 1-198, 1-249 to 1-251, 1-257, 1-270, 1-298, 1-307, 1-317, 1-368 to 1-370, 1-376, 1-389, 1-417, 1-426, 1-436, 1-487 to 1-489, 1-495, 1-508, 1-536, 1-545, 1-555, 1-606 to 1-608, 1-614, 1-627, 1-655, 1-664, 1-674, 1-725 to 1-727, 1-733, 1-746, 1-774, 1-783, 1-793, 1-836 to 1-838, 1-844, 1-857, 1-868, 1-911 to 1-913, 1-919, 1-932, 1-943, 1-986 to 1-988, 1-994, 1-1007, 1-1018, 1-1061 to 1-1063, 1-1069, 1-1082, 1-1093, 1-1136 to 1-1138, 1-1144, 1-1157, 1-1168, 1-1211 to 1-1213, 1-1219, 1-1232, 1-1243, 1-1341, 1-1342, 1-1346, 1-1347,

7-11 to 7-13, 7-19, 7-32, 7-60, 7-69, 7-79, 7-130 to 7-132, 7-138, 7-151, 7-179, 7-188, 7-198, 7-249 to 7-251, 7-257, 7-270, 7-298, 7-307, 7-317, 7-368 to 7-370, 7-376, 7-389, 7-417, 7-426, 7-436, 7-487 to 7-489, 7-495, 7-508, 7-536, 7-545, 7-555, 7-606 to 7-608, 7-614, 7-627, 7-655, 7-664, 7-674, 7-725 to 7-727, 7-733, 7-746, 7-774, 7-783, 7-793, 7-836 to 7-838, 7-844, 7-857, 7-868, 7-911 to 7-913, 7-919, 7-932, 7-943, 7-986 to 7-988, 7-994, 7-1007, 7-1018, 7-1061 to 7-1063, 7-1069, 7-1082, 7-1093, 7-1136 to 7-1138, 7-1144, 7-1157, 7-1168, 7-1211 to 7-1213, 7-1219, 7-1232, 7-1243,

10-11 to 10-13, 10-19, 10-32, 10-60, 10-69, 10-79, 10-130 to 10-132, 10-138, 10-151, 10-179, 10-188, 10-198, 10-249 to 10-251, 10-257, 10-270, 10-298, 10-307, 10-317, 10-368 to 10-370, 10-376, 10-389, 10-417, 10-426, 10-436, 10-487 to 10-489, 10-495, 10-50 8, 10-536, 10-545, 10-555, 10-606 to 10-608, 10-614, 10-627, 10-655, 10-664, 10-674, 10-725 to 10-727, 10-733, 10-746, 10-774, 10-783, 10-793, 10-836 to 10-838, 10-844, 10-857, 10-868, 10-911 to 10-913, 10-919, 10-932, 10-943, 10-986 to 10-988, 10-994, 10-1007, 10-1018, 10-1061 to 10-1063, 10-1069, 10-1082, 10-1093, 10-1136 to 10-1138, 10-1144, 10-1157, 10-1168, 10-1211 to 10-1213, 10-1219, 10-1232, 10-1243,

11-11 to 11-13, 11-19, 11-32, 11-60, 11-69, 11-79, 11-130 to 11-132, 11-138, 11-151, 11-179, 11-188, 11-198, 11-249 to 11-251, 11-257, 11-270, 11-298, 11-307, 11-317, 11-368 to 11-370, 11-376, 11-389, 11-417, 11-426, 11-436, 11-487

to 11-489, 11-495, 11-508, 11-536, 11-545, 11-555, 11-606 to 11-608, 11-614, 11-627, 11-655, 11-664, 11-674, 11-725 to 11-727, 11-733, 11-746, 11-774, 11-783, 11-793, 11-836 to 11-838, 11-844, 11-857, 11-868, 11-911 to 11-913, 11-919, 11-932, 11-943, 11-986 to 11-988, 11-994, 11-1007, 11-1018, 11-1061 to 11-1063, 11-1069, 11-1082, 11-1093, 11-1136 to 11-1138, 11-1144, 11-1157, 11-1168, 11-1211 to 11-1213, 11-1219, 11-1232, and 11-1243.

The exemplification compound numbers of particularly prefer red compounds include

Exemplification compound number 1-366:
4-[1-(4-Carbamoylphenethyl)-1,2,3,6-tetrahydropyridin-4-yl]-2-(4-fluorophenyl)-3-(pyridin-4-yl)-1H-pyrrole,
Exemplification compound number 1-367:
2-(4-Fluorophenyl)-4-[1-[4-(N-methylcarbamoyl)phenethyl]-1,2,3,6-tetrahydropyridin-4-yl]-3-(pyridin-4-yl)-1H-pyrrole,
Exemplification compound number 1-368:
4-[1-[4-(N-Ethylcarbamoyl)phenethyl]-1,2,3,6-tetrahydropyridin-4-yl]-2-(4-fluorophenyl)-3-(pyridin-4-yl)-1H-pyrrole,
Exemplification compound number 1-369:
2-(4-Fluorophenyl)-4-[1-[4-(N-propylcarbamoyl)phenethyl]-1,2,3,6-tetrahydropyridin-4-yl]-3-(pyridin-4-yl)-1H-pyrrole,
Exemplification compound number 1-370:
2-(4-Fluorophenyl)-4-[1-[4-(N-isopropylcarbamoyl)phenethyl]-1,2,3,6-tetrahydropyridin-4-yl]-3-(pyridin-4-yl)-1H-pyrrole,
Exemplification compound number 1-374:
4-[1-[4-(N-Cyclopropylcarbamoyl)phenethyl]-1,2,3,6-tetrahydropyridin-4-yl]-2-(4-fluorophenyl)-3-(pyridin-4-yl)-1H-pyrrole,
Exemplification compound number 1-376:
4-[1-[4-(N-Cyclopentylcarbamoyl)phenethyl]-1,2,3,6-tetrahydropyridin-4-yl]-2-(4-fluorophenyl)-3-(pyridin-4-yl)-1H-pyrrole,
Exemplification compound number 1-389:
4-[1-[4-[N-(2-Fluoroethyl)carbamoyl]phenethyl]-1,2,3,6-tetrahydropyridin-4-yl]-2-(4-fluorophenyl)-3-(pyridin-4-yl)-1H-pyrrole,
Exemplification compound number 1-396:
4-[1-[4-[N-(Ethoxycarbonylmethyl)carbamoyl]phenethyl]-1,2,3,6-tetrahydropyridin-4-yl]-2-(4-fluorophenyl)-3-(pyridin-4-yl)-1H-pyrrole
Exemplification compound number 1-417:
2-(4-Fluorophenyl)-4-[1-(4-methanesulfinylphenethyl]-1,2,3,6-tetrahydropyridin-4-yl]-3-(pyridin-4-yl)-1H-pyrrole,
Exemplification compound number 1-426:
2-(4-Fluorophenyl)-4-[1-(4-methanesulfonylphenethyl]-1,2,3,6-tetrahydropyridin-4-yl]-3-(pyridin-4-yl)-1H-pyrrole,
Exemplification compound number 1-436:
2-(4-Fluorophenyl)-4-[1-[4-(N-methylsulfamoyl)phenethyl]-1,2,3,6-tetrahydropyridin-4-yl]-3-(pyridin-4-yl)-1H-pyrrole,
Exemplification compound number 1-437:
4-[1-[4-(N-Ethylsulfamoyl)phenethyl]-1,2,3,6-tetrahydropyridin-4-yl]-2-(4-fluorophenyl)-3-(pyridin-4-yl)-1H-pyrrole,
Exemplification compound number 1-438:
2-(4-Fluorophenyl)-4-[1-[4-(N-propylsulfamoyl)phenethyl]-1,2,3,6-tetrahydropyridin-4-yl]-3-(pyridin-4-yl)-1H-pyrrole,
Exemplification compound number 1-439:
2-(4-Fluorophenyl)-4-[1-[4-(N-isopropylsulfamoyl)phenethyl]-1,2,3,6-tetrahydropyridin-4-yl]-3-(pyridin-4-yl)-1H-pyrrole,
Exemplification compound number 1-442:
4-[1-[4-(N,N-Dimethylsulfamoyl)phenethyl]-1,2,3,6-tetrahydropyridin-4-yl]-2-(4-fluorophenyl)-3-(pyridin-4-yl)-1H-pyrrole,
Exemplification compound number 1-443:
4-[1-[4-(N-Cyclopropylsulfamoyl)phenethyl]-1,2,3,6-tetrahydropyridin-4-yl]-2-(4-fluorophenyl)-3-(pyridin-4-yl)-1H-pyrrole,
Exemplification compound number 1-458:
4-[1-[4-[N-(2-Fluoroethyl)sulfamoyl]phenethyl]-1,2,3,6-tetrahydropyridin-4-yl]-2-(4-fluorophenyl)-3-(pyridin-4-yl)-1H-pyrrole,
Exemplification compound number 1-459:
2-(4-Fluorophenyl)-4-[1-[4-[N-(2-methoxyethyl)sulfamoyl]phenethyl]-1,2,3,6-tetrahydropyridin-4-yl]-3-(pyridin-4-yl)-1H-pyrrole,

Exemplification compound number 1-470:
4-[1-[4-(N-Ethoxysulfamoyl)phenethyl]-1,2,3,6-tetrahydropyridin-4-yl]-2-(4-fluorophenyl)-3-(pyridin-4-yl)-1H-pyrrole,
Exemplification compound number 1-472:
4-[1-[4-(N-Allyloxysulfamoyl)phenethyl]-1,2,3,6-tetrahydropyridin-4-yl]-2-(4-fluorophenyl)-3-(pyridin-4-yl)-1H-pyrrole,
Exemplification compound number 1-527:
4-[1-(4-Acetylphenethyl)-1,2,3,6-tetrahydropyridin-4-yl]-2-(3-chloro-4-fluorophenyl)-3-(pyridin-4-yl)-1H-pyrrole,
Exemplification compound number 1-536:
2-(3-Chloro-4-fluorophenyl)-4-[1-(4-methanesulfinylphenethyl)-1,2,3,6-tetrahydropyridin-4-yl]-3-(pyridin-4-yl)-1H-pyrrole,
Exemplification compound number 1-545:
2-(3-Chloro-4-fluorophenyl)-4-[1-(4-methanesulfonylphenethyl)-1,2,3,6-tetrahydropyridin-4-yl]-3-(pyridin-4-yl)-1H-pyrrole,
Exemplification compound number 1-555:
2-(3-Chloro-4-fluorophenyl)-4-[1-[4-(N-methylsulfamoyl)phenethyl]-1,2,3,6-tetrahydropyridin-4-yl]-3-(pyridin-4-yl)-1H-pyrrole,
Exemplification compound number 1-1341:
2-(4-Fluorophenyl)-4-[1-[4-(2-methoxyethyl)sulfonylphenethyl]-1,2,3,6-tetrahydropyridin-4-yl]-3-(pyridin-4-yl)-1H-pyrrole,
Exemplification compound number 1-1342:
2-(4-Fluorophenyl)-4-[1-[4-(2-hydroxyethyl)sulfonylphenethyl]-1,2,3,6-tetrahydropyridin-4-yl]-3-(pyridin-4-yl)-1H-pyrrole,
Exemplification compound number 1-1346:
2-(3-Chloro-4-fluorophenyl)-4-[1-[4-(2-methoxyethyl)sulfonylphenethyl]-1,2,3,6-tetrahydropyridin-4-yl]-3-(pyridin-4-yl)-1H-pyrrole,
Exemplification compound number 1-1347:
2-(3-Chloro-4-fluorophenyl)-4-[1-[4-(2-hydroxyethyl)sulfonylphenethyl]-1,2,3,6-tetrahydropyridin-4-yl]-3-(pyridin-4-yl)-1H-pyrrole,
Exemplification compound number 7-366:
4-[(2S,8aS)-2-(4-Carbamoylphenyl)-1,2,3,5,6,8a-hexahydroindolidin-7-yl]-2-(4-fluorophenyl)-3-(pyridin-4-yl)-1H-pyrrole,
Exemplification compound number 7-367:
2-(4-Fluorophenyl)-4-[(2S,8aS)-2-[4-(N-methylcarbamoyl)phenyl]-1,2,3,5,6,8a-hexahydroindolidin-7-yl]-3-(pyridin-4-yl)-1H-pyrrole,
Exemplification compound number 7-368:
4-[(2S,8aS)-2-[4-(N-Ethylcarbamoyl)phenyl]-1,2,3,5,6,8a-hexahydroindolidin-7-yl]-2-(4-fluorophenyl)-3-(pyridin-4-yl)-1H-pyrrole,
Exemplification compound number 7-369:
2-(4-Fluorophenyl)-4-[(2S,8aS)-2-[4-(N-propylcarbamoyl)phenyl]-1,2,3,5,6,8a-hexahydroindolidin-7-yl]-3-(pyridin-4-yl)-1H-pyrrole,
Exemplification compound number 7-370:
2-(4-Fluorophenyl)-4-[(2S,8aS)-2-[4-(N-isopropylcarbamoyl)phenyl]-1,2,3,5,6,8a-hexahydroindolidin-7-yl]-3-(pyridin-4-yl)-1H-pyrrole,
Exemplification compound number 7-372:
4-[(2S,8aS)-2-[4-(N-Benzylcarbamoyl)phenyl]-1,2,3,5,6,8a-hexahydroindolidin-7-yl]-2-(4-fluorophenyl)-3-(pyridin-4-yl)-1H-pyrrole,
Exemplification compound number 7-374:
4-[(2S,8aS)-2-[4-(N-Cyclopropylcarbamoyl)phenyl]-1,2,3,5,6,8a-hexahydroindolidin-7-yl]-2-(4-fluorophenyl)-3-(pyridin-4-yl)-1H-pyrrole,
Exemplification compound number 7-389:
4-[(2S,8aS)-2-[4-[N-(2-Fluoroethyl)carbamoyl]phenyl]-1,2,3,5,6,8a-hexahydroindolidin-7-yl]-2-(4-fluorophenyl)-3-(pyridin-4-yl)-1H-pyrrole,
Exemplification compound number 7-390:
2-(4-Fluorophenyl)-4-[(2S,8aS)-2-[4-[N-(2-methoxyethyl)carbamoyl]phenyl]-1,2,3,5,6,8a-hexahydroindolidin-7-yl]-3-(pyridin-4-yl)-1H-pyrrole,
Exemplification compound number 7-486:
2-(3-Chloro-4-fluorophenyl)-4-[(2S,8aS)-2-[4-(N-methylcarbamoyl)phenyl]-1,2,3,5,6,8a-hexahydroindolidin-7-yl]-3-(pyridin-4-yl)-1H-pyrrole,
and
Exemplification compound number 10-366:
3-[1-(4-Carbamoylphenethyl)-1,2,3,6-tetrahydropyridin-4-yl]-5-(4-fluorophenyl)-4-(pyridin-4-yl)-1H-pyrazole.

[Mode for carrying out the invention]

The compounds having the general formula (I) can easily be prepared according to methods A to H described hereinafter.

Method A is a process for the preparation of compounds wherein $R^3$ is bonded to a carbon atom on Ring A, among the compounds having the general formula (I).

⟨Method A⟩

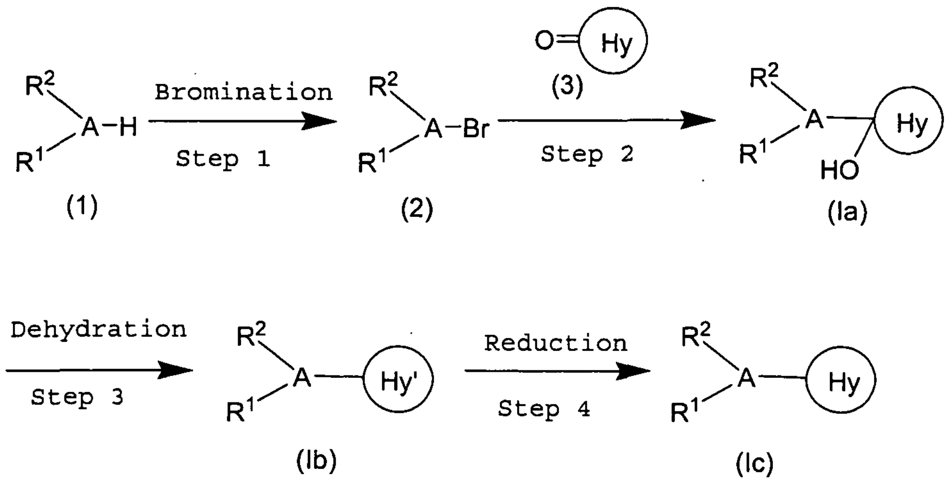


In the above reaction scheme, A, $R^1$ and $R^2$ have the same meanings as those indicated hereinbefore;
cyclic group Hy represents a group wherein the bond containing a dotted line moiety shown in general formulae (IIa) or (IIb) is a single bond; and
cyclic group Hy' represents a group wherein the bond containing a dotted line moiety shown in general formulae (IIa) or (IIb) is a double bond.

Step 1 is a process for the preparation of a bromocyclic compound (2) by brominating a cyclic compound (1) with a brominating agent (for example, N-bromosuccinimide or the like), and Step 2 is a process for the preparation of a compound (Ia) of the present invention by lithiating bromocyclic compound (2), followed by reacting the lithiated product with a heterocyclyl ketone (3). These Steps 1 and 2 can be carried out according to the procedure described in detail by Brian L. Bray et al., J. Org. Chem., vol. 55, 6317-6318 (1990).

Further, the compounds (Ia) can be also prepared by directly lithiating the cyclic compound (1) using the same procedure as that indicated by L. Revesz et al., Bioorg. Med. Chem. Lett., vol. 10, 1261-1264 (2000), followed by reacting the lithiated product obtained with a heterocyclyl ketone (3).

Step 3 is a process for the preparation of a compound (Ib) of the present invention by subjecting compound (Ia) of the present invention to a dehydration reaction. This dehydration reaction is generally carried out in the presence of an acid catalyst such sulfuric acid or the like, a solid catalyst such as alumina or the like, or a halogenation agent such as thionyl chloride or the like [these reactions are described in detail, for example, by G. H. Coleman and H. F. Johnstone, Org. Synth., I, 183 (1941), R. L. Sawyer and D. W. Andrus, Org. Synth., III, 276 (1955) and J. S. Lomas et al., Tetrahedron Lett., 599 (1971)]. Additionally, the dehydration reaction of this step can be attained by the reaction using a trialkylsilane such as triethylsilane, tripropylsilane, tributylsilane or the like, and trifluoroacetic acid [for example, Francis A. Carey and Henry S. Tremper, J. Am. Chem. Soc., vol. 91, 2967-2972 (1969)].

Step 4 is a process for the preparation of a compound (Ic) of the present invention by reducing a double bond of compound (Ib) of the present invention, and can be carried out according to the procedure described by S.M. Kerwin et al., J. Org. Chem., vol. 52, 1686 (1987) and T. Hudlicky et al., J. Org. Chem., vol. 52, 4641 (1987).

In the above Method A, when a compound wherein A is a pyrrole ring which may optionally be substituted with two groups selected from Substituent group δ is synthesized, it is desirable to synthesize such compound by first

subjecting to Step 1 a compound in which the nitrogen atom at the 1-position of the pyrrole ring is protected beforehand, and then introducing such groups selected from Substituent group δ onto such nitrogen atom, if necessary, after conducting the deprotection reaction in Step 2 or Step 3.

The protection of the nitrogen atom at the 1-position of pyrrole ring can be carried out, for example, by adding successively butyllithium or sodium hydride, and triisopropylsilyl triflate or (t-butyl)diphenylsilyl chloride to a solution of the pyrrole compound corresponding to a compound (1) in tetrahydrofuran (300 ml) under ice-cooling (for example, below 0°C) under stirring, followed by stirring the resulting mixture at room temperature. The deprotection reaction is usually carried out in the presence of a base in a solvent, and can be attained, for example, by adding an aqueous sodium hydroxide solution, saturated aqueous sodium hydrogencarbonate solution or tetrabutylammonium fluoride to the protected compound.

The introduction of the group selected from Substituent group δ into the 1-postion of the pyrrole ring can be attained, for example, by conducting a similar reaction to that in <Method B> described below.

<Method B>

Method B is a process for the preparation of compounds wherein cyclic ring A is a pyrrole, pyrazole or imizadole ring, and $R^3$ is bonded to the nitrogen atom of the ring A.

〈Method B〉

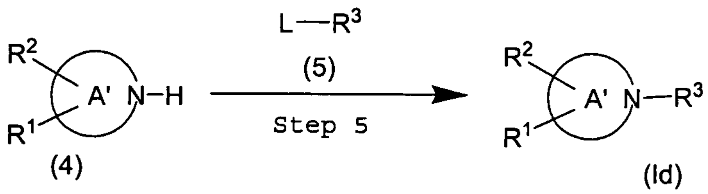


(4) (Id)

In the above reaction scheme, $R^1$, $R^2$ and $R^3$ have the same meanings as those indicated hereinbefore; cyclic group A' represents a pyrrole ring which may optionally be substituted with two groups selected from Substituent group δ, a pyrazole ring which may optionally be substituted with one group selected from Substituent group δ, or an imidazole ring which may optionally be substituted with one group selected from Substituent group δ, which are described in the definition of A, and L represents a leaving group.

The "leaving group" in the definition of L represents generally a group which leaves as a nucleophilic residue, and can be, for example, a halogen atom such as a fluorine atom, a chlorine atom, a bromine atom or an iodine atom; a lower alkanesulfonyloxy group such as a methanesulfonyloxy or ethenesulfonyloxy group; a halogeno lower alkanesulfonyloxy group such as a trifluoromethanesulfonyloxy or pentafluoroethanesulfonyloxy group; or an arylsulfonyloxy group such as a benzenesulfonyloxy, p-toluenesulfonyloxy or p-nitrobenzenesulfonyloxy group, and is preferably a halogen atom, and particularly preferably a bromine atom.

Step 5 is a process for the preparation of a compound (Id) of the present invention by reacting a compound (4) with a heterocyclyl compound (5), and this reaction is carried out in the presence or absence of a base in a solvent.

The solvent employed can be, for example, an alcohol such as methanol, ethanol, propanol or isopropanol; an ether such as diethyl ether, diisopropyl ether, tetrahydrofuran or dioxane; an aprotic polar solvent such as dimethylformamide, dimethylacetamide or dimethyl sulfoxide; a nitrile such as acetonitrile; an ester such as methyl acetate or ethyl acetate; an aromatic hydrocarbon such as benzene, toluene or xylene; or an aliphatic hydrocarbon such as pentane, hexane or heptane, and is preferably an ether, alcohol or aprotic solvent, and more preferably tetrahydrofuran, methanol, ethanol or dimethylformamide.

The base employed can be, for example, an alkali metal alkoxide such as sodium methoxide, sodium ethoxide or potassium tert-butoxide; an alkali metal hydride such as sodium hydride or lithium hydride; or alkali metal hydroxide such as sodium hydroxide or potassium hydroxide; an alkali metal carbonate such as sodium carbonate, potassium carbonate, sodium hydrogencarbonate or potassium hydrogencarbonate; or an amine such as triethylamine, tributylamine, pyridine, picoline or 1,8-diazabicyclo[5.4.0]undec-7-ene; and is preferably an alkali metal carbonate or an amine, and more preferably triethylamine, pyridine, 1,8-diazabicyclo[5.4.0]undec-7-ene, sodium carbonate, potassium carbonate, sodium hydrogencarbonate or potassium hydrogencarbonate.

The reaction temperature is generally between -20°C and 150°C, and preferably between 0°C and 100°C. The reaction time is generally from 10 minutes to 48 hours, and preferably from 30 minutes to 12 hours.

When Ring A is a pyrazole or imidazole group, and its nitrogen atom is substituted with a substituent (a group other than a hydrogen atom in the definition of $R^1$, $R^2$, $R^4$ or $R^{4'}$), the desired substituent can be introduced by conducting a reaction in a similar manner to that in the above-mentioned <Method B>.

<Method C>

Method C is an alternative method for the preparation of a compound wherein cyclic group A' in general formula (Id) described above is an imidazole group.

(Method C)

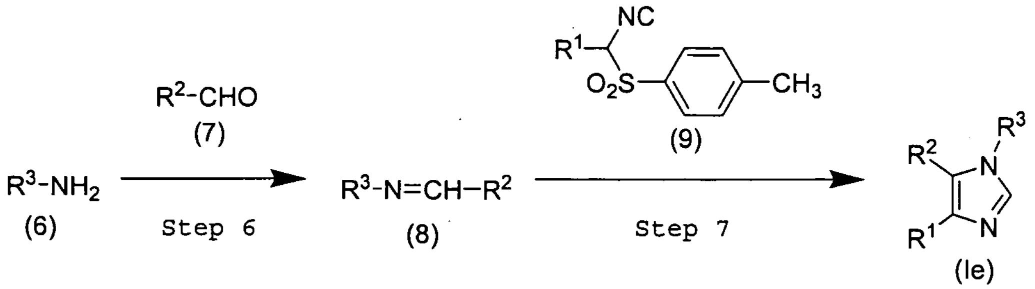


(6) (8) (Ie)

In the above reaction scheme, $R^1$, $R^2$ and $R^3$ have the same meanings as those indicated hereinbefore.

Step 6 is a process for the preparation of an imine compound (8) by a dehydration/condensation reaction between an amino compound (6) and an aldehyde compound (7), and Step 7 is a process for the preparation of a compound (Ie) of the present invention by reacting the imine compound (8) with an isocyanide compound (9) .

Steps 6 and 7 are carried out, for example, according to the procedures described in detail in WO 97/23479, WO 97/25046, WO 97/25047, WO 97/25048, WO 95/02591, J. L. Adams et al., Bioorg. Med. Chem. Lett., vol. 8, 3111-3116 (1998).

Among the compounds (I) of the present invention, a compound (If) wherein $R^2$ is a heteroaryl group having at least one nitrogen atom substituted with a group $NR^dR^e$ can also be prepared, by <Method D> described hereinafter.

(Method D)

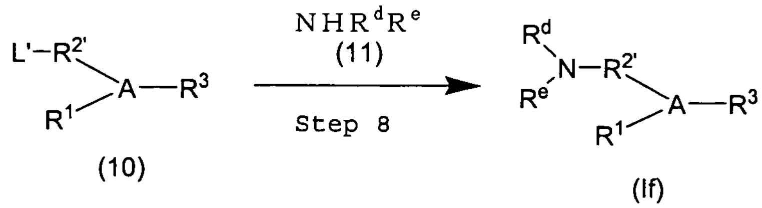


(10) (If)

In the above reaction scheme, A, $R^1$, $R^3$, $R^d$ and $R^e$ have the same meanings as those indicated hereinbefore; L' represents a leaving group; a -$R^{2'}$-L' group represents a "heteroaryl group having at least one nitrogen atom" having a leaving group (L' group) (for example, a 2-methanesulfonylpyrimidin-4-yl or 2-methanesulfonylpyridin-4-yl group); and said "heteroaryl group having at least one nitrogen atom" represents the same group as the "heteroaryl group having at least one nitrogen atom" in the definition of $R^2$.

The leaving group in the definition of L' represents a similar group to the leaving group in the definition of L; a lower alkylsulfonyl group such as a methansulfonyl, ethanesulfonyl, propanesulfonyl or butanesulfonyl group; or an arylsulfonyl group such as a benzenesulfonyl, p-toluenesulfonyl or p-nitrobenzenesulfonyl group; and is preferably a lower alkylsulfonyl group and more preferably a methanesulfonyl group.

Step 8 is a.process for the preparation of a compound (If) of the present invention by converting the leaving group into a group having formula: $NR^dR^e$ by reacting a compound (10) with an amine compound (11). This reaction is carried out in a similar manner to that described in Step 5.

Among the compounds (I) of the present invention, a compound (Ig) wherein $R^3$ is a group having general

formula (IIa) can also be prepared, by <Method E> described hereinafter.

(Method E)

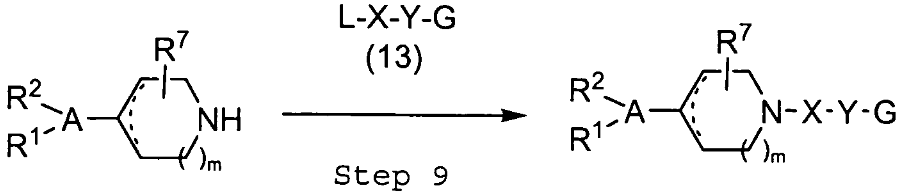


(12) (Ig)

In the above reaction scheme, A, L, m, $R^1$, $R^2$, $R^7$, X and Y have the same meanings as those indicated hereinbefore, and G represents a group having formula: $Z(R^5)(R^6)_n$ (wherein, $R^5$ and $R^6$, n and Z have the same meanings as those indicated hereinbefore).

Step 9 is a process for the preparation of a compound (Ig) of the present invention by reacting a compound (12) with a compound (13), and is carried out in a similar manner to that described in Step 5 of <Method B>.

The compound (Ig) of the present invention can also be prepared by <Method F>.

(Method F)

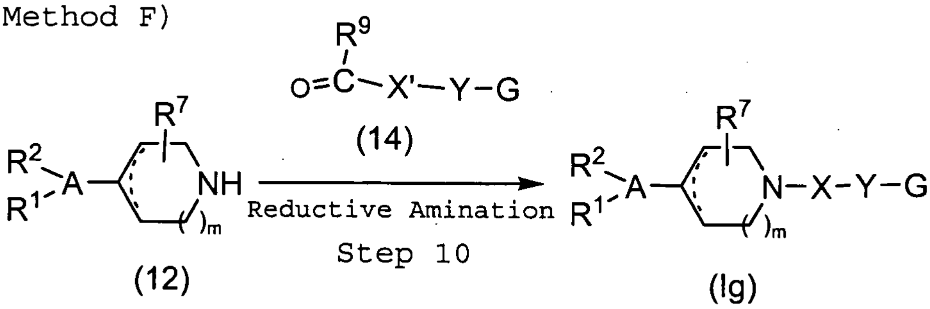


(12) (Ig)

In the above reaction scheme, A, G, m, $R^1$ ,$R^2$, $R^7$, X and Y have the same meanings as those indicated hereinbefore; $R^9$ represents a hydrogen atom or an alkyl group having from 1 to 3 carbon atoms; and X' represents a single bond, or a straight or branched alkylene group having from 1 to 4 carbon atoms.

Step 10 is a process for the preparation of a compound (Ig) of the present invention by reacting a compound (12) with an oxo compound (14) . This reaction is the well known reductive amination reaction, and is carried out according to the procedure described in detail by C. F. Lane, Synthesis, 1975, 135 (1975).

Among the compounds (I) of the present invention, a series of compounds from a compound (Ih) to a compound (In) wherein $R^5$ is a carboxyl group, a lower alkoxycarbonyl group, an aralkyloxycarbonyl group, an aryloxycarbonyl group or a group having formula: $CONR^aR^b$ can also be prepared, according to <Method G> described hereinafter.

(Method G)

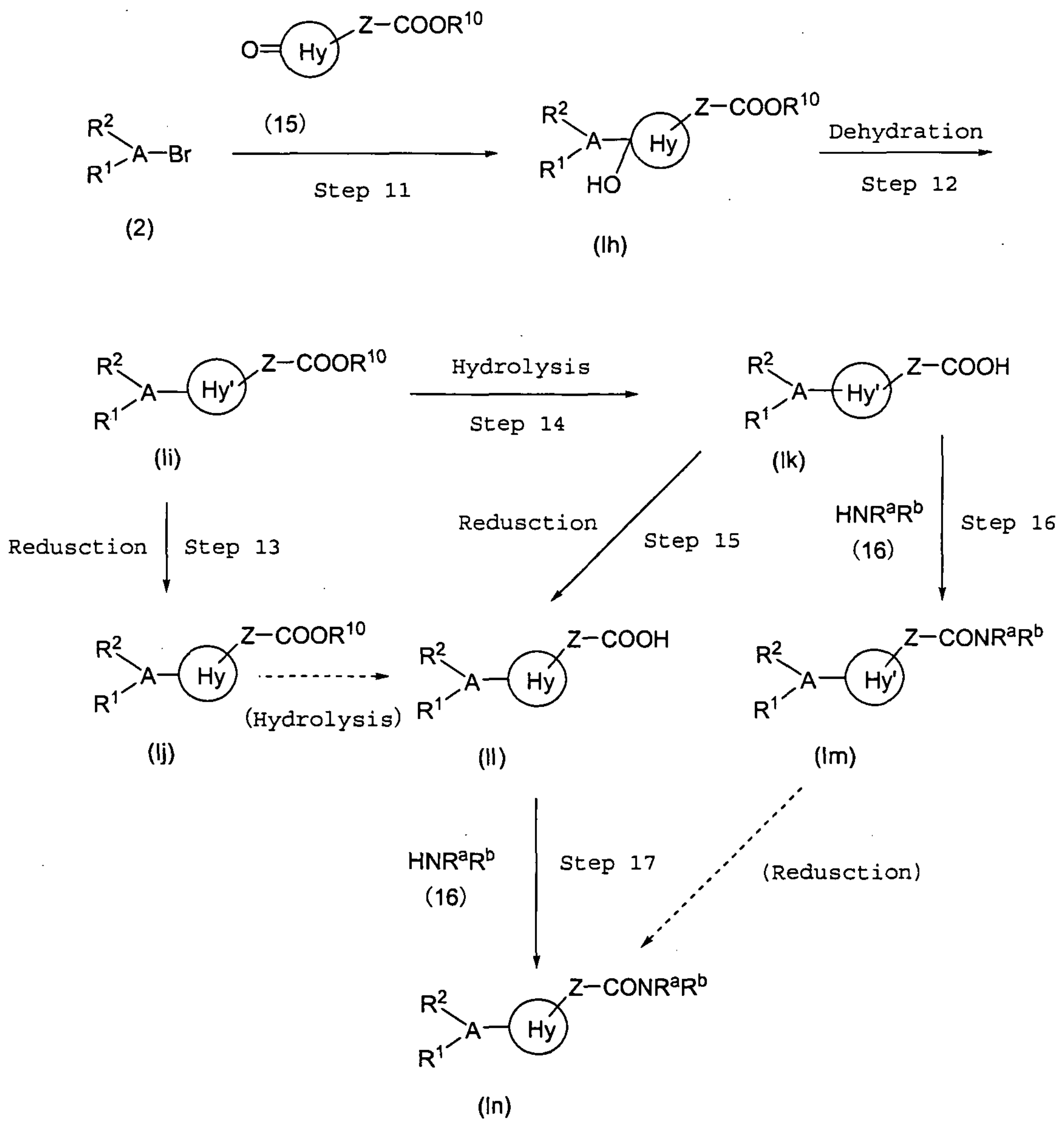


In the above reaction scheme, A, Hy, Hy', $R^1$, $R^2$, $R^a$, $R^b$ and Z have the same meanings as those indicated hereinbefore, and $R^{10}$ represents a lower alkyl group, an aralkyl group or an aryl group.

Step 11 is a process for the preparation of a compound (Ih) of the present invention by lithiating a bromocyclic compound (2), followed by reacting the lithiated product with a heterocyclyl ketone (15), and is carried out in a similar manner to that described in Step 2 of <Method A>.

Step 12 is a process for the preparation of a compound (Ii) of the present invention by subjecting compound (Ih) to a dehydration reaction, and is carried out in a similar manner to that described in Step 3 of <Method A>.

Step 13 is a process for the preparation of a compound (Ij) of the present invention by reducing a double bond of compound (Ii) of the present invention, and is carried out in a similar manner to that described in Step 4 of <Method A>.

Step 14 is a process for the preparation of a compound (Ik) of the present invention by hydrolyzing an ester moiety of compound (Ii) of the present invention. The hydrolysis reaction is carried out using an acid or a base under reaction conditions commonly used in organic synthesis.

Step 15 is a process for the preparation of a compound (Il) of the present invention by reducing a double bond of compound (Ik) of the present invention, and carried out in a similar manner to that described in Step 4 of <Method A>.

Steps 16 and 17 are processes for the preparation of a compound (Im or In: a carboxyl amide compound) by reacting a compound (Ik or Il: a carboxylic acid compound) with an amine compound (16). This amide-formation reaction

is carried out in the presence of a condensing agent in a solvent in the presence or absence of a base.

The solvent employed can be, for example, an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; an ester such as methyl acetate or ethyl acetate; water; or a mixture of solvents mentioned above, and is preferably a halogenated hydrocarbon, an ether or an ester, and more preferably dichloromethane, tetrahydrofuran or ethyl acetate.

The condensing reagent employed can be, for example, dicyclohexylcarbodiimide, 1-ethyl-3-(3-dimethylaminopropyl)carbodiimide or N,N'-carbonyldiimidazole.

The base employed can be, for example, an alkali metal alkoxide such as sodium methoxide, sodium ethoxide or potassium tert-butoxide; an alkali metal hydride such as sodium hydride or lithium hydride; an alkali metal hydroxide such as sodium hydroxide or potassium hydroxide; an alkali metal carbonate such as sodium carbonate or potassium carbonate; or an amine such as triethylamine, tributylamine, pyridine, picoline or 1,8-diazabicyclo[5.4.0]undec-7-ene, and is preferably an amine, and more preferably triethylamine, pyridine or 1,8-diazabicyclo[5.4.0]undec-7-ene.

The reaction temperature is generally between -20°C and 150°C, and preferably between 0°C and 100°C. The reaction time is generally from 10 minutes to 48 hours, and preferably from 30 minutes to 12 hours.

Further, the reaction can be also carried out after converting compound (Ik) or compound (Il) into a corresponding active derivative, respectively.

When an active derivative is used, the "active derivative" represents an acid halide, a mixed acid anhydride, an active ester or an activated amide, and the reaction is carried out in the presence of a condensing agent in a solvent in the presence or absence of a base.

The "acid halide" can be obtained by reacting compound (Ik) or compound (Il) with a halogenating agent (for example, thionyl chloride, oxalyl chloride or the like) ; the "mixed acid anhydride" can be obtained by reacting compound (Ik) or compound (Il) with an acid halide (for example, methyl chlorocarbonate, ethyl chlorocarbonate or the like); the "active ester" can be obtained by reacting compound (Ik) or compound (Il) with a hydroxy compound (for example, N-hydroxysuccinimide, N-hydroxyphthalimide or the like) in the presence of the "condensing agent" described hereinbefore; and the "active amide" (for example, Weinreb amide) can be obtained by reacting compound (Ik) or compound (1l) with a N-lower-alkoxy-N-lower alkylhydroxylamine (for example, N-methoxy-N-methylhydroxylamine or the like) in the presence of the "condensing agent" described hereinbefore. All these reactions mentioned above are carried out under the reaction conditions commonly used in organic synthesis.

In the above reactions, the solvent, condensing agent and base described hereinbefore can be used as the solvent, condensing agent and base.

The reaction temperature is generally between -20°C and 150°C, and preferably between 0°C and 100°C. The reaction time is generally from 10 minutes to 48 hours, and preferably from 30 minutes to 12 hours.

The hydrolysis of compound (Ij) into compound (Il) can be carried out in a similar manner to that described in Step 14, while the reduction of compound (Im) to compound (In) can be carried out in a similar manner to that described in Step 13 or Step 15.

Of the compounds (I) of the present invention, both a compound (Io) and a compound (Ip), wherein $R^5$ is a group having formula: $S(O)_qR^c$ (q represents an integer 1 or 2), can also be prepared, according to <Method H> described below.

(Method H)

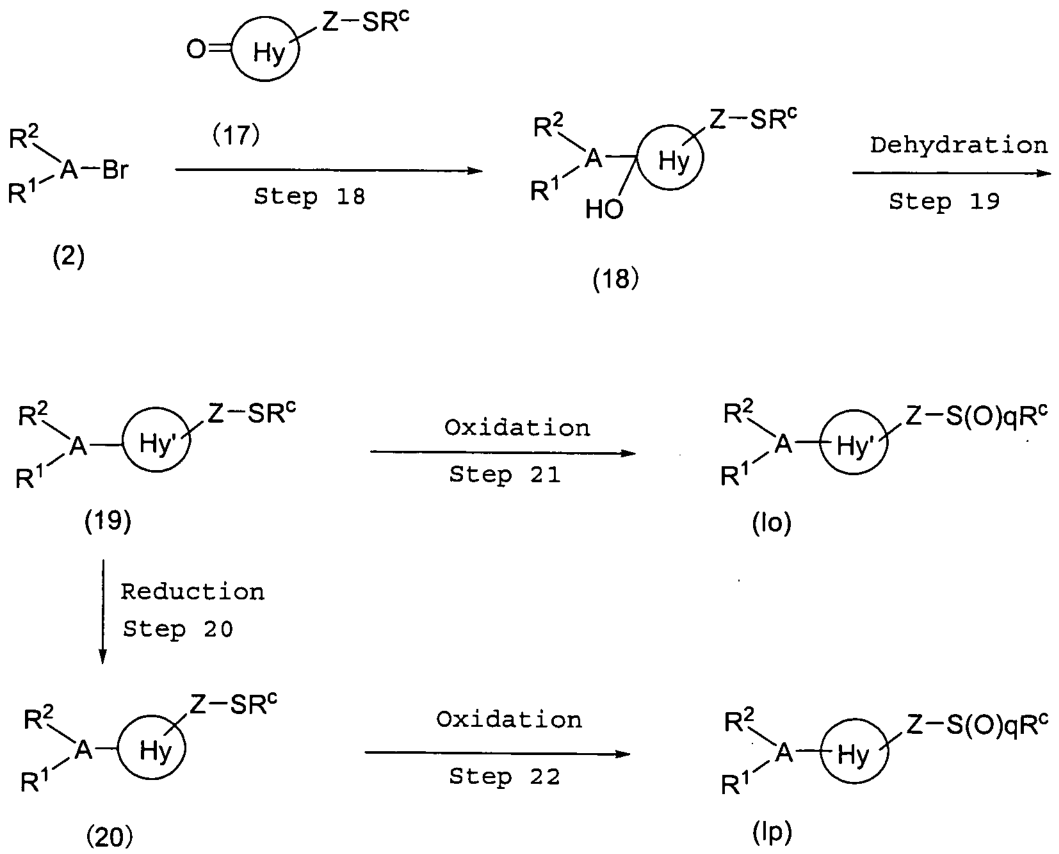


In the above reaction scheme, A, Hy, Hy' , $R^1$, $R^2$, $R^c$ and Z have the same meanings as those indicated hereinbefore, and q represents an integer 1 or 2.

Step 18 is a process for the preparation of a compound (18) by a lithiating a bromocyclic compound (2), followed by reacting the lithiated product with a heterocyclyl ketone (17), and is carried out in a similar manner to that described in Step 2 of <Method A>.

Step 19 is a process for the preparation of a compound (19) by subjecting compound (18) to a dehydration reaction, and is carried out in a similar manner to that described in Step 3 of <Method A>.

Step 20 is a process for the preparation of a compound (20) by reducing a double bond of compound (19), and is carried out in a similar manner to that described in Step 4 of <Method A>.

Step 21 and Step 22 are processes for the preparation of a compound (Io) and a compound (Ip) by oxidizing the sulfide moiety of compound (19) and compound (20), respectively.

This oxidation reaction is carried out by reacting the sulfide compound with an oxidizing agent (said oxidizing agent can be, for example, a peracid such as peracetic acid, perbenzoic acid or m-chloroperbenzoic acid; hydrogen peroxide; or an alkali metal perhalogenate such as sodium metaperchlorate, sodium metaperiodate or potassium metaperiodate, and is preferably a peracid or hydrogen peroxide, and particularly preferably m-chloroperbenzoic acid) in an inert solvent (said inert solvent can be, for example, an aliphatic hydrocarbon such as hexane, heptane or petroleum ether; an aromatic hydrocarbon such as benzene, toluene or xylene; a halogenated hydrocarbon such as dichloromethane, chloroform, carbon tetrachloride or dichloroethane; an alcohol such as methanol, ethanol, propanol or butanol; an ester such as ethyl acetate, propyl acetate, butyl acetate or ethyl propionate; a carboxylic acid such as acetic acid or propionic acid; water; or a mixture of solvents mentioned above, and is preferably a halogenated hydrocarbon or a carboxylic acid, and particularly preferably dichloromethane, chloroform, dichloroethane or acetic acid) at a temperature between -20°C and 150°C (preferably between 0°C and 100°C) for from 10 minutes to 10 hours (preferably from 30 minutes to 5 hours).

When a compound wherein q represents 1 is prepared by addition of one oxygen atom using the above oxidation reaction, the amount of the oxidizing reagent used is from 0.6 to 1.4 molar equivalents of the amount of the substrate

(preferably from 0.8 to 1.2 molar equivalents) . On the other hand, when a sulfide compound is used as the substrate, a compound wherein q represents 2 can be prepared by conducting the above oxidation reaction using the oxidizing reagent of from 1.5 to 3 molar equivalents of the amount of the substrate (preferably from 1.8 to 2.5 molar equivalents).

The compounds of (1), (3), (4), (5), (6), (7), (9), (11), (12), (13), (14), (15), (16), and (17), which are used as the starting materials in the methods from A to H, are well known compounds or can easily be prepared from known compounds according to the known procedures. Compound (10) can easily be prepared from known compounds by conducting the similar reactions to those described from method A to method H.

Compounds (1) can, for example, be prepared as follows:

compounds wherein A represents a "benzene which may optionally be substituted with group(s) selected from Substituent group δ" can be prepared in a similar manner to those described by D. J. P. Pinto et al., Bioorg. Med. Chem. Lett., vol. 6, 2907-2912 (1996), D.J.P. Pinto et al., Bioorg. Med. Chem. Lett., vol. 9, 919-924 (1999), M.B. Nortonet et al., J. Med. Chem., vol. 39, 1846-1856 (1996), WO 96/10012, WO 96/26921 and WO 96/16934, and the like,

compounds wherein A represents a "pyridine which may optionally be substituted with group(s) selected from Substituent group δ" can be prepared in a similar manner to that described by R.W. Friesen et al., Bioorg. Med. Chem. Lett., vol. 8, 2777-2782 (1998), and the like,

compounds wherein A represents a "pyridazine which may optionally be substituted with group(s) selected from Substituent group δ" or a "pyrimidine which may optionally be substituted with group(s) selected from Substituent group δ" can be prepared in a similar manner to that described in WO 00/31065, and the like,

compounds wherein A represents a "pyrrole which may optionally be substituted with group(s) selected from Substituent group δ" can be prepared in a similar manner to that described in EP1070711, and the like,

compounds wherein A represents a "furan which may optionally be substituted with group(s) selected from Substituent group δ" can be prepared in a similar manner to that described in USP 6, 048, 880, and the like,

compounds wherein A represents a "thiophene which may optionally be substituted with group(s) selected from Substituent group δ" can be prepared in a similar manner to those described by WO 94/26731, Y. Leblanc et al., Bioorg. Med. Chem. Lett., vol. 5, 2123-2128 (1995), S.R. Bertenshaw et al. , Bioorg. Med. Chem. Lett. , vol. 5, 2919-2922 (1995), D.J.P. Pinto et al., Bioorg. Med. Chem. Lett., vol. 6, 2907-2912 (1996) and WO 95/00501, and the like,

compounds wherein A represents a "pyrazole which may optionally be substituted with group(s) selected from Substituent group δ" can be prepared in a similar manner to those described in WO 00/31063, WO 99/58523, WO 00/39116 and WO 95/31451, and the like,

compounds wherein A represents an "imidazole which may optionally be substituted with group(s) selected from Substituent group δ" can be prepared in a similar manner to those described by I.K. Khanna et al., J. Med. Chem., vol. 40, 1634-1647 (1997), WO 93/14081, WO 97/23479, USP 5,716,955, WO 97/25046, WO 97/25047, WO 97/25048, WO 95/02591 and J.L. Adams et al. , Bioorg. Med. Chem. Lett., vol. 8, 3111-3116 (1998), and the like,

compounds wherein A represents an "isoxazole" can be prepared in a similar manner to that described in Japanese Patent Publication (Tokkai) 2000-86657, and the like, and

compounds wherein A represents an "isothiazole" can be prepared in a similar manner to that described in WO 95/00501, and the like.

Furthermore, compounds (3) can be, for example, prepared as follows.

Compounds (3) wherein cyclic group Hy represents a group wherein the bond shown by a dotted line moiety in general formula (IIa) described hereinbefore is a single bond can be prepared by <Method I> described below.

(Method I)

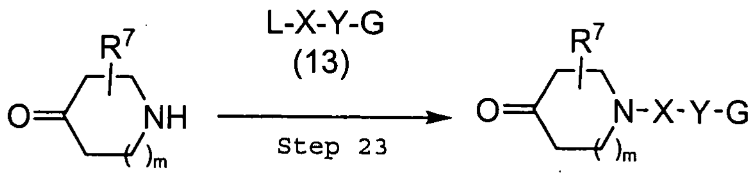


(21) (22)

In the above reaction scheme, G, L, m, $R^7$, X and Y have the same meanings as those indicated hereinbefore.

Step 23 is a process for the preparation of a compound (22) by reacting a compound (21) with a compound (13), and is carried out in a similar manner to that described in Step 5 of <Method B>.

The compound (21) used as the starting material is a known compound or can be easily prepared from known compounds by conventional methods.

On the other hand, compound (3) wherein cyclic group Hy represents a group wherein the bond shown by a dotted line moiety in general formula (IIb) described hereinbefore is a single bond can be prepared by described methods <Method J> to <Method P>.

(Method J)

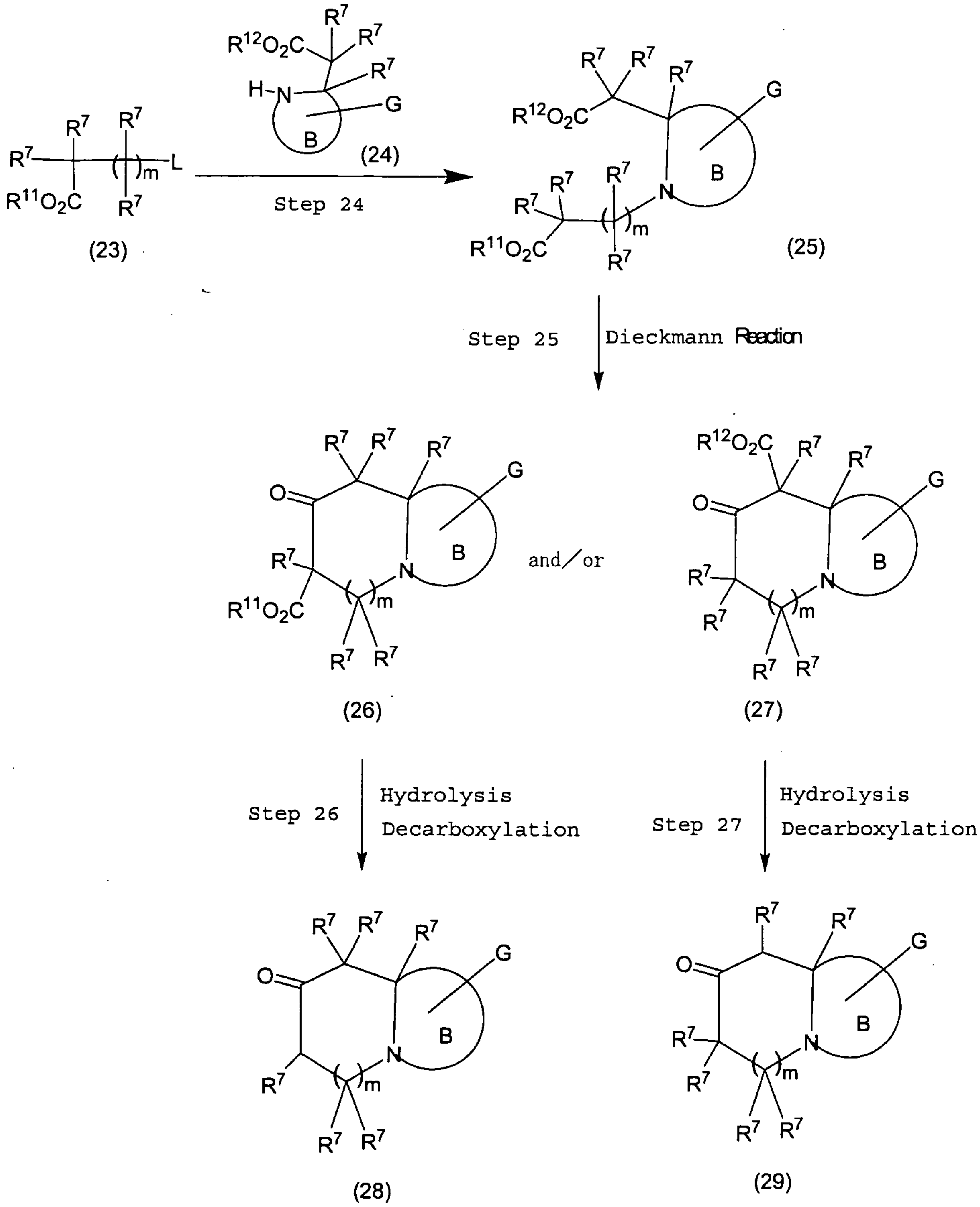


(23) (24) (25)

(26) and/or (27)

(28) (29)

In the above reaction scheme, B, L, m and $R^7$ have the same meanings as those indicated hereinbefore, and $R^{11}$ and $R^{12}$ are the same or different and each represents independently a "lower alkyl group" described hereinbefore or an "aralkyl group" described hereinbefore. In both compounds (28) and (29), however, at least three of six $R^7$ are a hydrogen atom.

Step 24 is a process for the preparation of a cyclic-amine diester compound (25) by reacting a cyclic amino acid ester compound (24) with a carboxylic acid ester compound (23) having a leaving group (L).

This reaction is generally carried out in the presence or absence of a base in a solvent.

The solvent employed can be, for example, an alcohol such as methanol, ethanol, propanol or isopropanol; an ether such as diethyl ether, diisopropyl ether, tetrahydrofuran or dioxane; an aprotic polar solvent such as dimethylformamide, dimethylacetamide or dimethyl sulfoxide; a nitrile such as acetonitrile; an ester such as methyl acetate or ethyl acetate; an aromatic hydrocarbon such as benzene, toluene or xylene; or an aliphatic hydrocarbon such as pentane, hexane or heptane.

The base employed can be, for example, an alkali metal alkoxide such as sodium methoxide, sodium ethoxide or potassium tert-butoxide; an alkali metal hydride such as sodium hydride or lithium hydride; an alkali metal hydroxide such as sodium hydroxide or potassium hydroxide; an alkali metal carbonate such as sodium carbonate or potassium carbonate; or an amine such as triethylamine, tributylamine, pyridine, picoline or 1,8-diazabicyclo[5.4.0]undec-7-ene.

The reaction temperature is generally between -20°C and 150°C, and preferably between 0°C and 100°C. The reaction time is generally from 10 minutes to 48 hours, and preferably from 30 minutes to 12 hours.

Subsequently, the cyclic-amine diester compound (25) is converted into a keto ester compound [compound (26) and/or compound (27)] by Dieckmann reaction, and then each of these products is subjected successively to hydrolysis and decarboxylation reactions to prepare the corresponding desired cyclic amino ketone compounds [compounds (28) and (29)] (Steps 26 and 27).

The reactions used in Steps from 25 to 27 can be carried out in a similar manner to those described by J.R. Harrison et al., J. Chem. Soc., Perkin Trans. 1, 1999, 3623-3631 (1999), and Steps 26 and 27 are, for example, carried out as described below.

The reactions of Steps 26 and 27 are generally carried out in the presence or absence of an acid or a base, in the presence or absence of a solvent.

The solvent employed can be water or a mixed solvent of water and an organic solvent (for example, an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; or an ester such as methyl acetate or ethyl acetate), and is preferably water, or a mixed solvent such as water and alcohol or ether.

The acid employed is not particularly restricted provided that it can be used as an acid in general hydrolysis reactions, and such acid can be, for example, a mineral acid such as hydrochloric acid, sulfuric acid or phosphoric acid; a carboxylic acid such as formic acid, acetic acid, propionic acid or trifluoroacetic acid; or a sulfonic acid such as methanesulfonic acid or ethanesulfonic acid; and is preferably a mineral acid or a carboxylic acid, and more preferably hydrochloric acid, sulfuric acid, formic acid or acetic acid.

Furthermore, this reaction can be accelerated by addition of an acid.

The base employed is not particularly restricted provided that it can be used as a base in general hydrolysis reactions, and can be, for example, an alkali metal hydride such as sodium hydride or lithium hydride; an alkali metal hydroxide such as sodium hydroxide or potassium hydroxide; an alkali metal carbonate such as sodium carbonate or potassium carbonate; and an amine such as triethylamine, tributylamine, pyridine, picoline or 1,8-diazabicyclo[5.4.0]undec-7-ene, and is preferably an alkali metal hydroxide, and more preferably sodium hydroxide or potassium hydroxide.

The reaction temperature is generally between -20°C and 150°C, and preferably between 0°C and 100°C. The reaction time is generally from 10 minutes to 48 hours, and preferably from 30 minutes to 12 hours.

(Method K)

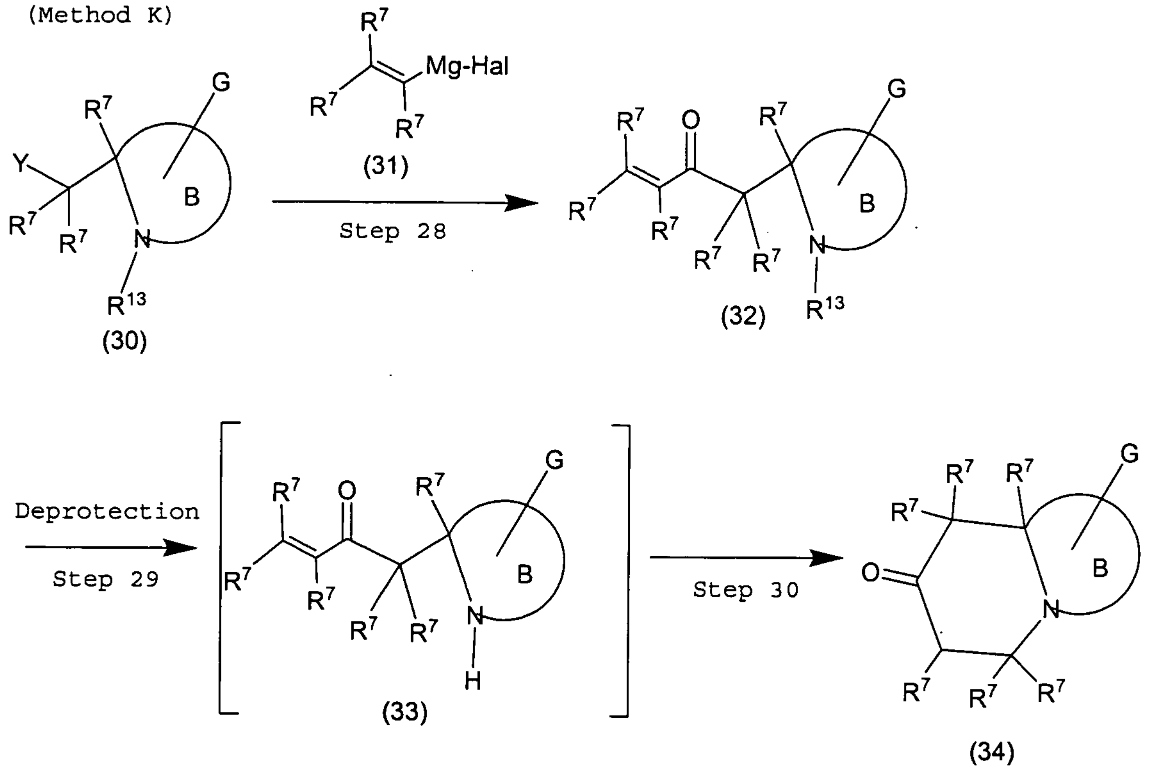


(30) (31) (32) (33) (34)

In the above reaction scheme, B, G and $R^7$ have the same meanings as those indicated hereinbefore,
$R^{13}$ represents a protective group for an amino group,
Hal represents a halogen atom (preferably a chlorine atom, a bromine atom or an iodine atom); and
Y presents a halogenocarbonyl group (for example, -CO-Cl, -CO-Br or -CO-I), N-lower-alkoxy-N-lower alkylcarbamoyl group (for example, a N-methoxy-N-methylcarbamoyl, N-ethoxy-N-methylcarbamoyl, N-ethyl-N-methoxycarbamoyl group or the like) or a cyano group.

In compound (34), however, at least three of the six $R^7$ groups are hydrogen atoms.

The "protective group for an amino group" in the definition of $R^{13}$ means a protective group for an amino group which is usually used in organic synthesis, and such group can be, for example, an "aliphatic acyl group" consisting of: an alkanoyl group such as a formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, icosanoyl or heneicosanoyl group, a halogenated alkylcarbonyl group such as a chloroacetyl, dichloroacetyl, trichloroacetyl or trifluoroacetyl group, a lower alkoxyalkylcarbonyl group such as a methoxyacetyl group, and an unsaturated alkylcarbonyl group such as an acryloyl, propioloyl, methacryloyl, crotonoyl, isocrotonoyl or (E)-2-methyl-2-butenoyl group (preferably a lower aliphatic acyl group having from 1 to 6 carbon atoms); an "aromatic acyl group" consisting of: an arylcarbonyl group such as a benzoyl, α-naphthoyl or β-naphthoyl group, a halogenated arylcarbonyl group such as a 2-bromobenzoyl, 4-chlorobenzoyl or 2,4,6-trifluorobenzoyl group, a lower alkylated arylcarbonyl group such as a 2,4,6-trimethylbenzoyl or 4-toluoyl group, a lower alkoxylated arylcarbonyl group such as a 4-anisoyl group, a nitrated arylcarbonyl group such as a 4-nitrobenzoyl or 2-nitrobenzoyl group, a lower alkoxycarbonylated arylcarbonyl group such as a 2-(methoxycarbonyl)benzoyl group, and an arylated arylcarbonyl group such as a 4-phenylbenzoyl group; a "silyl group" consisting of: tri-lower alkylsilyl group such as a trimethylsilyl, triethylsilyl, isopropyldimethylsilyl, t-butyldimethylsilyl, methyldiisopropylsilyl, methyldi-t-butylsilyl or triisopropylsilyl group, and a tri-lower alkylsilyl group substituted with from 1 to 2 aryl groups such as a diphenylmethylsilyl, diphenylbutylsilyl, diphenylisopropylsilyl or phenyldiisopropylsilyl group; an "aralkyl group" described above, an "alkoxycarbonyl group" described above, an "alkenyloxycarbonyl group" described above, or an "aralkyloxycarbonyl group" described above.

Step 28 is a process for the preparation of a α,β-unsaturated ketone form (32) by reacting a cyclic amino acid derivative (30) with a Grignard reagent of olefin group (31). In this step, a reaction which is well known as a reaction for preparing a ketone from a carboxylic acid derivative and a Grignard reagent can be applied, and this step can be, for

example, carried out according to the methods described in detail by H. R. Snyder et al., Org. Synth. , III, 798 (1955) ; J. Cason et al. , J. Org. Chem. , vol. 26, 1768 (1961) ; G. H. Posner et al., J. Am. Chem. Soc., vol. 94, 5106 (1972); and G. H. Posner, Org. React., vol. 19, 1 (1972).

Subsequently, the protective group ($R^{13}$) for the nitrogen atom of the α,β-unsaturated ketone form (32) is removed to prepare the corresponding free form (33) (Step 29), and then the product obtained is subjected to a ring closure reaction (Step 30) to prepare the desired cyclic amino ketone compound (34). In Step 29, a deprotection reaction generally used in the organic synthesis (for example, the reaction described by T.W.Greene et al., Protective Groups in Organic Synthesis, John Willey and Sons, Inc.) can be used, and preferably, a deprotection reaction under neutral or acidic conditions is used. The product (33) prepared by this deprotection reaction immediately develops a ring closure to prepare the desired amino ketone compound (34). In Step 29, when the deprotection reaction is carried out under acidic conditions, the amino ketone compound (34) can be obtained immediately by neutralizing the reaction mixture.

(Method L)

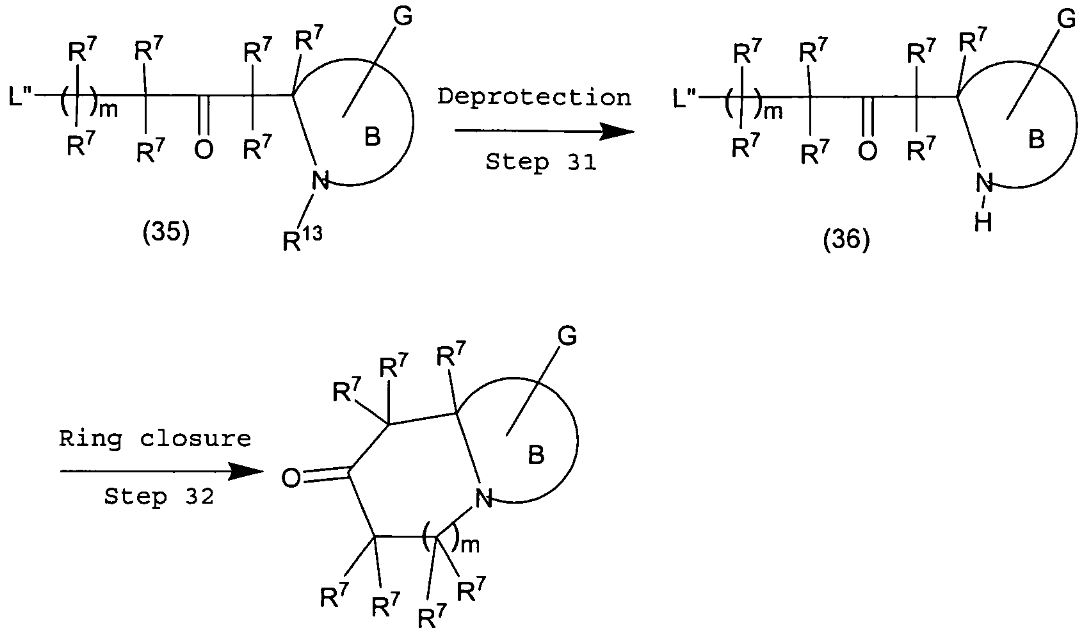


(35) (36)

(37)

In the above reaction scheme, B, G, $R^7$; $R^{13}$ and m have the same meanings as those indicated hereinbefore; and L" represents a leaving group in the definition of L; a "lower alkylsulfonyl group" described hereinbefore; an "arylsulfonyl group" such as a benzenesulfonyl or p-toluenesulfonyl group; or a halogeno lower alkylsulfonyl group (for example, trifluoromethanesulfonyl, pentafluoroethanesulfonyl group or the like). In compound (37), however, at least four of the seven $R^7$ groups are hydrogen atoms.

In Steps 31 and 32, a protective group ($R^{13}$) in the ketone compound (32) having the leaving group is removed to prepare the corresponding free form (36), and then the free form obtained is subjected to a ring closure reaction to prepare the desired amino ketone compound (37). These processes can be carried out in a similar manner to those described in Steps 29 and 30 of <Method K>. The compounds (35) used as the starting material in this method are known compounds or can be prepared from known compounds by known procedures (for example, methods described by S.W. Goldstein et al., J. Org. Chem., vol. 57, 1179-1190 (1992) and B. Achille et al., J. Comb. Chem., vol. 2, 337-340 (2000), or the like).

(Method M)

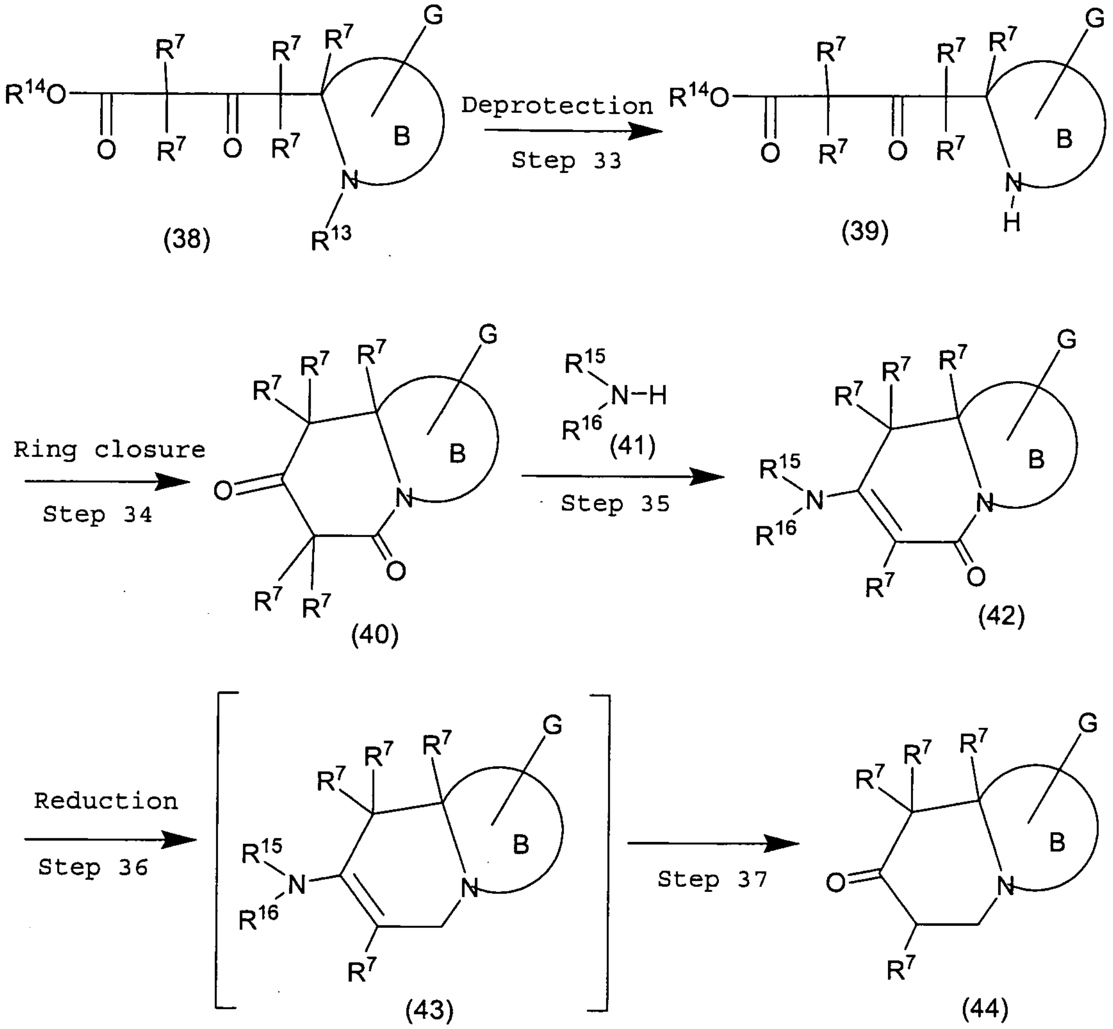


In the above reaction scheme, G, $R^7$, $R^{13}$ and B have the same meanings as those indicated hereinbefore, $R^{14}$ represents a hydrogen atom or a protective group for a carboxyl group; and
$R^{15}$ and $R^{16}$ are the same or different and each represents independently a hydrogen atom, a "lower alkyl group" described hereinbefore or an "aralkyl group" described hereinbefore or $R^{15}$ and $R^{16}$ together with the nitrogen atom to which they are bonded form a 5- or 6-membered heterocyclyl group which contains one nitrogen atom and furthermore may optionally contain one heteroatom selected from an oxygen atom, a sulfur atom and a nitrogen atom such as a piperidyl, piperazinyl, morpholinyl or thiomorpholinyl group.

In compound (44), however, at least one of the four $R^7$ groups is a hydrogen atom.

The "protective group for a carboxyl group" in the definition of $R^{14}$ represents a protective group for a carboxyl group which can generally be used in the field of organic synthesis, and said protecting group can be, for example, a "lower alkyl group" described hereinbefore, a "lower alkenyl group" described hereinbefore or an "aralkyl group" described hereinbefore, and is preferably a "lower alkyl group" described hereinbefore or a "lower alkenyl group" described hereinbefore.

In Steps 33 and 34, the protective group ($R^{13}$) in the $\alpha$-keto acid compound (38) is removed to prepare the free form (39), and then the free form obtained is subjected to a ring closure reaction to prepare a ketolactam compound (40). These steps are carried out in a similar manner to those described in Steps 29 and 30 of <Method K>.

Step 35 is a process for the preparation of a cyclic enaminolactam compound (42) by reacting keto lactam compound (40) with a secondary amine compound (41). In this Step, an enamine synthesis method used generally in the field of organic synthesis can be used, and the reaction is carried out, for example, according to the procedure described by G. Stork et al., J. Am. Chem. Soc . , vol. 85, 207 (1963) as shown below.

The reaction is generally carried out in the presence or absence of an acid in a solvent.

The solvent employed can be, for example, an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; or an ester such as methyl acetate or ethyl acetate, and is preferably an ether.

The acid employed can be, for example, an inorganic acid such as hydrogen chloride, hydrobromic acid, sulfuric acid, perchloric acid or phosphoric acid; or an organic acid such as acetic acid, formic acid, oxalic acid, methanesulfonic acid, p-toluenesulfonic acid, trifluoroacetic acid or trifluoromethanesulfonic acid, and is preferably sulfuric acid, hydrogen chloride or p-toluenesulfonic acid.

In this Step, the reaction can be effectively carried out by adding molecular sieves or by removing generated water using a water separator (for example, Dean Stark Water Separator).

The reaction temperature is generally between -20°C and 150°C, and preferably between 0°C and 100°C. The reaction time is generally from 10 minutes to 48 hours, and preferably from 30 minutes to 12 hours.

Step 36 is a process for the preparation of a cyclic enamine compound (43) by reducing cyclic enaminolactam compound (42). In this Step, a reduction reaction for preparing an amine derivative from an amide derivative, which is used generally in the field of organic synthesis, can be used, and the reaction is carried out, for example, according to the procedures described by S. Cortes et al., J. Org. Chem., vol. 48, 2246 (1983), Y. Tsuda et al., Synthesis, 1977, 652 (1977), H.C. Brown et al., J. Am. Chem. Soc. , vol. 86, 3566 (1964) and R.J. Sundberg et al., J. Org. Chem., vol. 46, 3730 (1981) as shown below.

The reaction is generally carried out in the presence of a reducing agent in a solvent.

The reducing agent employed can be, for example, a hydride reagent consisting of: an alkali metal borohydride such as sodium borohydride or lithium borohydride, or an aluminium hydride compound such as lithium aluminium hydride or lithium triethoxyaluminohydride; a combination of a Lewis acid, such as aluminium chloride, tin tetrachloride or titanium tetrachloride, and a "hydride reagent" described above; and a boron compound such as diborane, and is preferably lithium aluminium hydride.

An aprotic polar solvent can be used as a solvent, and a preferable solvent is an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; or an ether such as diethyl ether, diisopropyl ether, tetrahydrofuran or dioxane. Of these solvents, the most preferable solvent is an ether.

The reaction temperature is generally between -20°C and 150°C, and preferably between 0°C and 100°C. The reaction time is generally from 10 minutes to 48 hours, and preferably from 30 minutes to 12 hours.

Step 37 is a process for the preparation of a cyclic aminoketone compound (44) by hydrolyzing cyclic enamine compound (43), and the reaction is carried out by contacting cyclic enamine (43) with water in the presence or absence of an acid or base in the presence or absence of a solvent.

The solvent employed can be water or a mixed solvent of water and an organic solvent (for example, an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; or an ester such as methyl acetate or ethyl acetate), and is preferably water, or a mixed solvent such as water and alcohol or ether.

The acid employed is not particularly restricted provided that it can be used as an acid in general hydrolysis reactions, and can be, for example, a mineral acid such as hydrochloric acid, sulfuric acid or phosphoric acid; a carboxylic acid such as formic acid, acetic acid, propionic acid or trifluoroacetic acid; or a sulfonic acid such as methanesulfonic acid or ethanesulfonic acid, and is preferably hydrochloric acid, sulfuric acid or acetic acid. This reaction is accelerated by addition of an acid.

The base employed is not particularly restricted provided that it can be used as a base in general hydrolysis reactions, and can be, for example, an alkali metal hydride such as sodium hydride or lithium hydride; an alkali metal hydroxide such as sodium hydroxide or potassium hydroxide; an alkali metal carbonate such as sodium carbonate or potassium carbonate; or an amine such as triethylamine, tributylamine, pyridine, picoline or 1,8-diazabicyclo[5.4.0]undec-7-ene, and is preferably sodium hydroxide or potassium hydroxide.

The reaction temperature is generally between -20°C and 150°C, and preferably between 0°C and 100°C. The reaction time is generally from 10 minutes to 48 hours, and preferably from 30 minutes to 12 hours.

The compound (42) which is an intermediate in the synthesis of cyclic-amino ketone compound (44) can also be prepared by <Method N> described below.

(Method N)

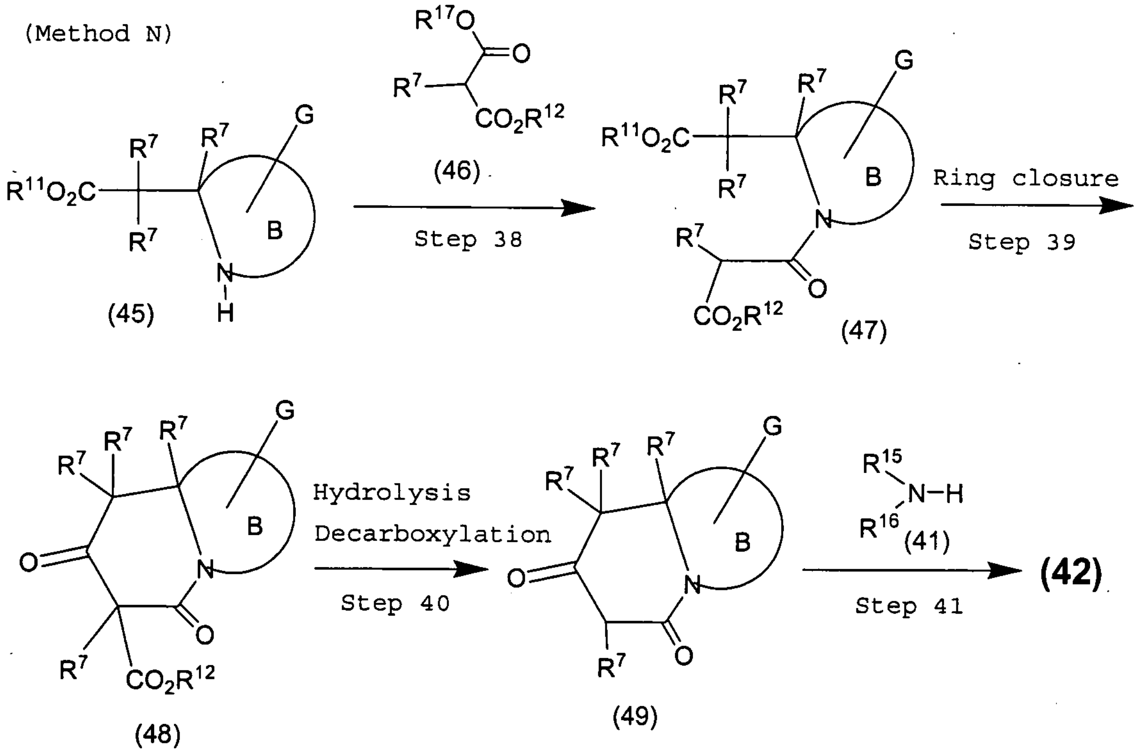


(45) (47) (48) (49)

In the above reaction scheme, B, G, $R^7$, $R^{11}$, $R^{12}$, $R^{15}$ and $R^{16}$ have the same meanings as those indicated hereinbefore; and

$R^{17}$ represents a hydrogen atom or a protective group for a carboxyl group.

In compound (42), however, at least one of the four $R^7$ groups is a hydrogen atom.

The "protective group for a carboxyl group" in the definition of $R^{17}$ represents a protective group for a carboxyl group which can generally be used in the field of organic synthesis, and said protecting group can be, for example, a "lower alkyl group" described hereinbefore or an "aralkyl group" described hereinbefore.

Step 38 is a process for the preparation of an amide-diester compound (47) by reacting a cyclic amino acid ester compound (45) with a malonic acid derivative (46) or its reactive derivative. In this Step, an amidation reaction used generally in the field of organic synthesis can be used, and the reaction is carried out, for example, as described in following (a), (b) and (c).

(a) In the case that $R^{17}$ represents a hydrogen atom, the reaction is carried out in the presence of a condensing agent in a solvent in the presence or absence of a base.

The solvent employed can be, for example, an aliphatic hydrocarbon such as pentane, hexane or heptane; an aromatic hydrocarbon such as benzene, toluene or xylene; a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; an ester such as methyl acetate or ethyl acetate; water; or a mixture of solvents mentioned above, and is preferably a halogenated hydrocarbon, an ether or an ester, and more preferably dichloromethane, tetrahydrofuran or ethyl acetate.

The condensing reagent employed can be, for example, dicyclohexylcarbodiimide, 1-ethyl-3-(3-dimethylaminopropyl)carbodiimide or N,N'-carbonyldiimidazole.

The base employed in the above reaction can be, for example, an alkali metal alkoxide such as sodium methoxide, sodium ethoxide or potassium tert-butoxide; an alkali metal hydride such as sodium hydride or lithium hydride; an alkali metal hydroxide such as sodium hydroxide or potassium hydroxide; an alkali metal carbonate such as sodium carbonate or potassium carbonate; or an amine such as triethylamine, tributylamine, pyridine, picoline or 1,8-diazabicyclo[5.4.0]undec-7-ene, and is preferably an amine, and more preferably triethylamine, pyridine or 1,8-diazabicyclo[5.4.0]undec-7-ene.

The reaction temperature is generally between -20°C and 150°C, and preferably between 0°C and 100°C . The

reaction time is generally from 10 minutes to 48 hours, and preferably from 30 minutes to 12 hours.

Furthermore, in the case that $R^{17}$ represents a hydrogen atom, the compound (46) is converted to the corresponding active derivative first, and then the reaction can also be carried out according to the procedure in (c) described below.

(b) In the case that $R^{17}$ represents a protective group for a carboxyl group (preferably a "lower alkyl group" described hereinbefore or an "aralkyl group" described hereinbefore), the reaction can be attained by heating in the presence or absence of a solvent.

In the case that the reaction is carried out in a solvent, the same solvent as that described in (a) can be used, and the reaction temperature is between 30°C and 100°C, and preferably in the range of the boiling point of the solvent used ± 5°C. Particularly preferably, this reaction is carried out by heating the reaction mixture under refluxing.

In the case that a solvent is not used, this reaction can be carried out by heating a mixture of compound (45) and compound (46) . The reaction temperature is between 30°C and 150°C, and preferably between 50°C and 120°C. The reaction time is usually from 10 minutes to 48 hours, and preferably from 30 minutes to 12 hours.

(c) In the case that a reactive derivative of compound (46) is used, the "reactive derivative" represents an acid halide, a mixed acid anhydride, an active ester or an active amide, and the reaction is carried out in the presence of a condensing agent in a solvent in the presence or absence of a base.

The "acid halide" can be obtained by reacting a compound (46) wherein $R^{17}$ is a hydrogen atom with a halogenating agent (for example, thionyl chloride, oxalyl chloride or the like); the "mixed acid anhydride" can be obtained by reacting a compound (46) wherein $R^{17}$ is a hydrogen atom with an acid halide (for example, methyl chlorocarbonate, ethyl chlorocarbonate or the like); the "active ester" can be obtained by reacting a compound (46) wherein $R^{17}$ is a hydrogen atom with a hydroxy compound (for example, N-hydroxysuccinimide, N-hydroxyphthalimide or the like) in the presence of the "condensing agent" described in (a) ; and the "active amide" (for example, Weinreb amide) can be obtained by reacting a compound (46) wherein $R^{17}$ is a hydrogen atom with a N-lower-alkoxy-N-lower alkylhydroxylamine (for example, N-methoxy-N-methylhydroxylamine or the like) in the presence of the "condensing agent" described in (a). All these reactions mentioned above are carried out under the reaction conditions commonly used in_organic synthesis.

The solvent, condensing agent and base described in (a) can be used in the above reactions as the solvent, condensing agent and base.

The reaction temperature is generally between -20°C and 150°C, and preferably between 0°C and 100°C. The reaction time is generally from 10 minutes to 48 hours, and preferably from 30 minutes to 12 hours.

Steps 39 and 40 are processes for the preparation of a ketolactam compound (49) by converting amide-diester compound (47) into a ketolactam ester compound (48) by Dieckmann reaction, followed by subjecting successively the compound (48) obtained to hydrolysis and decarboxylation reactions, and these reaction are carried out in a similar manner to those described in Steps 25 and 26 of <Method J>.

Step 41 is a process for the preparation of a cyclic enaminolactam compound (42) by reacting keto lactam compound (49) with a secondary amine compound (41), and the reaction is carried out in a similar manner to that described in Step 35 of <Method M>.

Furthermore, compound (48) which is an intermediate in the <Method N> mentioned above can also be prepared by <Method O> described below.

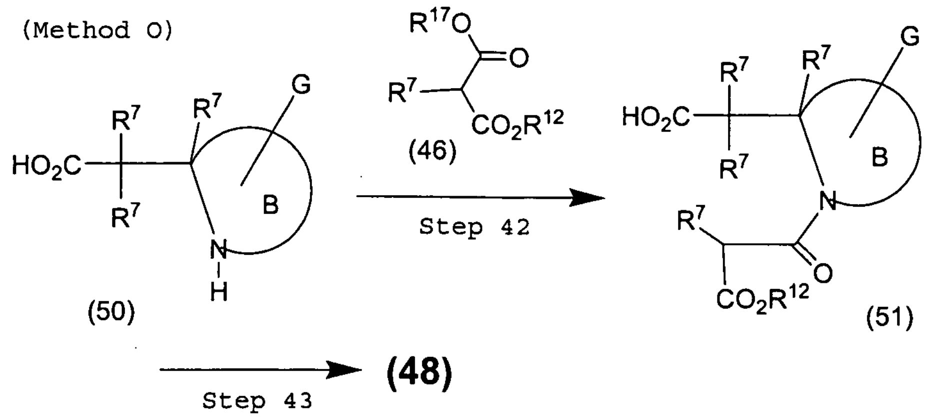


In the above reaction scheme, B, G, $R^7$, $R^{12}$ and $R^{17}$ have the same meanings as those indicated hereinbefore.

In compound (48), however, at least one of the four $R^7$ groups represents a hydrogen atom.

Step 42 is a process for the preparation of an amide-monoester compound (51) by reacting a cyclic amino acid

compound (50) with a malonic acid derivative (46) or its reactive derivative, and the reaction is carried out in a similar manner to those described in (a), (b) and (c) of Step 38 in <Method N>.

Step 43 is a process for the preparation of a ketolactam ester compound (48) by an intramolecular cyclization between a carboxyl group of the amide-monoester compound (51) and an active methylene group. In this step, compound (51) or its reactive derivative can be used.

(a) In the case that the compound (51) is used without derivatization, the reaction is carried out in the presence of a condensing agent in a solvent in the presence or absence of a base.
The solvent employed can be, for example, a halogenated hydrocarbon such as dichloromethane, chloroform, carbon tetrachloride or dichloroethane; an ether such as diethyl ether, diisopropyl ether, tetrahydrofuran or dioxane; an alcohol such as methanol, ethanol, propanol, isopropanol, butanol, s-butanol, isobutanol or t-butanol; an aprotic polar solvent such as N,N-dimethylformamide, N,N-dimethylacetamide or dimethyl sulfoxide; a nitrile such as acetonitrile; an ester such as methyl acetate or ethyl acetate; water; or a mixture of solvents mentioned above, and is preferably a halogenated hydrocarbon, an ether or an ester, and more preferably dichloromethane, tetrahydrofuran or ethyl acetate.
The condensing reagent employed can be, for example, dicyclohexylcarbodiimide, 1-ethyl-3-(3-dimethylaminopropyl)carbodiimide or N,N'-carbonyldiimidazole.
The base employed can be, for example, an alkali metal alkoxide such as sodium methoxide, sodium ethoxide or potassium tert-butoxide; an alkali metal hydride such as sodium hydride or lithium hydride; an alkali metal hydroxide such as sodium hydroxide or potassium hydroxide; an alkali metal carbonate such as sodium carbonate or potassium carbonate; or an amine such as triethylamine, tributylamine, pyridine, picoline or 1,8-diazabicyclo[5.4.0]undec-7-ene, and is preferably an amine, and more preferably triethylamine, pyridine or 1,8-diazabicyclo[5.4.0]undec-7-ene.
The reaction temperature is generally between -20°C and 150°C, and preferably between 0°C and 100°C . The reaction time is generally from 10 minutes to 48 hours, and preferably from 30 minutes to 12 hours.
(b) In the case that the compound (51) converted into a reactive derivative is used, said reactive derivative can be an acid halide, a mixed acid anhydride, an active ester, an active amide or the like.

The "acid halide" can be obtained by reacting compound (51) with a halogenating agent (for example, thionyl chloride, oxalyl chloride or the like) ; the "mixed acid anhydride" can be obtained by reacting compound (51) with an acid halide (for example, methyl chlorocarbonate, ethyl chlorocarbonate or the like); the "active ester" can be obtained by reacting compound (51) with a hydroxy compound (for example, N-hydroxysuccinimide, N-hydroxyphthalimide or the like) in the presence of the "condensing agent" described in (a); and the "active amide" (for example, Weinreb amide) can be obtained by reacting compound (51) with a N-lower-alkoxy-N-lower alkylhydroxylamine (for example, N-methoxy-N-methylhydroxylamine or the like) in the presence of the "condensing agent" described in (a) . All these reactions mentioned above are carried out under the reaction conditions commonly used in organic synthesis.

The ring closure reaction of said reactive derivative is generally carried out in the presence or absence of a base in a solvent.

The solvent, condensing agent and base described in (a) can be used in (b) as the solvent, condensing agent and base.

The reaction temperature is generally between -20°C and 150°C, and preferably between 0°C and 100°C. The reaction time is generally from 10 minutes to 48 hours, and preferably from 30 minutes to 12 hours.

(Method P)

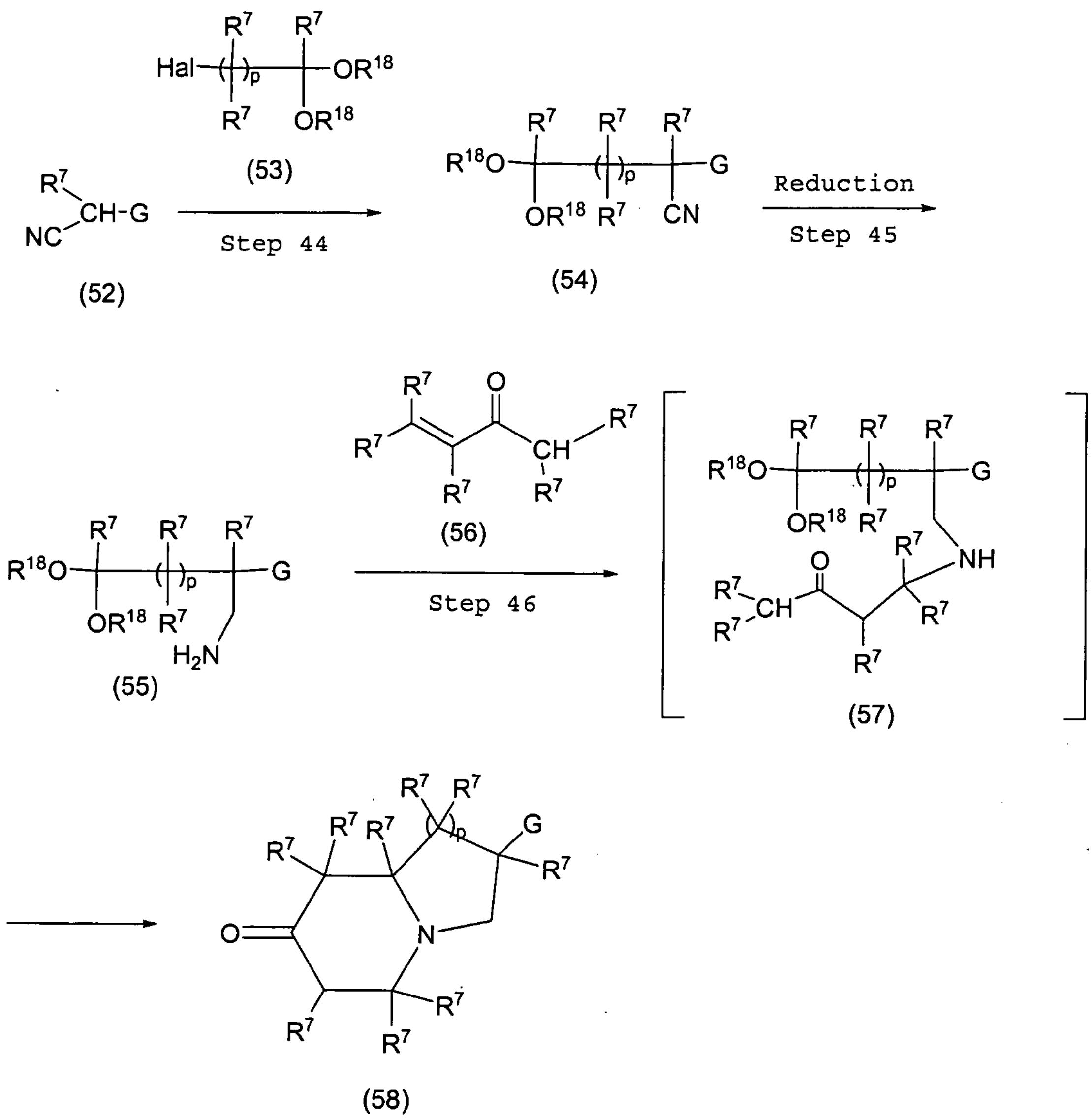


(52) (53) (54) (55) (56) (57) (58)

In the above reaction scheme, G, Hal and $R^7$ have the same meanings as those indicated hereinbefore,

$R^{18}$ represents a lower alkyl group (preferably a methyl or ethyl group); and p represents an integer of from 1 to 3. In compound (58), however, at least six of the nine $R^7$ groups are hydrogen atoms.

Step 44 is a process for the preparation of a compound (54) by reacting a cyano compound (52) with a halogenated compound (53), and Step 45 is a process for the preparation of an amino compound (55) by reducing a cyano group of the compound (54).

Step 46 is a process for the preparation of a compound (58) through a compound (57) obtained by reacting amino compound (55) with a $\alpha,\beta$-unsaturated ketone compound (56).

The series of reactions of Steps 44, 45 and 46 [that is, <Method P>] is carried out according to the procedure described in detail by Frank D. King, J. Chem. Soc., Perkin Trans. 1, 1986, 447-453 (1986).

In the methods from <Method I> to <Method P> described above as preparation methods for the compound (3), G may be a group having formula: $Z(R^5{}_a)(R^6)_n$ [$R^6$, n and Z have the same meanings as those indicated hereinbefore; $R^5{}_a$ represents a substituent group in the definition of $R^5$ or a halogen atom or a group having formula: $SR^c$ ($R^c$ has the same meaning as that indicated hereinbefore)]; and the group having formula: $SR^c$ can be converted into a group having formula: $SOR^c$ or a group having formula: $SO_2R^c$ in any of the subsequent steps in a similar manner to that described in Step 21 or Step 22 of <Method H>.

Further, a halogen atom can be converted into a carboxyl group or an alkoxycarbonyl group in any of subsequent steps of <Method Q> described below.

(Method Q)

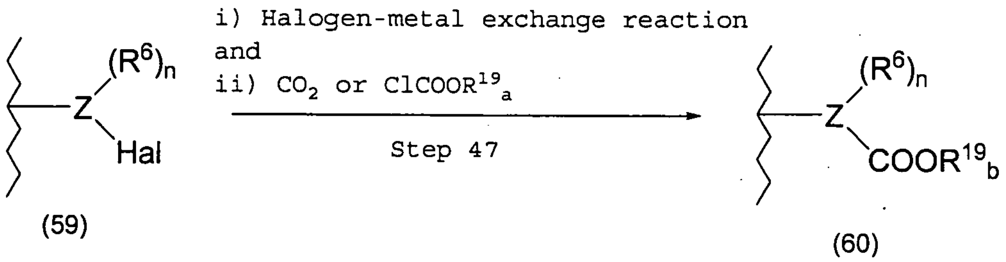


(59) (60)

In the above reaction scheme, Hal, n, $R^6$ and Z have the same meanings as those indicated hereinbefore,

$R^{19}_a$ represents a lower alkyl group (preferably a methyl or ethyl group) ; and $R^{19}_b$ represents a halogen atom or a lower alkyl group.

The halogen-metal exchange reaction (i) shown as a first stage in Step 47 is carried out, for example, according to the procedure described in detail by M. Schlosser et al., Organometallics in Synthesis, John Wiley and Sons, Ltd.

The inert solvent employed in the halogen-metal exchange reaction can be a halogenated hydrocarbon such as dichloromethane, chloroform, carbon tetrachloride or dichloroethane; or an ether such as diethyl ether, diisopropyl ether, tetrahydrofuran or dioxane; and is preferably an ether.

The metalation agent employed in the halogen-metal exchange reaction can be, for example, an alkali metal such as lithium, sodium, potassium, methyllithium, butyllithium, lithium diisopropylamide, lithium bis (trimethylsilyl) amide or the like; or an alkaline earth metal such as magnesium, isopropylmagnesium bromide, diisopropylmagnesium or the like; and is preferably butyllithium.

The reaction temperature employed depends on various factors such as the starting materials, the solvent and the metalation agent used, but is generally between -100°C and 100°C, and preferably between -70°C and 0°C.

The reaction time depends on a number of factors such as the starting materials, the solvent, the metalation agent and the reaction temperature, but is usually from 5 minutes to 10 hours, and preferably from 10 minutes to 5 hours.

The metalated compounds thus obtained can be used for the following reaction with carbon dioxide or chlorocarbonic acid ester without any further treatment or isolation.

The inert solvent employed in the reaction with carbon dioxide or chlorocarbonic acid ester can be a halogenated hydrocarbon such as dichloromethane, chloroform, carbon tetrachloride or dichloroethane; or an ether such as diethyl ether, diisopropyl ether, tetrahydrofuran or dioxane; and is preferably an ether.

The reaction temperature employed depends on various factors such as the starting materials, the solvent and the metalation agent used, but is generally between -100°C and 100°C, and preferably between -70°C and 0°C.

The reaction time depends on a number of factors such as the starting materials, the solvent, the metalation agent and the reaction temperature, but is usually from 5 minutes to 10 hours, and preferably from 10 minutes to 5 hours.

Compounds (24), (30), (35), (38), (45) and (50), which are used as the starting materials or as materials other than the starting materials in the methods from <Method J> to <Method O>, are derivatives of homopurines, and can be prepared according to the procedures described in Japanese Patent Publication (Official Gazette) 2001-247564 (EP 1070711), Japanese Patent Publication (Official Gazette) 2002-284780 (WO 02/57255), Japanese Patent Publication (Official Gazette) 2002-284782 (WO 02/57265), and Japanese Patent Publications (Official Gazette) 2002-284783 (WO 02/57264) - 2004-099600 (WO 2004/009592).

The starting materials (23), (31), (41), (46), (52) and (53) used in methods from <Method J> to <Method P> are known compounds or can easily be prepared from known compounds according to known procedures.

Compound (5) can be prepared according to the procedure <Method R> described below.

(Method R)

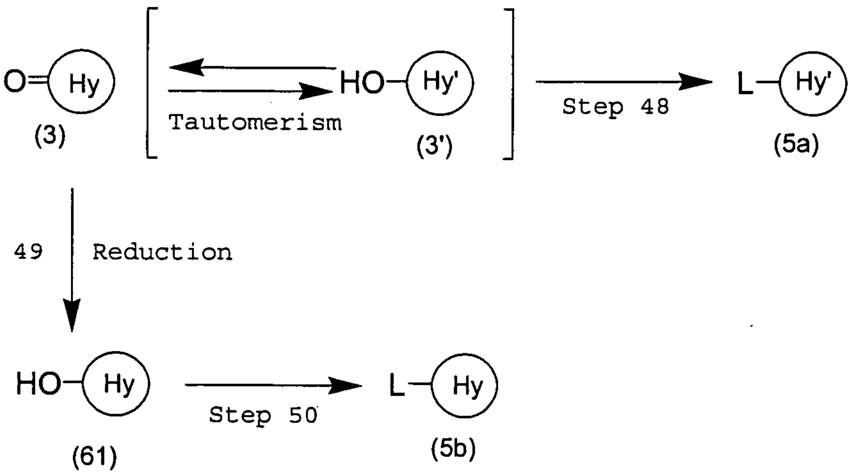


(3) (3') (5a) (61) (5b)

In the above reaction scheme, Hy, Hy' and L have the same meanings as those indicated hereinbefore.

Step 48 is a process for the preparation of a compound (5a) by converting a hydroxyl group of a compound (3'), which is a tautomer of a heterocylyl ketone compound (3), into a leaving group, and this reaction is carried out by reacting compound (3') with a halogenating agent (for example, a fluorinating agent such as (diethylamino)sulfur trifluoride (DSAT); a chlorinating agent such as thionyl chloride, phosphorus trichloride, phophorus pentachloride, phosphorus oxychloride or triphenylphosphine/carbon tetrachloride; a brominating agent such as hydrobromic acid, thionyl bromide, phosphorus tribromide or triphenylphosphine/carbon tetrachloride; or an iodinating agent such as hydroiodic acid or phosphorus triiodide); sulfonyl halide (for example, methanesulfonyl chloride, p-toluenesulfonyl chloride or the like); or sulfonic anhydride (for example, trifluoromethansulfonic anhydride or the like).

Step 49 is a process for the preparation of a heterocyclyl alcohol (61) by reducing heterocyclyl ketone (3), and in this reaction, a reduction reaction using a hydride reagent or a catalytic reduction in a hydrogen atmosphere can be used. The hydride reagent employed in the above reaction can be, for example, an alkali metal borohydride such as sodium borohydride or lithium borohydride; an aluminium hydride compound such as lithium aluminium hydride, lithium triethoxyaluminium hydride; sodium tellurium hydride; or an organic aluminium hydride reductant such as diisobutylaluminium hydride or sodium di(methoxyethoxy)aluminium dihydride. The above reaction is carried out according to the procedures described in detail by J. Dale, J. Chem. Soc., 910 (1961) and F.G. Bordwell et al., J. Org. Chem., vol. 33, 3385 (1968).

Step 50 is a process for the preparation of a compound (5a) by converting a hydroxyl group of heterocyclyl alcohol compound (61) into a leaving group, and the reaction is carried out in a similar manner to that described in Step 48.

Compound (6) is easily prepared according to the conventional methods, from a compound (5a) or (5b) previously described in "Method R".

Furthermore, compounds (12) can be prepared by <Method S> described below.

(Method S)

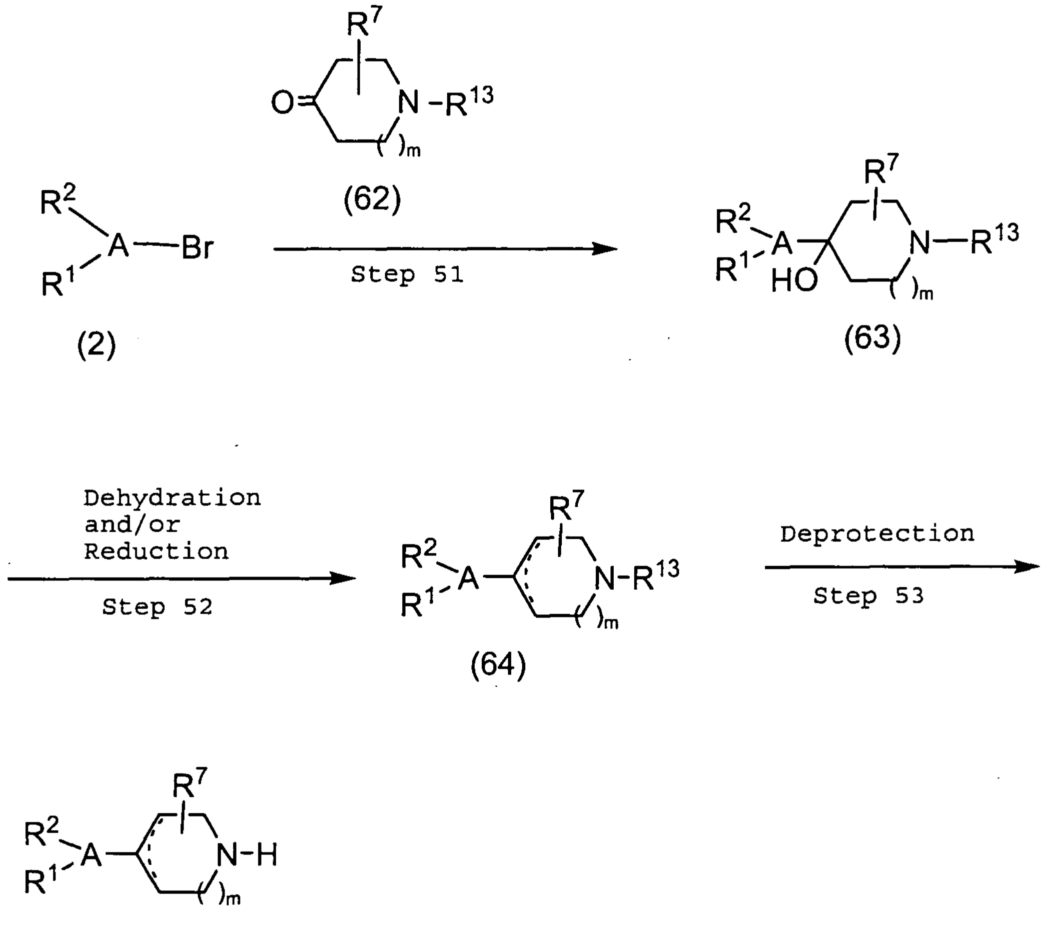


(62)

(2)

(63)

(64)

(12)

In the above reaction scheme, the bond containing a dotted line moiety, $R^1$, $R^2$, $R^7$, $R^{13}$ and m have the same meanings as those indicated hereinbefore.

Steps 51 and 52 can be carried out in a similar manner to those described in Steps 2, 3 and 4 of <Method A>.

Of compounds (13), a compound wherein $R^5$ in the definition of G is a group having formula: $SO_2NR^aR^b$ is prepared by <Method T> described below.

(Method T)

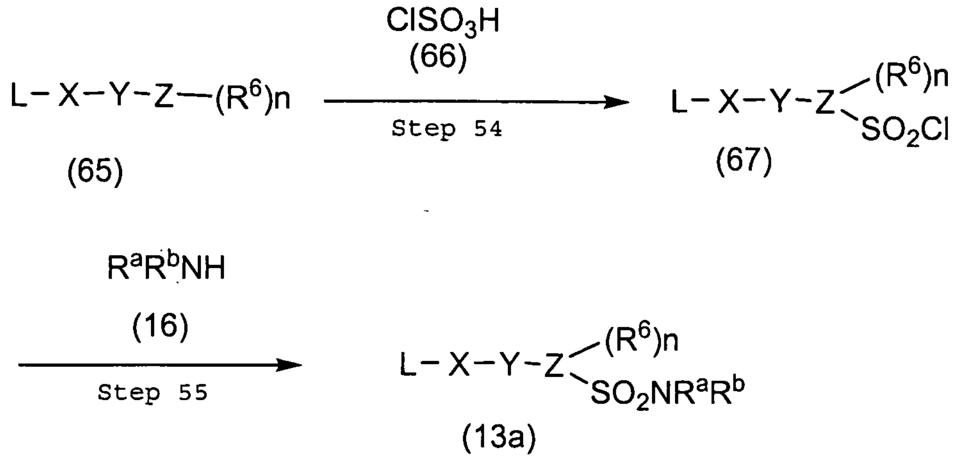


(65) (67) (13a)

In the above reaction scheme, $R^a$, $R^b$, $R^6$, L, n, X, Y and Z have the same meanings as those indicated hereinbefore.

Step 54 is a process for the preparation of a compound (66) by chlorosulfonylation of an aromatic ring moiety (Z) of a compound (65) with chlorosulfonic acid, and Step 55 is a process for the preparation of a sulfonamide compound (13a) by reacting compound (66) with an amine compound (16).

Steps 54 and 55 can be carried out according to procedures described in detail by G. E. Inskeep et al., JAGS., vol. 69, 2237-2238 (1947).

Furthermore, compounds (15) can be also prepared by <Method Q> described above.

After each reaction described above is completed, the desired compounds can be isolated from the reaction mixture by conventional treatments.

For example, neutralization of the reaction mixture, if necessary, or filtration of the reaction mixture when insoluble material is present in the reaction mixture, addition of a solvent immiscible with water such as ethyl acetate to the neutralized solution or the filtrate, washing the resulting organic layer with water, separation of the organic layer containing the desired compound, drying of the organic layer over anhydrous magnesium sulfate or the like, and then evaporation of the organic solvent to give the desired product.

The objective product thus obtained, if necessary, is further purified by conventional treatments, for example, by recrystallization or reprecipitation, or by conventional procedures in organic chemistry, for example, absorption column chromatography using a carrier such as silica gel, alumina, or Florisil consisting of magnesium and silica gel; partition column chromatography using a synthetic absorbent such as Sephadex LH-20 (product of Pharmacia Co. , Ltd.), Amberlite XAD-11 (product of Rohm & Hass Co. , Ltd.), or Diaion HP-20 (product of Mitsubishi Chemicals Co., Ltd.) ; ion exchange chromatography; normal phase or reversed phase column chromatography using silica gel or alkylated silica gel (preferably high performance liquid column chromatography); or an appropriate combination of these chromatographic techniques; and elution using an appropriate solvent to isolate and purify the desired product.

Since the cyclic tertiary amine compounds of the present invention exert excellent inhibitory activity against production of inflammatory cytokines, the compounds of the present invention are useful as prophylactic or therapeutic agents for diseases associated with inflammatory cytokines in warm-blooded animals (preferably humans). Thus the compounds of the present invention are useful, for example, as antipyretic, analgesic and anti-inflammatory drugs and antiviral agents, and as prophylactic or therapeutic agents for rheumatoid arthritis, osteoarthritis, allergic diseases, asthma, septicemic disease, psoriasis, osteoporosis, autoimmune diseases (for example, diffuse lupus erythematosus, ulcerative colitis, Crohn's disease, or the like), diabetes mellitus (particularly type I diabetes mellitus), nephritis, hepatitis, tumour, ischemic heart disease, Alzheimer's disease, or arterial sclerosis, preferably as antipyretic, analgesic and anti-inflammatory drugs and as prophylactic or therapeutic agents for rheumatoid arthritis, osteoarthritis, allergic diseases, septicemic disease, psoriasis, osteoporosis, ulcerative colitis, diabetes mellitus (particularly type I diabetes mellitus), hepatitis, arterial sclerosis, or Crohn's disease, particularly preferably as antipyretic, analgesic and anti-inflammatory drugs and as prophylactic or therapeutic agents for rheumatoid arthritis, osteoarthritis, septicemic disease, psoriasis, Crohn's disease, ulcerative colitis, diabetes mellitus (particularly type I diabetes mellitus), or hepatitis.

In cases where the compounds of the present invention are used as prophylactic or therapeutic agents for the disorders described above, the compounds expressed by the general formula (I) described above or pharmacologically

acceptable salts or esters thereof may be orally administered in formulations such as tablets, capsules, granules, powder, or syrups, or non-orally administered in formulations such as injections or suppositories. These preparations are prepared by conventionally known methods using additives such as excipients, lubricants, binders, disintegrants, stabilizers, flavouring agents, diluents, or the like.

In these preparations, the following excipients are used, for example, organic excipients including sugar derivatives such as lactose, sucrose, glucose, mannitol or sorbitol; starch derivatives such as corn starch, potato starch, α-starch, dextrin, or carboxymethyl starch; and cellulose derivatives such as crystalline cellulose, low substituted hydroxypropylcellulose, hydroxypropylmethylcellulose, carboxymethylcellulose, calcium carboxymethylcellulose, or internally crosslinked sodium carboxymethylcellulose; gum Arabic; dextran; pullulan, or the like; or inorganic excipients including silicate derivatives such as light anhydrous silicic acid, synthetic aluminium silicate, or magnesium aluminometasilicate; phosphates such as calcium phosphate; carbonates such as calcium carbonate; and sulfates such as calcium sulfate.

As the lubricants the following compounds, for example, stearic acid, metal salts of stearic acid such as calcium stearate or magnesium stearate; talc; colloidal silica; waxes such as veegum or spermaceti; boric acid; adipic acid; sulfates such as sodium sulfate; glycol; fumaric acid; sodium benzoate; DL-leucine; fatty acid sodium salt; laurylsulphates such as sodium lauryl sulfate or magnesium lauryl sulfate; silicates such as silicic anhydride or silicic hydrate; and starch derivatives described above can be listed.

As the binders, the following compounds, for instance, polyvinylpyrrolidone, Macrogol or similar excipients described above can be listed.

As the disintegrants, the following compounds, for instance, the similar compounds described above as excipients or chemically modified starch/cellulose derivatives such as croscarmellose sodium, or sodium carboxymethylstarch, and crosslinked polyvinylpyrrolidone can be listed.

As the stabilizers the following compounds, for example, para-oxy benzoates such as methylparaben or propylparaben; alcohols such as chlorobutanol, benzylalcohol or phenylethylalcohol; benzalkonium chloride; phenols such as phenol or cresol; thimerosal; dehydroacetic acid; and sorbic acid can be listed.

As the flavouring agents, the following compounds, for example, conventionally employed sweeteners, acidifiers and flavourings can be listed.

The dosage of the compounds having the general formula (I) or pharmacologically acceptable salts thereof in the present invention varies depending on the symptoms, age, route of administration, and the like of the patient. For example, in the case of oral administration, it is desirable to administer 0.1 mg (preferably 0.5 mg) as a lower limit and 2000 mg (preferably 500 mg) as an upper limit per one time for an adult human and one to six times a day depending on the symptoms. In the case of intravenous administration, it is desirable to administer 0.01 mg (preferably 0.05 mg) as a lower limit and 200 mg (preferably 50 mg) as an upper limit per one time for an adult human and one to six times a day depending on the symptoms.

[Best mode for carrying out the invention]

The present invention will be hereinafter described in more detail by way of the following Examples, Reference Examples, Formulation Examples, and Test Examples, which are intended to further illustrate the present invention but are not intended to limit the scope of the invention in any way.

(Example 1)

4-[1-(4-Ethoxycarbonylphenethyl)-1,2,3,6-tetrahydropyridin-4-yl]-2-(4-fluorophenyl)-3-(pyridin-4-yl)-1H-pyrrole (Exemplification compound number 1-360)

To a solution of 2-(4-fluorophenyl)-3-(pyridin-4-yl)-4-(1,2,3,6-tetrahydropyridin-4-yl)-1H-pyrrole (compound of Example 10 described in the Specification of European Patent Publication No. 1070711) (639 mg, 2 mmol) and 4-ethoxycarbonylphenethyl bromide (1.03 g, 4 mmol) in dimethylformamide (15 ml) was added potassium carbonate (1.38 g, 10 mmol), and the resulting mixture was stirred at 80°C for 5 hours. After stirring, water (40 ml) was added to the reaction mixture, and the resulting mixture was extracted with ethyl acetate. The separated organic layer containing the desired compound was washed with water, dried over anhydrous magnesium sulfate and evaporated in vacuo. The solid product thus obtained was washed with a small amount of methanol and re-crystallized from methanol to afford the title compound (432 mg) in a yield of 44% as a white fine-needle crystal.
Melting point: 217-218°C
$^1$H-NMR (400MHz, DMSO-$d_6$) δppm:
11.36 (1H, s), 8.45 (2H, d, J=6Hz), 7.87 (2H, d, J=8Hz), 7.37 (2H, d, J=8Hz), 7.18-7.09 (6H, m), 6.90 (1H, d, J=3Hz), 5.25 (1H, s), 4.30 (2H, quartet, J=7Hz), 2.94 (2H, br.s), 2.82 (2H, t, J=8Hz), 2.62-2.56 (4H, m), 2.16 (2H, br.s), 1.31 (3H,

t, J=7Hz).

(Example 2)

4-[1-(4-Carbamoylphenethyl)-1,2,3,6-tetrahydropyridin-4-yl]-2-(4-fluorophenyl)-3-(pyridin-4-yl)-1H-pyrrole (Exemplification compound number 1-366)

The title compound was synthesized in a yield of 32% as a pale brownish powder by conducting a reaction similar to that mentioned in Example 1, using 4-carbamoylphenethyl chloride instead of 4-ethoxycarbonylphenethyl bromide.
Melting point: 202-204°C
$^1$H-NMR (500MHz, DMSO-$d_6$) δppm:
11.36 (1H, s), 8.46 (2H, d, J=5Hz), 7.88 (1H, s), 7.78 (2H, d, J=10Hz), 7.28 (2H, d, J=10Hz), 7.25 (1H, s), 7.16-7.10 (6H, m), 6.90 (1H, s), 5.25 (1H, s), 2.92 (2H, br.s), 2.78 (2H, t, J=10Hz), 2.58-2.53 (4H, m), 2.16 (2H, m).

(Example 3)

3-[1-(4-Carbamoylphenethyl)-1,2,3,6-tetrahydropyridin-4-yl]-5-(4-fluorophenyl)-4-(pyridin-4-yl)-1H-pyrazole (Exemplification compound number 10-366)

The title compound was synthesized in a yield of 5% as a pale yellowish powder by conducting a reaction similar to that mentioned in Example 2, using 5-(4-fluorophenyl)-4-(pyridin-4-yl)-3-(1,2,3,6-tetrahydropyridin-4-yl)-1H-pyrazole instead of 2-(4-fluorophenyl)-3-(pyridin-4-yl)-4-(1,2,3,6-tetrahydropyridin-4-yl)-1H-pyrrole.
Melting point: 182-186°C
$^1$H-NMR (400MHz, DMSO-$d_6$) δppm:
13.17 (1H, br.s), 8.50 (2H, d, J=6Hz), 7.86 (1H, br.s), 7.75 (2H, d, J=8Hz), 7.31-7.21 (5H, m), 7.16 (2H, d, J=6Hz), 7.19-7.07 (2H, br.s), 5.72 (1H, br), 3.06-2.93 (2H, br.s), 2.79 (2H, t, J=8Hz), 2.64-2.54 (4H, m), 2.37-2.12 (2H, br.s).

(Example 4)

4-[1-(4-Carboxyphenethyl)-1,2,3,6-tetrahydropyridin-4-yl]-2-(4-fluorophenyl)-3-(pyridin-4-yl)-1H-pyrrole (Exemplification compound number 1-358)

To a solution of 4-[1-(4-ethoxycarbonylphenethyl)-1,2,3,6-tetrahydropyridin-4-yl]-2-(4-fluorophenyl)-3-(pyridin-4-yl)-1H-pyrrole (140 mg, 0.28 mmol) obtained in Example 1 in ethanol (7 ml) was added 1M aqueous lithium hydroxide solution (0.85 ml, 0.85 mmol), and the resulting mixture was stirred at 80°C for 30 minutes. After cooling to room temperature, the reaction mixture was acidified with 1N hydrochloric acid under stirring and then evaporated in vacuo. The solid precipitated was collected by filtration and washed with ethanol to afford the title compound in a yield of 89% (117 mg) as a pale yellow powder.
Melting point: 239-240°C
$^1$H-NMR (400MHz, DMSO-$d_6$) δppm:
11.36 (1H, s), 8.44 (2H, d, J=5Hz), 7.85 (2H, d, J=8Hz), 7.34 (2H, d, J=8Hz), 7.18-7.09 (6H, m), 6.91 (1H, d, J=3Hz), 5.25 (1H, br.s), 2.94 (2H, d, J=3Hz), 2.81 (2H, t, J=7Hz), 2.61-2.54 (4H, m), 2.16 (2H, br.s).

(Example 5)

2-(4-Fluorophenyl)-4-[1-[4-(N-methylcarbamoyl)phenethyl]-1,2,3,6-tetrahydropyridin-4-yl]-3-(pyridin-4-yl)-1H-pyrrole (Exemplification compound number 1-367)

To a solution of 4-[1-(4-carboxyphenethyl)-1,2,3,6-tetrahydropyridin-4-yl]-2-(4-fluorophenyl)-3-(pyridin-4-yl)-1H-pyrrole (500 mg, 1.07 mmol) obtained in Example 4 in dimethylformamide (8 ml) was added carbonyldiimidazole (347 mg, 2.14 mmol) under stirring, and the resulting mixture was stirred at room temperature for 30 minutes. Furthermore, to the reaction mixture was added 2M solution of methylamine in tetrahydrofuran (1.07 ml, 2.14 mmol) under stirring, and the resulting mixture was stirred at room temperature for 30 minutes. Subsequently, to the reaction mixture was added saturated aqueous sodium hydrogencarbonate solution (10 ml), and the resulting mixture was stirred for a while and then extracted with ethyl acetate. The separated organic layer containing the desired compound was washed with water, dried over anhydrous sodium sulfate and concentrated under reduced pressure. The solid product thus obtained was washed with a small amount of ethanol to afford the title compound (473 mg) in a yield of 92% as a pale brown powder.

Melting point: 231-233°C
$^1$H-NMR (400MHz, DMSO-$d_6$) δppm:
11.37 (1H, s), 8.46 (2H, d, J=6Hz), 8.34-8.33 (1H, m), 7.74 (2H, d, J=8Hz), 7.30 (2H, d, J=8Hz), 7.18-7.09 (6H, m), 6.91 (1H, d, J=2Hz), 5.25 (1H, s), 2.92 (2H, s), 2.80-2.73 (5H, m), 2.58-2.53 (4H, m), 2.15 (2H, br.s).

(Example 6)

4-[1-[4-(N-Ethylcarbamoyl)phenethyl]-1,2,3,6-tetrahydropyridin-4-yl]-2-(4-fluorophenyl)-3-(pyridin-4-yl)-1H-pyrrole (Exemplification compound number 1-368)

The title compound was synthesized in a yield of 91% as a pale brownish powder by conducting a reaction similar to that mentioned in Example 5, using ethylamine instead of methylamine.
Melting point: 228-230°C
$^1$H-NMR (500MHz, DMSO-$d_6$) δppm:
11.36 (1H, s), 8.46 (2H, dd, J=4Hz, 2Hz), 8.36 (1H, t, J=6Hz), 7.75 (2H, d, J=8Hz), 7.29 (2H, d, J=8Hz), 7.18-7.09 (6H, m), 6.91 (1H, d, J=3Hz), 5.25 (1H, s), 3.29-3.23 (2H, m), 2.92 (2H, d, J=2Hz), 2.78 (2H, t, J=8Hz), 2.58-2.52 (4H, m), 2.15 (2H, s), 1.11 (3H, t, J=7Hz).

(Example 7)

4-[1-[4-(N-Cyclopropylcarbamoyl)phenethyl]-1,2,3,6-tetrahydropyridin-4-yl]-2-(4-fluorophenyl)-3-(pyridin-4-yl)-1H-pyrrole (Exemplification compound number 1-374)

The title compound was synthesized in a yield of 96% as a pale brownish powder by conducting a reaction similar to that mentioned in Example 5, using cyclopropylamine instead of methylamine.
Melting point: 226-228°C
$^1$H-NMR (400MHz, DMSO-$d_6$) δppm:
11.37 (1H, br.s), 8.45 (2H, d, J=6Hz), 8.34 (1H, d, J=5Hz), 7.72 (2H, d, J=8Hz), 7.27 (2H, d, J=8Hz), 7.20-7.07 (6H, m), 6.90 (1H, d, J=3Hz), 5.28-5.22 (1H, m), 2.95-2.88 (2H, m), 2.87-2.72 (3H, m), 2.61-2.48 (4H, m), 2.19-2.10 (2H, m), 0.71-0.65 (2H, m), 0.58-0.52 (2H, m).

(Example 8)

2-(4-Fluorophenyl)-4-[1-[4-(N-isopropylcarbamoyl)phenethyl]-1,2,3,6-tetrahydropyridin-4-yl]-3-(pyridin-4-yl)-1H-pyrrole (Exemplification compound number 1-370)

The title compound was synthesized in a yield of 94% as a pale brownish powder by conducting a reaction similar to that mentioned in Example 5, using isopropylamine instead of methylamine.
Melting point: 178-180°C
$^1$H-NMR (400MHz, DMSO-$d_6$) δppm:
11.38 (1H, br.s), 8.45 (2H, d, J=6Hz), 8.11 (1H, d, J=8Hz), 7.75 (2H, d, J=8Hz), 7.28 (2H, d, J=8Hz), 7.22-7.06 (6H, m), 6.90 (1H, d, J=3Hz), 5.28-5.22 (1H, m), 4.14-4.01 (1H, m), 2.98-2.88 (2H, m), 2.82-2.72 (2H, m), 2.62-2.48 (4H, m), 2.21-2.10 (2H, m), 1.15 (6H, d, J=7Hz).

(Example 9)

2-(4-Fluorophenyl)-4-[1-[4-(N-propylcarbamoyl)phenethyl]-1,2,3,6-tetrahydropyridin-4-yl]-3-(pyridin-4-yl)-1H-pyrrole (Exemplification compound number 1-369)

The title compound was synthesized in a yield of 83% as a white powder by conducting a reaction similar to that mentioned in Example 5, using propylamine instead of methylamine.
Melting point: 227-230°C
$^1$H-NMR (400MHz, DMSO-$d_6$) δppm:
11.37 (1H, br.s), 8.45 (2H, d, J=6Hz), 8.36 (1H, t, J=6Hz), 7.74 (2H, d, J=8Hz), 7.29 (2H, d, J=8Hz), 7.19-7.08 (6H, m), 6.90 (1H, d, J=2Hz), 5.25 (1H, s), 3.24-3.16 (2H, m), 2.95-2.89 (2H, m), 2.82-2.74 (2H, m), 2.60-2.52 (4H, m), 2.19-2.12 (2H, m), 1.57-1.46 (2H, m), 0.88 (3H, t, J=7Hz).

(Example 10)

4-[1-[4-[N-(2-Fluoroethyl)carbamoyl]phenethyl]-1,2,3,6-tetrahydropyridin-4-yl]-2-(4-fluorophenyl)-3-(pyridin-4-yl)-1H-pyrrole (Exemplification compound number 1-389)

The title compound was synthesized in a yield of 78% as a white powder by conducting a reaction similar to that mentioned in Example 5, using 2-fluoroethylamine instead of methylamine.
Melting point: 209-211°C
$^1$H-NMR (400MHz, DMSO-$d_6$) δppm:
11.37 (1H, br.s), 8.61 (1H, t, J=6Hz), 8.45 (2H, d, J=6Hz), 7.78 (2H, d, J=8Hz), 7.31 (2H, d, J=8Hz), 7.18-7.09 (6H, m), 6.90 (1H, d, J=2Hz), 5.25 (1H, s), 4.59 (1H, t, J=5Hz), 4.47 (1H, t, J=5Hz), 3.58 (1H, dd, J=11Hz, 5Hz), 3.52 (1H, dd, J=11Hz, 5Hz), 2.93-2.88 (2H, m), 2.80-2.73 (2H, m), 2.58-2.50 (4H, m), 2.16-2.10 (2H, m).

(Example 11)

4-[1-[4-(N-Cyclohexylcarbamoyl)phenethyl]-1,2,3,6-tetrahydropyridin-4-yl]-2-(4-fluorophenyl)-3-(pyridin-4-yl)-1H-pyrrole (Exemplification compound number 1-377)

The title compound was synthesized in a yield of 94% as a pale brownish powder by conducting a reaction similar to that mentioned in Example 5, using cyclohexylamine instead of methylamine.
Melting point: 243-245°C
$^1$H-NMR (400MHz, DMSO-$d_6$) δppm:
11.37 (1H, s), 8.46 (2H, d, J=6Hz), 8.09 (1H, d, J=8Hz), 7.75 (2H, d, J=8Hz), 7.28 (2H, d, J=8Hz), 7.18-7.09 (6H, m), 6.90 (1H, d, J=3Hz), 5.25 (1H, s), 3.75 (1H, br.s), 2.92 (2H, s), 2.78 (2H, t, J=8Hz), 2.58-2.52 (4H, m), 2.15 (2H, br.s), 1.80 (2H, br.s), 1.73 (2H, br.s), 1.59 (2H, br.s), 1.29 (4H, t, J=10Hz).

(Example 12)

4-[1-[4-(N-Cyclopentylcarbamoyl)phenethyl]-1,2,3,6-tetrahydropyridin-4-yl]-2-(4-fluorophenyl)-3-(pyridin-4-yl)-1H-pyrrole (Exemplification compound number 1-376)

The title compound was synthesized in a yield of 95% as a pale brownish powder by conducting a reaction similar to that mentioned in Example 5, using cyclopentylamine instead of methylamine.
Melting point: 192-195°C
$^1$H-NMR (400MHz, DMSO-$d_6$) δppm:
11.37 (1H, s), 8.46 (2H, d, J=6Hz), 8.17 (1H, d, J=7Hz), 7.75 (2H, d, J=8Hz), 7.29 (2H, d, J=9Hz), 7.15-7.09 (6H, m), 6.90 (1H, s), 5.25 (1H, s), 4.24-4.19 (1H, m), 2.92 (2H, s), 2.78 (2H, t, J=7Hz), 2.58-2.54 (4H, m), 2. 15 (2H, br.s), 1.87 (2H, br.s), 1.69 (2H, br.s), 1.52 (4H, br.s).

(Example 13)

4-[1-[4-[N-[(1S)-Ethoxycarbonylethyl]carbamoyl]phenethyl]-1,2,3,6-tetrahydropyridin-4-yl]-2-(4-fluorophenyl)-3-(pyridin-4-yl)-1H-pyrrole (Exemplification compound number 1-397)

The title compound was synthesized in a yield of 86% as a white powder by conducting a reaction similar to that mentioned in Example 5, using L-alanine ethyl ester instead of methylamine.
Melting point: 169-172°C (degradation)
$^1$H-NMR (400MHz, DMSO-$d_6$) δppm:
11.36 (1H, br.s), 8.66 (1H, d, J=7Hz), 8.45 (2H, d, J=6Hz), 7.79 (2H, d, J=8Hz), 7.32 (2H, d, J=8Hz), 7.18-7.06 (6H, m), 6.90 (1H, d, J=2Hz), 5.25 (1H, s), 4.47-4.40 (1H, m), 4.14-4.06 (2H, m), 2.93-2.88 (2H, m), 2.80-2.73 (2H, m), 2.59-2.50 (4H, m), 2.16-2.10 (2H, m), 1.39 (3H, d, J=7Hz), 1.18 (3H, t, J=7Hz).

(Example 14)

4-[1-[4-[N-(Ethoxycarbonylmethyl)carbamoyl]phenethyl]-1,2,3,6-tetrahydropyridin-4-yl]-2-(4-fluorophenyl)-3-(pyridin-4-yl)-1H-pyrrole (Exemplification compound number 1-396)

The title compound was synthesized in a yield of 94% as a white powder by conducting a reaction similar to

that mentioned in Example 5, using glycine ethyl ester instead of methylamine.
Melting point: 170-173°C (degradation)
$^1$H-NMR (400MHz, DMSO-$d_6$) δppm:
11.37 (1H, br.s), 8.82 (1H, t, J=6Hz), 8.45 (2H, d, J=7Hz), 7.78 (2H, d, J=8Hz), 7.33 (2H, d, J=8Hz), 7.18-7.06 (6H, m), 6.90 (1H, d, J=2Hz), 5.26 (1H, s), 4.12 (2H, quartet, 7Hz), 3.98 (2H, d, J=6Hz), 2.93-2.88 (2H, m), 2.80-2.73 (2H, m), 2.58-2.50 (4H, m), 2.16-2.10 (2H, m), 1.20 (3H, t, J=7Hz).

(Example 15)

4-[1-[4-[2-(N,N-Dimethylamino)ethoxycarbonyl]phenethyl]-1,2,3,6-tetrahydropyridin-4-yl]-2-(4-fluorophenyl)-3-(pyridin-4-yl)-1H-pyrrole (Exemplification compound number 1-1338)

To a solution of 4-[1-(4-carboxyphenethyl)-1,2,3,6-tetrahydropyridin-4-yl]-2-(4-fluorophenyl)-3-(pyridin-4-yl)-1H-pyrrole (500 mg, 1.07 mmol) obtained in Example 4 in dimethylformamide (10 ml) was added carbonyldiimidazole (347 mg, 2.14 mmol) under stirring, and the resulting mixture was stirred at room temperature for 30 minutes. Furthermore, to the reaction mixture was added 2-(N,N-dimethylamino)ethanol (0.21 ml, 2.14 mmol), and the resulting mixture was stirred at 60°C for 2 days. After stirring, water (50 ml) was added to the reaction mixture, and the solid precipitated was collected by filtration and washed with diethyl ether to afford the title compound (433 mg) in a yield of 75% as a white powder.
Melting point: 178-181°C (degradation)
$^1$H-NMR (400MHz, DMSO-$d_6$) δppm:
11.36 (1H, br.s), 8.45 (2H, d, J=6Hz), 7.86 (2H, d, J=8Hz), 7.36 (2H, d, J=8Hz), 7.18-7.09 (6H, m), 6.90 (1H, d, J=2Hz), 5.25 (1H, s), 4.34 (2H, t, J=6Hz), 2.93-2.88 (2H, m), 2.83-2.77 (2H, m), 2.61-2.50 (6H, m), 2.21 (6H, s), 2.16-2.11 (2H, m).

(Example 16)

4-[1-(3-Carboxyphenethyl)-1,2,3,6-tetrahydropyridin-4-yl]-2-(4-fluorophenyl)-3-(pyridin-4-yl)-1H-pyrrole (Exemplification compound number 3-2)

4-[1-(3-Ethoxycarbonylphenethyl)-1,2,3,6-tetrahydropyridin-4-yl]-2-(4-fluorophenyl)-3-(pyridin-4-yl)-1H-pyrrole was synthesized by conducting a reaction similar to that mentioned in Example 1, using 3-ethoxycarbonylphenethyl bromide instead of 4-ethoxycarbonylphenethyl bromide. Subsequently, this ester form was hydrolyzed according to the similar methods mentioned in Example 4 to afford the title compound in a yield of 83% as a pale brownish powder.
Melting point: 256-260°C (degradation)
$^1$H-NMR (400MHz, DMSO-$d_6$) δppm:
11.38 (1H, s), 8.45 (2H, d, J=6Hz), 7.80-7.75 (2H, m), 7.47 (1H, d, J=7Hz), 7.39 (1H, t, J=8Hz), 7.18-7.08 (6H, m), 6.91 (1H, s), 5.25 (1H, s), 2.95 (2H, s), 2.81 (2H, t, J=7Hz), 2.61-2.56 (4H, m), 2.17 (2H, s).

(Example 17)

4-[1-(3-Carbamoylphenethyl)-1,2,3,6-tetrahydropyridin-4-yl]-2-(4-fluorophenyl)-3-(pyridin-4-yl)-1H-pyrrole (Exemplification compound number 3-4)

The title compound was synthesized in a yield of 69% as a brown powder by conducting a reaction similar to that mentioned in Example 1, using 3-carbamoylphenethyl bromide instead of 4-ethoxycarbonylphenethyl bromide.
Melting point: 185-188°C
$^1$H-NMR (400MHz, DMSO-$d_6$) δppm:
11.37 (1H, s), 8.46 (2H, d, J=6Hz), 7.91 (1H, s), 7.73 (1H, s), 7.69 (1H, d, J=7Hz), 7.36-7.30 (3H, m), 7.18-7.09 (6H, m), 6.91 (2H, d, J=2Hz), 5.26 (1H, s), 2.94 (1H, s), 2.78 (2H, t, J=7Hz), 2.59-2.54 (4H, m), 2.16 (2H, br.s).

(Example 18)

2-(4-Fluorophenyl)-3-(pyridin-4-yl)-4-[1-(4-sulfamoylphenethyl)-1,2,3,6-tetrahydropyridin-4-yl]-1H-pyrrole (Exemplification compound number 1-435)

The title compound was synthesized in a yield of 25% as a yellow powder by conducting a reaction similar to that mentioned in Example 1, using 4-sulfamoylphenethyl bromide instead of 4-ethoxycarbonylphenethyl bromide.
Melting point: 220-221°C

$^1$H-NMR (400MHz, DMSO-$d_6$) δppm:
11.36 (1H, br.s), 8.45 (2H, d, J=6Hz), 7.72 (2H, d, J=8Hz), 7.40 (2H, d, J=8Hz), 7.26 (2H, br.s), 7.20-7.06 (6H, m), 6.90 (1H, d, J=2Hz), 5.26 (1H, br.s), 2.96-2.88 (2H, br.s), 2.81 (2H, t, J=8Hz), 2.61-2.52 (4H, m), 2.19-2.10 (2H, br.s).

(Example 19)

2-(4-Fluorophenyl)-4-[1-[4-(N-methylsulfamoyl)phenethyl]-1,2,3,6-tetrahydropyridin-4-yl]-3-(pyridin-4-yl)-1H-pyrrole monohydrochloride (Exemplification compound number 1-436)

2-(4-Fluorophenyl)- 4-[1-[4-(N-methylsulfamoyl) phenethyl]- 1,2,3,6-tetrahydropyridin- 4-yl]- 3-(pyridin- 4-yl)-1H-pyrrole was synthesized by conducting a reaction similar to that mentioned in Example 1, using 4-(N-methylsulfamoyl) phenethyl bromide instead of 4-ethoxycarbonylphenethyl bromide. Subsequently, this product was suspended in ethanol and one molar equivalent of 1N hydrochloric acid solution was added to the suspended solution. After evaporation of the solvent, the residue was washed with ethanol to afford the title compound in a yield of 60% as a pale yellowish powder.
Melting point: 222-223°C
$^1$H-NMR (500MHz, DMSO-$d_6$) δppm of the free form:
11.40-11.35 (1H, br.s), 8.45 (2H, d, J=6Hz), 7.67 (2H, d, J=8Hz), 7.67 (2H, d, J=8Hz), 7.39-7.33 (1H,m), 7.18-7.08 (6H, m), 6.92-6.89 (1H, m), 5.28-5.24 (1H, m), 2.95-2.90 (2H, m), 2.83 (2H, t, J=8Hz), 2.60 (2H, t, J=8Hz), 2.56-2.52 (2H, m), 2.39 (3H, s), 2.19-2.12 (2H, m).

(Example 20)

4-[1-[4-(N-Ethylsulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-437)

The title compound was synthesized in a yield of 60% as a pale yellowish powder by conducting a reaction similar to that mentioned in Example 19, using 4-(N-ethylsulfamoyl)phenethyl bromide instead of 4-(N-methylsulfamoyl) phenethyl bromide.
Melting point: 260-261°C (degradation)
$^1$H-NMR (500MHz, DMSO-$d_6$) δppm of the free form:
11.39-11.34 (1H, br.s), 8.45 (2H, d, J=6Hz), 7.68 (2H, d, J=8Hz), 7.49-7.41 (3H, m), 7.19-7.08 (6H, m), 6.90 (1H, d, J=2Hz), 5.27-5.23 (1H, m), 2.95-2.90 (2H, m), 2.82 (2H, t, J=8Hz), 2.78-2.71 (2H, m), 2.58 (2H, t, J=8Hz), 2.54 (2H, t, J=6Hz), 2.18-2.12 (2H, m), 0.96 (3H, t, J=8Hz).

(Example 21)

2-(4-Fluorophenyl)-4-[1-[4-(N-propylsulfamoyl)phenethyl]-1,2,3,6-tetrahydropyridin-4-yl]-3-(pyridin-4-yl)-1H-pyrrole monohydrochloride (Exemplification compound number 1-438)

The title compound was synthesized in a yield of 59% as a pale yellowish powder by conducting a reaction similar to that mentioned in Example 19, using 4-(N-propylsulfamoyl)phenethyl bromide instead of 4-(N-methylsulfamoyl) phenethyl bromide.
Melting point: 253-254°C (degradation)
$^1$H-NMR (500MHz, DMSO-$d_6$) δppm of the free form:
11.39-11.35 (1H, br.s), 8.45 (2H, d, J=6Hz), 7.68 (2H, d, J=8Hz), 7.51-7.46 (1H, m), 7.43 (2H, d, J=8Hz), 7.19-7.08 (6H, m), 6.90 (1H, d, J=2Hz), 5.27-5.23 (1H, m), 2.96-2.89 (2H, m), 2.82 (2H, t, J=8Hz), 2.70-2.63 (2H, m), 2.58 (2H, t, J=8Hz), 2.54 (2H, t, J=6Hz), 2.19-2.12 (2H, m), 1.40-1.31 (2H, m), 0.78 (3H, t, J=8Hz) .

(Example 22)

2-(4-Fluorophenyl)-4-[1-[4-(N-isopropylsulfamoyl)phenethyl]-1,2,3,6-tetrahydropyridin-4-yl]-3-(pyridin-4-yl)-1H-pyrrole monohydrochloride (Exemplification compound number 1-439)

The title compound was synthesized in a yield of 40% as a pale yellowish powder by conducting a reaction similar to that mentioned in Example 19, using 4-(N-isopropylsulfamoyl)phenethyl bromide instead of 4-(N-methylsulfamoyl)phenethyl bromide.
Melting point: 178-180°C (degradation)
$^1$H-NMR (500MHz, DMSO-$d_6$) δppm of the free form:

11.39-11.34 (1H, br.s), 8.45 (2H, d, J=6Hz), 7.70 (2H, d, J=8Hz), 7.50-7.45 (1H, m), 7.42 (2H, d, J=8Hz), 7.19-7.08 (6H, m), 6.90 (1H, d, J=2Hz), 5.27-5.23 (1H, m), 3.25-3.16 (1H, m), 2.94-2.89 (2H, m), 2.82 (2H, t, J=8Hz), 2.58 (2H, t, J=8Hz), 2.54 (2H, t, J=6Hz), 2.18-2.12 (2H, m), 0.92 (6H, d, J=6Hz).

(Example 23)

2-(3-Chloro-4-fluorophenyl)-4-[1-[4-(N-methylsulfamoyl)phenethyl]-1,2,3,6-tetrahydropyridin-4-yl]-3-(pyridin-4-yl)-1H-pyrrole monohydrochloride (Exemplification compound number 1-555)

The title compound was synthesized in a yield of 45% as a brown powder by conducting a reaction similar to that mentioned in Example 19, using 2-(3-chloro-4-fluorophenyl)-3-(pyridin-4-yl)-4-(1,2,3,6-tetrahydropyridin-4-yl]-1H-pyrrole instead of 2-(4-fluorophenyl)-3-(pyridin-4-yl)-4-(1,2,3,6-tetrahydropyridin-4-yl)-1H-pyrrole.
Melting point: 183-185°C
$^1$H-NMR (400MHz, DMSO-$d_6$) δppm of the free form:
11.49 (1H, s), 8.50 (2H, d, J=6Hz), 7.68 (2H, d, J=8Hz), 7.46 (2H, d, J=9Hz), 7.36-7.29 (3H, m), 7.17 (2H, d, J=6Hz), 7.05-7.01 (1H, m), 6.97 (1H, d, J=3Hz), 5.25 (1H, s), 2.89 (2H, br.s), 2.83 (2H, t, J=8Hz), 2.62-2.53 (4H, m), 2.39 (3H, d, J=5Hz), 2.16 (2H, br.s) .

(Example 24)

4-[1-[4-(N-Cyclopropylsulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-443)

The title compound was synthesized in a yield of 55% as a pale yellowish powder by conducting a reaction similar to that mentioned in Example 19, using 4-(N-cyclopropylsulfamoyl)phenethyl bromide instead of 4-(N-methylsulfamoyl)phenethyl bromide.
Melting point: 261-262°C
$^1$H-NMR (500MHz, DMSO-$d_6$) δppm of the free form:
11.39-11.34 (1H, br.s), 8.45 (2H, t, J=6Hz), 7.84-7.79 (1H, m), 7.71 (2H, d, J=8Hz), 7.45 (2H, d, J=8Hz), 7.18-7.08 (6H, m), 6.90 (1H, d, J=3Hz), 5.27-5.23 (1H, m), 2.95-2.90 (2H, m), 2.83 (2H, t, J=8Hz), 2.59 (2H, t, J=8Hz), 2.54 (2H, t, J=6Hz), 2.18-2.12 (2H, m), 2.09-2.03 (1H, m), 0.48-0.43 (2H, m), 0.38-0.33 (2H, m).

(Example 25)

4-[1-[4-[N-(2-Fluoroethyl)sulfamoyl]phenethyl]-1,2,3,6-tetrahydropyridin-4-yl]-2-(4-fluorophenyl)-3-(pyridin-4-yl)-1H-pyrrole monohydrochloride (Exemplification compound number 1-458)

The title compound was synthesized in a yield of 42% as a pale yellowish powder by conducting a reaction similar to that mentioned in Example 19, using 4-[N-(2-fluoroethyl)sulfamoyl]phenethyl bromide instead of 4-(N-methylsulfamoyl)phenethyl bromide.
Melting point: 259-260°C (degradation)
$^1$H-NMR (500MHz, DMSO-$d_6$) δppm of the free form:
11.40-11.34 (1H, br.s), 8.45 (2H, d, J=6Hz), 7.90-7.82 (1H, m), 7.70 (2H, d, J=8Hz), 7.44 (2H, d, J=8Hz), 7.19-7.08 (6H, m), 6.90 (1H, d, J=2Hz), 5.27-5.23 (1H, m), 4.42 (1H, t, J=5Hz), 4.32 (1H, t, J=5Hz), 3.06 (1H, t, J=5Hz), 3.01 (1H, t, J=5Hz), 2.95-2.90 (2H, m), 2.83 (2H, t, J=8Hz), 2.58 (2H, t, J=8Hz), 2.54 (2H, t, J=6Hz), 2.19-2.12 (2H, m).

(Example 26)

2-(4-Fluorophenyl)-4-[1-[4-[N-(phenylsulfamoyl)phenethyl]-1,2,3,6-tetrahydropyridin-4-yl]-3-(pyridin-4-yl)-1H-pyrrole monohydrochloride (Exemplification compound number 1-451)

The title compound was synthesized in a yield of 52% as a pale yellowish powder by conducting a reaction similar to that mentioned in Example 19, using 4-(N-phenylsulfamoyl)phenethyl bromide instead of 4-(N-methylsulfamoyl) phenethyl bromide.
Melting point: 210-211°C (degradation)
$^1$H-NMR (500MHz, DMSO-$d_6$) δppm of the free form:
11.39-11.34 (1H, br.s), 10.27-10.16 (1H, br.s), 8.44 (2H, d, J=6Hz), 7.65 (2H, d, J=8Hz), 7.38 (2H, d, J=8Hz), 7.24-7.05 (10H, m), 6.99 (1H, t, J=8Hz), 6.89 (1H, d, J=3Hz), 5.25-5.21 (1H, m), 2.92-2.86 (2H, m), 2.77 (2H, t, J=8Hz), 2.57-2.47

(4H, m), 2.16-2.09 (2H, m).

(Example 27)

2-(4-Fluorophenyl)-4-[1-[4-(N-methoxysulfamoyl)phenethyl]-1,2,3,6-tetrahydropyridin-4-yl]-3-(pyridin-4-yl)-1H-pyrrole monohydrochloride (Exemplification compound number 1-469)

The title compound was synthesized in a yield of 42% as a pale yellowish powder by conducting a reaction similar to that mentioned in Example 19, using 4-(N-methoxysulfamoyl)phenethyl bromide instead of 4-(N-methylsulfamoyl)phenethyl bromide.
Melting point: 247-248°C (degradation)
$^1$H-NMR (500MHz, DMSO-$d_6$) δppm of the free form:
11.40-11.32 (1H, br.s), 10.52-10.32 (1H, br), 8.45 (2H, d, J=6Hz), 7.73 (2H, d, J=8Hz), 7.47 (2H, d, J=8Hz), 7.19-7.08 (6H, m), 6.90 (1H, d, J=3Hz), 5.28-5.23 (1H, m), 3.65 (3H, s), 2.95-2.90 (2H, m), 2.84 (2H, t, J=8Hz), 2.59 (2H, t, J=8Hz), 2.55 (2H, t, J=6Hz), 2.18-2.12 (2H, m).

(Example 28)

4-[1-[4-(N,N-Dimethylsulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-442)

The title compound was synthesized in a yield of 25% as a white powder by conducting a reaction similar to that mentioned in Example 19, using 4-(N,N-dimethylsulfamoyl)phenethyl bromide instead of 4-(N-methylsulfamoyl) phenethyl bromide.
Melting point: 274-275°C (degradation)
$^1$H-NMR (500MHz, DMSO-$d_6$) δppm of the free form:
11.40-11.35 (1H, br.s), 8.45 (2H, d, J=6Hz), 7.65 (2H, d, J=8Hz), 7.50 (2H, d, J=8Hz), 7.19-7.08 (6H, m), 6.90 (1H, d, J=2Hz), 5.28-5.24 (1H, m), 2.96-2.91 (2H, m), 2.85 (2H, t, J=8Hz), 2.60 (2H, t, J=8Hz), 2.58 (6H, s), 2.55 (2H, t, J=6Hz), 2.19-2.13 (2H, m).

(Example 29)

2-(4-Fluorophenyl)-4-[1-[4-[N-(2-methoxyethyl)sulfamoyl]phenethyl]-1,2,3,6-tetrahydropyridin-4-yl]-3-(pyridin-4-yl)-1H-pyrrole monohydrochloride (Exemplification compound number 1-459)

The title compound was synthesized in a yield of 43% as a pale yellowish powder by conducting a reaction similar to that mentioned in Example 19, using 4-[N-(2-methoxyethyl)sulfamoyl]phenethyl bromide instead of 4-(N-methylsulfamoyl)phenethyl bromide.
Melting point: 235-236°C (degradation)
$^1$H-NMR (500MHz, DMSO-$d_6$) δppm of the free form:
11.39-11.34 (1H, br.s), 8.45 (2H, d, J=6Hz), 7.69 (2H, d, J=8Hz), 7.65-7.60 (1H, m), 7.43 (2H, d, J=8Hz), 7.18-7.08 (6H, m), 6.90 (1H, d, J=2Hz), 5.27-5.23 (1H, m), 3.28 (2H, t, J=6Hz), 3.14 (3H, s), 2.94-2.90 (2H, m), 2.88 (2H, t, J=6Hz), 2.82 (2H, t, J=8Hz), 2.58 (2H, t, J=8Hz), 2.54 (2H, t, J=6Hz), 2.18-2.12 (2H, m).

(Example 30)

2-(4-Fluorophenyl)-4-[1-[4-[N-(2-hydroxyethyl)sulfamoyl]phenethyl]-1,2,3,6-tetrahydropyridin-4-yl]-3-(pyridin-4-yl)-1H-pyrrole monohydrochloride (Exemplification compound number 1-454)

The title compound was synthesized in a yield of 27% as a pale yellowish powder by conducting a reaction similar to that mentioned in Example 19, using 4-[N-(2-hydroxyethyl)sulfamoyl]phenethyl bromide instead of 4-(N-methylsulfamoyl)phenethyl bromide.
Melting point: 270-271°C (degradation)
$^1$H-NMR (500MHz, DMSO-$d_6$) δppm of the free form:
11.39-11.35 (1H, br.s), 8.45 (2H, d, J=6Hz), 7.69 (2H, d, J=8Hz), 7.54-7.48 (1H, m), 7.43 (2H, d, J=8Hz), 7.19-7.08 (6H, m), 6.92-6.89 (1H, m), 5.27-5.24 (1H, m), 4.69-4.65 (1H, m), 3.38-3.33 (2H, m), 2.95-2.90 (2H, m), 2.83 (2H, t, J=8Hz), 2.76 (2H, t, J=7Hz), 2.58 (2H, t, J=8Hz), 2.55 (2H, t, J=6Hz), 2.19-2.12 (2H, m).

(Example 31)

4-[1-[4-(2,2-Dimethylhydrazinosulfonyl)phenethyl]-1,2,3,6-tetrahydropyridin-4-yl]-2-(4-fluorophenyl)-3-(pyridin-4-yl)-1H-pyrrole monohydrochloride (Exemplification compound number 1-476)

The title compound was synthesized in a yield of 9% as a pale yellowish powder by conducting a reaction similar to that mentioned in Example 19, using 4-(2,2-dimethylhydrazinosulfonyl)phenethyl bromide instead of 4-(N-methylsulfamoyl)phenethyl bromide.
Melting point: 153-155°C (degradation)
$^1$H-NMR (500MHz, DMSO-$d_6$) δppm of the free form:
11.39-11.35 (1H, br.s), 8.57 (1H, s), 8.45 (2H, d, J=6Hz), 7.72 (2H, d, J=8Hz), 7.44 (2H, d, J=8Hz), 7.18-7.08 (6H, s), 6.90 (1H, d, J=3Hz), 5.26-5.23 (1H, m), 2.95-2.89 (2H, m), 2.87-2.80 (2H, m), 2.63-2.49 (4H, m), 2.22 (6H, s), 2.19-2.12 (2H, m).

(Example 32)

2-(4-Fluorophenyl)-3-(pyridin-4-yl)-4-[1-[4-[N-(pyridin-3-yl)methylsulfamoyl]phenethyl]-1,2,3,6-tetrahydropyridin-4-yl]-1H-pyrrole monohydrochloride (Exemplification compound number 1-453)

The title compound was synthesized in a yield of 27% as a pale yellowish powder by conducting a reaction similar to that mentioned in Example 19, using 4-[N-(pyridin-3-yl)methylsulfamoyl]phenethyl bromide instead of 4-(N-methylsulfamoyl)phenethyl bromide.
Melting point: 254-255°C (degradation)
$^1$H-NMR (500MHz, DMSO-$d_6$) δppm of the free form:
11.40-11.35 (1H, br.s), 8.46 (2H, d, J=6Hz), 8.41 (1H, d, J=3Hz), 8.20-8.13 (1H, m), 7.69 (2H, d, J=8Hz), 7.61 (1H, d, J=8Hz), 7.41 (2H, d, J=8Hz), 7.28 (1H, dd, J=8Hz, 3Hz), 7.19-7.08 (7H, m), 6.91 (1H, d, J=3Hz), 5.28-5.25 (1H, m), 4.02 (2H, s), 2.96-2.91 (2H, m), 2.82 (2H, t, J=8Hz), 2.58 (2H, t, J=8Hz), 2.56 (2H, t, J=6Hz), 2.20-2.13 (2H, m).

(Example 33)

4-[1-[4-[N-(2-Dimethylaminoethyl)sulfamoyl]phenethyl]-1,2,3,6-tetrahydropyridin-4-yl]-2-(4-fluorophenyl)-3-(pyridin-4-yl)-1H-pyrrole dihydrochloride (Exemplification compound number 1-463)

The title compound was synthesized in a yield of 37% as a pale yellowish powder by conducting a reaction similar to that mentioned in Example 19, using 4-[N-(2-dimethylaminoethyl)sulfamoyl]phenethyl bromide instead of 4-(N-methylsulfamoyl)phenethyl bromide (however, 2 molar equivalents of 1N hydrochloric acid were used).
Melting point: 197-198°C (degradation)
$^1$H-NMR (500MHz, DMSO-$d_6$) δppm of the free form:
8.49 (2H, d, J=6Hz), 8.27-8.22 (1H, br.s), 7.80 (2H, d, J=8Hz), 7.35 (2H, d, J=8Hz), 7.28-7.24 (1H, br.s), 7.19 (2H, d, J=6Hz), 7.14 (2H, dd, J=9Hz, 4Hz), 6.98 (2H, t, J=9Hz), 6.85 (1H, d, J=3Hz), 5.50-5.46 (1H, m), 3.11-3.06 (2H, m), 2.97 (2H, t, J=6Hz), 2.94-2.89 (2H, m), 2.71-2.62 (4H, m), 2.34-2.27 (4H, m), 2.07 (6H, s).

(Example 34)

4-[1-[4-(N-(1,3-Dihydroxypropan-2-yl)sulfamoyl))phenethyl]-1,2,3,6-tetrahydropyridin-4-yl]-2-(4-fluorophenyl)-3-(pyridin-4-yl)-1H-pyrrole monohydrochloride (Exemplification compound number 1-455)

The title compound was synthesized in a yield of 35% as a pale yellowish powder by conducting a reaction similar to that mentioned in Example 19, using 4-[N-(1,3-dihydroxypropan-2-yl)sulfamoyl]phenethyl bromide instead of 4-(N-methylsulfamoyl)phenethyl bromide.
Melting point: 261-262°C (degradation)
$^1$H-NMR (500MHz, DMSO-$d_6$) δppm of the free form:
11.39-11.34 (1H, br.s), 8.45 (2H, d, J=6Hz), 7.73 (2H, d, J=8Hz), 7.40 (2H, d, J=8Hz), 7.38-7.31 (1H, m), 7.19-7.08 (6H, m), 6.91 (1H, d, J=2Hz), 5.28-5.24 (1H, m), 4.53 (2H, br.t, J=5Hz), 3.31-3.27 (4H, m), 3.06-2.99 (1H, m), 2.95-2.90 (2H, m), 2.82 (2H, t, J=8Hz), 2.58 (2H, t, J=8Hz), 2.55 (2H, t, J=6Hz), 2.19-2.12 (2H, m).

(Example 35)

4-[1-[4-(N-Ethoxysulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-470)

The title compound was synthesized in a yield of 49% as a pale yellowish powder by conducting a reaction similar to that mentioned in Example 19, using 4-(N-ethoxysulfamoyl)phenethyl bromide instead of 4-(N-methylsulfamoyl) phenethyl bromide.
Melting point: 217-218°C (degradation)
$^1$H-NMR (500MHz, DMSO-$d_6$) δppm of the free form:
11.40-11.33 (1H, br.s), 10.31-10.22 (1H, br.s), 8.45 (2H, d, J=6Hz), 7.74 (2H, d, J=8Hz), 7.47 (2H, d, J=8Hz), 7.19-7.07 (6H, m), 6.90 (1H, d, J=3Hz), 5.27-5.23 (1H, m), 3.89 (2H, quartet, J=7Hz), 2.96-2.89 (2H, m), 2.84 (2H, t, J=8Hz), 2.59 (2H, t, J=8Hz), 2.55 (2H, t, J=6Hz), 2.19-2.12 (2H, m), 1.09 (3H, t, J=7Hz).

(Example 36)

4-[1-[4-(N-Benzyloxysulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-473)

The title compound was synthesized in a yield of 9% as a pale yellowish powder by conducting a reaction similar to that mentioned in Example 19, using 4-(N-benzyloxysulfamoyl)phenethyl bromide instead of 4-(N-methylsulfamoyl)phenethyl bromide.
Melting point: 237-238°C (degradation)
$^1$H-NMR (500MHz, DMSO-$d_6$) δppm of the free form:
11.39-11.34 (1H, br.s), 10.44-10.37 (1H, br.s), 8.45 (2H, d, J=6Hz), 7.76 (2H, d, J=8Hz), 7.46 (2H, d, J=8Hz), 7.39-7.29 (5H, m), 7.19-7.08 (6H, m), 6.89 (1H, d, J=2Hz), 5.26-5.23 (1H, m), 4.88 (2H, s), 2.94-2.88 (2H, m), 2.83 (2H, t, J=8Hz), 2.58 (2H, t, J=8Hz), 2.54 (2H, t, J=6Hz), 2.17-2.11 (2H, m).

(Example 37)

4-[1-[4-(N-Allyloxysulfamoyl)phenethyl]-1,2,3,6-tetrahydropyridin-4-yl]-2-(4-fluorophenyl)-3-(pyridin-4-yl)-1H-pyrrole monohydrochloride (Exemplification compound number 1-472)

The title compound was synthesized in a yield of 23% as a pale yellowish powder by conducting a reaction similar to that mentioned in Example 19, using 4-(N-allyloxysulfamoyl)phenethyl bromide instead of 4-(N-methylsulfamoyl)phenethyl bromide.
Melting point: 239-240°C (degradation)
$^1$H-NMR (500MHz, DMSO-$d_6$) δppm of the free form:
11.39-11.34 (1H, br.s), 10.40-10.32 (1H, br.s), 8.45 (2H, d, J=6Hz), 7.74 (2H, d, J=8Hz), 7.47 (2H, d, J=8Hz), 7.18-7.08 (6H, m), 6.90 (1H, d, J=2Hz), 5.91-5.81 (1H, m), 5.29-5.17 (3H, m), 4.35 (2H, d, J=6Hz), 2.95-2.90 (2H, m), 2.84 (2H, t, J=8Hz), 2.59 (2H, t, J=8Hz), 2.54 (2H, t, J=6Hz), 2.18-2.12 (2H, m).

(Example 38)

2-(4-Fluorophenyl)-4-[1-[4-(N-methylsulfamoyl)-β-oxophenethyl]-1,2,3,6-tetrahydropyridin-4-yl]-3-(pyridin-4-yl)-1H-pyrrole monohydrochloride (Exemplification compound number 1-1290)

2-(4-Fluorophenyl)- 4-[1-[4-(N-methylsulfamoyl)- β-oxophenethyl]- 1,2,3,6-tetrahydropyridin- 4-yl]- 3-(pyridin-4-yl)-1H-pyrrole was synthesized by conducting a reaction similar to that mentioned in Example 1, using α-bromo-4-(N-methylsulfamoyl)acetophenone instead of 4-ethoxycarbonylphenethyl bromide. This product was derived to its monohydrochloride salt by conducting a reaction similar to that mentioned in Example 19 to afford the title compound in a yield of 85% as a yellow powder.
Melting point: 223-224°C (degradation)
$^1$H-NMR (500MHz, DMSO-$d_6$) δppm of the free form:
11.40-11.35 (1H, br.s), 8.44 (2H, d, J=6Hz), 8.16 (2H, d, J=8Hz), 7.90 (2H, d, J=8Hz), 7.64 (1H, quartet, J=5Hz), 7.19-7.08 (6H, m), 6.91 (1H, d, J=3Hz), 5.27-5.23 (1H, m), 3.93 (2H, s), 3.04-2.99 (2H, m), 2.64 (2H, t, J=6Hz), 2.44 (3H, d, J=5Hz), 2.19-2.13 (2H, m).

(Example 39)

(±)-2-(4-Fluorophenyl)-4-[1-[β-hydroxy-4-(N-methylsulfamoyl)phenethyl]-1,2,3,6-tetrahydropyridin-4-yl]-3-(pyridin-4-yl)-1H-pyrrole monohydrochloride (Exemplification compound number 1-1284)

To a suspended solution of 2-(4-fluorophenyl)-4-[1-[4-(N-methylsulfamoyl)-β-oxophenethyl]-1,2,3,6-tetrahydropyridin-4-yl]-3-(pyridin-4-yl)-1H-pyrrole (free form) (763 mg, 1.44 mmol) obtained in Example 38 in a mixed solvent of methanol (15 ml) and tetrahydrofuran (15 ml) was added sodium borohydride (57 mg, 1.44 mmol) under stirring, and the resulting mixture was stirred at room temperature for one hour. After stirring, water (50 ml) was added to the reaction mixture, and the resulting mixture was extracted with ethyl acetate. The separated organic layer containing the desired compound was washed with water, dried over anhydrous sodium sulfate and evaporated in vacuo. The solid product thus obtained was washed with a small amount of ethyl acetate to afford the free form of the title compound. Subsequently, the free form obtained above was treated in a similar manner to that described in Example 19 to afford the title compound (monohydrochloride) (648 mg) in a yield of 79% as a pale yellow powder.
Melting point: 184-185°C (degradation)
$^1$H-NMR (500MHz, DMSO-$d_6$) δppm of the free form:
11.40-11.34 (1H, br.s), 8.45 (2H, d, J=6Hz), 7.72 (2H, d, J=8Hz), 7.55 (2H, d, J=8Hz), 7.38 (1H, quartet, J=5Hz), 7.19-7.09 (6H, m), 6.90 (1H, d, J=3Hz), 5.27 (1H, d, J=5Hz), 5.26-5.23 (1H, m), 4.83-4.77 (1H, m), 3.01-2.95 (2H, m), 2.64-2.52 (3H, m), 2.46 (1H, dd, J=13Hz, 5Hz), 2.39 (3H, d, J=5Hz), 2.17-2.11 (2H, m).

(Example 40)

2-(4-Fluorophenyl)4-[1-(4-methanesulfonylphenethyl)-1,2,3,6-tetrahydropyridin-4-yl]-3-(pyridin-4-yl)-1H-pyrrole (Exemplification compound number 1-426)

To a solution of 2-(4-fluorophenyl)-3-(pyridin-4-yl)-4-(1,2,3,6-tetrahydropyridin-4-yl)-1H-pyrrole (479 mg, 1.5 mmol) and 4-methanesulfonylphenylacetaldehyde (357 mg, 1.8 mmol) in methanol (10 ml) was added acetic acid (0.2 ml, 3.6 mmol) under stirring, and furthermore to the resulting mixture was added sodium borocyanohydride (226 mg, 3.6 mmol) under stirring and ice-cooling. The resulting mixture was stirred at the same temperature for 30 minutes and then at room temperature for 30 minutes. After stirring, the reaction mixture was concentrated under reduced pressure, and water was added to the residue. The solid product separated was collected by filtration and washed successively with diethyl ether and methanol to afford the title compound (291 mg) in a yield of 39% as a white powder.
Melting point: 239-241°C
$^1$H-NMR (500MHz, DMSO-$d_6$) δppm:
8.49 (2H, d, J=6Hz), 8.25 (1H, br.s), 7.87 (2H, d, J=8Hz), 7.42 (2H, d, J=9Hz), 7.19 (2H, d, J=6Hz), 7.14 (2H, dd, J=9, 5Hz), 6.98 (2H, t, J=9Hz), 6.85 (1H, d, J=3Hz), 5.48 (1H, br.s), 3.11-3.09 (2H, m), 3.05 (3H, s), 2.95 (2H, dd, J=9Hz, 6Hz), 2.72 (2H, dd, J=9Hz, 6Hz), 2.67 (2H, t, J=6Hz), 2.31 (2H, br.s).

(Example 41)

2-(3-Chloro-4-fluorophenyl)-4-[1-(4-methanesulfonylphenethyl)-1,2,3,6-tetrahydropyridin-4-yl]-3-(pyridin-4-yl)-1H-pyrrole monohydrochloride (Exemplification compound number 1-545)

2-(3-Chloro- 4-fluorophenyl)- 4-[1-(4-methanesulfonylphenethyl)- 1,2,3,6-tetrahydropyridin- 4-yl]- 3-(pyridin-4-yl)-1H-pyrrole was synthesized by conducting a reaction similar to that mentioned in Example 40, using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl)-1H-pyrrole. The product was derived to its monohydrochloride by conducting a reaction similar to that mentioned in Example 19 to afford the title compound in a yield of 61% as a pale yellowish powder.
Melting point: 225-230°C (degradation)
$^1$H-NMR (400MHz, DMSO-$d_6$) δppm of the free form:
11.49 (1H, s), 8.50 (2H, d, J=7Hz), 7.83 (2H, d, J=8Hz), 7.49 (2H, d, J=9Hz), 7.35-7.28 (2H, m), 7.16 (2H, d, J=6Hz), 7.05-7.01 (1H, m), 6.96 (1H, d, J=3Hz), 5.25 (1H, s), 3.18 (3H, s), 2.92 (2H, br.s), 2.85 (2H, t, J=7Hz),2.61-2.50 (4H, m), 2.15 (2H, br.s).

(Example 42)

(±)-2-(4-Fluorophenyl)-4-[1-(4-methanesulfonylphenethyl)-1,2,3,6-tetrahydropyridin-4-yl]-3-(pyridin-4-yl)-1H-pyrrole (Exemplification compound number 1-417)

The title compound was synthesized in a yield of 73% as a pale peach coloured powder by conducting a reaction similar to that mentioned in Example 1, using (±)-4-methanesulfonylphenethyl bromide instead of 4-ethoxycarbonylphenethyl bromide.
Melting point: 207-209°C (degradation)
$^1$H-NMR (400MHz, DMSO-$d_6$) δppm:
11.38 (1H, br.s), 8.45 (2H, d, J=6Hz), 7.58 (2H, d, J=8Hz), 7.43 (2H, d, J=8Hz), 7.20-7.07 (6H, m), 6.90 (1H, d, J=2Hz), 5.28-5.23 (1H, m), 2.97-2.88 (2H, m), 2.85-2.77 (2H, m), 2.71 (3H, s), 2.62-2.50 (4H, m), 2.20-2.12 (2H, m).

(Example 43)

2-(4-Fluorophenyl)-4-[1-(4-methanesulfonyl-β-oxophenethyl)-1,2,3,6-tetrahydropyridin-4-yl]-3-(pyridin-4-yl)-1H-pyrrole monohydrochloride (Exemplification compound number 1-1308)

2-(4-Fluorophenyl)-4-[1-(4-methanesulfonyl-β-oxophenethyl)-1,2,3,6-tetrahydropyridin-4-yl]-3-(pyridin-4-yl)-1H-pyrrole was synthesized by conducting a reaction similar to that mentioned in Example 1, using α-bromo-4-methanesulfonylacetophenone instead of 4-ethoxycarbonylphenethyl bromide. Subsequently the product thus obtained was formed to its monohydrochloride to afford the title compound in a yield of 75% as a pale yellowish powder by conducting a reaction similar to that mentioned in Example 19.
Melting point: 183-184°C (degradation)
$^1$H-NMR (500MHz, DMSO-$d_6$) δppm of the free form:
11.39-11.34 (1H, br.s), 8.44 (2H, d, J=6Hz), 8.19 (2H, d, J=8Hz), 8.06 (2H, d, J=8Hz), 7.19-7.07 (6H, m), 6.91 (1H, d, J=3Hz), 5.27-5.23 (1H, m), 3.94 (2H, s), 3.29 (3H, s), 3.04-2.99 (2H, m), 2.64 (2H, t, J=6Hz), 2.19-2.12 (2H, m).

(Example 44)

(±)-2-(4-Fluorophenyl)-4-[1-(β-hydroxy-4-methanesulfonylphenethyl)-1,2,3,6-tetrahydropyridin-4-yl]-3-(pyridin-4-yl)-1H-pyrrole monohydrochloride (Exemplification compound number 1-1302)

The title compound was synthesized in a yield of 77% as a pale yellowish powder by conducting a reaction similar to that mentioned in Example 39, using 2-(4-fluorophenyl)-4-[1-(4-methanesulfonyl-β-oxophenethyl)-1,2,3,6-tetrahydropyridin-4-yl]-3-(pyridin-4-yl)-1H-pyrrole (free form) that was obtained in Example 43.
Melting point: 271-272°C (degradation)
$^1$H-NMR (500MHz, DMSO-$d_6$) δppm of the free form:
11.40-11.34 (1H, br.s), 8.45 (2H, d, J=6Hz), 7.87 (2H, d, J=8Hz), 7.60 (2H, d, J=8Hz), 7.19-7.08 (6H, m), 6.90 (1H, d, J=3Hz), 5.32 (1H , d, J=4Hz), 5.26-5.23 (1H, m), 4.85-4.80 (1H, m), 3.19 (3H, s), 3.00-2.95 (2H, m), 2.65-2.43 (4H, m), 2.18-2.10 (2H, m).

(Example 45)

(±)-2-(3-Chloro-4-fluorophenyl)-4-[1-(4-methanesulfinylphenethyl)-1,2,3,6-tetrahydropyridin-4-yl]-3-(pyridin-4-yl)-1H-pyrrole (Exemplification compound number 1-536)

The title compound was synthesized in a yield of 37% as a pale pinkish powder by conducting a reaction similar to that mentioned in Example 1, using (±)-4-methanesulfinylphenethyl bromide instead of 4-ethoxycarbonylphenethyl bromide, and using 2-(3-chloro-4-fluorophenyl)-3-(pyridin-4-yl)-4-(1,2,3,6-tetrahydropyridin-4-yl]-1H-pyrrole instead of 2-(4-fluorophenyl)-3-(pyridin-4-yl)-4-(1,2,3,6-tetrahydropyridin-4-yl]-1H-pyrrole.
Melting point: 180-182°C (degradation)
$^1$H-NMR (400MHz, DMSO-$d_6$) δppm of the free form:
11.50 (1H, br.s), 8.49 (2H, d, J=6Hz), 7.58 (2H, d, J=8Hz), 7.42 (2H, d, J=8Hz), 7.36-7.27 (2H, m), 7.16 (2H, d, J=6Hz), 7.06-7.00 (1H, m), 6.90 (1H, d, J=2Hz), 5.25 (1H, s), 2.95-2.89 (2H, m), 2.84-2.77 (2H, m), 2.61-2.52 (4H, m), 2.71 (3H, s), 2.19-2.12 (2H, m) .

(Example 46)

(±)-2-(3-Chloro-4-fluorophenyl)-4-[1-(4-methanesulfinyl-β-oxophenethyl)-1,2,3,6-tetrahydropyridin-4-yl]-3-(pyridin-4-yl)-1H-pyrrole (Exemplification compound number 1-1326)

The title compound was synthesized in a yield of 16% as a pale brownish powder by conducting a reaction similar to that mentioned in Example 1, using (±)-α-bromo-4-methanesulfinylacetophenone instead of 4-ethoxycarbonylphenethyl bromide, and using 2-(3-chloro-4-fluorophenyl)-3-(pyridin-4-yl)-4-(1,2,3,6-tetrahydropyridin-4-yl)-1H-pyrrole instead of 4-ethoxycarbonylphenethyl bromide, and using 2-(3-chloro-4-fluorophenyl)-3-(pyridin-4-yl)-4-(1,2,3,6-tetrahydropyridin-4-yl]-1H-pyrrole instead of 2-(4-fluorophenyl)-3-(pyridin-4-yl)-4-(1,2,3,6-tetrahydropyridin-4-yl]-1H-pyrrole.
Melting point: 190-192°C (degradation)
$^1$H-NMR (500MHz, DMSO-$d_6$) δppm:
11.52-11.48 (1H, br.s), 8.48 (2H, d, J=6Hz), 8.14 (2H, d, J=8Hz), 7.81 (2H, d, J=8Hz), 7.36-7.28 (2H, m), 7.16 (2H, d, J=6Hz), 7.05-7.01 (1H, m), 6.96 (1H, d, J=3Hz), 5.26-5.23 (1H, m), 3.92 (2H, s), 3.02-2.99 (2H, m), 2.80 (3H, s), 2.64 (2H, t, J=6Hz), 2.18-2.13 (2H, m).

(Example 47)

4-[1-(4-Acetylphenethyl)-1,2,3,6-tetrahydropyridin-4-yl]-2-(3-chloro-4-fluorophenyl)-3-(pyridin-4-yl)-1H-pyrrole monohydrochloride (Exemplification compound number 1-527)

4-[1-(4-Acetylphenethyl)-1,2,3,6-tetrahydropyridin-4-yl]-2-(3-chloro-4-fluorophenyl)-3-(pyridin-4-yl)-1H-pyrrole was synthesized by conducting a reaction similar to that mentioned in Example 1, using 4-acetylphenethyl bromide instead of 4-ethoxycarbonylphenethyl bromide, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl]-1H-pyrrole. Subsequently the product thus obtained was derived to its monohydrochloride salt in a yield of 34% as a pale brownish powder by conducting a reaction similar to that mentioned in Example 19 to afford the title compound.
Melting point: 254-255°C (degradation)
$^1$H-NMR (500MHz, DMSO-$d_6$) δppm of the free form:
11.52-11.48 (1H, br.s), 8.49 (2H, d, J=6Hz), 7.87 (2H, d, J=8Hz), 7.39-7.28 (4H, m), 7.16 (2H, d, J=6Hz), 7.06-7.01 (1H, m), 6.97-6.94 (1H, m), 5.27-5.23 (1H, m), 2.94-2.90 (2H, m), 2.81 (2H, t, J=8Hz), 2.61-2.51 (7H, m), 2.18-2.12 (2H, m).

(Example 48)

2-(3-Chloro-4-fluorophenyl)-4-[(2S,8aS)-2-(4-methoxycarbonylphenyl)-1,2,3,5,6,8a-hexahydroindolizin-7-yl]-3-(pyridin-4-yl)-1H-pyrrole (Exemplification compound number 7-478)

a)
To a solution of 4-bromo-1-(t-butyl)diphenylsilyl-2-(3-chloro-4-fluorophenyl)-3-(pyridin-4-yl)-1H-pyrrole (3.24 g, 5.49 mmol) in tetrahydrofuran (65 ml) was added dropwise 1.57M solution of butyllithium in hexane (3.85 ml, 6.04 mmol) at -78°C under stirring, and the resulting mixture was stirred at the same temperature for 15 minutes. Subsequently, to the reaction mixture was slowly added dropwise a solution of (2S,8aS)-2-(4-methoxycarbonylphenyl)-1,2,3,5,6,7,8,8a-octahydroindolizin-7-one (1.50 g, 5.49 mmol), that was prepared separately, and lithium chloride (0.26 g, 6.04 mmol) in tetrahydrofuran (17 ml) at -78°C dropwise and slowly under stirring, and the resulting mixture was stirred at the same temperature for one hour.

b)
After stirring, methanesulfonic acid (0.78 ml, 12.08 mmol), dichloromethane (65 ml), pyridine (1.78 ml, 22 mmol), thionyl chloride (0.80 ml, 11 mmol), and methanol (6.5 ml, 22 mmol) were added successively to the reaction mixture at -78°C under stirring, and then the cooling bath was removed. The reaction mixture was stirred continuously until the temperature of the reaction mixture reached room temperature and was then evaporated in vacuo. Subsequently, to a solution of the residue in a mixed solvent of tetrahydrofuran (200 ml) and methanol (200 ml) was added 1M solution of tetrabutylammonium fluoride in tetrahydrofuran (55 ml, 55 mmol) under stirring, and the resulting mixture was stirred at room temperature for 15 minutes, and the solvent was evaporated in vacuo. To the residue were added ethyl acetate (100 ml) and water (50 ml), and the resulting mixture was partitioned to separate the organic layer, which was washed with water, dried over anhydrous sodium sulfate and concentrated under reduced pressure.

The residue was purified by chromatography on a silica gel column using a mixed solvent of ethyl acetate, methanol and isopropylamine (20 : 1 : 1) as the eluent to afford the title compound (1.13 g) in a overall yield of 39% as a brown powder.
$^1$H-NMR (500MHz, DMSO-$d_6$) δppm:
11.56-11.50 (1H, br.s), 8.50 (2H, d, J=6Hz), 7.87 (2H, d, J=8Hz), 7.42 (2H, d, J=8Hz), 7.36 (1H, d, J=8Hz), 7.32 (1H, t, J=9Hz), 7.18 (2H, d, J=6Hz), 7.08-7.03 (1H, m), 7.01 (1H, d, J=3Hz), 5.22-5.18 (1H, m), 3.83 (3H, s), 3.55-3.49 (1H, m), 3.47-3.39 (1H, m), 3.26-3.19 (1H, m), 2.92-2.85 (1H, m), 2.77-2.68 (1H, m), 2.64 (1H, dd, J=10Hz, 7Hz), 2.35-2.25 (1H, m), 2.11-2.03 (1H, m), 1.90-1.82 (1H, m), 1.80-1.72 (1H, m).

(Example 49)

4-[(2S,8aS)-2-(4-Carboxyphenyl)-1,2,3,5,6,8a-hexahydroindolizin-7-yl]-2-(3-chloro-4-fluorophenyl)-3-(pyridin-4-yl)-1H-pyrrole (Exemplification compound number 7-477)

The title compound was synthesized in a yield of 92% as a brown powder by conducting a reaction similar to that mentioned in Example 4, using 2-(3-chloro-4-fluorophenyl)-4-[(2S,8aS)-2-(4-methoxycarbonylphenyl)-1,2,3,5,6,8a-hexahydroindolizin-7-yl]-3-(pyridin-4-yl)-1H-pyrrole that was obtained in Example 48 instead of 4-[1-(4-ethoxycarbonylphenethyl)-1,2,3,6-tetrahydropyridin-4-yl]-2-(4-fluorophenyl)-3-(pyridin-4-yl)-1H-pyrrole.
$^1$H-NMR (500MHz, DMSO-$d_6$) δppm:
11.64-11.58 (1H, br.s), 8.51 (2H, d, J=6Hz), 7.88 (2H, d, J=8Hz), 7.42 (2H, d, J=8Hz), 7.37 (1H, d, J=8Hz), 7.32 (1H, t, J=9Hz), 7.21 (2H, d, J=6Hz), 7.09-7.02 (2H, m), 5.19-5.13 (1H, m), 3.86-3.74 (1H, m), 3.49 (1H, quintet, J=8Hz), 3.38 (1H, t, J=10Hz), 3.09-3.00 (1H, m), 2.97-2.84 (2H, m), 2.46-2.35 (1H, m), 2.27-2.16 (1H, m), 2.01-1.90 (1H, m), 1.87-1.77 (1H, m).

(Example 50)

2-(3-Chloro-4-fluorophenyl)-4-[(2S,8aS)-2-(4-carbamoylphenyl)-1,2,3,5,6,8a-hexahydroindolizin-7-yl]-3-(pyridin-4-yl)-1H-pyrrole (Exemplification compound number 7-485)

The title compound was synthesized in a yield of 71% as a brown powder by conducting a reaction similar to that mentioned in Example 5, using 4-[(2S,8aS)-2-(4-carboxyphenyl)-1,2,3,5,6,8a-hexahydroindolizin-7-yl]-2-(3-chloro-4-fluorophenyl)-3-(pyridin-4-yl)-1H-pyrrole that was obtained in Example 49 instead of 4-[1-(4-carboxyphenethyl)-1,2,3,6-tetrahydropyridin-4-yl]-2-(4-fluorophenyl)-3-(pyridin-4-yl)-1H-pyrrole, and using ammonia gas instead of methylamine.
Melting point: 215-217°C
$^1$H-NMR (500MHz, DMSO-$d_6$) δppm:
11.55-11.49 (1H, br.s), 8.49 (2H, d, J=6Hz), 7.90-7.84 (1H, br.s), 7.78 (2H, d, J=8Hz), 7.36 (1H, dd, J=7Hz, 2Hz), 7.34-7.28 (3H, m), 7.27-7.22 (1H, br.s), 7.18 (2H, d, J=6Hz), 7.07-7.02 (1H, m), 7.00 (1H, d, J=2Hz), 5.22-5.18 (1H, m), 3.54-3.47 (1H, m), 3.39 (1H, quintet, J=8Hz), 3.20 (1H, t, J=9Hz), 2.92-2.84 (1H, m), 2.75-2.67 (1H, m), 2.62 (1H, dd, J=10Hz, 8Hz), 2.34-2.25 (1H, m), 2.10-2.02 (1H, m), 1.89-1.81 (1H, m), 1.79-1.70 (1H, m).

(Example 51)

2-(3-Chloro-4-fluorophenyl)-4-[(2S,8aS)-2-[4-(N-methylcarbamoyl)phenyl]-1,2,3,5,6,8a-hexahydroindolizin-7-yl]-3-(pyridin-4-yl)-1H-pyrrole (Exemplification compound number 7-486)

The title compound was synthesized in a yield of 71% as a pale brownish powder by conducting a reaction similar to that mentioned in Example 50, using methylamine instead of ammonia gas.
Melting point: 176-178°C
$^1$H-NMR (500MHz, DMSO-$d_6$) δppm:
11.56-11.48 (1H, br.s), 8.49 (2H, d, J=6Hz), 8.35-8.31 (1H, m), 7.73 (2H, d, J=8Hz), 7.38-7.27 (4H, m), 7.17 (2H, d, J=6Hz), 7.02 (1H, m), 7.00 (1H, d, J=2Hz), 5.22-5.18 (1H, m), 3.54-3.46 (1H, m), 3.38 (1H, quintet, J=8Hz), 3.20 (1H, t, J=9Hz), 2.91-2.84 (1H, m), 2.76-2.67 (4H, m), 2.62- (1H, dd, J=10Hz, 3Hz), 2.34-2.24 (1H, m), 2.10-2.01 (1H, m), 1.89-1.81 (1H, m), 1.78-1.71 (1H, m).

(Example 52)

4-[(2S,8aS)-2-(4-Carbamoylphenyl)-1,2,3,5,6,8a-hexahydroindolidin-7-yl]-2-(4-fluorophenyl)-3-(pyridin-4-yl)-1H-pyrrole (Exemplification compound number 7-366)

4-[(2S, 8aS)- 2-(4-Carboxyphenyl)- 1,2,3,5,6,8a-hexahydroindolizin- 7-yl]- 2-(4-fluorophenyl)- 3-(pyridin- 4-yl)-1H-pyrrole was synthesized by conducting reactions similar to those mentioned in Example 48 and 49, using 4-bromo-1-(t-butyl)diphenylsilyl-2-(4-fluorophenyl)-3-pyridin-4-yl)-1H-pyrrole instead of 4-bromo-1-(t-butyl)diphenylsilyl-2-(3-chloro-4-fluorophenyl)-3-(pyridin-4-yl)-1H-pyrrole. Subsequently the title compound was synthesized in a yield of 66% as a pale brownish powder by conducting a reaction similar to that mentioned in Example 50, using this product thus obtained instead of 4-[(2S,8aS)-2-(4-carboxyphenyl)-1,2,3,5,6,8a-hexahydroindolizin-7-yl]-2-(3-chloro-4-fluorophenyl)-3-(pyridin-4-yl)-1H-pyrrole.
Melting point: 247-250°C
$^1$H-NMR (500MHz, DMSO-$d_6$) δppm:
11.42-11.36 (1H, br.s), 8.45 (2H, d, J=6Hz), 7.90-7.82 (1H, br.s), 7.77 (2H, d, J=8Hz), 7.32 (2H, d, J=8Hz), 7.26-7.20 (1H, br.s), 7.19-7.08 (6H, m), 6.95 (1H, d, J=3Hz), 5.22-5.19 (1H, m), 3.54-3.58 (1H, m), 3.44-3.35 (1H, m), 3.24-3.27 (1H, m), 2.91-2.85 (1H, m), 2.75-2.68 (1H, m), 2.65-2.59 (1H, m), 2.34-2.26 (1H, m), 2.10-2.02 (1H, m), 1.89-1.82 (1H, m), 1.79-1.72 (1H, m).

Compounds shown in Example 53 to Example 61 were synthesized by conducting a reaction similar to that mentioned in Example 52, using various amines instead of ammonia gas.

(Example 53)

2-(4-Fluorophenyl)-4-[(2S,8aS)-2-[4-(N-methylcarbamoyl)phenyl]-1,2,3,5,6,8a-hexahydroindolizin-7-yl]-3-(pyridin-4-yl)-1H-pyrrole (Exemplification compound number 7-367)

A pale brownish powder (yield: 69%)
Melting point: 171-173°C
$^1$H-NMR (500MHz, DMSO-$d_6$) δppm:
11.43-11.36 (1H, br.s), 8.45 (2H, d, J=6Hz), 8.35-8.30 (1H, m), 7.73 (2H, d, J=8Hz), 7.33 (2H, d, J=8Hz), 7.21-7.07 (6H, m), 6.95 (1H,d, J=3Hz), 5.22-5.18 (1H, m), 3.54-3.47 (1H, m), 3.39 (1H, quintet, J=8Hz), 3.21 (1H, t, J=9Hz), 2.91-2.84 (1H, m), 2.76 (3H, d, J=4Hz), 2.75-2.67 (1H, m), 2.62 (1H, dd, J=10Hz, 8Hz), 2.34-2.26 (1H, m), 2.10-2.02 (1H, m), 1.89-1.81 (1H, m), 1.80-1.72 (1H, m).

(Example 54)

4-[(2S,8aS)-2-[4-(N-Ethylcarbamoyl)phenyl]-1,2,3,5,6,8a-hexahydroindolizin-7-yl]-2-(4-fluorophenyl)-3-(pyridin-4-yl)-1H-pyrrole (Exemplification compound number 7-368)

A pale brownish powder (yield: 59%)
Melting point: 178-180°C
$^1$H-NMR (500MHz, DMSO-$d_6$) δppm:
11.43-11.37 (1H, br.s), 8.45 (2H, d, J=6Hz), 8.38-8.33 (1H, m), 7.74 (2H, d, J=8Hz), 7.33 (2H, d, J=8Hz), 7.20-7.08 (6H, m), 6.97-6.93 (1H, m), 5.22-5.19 (1H, m), 3.54-3.47 (1H, m), 3.44-3.35 (1H, m), 3.30-3.18 (3H, m), 2.92-2.84 (1H, m), 2.75-2.67 (11H, m), 2.62 (1H, dd, J=10Hz, 8Hz), 2.35-2.26 (1H, m), 2.10-2.02 (1H, m), 1.89-1.81 (1H, m), 1.80-1.71 (1H, m), 1.11 (3H, t, J=7Hz).

(Example 55)

4-[(2S,8aS)-2-[4-(N-Benzylcarbamoyl)phenyl]-1,2,3,5,6,8a-hexahydroindolizin-7-yl]-2-(4-fluorophenyl)-3-(pyridin-4-yl)-1H-pyrrole (Exemplification compound number 7-372)

A pale brownish powder (yield: 72%)
Melting point: 173-175°C
$^1$H-NMR (500MHz, DMSO-$d_6$) δppm:
11.42-11.37 (1H, br.s), 8.05 (1H, t, J=6Hz), 8.45 (2H, d, J=6Hz), 7.81 (2H, d, J=8Hz), 7.35 (2H, d, J=8Hz), 7.33-7.09 (1H, m), 6.95 (1H, d, J=2Hz), 5.22-5.19 (1H, m), 4.46 (2H, d, J=6Hz), 3.55-3.48 (1H, m), 3.41 (1H, quintet, J=8Hz), 3.22 (1H, t, J=9Hz), 2.92-2.85 (1H, m), 2.75-2.68 (1H, m), 2.66-2.60 (1H, m), 2.35-2.26 (1H, m), 2.10-2.02 (1H, m), 1.90-1.82

(1H, m), 1.80-1.72 (1H, m).

(Example 56)

4-[(2S,8aS)-2-[4-(N-Cyclopropylcarbamoyl)phenyl]-1,2,3,5,6,8a-hexahydroindolizin-7-yl]-2-(4-fluorophenyl)-3-(pyridin-4-yl)-1H-pyrrole (Exemplification compound number 7-374)

A pale brownish powder (yield: 38%)
Melting point: 200-202°C
$^1$H-NMR (500MHz, DMSO-$d_6$) δppm:
11.44-11.36 (1H, br.s), 8.45 (2H,d, J=6Hz), 8.15 (1H, d, J=4Hz), 7.72 (2H, d, J=8Hz), 7.32 (2H, d, J=8Hz), 7.20-7.08 (6H, m), 6.95 (1H, d, J=2Hz), 5.22-5.17 (1H, m), 3.54-3.47 (1H, m), 3.39 (1H, quintet, J=8Hz), 3.24-3.17 (1H, m), 2.91-2.78 (2H, m), 2.75-2.67 (1H, m), 2.61 (1H, dd, J=10Hz, 3Hz), 2.35-2.25 (1H, m), 2.11-2.01 (1H, m), 1.88-1.80 (1H, m), 1.80-1.71 (1H, m), 0.69-0.64 (2H, m), 0.57-0.52 (2H, m).

(Example 57)

4-[(2S,8aS)-2-[4-[N-(2-Fluoroethyl)carbamoyl]phenyl]-1,2,3,5,6,8a-hexahydroindolizin-7-yl]-2-(4-fluorophenyl)-3-(pyridin-4-yl)-1H-pyrrole (Exemplification compound number 7-389)

A pale brownish powder (yield: 27%)
Melting point: 183-185°C
$^1$H-NMR (500MHz, DMSO-$d_6$) δppm:
11.43-11.36 (1H, br.s), 8.60 (1H, t, J=5Hz), 8.23 (2H, d, J=6Hz) 7.78 (2H, d, J=8Hz), 7.35 (2H, d, J=8Hz), 7.20-7.09 (6H, m), 6.95 (1H, d, J=3Hz), 5.24-5.18 (1H, m), 4.57 (1H, t, J=5Hz), 4.47 (1H, t, J=5Hz), 3.57 (1H, quartet, J=5Hz), 3.52 (2H, quartet, J=5Hz), 3.41 (1H, quintet, J=8Hz), 3.25-3.19 (1H, m), 2.92-2.84 (1H, m), 2.76-2.67 (1H, m), 2.63 (1H, dd, J=10Hz, 3Hz), 2.35-2.25 (1H, m), 2.10-2.02 (1H, m), 1.89-1.82 (1H, m), 1.81-1.72 (1H, m).

(Example 58)

2-(4-Fluorophenyl)-4-[(2S,8aS)-2-[4-(N-propylcarbamoyl)phenyl]-1,2,3,5,6,8a-hexahydroindolizin-7-yl]-3-(pyridin-4-yl)-1H-pyrrole (Exemplification compound number 7-369)

A pale brownish powder (yield: 79%)
Melting point: 190-192°C
$^1$H-NMR (500MHz, DMSO-$d_6$) δppm:
11.44-11.36 (1H, br.s), 8.46 (2H, d, J=6Hz), 8.38-8.32 (1H, m), 7.75 (2H, d, J=8Hz), 7.33 (2H, d, J=8Hz), 7.21-7.09 (6H, m), 6.96 (1H, d, J=3Hz), 5.23-5.19 (1H, m), 3.55-3.48 (1H, m), 3.46-3.36 (1H, m), 3.25-3.16 (3H, m), 2.93-2.85 (1H, m), 2.76-2.68 (1H, m), 2.63 (1H, dd, J=10Hz, 3Hz), 2.37-2.26 (1H, m), 2.11-2.03 (1H, m), 1.90-1.82 (1H, m), 1.81-1.72 (1H, m), 1.56-1.47 (2H, m), 0.91-0.85 (3H, m).

(Example 59)

2-(4-Fluorophenyl)-4-[(2S,8aS)-2-[4-(N-isopropylcarbamoyl)phenyl]-1,2,3,5,6,8a-hexahydroindolizin-7-yl]-3-(pyridin-4-yl)-1H-pyrrole (Exemplification compound number 7-370)

A pale brownish powder (yield: 79%)
Melting point: 202-204°C
$^1$H-NMR (500MHz, DMSO-$d_6$) δppm:
11.44-11.37 (1H, br.s), 8.46 (2H, d, J=6Hz), 8.10 (1H, d, J=7Hz), 7.75 (2H, d, J=8Hz), 7.33 (2H, d, J=8Hz), 7.20-7.09 (6H, m), 6.96 (1H, d, J=3Hz), 5.23-5.19 (1H, m), 4.08 (1H, octet, J=7Hz), 3 .55-3 .48 (1H, m), 3.40 (1H, quintet, J=8Hz), 3.32 (1H, t, J=9Hz), 2.93-2.85 (1H, m), 2.76-2.68 (1H, m), 2.63 (1H, dd, J=10Hz, 3Hz), 2.37-2.26 (1H, m), 2.11-2.02 (1H, m), 1.90-1.82 (1H, m), 1.81-1.72 (1H, m), 1.15 (6H, d, J=7Hz).

(Example 60)

2-(4-Fluorophenyl)-4-[(2S,8aS)-2-[4-[N-(2-hydroxymethyl)carbamoyl]phenyl]-1,2,3,5,6,8a-hexahydroindolizin-7-yl]-3-(pyridin-4-yl)-1H-pyrrole (Exemplification compound number 7-385)

A pale brownish powder (yield: 52%)
Melting point: 189-191°C
$^1$H-NMR (500MHz, DMSO-$d_6$) δppm:
11.44-11.38 (1H, br.s), 8.46 (2H, d, J=6Hz), 8.36-8.31 (1H, m), 7.77 (2H, d, J=8Hz), 7.34 (2H, d, J=8Hz), 7.21-7.10 (6H, m), 6.96 (1H, d, J=3Hz), 5.23-5.19 (1H, m), 4.73-4.68 (1H, m), 3.55-3.46 (3H, m), 3.41 (1H, quintet, J=8Hz), 3.34-3.28 (2H, m), 3.22 (1H, t, J=9Hz), 2.93-2.85 (1H, m), 2.76-2.68 (1H, m), 2.63 (1H, dd, J=10Hz, 2Hz), 2.36-2.27 (1H, m), 2.11-2.03 (1H, m), 1.90-1.82 (1H, m), 1.81-1.73 (1H, m).

(Example 61)

2-(4-Fluorophenyl)-4-[(2S,8aS)-2-[4-(N-(2-methoxyethyl)carbamoyl)phenyl]-1,2,3,5,6,8a-hexahydroindolizin-7-yl]-3-(pyridin-4-yl)-1H-pyrrole (Exemplification compound number 7-390)

A pale brownish powder (yield: 66%)
Melting point: 166-168°C
$^1$H-NMR (500MHz, DMSO-$d_6$) δppm:
11.43-11.38 (1H, br.s), 8.46 (2H, d, J=6Hz), 8.44-8.39 (1H, m), 7.76 (2H, d, J=8Hz), 7.34 (2H, d, J=8Hz), 7.21-7.09 (6H, m), 6.96 (1H, d, J=2Hz), 5.23-5.20 (1H, m), 3.55-3.48 (1H, m), 3.47-3.37 (5H, m), 3.26 (3H, s), 3.25-3.19 (1H, m), 2.92-2.85 (1H, m), 2.76-2.68 (1H, m), 2.63 (1H, dd, J=10Hz, 3Hz), 2.35-2.27 (1H, m), 2.11-2.02 (1H, m), 1.90-1.82 (1H, m), 1.81-1.73 (1H, m).

(Example 62)

(±)-4-[1-(β-Ethyl-4-methanesulfonylphenethyl)-1,2,3,6-tetrahydropyridin-4-yl]-2-(4-fluorophenyl)-3-(pyridin-4-yl)-1H-pyrrole monohydrochloride (Exemplification compound number 1-1304)

(±)-4-[1-(β-Ethyl-4-methanesulfonylphenethyl)-1,2,3,6-tetrahydropyridin-4-yl]-2-(4-fluorophenyl)-3-(pyridin-4-yl)-1H-pyrrole was synthesized by conducting a reaction similar to that mentioned in Example 40, using (±)-α-4-ethyl-4-methanesulfonylphenylacetaldehyde instead of 4-methanesulfonylphenylacetaldehyde. Subsequently the product thus obtained was formed to its monohydrochloride salt by conducting a reaction similar to that mentioned in Example 19 to afford the title compound in a yield of 54% as a yellow powder.
Melting point: 183-185°C (degradation)
$^1$H-NMR (400MHz, DMSO-$d_6$) δppm of the free form:
11.34 (1H, s), 8.44 (2H, d, J=6Hz), 7.84 (2H, d, J=9Hz), 7.48 (2H, d, J=8Hz), 7.16-7.08 (6H, m), 6.88 (1H, d, J=2Hz), 5.21 (1H, s), 3.20 (3H, s), 2.89-2.84 (3H, m), 2.56-2.43 (4H, m), 2.07 (2H, s), 1.80-1.73 (1H, m), 1.52-1.44 (1H, m), 0.70 (3H, t, J=8Hz).

(Example 63)

(±)-2-(3=Chloro-4-fluorophenyl)-4-[1-([3-ethyl-4-methanesulfonyl phenethyl)-1,2,3,6-tetrahydropyridin-4-yl]-3-(pyridin-4-yl)-1H-pyrrole monohydrochloride (Exemplification compound number 1-1331)

(±)-2-(3-Chloro-4-fluorophenyl)-4-[1-(β-ethyl-4-methanesulfonyl phenethyl)-1,2,3,6-tetrahydropyridin-4-yl]-3-(pyridin-4-yl)-1H-pyrrole was synthesized by conducting a reaction similar to that mentioned in Example 40, using (±)-α-ethyl-4-methanesulfonylphenylacetaldehyde instead of 4-methanesulfonylphenylacetaldehyde, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl)-1H-pyrrole. Subsequently the product thus obtained was formed to its monohydrochloride salt by conducting a reaction similar to that mentioned in Example 19 to afford the title compound in a yield of 10% as a yellow powder.
Melting point: 183-185°C (degradation)
$^1$H-NMR (400MHz, DMSO-$d_6$) δppm of the free form:
11.48 (1H, d, J=2Hz), 8.47 (2H, d, J=6Hz), 7.83 (2H, d, J=8Hz), 7.47 (2H, d, J=8Hz), 7.33-7.27 (2H, m), 7.13 (2H, d, J=6Hz), 7.03-6.99 (1H, m), 6.92 (1H, d, J=2Hz), 5.20 (1H, s), 3.20 (3H, s), 2.91-2.78 (3H, m), 2.58-2.41 (4H, m), 2.06

(2H, s), 1.79-1.71 (1H, m), 1.53-1.44 (1H, m), 0.69 (3H, t, J=7Hz).

(Example 64)

4-[1-[β,β-Ethylene-4-(N-methylsulfamoyl)phenethyl]-2-(4-fluorophenyl)-1,2,3,6-tetrahydropyridin-4-yl]-3-(pyridin-4-yl)-1H-pyrrole monohydrochloride (Exemplification compound number 1-1292)

4-[1-[β,β-Ethylene-4-(N-methylsulfamoyl)phenethyl]-2-(4-fluorophenyl)-1,2,3,6-tetrahydropyridin-4-yl]-3-(pyridin-4-yl)-1H-pyrrole was synthesized by conducting a reaction similar to that mentioned in Example 40 using 1-[4-(N-methylsulfamoyl)phenyl]cycloprpane-1-carboxyaldehyde instead of 4-methanesulfonylphenylacetaldehyde. Subsequently the product thus obtained was derived to its monohydrochloride salt by conducting a reaction similar to that mentioned in Example 19 to afford the title compound in a yield of 28% as a pale yellowish powder.
Melting point: 260-261°C (degradation)
$^1$H-NMR (500MHz, DMSO-$d_6$) δppm of the free form:
11.33-11.28 (1H, m), 8.42 (2H, d, J=6Hz), 7.65 (2H, d, J=8Hz), 7.51 (2H, d, J=8Hz), 7.36-7.29 (1H, m), 7.17-7.06 (6H, m), 6.85 (1H, d, J=2Hz), 5.23-5.19 (1H, m), 2.91-2.86 (2H, m), 2.62 (2H, s), 2.54 (2H, t, J=6Hz), 2.41 (3H, s), 2.08-2.03 (2H, m), 0.92-0.88 (2H, m) , 0.82-0.78 (2H, m).

(Example 65)

2-(3-Chloro-4-fluorophenyl)-4-[1-[β,β-ethylene-4-(N-methylsulfamoyl)phenethyl]-1,2,3,6-tetrahydropyridin-4-yl]-3-(pyridin-4-yl)-1H-pyrrole monohydrochloride (Exemplification compound number 1-1319)

2-(3-Chloro- 4-fluorophenyl)- 4-[1-[β, β-ethylene- 4-(N-methylsulfamoyl) phenethyl]- 1,2,3,6-tetrahydropyridin-4-yl]-3-(pyridin-4-yl)-1H-pyrrole was synthesized by conducting a reaction similar to that mentioned in Example 40, using 1-[4-(N-methylsulfamoyl)phenyl]cyclopropane-1-carboxyaldehyde instead of 4-methanesulfonylphenylacetaldehyde, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl)-1H-pyrrole. Subsequently the product thus obtained was formed to its monohydrochloride salt by conducting a reaction similar to that mentioned in Example 19 to afford the title compound in a yield of 16% as a pale yellowish powder.
Melting point: 168-170°C (degradation)
$^1$H-NMR (500MHz, DMSO-$d_6$) δppm of the free form:
11.48-11.44 (1H, br.s), 8.46 (2H, d, J=6Hz), 7.64 (2H, d, J=8Hz), 7.50 (2H, d, J=8Hz), 7.37-7.26 (3H, m), 7.12 (2H, d, J=6Hz), 7.04-6.99 (1H, m), 6.91 (1H, d, J=3Hz), 5.22-5.18 (1H, m), 2.91-2.85 (2H, m), 2.61 (2H, s), 2.53 (2H, t, J=5Hz), 2.40 (3H, s), 2.08-2.02 (2H, m), 0.92-0.88 (2H, m), 0.82-0.78 (2H, m).

(Example 66)

(±)-2-(4-Fluorophenyl)-4-[1-[β-methyl-4-(N-methylsulfamoyl)phenethyl]-1,2,3,6-tetrahydropyridin-4-yl]-3-(pyridin-4-yl)-1H-pyrrole monohydrochloride (Exemplification compound number 1-1285)

(±)-2-(4-Fluorophenyl)-4-[1-[β-methyl-4-(N-methylsulfamoyl)phenethyl]-1,2,3,6-tetrahydropyridin-4-yl]-3-(pyridin-4-yl)-1H-pyrrole was synthesized by conducting a reaction similar to that mentioned in Example 40, using (±)-α-methyl-4-(N-methylsulfamoyl)phenylacetaldehyde instead of 4-methanesulfonylphenylacetaldehyde. Subsequently the product thus obtained was derived to its monohydrochloride salt by conducting the reaction similar to that mentioned in Example 19 to afford the title compound in a yield of 37% as a yellow powder.
Melting point: 175-177°C (degradation)
$^1$H-NMR (400MHz, DMSO-$d_6$) δppm of the free form:
11.69 (1H, s), 10.34 (1H, br.s), 8.49 (2H, d, J=6H), 7.74 (2H, d, J=9Hz), 7.59 (2H, d, J=8Hz), 7.48-7.42 (1H, m), 7.36 (2H, d, J=6Hz), 7.21-7.09 (4H, m), 7.06 (1H, d, J=3Hz), 5.09 (1H, br.s), 3.78-2.55 (9H, m), 2.40 (3H, d, J=5Hz), 1.34 (3H, m).

(Example 67)

(±)-2-(3-Chloro-4-fluorophenyl)-4-[1-[β-methyl-4-(N-methylsulfamoyl)phenethyl]-1,2,3,6-tetrahydropyridin-4-yl]-3-(pyridin-4-yl)-1H-pyrrole monohydrochloride (Exemplification compound number 1-1312)

(±)- 2-(3-Chloro- 4-fluorophenyl)- 4-[1-[β-methyl- 4-(N-methylsulfamoyl) phenethyl]- 1,2,3,6-tetrahydropyridin-4-yl]-3-(pyridin-4-yl)-1H-pyrrole was synthesized by conducting a reaction similar to that mentioned in Example 40, using (±)-α-methyl-4-(N-methylsulfamoyl)phenylacetaldehyde instead of 4-methanesulfonylphenylacetaldehyde, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl)-1H-pyrrole. Subsequently the product thus obtained was derived to its monohydrochloride salt by conducting a reaction similar to that mentioned in Example 19 to afford the title compound in a yield of 15% as a yellow powder.
Melting point: 177-179°C (degradation)
$^1$H-NMR (400MHz, DMSO-$d_6$) δppm:
11.85 (1H, s), 10.34 (1H, br.s), 8.54 (2H, d, J=6Hz), 7.74 (2H, d, J=8Hz), 7.63-7.53 (2H, m), 7.51-7.38 (4H, m), 7.33 (1H, t, J=9Hz), 7.12 (1H, s), 7.10-7.02 (1H, m), 5.12 (0.5H, s), 5.09 (0.5H, s), 3.79-2.59 (9H, m), 2.44-2.36 (3H, m), 1.40-1.29 (3H, m).

(Example 68)

(±)-4-[1-[β-Ethyl-4-(N-methylsulfamoyl)phenethyl]-2-(4-fluorophenyl)-1,2,3,6-tetrahydropyridin-4-yl]-3-(pyridin-4-yl)-1H-pyrrole monohydrochloride (Exemplification compound number 1-1286)

(±)-4-[1-[β-Ethyl-4-(N-methylsulfamoyl)phenethyl]-2-(4-fluorophenyl)-1,2,3,6-tetrahydropyridin-4-yl]-3-(pyridin-4-yl)-1H-pyrrole was synthesized by conducting a reaction similar to that mentioned in Example 40, using (±)-α-ethyl-4-(N-methylsulfamoyl)phenylacetaldehyde instead of 4-methanesulfonylphenylacetaldehyde. Subsequently the product thus obtained was derived to its monohydrochloride salt by conducting a reaction similar to that mentioned in Example 19 to afford the title compound in a yield of 46% as a yellow powder.
Melting point: 168-1719°C (degradation)
$^1$H-NMR (400MHz, DMSO-$d_6$) δppm of the free form:
11.34 (1H, s), 8.43 (2H, d, J=7Hz), 7.68 (2H, d, J=8Hz), 7.42 (2H, d, J=8Hz), 7.35 (1H, s), 7.16-7.09 (6H, m), 6.87 (1H, d, J=2Hz), 5.21 (1H, s), 3.32 (3H, s), 2.84 (3H, s), 2.54-2.44 (4H, m), 2.06 (2H, s), 1.81-1.72 (1H, m), 1.51-1.43 (1H, m), 0.69 (3H, t, J=7Hz) .

(Example 69)

(±)-2-(3-Chloro-4-fluorophenyl)-4-[1-[β-ethyl-4-(N-methylsulfamoyl)phenethyl]-1,2,3,6-tetrahydropyridin-4-yl]-3-(pyridin-4-yl)-1H-pyrrole monohydrochloride (Exemplification compound number 1-1313)

(±)- 2-(3-Chloro- 4-fluorophenyl)- 4-[1-[β-ethyl- 4-(N-methylsulfamoyl) phenethyl]- 1,2,3,6-tetrahydropyridin-4-yl]-3-(pyridin-4-yl)-1H-pyrrole was synthesized by conducting a reaction similar to that mentioned in Example 40, using (±)-α-ethyl-4-(N-methylsulfamoyl)phenylacetaldehyde instead of 4-methanesulfonylphenylacetaldehyde, and using 2-(3-chloro-4-fluorophenyl)-3-(pyridin-4-yl)-4-(1,2,3,6-tetrahydropyridin-4-yl]-1H-pyrrole instead of 2-(4-fluorophenyl)-3-(pyridin-4-yl)-4-(1,2,3,6-tetrahydropyridin-4-yl)-1H-pyrrole. Subsequently the product thus obtained was derived to its monohydrochloride salt by conducting a reaction similar to that mentioned in Example 19 to afford the title compound in a yield of 35% as a yellow powder.
Melting point: 165-168°C (degradation)
$^1$H-NMR (400MHz, DMSO-$d_6$) δppmof the free form:
11.43 (1H, d, J=2Hz), 8.44 (2H, d, J=6Hz), 7.66 (2H, d, J=8Hz), 7.40 (2H, d, J=8Hz), 7.34-7.25 (3H, m), 7.11 (2H, d, J=6Hz), 7.02-6.98 (1H, m), 6.90 (1H, d, J=2Hz), 5.19 (1H, s), 3.30 (3H, s), 2.83 (3H, s), 2.53-2.43 (4H, m), 2.06 (2H, s), 1.80-1.73 (1H, m), 1.50-1.43 (1H, m), 0.69 (3H, t, J=7Hz).

(Example 70)

2-(4-Fluorophenyl)-4-[1-[2-[5-(N-methylsulfamoyl)thiophen-2-yl]ethyl]-1,2,3,6-tetrahydropyridin-4-yl]-3-(pyridin-4-yl)-1H-pyrrole monohydrochloride (Exemplification compound number 6-134)

2-(4-Fluorophenyl)-4-[1-[2-[5-(N-methylsulfamoyl)thiophen-2-yl]ethyl]-1,2,3,6-tetrahydropyridin-4-yl]-3-(pyridin-4-yl)-1H-pyrrole was synthesized by conducting a reaction similar to that mentioned in Example 40, using 2-[5-(N-methylsulfamoyl) thiophen-2-yl]ethyl bromide instead of 4-ethoxycarbonylphenethyl bromide. Subsequently the product thus obtained was formed to its monohydrochloride salt by conducting a reaction similar to that mentioned in Example 19 to afford the title compound in a yield of 44% as a pale yellowish powder.
Melting point: 225-226°C (degradation)
$^1$H-NMR (500MHz, DMSO-$d_6$) δppm of the free form:

11.40-11.35 (1H, br.s), 8.46 (2H, d, J=6Hz), 7.53-7.48 (1H, br.s), 7.38 (1H, d, J=4Hz), 7.19-7.09 (6H, m), 6.95 (1H, d, J=4Hz), 6.93 (1H, d, J=3Hz), 5.27-5.24 (1H, m), 3.02 (2H, t, J=7Hz), 2.96-2.92 (2H, m), 2.60 (2H, t, J=7Hz), 2.58 (2H, t, J=6Hz), 2.48 (3H, s), 2.23-2.28 (2H, m).

(Example 71)

2-(4-Fluorophenyl)-4-[1-(4-(N-methoxycarbonylmethylsulfamoyl)phenethyl]-1,2,3,6-tetrahydropyridin-4-yl]-3-(pyridin-4-yl)-1H-pyrrole (Exemplification compound number 1-465)

The title compound was synthesized in a yield of 23% as a pale yellowish powder by conducting the reaction similar to that mentioned in Example 1, using 4-(N-methoxycarbonylmethylsulfamoyl)phenethyl bromide instead of 4-ethoxycarbonylphenethyl bromide.
Melting point: 220-223°C (degradation)
$^1$H-NMR (400MHz, DMSO-$d_6$) δppm:
11.33 (1H, s), 8.42 (2H, d, J=6Hz), 8.10 (1H, t, J=6Hz), 7.65 (2H, d, J=8Hz), 7.40 (2H, d, J=9Hz), 7.15-7.07 (6H, m), 6.88 (1H, d, J=3Hz), 5.24 (1H, s), 3.67 (2H, d, J=6Hz), 3.49 (3H, s), 2.92 (2H, s), 2.81 (2H, t, J=7Hz), 2.59-2.52 (4H, m), 2.15 (2H, s).

(Example 72)

4-[1-[4-(N-Carboxymethylsulfamoyl)phenethyl]-2-(4-fluorophenyl)-1,2,3,6-tetrahydropyridin-4-yl]-3-(pyridin-4-yl)-1H-pyrrole (Exemplification compound number 1-464)

The title compound was afforded in a yield of 81% as a pale yellowish powder by conducting a hydrolysis reaction similar to that mentioned in Example 4, using 2-(4-fluorophenyl)-4-[1-[4-(N-methoxycarbonylmethylsulfamoyl) phenethyl]-1,2,3,6-tetrahydropyridin-4-yl]-3-(pyridin-4-yl)-1H-pyrrole that was obtained in Example 71.
Melting point: 233-235°C (degradation)
$^1$H-NMR (400MHz, DMSO-$d_6$) δppm of the free form:
11.40 (1H, s), 8.43 (2H, d, J=6Hz), 7.66 (2H, d, J=9Hz), 7.39 (2H, d, J=8Hz), 7.16-7.06 (6H, m), 6.88 (1H, d, J=3Hz), 5.23 (1H, s), 3.08 (2H, s), 2.93 (2H, s), 2.83-2.79 (2H, m), 2.60-2.53 (4H, m), 2.15 (2H, s).

(Example 73)

4-[1-[4-(N-Carbamoylmethylsulfamoyl)phenethyl]-2-(4-fluorophenyl)-1,2,3,6-tetrahydropyridin-4-yl]-3-(pyridin-4-yl)-1H-pyrrole (Exemplification compound number 1-467)

The title compound was synthesized in a yield of 15% as a pale brownish powder by conducting the reaction similar to that mentioned in Example 1, using 4-(N-carbamoylmethylsulfamoyl)phenethyl bromide instead of 4-ethoxycarbonylphenethyl bromide.
Melting point: 230-233°C (degradation)
$^1$H-NMR (400MHz, DMSO-$d_6$) δppm of the free form:
11.40-11.35 (1H, br.s), 8.46 (2H, d, J=6Hz), 7.53-7.48 (1H, br.s), 11.34 (1H, s), 8.42 (2H, d, J=6Hz), 7.67 (2H, d, J=8Hz), 7.40 (2H, d, J=9Hz), 7.24 (1H, s), 7.15-7.07 (6H, m), 6.88 (1H, d, J=3Hz), 5.24 (1H, s), 3.32 (2H, s), 2.92 (2H, s), 2.83-2.80 (2H, m), 2.59-2.53 (4H, m), 2.15 (2H, s).

(Example 74)

4-[1-[4-(N-Cyanomethylsulfamoyl)phenethyl]-2-(4-fluorophenyl)-1,2,3,6-tetrahydropyridin-4-yl]-3-(pyridin-4-yl)-1H-pyrrole (Exemplification compound number 1-457)

The title compound was synthesized in a yield of 16% as a pale yellowish powder by conducting a reaction similar to that mentioned in Example 1, using 4-(N-cyanomethylsulfamoyl)phenethyl bromide instead of 4-ethoxycarbonylphenethyl bromide.
Melting point: 196-199°C (degradation)
$^1$H-NMR (400MHz, DMSO-$d_6$) δppm
11.36 (1H, s), 8.47 (1H, s), 8.45 (2H, d, J=7Hz), 7.74 (2H, d, J=8Hz), 7.47 (2H, d, J=8Hz), 7.18-7.09 (6H, m), 6.90 (1H, d, J=2Hz), 5.25 (1H, s), 4.07 (2H, s), 2.92 (2H, d, J=3Hz), 2.84 (2H, t, J=8Hz), 2.60-2.53 (4H, m), 2.15 (2H, s).

(Example 75)

4-[1-[3-Fluoro-4-(N-methylsulfamoyl)phenethyl]-2-(4-fluorophenyl)-1,2,3,6-tetrahydropyridin-4-yl]-3-(pyridin-4-yl)-1H-pyrrole monohydrochloride (Exemplification compound number 2-1)

4-[1-[3-Fluoro-4-(N-methylsulfamoyl)phenethyl]-2-(4-fluorophenyl)-1,2,3,6-tetrahydropyridin-4-yl]-3-(pyridin-4-yl)-1H-pyrrole was synthesized by conducting a reaction similar to that mentioned in Example 1, using 3-fluoro-4-(N-methylsulfamoyl)phenethyl bromide instead of 4-ethoxycarbonylphenethyl bromide. Subsequently, by conducting a reaction similar to that mentioned in Example 19, the product thus obtained was derived to its monohydrochloride salt in a yield of 48% as a pale brownish powder.
Melting point: 239-241°C (degradation)
$^1$H-NMR (400MHz, DMSO-$d_6$) δppm of the free form:
11.38 (1H, br.s), 8.45 (2H, d, J=7Hz), 7.69-7.59 (2H, m), 7.35 (1H, dd, J=12Hz, 1Hz), 7.24 (1H, dd, J=8Hz, 1Hz), 7.19-7.08 (6H, m), 6.90 (1H, d, J=3Hz), 5.28-5.22 (1H, m), 2.97-2.89 (2H, m), 2.87-2.79 (2H, m), 2.64-2.50 (4H, m), 2.48 (3H, s), 2.21-2.12 (2H, m).

(Example 76)

4-[1-[3-Chloro-4-(N-methylsulfamoyl)phenethyl]-2-(4-fluorophenyl)-1,2,3,6-tetrahydropyridin-4-yl]-3-(pyridin-4-yl)-1H-pyrrole monohydrochloride (Exemplification compound number 2-2)

4-[1-[3-Chloro-4-(N-methylsulfamoyl)phenethyl]-2-(4-fluorophenyl)-1,2,3,6-tetrahydropyridin-4-yl]-3-(pyridin-4-yl)-1H-pyrrole was synthesized by conducting a reaction similar to that mentioned in Example 1, using 3-chloro-4-(N-methylsulfamoyl)phenethyl bromide in stead of 4-ethoxycarbonylphenethyl bromide. Subsequently, by conducting a reaction similar to that mentioned in Example 19, the product thus obtained was derived to its monohydrochloride salt in a yield of 8% as a pale yellowish powder.
Melting point: 229-231°C (degradation)
$^1$H-NMR (400MHz, DMSO-$d_6$) δppm of the free form:
11.33 (1H, br.s), 8.42 (2H, d, J=6Hz), 7.81 (1H, d, J=8Hz), 7.59-7.54 (1H,m), 7.53 (1H, d, J=2Hz), 7.37 (1H, dd, J=8Hz, 2Hz), 7.17-7.06 (6H, m), 6.89 (1H, d, J=2Hz), 5.23 (1H, br.s), 2.93-2.88 (2H, m), 2.84-2.78 (2H, m), 2.62-2.51 (4H, m), 2.45 (3H, d, J=5Hz), 2.17-2.12 (2H, m).

(Example 77)

2-(4-Fluorophenyl)-4-[1-[3-methyl-4-(N-methylsulfamoyl)phenethyl]-1,2,3,6-tetrahydropyridin-4-yl]-3-(pyridin-4-yl)-1H-pyrrole monohydrochloride (Exemplification compound number 2-4)

2-(4-Fluorophenyl)-4-[1-[3-methyl-4-(N-methylsulfamoyl)phenethyl]-1,2,3,6-tetrahydropyridin-4-yl]-3-(pyridin-4-yl)-1H-pyrrole was synthesized by conducting a reaction similar to that mentioned in Example 1, using 3-methyl-4-(N-methylsulfamoyl)phenethyl bromide instead of 4-ethoxycarbonylphenethyl bromide. Subsequently, by conducting a reaction similar to that mentioned in Example 19, the product thus obtained was derived to its monohydrochloride salt in a yield of 55% as a pale yellowish powder.
Melting point: 192-194°C (degradation)
$^1$H-NMR (400MHz, DMSO-$d_6$) δppm of the free form:
11.33 (1H, br.s), 8.43 (2H, d, J=6Hz), 7.66 (1H, d, J=8Hz), 7.37-7.31 (1H, m), 7.23(1H, s), 7.20 (1H, d, J=8Hz), 7.16-7.06 (6H, m), 6.89 (1H, d, J=3Hz), 5.24 (1H, br.s), 2.94-2.89 (2H, m), 2.79-2.73 (2H, m), 2.60-2.50 (4H, m), 2.51 (3H, s), 2.39 (3H, d, J=5Hz), 2.18-2.12 (2H, m).

(Example 78)

2-(4-Fluorophenyl)-4-[1-[3-methoxy-4-(N-methylsulfamoyl)phenethyl]-1,2,3,6-tetrahydropyridin-4-yl]-3-(pyridin-4-yl)-1H-pyrrole monohydrochloride (Exemplification compound number 2-3)

2-(4-Fluorophenyl)-4-[1-[3-methoxy-4-(N-methylsulfamoyl)phenethyl]-1,2,3,6-tetrahydropyridin-4-yl]-3-(pyridin-4-yl)-1H-pyrrole was synthesized by conducting a reaction similar to that mentioned in Example 1, using 3-methoxy-4-(N-methylsulfamoyl)phenethyl bromide instead of 4-ethoxycarbonylphenethyl bromide. Subsequently, by conducting a reaction similar to that mentioned in Example 19, the product thus obtained was derived to its monohydrochloride salt in a yield of 74% as a pale yellowish powder.

Melting point: 245-247°C (degradation)
$^1$H-NMR (400MHz, DMSO-$d_6$) δppm of the free form:
11.37 (1H, br.s), 8.45 (2H, d, J=6Hz), 7.59 (1H, d, J=8Hz), 7.19-7.08 (7H, m), 6.95-6.88 (3H, m), 5.28-5.23 (1H, m), 3.87 (3H, s), 2.97-2.91 (2H, m), 2.83-2.77 (2H, m), 2.64-2.51 (4H, m), 2.37 (3H, d, J=5Hz), 2.21-2.12 (2H, m).

(Example 79)

2-(3-Chloro-4-fluorophenyl)-4-[1-[4-(2-methoxyethyl)sulfonylphenethyl]-1,2,3,6-tetrahydropyridin-4-yl]-3-(pyridin-4-yl)-1H-pyrrole monohydrochloride (Exemplification compound number 1-1346)

2-(3-Chloro-4-fluorophenyl)-4-[1-[4-(2-methoxyethyl)sulfonylphenethyl]-1,2,3,6-tetrahydropyridin-4-yl]-3-(pyridin-4-yl)-1H-pyrrole was synthesized by conducting a reaction similar to that mentioned in Example 1, using 4-(2-methoxyethyl) sulfonylphenethyl bromide in stead of 4-ethoxycarbonylphenethyl bromide, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2.,3,6-tetrahydropyridin-4-yl)-1H-pyrrole. Subsequently, by conducting a reaction similar to that mentioned in Example 19, the product thus obtained was derived to its monohydrochloride salt in a yield of 50% as a pale yellowish powder.
Melting point: 208-210°C (degradation)
$^1$H-NMR (400MHz, DMSO-$d_6$) δppm of the free form:
11.50 (1H, br.s), 8.48 (2H, d, J=6Hz), 7.78 (2H, d, J=8Hz), 7.48 (2H, d, J=8Hz), 7.36-7.27 (2H, m), 7.15 (2H, d, J=6Hz), 7.05-7 (1H, m), 6.95 (1H, d, J=3Hz),5.27-5.22 (1H, m), 3.62-3.5 3.09 (3H, s), 2.95-2.88 (2H, m), 2.88-2.82 (2H, m), 2.63-. m), 2.18-2.12 (2H, m).

(Example 80)

2-(3-Chloro-4-fluorophenyl)-4-[1-[4-(2-hydroxyethyl)sulfonylphenethyl]-1,2,3,6-tetrahydropyridin-4-yl]-3-(pyridin-4-yl)-1H-pyrrole monohydrochloride (Exemplification compound number 1-1347)

1)
2-(3-Chloro-4-fluorophenyl)-3-(pyridin-4-yl)-4-[1-[4-[2-(tetrahydropyran-2-yloxy)ethyl]sulfonylphenethyl]-1,2,3,6-tetrahydropyridin-4-yl]-1H-pyrrole was synthesized in a yield of 37% as a pale brownish powder by conducting a reaction similar to that mentioned in Example 1, using 4-[2-(tetrahydropyran-2-yloxy)ethyl]sulfonylphenethyl bromide instead of 4-ethoxycarbonylphenethyl bromide, and using 2-(3-chloro-4-fluorophenyl)-3-(pyridin-4-yl)-4-(1,2,3,6-tetrahydropyridin-4-yl)-1H-pyrrole instead of 2-(4-fluorophenyl)-3-(pyridin-4-yl)-4-(1,2,3,6-tetrahydropyridin-4-yl)-1H-pyrrole.
$^1$H-NMR (400MHz, DMSO-$d_6$) δppm:
11.50 (1H, br.s), 8.49 (2H, d, J=7Hz), 7.79 (2H, d, J=8Hz), 7.48 (2H, d, J=8Hz), 7.36-7.27 (2H, m), 7.15 (2H, d, J=7Hz), 7.05-7.00 (1H, m), 6.95 (1H, d, J=3Hz),5.27-5.22 (1H, m), 4.46-4.42 (1H, m), 3.90-3.81 (1H, m), 3.67-3.52 (4H, m), 3.38-3.28 (1H, m), 2.94-2.88 (2H, m), 2.88-2.80 (2H, m), 2.61-2.48 (4H, m), 2.19-2.11 (2H, m), 1.44-1.22 (5H, m), 1.15-1.05 (1H, m).
2)
To a solution of 2-(3-chloro-4-fluorophenyl)-3-(pyridin-4-yl)-4-[1-[4-[2-(tetrahydropyran-2-yloxy)ethyl]sulfonylphenethyl]-1,2,3,6-tetrahydropyridin-4-yl]-1H-pyrrole (197 mg, 0.30 mmol) in a mixed solvent (10 ml) of tetrahydrofuran and 1N hydrochloric acid (1 : 1) was stirred at room temperature for 3 days. After the reaction mixture was neutralized with saturated aqueous sodium hydrogencarbonate solution, the resulting reaction mixture was extracted with ethyl acetate. The separated organic layer containing the desired compound was dried over anhydrous sodium sulfate and concentrated under reduced pressure. The solid residue thus obtained was washed with ether and the free form of the title compound was afforded. By conducting a reaction similar to that mentioned in Example 19, the free form of the title compound was derived to its monohydrochloride salt in a yield of 86% as a pale yellow powder (151 mg) .
Melting point: 2478-249°C (degradation)
$^1$H NMR (400 MHz, DMSO-$d_6$) δppm of the free form:
11.50 (1H, br.s), 8.49 (2H, d, J=6Hz), 7.79 (2H, d, J=8Hz), 7.49 (2H, d, J=8Hz), 7.37-7.27 (2H, m), 7.16 (2H, d, J=6Hz), 7.05-7.00 (1H, m), 6.96 (1H, d, J=3Hz),5.27-5.23 (1H, m), 4.91-4.85 (1H, m), 3.70-3.62 (2H, m), 3.41 (2H, t, J=7Hz), 2.96-2.88 (2H, m), 2.88-2.81 (2H, m), 2.63-2.49 (4H, m), 2.19-2.12 (2H, m).

(Reference Example 1)

4-Bromo-2-(4-fluorophenyl)-3-(pyridin-4-yl)-1-triisopropylsilyl-1H-pyrrole

1) 4-Ethoxycarbonyl-2-(4-fluorophenyl)-3-(pyridin-4-yl)-1H-pyrrole
To a mixture of 1.53M solution of butyllithium in hexane (36 ml, 54.7 mmol) and tetrahydrofuran (240 ml) was added a solution of α-(p-toluenesulfonyl)-4-fluorobenzylisocyanide (15.90 g, 54.7 mmol) in tetrahydrofuran (120 ml) at -45°C under stirring, and the resulting mixture was stirred at the same temperature for 10 minutes. Furthermore, to the reaction mixture was added 95% lithium bromide (25.00 g, 273 mmol) at the same temperature under stirring, and the resulting mixture was stirred at the same temperature for 30 minutes. After stirring, a solution of ethyl 3-(4-pyridyl)acrylate (8.73 g, 49.2 mmol) in tetrahydrofuran (120 ml) was added to the reaction mixture at the same temperature under stirring, and the resulting mixture was stirred at the same temperature for one hour. Subsequently, the reaction vessel was removed from the cooling bath, and the reaction mixture was stirred at room temperature for one hour. After stirring, water (500 ml) was added to the reaction mixture, and the resulting mixture was extracted with ethyl acetate. The separated organic layer containing the desired compound was washed with water, dried over anhydrous sodium sulfate and concentrated under reduced pressure. The solid thus obtained was washed with diethyl ether to afford the title compound (13.61 g) in a yield of 89% as a pale yellow powder.
$^1$H-NMR (500MHz, DMSO-$d_6$) δppm:
8.84 (1H, br.s), 8.51 (2H, d, J=7Hz), 7.58 (1H, d, J=3Hz), 7.21 (2H, d, J=6Hz), 7.11 (2H, dd, J=9Hz, 5Hz), 6.97 (2H, t, J=9Hz), 4.18 (2H, q, J=7Hz), 1.20 (3H, t, J=7Hz).
2) 2-(4-Fluorophenyl)-3-(pyridin-4-yl)-1H-pyrrole
4-Ethoxycarbonyl-2-(4-fluorophenyl)-3-(pyridin-4-yl)-1H-pyrrole (15.00 g, 48.3 mmol) obtained in l) was dissolved in a mixed solvent of acetic acid (90 ml), sulfuric acid (30 ml) and water (60 ml) under stirring, and the resulting mixture was stirred at 100°C for 16 hours. After cooling to room temperature, the reaction mixture was alkalified with 10% aqueous sodium hydroxide solution and extracted with ethyl acetate. The extract was washed with water, dried over anhydrous sodium sulfate and evaporated in vacuo to afford the title compound (11.40 g) in a yield of 99% as a pale red powder.
$^1$H-NMR (500MHz, DMSO-$d_6$) δppm:
9.78 (1H, br.s), 8.42 (2H, d, J=7Hz), 7.37 (2H, dd, J=9Hz, 5Hz), 7.22 (2H, d, J=6Hz), 7.06 (2H, t, J=9Hz), 6.90 (1H, t, J=3Hz), 6.47 (1H, t, J=3Hz).
3) 2-(4-Fluorophenyl)-3-(pyridin-4-yl)-1-triisopropylsilyl-1H-pyrrole
To a solution of 2-(4-fluorophenyl)-3-(pyridin-4-yl)-1H-pyrrole (11.30 g, 47.4 mmol) obtained in 2) in tetrahydrofuran (300 ml) was added 1.57N solution of butyllithium in hexane (31 ml, 47.4 mmol) at -78°C under stirring, and the resulting mixture was stirred at the same temperature for 10 minutes. After stirring, triisopropylsilyl triflate (13.4 ml, 49.8 mmol) was added to the reaction mixture at the same temperature under stirring. Subsequently, the reaction vessel was removed from the cooling bath, and the reaction mixture was stirred at room temperature for 30 minutes. After stirring, water (200ml) and saturated aqueous sodium hydrogencarbonate solution (300 ml) were added successively to the reaction mixture under stirring, and the resulting mixture was extracted with ethyl acetate. The extract was washed with water, dried over anhydrous sodium sulfate and evaporated in vacuo to afford the title compound (18.70 g, yield: quantitative) as a purplish red coloured oily product.
$^1$H-NMR (500MHz, DMSO-$d_6$) δppm:
8.25 (2H, d, J=6Hz), 7.39 (2H, dd, J=9Hz, 6Hz), 7.28 (2H, t, J=9Hz), 7.00 (1H, d, J=3Hz), 6.91(2H, d, J=7Hz), 6.71 (1H, d, J=3Hz), 1.15-1.05 (3H, m), 0.98 (18H, d, J=8Hz).
4) 4-Bromo-2-(4-fluorophenyl)-3-(pyridin-4-yl)-1-triisopropylsilyl-1H-pyrrole
To a solution of 2-(4-fluorophenyl)-3-(pyridin-4-yl)-1-triisopropylsilyl-1H-pyrrole (18.70 g, 47.4 mmol) obtained in 3) in tetrahydrofuran (300 ml) was added gradually a suspension of N-bromosuccinimide (8.61 g, 47.4 mmol) in tetrahydrofuran (100 ml) at -78°C under stirring, and the resulting mixture was stirred at the same temperature for 6 hours. Subsequently, the reaction vessel was removed from the cooling bath, and the reaction mixture was further stirred at room temperature for one hour. After stirring, hexane (400 ml) was added to the reaction mixture, and the insoluble materials were removed by filtration. The filtrate was evaporated in vacuo, and the residue was purified by chromatography on a silica gel column using a mixed solvent of hexane and ethyl acetate (2:1) as the eluent to afford the title compound (9.57 g) in a yield of 43% as a pale yellow prism crystal.
$^1$H-NMR (500MHz, DMSO-$d_6$) δppm:
8.36 (2H, d, J=6Hz), 7.34 (2H, dd, J=9Hz, 6Hz), 7.18 (2H, t, J=9Hz), 7.12 (1H, s), 7.04(2H, d, J=6Hz), 1.16-1.08 (3H, m), 0.99 (18H, d, J=8Hz).

(Reference Example 2)

2-(3-Chloro-4-fluorophenyl)-3-(pyridin-4-yl)-4-(1,2,3,6-tetrahydropyridin-4-yl)-1H-pyrrole

1) 4-Bromo-1-(t-butyl)diphenylsilyl-2-(3-chloro-4-fluorophenyl)-3-(pyridin-4-yl)-1H-pyrrole

a)
2-(3-Chloro-4-fluorophenyl)-3-(pyridin-4-yl)-1H-pyrrole was synthesized in a yield of 78% as a pale peach coloured powder by conducting successively the reactions described in Reference Example 1-1) and 2), using α-(p-toluenesulfonyl)-(3-chloro-4-fluorobenzyl)isocyanide as the starting material instead of α-(p-toluenesulfonyl)-4-fluorobenzylisocyanide.
b)
Subsequently, a solution of 2-(3-chloro-4-fluorophenyl)-3-(pyridin-4-yl)-1H-pyrrole (29.6 g, 109 mmol) obtained in a) in tetrahydrofuran (300 ml) was added dropwise to a suspended solution of sodium hydride (55%) (9.5 g, 218 mmol) in tetrahydrofuran (600 ml) under stirring and ice-cooling, and the resulting mixture was stirred at room temperature for 30 minutes. After stirring, (t-butyl) diphenylsilyl chloride (56.7 ml, 218 mmol) was added to the reaction mixture, and the resulting mixture was stirred at room temperature for one hour, and then the reaction was stopped by addition of ice-cold water. After removal of tetrahydrofuran in vacuo, the residue was extracted with ethyl acetate, and the extract was washed with water, dried over anhydrous sodium sulfate and evaporated in vacuo. The residue was purified by chromatography on a silica gel column using ethyl acetate as the eluent to afford 1-(t-butyl)diphenylsilyl-2-(3-chloro-4-fluorophenyl)-3-(pyridin-4-yl)-1H-pyrrole (45.2 g) in a yield of 81% as a white powder.
c)
Subsequently, to a solution of the product (45.2 g, 88.4 mmol) obtained in b) in toluene (900 ml) were added successively pyridine (14.3 ml, 176.4 mmol) and pyridinium perbromide hydrobromide (33.9 g, 106.1 mmol) under stirring, and the resulting mixture was stirred at room temperature for an overnight. After stirring, the reaction mixture was filtered, and the filtrate was evaporated in vacuo. The residue was purified by chromatography on a silica gel column using a mixed solvent of ethyl acetate and hexane (1 : 2) as the eluent to afford the title compound (42.3 g) in a yield of 81% as a white powder.
$^1$H-NMR (400MHz, DMSO-$d_6$) δppm:
8.36 (2H, d, J=6Hz), 7.46-7.29 (11H, m), 7.03 (2H, d, J=6Hz), 6.72-6.66 (1H, m), 6.56-6.53 (1H, m), 6.53-6.47 (1H, m), 1.06 (9H, s).

2) 2-(3-Chloro-4-fluorophenyl)-3-(pyridin-4-yl)-4-(1,2,3,6-tetrahydropyridin-4-yl)-1H-pyrrole
The reaction (coupling reaction) similar to that described in Example 48-a) was conducted using 4-bromo-1-(t-butyl) diphenylsilyl-2-(3-chloro-4-fluorophenyl)-3-(pyridin-4-yl)-1H-pyrrole (14.0 g, 23.73 mmol) obtained in 1) and 1-(t-butoxycarbonyl)piperidin-4-one (4.73 g, 23.73 mmol). When the reaction was completed, saturated aqueous ammonium chloride solution was added to the reaction mixture to stop the reaction. After removal of the solvent in vacuo, the residue was extracted with ethyl acetate, and the extract was washed with water, dried over anhydrous sodium sulfate and evaporated in vacuo. Subsequently, to the residue were added successively dichloromethane (300 ml) and trifluoroacetic acid (50 ml) under stirring, and the resulting mixture was stirred overnight at room temperature (reactions for dehydration and removal of BOC group) . After stirring, the reaction mixture was concentrated under the reduced pressure, and to the residue were added successively 25% aqueous sodium hydroxide solution (23.73 ml, 237.3 mmol), methanol (200 ml), tetrahydrofuran (100 ml), and water (50 ml) under stirring. The resulting mixture was stirred at room temperature for one hour (desilylation reaction), and then a large portion of the solvent was evaporated in vacuo. The residue was extracted with ethyl acetate, and the extract was washed with water, dried over anhydrous sodium sulfate and evaporated in vacuo. The residue was purified by chromatography on a silica gel column using a mixed solvent of ethyl acetate, methanol and isopropylamine (100:10:1) as the eluent to afford the title compound (6.52 g) in a yield of 78% as a white powder.
$^1$H-NMR (400MHz, DMSO-$d_6$) δppm:
11.64 (1H, br.s), 8.51 (2H, d, J=6Hz), 7.37-7.28 (2H, m), 7.18 (2H, d, J=6Hz), 7.09-7.00 (2H, m), 5.22 (1H, br.s), 3.48-3.41 (2H, m), 3.13 (2H, t, J=6Hz), 2.39-2.30 (2H, m).

(Reference Example 3)

5-(4-Fluorophenyl)-4-(pyridin-4-yl)-3-(1,2,3,6-tetrahydropyridin-4-yl)-1H-pyrazole

1) 3-Bromo-5-(4-fluorophenyl)-4-(pyridin-4-yl)-pyrazole
To a solution of 5-(4-fluorophenyl)-4-(pyridin-4-yl)pyrazole (compound with the compound number A-55 described in International Patent Publication No. 00/31063 Pamphlet) (6.0 g, 25 mmol) in dimethylformamide (60 ml) was added N-bromosuccinimide (8.93 g, 50 mmol) under stirring, and the resulting mixture was stirred at room temperature for 3 days. After stirring, water was added to the reaction mixture, and the crystal precipitated was collected by filtration and washed with diethyl ether to afford the title compound (5.73 g) in a yield of 72% as a white powder.
$^1$H-NMR (400MHz, DMSO-$d_6$) δppm:
8.56 (2H, d, J=5Hz), 7.33 (2H, dd, J=8Hz, 5Hz), 7.24 (2H, d, J=5Hz), 7.05 (2H, t, J=8Hz).
2) 5-(4-Fluorophenyl)-4-(pyridin-4-yl)-3-(1,2,3,6-tetrahydropyrid in-4-yl)-1H-pyrazole
A reaction (coupling reaction) similar to that described in Example 48-a) was conducted using 3-bromo-5-(4-fluorophenyl)-4-(pyridin-4-yl)pyrazole (1.20 g, 3.77 mmol) obtained in 1) and 1-(t-butoxycarbonyl)piperidin-4-one (767 mg, 3.77 mmol) . After completion of the reaction, saturated aqueous sodium hydrogencarbonate solution (50 m) was added to the reaction mixture under stirring, and the resulting mixture was extracted with ethyl acetate. The extract was washed with water, dried over anhydrous sodium sulfate and evaporated in vacuo. Subsequently, to the residue was added concentrated hydrochloric acid (40 ml) under stirring, and the resulting mixture was stirred at 110°C for 7 hours (reactions for dehydration and removal of BOC group). After cooling, the reaction mixture was alkalified with 10% aqueous sodium hydroxide solution under stirring and ice-cooling. The solid precipitated was collected by filtration and washed with ethyl acetate to afford the title compound (660 mg) in a yield of 55% as a pale brown powder.
$^1$H-NMR (500MHz, DMSO-$d_6$) δppm:
8.52 (2H, d, J=7Hz), 7.29 (2H, dd, J=9Hz, 5Hz), 7.18 (2H, d, J=6Hz), 7.15 (2H, t, J=9Hz), 5.74 (1H, s), 3.21 (2H, d, J=3Hz), 2.79 (2H, t, J=6Hz), 2.13 (2H, br.s).

(Reference Example 4)

4-Carbamoylphenethyl chloride

To a suspended solution of 4-carboxyphenethyl chloride (185 mg, 1.0 mmol) in dichloromethane (2 ml) were added successively oxalyl chloride (0.1 ml, 1.1 mmol) and one drop of dimethylformamide under stirring, and the resulting mixture was stirred at room temperature for 2 hours. Subsequently, the reaction mixture was added in small portions to a mixture of t-butanol (1 ml), tetrahydrofuran (2 ml) and 28% ammonia water (0.5 ml) under stirring, and the resulting mixture was stirred at room temperature for 3 hours. After stirring, the reaction mixture was evaporated in vacuo, and the solid residue thus obtained was washed successively with hexane and diethyl ether to afford the title compound (170 mg) in a yield of 92% as a white powder.
$^1$H-NMR (400MHz, DMSO-$d_6$) δppm:
7.78 (2H, d, J=8Hz), 7.31 (2H, d, J=8Hz), 6.15 (2H, br.s), 3.74 (2H, t, J=7Hz), 3.12 (2H, t, J=7Hz).

(Reference Example 5)

3-Carbamoylphenethyl bromide

The title compound was synthesized by conducting a reaction similar to that mentioned in Reference Example 4, using 3-carboxyphenethyl bromide instead of 4-carboxylphenethyl bromide.

(Reference Example 6)

4-Methanesulfonylphenylacetaldehyde

To a solution of (methoxymethyl)triphenylphophonium chloride (24.80 g, 72.3 mmol) in methanol (250 ml) was added 28% solution of sodium methoxide in methanol (15.35 ml, 79.6 mmol) under stirring, and the resulting mixture was stirred at room temperature for 30 minutes and then evaporated in vacuo. Subsequently, to the residue were added successively toluene (375 ml) and then 4-methanesulfonylbenzaldehyde (10.25 g, 55.6 mmol) under stirring, and the

resulting mixture was stirred at 80°C for 7 hours. After cooling to room temperature, the reaction mixture was filtered, and the filtrate was evaporated in vacuo. The residue was purified by chromatography on a silica gel column using a mixed solvent of hexane and ethyl acetate (2 : 1) as the eluent to afford 4-methanesulfonyl-β-methoxystyrene (10.21 g) as a white powder. Subsequently, to this product were added successively tetrahydrofuran (225 ml) and water (40.5 ml) under stirring, and furthermore, to the resulting mixture was added concentrated sulfuric acid (4.5 ml) under stirring. The resulting mixture was refluxed for 19 hours and then evaporated in vacuo. To the residue was added water, and the resulting mixture was extracted with dichloromethane. The extract was washed with water, dried over anhydrous sodium sulfate and evaporated in vacuo to afford the title compound (9.16 g) in a yield: 83% as a colourless oily product.
$^1$H-NMR (400MHz, $CDCl_3$) δppm:
9.82 (1H, s), 7.95 (2H, d, J=8Hz), 7.43 (2H, d, J=8Hz), 3.86 (2H, s), 3.06 (3H, s).

(Reference Example 7)

(±)-4-Methanesulfinylphenethyl bromide

a)
To a solution of 4-methylthiophenylacetic acid (5.97 g, 32.8 mmol) in methanol (100 ml) was added dropwise thionyl chloride (2.63 ml, 36.0 mmol) under stirring and ice-cooling, and the resulting mixture was stirred at room temperature for 48 hours. After removal of the solvent in vacuo, water was added to the residue, and the resulting mixture was extracted with ethyl acetate. The extract was washed successively with saturated aqueous sodium hydrogencarbonate solution and water, dried over anhydrous magnesium sulfate and evaporated in vacuo to afford methyl (±)-4-methylthiophenylacetate (ester form) (6.37 g, yield: quantitative) as a colourless oil.
b)
Subsequently, a solution of the ester form (whole amount of ester form) obtained in a) in tetrahydrofuran (60 ml) was added dropwise to a suspended solution of lithium aluminium hydride (2.47 g, 65.5 mmol) in tetrahydrofuran (100 ml) under stirring and ice-cooling, and the resulting mixture was stirred at room temperature for 2 hours. After stirring, the reaction mixture was cooled to 0°C, and to the reaction mixture was added carefully 4% aqueous sodium hydroxide solution (10 ml) at 0°C under stirring. The resulting mixture was filtered using celite, and the filtrate was evaporated invacuo to afford (±)-4-methylthiophenethyl alcohol (alcohol form) (5.40 g) in a yield of 98% as a white powder.
c)
Subsequently, to a solution of the alcohol form (2.50 g, 14.9 mmol) obtained in b) in dichloromethane (50 ml) were added successively triphenylphosphine (4.68 g, 17.8 mmol) and carbon tetrabromide (6.41 g, 19.3 mmol) under stirring and ice-cooling, and the resulting mixture was stirred at room temperature for one hour. After evaporating the reaction mixture in vacuo, diethyl ether was added to the residue, and insoluble materials were removed by filtration. The filtrate was evaporated in vacuo, and the residue was purified by chromatography on a silica gel column using a mixed solvent of hexane and ethyl acetate (19 : 1) as the eluent to afford (±)-4-methylthiophenethyl bromide (bromide form) (3.17 g) in a yield of 92% as a colourless oily product.
d)
Furthermore, to a solution of the bromide form (3.15 g, 13.6 mmol) obtained in c) in dichloromethane (65 ml) was added m-chloroperbenzoic acid (65%) (3.62 g, 13.6 mmol) in small portions under stirring and ice-cooling, and the resulting mixture was stirred at the same temperature for 10 minutes. After stirring, the reaction mixture was filtered using celite, and the filtrate was washed successively with 10% aqueous sodium thiosulfate solution (30 ml) and saturated aqueous sodium hydrogencarbonate solution (30 ml), dried over anhydrous sodium sulfate and evaporated in vacuo. The residue was purified by chromatography on a silica gel column using a mixed solvent of hexane and ethyl acetate (1 : 3) as the eluent to afford the title compound (2.58 g) in a yield of 77% as a colourless oily product.
$^1$H-NMR (400MHz, $CDCl_3$) δppm:
7.62 (2H, d, J=8Hz), 7.39 (2H, d, J=8Hz), 3.60 (2H, t, J=7Hz), 3.24 (2H, t, J=7Hz), 2.73 (3H, s).

(Reference Example 8)

4-(N-methylsulfamoyl)phenethyl bromide

To a solution of 4-(chlorosulfonyl)phenethyl bromide (10.00 g, 35.2 mmol) in tetrahydrofuran (200 ml) was added 40% aqueous methylamine solution (9.14 ml, 105.6 mmol) under stirring, and the resulting mixture was stirred at room temperature for one hour. After evaporating the reaction mixture in vacuo, water was added to the residue, and

the resulting mixture was extracted with dichloromethane. The extract was washed with water, dried over anhydrous magnesium sulfate and evaporated in vacuo to afford the title compound (9.63 g) in a yield of 98% as a white powder.
$^1$H-NMR (500MHz, $CDCl_3$) δppm:
7.82 (2H, d, J=8Hz), 7.38 (2H, d, J=8Hz), 4.40-4.30 (1H, m), 3.60 (2H, t, J=8Hz), 3.25 (2H, t, J=8Hz), 2.68 (3H, d, J=5Hz).
Compounds of Reference Example 9 to Reference Example 25 were synthesized by conducting a reaction similar to that mentioned in Reference Example 8, using various amines instead of methylamine.

(Reference Example 9)

4-(N-Ethylsulfamoyl)phenethyl bromide

A white powder (yield: 99%)

(Reference Example 10)

4-(N-Propylsulfamoyl)phenethyl bromide

A white powder (yield: 74%)

(Reference Example 11)

4-(N-Isopropylsulfamoyl)phenethyl bromide

A white powder (yield: 98%)

(Reference Example 12)

4-(N-Cyclopropylsulfamoyl)phenethyl bromide

A white powder (yield: 80%)

(Reference Example 13)

4-[N-(2-Fluorophenyl)sulfamoyl]phenethyl bromide

A white powder (yield: 82%)

(Reference Example 14)

4-(N-Phenylsulfamoyl)phenethyl bromide

A pale brownish powder (yield: 83%)

(Reference Example 15)

4-(N-Methoxysulfamoyl)phenethyl bromide

A white powder (yield: 77%)

(Reference Example 16)

4-(N,N-Dimethylsulfamoyl)phenethyl bromide

A white powder (yield: 98%)

(Reference Example 17)

4-[N-(2-Methoxyethyl)sulfamoyl]phenethyl bromide

A white powder (yield: 98%)

(Reference Example 18)

4-[N-(2-Hydroxyethyl)sulfamoyl]phenethyl bromide

A white powder (yield: 95%)

(Reference Example 19)

4-(2,2-Dimethylhydrazinosulfamoyl)phenethyl bromide

A pale yellowish powder (yield: 95%)

(Reference Example 20)

4-[N-(Pyridin-3-yl)methylsulfamoyl]phenethyl bromide

A white powder (yield: quantitative)

(Reference Example 21)

4-[N-(2-Dimethylaminoethyl)sulfamoyl]phenethyl bromide

A white powder (yield: 56%)

(Reference Example 22)

4-[N-(1,3-Dihydroxypropan-2-yl)sulfamoyl]phenethyl bromide

A white powder (yield: 97%)

(Reference Example 23)

4-(N-Ethoxysulfamoyl)phenethyl bromide

A white powder (yield: 97%)

(Reference Example 24)

4-(N-Benzyloxysulfamoyl)phenethyl bromide

A white powder (yield: quantitative)

(Reference Example 25)

4-(N-Allyloxysulfamoyl)phenethyl bromide

A white powder (yield: 95%)

(Reference Example 26)

α-Bromo-4-(N-methylsulfamoyl)acetophenone

a)
4-(N-Methylsulfamoyl)acetophenone was synthesized in a yield of 97% as a white powder by conducting a reaction in a similar manner to that described in Reference Example 8, using 4-(chlorosulfonyl)acetophenone instead of 4-(chlorosulfonyl)phenethyl bromide.
b)
Subsequently, to a solution of 4-(N-methylsulfamoyl) acetophenone (3.30 g, 15.5 mmol) obtained in a) in tetrahydrofuran (33 ml) was slowly added dropwise bromine (0.79 ml, 15.5 mmol) at room temperature under stirring, and the resulting mixture was stirred at room temperature for one hour. After stirring, ethyl acetate (200 ml) and water (50 ml) were added successively to the reaction mixture, and the resulting mixture was partitioned. The organic layer separated was washed with water, dried over anhydrous magnesium sulfate and evaporated in vacuo. The residue was purified by chromatography on a silica gel column using a mixed solvent of hexane and ethyl acetate (2: 1) as the eluent to afford the title compound (3.79 g) in a yield of 84% as a white powder.
$^1$H-NMR (500MHz, $CDCl_3$) δppm:
8.13 (2H, d, J=8Hz), 7.99 (2H, d, J=8Hz), 4.54 (1H, quartet, J=5Hz), 4.45 (2H, s), 2.72 (3H, d, J=5Hz).

(Reference Example 27)

(2S,8aS)-2-(4-Methoxycarbonylphenyl)-1,2,3,5,6,7,8,8a-octahydroindolizin-7-one

1) (S)-4-Bromo-β-(2,2-diethoxyethyl)phenethylamine

a)
To a solution of 4-bromobenzylcyanide (75.0 g, 0.383 mol) in tetrahydrofuran (750 ml) was added 1.0M solution of lithium bis(trimethylsilyl)amide in tetrahydrofuran (423 ml, 0.423 mol) at below -15°C under stirring, and the resulting mixture was stirred at the same temperature for one hour. Subsequently, the reaction vessel was removed from the cooling bath, and to the reaction mixture was added dropwise 2,2-diethoxyethylbromide (86.5 ml, 0.575 mol) at room temperature under stirring, and the resulting mixture was stirred at room temperature for 6 hours. After removal of the solvent in vacuo, water was added to the residue, and the resulting mixture was extracted with ethyl acetate. The extract was washed with water, dried over anhydrous magnesium sulfate and evaporated in vacuo. The residue was purified by chromatography on a silica gel column using a mixed solvent of hexane and ethyl acetate (10 : 1) as the eluent, followed by reduced-pressure distillation of the crude product obtained to afford (±)-4-bromo-α-(2,2-diethoxyethyl)benzylcyanide (88.95 g, boiling point: 51-54°C/8 mmHg) in a yield of 95% as a brown oily product.
b)
Separately, to a suspension of lithium aluminium hydride (20.34 g, 0.536 mol) in tetrahydrofuran (700 ml) was added dropwise concentrated sulfuric acid (13.49 ml, 0.253 mol) at below 10°C under stirring, and the resulting mixture was stirred at 0-5C° for one hour. Subsequently, the reaction temperature was raised to room temperature, and to the reaction mixture was added dropwise a solution of (±)-4-bromo-α-(2,2-diethoxyethyl)benzylcyanide (119.5 g, 0.383 mol) obtained in a) in tetrahydrofuran (50 ml) under stirring, and the resulting mixture was stirred for 2 hours. After stirring, tetrahydrofuran (750 ml) was added to the reaction mixture under stirring, and furthermore 1N aqueous sodium hydroxide solution (107 ml, 0.28 ml) was added carefully under stirring, and then the resulting mixture was stirred at room temperature for one hour. After stirring, the reaction mixture was filtered using celite, and the filtrate was evaporated in vacuo. The residue was purified by chromatography on a silica gel column using a mixed solvent of ethyl acetate, methanol and isopropylamine (20 : 1 : 1) as the eluent to afford (±)-4-bromo-β-(2,2-diethoxyethyl) phenethylamine (80.40 g) as in a yield of 66% a yellow oily product.
c)
Subsequently, (+)-4-bromo-β-(2,2-diethoxyethyl)phenethylamine (80.40 g, 0.254 mol) obtained in b) and D-tartaric acid (28.60 g, 0.191 mol) were suspended in isopropanol (402 ml) and stirred at 100°C for 30 minutes to prepare a homogeneous solution, which was allowed to stand at room temperature overnight. The solid precipitated was collected by filtration, washed with isopropanol (160 ml each) for two times to afford (S)-4-bromo-β-(2,2-diethoxyethyl) phenethylamine D-tartrate (1 : 1) (46.81g). Subsequently, to the product thus obtained were added successively toluene (470 ml) and water (95 ml), and the resulting mixture was adjusted to a pH of 12-13 by adding dropwise

25% aqueous sodium hydroxide solution while vigorously stirring. The alkalified mixture was partitioned, and the organic layer separated was washed with water, dried over anhydrous sodium sulfate and evaporated in vacuo. The residue was distilled under reduced pressure to afford (S)-4-bromo-β-(2,2-diethoxyethyl)phenethylamine (29.01 g, boiling point: 176-178°C/5.8 mmHg) in a yield of 72% as a colourless oily product.

$^{1}$H-NMR (500MHz, $CDCl_3$) δppm:

7.45 (2H, d, J=9Hz), 7.09 (2H, d, J=9Hz), 4.25-4.20 (1H, m), 3.66-3.58 (1H, m), 3.55-3.48 (1H, m), 3.43-3.33 (2H, m), 2.95-2.89 (1H, m), 2.82 (1H, dd, J=12Hz, 3Hz), 2.79-2.72 (1H, m), 2.03-1.96 (1H, m), 1.80 (1H, ddd, J=14Hz, 10Hz, 6Hz), 1.19 (3H, t, J=7Hz), 1.13 (3H, t, J=7Hz).

2) (2S,8aS)-2-(4-Bromophenyl)-1,2,3,5,6,7,8,8a-octahydroindolizin-7-one

To a solution of (S)-4-bromo-β-(2,2-diethoxyethyl)phenethylamine (29.01 g, 91.7 mmol) obtained in 1) in toluene (145 ml) was added methylvinyl ketone (9.92 ml, 119.2 mmol) at 60°C under stirring, and the resulting mixture was stirred at the same temperature for 30 minutes. After cooling to room temperature, concentrated hydrochloric acid (15.25 ml, 183 mmol) and water (15.25 ml) were added successively to the reaction mixture, and the resulting mixture was partitioned. To the aqueous layer separated were added successively concentrated hydrochloric acid (22.92 ml) and ethanol (57.3 ml), and the resulting mixture was stirred at 50°C for 6 hours. After cooling to room temperature, the resulting mixture was adjusted to a pH of 12-13 by adding dropwise 25% aqueous sodium hydroxide solution while vigorously stirring, and the alkalified mixture was extracted with ethyl acetate. The extract was washed with water, dried over anhydrous sodium sulfate and evaporated in vacuo. The residue was purified by chromatography on a silica gel column using a mixed solvent of hexane and ethyl acetate (1 : 1) as the eluent to afford the title compound (17.34 g) in a yield of 64% as a brown powder.

$^{1}$H-NMR (500MHz, $CDCl_3$)δppm :

7.42 (2H, d, J=8Hz), 7.11 (2H, d, J=8Hz), 3.58-3.47 (2H, m), 3.35 (1H, dd, J=10Hz, 7Hz), 2.69-2.54 (3H, m), 2.44 (1H, dd, J=10Hz, 3Hz), 2.42-2.33 (2H, m), 2.32-2.27 (1H, m), 2.15-2.01 (2H, m).

3) (2S,8aS)-2-(4-Methoxycarbonylphenyl)-1,2,3,5,6,7,8,8a-octahydroindolizin-7-one

To a solution of (2S,8aS)-2-(4-bromophenyl)-1,2,3,5,6,7,8,8a-octahydroindolizin-7-one (8.00 g, 27.2 mmol) obtained in 2) in a mixed solvent of methanol (272 ml) and N,N-dimethylformamide (54 ml) were added successively [1,1'-bis (diphenylphosphino)ferrocene]dichloropalladium(II) (4.44 g, 5.44 mmol) and ethyldiisopropylamine (9.48 ml, 54.4 mmol) under stirring, and the resulting mixture was stirred at 70°C under a carbon monoxide atmosphere for 5 hours. After cooling to room temperature, ethyl acetate and water were added to the reaction mixture, and the resulting mixture was partitioned. The organic layer separated was washed with water, dried over anhydrous sodium sulfate and evaporated in vacuo. The residue was purified by chromatography on a silica gel column using ethyl acetate as the eluent to afford the title compound (6.50 g, yield: 87%) as a pale brown powder. The product thus obtained was analyzed by HPLC with the following operating conditions. As a result, the optical purity of the product synthesized was 91% ee.

HPLC operating conditions:

| | |
|---|---|
| Column: | CHIRALPAK AS-H (0.46 cm × 25 cm), Daicel Chemical Industries, Ltd. |
| Mobile phase: | n-Hexane/ethanol/isopropylamine (90 : 10 : 0.1) |
| Flow rate: | 1.0 ml/min |
| Temperature: | 40°C |
| Detection: | 254 nm (UV) |
| Retention time: | (2S,8aS) isomer; 15.48 minutes (2R,8aR) isomer; 11.80 minutes |

$^{1}$H-NMR (500MHz, $CDCl_3$) δppm:

7.98 (2H , d, J=8Hz), 7.30 (2H, d, J=8Hz), 3.91 (3H, s), 3.69-3.58 (1H, m), 3.57-3.50 (1H, m), 3.39-3.32 (1H, m), 2.70-2.55 (3H, m), 2.46 (1H, dd, J=11Hz, 4Hz), 2.43-2.32 (3H, m), 2.16-2.09 (2H, m).

(Reference Example 28)

1-[4-(N-Methylsulfamoyl)phenyl]cyclopropane-1-carboxyaldehyde

1) Methyl 1-(4-chlorosulfonylphenyl)cyclopropane-1-carboxylate

To a solution of methyl 1-phenylcyclopropane-1-carboxylate (2.67 g, 15.15 mmol) in dichloromethane (27 ml) was added dropwise chlorosulfonic acid (5.15 ml, 77.5 mmol) under stirring and ice-cooling, and the resulting mixture

was stirred at room temperature for 5 hours and then concentrated in vacuo. To the residue was added ethyl acetate, and then water was added carefully to the resulting mixture under stirring. The resulting mixture was partitioned, and the organic layer separated was washed with water, dried over anhydrous magnesium sulfate and evaporated in vacuo. The residue was purified by chromatography on a silica gel column using a mixed solvent of hexane and ethyl acetate (10 : 1) as the eluent to afford the title compound in a yield of 52% as a white powder.
$^1$H-NMR (500MHz, $CDCL_3$) δppm:
7.98 (2H, d, J=8Hz), 7.59 (2H, d, J=8Hz), 3.66 (3H, s), 1.75-1.70 (2H, m), 1.27-1.23 (2H, m).
2) Methyl 1-[4-(N-methylsulfamoyl)phenyl]cyclopropane-1-carboxylate
To a solution of methyl 1-(4-chlorosulfonylphenyl)cyclopropane-1-carboxylate (2.16 g, 7.86 mmol) obtained in 1) in tetrahydrofuran (22 ml) was added 30% solution of methylamine in ethanol (2.44 g, 23.6 mmol) under stirring and ice-cooling, and the resulting mixture was stirred at room temperature for 30 minutes. After stirring, ethyl acetate and water were added to the reaction mixture, and the resulting mixture was partitioned. The organic layer separated was washed with water, dried over anhydrous magnesium sulfate and evaporated in vacuo. The solid residue thus obtained was washed with hexane to afford the title compound (2.13 g, yield: quantitative) as a white powder.
$^1$H-NMR (500MHz, $CDCl_3$) δppm:
7.80 (2H, d, J=8Hz), 7.50 (2H, d, J=8Hz), 4.35 (1H, quartet, J=5Hz), 3.64 (3H, s), 2.69 (3H, d, J=5Hz), 1.70-1.66 (2H, m), 1.24-1.20 (2H, m).
3) 1-[4-(N-Methylsulfamoyl)phenyl]cyclopropane-1-methanol
The title compound was synthesized in a yield of 94% by reducing methyl 1-[4-(N-methylsulfamoyl)phenyl]cyclopropane-1-carboxylate obtained in 2) with lithium aluminium hydride in a similar manner to that described in Reference example 7.
$^1$H-NMR (500MHz, $CDCl_3$) δppm :
7.79 (2H, d, J=9Hz), 7.50 (2H, d, J=9Hz), 4.46-4.33 (1H, m), 3.74 (2H, s), 2.66 (3H, d, J=6Hz), 0.99-0.92 (4H, m).
4) 1-[4-(N-Methylsulfamoyl)phenyl]cyclopropane-1-carboxyaldehyde
To a suspension of pyridinium chlorochromate (4.33 g, 20.1 mmol) and molecular sieve 4A (4.33 g) in dichloromethane (50 ml) was added 1-[4-(N-methylsulfamoyl)phenyl]cyclopropane-1-methanol (0.97 g, 4.02 mmol) obtained in 3) under stirring, and the resulting mixture was stirred at room temperature for one hour. After stirring, diethyl ether (50 ml) was added to the reaction mixture, and the resulting mixture was filtered. The filtrate was evaporated in vacuo, and the residue was purified by chromatography on a silica gel column using a mixed solvent of hexane and ethyl acetate (1 : 2) as the eluent to afford the title compound (0.88 g) in a yield of 92% as a white powder.
$^1$H-NMR (500MHz, $CDCl_3$) δppm:
9.09 (1H, s), 7.83 (2H, d, J=9Hz), 7.44 (2H, d, J=9Hz), 4.28 (1H, quartet, J=5Hz), 2.69 (3H, d, J=5Hz), 1.67-1.63 (2H, m), 1.49-1.45 (2H, m).

(Reference Example 29)

(±)-α-Methyl-4-(N-methylsulfamoyl)phenylacetaldehyde

The title compound was synthesized in a yield of 26% as a white powder by conducting reactions similar to those mentioned in Reference Example 28-1), 2), 3), and 4), using ethyl (±)-α-methylphenylacetate instead of methyl 1-phenylcyclopropane-1-carboxylate as a starting material.
$^1$H-NMR (400MHz, $CDCl_3$) δppm :
9.71 (1H, s), 7.88 (2H, d, J=8Hz), 7.39 (2H, d, J=8Hz), 4.45-4.38 (1H, m), 3.75 (1H, quartet, J=7Hz), 2.69 (3H, d, J=5Hz), 1.50 (3H, d, J=7Hz).

(Reference Example 30)

(±)-α-Ethyl-4-(N-methylsulfamoyl)phenylacetaldehyde

The title compound was synthesized in a yield of 5% as a colourless oily product by conducting reactions similar to those mentioned in Reference Example 28-1), 2), 3), and 4), using ethyl (±)-α-ethylphenylacetate instead of 1-phenylcyclopropane-1-carboxylic acid methyl ester as a starting material.
Melting point: 202-204°C
$^1$H-NMR (400MHz, $CDCl_3$) δppm :
9.68 (1H, d, J=2Hz), 7.85 (2H, d, J=8Hz), 7.34 (2H, d, J=8Hz), 4.40 (1H, quartet, J=5Hz), 3.54-3.50 (1H, m), 2.69-2.68 (3H, m), 2.22-2.11 (1H, m), 1.85-1.74 (1H, m), 0.92 (3H, t, J=7Hz).

(Reference Example 31)

(±)-α-Ethyl-4-methanesulfonylphenylacetaldehyde

The title compound was synthesized in a yield of 56% as a yellow oily product by conducting reactions similar to those mentioned in Reference Example 28-2) and 3), using methyl (±)-α-ethyl-4-methanesulfonylphenylacetate instead of methyl 1—[4-(N-methylsulfamoyl)phenyl]cyclopropane-1-carboxylate as a starting material.
Melting point: 202-204°C
$^1$H-NMR (400MHz, $CDCl_3$) δppm :
9.72 (1H, s), 7.96 (2H, d, J=8Hz), 7.42 (2H, d, J=8Hz), 3.59-3.54 (1H, m), 3.07 (3H, s), 2.24-2.14 (1H, m), 1.86-1.75 (1H, m), 0.93 (3H, t, J=7Hz).
Compounds of Reference Examples 32 to 34 were synthesized by conducting a reaction similar to that mentioned in Reference Example 8, using glycine derivatives (methyl esters, amides, or nitriles) instead of methylamine.

(Reference Example 32)

4-(N-Methoxycarbonylmethylsulfamoyl)phenethyl bromide

A white powder (yield: quantitative)

(Reference Example 33)

4-(N-Carbamoylmethylsulfamoyl)phenethyl bromide

A yellow powder (yield: 49%)

(Reference Example 34)

4-(N-Cyanomethylsulfamoyl)phenethyl bromide

A yellow oily product (yield: 81%)

(Reference Example 35)

2-[5-(N-Methylsulfamoyl)thiophen-2-yl]ethyl bromide

2-(2-Bromoethyl)-5-chlorosulfonylthiophene was synthesized by conducting reactions similar to those mentioned in Reference Example 28, using 2-(2-bromoethyl)thiophene. Subsequently, the title compound was synthesized in a yield of 44% as a white powder by reacting this product obtained with methylamine in a similar manner to that mentioned in Reference Example 8.
$^1$H-NMR (500MHz, $CDCl_3$) δppm:
7.45 (1H, d, J=4Hz), 6.88 (1H, d, J=4Hz), 4.45-4.30 (1H, m), 3.57 (2H, t, J=7Hz), 3.39 (2H, t, J=7Hz), 2.73 (3H, d, J=5Hz).

(Reference Example 36)

3-Methoxy-4-(N-methylsulfamoyl)phenethyl bromide

4-Chlorosulfonyl-3-methoxyphenethyl bromide was synthesized in a yield of 6% as a white powder by conducting a chlorosulfonylation reaction in a similar manner to that described in Reference Example 28, using 3-methoxyphenethyl bromide. Subsequently, the product thus obtained was reacted with methylamine in a similar manner to that described in Reference example 8 to afford the title compound in yield of 6% as a white powder.
$^1$H-NMR (400MHz, $CDCl_3$) δppm:
7.86 (1H, d, J=8Hz), 6.94 (1H, d, J=8Hz), 6.89 (1H, s), 4.82-4.74 (1H, m), 3.99 (3H, s), 3.59 (2H, t, J=7Hz), 3.22 (2H, t, J=7Hz), 2.59 (3H, d, J=6Hz).

(Reference Example 37)

3-Fluoro-4-(N-methylsulfamoyl)phenethyl bromide

The title compound was synthesized in a yield of 5% as a white powder by conducting reactions similar to those mentioned in Reference Example 36, using 3-fluorophenethyl bromide.

(Reference Example 38)

3-Chloro-4-(N-methylsulfamoyl)phenethyl bromide

The title compound was synthesized in a yield of 6% as a white powder by conducting reactions similar to those mentioned in Reference Example 36, using 3-chlorophenethyl bromide

(Reference Example 39)

3-Methyl-4-(N-methylsulfamoyl)phenethyl bromide

The title compound was synthesized in a yield of 11% as a white powder by conducting reactions similar to those mentioned in Reference Example 36, using 3-methylpheriethyl bromide.

(Reference Example 40)

4-(2-Methoxyethyl)sulfonylphenethyl bromide

1)
To a solution of 4-mercaptophenethyl alcohol (1. 10 g, 7.13 mmol) and 2-methoxyethyl chloride (0.98 ml, 10.7 mmol) in methanol (22 ml) was added potassium carbonate (1.48 g, 10.7 mmol) under stirring, and the resulting mixture was refluxed for 4 hours. After cooling to room temperature, water was added to the reaction mixture, and the resulting mixture was extracted with ethyl acetate. The extract was washed with water, dried over anhydrous magnesium sulfate and evaporated in vacuo. The residue was purified by chromatography on a silica gel column using a mixed solvent of hexane and ethyl acetate (3:1) as the eluent to afford 4-(2-methoxyethyl)sulfonylphenethyl alcohol (sulfide form) (1.18 g) in a yield of 78% as a colourless oil.
2) Subsequently, to a solution of the sulfide form (1.17 g, 5.51 mmol) obtained in 1) in dichloromethane (30 ml) was added m-chloroperbenzoic acid (65%) (2.93 g, 11.0 mmol) under stirring under ice-cooling, and the resulting mixture was stirred at room temperature for 20 hours. After stirring, calcium hydroxide (1.23 g, 16.5 mmol) was added to the reaction mixture under stirring, and the resulting mixture was stirred for 15 minutes and then filtered using celite. The filtrate was evaporated in vacuo, and the residue was purified by chromatography on a silica gel column using a mixed solvent of hexane and ethyl acetate (1 : 3) as the eluent to afford 4-(2-methoxyethyl)sulfonylphenethyl alcohol (alcohol form) (1.26 g) in a yield of 94% as a colourless oily product.
3)
Furthermore, to a solution of the alcohol form (1.04 g, 4.26 mmol) obtained in 2) in dichloromethane (21 ml) were added successively triphenylphosphine (1.34 g, 5.11 mmol) and carbon tetrabromide (1.84 g, 5.53 mmol) under stirring and ice-cooling, and the resulting mixture was stirred at room temperature for one hour. After stirring, the reaction mixture was evaporated in vacuo, and the residue was purified by chromatography on a silica gel column using a mixed solvent of hexane and ethyl acetate (3 : 2) as the eluent to afford the title compound (1.25 g) in a yield of 95% as a colourless oily product.
$^1$H-NMR (400MHz, $CDCl_3$) δppm:
7.88 (2H, d, J=8Hz), 7.42 (2H, d, J=8Hz), 3.75 (2H, t, J=7Hz), 3.61 (2H, t, J=7Hz), 3.39 (2H, t, J=7Hz), 3.27 (2H, t, J=7Hz), 3.23 (3H, s).

(Reference Example 41)

4-[2-(Tetrahydropyran-2-yloxy)ethyl]sulfonylphenethyl bromide

The title compound was synthesized in a yield of 22% as a white crystal by conducting reactions similar to

those mentioned in Reference Example 40, using 2-(tetrahydropyran-2-yloxy)ethyl chloride instead of 2-methoxyethyl chloride.
$^1$H-NMR (400MHz, $CDCl_3$) δppm :
7.88 (2H, d, J=8Hz), 7.41 (2H, d, J=8Hz), 4.51-4.47 (1H, m), 4.09-4.02 (1H, m), 3.84-3.76 (1H, m), 3.75-3.67 (1H, m), 3.59 (2H, t, J=7Hz), 3.50-3.40 (3H, m), 3.26 (2H, t, J=7Hz), 1.62-1.36 (5H, m), 1.36-1.25 (1H, m).

[Formulation Example]

The compounds of the present invention having the general formula (I) or pharmacologically acceptable salts thereof or pharmacological esters thereof, or formulations containing other derivatives thereof as an active ingredient can be manufactured, for example, according to the following methods.

[Formulation Example 1] Powder

Powders are manufactured according to conventional methods, by mixing 5 g of Example 19, 95 g of lactose, and 100 g of corn starch in a Blender.

[Formulation Example 2] Granules

Granules are manufactured according to conventional methods, by mixing 5 g of the compound of Example 23, 865 g of lactose, and 100 g of low substituted hydroxypropylcellulose and then by kneading with 300 g of 10% low substituted hydroxypropylcellulose aqueous solution. After granulating the resulting mixture by an extrusion granulator, the granules are dried.

[Formulation Example 3] Capsules

Single capsules are manufactured by mixing 5 g of the compound of Example 40, 115 mg of lactose, 58 g of corn starch, and 2 g of magnesium stearate with V-shaped blenders, and filling 180 mg of the resulting mixture per single capsule using a capsule filling machine.

(Formulation Example 4) Tablets

Tablets are prepared according to conventional methods, by mixing 5 g of the compound of Example 41, 90 g of corn starch, 20 g of microcrystalline cellulose, and 1 g of magnesium stearate in a Blender, and tableting the mixture using tableting machines.

[Test Example 1]

Inhibition of Production of IL-1β and TNF-α in human whole blood (in vitro)

The experiments were carried out according to methods described by Hartman et al. (D.A. Hartman, S.J. Ochalski and R.P. Carlson; The effects of antiinflammatory and antiallergic drugs on cytokine release after stimulation of human whole blood by lipopolysaccharide and zymosan A: Inflamm. Res., 44, 269 (1995)).
Peripheral blood was collected under heparin from healthy volunteers. 1000 μl of the whole blood was placed into an Eppendorf micro test tube in which 2 μl of dimethylsulfoxide solution of the test compound was previously placed, and 10 μl of lipopolysaccharide (LPS) (from E. coli O26:B6 (Difco Laboratories)) (final concentration: 10 μg/ml) was added as an activator. The resulting mixture was stirred and cultivated at 37°C for 6 hrs under exposure to 5% $CO_2$ gas. After cultivation, the mixture was cooled at 4°C to stop the reaction, immediately centrifuged at 14,000 rpm for 5 min, and the supernatant, which was plasma, was isolated and recovered. IL-1β and TNF-α produced and released into the plasma were determined by enzymatic immunoassay using ELISA kit (Cayman Chemical Co. and Genzyme Co.) . The cytokine levels produced in the presence and absence of the test compound were determined and the rate of inhibition of these inflammatory cytokines elicited by the test compound was calculated. Based on the average inhibitory rate, the $IC_{50}$ value was calculated by the least-square method.
In the present experiments, the compounds of the present invention exhibited excellent inhibitory activities against production of the inflammatory cytokines.

[Test Example 2]

Inhibition of Production of TNF-α (in vivo)

The experiments were carried out according to methods described by Ochalski et al. (S.J. Ochalski, D.A. Hartman, M.T. Belfast, T.L. Walter, K.B. Glaser and R.P. Carlson; Inhibition of endotoxin-induced hypothermia and serum TNF-α levels in CD-1 mice by various pharmacological agents: Agents Actions 39, C52-C54 (1993)).

Production of TNF-α was induced by intravenous injection of LPS into the tail vein of mice. The LPS (from E. coli 026:B6 (Difco)) in saline prepared as to be 0.045 mg/ml of concentration was injected into the tail vein of Balb/c mice (male: 5 mice, 7 weeks of age, body weight: approximately 22 g, Charles River Japan Inc.) that were fasted overnight at a volume of 10 ml/kg body weight. One hour later, the mice were laparotomized under ether anesthesia and blood was collected from the abdominal aorta. A disposable injection syringe to which was attached an injection needle of 23 gauge with the inner wall of the syringe wetted by heparin solution) with 1 ml volume was used for the blood collection. Immediately after collection, the blood was transferred into an Eppendorf micro test tube of 1.5 μl volume and centrifuged at 4°C and 14,000 rpm for isolation of plasma. The plasma obtained was stored at -20°C until assay of TNF-α.

TNF-α contained in the plasma was determined by enzymatic immunoassay using ELISA kit. (Cayman Chemical Co. and Genzyme Co.) .

The test compound was suspended in 0.5% tragacantha solution and orally administered 30 min prior to the LPS injection at a dose of 10 ml/kg body weight. At least 3 doses of compound were administered to 5 mice each. The average TNF-α level in rats administered each dose of the test compound compared with that of untreated control mice was calculated.

In the present experiments, the compounds of the present invention exhibited excellent inhibitory activities against production of TNF-α.

[Test Example 3]

Inhibition of Production of IL-β (in vivo)

The experiments were carried out according to methods described by Griffith et al. (Richard J. Griffiths, Ethan J. Stam, James T. Downs and Ivan G. Otterness; ATP Induces the Release of IL-1 from LPS-Primed Cells In Vivo: J. Immunol., 154, 2821-2828 (1995)).

Production of IL-β was induced by intraperitoneal injection of LPS and adenosine triphosphate (ATP) in mice. The LPS (from E. coli 026:B6 (Difco)) in saline prepared as to be 0.0045 mg/ml of concentration was intraperitoneally injected in Balb/c mice (male: 5 mice, 7 weeks of age, body weight: approximately 22 g, Charles River Japan Inc.) that were fasted overnight at a volume of 10 ml/kg body weight. Two hours later, 0.5 ml of ATP prepared in saline (6.03 mg/ml) was intraperitoneally injected in the mice. At 0.5 hr after the ATP injection, the mice were sacrificed by asphyxiation with dri-ice and immediately after that phosphate buffered saline (PBS, containing 10 U/ml of heparin, 0.25 ml of PMSF, 1 μg/ml of leupepsin, 1μg/ml of pepstatin, and 1 mM EDTA) for washing was intraperitoneally injected and washed. A disposable injection syringe with 1 ml volume to which was attached an injection needle 21G was used for recovery of the washing fluid. Immediately after recovery, the washed fluid recovered from the abdominal cavity was placed into an Eppendorf micro test tube of 1.5ug volume and centrifuged at 4°C and 7,500 rpm for isolation of supernatant. The supernatant was stored at -20°C until assay of IL-β.

IL-β level was determined by enzymatic immunoassay using ELISA kit (mouse ELISA KIT, Genzyme Co).

The test compound was suspended in 0.5% tragacantha solution and orally administered 30 min prior to the LPS injection at a dose of 10 ml/kg body weight. The average IL-β level in rats administered each dose of the test compound versus that of untreated control mice was calculated.

In the present experiments, the compounds of the present invention exhibited excellent inhibitory activities against production of IL-β.

[Test Example 4]

Activity in Preventing the Development of Adjuvant-Induced Arthritis (in vivo)

The test was performed according to the method described by Winder et al. (Arthritis Rheum., 12, 472-482, 1969).

Heat-killed dried Mycobacterium butyricum (Difco Laboratories, Lot 679123) was ground on an agate mortar, and was then suspended in dry-sterilised liquid paraffin (first grade, Wako Pure Chemical Industries, Ltd.) to make a 2 mg/ml suspension. The resulting suspension was then sonicated and used as an adjuvant. Arthritis was induced by the

intradermal injection of the adjuvant (100 μg of heat killed dried bacterium/0.05 ml of paraffin/paw) into the heel of the right hind paw of a Lewis rat (male, age 9 weeks, 190 g, Japan Charles River). The test compounds, which had been suspended in a 0.5% aqueous sodium carboxymethyl cellulose solution (CMC, Daiichi Pure Chemicals, Co., Ltd.), were administered orally at the rate of 5 ml/kg once a day from the day of injection of the adjuvant (day 0) to day 20.

The volumes of the right hind paw (adjuvant-inj ected paw) and left hind paw (non-injected paw) were measured on days 3, 5, 7, 10, 13, 15, 18 and 21 using a Plethysmometer™ (Ugo Basile), the hind paws being soaked from the toe to the hairline in the bath of the Plethysmometer™. The volumes of the swollen feet (adjuvant-injected right hind foot volume - non-injected left hind foot volume) were calculated. The percent inhibition of swelling of the injected foot of the treated animals as compared to that of the control animals on day 21 was calculated as follows.

Inhibition (%) = {1-(swollen foot volume of compound-treated animals)/

(swollen foot volume of control animals)} × 100

In the present experiments, the compounds of the present invention exhibited excellent inhibitory activities against adjuvant-induced arthritis.

[Test Example 5]

Activity in Preventing the Development of Arthritis Induced by Anti-Collagen Antibody (in vivo)

In this test, an anti-collagen antibody-induced mouse arthritis model was employed.

Anti-collagen antibody solution (4 mg/ml, Immuno-Biological Laboratories Co., Ltd., Arthritogenic mAb Cocktail) was injected into the tail vein of Balb/c mice (male, 5-6 weeks of age, Charles River Japan Inc.) at a volume of 0.5 ml, and 3 days later lipopolysaccharide solution (0.5 mg/ml, Immuno-Biological Laboratories Co., Ltd., Arthritogenic mAb Cocktail) was intraperitoneally injected at a volume of 0.1 ml and arthritis was thereby induced.

The test compound was suspended in 0.5% tragacantha solution and orally administered for 7 days starting on the day of injection of the anti-collagen antibody solution once daily at a dose of 10 ml/kg body weight. Vehicle (0.5% tragacantha solution) was administered to control mice instead of suspended solution of the test compound.

After administration of either the test compound or tragacantha solution, swelling of the 4 limbs was scored according the following criteria:

0: normal (no swelling is observed),
1: swelling is observed in one digit,
2: swelling is observed in 2 and more digits, and
3: whole limb is swollen.

The score of each limb of individual mouse was summed and the summed value was expressed as the swelling score of the animal. Inhibitory rate of each individual animal was calculated from the individual swelling score in the drug treated group against the average of the swelling score in the control group, and the inhibitory activity of the compound was determined from the inhibitory rate.

In the present experiments, the compounds of the present invention exhibited excellent inhibitory activities against arthritis induced by anti-collagen antibody.

[Test Example 6]

Activity in Treating Arthritis Induced by Anti-Collagen Antibody (in vivo)

In this test, an anti-collagen antibody-induced mouse arthritis model was employed.

Anti-collagen antibody solution (4 mg/ml, Immuno-Biological Laboratories Co., Ltd., Arthritogenic mAb Cocktail)

was injected into the tail vein of Balb/c mice (male, 5-6 weeks of age, Charles River Japan Inc.) at a volume of 0.5 ml, and 3 days later lipopolysaccharide solution (0.5 mg/ml, Immuno-Biological Laboratories Co., Ltd., Arthritogenic mAb Cocktail) was intraperitoneally injected at a volume of 0.1 ml and arthritis was thereby induced.

The degree of swelling was scored on 7th day after the anti-collagen antibody was injected according to the criteria described in Test Eaxmple 5. Mice whose hindlimb scores on both sides were 3 and higher were selected, and therapeutic effects of the compounds were investigated in these selected mice.

The test compound was suspended in 0.5% tragacantha solution and orally administered for 3 days starting on the day of selection of the mice once a day at a dose of 10 ml/kg of the body weight. Vehicle (0.5% tragacantha solution) was administered to the control mice instead of suspended solution of the test compound.

After administration of either the test compound or tragacantha solution, swelling of the 4 limbs was scored again according the criteria described in Test Example 5. From the individual swelling score in the drug-treated group against the avarage of the swelling score in the control group, the inhibitory rate of the compound at the given dose was calculated and the inhibitory activity of the compound was determined from the inhibitory rate.

In the present experiments, the compounds of the present invention exhibited excellent inhibitory activities against arthritis induced by anti-collagen antibody.

[Test Example 7]

Determination of survival effects of the compounds on lethality of mice induced by galactosamine (GalN) and lipolysaccharide (LPS) (a model of septicemic disease)

Septicemic disease was induced by intravenous injection of GalN and LPS into the tail vein of the mouse.

GalN (Sigma Chemical Company) solution prepared at a concentration of 1 g/5 ml with saline and LPS (Sigma Chemical Company) solution prepared at a concentration of 0.05 mg/5 ml with saline were mixed at equivalent volume of each. The mixed solution was injected into the tail vein of the mouse [C3H/He.N (male, 7 weeks of age, Charles River Japan Inc.) at a volume of 10 ml/kg and lethality due to septicemic disease was thereby induced.

The test compound was dissolved in saline and intravenously injected into the tail vein of the mouse immediately before injection of the mixed solution of GalN and LPS at a volume of 10 ml/kg.

Effects of the test compound on survival rate of the mice were determined by observation of the mice for 3 days after induction of lethality.

[Industrial applicability]

Since cyclic tertiary amine compounds of the present invention exert excellent inhibitory action against production of inflammatory cytokines, exhibit excellent oral absorption, and have low toxicities, the compounds of the present invention are useful as an active ingredient in an agent against diseases mediated by inflammatory cytokines in warm-blooded animals (preferably humans). For example, the compounds of the present invention are useful as antipyretic, analgesic and anti-inflammatory drugs and antiviral agents, and as prophylactic or therapeutic agents for rheumatoid arthritis, osteoarthritis, allergic diseases, asthma, septicemic disease, psoriasis, osteoporosis, autoimmune diseases (for example, diffuse lupus erythematosus, ulcerative colitis, Crohn's disease, or the like), diabetes mellitus (particularly type I diabetes mellitus), nephritis, hepatitis, tumour, ischemic heart disease, Alzheimer's disease, or arterial sclerosis, preferably as antipyretic, analgesic and anti-inflammatory drugs and as prophylactic or therapeutic agents for rheumatoid arthritis, osteoarthritis, allergic diseases, septicemic disease, psoriasis, osteoporosis, ulcerative colitis, diabetes mellitus (particularly type I diabetes mellitus), hepatitis, arterial sclerosis, or Crohn's disease, particularly preferably as antipyretic, analgesic and anti-inflammatory drugs and as prophylactic or therapeutic agents for rheumatoid arthritis, osteoarthritis, septicemic disease, psoriasis, Crohn's disease, ulcerative colitis, diabetes mellitus (particularly type I diabetes mellitus), or hepatitis.

## Claims

**1.** A compound having the general formula (I),

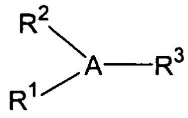 (I)

wherein, A represents a trivalent group selected from the group consisting of benzene, pyridine, pyridazine, pyrimidine, pyrrole, furan, thiophene, pyrazole, imidazole, isoxazole and isothiazole which may optionally be substituted with group(s) selected from Substituent group $\delta$,
$R^1$ represents an aryl group which may optionally be substituted with group(s) selected from the group consisting of Substituent group $\alpha$ and Substituent group $\beta$; or a heteroaryl group which may optionally be substituted with group (s) selected from the group consisting of Substituent group $\alpha$ and Substituent group $\beta$,
$R^2$ represents a heteroaryl group which contains at least one nitrogen atom and further may optionally be substituted with group(s) selected from the group consisting of Substituent group $\alpha$ and Substituent group $\beta$, and
$R^3$ represents a group having general formula (IIa) or (IIb) shown below:

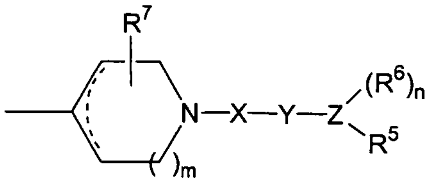

(IIa)

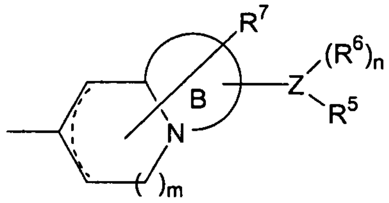

(IIb)

[wherein,
the bond including a dotted line moiety represents a single bond or a double bond,
Ring B represents a 4- to 7-membered heterocyclyl ring (said ring is saturated or unsaturated; and may optionally be fused with another group such as an aryl group, a heteroaryl group, a cycloalkyl group or a heterocyclyl group),
X represents a straight or branched alkylene group having from 1 to 5 carbon atoms,
Y represents a single bond or a group having formula: $C(R^8a)$ $(R^8b)$ ($R^8a$ and $R^8b$ are the same or different and each represents independently a hydrogen atom, a hydroxyl group, a halogen atom, a lower alkyl group or a lower alkoxy group, or $R^8a$ and $R^8b$ together form an oxo group or a methylene group, or $R^8a$ and $R^8b$ together with the carbon atom to which they are bonded form a 3- to 6-membered cycloalkyl group),
Z represents an arylene group or a heteroarylene group,
m represents an integer of from 0 to 2,
$R^5$ represents a carboxyl group, a lower alkoxycarbonyl group, an aralkyloxycarbonyl group, an aryloxycarbonyl group, a group having formula: $CONR^aR^b$, a group having formula: $COR^c$, a group having formula: $SO_2NR^aR^b$, a group having formula: $SO_2R^c$ or a group having formula: $SOR^c$,
$R^a$ and $R^b$ are the same or different and each represents independently a hydrogen atom; a hydroxy group; a lower alkye group which may optionally be substituted with group(s) selected from Substituent group $\alpha$; a lower alkenyl group which may optionally be substituted with group(s) selected from Substituent group $\alpha$; a lower alkynyl group which may optionally be substituted with group(s) selected from Substituent group $\alpha$; a lower alkoxy group; a lower alkenyloxy group; a lower alkynyloxy group; an aralkyloxy group; a cycloalkyl group; a lower alkyl group substituted with a cycloalkyl group; an aryl group; an aralkyl group; a heteroaryl group; a lower alkyl group substituted with a heteroaryl group; an amino group; or a mono- or di-lower alkylamino group,
$R^c$ represents a hydrogen atom, a lower alkyl group, a halogeno lower alkyl group, a lower alkoxy lower alkyl group or a hydroxy lower alkyl group,
$R^6$ represents 1 to 2 groups selected from
the group consisting of a hydrogen atom, a hydroxyl group, an amino group, a nitro group, a cyano group, a halogen atom, a lower alkyl group, a lower alkoxy group, a lower alkylthio group, a halogeno lower alkyl group, a halogeno lower alkoxy group and a halogeno lower alkylthio group,
n represents an integer of from 1 to 2 (in the case that n is 2, each $R^6$ may be the same or different),
$R^7$ represents. 1 to 3 groups selected from the group consisting of a hydrogen atom, a hydroxyl group, a halogen

atom, a lower alkyl group, a lower alkoxy group, a lower alkylthio group, a halogeno lower alkyl group, a halogeno lower alkoxy group and a halogeno lower alkylthio group],
Substituent group α is the group consisting of a hydroxyl group, a nitro group, a cyano group, a halogen atom, a lower alkoxy group, a halogeno lower alkoxy group, a carboxyl group, a lower alkoxycarbonyl group, a carbamoyl group, a lower acyloxy group, a lower alkylthio group, a halogeno lower alkylthio group and a group having formula: $-NR^dR^e$ (wherein, $R^d$ and $R^e$ are the same or different and each represents independently a hydrogen atom, a lower alkyl group, a lower alkenyl group, a lower alkynyl group, an aralkyl group, a lower alkylsulfonyl group or a lower alkylcarbonyl group, or $R^d$ and $R^e$ together with the nitrogen atom to which they are bonded form a heterocyclyl group),
Substituent group β is the group consisting of a lower alkyl group which may optionally be substituted with group (s) selected from Substituent group α; a lower alkenyl group which may optionally be substituted with group(s) selected from Substituent group α; a lower alkynyl group which may optionally be substituted with group(s) selected from Substituent group α; an aralkyl group and a cycloalkyl group,
Substituent group γ is the group consisting of an oxo group, a hydroxyimino group, a lower alkoxyimino group, a lower alkylene group, a lower alkylenedioxy group, a lower alkylsulfinyl group and a lower alkylsulfonyl group,
Substituent group δ is the group consisting of: a group selected from Substituent group β; a cycloalkyl group substituted with group(s) selected from the group consisting of Substituent group α, Substituent group β and Substituent group γ; an aryl group which may optionally be substituted with group(s) selected from the group consisting of Substituent group α, Substituent group β and Substituent group γ; a heteroaryl group which may optionally be substituted with group(s) selected from the group consisting of Substituent group α, Substituent group β and Substituent group γ; and a heterocyclyl group which may optionally be substituted with group(s) selected from the group consisting of Substituent group α, Substituent group β and Substituent group γ, or a pharmacologically acceptable salt thereof,
PROVIDED THAT each of the atoms of the Ring A to which $R^1$ and $R^3$ are bonded is adjacent to the atom of the Ring A to which $R^2$ is bonded.

**2.** A compound according to Claim 1 wherein A is a trivalent group selected from the group consisting of a pyrrole group which may optionally be substituted with two groups selected from Substituent group δ and a pyrazole group which may optionally be substituted with one group selected from Substituent group δ, or a pharmacologically acceptable salt thereof.

**3.** A compound according to Claim 1 wherein A is a pyrrole group which may optionally be substituted with two groups selected from Substituent group δ, or a pharmacologically acceptable salt thereof.

**4.** A compound according to Claim 1 wherein A is a pyrrole group, or a pharmacologically acceptable salt thereof.

**5.** A compound according to any one claim selected from Claim 1 to Claim 4 wherein $R^1$ is an aryl group which may optionally be substituted with group(s) selected from the group consisting of Substituent group α and Substituent group β, or a pharmacologically acceptable salt thereof.

**6.** A compound according to any one claim selected from Claim 1 to Claim 4 wherein $R^1$ is a phenyl group or a naphthyl group which may optionally be substituted with group(s) selected from the group consisting of Substituent group α and Substituent group β, or a pharmacologically acceptable salt thereof.

**7.** A compound according to any one claim selected from Claim 1 to Claim 4 wherein $R^1$ is a phenyl group which may optionally be substituted with group(s) selected from the group consisting of Substituent group $\alpha^1$ and Substituent group $\beta^1$,
Substituent group $\alpha^1$ is the group consisting of a hydroxyl group, a cyano group, a halogen atom, a lower alkoxy group and a halogeno lower alkoxy group, and
Substituent group $\beta^1$ is the group consisting of a lower alkyl group, a halogeno lower alkyl group and a hydroxy lower alkyl group, or a pharmacologically acceptable salt thereof.

**8.** A compound according to any one claim selected from Claim 1 to Claim 4 wherein $R^1$ is a phenyl group or a phenyl group which is substituted with group(s) selected from the group consisting of a hydroxyl group, a cyano group, a halogen atom, a lower alkoxy group, a halogeno lower alkyl group and a halogeno lower alkoxy group, or a pharmacologically acceptable salt thereof.

**9.** A compound according to any one claim selected from Claim 1 to Claim 4 wherein $R^1$ is a phenyl, 3-cyanophenyl,

4-fluorophenyl, 3-fluorophenyl, 4-chlorophenyl, 3-chlorophenyl, 2,4-difluorophenyl, 3,4-difluorophenyl, 3,4-dichlorophenyl, 3,4,5-trifluorophenyl, 3-chloro-4-fluorophenyl, 3-methoxyphenyl, 3-difluoromethoxyphenyl, 3-trifluoromethoxyphenyl, 3-trifluoromethylphenyl or 4-fluoro-3-methoxyphenyl group, or a pharmacologically acceptable salt thereof.

**10.** A compound according to any one claim selected from Claim 1 to Claim 4 wherein $R^1$ is a phenyl, 3-cyanophenyl, 4-fluorophenyl, 3-fluorophenyl, 3-chlorophenyl, 3,4-difluorophenyl, 3,4,5-trifluorophenyl, 3-chloro-4-fluorophenyl, 3-methoxyphenyl, 3-difluoromethoxyphenyl, 3-trifluoromethylphenyl or 4-fluoro-3-methoxyphenyl, or a pharmacologically acceptable salt thereof.

**11.** A compound according to any one claim selected from Claim 1 to Claim 4 wherein $R^1$ is a phenyl, 4-fluorophenyl, 3-fluorophenyl, 3-chlorophenyl, 3,4-difluorophenyl, 3-chloro-4-fluorophenyl or 3-trifluoromethylphenyl group, or a pharmacologically acceptable salt thereof.

**12.** A compound according to any one claim selected from Claim 1 to Claim 11 wherein $R^2$ is a 5- to 6-membered heteroaryl group which contains one or two nitrogen atoms and further may optionally be substituted with group(s) selected from the group consisting of Substituent group $\alpha$ and Substituent group $\beta$, or a pharmacologically acceptable salt thereof.

**13.** A compound according to any one claim selected from Claim 1 to Claim 11 wherein $R^2$ is a pyridyl group or a pyrimidinyl group which may optionally be substituted with group(s) selected from the group consisting of Substituent group $\alpha$ and Substituent group $\beta$, or a pharmacologically acceptable salt thereof.

**14.** A compound according to any one claim selected from Claim 1 to Claim 11 wherein $R^2$ is a 4-pyridyl group or a 4-pyrimidinyl group which may optionally be substituted with group(s) selected from the group consisting of Substituent group $\alpha$ and Substituent group $\beta$, or a pharmacologically acceptable salt thereof.

**15.** A compound according to any one claim selected from Claim 1 to Claim 11 wherein $R^2$ is a 4-pyridyl group or a 4-pyrimidinyl group, of which the 2-position may optionally be substituted with one group selected from the group consisting of Substituent group $\alpha$ and Substituent group $\beta$, or a pharmacologically acceptable salt thereof.

**16.** A compound according to any one claim selected from Claim 1 to Claim 11 wherein $R^2$ is a 4-pyridyl group or a 4-pyrimidinyl group, of which the 2-position may optionally be substituted with one group selected from the group consisting of a methoxy, amino, methylamino, benzylamino and $\alpha$-methylbenzylamino group, or a pharmacologically acceptable salt thereof.

**17.** A compound according to any one claim selected from Claim 1 to Claim 16 wherein m is 1, or a pharmacologically acceptable salt thereof.

**18.** A compound according to any one claim selected from Claim 1 to Claim 17 wherein $R^3$ is a group having formula: (IIa), X is an alkylene group having from 1 to 4 carbon atoms, Y is a group having formula: C($R^8$a) ($R^8$b) ($R^8$a and $R^8$b are the same or different and each represents independently a hydrogen atom, a hydroxyl group, a halogen atom, an alkyl group having from 1 to 4 carbon atoms or an alkoxy group having from 1 to 4 carbon atoms, or $R^8$a and $R^8$b together form an oxo group or methylene group, or $R^8$a and $R^8$b together with the carbon atom to which they are bonded form a 3-to 6-membered cycloalkyl group), or a pharmacologically acceptable salt thereof.

**19.** A compound according to any one claim selected from Claim 1 to Claim 17 wherein $R^3$ is a group having formula: (IIa); X is a methylene group; and Y is a group having formula: C($R^8$a) ($R^8$b) ($R^8$a and $R^8$b are the same or different and each represents independently a hydrogen atom, a hydroxyl group, a fluorine atom, a methyl group, an ethyl group, a methoxy group or an ethoxy group, or $R^8$a and $R^8$b together form an oxo group or a methylene group, or $R^8$a and $R^8$b together with the carbon atom to which they are bonded form a cyclopropyl group), or a pharmacologically acceptable salt thereof.

**20.** A compound according to any one claim selected from Claim 1 to Claim 17 wherein $R^3$ is a group having formula: (IIa); X is a methylene group; and Y is a group having formula: C($R^8$a) ($R^8$b) ($R^8$a and $R^8$b are the same or different and each represents independently a hydrogen atom, a fluorine atom, a methyl group, a hydroxyl group or an oxo group), or a pharmacologically acceptable salt thereof.

**21.** A compound according to any one claim selected from Claim 1 to Claim 17 wherein $R^3$ is a group having formula: (IIa); X is a methylene group; and Y is a group having formula: $CH_2$, or a pharmacologically acceptable salt thereof.

**22.** A compound according to any one claim selected from Claim 1 to Claim 17 wherein $R^3$ is a group having formula: (IIb), and Ring B is a 5- or 6-membered heterocyclyl ring which contains one nitrogen atom and further may optionally contain one atom or group selected from the group consisting of a nitrogen atom, an oxygen atom, a sulfur atom, a group having formula: =SO and a group having formula: $=SO_2$ (said ring is saturated or unsaturated, and may optionally be fused with an aryl group, a heteroaryl group, a cycloalkyl group or a heterocyclyl group), or a pharmacologically acceptable salt thereof.

**23.** A compound according to any one claim selected from Claim 1 to Claim 17 wherein $R^3$ is a group having formula: (IIb), and Ring B is a 5- or 6-membered heterocyclyl ring which contains one nitrogen atom (said ring is saturated or unsaturated, and may optionally be fused with an aryl group, a heteroaryl group, a cycloalkyl group or a heterocyclyl group), or a pharmacologically acceptable salt thereof.

**24.** A compound according to any one claim selected from Claim 1 to Claim 17 wherein $R^3$ is a group having formula: (IIb), and Ring B is a pyrrolidine or pyrroline group, or a pharmacologically acceptable salt thereof.

**25.** A compound according to any one claim selected from Claim 1 to Claim 24 wherein Z is a phenylene group, a thiophenediyl group, a furandiyl group, a pyrrolediyl group, an oxazolediyl group, a thiazolediyl group, a thiadiazolediyl group or a pyridinediyl group, or a pharmacologically acceptable salt thereof.

**26.** A compound according to any one claim selected from Claim 1 to Claim 24 wherein Z is a phenylene group or a thiophenediyl group, or a pharmacologically acceptable salt thereof.

**27.** A compound according to any one claim selected from Claim 1 to Claim 26 wherein $R^5$ is a group having formula: $CONR^aR^b$, a group having formula: $COR^c$, a group having formula: $SO_2NR^aR^b$ , a group having formula: $SO_2R^c$ or a group having formula: $SOR^c$ (wherein, $R^a$ and $R^b$ are the same or different and each is independently a hydrogen atom, a lower alkyl group which may optionally be substituted with group(s) selected from Substituent group $\alpha$, a lower alkoxy group, a lower alkenyloxy group, a cycloalkyl group, an amino group, or mono- or di-lower alkylamino group; and $R^c$ is a lower alkyl group), or a pharmacologically acceptable salt thereof.

**28.** A compound according to any one claim selected from Claim 1 to Claim 26 wherein $R^5$ is a group having formula: $CONR^aR^b$, a group having formula: $SO_2NR^aR^b$, a group having formula: $SO_2R^c$ or a group having formula: $SOR^c$ (wherein, $R^a$ and $R^b$ are the same or different and each is independently a hydrogen atom, a lower alkyl group, a halogeno lower alkyl group, a hydroxy lower alkyl group, a lower alkoxy lower alkyl group, a lower alkoxy group or a cycloalkyl group; and $R^c$ is a lower alkyl group), or a pharmacologically acceptable salt thereof.

**29.** A compound according to any one claim selected from Claim 1 to Claim 26 wherein $R^5$ is a carbamoyl, N-methylcarbamoyl, N-ethylcarbamoyl, N-propylcarbamoyl, N-isopropylcarbamoyl, N-cyclopropylcarbamoyl, N-cyclopentylcarbamoyl, N-(2-fluoroethyl)carbamoyl, N-(2-methoxyethyl)carbamoyl, N-methylsulfamoyl, N-ethylsulfamoyl, N-propylsulfamoyl, N-isopropylsulfamoyl, N-cyclopropylsulfamoyl, N-cyclopentylsulfamoyl, N-(2-fluoroethyl)sulfamoyl, N-(methoxyethyl)sulfamoyl, N-methoxysulfamoyl, methanesulfonyl, ethanesulfonyl, propanesulfonyl, methanesulfinyl, ethanesulfinyl or propanesulfinyl group, or a pharmacologically acceptable salt thereof.

**30.** A compound according to any one claim selected from Claim 1 to Claim 29 wherein $R^6$ is 1 to 2 groups selected from the group consisting of a hydrogen atom, a fluorine atom and a methoxy group, or a pharmacologically acceptable salt thereof.

**31.** A compound according to any one claim selected from Claim 1 to Claim 29 wherein $R^6$ is a hydrogen atom, or a pharmacologically acceptable salt thereof.

**32.** A compound according to any one claim selected from Claim 1 to Claim 31 wherein $R^7$ is 1 to 2 groups selected from the group consisting of a hydrogen atom, a hydroxyl group and a lower alkyl group, or a pharmacologically acceptable salt thereof.

**33.** A compound according to any one claim selected from Claim 1 to Claim 31 wherein $R^7$ is a hydrogen atom, or a pharmacologically acceptable salt thereof.

**34.** A compound according to any one claim selected from Claim 1 to Claim 33 wherein the compound having the general formula (I) is a compound having one of the following general formulae:

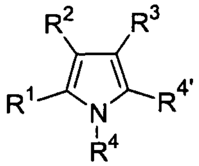 , 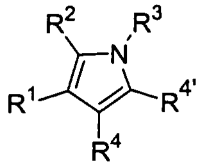 , 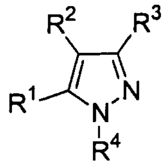 or 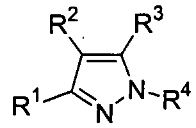

(wherein, $R^4$ and $R^{4'}$ are the same or different and each represents independently a hydrogen atom or a group selected from Substituent group δ, or a pharmacologically acceptable salt thereof.

**35.** A compound according to any one claim selected from Claim 1 to Claim 33 wherein the compound having the general formula (I) is a compound having either one of the following general formulae:

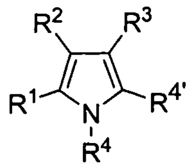 or 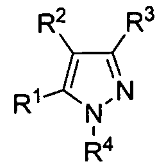

(wherein $R^4$ and $R^{4'}$ are the same or different and each represents independently a hydrogen atom or a group selected from Substituent group δ), or a pharmacologically acceptable salt thereof.

**36.** A compound according to Claim 1 wherein the compound having the general formula (I) is a compound selected from the compounds shown below, or a pharmacologically acceptable salt thereof:

4-[1-(4-Carbamoylphenethyl)-1,2,3,6-tetrahydropyridin-4-yl]-2-(4-fluorophenyl)-3-(pyridin-4-yl)-1H-pyrrole,
3-[1-(4-Carbamoylphenethyl)-1,2,3,6-tetrahydropyridin-4-yl]-5-(4-fluorophenyl)-4-(pyridin-4-yl)-1H-pyrazole,
2-(4-Fluorophenyl)- 4-[1-[4-(N-methylcarbamoyl) phenethyl]- 1,2,3,6-tetrahydropyridin- 4-yl]- 3-(pyridin- 4-yl)-1H-pyrrole,
4-[1-[4-(N-Ethylcarbamoyl)phenethyl]-1,2,3,6-tetrahydropyridin4-yl]-2-(4-fluorophenyl)-3-(pyridin-4-yl)-1H-pyrrole,
4-[1-[4-(N-Cyclopropylcarbamoyl)phenethyl]-1,2,3,6-tetrahydropyridin-4-yl]-2-(4-fluorophenyl)-3-(pyridin-4-yl)-1H-pyrrole,
2-(4-Fluorophenyl)- 4-[1-[4-(N-isopropylcarbamoyl) phenethyl]- 1,2,3,6-tetrahydropyridin- 4-yl]- 3-(pyridin- 4-yl)-1H-pyrrole,
2-(4-Fluorophenyl)- 4-[1-[4-(N-propylcarbamoyl) phenethyl]- 1,2,3,6-tetrahydropyridin- 4-yl]- 3-(pyridin- 4-yl)-1H-pyrrole,
4-[1-[4-[N-(2-Fluoroethyl) carbamoyl] phenethyl]- 1,2,3,6-tetrahydropyridin- 4-yl]- 2-(4-fluorophenyl)- 3-(pyridin-4-yl)-1H-pyrrole,
4-[1-[4-(N-Cyclopentylcarbamoyl)phenethyl]-1,2,3,6-tetrahydropyridin-4-yl]-2-(4-fluorophenyl)-3-(pyridin-4-yl)-1H-pyrrole,
4-[1-[4-[N-(Ethoxycarbonylmethyl) carbamoyl] phenethyl]- 1,2,3,6-tetrahydropyridin- 4-yl]- 2-(4-fluorophenyl)-3-(pyridin-4-yl)-1H-pyrrole,
2-(4-Fluorophenyl)- 4-[1-[4-(N-methylsulfamoyl) phenethyl]- 1,2,3,6-tetrahydropyridin- 4-yl]- 3-(pyridin- 4-yl)-1H-pyrrole,
4-[1-[4-(N-Ethylsulfamoyl)phenethyl]-1,2,3,6-tetrahydropyridin-4-yl]-2-(4-fluorophenyl)-3-(pyridin-4-yl)-1H-pyrrole,
2-(4-Fluorophenyl)- 4-[1-[4-(N-propylsulfamoyl) phenethyl]- 1,2,3,6-tetrahydropyridin- 4-yl]- 3-(pyridin- 4-yl)-1H-pyrrole,
2-(4-Fluorophenyl)- 4-[1-[4-(N-isopropylsulfamoyl) phenethyl]- 1,2,3,6-tetrahydropyridin- 4-yl]- 3-(pyridin- 4-yl)-1H-pyrrole,

2-(3-Chloro-4-fluorophenyl)-4-[1-[4-(N-methylsulfamoyl)phenethyl]-1,2,3,6-tetrahydropyridin-4-yl]-3-(pyridin-4-yl)-1H-pyrrole,
4-[1-[4-(N-Cyclopropylsulfamoyl)phenethyl]-1,2,3,6-tetrahydropyridin-4-yl]-2-(4-fluorophenyl)-3-(pyridin-4-yl)-1H-pyrrole,
4-[1-[4-[N-(2-Fluoroethyl) sulfamoyl] phenethyl]-1,2,3,6-tetrahydropyridin-4-yl]-2-(4-fluorophenyl)-3-(pyridin-4-yl)-1H-pyrrole,
4-[1-[4-(N, N-Dimethylsulfamoyl)phenethyl]-1,2,3,6-tetrahydropyridin-4-yl]-2-(4-fluorophenyl)-3-(pyridin-4-yl)-1H-pyrrole,
2-(4-Fluorophenyl)-4-[1-[4-[N-(2-methoxyethyl)sulfamoyl]phenethyl]-1,2,3,6-tetrahydropyridin-4-yl]-3-(pyridin-4-yl)-1H-pyrrole,
4-[1-[4-(N-Ethoxysulfamoyl) phenethyl]-1,2,3,6-tetrahydropyridin-4-yl]-2-(4-fluorophenyl)-3-(pyridin-4-yl)-1H-pyrrole,
4-[1-[4-(N-Allyloxysulfamoyl) phenethyl]-1,2,3,6-tetrahydropyridin-4-yl]-2-(4-fluorophenyl)-3-(pyridin-4-yl)-1H-pyrrole,
2-(4-Fluorophenyl)-4-[1-(4-methanesulfonylphenethyl]-1,2,3,6-tetrahydropyridin-4-yl]-3-(pyridin-4-yl)-1H-pyrrole,
2-(3-Chloro-4-fluorophenyl)-4-[1-(4-methanesulfonyl) phenethyl]-1,2,3,6-tetrahydropyridin-4-yl]-3-(pyridin-4-yl)-1H-pyrrole,
2-(4-Fluorophenyl)-4-[1-(4-methanesulfinylphenethyl)-1,2,3,6-tetrahydropyridin-4-yl]-3-(pyridin-4-yl)-1H-pyrrole,
2-(3-Chloro-4-fluorophenyl)-4-[1-(4-methanesulfinylphenethyl)-1,2,3,6-tetrahydropyridin-4-yl]-3-(pyridin-4-yl)-1H-pyrrole,
4-[1-(4-Acetylphenethyl)-1,2,3,6-tetrahydropyridin-4-yl]-2-(3-chloro-4-fluorophenyl)-3-(pyridin-4-yl)-1H-pyrrole,
2-(3-Chloro-4-fluorophenyl)-4-[(2S, 8aS)-2-[4-(N-methylcarbamoyl) phenyl]-1,2,3,5,6,8a-hexahydroindolidin-7-yl]-3-(pyridin-4-yl)-1H-pyrrole,
4-[(2S, 8aS)-2-(4-Carbamoylphenyl)-1,2,3,5,6,8a-hexahydroindolidin-7-yl]-2-(4-fluorophenyl)-3-(pyridin-4-yl)-1H-pyrrole,
2-(4-Fluorophenyl)-4-[(2S,8aS)-2-[4-(N-methylcarbamoyl)phenyl]-1,2,3,5,6,8a-hexahydroindolidin-7-yl]-3-(pyridin-4-yl)-1H-pyrrole,
4-[(2S,8aS)-2-[4-(N-Ethylcarbamoyl)phenyl]-1,2,3,5,6,8a-hexahydroindolidin-7-yl]-2-(4-fluorophenyl)-3-(pyridin-4-yl)-1H-pyrrole,
4-[(2S, 8aS)-2-[4-(N-Benzylcarbamoyl)phenyl]-1,2,3,5,6,8a-hexahydroindolidin-7-yl]-2-(4-fluorophenyl)-3-(pyridin-4-yl)-1H-pyrrole,
4-[(2S, 8aS)-2-[4-(N-Cyclopropylcarbamoyl) phenyl]-1,2,3,5,6,8a-hexahydroindolidin-7-yl]-2-(4-fluorophenyl)-3-(pyridin-4-yl)-1H-pyrrole,
4-[(2S,8aS)-2-[4-[N-(2-fluoroethyl)carbamoyl]phenyl]-1,2,3,5,6,8a-hexahydroindolidin-7-yl]-2-(4-fluorophenyl)-3-(pyridin-4-yl)-1H-pyrrole,
2-(4-Fluorophenyl)-4-[(2S,8aS)-2-[4-(N-propylcarbamoyl)phenyl]-1,2,3,5,6,8a-hexahydroindolidin-7-yl]-3-(pyridin-4-yl)-1H-pyrrole,
2-(4-Fluorophenyl)-4-[(2S, 8aS)-2-[4-(N-isopropylcarbamoyl) phenyl]-1,2,3,5,6,8a-hexahydroindolidin-7-yl]-3-(pyridin-4-yl)-1H-pyrrole,
2-(4-Fluorophenyl)-4-[(2S, 8aS)-2-[4-[N-(2-methoxyethyl) carbamoyl] phenyl]-1,2,3,5,6,8a-hexahydroindolidin-7-yl]-3-(pyridin-4-yl)-1H-pyrrole,
2-(4-Fluorophenyl)-4-[1-[4-(2-methoxyethyl)sulfonylphenethyl]-1,2,3,6-tetrahydropyridin-4-yl]-3-(pyridin-4-yl)-1H-pyrrole,
2-(4-Fluorophenyl)-4-[1-[4-(2-hydroxyethyl) sulfonylphenethyl]-1,2,3,5,6-tetrahydropyridin-4-yl]-3-(pyridin-4-yl)-1H-pyrrole,
2-(3-Chloro-4-fluorophenyl)-4-[1-[4-(2-methoxyethyl)sulfonylphenethyl]-1,2,3,6-tetrahydropyridin-4-yl]-3-(pyridin-4-yl)-1H-pyrrole, and
2-(3-Chloro-4-fluorophenyl)-4-[1-[4-(2-hydroxyethyl)sulfonylphenethyl]-1,2,3,6-tetrahydropyridin-4-yl]-3-(pyridin-4-yl)-1H-pyrrole.

**37.** A medicament containing a compound according to any one claim selected from Claim 1 to Claim 36 or a pharmacologically acceptable salt thereof as an active ingredient.

**38.** A medicament according to Claim 37 for use as a prophylactic or therapeutic agent against diseases mediated by inflammatory cytokines.

**39.** A medicament according to Claim 37 for use as an anti-pyretic analgesic and anti-inflammatory agent which exerts inhibitory activity against production of inflammatory cytokines.

**40.** A medicament according to Claim 37 for use as a prophylactic or therapeutic agent against rheumatoid arthritis.

**41.** A medicament according to Claim 37 for use as a prophylactic or therapeutic agent against osteoarthritis.

**42.** A medicament according to Claim 37 for use as a prophylactic or therapeutic agent against septicemic disease, psoriasis, Crohn's disease, ulcerative colitis, diabetes mellitus, or hepatitis.

**43.** A method of inhibiting production of inflammatory cytokines by administration of an effective amount of a compound as defined in any one claim selected from Claim 1 to Claim 36 or a pharmacologically acceptable salt thereof to a warm-blooded animal.

**44.** A method according to Claim 43 wherein the warm-blooded animal is a human.

**45.** A method for prevention or treatment of diseases associated with inflammatory cytokines by administration of an effective amount of a compound as defined in any one claim selected from Claim 1 to Claim 36 or a pharmacologically acceptable salt thereof to a warm-blooded animal.

**46.** A method according to Claim 45 wherein the warm-blooded animal is a human.

**47.** A method for treatment or relief of fever, pain and/or inflammation by administration of an effective amount of a compound as defined in any one claim selected from Claim 1 to Claim 36 or a pharmacologically acceptable salt thereof to a warm-blooded animal.

**48.** A method according to Claim 47 wherein the warm-blooded animal is a human.

**49.** A method for prevention or treatment of rheumatoid arthritis by administration of an effective amount of a compound as defined in any one claim selected from Claim 1 to Claim 36 or a pharmacologically acceptable salt thereof to a warm-blooded animal.

**50.** A method according to Claim 49 wherein the warm-blooded animal is a human.

**51.** A method for prevention or treatment of osteoarthritis by administration of an effective amount of a compound as defined in any one claim selected from Claim 1 to Claim 36 or a pharmacologically acceptable salt thereof to a warm-blooded animal.

**52.** A method according to Claim 51 wherein the warm-blooded animal is a human.

**53.** A method for prevention or treatment of septicemic disease, psoriasis, Crohn's disease, ulcerative colitis, diabetes mellitus, or hepatitis by administration of an effective amount of a compound as defined in any one claim selected from Claim 1 to Claim 36 or a pharmacologically acceptable salt thereof to a warm-blooded animal.

**54.** A method according to Claim 53 wherein the warm-blooded animal is a human.

**INTERNATIONAL SEARCH REPORT**

International application No.
PCT/JP2004/008492

A. CLASSIFICATION OF SUBJECT MATTER
Int.Cl[7] C07D401/14, 409/14, 471/04, A61K31/444, 31/437, A61P1/04, 1/16, 3/10, 9/10, 11/06, 13/12, 17/06, 19/00, 19/02, 19/10, 25/28, 29/00, 31/04, 31/12, 35/00, 37/02, 37/08, 43/00
According to International Patent Classification (IPC) or to both national classification and IPC

B. FIELDS SEARCHED
Minimum documentation searched (classification system followed by classification symbols)
Int.Cl[7] C07D401/14, 409/14, 471/04, A61K31/444, 31/437, A61P1/04, 1/16, 3/10, 9/10, 11/06, 13/12, 17/06, 19/00, 19/02, 19/10, 25/28, 29/00, 31/04, 31/12, 35/00, 37/02, 37/08, 43/00

Documentation searched other than minimum documentation to the extent that such documents are included in the fields searched

| | | | |
|---|---|---|---|
| Jitsuyo Shinan Koho | 1926-1996 | Jitsuyo Shinan Toroku Koho | 1996-2004 |
| Kokai Jitsuyo Shinan Koho | 1971-2004 | Toroku Jitsuyo Shinan Koho | 1994-2004 |

Electronic data base consulted during the international search (name of data base and, where practicable, search terms used)
CAPLUS/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 01/34632 A2 (MERCK & CO., INC.), 17 May, 2001 (17.05.01), & US 6528531 B1 | 1-42 |
| A | WO 01/34150 A1 (MERCK & CO., INC.), 17 May, 2001 (17.05.01), & US 6432980 B1 | 1-42 |
| A | WO 01/34149 A1 (MERCK & CO., INC.), 17 May, 2001 (17.05.01), & EP 1278520 A1 & JP 2003-527336 A & NZ 518718 A & US 6291480 B1 | 1-42 |

☐ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 July, 2004 (14.07.04) | Date of mailing of the international search report<br>03 August, 2004 (03.08.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/008492

**Box No. II Observations where certain claims were found unsearchable (Continuation of item 2 of first sheet)**

This international search report has not been established in respect of certain claims under Article 17(2)(a) for the following reasons:

1. [X] Claims Nos.: 43-54
because they relate to subject matter not required to be searched by this Authority, namely:
Claims 43-54 pertain to a method for treatment of the human body by therapy and thus relate to a subject matter for which this International Searching Authority is not required, under the provisions of Article 17(2)(a)(i) of the PCT and Rule 39.1(iv) of (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2004/008492

Continuation of Box No.II-1 of continuation of first sheet(2)

the Regulations under the PCT, to make an international search.

<With respect to subject matters for search>
·Claims 1-35 and 37-42

The subject matters of these claims of this application involve an extremely large number of compounds. However, the compounds which are disclosed in the meaning of Article 5 of the PCT are limited to an extremely small part of the compounds claimed. The compounds claimed are not sufficiently supported in the meaning of Article 6 of the PCT.

Therefore, a search was made for the part which is disclosed in and supported by the description, i.e., the compounds shown in Examples 1-80. With respect to the compounds in which $R^1$ is optionally substituted phenyl, $R^2$ is optionally substituted pyridyl, and A is an optionally substituted heterocycle, a complete search was made.

Form PCT/ISA/210 (extra sheet) (January 2004)